# EUROPEAN PATENT SPECIFICATION

(11) **EP 3 338 799 B1**
(45) Date of publication and mention of the grant of the patent: **07.04.2021**
(21) Application number: 17194768.2
(22) Date of filing: 24.11.2009
(51) Int. Cl.: C07K 16/24, A61P 9/00, A61P 7/00, A61P 39/00, A61P 37/00, A61P 3/00, A61P 21/00, A61P 17/00

(54) **ANTIBODIES TO IL-6 AND USE THEREOF**
ANTIKÖRPER GEGEN IL-6 UND IHRE VERWENDUNG
ANTICORPS ANTI-IL-6 ET UTILISATION DESDITS

(30) Priority: 25.11.2008 US 117811 P; 25.11.2008 US 117861 P; 25.11.2008 US 117839 P; 05.02.2009 US 366567; 24.02.2009 US 391717; 24.02.2009 US 391615; 06.03.2009 US 399156; 14.07.2009 US 502581
(43) Date of publication of application: 27.06.2018
(62) Divisional of application: 09830697.0
(73) Proprietor: Vitaeris Inc., Vancouver, BC V6C 2G8 (CA)
(72) Inventor: GARCIA-MARTINEZ, Leon, Woodinville, WA 98072 (US); CARVALHO-JENSEN, Anne Elisabeth, Mukilteo, WA 98725 (US); OLSON, Katie, Kenmore, WA 98028 (US); DUTZAR, Ben, Seattle, WA 98115 (US); LATHAM, JOHN, Seattle, WA 98119 (US); KOVACEVICH, Brian, Snohomish, WA 98296 (US); SMITH, Jeffrey T.L., Bellevue, WA 98004 (US); LITTON, Mark, Seattle, WA 98119 (US); SCHATZMAN, Randall, Redmond, WA 98053 (US)
(74) Representative: Boult Wade Tennant LLP

(56) References cited:
- WO-A1-2004/039826
- WO-A2-2008/065378
- WO-A2-2008/144763
- HENNIGAN STEPHANIE ET AL: "Interleukin-6 inhibitors in the treatment of rheumatoid arthritis", THERAPEUTICS AND CLINICAL RISK MANAGEMENT, DOVE MEDICAL PRESS LTD, NZ, vol. 4, no. 4, 1 August 2008 (2008-08-01), pages 767-775, XP002674098, ISSN: 1176-6336
- VAN ZAANEN H C T ET AL: "Endogenous interleukin 6 production in multiple myeloma patients treated with himeric monoclonal anti-IL6 antibodies indicates the existence of a positive feed-back loop", JOURNAL OF CLINICAL INVESTIGATION, B M J GROUP, GB, vol. 98, no. 6, 1 September 1996 (1996-09-01), pages 1441-1448, XP008092204, ISSN: 0021-9738, DOI: 10.1172/JCI118932

### SEQUENCE LISTING

<110> Smith, Jeffrey T.L.
   Schatzman, Randall C.
   Litton, Mark J.
   Latham, John
   Garcia, Leon
   Jensen, Anne Elisabeth Carvalho
   Olson, Katie
   Dutzar, Ben
   Kovacevich, Brian
<120> ANTIBODIES TO IL-6 AND USE THEREOF
<130> 67858.706002
<140> PCT/US2009/006274
   <141> 2009-11-24
<150> 61/117,861
   <151> 2008-11-25
<150> 61/117,839
   <151> 2008-11-25
<150> 61/117,811
   <151> 2008-11-25
<150> 12/502,581
   <151> 2009-07-14
<150> 12/399,156
   <151> 2009-03-06
<150> 12/391,717
   <151> 2009-02-24
<150> 12/366,567
   <151> 2009-02-05
<150> 12/391,615
   <151> 2009-02-24
<160> 742
<170> PatentIn version 3.5
<210> 1
   <211> 183
   <212> PRT
   <213> Homo sapiens
<400> 1
   Val Pro Pro Gly Glu Asp Ser Lys Asp Val Ala Ala Pro His Arg Gln
   1               5                   10                  15
   Pro Leu Thr Ser Ser Glu Arg Ile Asp Lys Gln Ile Arg Tyr Ile Leu
               20                  25                  30
   Asp Gly Ile Ser Ala Leu Arg Lys Glu Thr Cys Asn Lys Ser Asn Met
           35                  40                  45
   Cys Glu Ser Ser Lys Glu Ala Leu Ala Glu Asn Asn Leu Asn Leu Pro
       50                  55                  60
   Lys Met Ala Glu Lys Asp Gly Cys Phe Gln Ser Gly Phe Asn Glu Glu
   65                  70                  75                  80
   Thr Cys Leu Val Lys Ile Ile Thr Gly Leu Leu Glu Phe Glu Val Tyr
                   85                  90                  95
   Leu Glu Tyr Leu Gln Asn Arg Phe Glu Ser Ser Glu Glu Gln Ala Arg
               100                 105                 110
   Ala Val Gln Met Ser Thr Lys Val Leu Ile Gln Phe Leu Gln Lys Lys
           115                 120                 125
   Ala Lys Asn Leu Asp Ala Ile Thr Thr Pro Asp Pro Thr Thr Asn Ala
       130                 135                 140
   Ser Leu Leu Thr Lys Leu Gln Ala Gln Asn Gln Trp Leu Gln Asp Met
   145                 150                 155                 160
   Thr Thr His Leu Ile Leu Arg Ser Phe Lys Glu Phe Leu Gln Ser Ser
                   165                 170                 175
   Leu Arg Ala Leu Arg Gln Met
               180
<210> 2
   <211> 163
   <212> PRT
   <213> Oryctolagus cuniculus
<400> 2
   Met Asp Thr Arg Ala Pro Thr Gln Leu Leu Gly Leu Leu Leu Leu Trp
   1               5                   10                  15
   Leu Pro Gly Ala Arg Cys Ala Tyr Asp Met Thr Gln Thr Pro Ala Ser
               20                  25                  30
   Val Ser Ala Ala Val Gly Gly Thr Val Thr Ile Lys Cys Gln Ala Ser
           35                  40                  45
   Gln Ser Ile Asn Asn Glu Leu Ser Trp Tyr Gln Gln Lys Pro Gly Gln
       50                  55                  60
   Arg Pro Lys Leu Leu Ile Tyr Arg Ala Ser Thr Leu Ala Ser Gly Val
   65                  70                  75                  80
   Ser Ser Arg Phe Lys Gly Ser Gly Ser Gly Thr Glu Phe Thr Leu Thr
                   85                  90                  95
   Ile Ser Asp Leu Glu Cys Ala Asp Ala Ala Thr Tyr Tyr Cys Gln Gln
               100                 105                 110
   Gly Tyr Ser Leu Arg Asn Ile Asp Asn Ala Phe Gly Gly Gly Thr Glu
           115                 120                 125
   Val Val Val Lys Arg Thr Val Ala Ala Pro Ser Val Phe Ile Phe Pro
       130                 135                 140
   Pro Ser Asp Glu Gln Leu Lys Ser Gly Thr Ala Ser Val Val Cys Leu
   145                 150                 155                 160
   Leu Asn Asn
<210> 3
   <211> 166
   <212> PRT
   <213> Oryctolagus cuniculus
<400> 3
   Met Glu Thr Gly Leu Arg Trp Leu Leu Leu Val Ala Val Leu Lys Gly
   1               5                   10                  15
   Val Gln Cys Gln Ser Leu Glu Glu Ser Gly Gly Arg Leu Val Thr Pro
               20                  25                  30
   Gly Thr Pro Leu Thr Leu Thr Cys Thr Ala Ser Gly Phe Ser Leu Ser
           35                  40                  45
   Asn Tyr Tyr Val Thr Trp Val Arg Gln Ala Pro Gly Lys Gly Leu Glu
       50                  55                  60
   Trp Ile Gly Ile Ile Tyr Gly Ser Asp Glu Thr Ala Tyr Ala Thr Trp
   65                  70                  75                  80
   Ala Ile Gly Arg Phe Thr Ile Ser Lys Thr Ser Thr Thr Val Asp Leu
                   85                  90                  95
   Lys Met Thr Ser Leu Thr Ala Ala Asp Thr Ala Thr Tyr Phe Cys Ala
               100                 105                 110
   Arg Asp Asp Ser Ser Asp Trp Asp Ala Lys Phe Asn Leu Trp Gly Gln
           115                 120                 125
   Gly Thr Leu Val Thr Val Ser Ser Ala Ser Thr Lys Gly Pro Ser Val
       130                 135                 140
   Phe Pro Leu Ala Pro Ser Ser Lys Ser Thr Ser Gly Gly Thr Ala Ala
   145                 150                 155                 160
   Leu Gly Cys Leu Val Lys
                   165
<210> 4
   <211> 11
   <212> PRT
   <213> Oryctolagus cuniculus
<400> 4
   Gln Ala Ser Gln Ser Ile Asn Asn Glu Leu Ser
   1               5                   10
<210> 5
   <211> 7
   <212> PRT
   <213> Oryctolagus cuniculus
<400> 5
   Arg Ala Ser Thr Leu Ala Ser
   1               5
<210> 6
   <211> 12
   <212> PRT
   <213> Oryctolagus cuniculus
<400> 6
   Gln Gln Gly Tyr Ser Leu Arg Asn Ile Asp Asn Ala
   1               5                   10
<210> 7
   <211> 5
   <212> PRT
   <213> Oryctolagus cuniculus
<400> 7
   Asn Tyr Tyr Val Thr
   1               5
<210> 8
   <211> 16
   <212> PRT
   <213> Oryctolagus cuniculus
<400> 8
   Ile Ile Tyr Gly Ser Asp Glu Thr Ala Tyr Ala Thr Trp Ala Ile Gly
   1               5                   10                  15
<210> 9
   <211> 12
   <212> PRT
   <213> Oryctolagus cuniculus
<400> 9
   Asp Asp Ser Ser Asp Trp Asp Ala Lys Phe Asn Leu
   1               5                   10
<210> 10
   <211> 491
   <212> DNA
   <213> Oryctolagus cuniculus
<400> 10
   atggacacga gggcccccac tcagctgctg gggctcctgc tgctctggct cccaggtgcc      60
   agatgtgcct atgatatgac ccagactcca gcctcggtgt ctgcagctgt gggaggcaca     120
   gtcaccatca agtgccaggc cagtcagagc attaacaatg aattatcctg gtatcagcag     180
   aaaccagggc agcgtcccaa gctcctgatc tatagggcat ccactctggc atctggggtc     240
   tcatcgcggt tcaaaggcag tggatctggg acagagttca ctctcaccat cagcgacctg     300
   gagtgtgccg atgctgccac ttactactgt caacagggtt atagtctgag gaatattgat     360
   aatgctttcg gcggagggac cgaggtggtg gtcaaacgta cggtagcggc cccatctgtc     420
   ttcatcttcc cgccatctga tgagcagttg aaatctggaa ctgcctctgt tgtgtgcctg     480
   ctgaataact t                                                          491
<210> 11
   <211> 499
   <212> DNA
   <213> Oryctolagus cuniculus
<400> 11
   atggagactg ggctgcgctg gcttctcctg gtcgctgtgc tcaaaggtgt ccagtgtcag 60
   tcgctggagg agtccggggg tcgcctggtc acgcctggga cacccctgac actcacctgc 120
   acagcctctg gattctccct cagtaactac tacgtgacct gggtccgcca ggctccaggg 180
   aaggggctgg aatggatcgg aatcatttat ggtagtgatg aaacggccta cgcgacctgg 240
   gcgataggcc gattcaccat ctccaaaacc tcgaccacgg tggatctgaa aatgaccagt 300
   ctgacagccg cggacacggc cacctatttc tgtgccagag atgatagtag tgactgggat 360
   gcaaaattta acttgtgggg ccaaggcacc ctggtcaccg tctcgagcgc ctccaccaag 420
   ggcccatcgg tcttccccct ggcaccctcc tccaagagca cctctggggg cacagcggcc 480
   ctgggctgcc tggtcaagg 499
<210> 12
   <211> 33
   <212> DNA
   <213> Oryctolagus cuniculus
<400> 12
   caggccagtc agagcattaa caatgaatta tcc 33
<210> 13
   <211> 21
   <212> DNA
   <213> Oryctolagus cuniculus
<400> 13
   agggcatcca ctctggcatc t 21
<210> 14
   <211> 36
   <212> DNA
   <213> Oryctolagus cuniculus
<400> 14
   caacagggtt atagtctgag gaatattgat aatgct 36
<210> 15
   <211> 15
   <212> DNA
   <213> Oryctolagus cuniculus
<400> 15
   aactactacg tgacc 15
<210> 16
   <211> 48
   <212> DNA
   <213> Oryctolagus cuniculus
<400> 16
   atcatttatg gtagtgatga aacggcctac gcgacctggg cgataggc 48
<210> 17
   <211> 36
   <212> DNA
   <213> Oryctolagus cuniculus
<400> 17
   gatgatagta gtgactggga tgcaaaattt aacttg 36
<210> 18
   <211> 109
   <212> PRT
   <213> Oryctolagus cuniculus
<400> 18
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Ser Leu Ser Asn Tyr
               20                  25                  30
   Tyr Val Thr Trp Val Arg Gln Ala Pro Gly Lys Gly Leu Glu Trp Val
           35                  40                  45
   Gly Ile Ile Tyr Gly Ser Asp Glu Thr Ala Tyr Ala Thr Trp Ala Ile
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Arg Asp Asp Ser Ser Asp Trp Asp Ala Lys Phe Asn Leu
               100                 105
<210> 19
   <211> 109
   <212> PRT
   <213> Oryctolagus cuniculus
<400> 19
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Ser Leu Ser Asn Tyr
               20                  25                  30
   Tyr Val Thr Trp Val Arg Gln Ala Pro Gly Lys Gly Leu Glu Trp Val
           35                  40                  45
   Gly Ile Ile Tyr Gly Ser Asp Glu Thr Ala Tyr Ala Thr Ser Ala Ile
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Arg Asp Asp Ser Ser Asp Trp Asp Ala Lys Phe Asn Leu
               100                 105
<210> 20
   <211> 99
   <212> PRT
   <213> Oryctolagus cuniculus
<400> 20
   Ile Gln Met Thr Gln Ser Pro Ser Ser Leu Ser Ala Ser Val Gly Asp
   1               5                   10                  15
   Arg Val Thr Ile Thr Cys Gln Ala Ser Gln Ser Ile Asn Asn Glu Leu
               20                  25                  30
   Ser Trp Tyr Gln Gln Lys Pro Gly Lys Ala Pro Lys Leu Leu Ile Tyr
           35                  40                  45
   Arg Ala Ser Thr Leu Ala Ser Gly Val Pro Ser Arg Phe Ser Gly Ser
       50                  55                  60
   Gly Ser Gly Thr Asp Phe Thr Leu Thr Ile Ser Ser Leu Gln Pro Asp
   65                  70                  75                  80
   Asp Phe Ala Thr Tyr Tyr Cys Gln Gln Gly Tyr Ser Leu Arg Asn Ile
                   85                  90                  95
   Asp Asn Ala
<210> 21
   <211> 170
   <212> PRT
   <213> Oryctolagus cuniculus
<400> 21
   Met Asp Thr Arg Ala Pro Thr Gln Leu Leu Gly Leu Leu Leu Leu Trp
   1               5                   10                  15
   Leu Pro Gly Ala Arg Cys Ala Tyr Asp Met Thr Gln Thr Pro Ala Ser
               20                  25                  30
   Val Glu Val Ala Val Gly Gly Thr Val Thr Ile Asn Cys Gln Ala Ser
           35                  40                  45
   Glu Thr Ile Tyr Ser Trp Leu Ser Trp Tyr Gln Gln Lys Pro Gly Gln
       50                  55                  60
   Pro Pro Lys Leu Leu Ile Tyr Gln Ala Ser Asp Leu Ala Ser Gly Val
   65                  70                  75                  80
   Pro Ser Arg Phe Ser Gly Ser Gly Ala Gly Thr Glu Tyr Thr Leu Thr
                   85                  90                  95
   Ile Ser Gly Val Gln Cys Asp Asp Ala Ala Thr Tyr Tyr Cys Gln Gln
               100                 105                 110
   Gly Tyr Ser Gly Ser Asn Val Asp Asn Val Phe Gly Gly Gly Thr Glu
           115                 120                 125
   Val Val Val Lys Arg Thr Val Ala Ala Pro Ser Val Phe Ile Phe Pro
       130                 135                 140
   Pro Ser Asp Glu Gln Leu Lys Ser Gly Thr Ala Ser Val Val Cys Leu
   145                 150                 155                 160
   Leu Asn Asn Phe Tyr Pro Arg Glu Ala Lys
                   165                 170
<210> 22
   <211> 167
   <212> PRT
   <213> Oryctolagus cuniculus
<400> 22
   Met Glu Thr Gly Leu Arg Trp Leu Leu Leu Val Ala Val Leu Lys Gly
   1               5                   10                  15
   Val Gln Cys Gln Glu Gln Leu Lys Glu Ser Gly Gly Arg Leu Val Thr
               20                  25                  30
   Pro Gly Thr Pro Leu Thr Leu Thr Cys Thr Ala Ser Gly Phe Ser Leu
           35                  40                  45
   Asn Asp His Ala Met Gly Trp Val Arg Gln Ala Pro Gly Lys Gly Leu
       50                  55                  60
   Glu Tyr Ile Gly Phe Ile Asn Ser Gly Gly Ser Ala Arg Tyr Ala Ser
   65                  70                  75                  80
   Trp Ala Glu Gly Arg Phe Thr Ile Ser Arg Thr Ser Thr Thr Val Asp
                   85                  90                  95
   Leu Lys Met Thr Ser Leu Thr Thr Glu Asp Thr Ala Thr Tyr Phe Cys
               100                 105                 110
   Val Arg Gly Gly Ala Val Trp Ser Ile His Ser Phe Asp Pro Trp Gly
           115                 120                 125
   Pro Gly Thr Leu Val Thr Val Ser Ser Ala Ser Thr Lys Gly Pro Ser
       130                 135                 140
   Val Phe Pro Leu Ala Pro Ser Ser Lys Ser Thr Ser Gly Gly Thr Ala
   145                 150                 155                 160
   Ala Leu Gly Cys Leu Val Lys
                   165
<210> 23
   <211> 11
   <212> PRT
   <213> Oryctolagus cuniculus
<400> 23
   Gln Ala Ser Glu Thr Ile Tyr Ser Trp Leu Ser
   1               5                   10
<210> 24
   <211> 7
   <212> PRT
   <213> Oryctolagus cuniculus
<400> 24
   Gln Ala Ser Asp Leu Ala Ser
   1 5
<210> 25
   <211> 12
   <212> PRT
   <213> Oryctolagus cuniculus
<400> 25
   Gln Gln Gly Tyr Ser Gly Ser Asn Val Asp Asn Val
   1 5 10
<210> 26
   <211> 5
   <212> PRT
   <213> Oryctolagus cuniculus
<400> 26
   Asp His Ala Met Gly
   1 5
<210> 27
   <211> 16
   <212> PRT
   <213> Oryctolagus cuniculus
<400> 27
   Phe Ile Asn Ser Gly Gly Ser Ala Arg Tyr Ala Ser Trp Ala Glu Gly
   1 5 10 15
<210> 28
   <211> 12
   <212> PRT
   <213> Oryctolagus cuniculus
<400> 28
   Gly Gly Ala Val Trp Ser Ile His Ser Phe Asp Pro
   1 5 10
<210> 29
   <211> 511
   <212> DNA
   <213> Oryctolagus cuniculus
<400> 29
   atggacacga gggcccccac tcagctgctg gggctcctgc tgctctggct cccaggtgcc 60
   agatgtgcct atgatatgac ccagactcca gcctctgtgg aggtagctgt gggaggcaca 120
   gtcaccatca attgccaggc cagtgagacc atttacagtt ggttatcctg gtatcagcag 180
   aagccagggc agcctcccaa gctcctgatc taccaggcat ccgatctggc atctggggtc 240
   ccatcgcgat tcagcggcag tggggctggg acagagtaca ctctcaccat cagcggcgtg 300
   cagtgtgacg atgctgccac ttactactgt caacagggtt atagtggtag taatgttgat 360
   aatgttttcg gcggagggac cgaggtggtg gtcaaacgta cggtagcggc cccatctgtc 420
   ttcatcttcc cgccatctga tgagcagttg aaatctggaa ctgcctctgt tgtgtgcctg 480
   ctgaataact tctatcccag agaggccaaa g 511
<210> 30
   <211> 501
   <212> DNA
   <213> Oryctolagus cuniculus
<400> 30
   atggagactg ggctgcgctg gcttctcctg gtcgctgtgc tcaaaggtgt ccagtgtcag 60
   gagcagctga aggagtccgg gggtcgcctg gtcacgcctg ggacacccct gacacttacc 120
   tgcacagcct ctggattctc cctcaatgac catgcaatgg gctgggtccg ccaggctcca 180
   gggaaggggc tggaatacat cggattcatt aatagtggtg gtagcgcacg ctacgcgagc 240
   tgggcagaag gccgattcac catctccaga acctcgacca cggtggatct gaaaatgacc 300
   agtctgacaa ccgaggacac ggccacctat ttctgtgtca gagggggtgc tgtttggagt 360
   attcatagtt ttgatccctg gggcccaggg accctggtca ccgtctcgag cgcctccacc 420
   aagggcccat cggtcttccc cctggcaccc tcctccaaga gcacctctgg gggcacagcg 480
   gccctgggct gcctggtcaa g 501
<210> 31
   <211> 33
   <212> DNA
   <213> Oryctolagus cuniculus
<400> 31
   caggccagtg agaccattta cagttggtta tcc 33
<210> 32
   <211> 21
   <212> DNA
   <213> Oryctolagus cuniculus
<400> 32
   caggcatccg atctggcatc t 21
<210> 33
   <211> 36
   <212> DNA
   <213> Oryctolagus cuniculus
<400> 33
   caacagggtt atagtggtag taatgttgat aatgtt 36
<210> 34
   <211> 15
   <212> DNA
   <213> Oryctolagus cuniculus
<400> 34
   gaccatgcaa tgggc 15
<210> 35
   <211> 48
   <212> DNA
   <213> Oryctolagus cuniculus
<400> 35
   ttcattaata gtggtggtag cgcacgctac gcgagctggg cagaaggc 48
<210> 36
   <211> 36
   <212> DNA
   <213> Oryctolagus cuniculus
<400> 36
   gggggtgctg tttggagtat tcatagtttt gatccc 36
<210> 37
   <211> 165
   <212> PRT
   <213> Oryctolagus cuniculus
<400> 37
   Met Asp Thr Arg Ala Pro Thr Gln Leu Leu Gly Leu Leu Leu Leu Trp
   1               5                   10                  15
   Leu Pro Gly Ala Thr Phe Ala Ala Val Leu Thr Gln Thr Pro Ser Pro
               20                  25                  30
   Val Ser Ala Ala Val Gly Gly Thr Val Ser Ile Ser Cys Gln Ala Ser
           35                  40                  45
   Gln Ser Val Tyr Asp Asn Asn Tyr Leu Ser Trp Phe Gln Gln Lys Pro
       50                  55                  60
   Gly Gln Pro Pro Lys Leu Leu Ile Tyr Gly Ala Ser Thr Leu Ala Ser
   65                  70                  75                  80
   Gly Val Pro Ser Arg Phe Val Gly Ser Gly Ser Gly Thr Gln Phe Thr
                   85                  90                  95
   Leu Thr Ile Thr Asp Val Gln Cys Asp Asp Ala Ala Thr Tyr Tyr Cys
               100                 105                 110
   Ala Gly Val Tyr Asp Asp Asp Ser Asp Asn Ala Phe Gly Gly Gly Thr
           115                 120                 125
   Glu Val Val Val Lys Arg Thr Val Ala Ala Pro Ser Val Phe Ile Phe
       130                 135                 140
   Pro Pro Ser Asp Glu Gln Leu Lys Ser Gly Thr Ala Ser Val Val Cys
   145                 150                 155                 160
   Leu Leu Asn Asn Phe
                   165
<210> 38
   <211> 166
   <212> PRT
   <213> Oryctolagus cuniculus
<400> 38
   Met Glu Thr Gly Leu Arg Trp Leu Leu Leu Val Ala Val Leu Lys Gly
   1               5                   10                  15
   Val Gln Cys Gln Ser Leu Glu Glu Ser Gly Gly Arg Leu Val Thr Pro
               20                  25                  30
   Gly Thr Pro Leu Thr Leu Thr Cys Thr Ala Ser Gly Phe Ser Leu Ser
               35                  40                  45
   Val Tyr Tyr Met Asn Trp Val Arg Gln Ala Pro Gly Lys Gly Leu Glu
       50                  55                  60
   Trp Ile Gly Phe Ile Thr Met Ser Asp Asn Ile Asn Tyr Ala Ser Trp
   65                  70                  75                  80
   Ala Lys Gly Arg Phe Thr Ile Ser Lys Thr Ser Thr Thr Val Asp Leu
                   85                  90                  95
   Lys Met Thr Ser Pro Thr Thr Glu Asp Thr Ala Thr Tyr Phe Cys Ala
               100                 105                 110
   Arg Ser Arg Gly Trp Gly Thr Met Gly Arg Leu Asp Leu Trp Gly Pro
           115                 120                 125
   Gly Thr Leu Val Thr Val Ser Ser Ala Ser Thr Lys Gly Pro Ser Val
       130                 135                 140
   Phe Pro Leu Ala Pro Ser Ser Lys Ser Thr Ser Gly Gly Thr Ala Ala
   145                 150                 155                 160
   Leu Gly Cys Leu Val Lys
                   165
<210> 39
   <211> 13
   <212> PRT
   <213> Oryctolagus cuniculus
<400> 39
   Gln Ala Ser Gln Ser Val Tyr Asp Asn Asn Tyr Leu Ser
   1               5                   10
<210> 40
   <211> 7
   <212> PRT
   <213> Oryctolagus cuniculus
<400> 40
   Gly Ala Ser Thr Leu Ala Ser
   1               5
<210> 41
   <211> 11
   <212> PRT
   <213> Oryctolagus cuniculus
<400> 41
   Ala Gly Val Tyr Asp Asp Asp Ser Asp Asn Ala
   1 5 10
<210> 42
   <211> 5
   <212> PRT
   <213> Oryctolagus cuniculus
<400> 42
   Val Tyr Tyr Met Asn
   1 5
<210> 43
   <211> 16
   <212> PRT
   <213> Oryctolagus cuniculus
<400> 43
   Phe Ile Thr Met Ser Asp Asn Ile Asn Tyr Ala Ser Trp Ala Lys Gly
   1 5 10 15
<210> 44
   <211> 12
   <212> PRT
   <213> Oryctolagus cuniculus
<400> 44
   Ser Arg Gly Trp Gly Thr Met Gly Arg Leu Asp Leu
   1 5 10
<210> 45
   <211> 496
   <212> DNA
   <213> Oryctolagus cuniculus
<400> 45
   atggacacga gggcccccac tcagctgctg gggctcctgc tgctctggct cccaggtgcc      60
   acatttgccg ccgtgctgac ccagactcca tctcccgtgt ctgcagctgt gggaggcaca     120
   gtcagcatca gttgccaggc cagtcagagt gtttatgaca acaactactt atcctggttt     180
   cagcagaaac cagggcagcc tcccaagctc ctgatctatg gtgcatccac tctggcatct     240
   ggggtcccat cgcggttcgt gggcagtgga tctgggacac agttcactct caccatcaca     300
   gacgtgcagt gtgacgatgc tgccacttac tattgtgcag gcgtttatga tgatgatagt     360
   gataatgcct tcggcggagg gaccgaggtg gtggtcaaac gtacggtagc ggccccatct     420
   gtcttcatct tcccgccatc tgatgagcag ttgaaatctg gaactgcctc tgttgtgtgc     480
   ctgctgaata acttct                                                     496
<210> 46
   <211> 499
   <212> DNA
   <213> Oryctolagus cuniculus
<400> 46
   atggagactg ggctgcgctg gcttctcctg gtggctgtgc tcaaaggtgt ccagtgtcag      60
   tcgctggagg agtccggggg tcgcctggtc acccctggga cacccctgac actcacctgc     120
   acagcctctg gattctccct cagtgtctac tacatgaact gggtccgcca ggctccaggg     180
   aaggggctgg aatggatcgg attcattaca atgagtgata atataaatta cgcgagctgg     240
   gcgaaaggcc gattcaccat ctccaaaacc tcgaccacgg tggatctgaa aatgaccagt     300
   ccgacaaccg aggacacggc cacctatttc tgtgccagga gtcgtggctg gggtacaatg    360
   ggtcggttgg atctctgggg cccaggcacc ctcgtcaccg tctcgagcgc ctccaccaag     420
   ggcccatcgg tcttccccct ggcaccctcc tccaagagca cctctggggg cacagcggcc     480
   ctgggctgcc tggtcaagg                                                  499
<210> 47
   <211> 39
   <212> DNA
   <213> Oryctolagus cuniculus
<400> 47
   caggccagtc agagtgttta tgacaacaac tacttatcc 39
<210> 48
   <211> 21
   <212> DNA
   <213> Oryctolagus cuniculus
<400> 48
   ggtgcatcca ctctggcatc t 21
<210> 49
   <211> 33
   <212> DNA
   <213> Oryctolagus cuniculus
<400> 49
   gcaggcgttt atgatgatga tagtgataat gcc 33
<210> 50
   <211> 15
   <212> DNA
   <213> Oryctolagus cuniculus
<400> 50
   gtctactaca tgaac 15
<210> 51
   <211> 48
   <212> DNA
   <213> Oryctolagus cuniculus
<400> 51
   ttcattacaa tgagtgataa tataaattac gcgagctggg cgaaaggc 48
<210> 52
   <211> 36
   <212> DNA
   <213> Oryctolagus cuniculus
<400> 52
   agtcgtggct ggggtacaat gggtcggttg gatctc 36
<210> 53
   <211> 164
   <212> PRT
   <213> Oryctolagus cuniculus
<400> 53
   Met Asp Thr Arg Ala Pro Thr Gln Leu Leu Gly Leu Leu Leu Leu Trp
   1               5                   10                  15
   Leu Pro Gly Ala Ile Cys Asp Pro Val Leu Thr Gln Thr Pro Ser Pro
               20                  25                  30
   Val Ser Ala Pro Val Gly Gly Thr Val Ser Ile Ser Cys Gln Ala Ser
           35                  40                  45
   Gln Ser Val Tyr Glu Asn Asn Tyr Leu Ser Trp Phe Gln Gln Lys Pro
       50                  55                  60
   Gly Gln Pro Pro Lys Leu Leu Ile Tyr Gly Ala Ser Thr Leu Asp Ser
   65                  70                  75                  80
   Gly Val Pro Ser Arg Phe Lys Gly Ser Gly Ser Gly Thr Gln Phe Thr
                   85                  90                  95
   Leu Thr Ile Thr Asp Val Gln Cys Asp Asp Ala Ala Thr Tyr Tyr Cys
               100                 105                 110
   Ala Gly Val Tyr Asp Asp Asp Ser Asp Asp Ala Phe Gly Gly Gly Thr
           115                 120                 125
   Glu Val Val Val Lys Arg Thr Val Ala Ala Pro Ser Val Phe Ile Phe
       130                 135                 140
   Pro Pro Ser Asp Glu Gln Leu Lys Ser Gly Thr Ala Ser Val Val Cys
   145                 150                 155                 160
   Leu Leu Asn Asn
<210> 54
   <211> 167
   <212> PRT
   <213> Oryctolagus cuniculus
<400> 54
   Met Glu Thr Gly Leu Arg Trp Leu Leu Leu Val Ala Val Leu Lys Gly
   1 5 10 15
   Val Gln Cys Gln Glu Gln Leu Lys Glu Ser Gly Gly Gly Leu Val Thr
   20 25 30
   Pro Gly Gly Thr Leu Thr Leu Thr Cys Thr Ala Ser Gly Phe Ser Leu
   35 40 45
   Asn Ala Tyr Tyr Met Asn Trp Val Arg Gln Ala Pro Gly Lys Gly Leu
   50 55 60
   Glu Trp Ile Gly Phe Ile Thr Leu Asn Asn Asn Val Ala Tyr Ala Asn
   65 70 75 80
   Trp Ala Lys Gly Arg Phe Thr Phe Ser Lys Thr Ser Thr Thr Val Asp
   85 90 95
   Leu Lys Met Thr Ser Pro Thr Pro Glu Asp Thr Ala Thr Tyr Phe Cys
   100 105 110
   Ala Arg Ser Arg Gly Trp Gly Ala Met Gly Arg Leu Asp Leu Trp Gly
   115 120 125
   His Gly Thr Leu Val Thr Val Ser Ser Ala Ser Thr Lys Gly Pro Ser
   130 135 140
   Val Phe Pro Leu Ala Pro Ser Ser Lys Ser Thr Ser Gly Gly Thr Ala
   145 150 155 160
   Ala Leu Gly Cys Leu Val Lys
   165
<210> 55
   <211> 13
   <212> PRT
   <213> Oryctolagus cuniculus
<400> 55
   Gln Ala Ser Gln Ser Val Tyr Glu Asn Asn Tyr Leu Ser
   1 5 10
<210> 56
   <211> 7
   <212> PRT
   <213> Oryctolagus cuniculus
<400> 56
   Gly Ala Ser Thr Leu Asp Ser
   1               5
<210> 57
   <211> 11
   <212> PRT
   <213> Oryctolagus cuniculus
<400> 57
   Ala Gly Val Tyr Asp Asp Asp Ser Asp Asp Ala
   1               5                   10
<210> 58
   <211> 5
   <212> PRT
   <213> Oryctolagus cuniculus
<400> 58
   Ala Tyr Tyr Met Asn
   1               5
<210> 59
   <211> 16
   <212> PRT
   <213> Oryctolagus cuniculus
<400> 59
   Phe Ile Thr Leu Asn Asn Asn Val Ala Tyr Ala Asn Trp Ala Lys Gly
   1               5                   10                  15
<210> 60
   <211> 12
   <212> PRT
   <213> Oryctolagus cuniculus
<400> 60
   Ser Arg Gly Trp Gly Ala Met Gly Arg Leu Asp Leu
   1               5                   10
<210> 61
   <211> 494
   <212> DNA
   <213> Oryctolagus cuniculus
<400> 61
   atggacacga gggcccccac tcagctgctg gggctcctgc tgctctggct cccaggtgcc    60
   atatgtgacc ctgtgctgac ccagactcca tctcccgtat ctgcacctgt gggaggcaca   120
   gtcagcatca gttgccaggc cagtcagagt gtttatgaga acaactattt atcctggttt   180
   cagcagaaac cagggcagcc tcccaagctc ctgatctatg gtgcatccac tctggattct   240
   ggggtcccat cgcggttcaa aggcagtgga tctgggacac agttcactct caccattaca   300
   gacgtgcagt gtgacgatgc tgccacttac tattgtgcag gcgtttatga tgatgatagt   360
   gatgatgcct tcggcggagg gaccgaggtg gtggtcaaac gtacggtagc ggccccatct   420
   gtcttcatct tcccgccatc tgatgagcag ttgaaatctg gaactgcctc tgttgtgtgc   480
   ctgctgaata actt                                                     494
<210> 62
   <211> 502
   <212> DNA
   <213> Oryctolagus cuniculus
<400> 62
   atggagactg ggctgcgctg gcttctcctg gtggctgtgc tcaaaggtgt ccagtgtcag    60
   gagcagctga aggagtccgg aggaggcctg gtaacgcctg gaggaaccct gacactcacc   120
   tgcacagcct ctggattctc cctcaatgcc tactacatga actgggtccg ccaggctcca   180
   gggaaggggc tggaatggat cggattcatt actctgaata ataatgtagc ttacgcgaac   240
   tgggcgaaag gccgattcac cttctccaaa acctcgacca cggtggatct gaaaatgacc   300
   agtccgacac ccgaggacac ggccacctat ttctgtgcca ggagtcgtgg ctggggtgca   360
   atgggtcggt tggatctctg gggccatggc accctggtca ccgtctcgag cgcctccacc   420
   aagggcccat cggtcttccc cctggcaccc tcctccaaga gcacctctgg gggcacagcg   480
   gccctgggct gcctggtcaa gg                                            502
<210> 63
   <211> 39
   <212> DNA
   <213> Oryctolagus cuniculus
<400> 63
   caggccagtc agagtgttta tgagaacaac tatttatcc 39
<210> 64
   <211> 21
   <212> DNA
   <213> Oryctolagus cuniculus
<400> 64
   ggtgcatcca ctctggattc t 21
<210> 65
   <211> 33
   <212> DNA
   <213> Oryctolagus cuniculus
<400> 65
   gcaggcgttt atgatgatga tagtgatgat gcc 33
<210> 66
   <211> 15
   <212> DNA
   <213> Oryctolagus cuniculus
<400> 66
   gcctactaca tgaac 15
<210> 67
   <211> 48
   <212> DNA
   <213> Oryctolagus cuniculus
<400> 67
   ttcattactc tgaataataa tgtagcttac gcgaactggg cgaaaggc 48
<210> 68
   <211> 36
   <212> DNA
   <213> Oryctolagus cuniculus
<400> 68
   agtcgtggct ggggtgcaat gggtcggttg gatctc 36
<210> 69
   <211> 164
   <212> PRT
   <213> Oryctolagus cuniculus
<400> 69
   Met Asp Thr Arg Ala Pro Thr Gln Leu Leu Gly Leu Leu Leu Leu Trp
   1               5                   10                  15
   Leu Pro Gly Ala Thr Phe Ala Gln Val Leu Thr Gln Thr Pro Ser Pro
               20                  25                  30
   Val Ser Ala Ala Val Gly Gly Thr Val Thr Ile Asn Cys Gln Ala Ser
           35                  40                  45
   Gln Ser Val Asp Asp Asn Asn Trp Leu Gly Trp Tyr Gln Gln Lys Arg
       50                  55                  60
   Gly Gln Pro Pro Lys Tyr Leu Ile Tyr Ser Ala Ser Thr Leu Ala Ser
   65                  70                  75                  80
   Gly Val Pro Ser Arg Phe Lys Gly Ser Gly Ser Gly Thr Gln Phe Thr
   85 90 95
   Leu Thr Ile Ser Asp Leu Glu Cys Asp Asp Ala Ala Thr Tyr Tyr Cys
   100 105 110
   Ala Gly Gly Phe Ser Gly Asn Ile Phe Ala Phe Gly Gly Gly Thr Glu
   115 120 125
   Val Val Val Lys Arg Thr Val Ala Ala Pro Ser Val Phe Ile Phe Pro
   130 135 140
   Pro Ser Asp Glu Gln Leu Lys Ser Gly Thr Ala Ser Val Val Cys Leu
   145 150 155 160
   Leu Asn Asn Phe
<210> 70
   <211> 164
   <212> PRT
   <213> Oryctolagus cuniculus
<400> 70
   Met Glu Thr Gly Leu Arg Trp Leu Leu Leu Val Ala Val Leu Lys Gly
   1 5 10 15
   Val Gln Cys Gln Ser Val Glu Glu Ser Gly Gly Arg Leu Val Thr Pro
   20 25 30
   Gly Thr Pro Leu Thr Leu Thr Cys Thr Val Ser Gly Phe Ser Leu Ser
   35 40 45
   Ser Tyr Ala Met Ser Trp Val Arg Gln Ala Pro Gly Lys Gly Leu Glu
   50 55 60
   Trp Ile Gly Ile Ile Gly Gly Phe Gly Thr Thr Tyr Tyr Ala Thr Trp
   65 70 75 80
   Ala Lys Gly Arg Phe Thr Ile Ser Lys Thr Ser Thr Thr Val Asp Leu
   85 90 95
   Arg Ile Thr Ser Pro Thr Thr Glu Asp Thr Ala Thr Tyr Phe Cys Ala
   100 105 110
   Arg Gly Gly Pro Gly Asn Gly Gly Asp Ile Trp Gly Gln Gly Thr Leu
   115 120 125
   Val Thr Val Ser Ser Ala Ser Thr Lys Gly Pro Ser Val Phe Pro Leu
       130                 135                 140
   Ala Pro Ser Ser Lys Ser Thr Ser Gly Gly Thr Ala Ala Leu Gly Cys
   145                 150                 155                 160
   Leu Val Lys Asp
<210> 71
   <211> 13
   <212> PRT
   <213> Oryctolagus cuniculus
<400> 71
   Gln Ala Ser Gln Ser Val Asp Asp Asn Asn Trp Leu Gly
   1               5                   10
<210> 72
   <211> 7
   <212> PRT
   <213> Oryctolagus cuniculus
<400> 72
   Ser Ala Ser Thr Leu Ala Ser
   1               5
<210> 73
   <211> 10
   <212> PRT
   <213> Oryctolagus cuniculus
<400> 73
   Ala Gly Gly Phe Ser Gly Asn Ile Phe Ala
   1               5                   10
<210> 74
   <211> 5
   <212> PRT
   <213> Oryctolagus cuniculus
<400> 74
   Ser Tyr Ala Met Ser
   1               5
<210> 75
   <211> 16
   <212> PRT
   <213> Oryctolagus cuniculus
<400> 75
   Ile Ile Gly Gly Phe Gly Thr Thr Tyr Tyr Ala Thr Trp Ala Lys Gly
   1               5                   10                  15
<210> 76
   <211> 9
   <212> PRT
   <213> Oryctolagus cuniculus
<400> 76
   Gly Gly Pro Gly Asn Gly Gly Asp Ile
   1               5
<210> 77
   <211> 493
   <212> DNA
   <213> Oryctolagus cuniculus
<400> 77
   atggacacga gggcccccac tcagctgctg gggctcctgc tgctctggct cccaggtgcc      60
   acatttgccc aagtgctgac ccagactcca tcgcctgtgt ctgcagctgt gggaggcaca     120
   gtcaccatca actgccaggc cagtcagagt gttgatgata acaactggtt aggctggtat     180
   cagcagaaac gagggcagcc tcccaagtac ctgatctatt ctgcatccac tctggcatct     240
   ggggtcccat cgcggttcaa aggcagtgga tctgggacac agttcactct caccatcagc     300
   gacctggagt gtgacgatgc tgccacttac tactgtgcag gcggttttag tggtaatatc     360
   tttgctttcg gcggagggac cgaggtggtg gtcaaacgta cggtagcggc cccatctgtc     420
   ttcatcttcc cgccatctga tgagcagttg aaatctggaa ctgcctctgt tgtgtgcctg     480
   ctgaataact tct                                                        493
<210> 78
   <211> 493
   <212> DNA
   <213> Oryctolagus cuniculus
<400> 78
   atggagactg ggctgcgctg gcttctcctg gtcgctgtgc tcaaaggtgt ccagtgtcag      60
   tcggtggagg agtccggggg tcgcctggtc acgcctggga cacccctgac actcacctgc     120
   acagtctctg gcttctccct cagtagctat gcaatgagct gggtccgcca ggctccagga     180
   aaggggctgg agtggatcgg aatcattggt ggttttggta ccacatacta cgcgacctgg     240
   gcgaaaggcc gattcaccat ctccaaaacc tcgaccacgg tggatctgag aatcaccagt     300
   ccgacaaccg aggacacggc cacctatttc tgtgccagag gtggtcctgg taatggtggt     360
   gacatctggg gccaagggac cctggtcacc gtctcgagcg cctccaccaa gggcccatcg     420
   gtcttccccc tggcaccctc ctccaagagc acctctgggg gcacagcggc cctgggctgc     480
   ctggtcaagg act                                                        493
<210> 79
   <211> 39
   <212> DNA
   <213> Oryctolagus cuniculus
<400> 79
   caggccagtc agagtgttga tgataacaac tggttaggc 39
<210> 80
   <211> 21
   <212> DNA
   <213> Oryctolagus cuniculus
<400> 80
   tctgcatcca ctctggcatc t 21
<210> 81
   <211> 30
   <212> DNA
   <213> Oryctolagus cuniculus
<400> 81
   gcaggcggtt ttagtggtaa tatctttgct 30
<210> 82
   <211> 15
   <212> DNA
   <213> Oryctolagus cuniculus
<400> 82
   agctatgcaa tgagc 15
<210> 83
   <211> 48
   <212> DNA
   <213> Oryctolagus cuniculus
<400> 83
   atcattggtg gttttggtac cacatactac gcgacctggg cgaaaggc 48
<210> 84
   <211> 27
   <212> DNA
   <213> Oryctolagus cuniculus
<400> 84
   ggtggtcctg gtaatggtgg tgacatc 27
<210> 85
   <211> 164
   <212> PRT
   <213> Oryctolagus cuniculus
<400> 85
   Met Asp Thr Arg Ala Pro Thr Gln Leu Leu Gly Leu Leu Leu Leu Trp
   1               5                   10                  15
   Leu Pro Gly Ala Thr Phe Ala Ala Val Leu Thr Gln Thr Pro Ser Pro
               20                  25                  30
   Val Ser Val Pro Val Gly Gly Thr Val Thr Ile Lys Cys Gln Ser Ser
           35                  40                  45
   Gln Ser Val Tyr Asn Asn Phe Leu Ser Trp Tyr Gln Gln Lys Pro Gly
       50                  55                  60
   Gln Pro Pro Lys Leu Leu Ile Tyr Gln Ala Ser Lys Leu Ala Ser Gly
   65                  70                  75                  80
   Val Pro Asp Arg Phe Ser Gly Ser Gly Ser Gly Thr Gln Phe Thr Leu
                   85                  90                  95
   Thr Ile Ser Gly Val Gln Cys Asp Asp Ala Ala Thr Tyr Tyr Cys Leu
               100                 105                 110
   Gly Gly Tyr Asp Asp Asp Ala Asp Asn Ala Phe Gly Gly Gly Thr Glu
           115                 120                 125
   Val Val Val Lys Arg Thr Val Ala Ala Pro Ser Val Phe Ile Phe Pro
       130                 135                 140
   Pro Ser Asp Glu Gln Leu Lys Ser Gly Thr Ala Ser Val Val Cys Leu
   145                 150                 155                 160
   Leu Asn Asn Phe
<210> 86
   <211> 170
   <212> PRT
   <213> Oryctolagus cuniculus
<400> 86
   Met Glu Thr Gly Leu Arg Trp Leu Leu Leu Val Ala Val Leu Lys Gly
   1               5                   10                  15
   Val Gln Cys Gln Ser Val Glu Glu Ser Gly Gly Arg Leu Val Thr Pro
               20                  25                  30
   Gly Thr Pro Leu Thr Leu Thr Cys Thr Val Ser Gly Ile Asp Leu Ser
           35                  40                  45
   Asp Tyr Ala Met Ser Trp Val Arg Gln Ala Pro Gly Lys Gly Leu Glu
       50                  55                  60
   Trp Ile Gly Ile Ile Tyr Ala Gly Ser Gly Ser Thr Trp Tyr Ala Ser
   65                  70                  75                  80
   Trp Ala Lys Gly Arg Phe Thr Ile Ser Lys Thr Ser Thr Thr Val Asp
                   85                  90                  95
   Leu Lys Ile Thr Ser Pro Thr Thr Glu Asp Thr Ala Thr Tyr Phe Cys
               100                 105                 110
   Ala Arg Asp Gly Tyr Asp Asp Tyr Gly Asp Phe Asp Arg Leu Asp Leu
           115                 120                 125
   Trp Gly Pro Gly Thr Leu Val Thr Val Ser Ser Ala Ser Thr Lys Gly
       130                 135                 140
   Pro Ser Val Phe Pro Leu Ala Pro Ser Ser Lys Ser Thr Ser Gly Gly
   145                 150                 155                 160
   Thr Ala Ala Leu Gly Cys Leu Val Lys Asp
                   165                 170
<210> 87
   <211> 12
   <212> PRT
   <213> Oryctolagus cuniculus
<400> 87
   Gln Ser Ser Gln Ser Val Tyr Asn Asn Phe Leu Ser
   1               5                   10
<210> 88
   <211> 7
   <212> PRT
   <213> Oryctolagus cuniculus
<400> 88
   Gln Ala Ser Lys Leu Ala Ser
   1 5
<210> 89
   <211> 11
   <212> PRT
   <213> Oryctolagus cuniculus
<400> 89
   Leu Gly Gly Tyr Asp Asp Asp Ala Asp Asn Ala
   1 5 10
<210> 90
   <211> 5
   <212> PRT
   <213> Oryctolagus cuniculus
<400> 90
   Asp Tyr Ala Met Ser
   1 5
<210> 91
   <211> 17
   <212> PRT
   <213> Oryctolagus cuniculus
<400> 91
   Ile Ile Tyr Ala Gly Ser Gly Ser Thr Trp Tyr Ala Ser Trp Ala Lys
   1 5 10 15
   Gly
<210> 92
   <211> 14
   <212> PRT
   <213> Oryctolagus cuniculus
<400> 92
   Asp Gly Tyr Asp Asp Tyr Gly Asp Phe Asp Arg Leu Asp Leu
   1 5 10
<210> 93
   <211> 492
   <212> DNA
   <213> Oryctolagus cuniculus
<400> 93
   atggacacga gggcccccac tcagctgctg gggctcctgc tgctctggct cccaggtgcc 60
   acatttgcag ccgtgctgac ccagacacca tcgcccgtgt ctgtacctgt gggaggcaca 120
   gtcaccatca agtgccagtc cagtcagagt gtttataata atttcttatc gtggtatcag 180
   cagaaaccag ggcagcctcc caagctcctg atctaccagg catccaaact ggcatctggg 240
   gtcccagata ggttcagcgg cagtggatct gggacacagt tcactctcac catcagcggc 300
   gtgcagtgtg acgatgctgc cacttactac tgtctaggcg gttatgatga tgatgctgat 360
   aatgctttcg gcggagggac cgaggtggtg gtcaaacgta cggtagcggc cccatctgtc 420
   ttcatcttcc cgccatctga tgagcagttg aaatctggaa ctgcctctgt tgtgtgcctg 480
   ctgaataact tc 492
<210> 94
   <211> 511
   <212> DNA
   <213> Oryctolagus cuniculus
<400> 94
   atggagactg ggctgcgctg gcttctcctg gtcgctgtgc tcaaaggtgt ccagtgtcag 60
   tcggtggagg agtccggggg tcgcctggtc acgcctggga cacccctgac gctcacctgc 120
   acagtctctg gaatcgacct cagtgactat gcaatgagct gggtccgcca ggctccaggg 180
   aaggggctgg aatggatcgg aatcatttat gctggtagtg gtagcacatg gtacgcgagc 240
   tgggcgaaag gccgattcac catctccaaa acctcgacca cggtggatct gaaaatcacc 300
   agtccgacaa ccgaggacac ggccacctat ttctgtgcca gagatggata cgatgactat 360
   ggtgatttcg atcgattgga tctctggggc ccaggcaccc tcgtcaccgt ctcgagcgcc 420
   tccaccaagg gcccatcggt cttccccctg gcaccctcct ccaagagcac ctctgggggc 480
   acagcggccc tgggctgcct ggtcaaggac t 511
<210> 95
   <211> 36
   <212> DNA
   <213> Oryctolagus cuniculus
<400> 95
   cagtccagtc agagtgttta taataatttc ttatcg 36
<210> 96
   <211> 21
   <212> DNA
   <213> Oryctolagus cuniculus
<400> 96
   caggcatcca aactggcatc t 21
<210> 97
   <211> 33
   <212> DNA
   <213> Oryctolagus cuniculus
<400> 97
   ctaggcggtt atgatgatga tgctgataat gct 33
<210> 98
   <211> 15
   <212> DNA
   <213> Oryctolagus cuniculus
<400> 98
   gactatgcaa tgagc 15
<210> 99
   <211> 51
   <212> DNA
   <213> Oryctolagus cuniculus
<400> 99
   atcatttatg ctggtagtgg tagcacatgg tacgcgagct gggcgaaagg c 51
<210> 100
   <211> 42
   <212> DNA
   <213> Oryctolagus cuniculus
<400> 100
   gatggatacg atgactatgg tgatttcgat cgattggatc tc 42
<210> 101
   <211> 164
   <212> PRT
   <213> Oryctolagus cuniculus
<400> 101 Leu Asn Asn Phe
<210> 102
   <211> 166
   <212> PRT
   <213> Oryctolagus cuniculus
<400> 102
   Met Glu Thr Gly Leu Arg Trp Leu Leu Leu Val Ala Val Leu Ser Gly
   1               5                   10                  15
   Val Gln Cys Gln Ser Leu Glu Glu Ser Gly Gly Arg Leu Val Thr Pro
               20                  25                  30
   Gly Thr Pro Leu Thr Leu Thr Cys Thr Ala Ser Gly Phe Ser Leu Ser
           35                  40                  45
   Asn Tyr Tyr Met Thr Trp Val Arg Gln Ala Pro Gly Lys Gly Leu Glu
       50                  55                  60
   Trp Ile Gly Met Ile Tyr Gly Ser Asp Glu Thr Ala Tyr Ala Asn Trp
   65                  70                  75                  80
   Ala Ile Gly Arg Phe Thr Ile Ser Lys Thr Ser Thr Thr Val Asp Leu
                   85                  90                  95
   Lys Met Thr Ser Leu Thr Ala Ala Asp Thr Ala Thr Tyr Phe Cys Ala
               100                 105                 110
   Arg Asp Asp Ser Ser Asp Trp Asp Ala Lys Phe Asn Leu Trp Gly Gln
           115                 120                 125
   Gly Thr Leu Val Thr Val Ser Ser Ala Ser Thr Lys Gly Pro Ser Val
       130                 135                 140
   Phe Pro Leu Ala Pro Ser Ser Lys Ser Thr Ser Gly Gly Thr Ala Ala
   145                 150                 155                 160
   Leu Gly Cys Leu Val Lys
                   165
<210> 103
   <211> 11
   <212> PRT
   <213> Oryctolagus cuniculus
<400> 103
   Gln Ala Ser Gln Ser Ile Asn Asn Glu Leu Ser
   1               5                   10
<210> 104
   <211> 7
   <212> PRT
   <213> Oryctolagus cuniculus
<400> 104
   Arg Ala Ser Thr Leu Ala Ser
   1               5
<210> 105
   <211> 12
   <212> PRT
   <213> Oryctolagus cuniculus
<400> 105
   Gln Gln Gly Tyr Ser Leu Arg Asn Ile Asp Asn Ala
   1               5                   10
<210> 106
   <211> 5
   <212> PRT
   <213> Oryctolagus cuniculus
<400> 106
   Asn Tyr Tyr Met Thr
   1               5
<210> 107
   <211> 16
   <212> PRT
   <213> Oryctolagus cuniculus
<400> 107
   Met Ile Tyr Gly Ser Asp Glu Thr Ala Tyr Ala Asn Trp Ala Ile Gly
   1               5                   10                  15
<210> 108
   <211> 12
   <212> PRT
   <213> Oryctolagus cuniculus
<400> 108
   Asp Asp Ser Ser Asp Trp Asp Ala Lys Phe Asn Leu
   1               5                   10
<210> 109
   <211> 492
   <212> DNA
   <213> Oryctolagus cuniculus
<400> 109
   atggacacga gggcccccac tcagctgctg gggctcctgc tgctctggct cccaggtgcc     60
   agatgtgcct atgatatgac ccagactcca gcctcggtgt ctgcagctgt gggaggcaca    120
   gtcaccatca aatgccaggc cagtcagagc attaacaatg aattatcctg gtatcagcag    180
   aaatcagggc agcgtcccaa gctcctgatc tatagggcat ccactctggc atctggggtc    240
   tcatcgcggt tcaaaggcag tggatctggg acagagttca ctctcaccat cagcgacctg    300
   gagtgtgccg atgctgccac ttactactgt caacagggtt atagtctgag gaatattgat    360
   aatgctttcg gcggagggac cgaggtggtg gtcaaacgta cggtagcggc cccatctgtc    420
   ttcatcttcc cgccatctga tgagcagttg aaatctggaa ctgcctctgt tgtgtgcctg    480
   ctgaataact tc                                                         492
<210> 110
   <211> 499
   <212> DNA
   <213> Oryctolagus cuniculus
<400> 110
   atggagactg ggctgcgctg gcttctcctg gtcgctgtgc tctcaggtgt ccagtgtcag     60
   tcgctggagg agtccggggg tcgcctggtc acgcctggga cacccctgac actcacctgc    120
   acagcctctg gattctccct cagtaactac tacatgacct gggtccgcca ggctccaggg    180
   aaggggctgg aatggatcgg aatgatttat ggtagtgatg aaacagccta cgcgaactgg    240
   gcgataggcc gattcaccat ctccaaaacc tcgaccacgg tggatctgaa aatgaccagt    300
   ctgacagccg cggacacggc cacctatttc tgtgccagag atgatagtag tgactgggat    360
   gcaaaattta acttgtgggg ccaagggacc ctcgtcaccg tctcgagcgc ctccaccaag    420
   ggcccatcgg tcttccccct ggcaccctcc tccaagagca cctctggggg cacagcggcc    480
   ctgggctgcc tggtcaagg                                                  499
<210> 111
   <211> 33
   <212> DNA
   <213> Oryctolagus cuniculus
<400> 111
   caggccagtc agagcattaa caatgaatta tcc 33
<210> 112
   <211> 21
   <212> DNA
   <213> Oryctolagus cuniculus
<400> 112
   agggcatcca ctctggcatc t 21
<210> 113
   <211> 36
   <212> DNA
   <213> Oryctolagus cuniculus
<400> 113
   caacagggtt atagtctgag gaatattgat aatgct 36
<210> 114
   <211> 15
   <212> DNA
   <213> Oryctolagus cuniculus
<400> 114
   aactactaca tgacc 15
<210> 115
   <211> 48
   <212> DNA
   <213> Oryctolagus cuniculus
<400> 115
   atgatttatg gtagtgatga aacagcctac gcgaactggg cgataggc 48
<210> 116
   <211> 36
   <212> DNA
   <213> Oryctolagus cuniculus
<400> 116
   gatgatagta gtgactggga tgcaaaattt aacttg 36
<210> 117
   <211> 109
   <212> PRT
   <213> Oryctolagus cuniculus
<400> 117
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Ser Leu Ser Asn Tyr
               20                  25                  30
   Tyr Met Thr Trp Val Arg Gln Ala Pro Gly Lys Gly Leu Glu Trp Val
           35                  40                  45
   Gly Met Ile Tyr Gly Ser Asp Glu Thr Ala Tyr Ala Asn Trp Ala Ile
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
   85 90 95
   Arg Asp Asp Ser Ser Asp Trp Asp Ala Lys Phe Asn Leu
   100 105
<210> 118
   <211> 109
   <212> PRT
   <213> Oryctolagus cuniculus
<400> 118
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Ser Leu Ser Asn Tyr
   20 25 30
   Tyr Met Thr Trp Val Arg Gln Ala Pro Gly Lys Gly Leu Glu Trp Val
   35 40 45
   Gly Met Ile Tyr Gly Ser Asp Glu Thr Ala Tyr Ala Asn Ser Ala Ile
   50 55 60
   Gly Arg Phe Thr Ile Ser Arg Asp Asn Ser Lys Asn Thr Leu Tyr Leu
   65 70 75 80
   Gln Met Asn Ser Leu Arg Ala Glu Asp Thr Ala Val Tyr Tyr Cys Ala
   85 90 95
   Arg Asp Asp Ser Ser Asp Trp Asp Ala Lys Phe Asn Leu
   100 105
<210> 119
   <211> 100
   <212> PRT
   <213> Oryctolagus cuniculus
<400> 119
   Asp Ile Gln Met Thr Gln Ser Pro Ser Thr Leu Ser Ala Ser Val Gly
   1 5 10 15
   Asp Arg Val Thr Ile Thr Cys Gln Ala Ser Gln Ser Ile Asn Asn Glu
   20 25 30
   Leu Ser Trp Tyr Gln Gln Lys Pro Gly Lys Ala Pro Lys Leu Leu Ile
   35 40 45
   Tyr Arg Ala Ser Thr Leu Ala Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Asp Asp Phe Ala Thr Tyr Tyr Cys Gln Gln Gly Tyr Ser Leu Arg Asn
                   85                  90                  95
   Ile Asp Asn Ala
               100
<210> 120
   <211> 16
   <212> PRT
   <213> Oryctolagus cuniculus
<400> 120
   Ile Ile Tyr Gly Ser Asp Glu Thr Ala Tyr Ala Thr Ser Ala Ile Gly
   1               5                   10                  15
<210> 121
   <211> 16
   <212> PRT
   <213> Oryctolagus cuniculus
<400> 121
   Met Ile Tyr Gly Ser Asp Glu Thr Ala Tyr Ala Asn Ser Ala Ile Gly
   1               5                   10                  15
<210> 122
   <211> 123
   <212> PRT
   <213> Oryctolagus cuniculus
<400> 122
   Met Asp Thr Arg Ala Pro Thr Gln Leu Leu Gly Leu Leu Leu Leu Trp
   1               5                   10                  15
   Leu Pro Gly Ala Thr Phe Ala Ala Val Leu Thr Gln Thr Pro Ser Pro
               20                  25                  30
   Val Ser Ala Ala Val Gly Gly Thr Val Thr Ile Ser Cys Gln Ser Ser
           35                  40                  45
   Gln Ser Val Gly Asn Asn Gln Asp Leu Ser Trp Phe Gln Gln Arg Pro
       50                  55                  60
   Gly Gln Pro Pro Lys Leu Leu Ile Tyr Glu Ile Ser Lys Leu Glu Ser
   65                  70                  75                  80
   Gly Val Pro Ser Arg Phe Ser Gly Ser Gly Ser Gly Thr His Phe Thr
                   85                  90                  95
   Leu Thr Ile Ser Gly Val Gln Cys Asp Asp Ala Ala Thr Tyr Tyr Cys
               100                 105                 110
   Leu Gly Gly Tyr Asp Asp Asp Ala Asp Asn Ala
           115                 120
<210> 123
   <211> 128
   <212> PRT
   <213> Oryctolagus cuniculus
<400> 123
   Met Glu Thr Gly Leu Arg Trp Leu Leu Leu Val Ala Val Leu Lys Gly
   1               5                   10                  15
   Val Gln Cys His Ser Val Glu Glu Ser Gly Gly Arg Leu Val Thr Pro
               20                  25                  30
   Gly Thr Pro Leu Thr Leu Thr Cys Thr Val Ser Gly Phe Ser Leu Ser
           35                  40                  45
   Ser Arg Thr Met Ser Trp Val Arg Gln Ala Pro Gly Lys Gly Leu Glu
       50                  55                  60
   Trp Ile Gly Tyr Ile Trp Ser Gly Gly Ser Thr Tyr Tyr Ala Thr Trp
   65                  70                  75                  80
   Ala Lys Gly Arg Phe Thr Ile Ser Lys Thr Ser Thr Thr Val Asp Leu
                   85                  90                  95
   Lys Ile Thr Ser Pro Thr Thr Glu Asp Thr Ala Thr Tyr Phe Cys Ala
               100                 105                 110
   Arg Leu Gly Asp Thr Gly Gly His Ala Tyr Ala Thr Arg Leu Asn Leu
           115                 120                 125
<210> 124
   <211> 13
   <212> PRT
   <213> Oryctolagus cuniculus
<400> 124
   Gln Ser Ser Gln Ser Val Gly Asn Asn Gln Asp Leu Ser
   1               5                   10
<210> 125
   <211> 7
   <212> PRT
   <213> Oryctolagus cuniculus
<400> 125
   Glu Ile Ser Lys Leu Glu Ser
   1               5
<210> 126
   <211> 11
   <212> PRT
   <213> Oryctolagus cuniculus
<400> 126
   Leu Gly Gly Tyr Asp Asp Asp Ala Asp Asn Ala
   1               5                   10
<210> 127
   <211> 5
   <212> PRT
   <213> Oryctolagus cuniculus
<400> 127
   Ser Arg Thr Met Ser
   1               5
<210> 128
   <211> 16
   <212> PRT
   <213> Oryctolagus cuniculus
<400> 128
   Tyr Ile Trp Ser Gly Gly Ser Thr Tyr Tyr Ala Thr Trp Ala Lys Gly
   1               5                   10                  15
<210> 129
   <211> 15
   <212> PRT
   <213> Oryctolagus cuniculus
<400> 129
   Leu Gly Asp Thr Gly Gly His Ala Tyr Ala Thr Arg Leu Asn Leu
   1               5                   10                  15
<210> 130
   <211> 369
   <212> DNA
   <213> Oryctolagus cuniculus
<400> 130
   atggacacga gggcccccac tcagctgctg gggctcctgc tgctctggct cccaggtgcc 60
   acatttgcag ccgtgctgac ccagacacca tcacccgtgt ctgcagctgt gggaggcaca 120
   gtcaccatca gttgccagtc cagtcagagt gttggtaata accaggactt atcctggttt 180
   cagcagagac cagggcagcc tcccaagctc ctgatctacg aaatatccaa actggaatct 240
   ggggtcccat cgcggttcag cggcagtgga tctgggacac acttcactct caccatcagc 300
   ggcgtacagt gtgacgatgc tgccacttac tactgtctag gcggttatga tgatgatgct 360
   gataatgct 369
<210> 131
   <211> 384
   <212> DNA
   <213> Oryctolagus cuniculus
<400> 131
   atggagactg ggctgcgctg gcttctcctg gtcgctgtgc tcaaaggtgt ccagtgtcac       60
   tcggtggagg agtccggggg tcgcctggtc acgcctggga cacccctgac actcacctgc      120
   acagtctctg gattctccct cagtagtcgt acaatgtcct gggtccgcca ggctccaggg      180
   aaggggctgg agtggatcgg atacatttgg agtggtggta gcacatacta cgcgacctgg      240
   gcgaaaggcc gattcaccat ctccaaaacc tcgaccacgg tggatctgaa aatcaccagt      300
   ccgacaaccg aggacacggc cacctatttc tgtgccagat tgggcgatac tggtggtcac      360
   gcttatgcta ctcgcttaaa tctc                                             384
<210> 132
   <211> 39
   <212> DNA
   <213> Oryctolagus cuniculus
<400> 132
   cagtccagtc agagtgttgg taataaccag gacttatcc 39
<210> 133
   <211> 21
   <212> DNA
   <213> Oryctolagus cuniculus
<400> 133
   gaaatatcca aactggaatc t 21
<210> 134
   <211> 33
   <212> DNA
   <213> Oryctolagus cuniculus
<400> 134
   ctaggcggtt atgatgatga tgctgataat gct 33
<210> 135
   <211> 15
   <212> DNA
   <213> Oryctolagus cuniculus
<400> 135
   agtcgtacaa tgtcc 15
<210> 136
   <211> 48
   <212> DNA
   <213> Oryctolagus cuniculus
<400> 136
   tacatttgga gtggtggtag cacatactac gcgacctggg cgaaaggc 48
<210> 137
   <211> 45
   <212> DNA
   <213> Oryctolagus cuniculus
<400> 137
   ttgggcgata ctggtggtca cgcttatgct actcgcttaa atctc 45
<210> 138
   <211> 123
   <212> PRT
   <213> Oryctolagus cuniculus
<400> 138
   Met Asp Thr Arg Ala Pro Thr Gln Leu Leu Gly Leu Leu Leu Leu Trp
   1               5                   10                  15
   Leu Pro Gly Ala Thr Phe Ala Ala Val Leu Thr Gln Thr Pro Ser Ser
               20                  25                  30
   Val Ser Ala Ala Val Gly Gly Thr Val Ser Ile Ser Cys Gln Ser Ser
           35                  40                  45
   Gln Ser Val Tyr Ser Asn Lys Tyr Leu Ala Trp Tyr Gln Gln Lys Pro
       50                  55                  60
   Gly Gln Pro Pro Lys Leu Leu Ile Tyr Trp Thr Ser Lys Leu Ala Ser
   65                  70                  75                  80
   Gly Ala Pro Ser Arg Phe Ser Gly Ser Gly Ser Gly Thr Gln Phe Thr
                   85                  90                  95
   Leu Thr Ile Ser Gly Val Gln Cys Asp Asp Ala Ala Thr Tyr Tyr Cys
               100                 105                 110
   Leu Gly Ala Tyr Asp Asp Asp Ala Asp Asn Ala
           115                 120
<210> 139
   <211> 126
   <212> PRT
   <213> Oryctolagus cuniculus
<400> 139
   Met Glu Thr Gly Leu Arg Trp Leu Leu Leu Val Ala Val Leu Lys Gly
   1 5 10 15
   Val Gln Cys Gln Ser Val Glu Glu Ser Gly Gly Arg Leu Val Lys Pro
   20 25 30
   Asp Glu Thr Leu Thr Leu Thr Cys Thr Ala Ser Gly Phe Ser Leu Glu
   35 40 45
   Gly Gly Tyr Met Thr Trp Val Arg Gln Ala Pro Gly Lys Gly Leu Glu
   50 55 60
   Trp Ile Gly Ile Ser Tyr Asp Ser Gly Ser Thr Tyr Tyr Ala Ser Trp
   65 70 75 80
   Ala Lys Gly Arg Phe Thr Ile Ser Lys Thr Ser Ser Thr Thr Val Asp
   85 90 95
   Leu Lys Met Thr Ser Leu Thr Thr Glu Asp Thr Ala Thr Tyr Phe Cys
   100 105 110
   Val Arg Ser Leu Lys Tyr Pro Thr Val Thr Ser Asp Asp Leu
   115 120 125
<210> 140
   <211> 13
   <212> PRT
   <213> Oryctolagus cuniculus
<400> 140
   Gln Ser Ser Gln Ser Val Tyr Ser Asn Lys Tyr Leu Ala
   1 5 10
<210> 141
   <211> 7
   <212> PRT
   <213> Oryctolagus cuniculus
<400> 141
   Trp Thr Ser Lys Leu Ala Ser
   1 5
<210> 142
   <211> 11
   <212> PRT
   <213> Oryctolagus cuniculus
<400> 142
   Leu Gly Ala Tyr Asp Asp Asp Ala Asp Asn Ala
   1 5 10
<210> 143
   <211> 5
   <212> PRT
   <213> Oryctolagus cuniculus
<400> 143
   Gly Gly Tyr Met Thr
   1 5
<210> 144
   <211> 16
   <212> PRT
   <213> Oryctolagus cuniculus
<400> 144
   Ile Ser Tyr Asp Ser Gly Ser Thr Tyr Tyr Ala Ser Trp Ala Lys Gly
   1 5 10 15
<210> 145
   <211> 12
   <212> PRT
   <213> Oryctolagus cuniculus
<400> 145
   Ser Leu Lys Tyr Pro Thr Val Thr Ser Asp Asp Leu
   1 5 10
<210> 146
   <211> 369
   <212> DNA
   <213> Oryctolagus cuniculus
<400> 146
   atggacacga gggcccccac tcagctgctg gggctcctgc tgctctggct cccaggtgcc 60
   acatttgcag ccgtgctgac ccagacacca tcgtccgtgt ctgcagctgt gggaggcaca 120
   gtcagcatca gttgccagtc cagtcagagt gtttatagta ataagtacct agcctggtat 180
   cagcagaaac cagggcagcc tcccaagctc ctgatctact ggacatccaa actggcatct 240
   ggggccccat cacggttcag cggcagtgga tctgggacac aattcactct caccatcagc 300
   ggcgtgcagt gtgacgatgc tgccacttac tactgtctag gcgcttatga tgatgatgct 360
   gataatgct 369
<210> 147
   <211> 378
   <212> DNA
   <213> Oryctolagus cuniculus
<400> 147
   atggagactg ggctgcgctg gcttctcctg gtcgctgtgc tcaaaggtgt ccagtgtcag 60
   tcggtggaag agtccggggg tcgcctggtc aagcctgacg aaaccctgac actcacctgc 120
   acagcctctg gattctccct ggagggcggc tacatgacct gggtccgcca ggctccaggg 180
   aaggggctgg aatggatcgg aatcagttat gatagtggta gcacatacta cgcgagctgg 240
   gcgaaaggcc gattcaccat ctccaagacc tcgtcgacca cggtggatct gaaaatgacc 300
   agtctgacaa ccgaggacac ggccacctat ttctgcgtca gatcactaaa atatcctact 360
   gttacttctg atgacttg 378
<210> 148
   <211> 39
   <212> DNA
   <213> Oryctolagus cuniculus
<400> 148
   cagtccagtc agagtgttta tagtaataag tacctagcc 39
<210> 149
   <211> 21
   <212> DNA
   <213> Oryctolagus cuniculus
<400> 149
   tggacatcca aactggcatc t 21
<210> 150
   <211> 33
   <212> DNA
   <213> Oryctolagus cuniculus
<400> 150
   ctaggcgctt atgatgatga tgctgataat gct 33
<210> 151
   <211> 15
   <212> DNA
   <213> Oryctolagus cuniculus
<400> 151
   ggcggctaca tgacc 15
<210> 152
   <211> 48
   <212> DNA
   <213> Oryctolagus cuniculus
<400> 152
   atcagttatg atagtggtag cacatactac gcgagctggg cgaaaggc 48
<210> 153
   <211> 36
   <212> DNA
   <213> Oryctolagus cuniculus
<400> 153
   tcactaaaat atcctactgt tacttctgat gacttg 36
<210> 154
   <211> 123
   <212> PRT
   <213> Oryctolagus cuniculus
<400> 154
   Met Asp Thr Arg Ala Pro Thr Gln Leu Leu Gly Leu Leu Leu Leu Trp
   1               5                   10                  15
   Leu Pro Gly Ala Thr Phe Ala Ala Val Leu Thr Gln Thr Pro Ser Pro
               20                  25                  30
   Val Ser Ala Ala Val Gly Gly Thr Val Thr Ile Ser Cys Gln Ser Ser
           35                  40                  45
   Gln Ser Val Tyr Asn Asn Asn Asp Leu Ala Trp Tyr Gln Gln Lys Pro
       50                  55                  60
   Gly Gln Pro Pro Lys Leu Leu Ile Tyr Tyr Ala Ser Thr Leu Ala Ser
   65                  70                  75                  80
   Gly Val Pro Ser Arg Phe Lys Gly Ser Gly Ser Gly Thr Gln Phe Thr
                   85                  90                  95
   Leu Thr Ile Ser Gly Val Gln Cys Asp Asp Ala Ala Ala Tyr Tyr Cys
               100                 105                 110
   Leu Gly Gly Tyr Asp Asp Asp Ala Asp Asn Ala
           115                 120
<210> 155
   <211> 129
   <212> PRT
   <213> Oryctolagus cuniculus
<400> 155
   Met Glu Thr Gly Leu Arg Trp Leu Leu Leu Val Ala Val Leu Lys Gly
   1               5                   10                  15
   Val Gln Cys Gln Ser Val Glu Glu Ser Gly Gly Arg Leu Val Thr Pro
   20 25 30
   Gly Thr Pro Leu Thr Leu Thr Cys Thr Val Ser Gly Leu Ser Leu Ser
   35 40 45
   Ser Asn Thr Ile Asn Trp Val Arg Gln Ala Pro Gly Lys Gly Leu Glu
   50 55 60
   Trp Ile Gly Tyr Ile Trp Ser Gly Gly Ser Thr Tyr Tyr Ala Ser Trp
   65 70 75 80
   Val Asn Gly Arg Phe Thr Ile Ser Lys Thr Ser Thr Thr Val Asp Leu
   85 90 95
   Lys Ile Thr Ser Pro Thr Thr Glu Asp Thr Ala Thr Tyr Phe Cys Ala
   100 105 110
   Arg Gly Gly Tyr Ala Ser Gly Gly Tyr Pro Tyr Ala Thr Arg Leu Asp
   115 120 125
   Leu
<210> 156
   <211> 13
   <212> PRT
   <213> Oryctolagus cuniculus
<400> 156
   Gln Ser Ser Gln Ser Val Tyr Asn Asn Asn Asp Leu Ala
   1 5 10
<210> 157
   <211> 7
   <212> PRT
   <213> Oryctolagus cuniculus
<400> 157
   Tyr Ala Ser Thr Leu Ala Ser
   1 5
<210> 158
   <211> 11
   <212> PRT
   <213> Oryctolagus cuniculus
<400> 158
   Leu Gly Gly Tyr Asp Asp Asp Ala Asp Asn Ala
   1 5 10
<210> 159
   <211> 5
   <212> PRT
   <213> Oryctolagus cuniculus
<400> 159
   Ser Asn Thr Ile Asn
   1 5
<210> 160
   <211> 16
   <212> PRT
   <213> Oryctolagus cuniculus
<400> 160
   Tyr Ile Trp Ser Gly Gly Ser Thr Tyr Tyr Ala Ser Trp Val Asn Gly
   1 5 10 15
<210> 161
   <211> 16
   <212> PRT
   <213> Oryctolagus cuniculus
<400> 161
   Gly Gly Tyr Ala Ser Gly Gly Tyr Pro Tyr Ala Thr Arg Leu Asp Leu
   1 5 10 15
<210> 162
   <211> 369
   <212> DNA
   <213> Oryctolagus cuniculus
<400> 162
   atggacacga gggcccccac tcagctgctg gggctcctgc tgctctggct cccaggtgcc 60
   acatttgcag ccgtgctgac ccagacacca tcacccgtgt ctgcagctgt gggaggcaca 120
   gtcaccatca gttgccagtc cagtcagagt gtttataata ataacgactt agcctggtat 180
   cagcagaaac cagggcagcc tcctaaactc ctgatctatt atgcatccac tctggcatct 240
   ggggtcccat cgcggttcaa aggcagtgga tctgggacac agttcactct caccatcagc 300
   ggcgtgcagt gtgacgatgc tgccgcttac tactgtctag gcggttatga tgatgatgct 360
   gataatgct 369
<210> 163
   <211> 387
   <212> DNA
   <213> Oryctolagus cuniculus
<400> 163
   atggagactg ggctgcgctg gcttctcctg gtcgctgtgc tcaaaggtgt ccagtgtcag     60
   tcggtggagg agtccggggg tcgcctggtc acgcctggga cacccctgac actcacctgc    120
   acagtatctg gattatccct cagtagcaat acaataaact gggtccgcca ggctccaggg    180
   aaggggctgg agtggatcgg atacatttgg agtggtggta gtacatacta cgcgagctgg    240
   gtgaatggtc gattcaccat ctccaaaacc tcgaccacgg tggatctgaa aatcaccagt    300
   ccgacaaccg aggacacggc cacctatttc tgtgccagag ggggttacgc tagtggtggt     360
   tatccttatg ccactcggtt ggatctc                                         387
<210> 164
   <211> 39
   <212> DNA
   <213> Oryctolagus cuniculus
<400> 164
   cagtccagtc agagtgttta taataataac gacttagcc 39
<210> 165
   <211> 21
   <212> DNA
   <213> Oryctolagus cuniculus
<400> 165
   tatgcatcca ctctggcatc t 21
<210> 166
   <211> 33
   <212> DNA
   <213> Oryctolagus cuniculus
<400> 166
   ctaggcggtt atgatgatga tgctgataat gct 33
<210> 167
   <211> 15
   <212> DNA
   <213> Oryctolagus cuniculus
<400> 167
   agcaatacaa taaac 15
<210> 168
   <211> 48
   <212> DNA
   <213> Oryctolagus cuniculus
<400> 168
   tacatttgga gtggtggtag tacatactac gcgagctggg tgaatggt 48
<210> 169
   <211> 48
   <212> DNA
   <213> Oryctolagus cuniculus
<400> 169
   gggggttacg ctagtggtgg ttatccttat gccactcggt tggatctc 48
<210> 170
   <211> 123
   <212> PRT
   <213> Oryctolagus cuniculus
<400> 170
   Met Asp Thr Arg Ala Pro Thr Gln Leu Leu Gly Leu Leu Leu Leu Trp
   1               5                   10                  15
   Leu Pro Gly Ala Thr Phe Ala Ala Val Leu Thr Gln Thr Pro Ser Ser
               20                  25                  30
   Val Ser Ala Ala Val Gly Gly Thr Val Thr Ile Asn Cys Gln Ser Ser
           35                  40                  45
   Gln Ser Val Tyr Asn Asn Asp Tyr Leu Ser Trp Tyr Gln Gln Arg Pro
       50                  55                  60
   Gly Gln Arg Pro Lys Leu Leu Ile Tyr Gly Ala Ser Lys Leu Ala Ser
   65                  70                  75                  80
   Gly Val Pro Ser Arg Phe Lys Gly Ser Gly Ser Gly Lys Gln Phe Thr
                   85                  90                  95
   Leu Thr Ile Ser Gly Val Gln Cys Asp Asp Ala Ala Thr Tyr Tyr Cys
               100                 105                 110
   Leu Gly Asp Tyr Asp Asp Asp Ala Asp Asn Thr
           115                 120
<210> 171
   <211> 123
   <212> PRT
   <213> Oryctolagus cuniculus
<400> 171
   Met Glu Thr Gly Leu Arg Trp Leu Leu Leu Val Ala Val Leu Lys Gly
   1               5                   10                  15
   Val Gln Cys Gln Ser Leu Glu Glu Ser Gly Gly Arg Leu Val Thr Pro
               20                  25                  30
   Gly Thr Pro Leu Thr Leu Thr Cys Thr Val Ser Gly Phe Thr Leu Ser
           35                  40                  45
   Thr Asn Tyr Tyr Leu Ser Trp Val Arg Gln Ala Pro Gly Lys Gly Leu
       50                  55                  60
   Glu Trp Ile Gly Ile Ile Tyr Pro Ser Gly Asn Thr Tyr Cys Ala Lys
   65                  70                  75                  80
   Trp Ala Lys Gly Arg Phe Thr Ile Ser Lys Thr Ser Ser Thr Thr Val
                   85                  90                  95
   Asp Leu Lys Met Thr Ser Pro Thr Thr Glu Asp Thr Ala Thr Tyr Phe
               100                 105                 110
   Cys Ala Arg Asn Tyr Gly Gly Asp Glu Ser Leu
           115                 120
<210> 172
   <211> 13
   <212> PRT
   <213> Oryctolagus cuniculus
<400> 172
   Gln Ser Ser Gln Ser Val Tyr Asn Asn Asp Tyr Leu Ser
   1               5                   10
<210> 173
   <211> 7
   <212> PRT
   <213> Oryctolagus cuniculus
<400> 173
   Gly Ala Ser Lys Leu Ala Ser
   1               5
<210> 174
   <211> 11
   <212> PRT
   <213> Oryctolagus cuniculus
<400> 174
   Leu Gly Asp Tyr Asp Asp Asp Ala Asp Asn Thr
   1               5                   10
<210> 175
   <211> 6
   <212> PRT
   <213> Oryctolagus cuniculus
<400> 175
   Thr Asn Tyr Tyr Leu Ser
   1               5
<210> 176
   <211> 16
   <212> PRT
   <213> Oryctolagus cuniculus
<400> 176
   Ile Ile Tyr Pro Ser Gly Asn Thr Tyr Cys Ala Lys Trp Ala Lys Gly
   1               5                   10                  15
<210> 177
   <211> 8
   <212> PRT
   <213> Oryctolagus cuniculus
<400> 177
   Asn Tyr Gly Gly Asp Glu Ser Leu
   1               5
<210> 178
   <211> 369
   <212> DNA
   <213> Oryctolagus cuniculus
<400> 178
   atggacacga gggcccccac tcagctgctg gggctcctgc tgctctggct cccaggtgcc      60
   acatttgcag ccgtgctgac ccagacacca tcctccgtgt ctgcagctgt gggaggcaca     120
   gtcaccatca attgccagtc cagtcagagt gtttataata acgactactt atcctggtat     180
   caacagaggc cagggcaacg tcccaagctc ctaatctatg gtgcttccaa actggcatct     240
   ggggtcccgt cacggttcaa aggcagtgga tctgggaaac agtttactct caccatcagc     300
   ggcgtgcagt gtgacgatgc tgccacttac tactgtctgg gcgattatga tgatgatgct     360
   gataatact                                                             369
<210> 179
   <211> 369
   <212> DNA
   <213> Oryctolagus cuniculus
<400> 179 gaaagtttg 369
<210> 180
   <211> 39
   <212> DNA
   <213> Oryctolagus cuniculus
<400> 180
   cagtccagtc agagtgttta taataacgac tacttatcc 39
<210> 181
   <211> 21
   <212> DNA
   <213> Oryctolagus cuniculus
<400> 181
   ggtgcttcca aactggcatc t 21
<210> 182
   <211> 33
   <212> DNA
   <213> Oryctolagus cuniculus
<400> 182
   ctgggcgatt atgatgatga tgctgataat act 33
<210> 183
   <211> 18
   <212> DNA
   <213> Oryctolagus cuniculus
<400> 183
   accaactact acctgagc 18
<210> 184
   <211> 48
   <212> DNA
   <213> Oryctolagus cuniculus
<400> 184
   atcatttatc ctagtggtaa cacatattgc gcgaagtggg cgaaaggc 48
<210> 185
   <211> 24
   <212> DNA
   <213> Oryctolagus cuniculus
<400> 185
   aattatggtg gtgatgaaag tttg 24
<210> 186
   <211> 119
   <212> PRT
   <213> Oryctolagus cuniculus
<400> 186
   Met Asp Thr Arg Ala Pro Thr Gln Leu Leu Gly Leu Leu Leu Leu Trp
   1               5                   10                  15
   Leu Pro Gly Ala Arg Cys Asp Val Val Met Thr Gln Thr Pro Ala Ser
               20                  25                  30
   Val Glu Ala Ala Val Gly Gly Thr Val Thr Ile Lys Cys Gln Ala Ser
           35                  40                  45
   Glu Thr Ile Gly Asn Ala Leu Ala Trp Tyr Gln Gln Lys Ser Gly Gln
       50                  55                  60
   Pro Pro Lys Leu Leu Ile Tyr Lys Ala Ser Lys Leu Ala Ser Gly Val
   65                  70                  75                  80
   Pro Ser Arg Phe Lys Gly Ser Gly Ser Gly Thr Glu Tyr Thr Leu Thr
                   85                  90                  95
   Ile Ser Asp Leu Glu Cys Ala Asp Ala Ala Thr Tyr Tyr Cys Gln Trp
               100                 105                 110
   Cys Tyr Phe Gly Asp Ser Val
           115
<210> 187
   <211> 128
   <212> PRT
   <213> Oryctolagus cuniculus
<400> 187
   Met Glu Thr Gly Leu Arg Trp Leu Leu Leu Val Thr Val Leu Lys Gly
   1               5                   10                  15
   Val Gln Cys Gln Glu Gln Leu Val Glu Ser Gly Gly Gly Leu Val Gln
               20                  25                  30
   Pro Glu Gly Ser Leu Thr Leu Thr Cys Thr Ala Ser Gly Phe Asp Phe
           35                  40                  45
   Ser Ser Gly Tyr Tyr Met Cys Trp Val Arg Gln Ala Pro Gly Lys Gly
       50                  55                  60
   Leu Glu Trp Ile Ala Cys Ile Phe Thr Ile Thr Thr Asn Thr Tyr Tyr
   65                  70                  75                  80
   Ala Ser Trp Ala Lys Gly Arg Phe Thr Ile Ser Lys Thr Ser Ser Thr
                   85                  90                  95
   Thr Val Thr Leu Gln Met Thr Ser Leu Thr Ala Ala Asp Thr Ala Thr
               100                 105                 110
   Tyr Leu Cys Ala Arg Gly Ile Tyr Ser Asp Asn Asn Tyr Tyr Ala Leu
           115                 120                 125
<210> 188
   <211> 11
   <212> PRT
   <213> Oryctolagus cuniculus
<400> 188
   Gln Ala Ser Glu Thr Ile Gly Asn Ala Leu Ala
   1               5                   10
<210> 189
   <211> 7
   <212> PRT
   <213> Oryctolagus cuniculus
<400> 189
   Lys Ala Ser Lys Leu Ala Ser
   1               5
<210> 190
   <211> 9
   <212> PRT
   <213> Oryctolagus cuniculus
<400> 190
   Gln Trp Cys Tyr Phe Gly Asp Ser Val
   1               5
<210> 191
   <211> 6
   <212> PRT
   <213> Oryctolagus cuniculus
<400> 191
   Ser Gly Tyr Tyr Met Cys
   1 5
<210> 192
   <211> 17
   <212> PRT
   <213> Oryctolagus cuniculus
<400> 192
   Cys Ile Phe Thr Ile Thr Thr Asn Thr Tyr Tyr Ala Ser Trp Ala Lys
   1 5 10 15
   Gly
<210> 193
   <211> 11
   <212> PRT
   <213> Oryctolagus cuniculus
<400> 193
   Gly Ile Tyr Ser Asp Asn Asn Tyr Tyr Ala Leu
   1 5 10
<210> 194
   <211> 357
   <212> DNA
   <213> Oryctolagus cuniculus
<400> 194
   atggacacga gggcccccac tcagctgctg gggctcctgc tgctctggct cccaggtgcc 60
   agatgtgatg ttgtgatgac ccagactcca gcctccgtgg aggcagctgt gggaggcaca 120
   gtcaccatca agtgccaggc cagtgagacc attggcaatg cattagcctg gtatcagcag 180
   aaatcagggc agcctcccaa gctcctgatc tacaaggcat ccaaactggc atctggggtc 240
   ccatcgcggt tcaaaggcag tggatctggg acagagtaca ctctcaccat cagcgacctg 300
   gagtgtgccg atgctgccac ttactactgt caatggtgtt attttggtga tagtgtt 357
<210> 195
   <211> 384
   <212> DNA
   <213> Oryctolagus cuniculus
<400> 195
   atggagactg ggctgcgctg gcttctcctg gtcactgtgc tcaaaggtgt ccagtgtcag      60
   gagcagctgg tggagtccgg gggaggcctg gtccagcctg agggatccct gacactcacc     120
   tgcacagcct ctggattcga cttcagtagc ggctactaca tgtgctgggt ccgccaggct     180
   ccagggaagg ggctggagtg gatcgcgtgt attttcacta ttactactaa cacttactac     240
   gcgagctggg cgaaaggccg attcaccatc tccaagacct cgtcgaccac ggtgactctg     300
   caaatgacca gtctgacagc cgcggacacg gccacctatc tctgtgcgag agggatttat     360
   tctgataata attattatgc cttg                                            384
<210> 196
   <211> 33
   <212> DNA
   <213> Oryctolagus cuniculus
<400> 196
   caggccagtg agaccattgg caatgcatta gcc 33
<210> 197
   <211> 21
   <212> DNA
   <213> Oryctolagus cuniculus
<400> 197
   aaggcatcca aactggcatc t 21
<210> 198
   <211> 27
   <212> DNA
   <213> Oryctolagus cuniculus
<400> 198
   caatggtgtt attttggtga tagtgtt 27
<210> 199
   <211> 18
   <212> DNA
   <213> Oryctolagus cuniculus
<400> 199
   agcggctact acatgtgc 18
<210> 200
   <211> 51
   <212> DNA
   <213> Oryctolagus cuniculus
<400> 200
   tgtattttca ctattactac taacacttac tacgcgagct gggcgaaagg c 51
<210> 201
   <211> 33
   <212> DNA
   <213> Oryctolagus cuniculus
<400> 201
   gggatttatt ctgataataa ttattatgcc ttg 33
<210> 202
   <211> 119
   <212> PRT
   <213> Oryctolagus cuniculus
<400> 202
   Met Asp Thr Arg Ala Pro Thr Gln Leu Leu Gly Leu Leu Leu Leu Trp
   1               5                   10                  15
   Leu Pro Gly Ala Arg Cys Asp Val Val Met Thr Gln Thr Pro Ala Ser
               20                  25                  30
   Val Glu Ala Ala Val Gly Gly Thr Val Thr Ile Lys Cys Gln Ala Ser
           35                  40                  45
   Glu Ser Ile Gly Asn Ala Leu Ala Trp Tyr Gln Gln Lys Pro Gly Gln
       50                  55                  60
   Pro Pro Lys Leu Leu Ile Tyr Lys Ala Ser Thr Leu Ala Ser Gly Val
   65                  70                  75                  80
   Pro Ser Arg Phe Ser Gly Ser Gly Ser Gly Thr Glu Phe Thr Leu Thr
                   85                  90                  95
   Ile Ser Gly Val Gln Cys Ala Asp Ala Ala Ala Tyr Tyr Cys Gln Trp
               100                 105                 110
   Cys Tyr Phe Gly Asp Ser Val
           115
<210> 203
   <211> 128
   <212> PRT
   <213> Oryctolagus cuniculus
<400> 203
   Met Glu Thr Gly Leu Arg Trp Leu Leu Leu Val Ala Val Leu Lys Gly
   1               5                   10                  15
   Val Gln Cys Gln Gln Gln Leu Val Glu Ser Gly Gly Gly Leu Val Lys
               20                  25                  30
   Pro Gly Ala Ser Leu Thr Leu Thr Cys Lys Ala Ser Gly Phe Ser Phe
           35                  40                  45
   Ser Ser Gly Tyr Tyr Met Cys Trp Val Arg Gln Ala Pro Gly Lys Gly
       50                  55                  60
   Leu Glu Ser Ile Ala Cys Ile Phe Thr Ile Thr Asp Asn Thr Tyr Tyr
   65                  70                  75                  80
   Ala Asn Trp Ala Lys Gly Arg Phe Thr Ile Ser Lys Pro Ser Ser Pro
                   85                  90                  95
   Thr Val Thr Leu Gln Met Thr Ser Leu Thr Ala Ala Asp Thr Ala Thr
               100                 105                 110
   Tyr Phe Cys Ala Arg Gly Ile Tyr Ser Thr Asp Asn Tyr Tyr Ala Leu
           115                 120                 125
<210> 204
   <211> 11
   <212> PRT
   <213> Oryctolagus cuniculus
<400> 204
   Gln Ala Ser Glu Ser Ile Gly Asn Ala Leu Ala
   1               5                   10
<210> 205
   <211> 7
   <212> PRT
   <213> Oryctolagus cuniculus
<400> 205
   Lys Ala Ser Thr Leu Ala Ser
   1               5
<210> 206
   <211> 9
   <212> PRT
   <213> Oryctolagus cuniculus
<400> 206
   Gln Trp Cys Tyr Phe Gly Asp Ser Val
   1               5
<210> 207
   <211> 6
   <212> PRT
   <213> Oryctolagus cuniculus
<400> 207
   Ser Gly Tyr Tyr Met Cys
   1               5
<210> 208
   <211> 17
   <212> PRT
   <213> Oryctolagus cuniculus
<400> 208
   Cys Ile Phe Thr Ile Thr Asp Asn Thr Tyr Tyr Ala Asn Trp Ala Lys
   1               5                   10                  15
   Gly
<210> 209
   <211> 11
   <212> PRT
   <213> Oryctolagus cuniculus
<400> 209
   Gly Ile Tyr Ser Thr Asp Asn Tyr Tyr Ala Leu
   1               5                   10
<210> 210
   <211> 357
   <212> DNA
   <213> Oryctolagus cuniculus
<400> 210
   atggacacga gggcccccac tcagctgctg gggctcctgc tgctctggct cccaggtgcc 60
   agatgtgatg ttgtgatgac ccagactcca gcctccgtgg aggcagctgt gggaggcaca 120
   gtcaccatca agtgccaggc cagtgagagc attggcaatg cattagcctg gtatcagcag 180
   aaaccagggc agcctcccaa gctcctgatc tacaaggcat ccactctggc atctggggtc 240
   ccatcgcggt tcagcggcag tggatctggg acagagttca ctctcaccat cagcggcgtg 300
   cagtgtgccg atgctgccgc ttactactgt caatggtgtt attttggtga tagtgtt 357
<210> 211
   <211> 384
   <212> DNA
   <213> Oryctolagus cuniculus
<400> 211
   atggagactg ggctgcgctg gcttctcctg gtcgctgtgc tcaaaggtgt ccagtgtcag        60
   cagcagctgg tggagtccgg gggaggcctg gtcaagccgg gggcatccct gacactcacc       120
   tgcaaagcct ctggattctc cttcagtagc ggctactaca tgtgctgggt ccgccaggct       180
   ccagggaagg ggctggagtc gatcgcatgc atttttacta ttactgataa cacttactac       240
   gcgaactggg cgaaaggccg attcaccatc tccaagccct cgtcgcccac ggtgactctg       300
   caaatgacca gtctgacagc cgcggacacg gccacctatt tctgtgcgag ggggatttat       360
   tctactgata attattatgc cttg                                              384
<210> 212
   <211> 33
   <212> DNA
   <213> Oryctolagus cuniculus
<400> 212
   caggccagtg agagcattgg caatgcatta gcc 33
<210> 213
   <211> 21
   <212> DNA
   <213> Oryctolagus cuniculus
<400> 213
   aaggcatcca ctctggcatc t 21
<210> 214
   <211> 27
   <212> DNA
   <213> Oryctolagus cuniculus
<400> 214
   caatggtgtt attttggtga tagtgtt 27
<210> 215
   <211> 18
   <212> DNA
   <213> Oryctolagus cuniculus
<400> 215
   agcggctact acatgtgc 18
<210> 216
   <211> 51
   <212> DNA
   <213> Oryctolagus cuniculus
<400> 216
   tgcattttta ctattactga taacacttac tacgcgaact gggcgaaagg c 51
<210> 217
   <211> 33
   <212> DNA
   <213> Oryctolagus cuniculus
<400> 217
   gggatttatt ctactgataa ttattatgcc ttg 33
<210> 218
   <211> 123
   <212> PRT
   <213> Oryctolagus cuniculus
<400> 218
   Met Asp Thr Arg Ala Pro Thr Gln Leu Leu Gly Leu Leu Leu Leu Trp
   1               5                   10                  15
   Leu Pro Gly Ala Arg Cys Asp Val Val Met Thr Gln Thr Pro Ala Ser
               20                  25                  30
   Val Glu Ala Ala Val Gly Gly Thr Val Thr Ile Lys Cys Gln Ala Ser
           35                  40                  45
   Gln Ser Val Ser Ser Tyr Leu Asn Trp Tyr Gln Gln Lys Pro Gly Gln
       50                  55                  60
   Pro Pro Lys Leu Leu Ile Tyr Arg Ala Ser Thr Leu Glu Ser Gly Val
   65                  70                  75                  80
   Pro Ser Arg Phe Lys Gly Ser Gly Ser Gly Thr Glu Phe Thr Leu Thr
                   85                  90                  95
   Ile Ser Asp Leu Glu Cys Ala Asp Ala Ala Thr Tyr Tyr Cys Gln Cys
               100                 105                 110
   Thr Tyr Gly Thr Ser Ser Ser Tyr Gly Ala Ala
           115                 120
<210> 219
   <211> 133
   <212> PRT
   <213> Oryctolagus cuniculus
<400> 219
   Met Glu Thr Gly Leu Arg Trp Leu Leu Leu Val Ala Val Leu Lys Gly
   1               5                   10                  15
   Val Gln Cys Gln Ser Val Glu Glu Ser Gly Gly Arg Leu Val Thr Pro
               20                  25                  30
   Gly Thr Pro Leu Thr Leu Thr Cys Thr Val Ser Gly Ile Ser Leu Ser
           35                  40                  45
   Ser Asn Ala Ile Ser Trp Val Arg Gln Ala Pro Gly Lys Gly Leu Glu
       50                  55                  60
   Trp Ile Gly Ile Ile Ser Tyr Ser Gly Thr Thr Tyr Tyr Ala Ser Trp
   65                  70                  75                  80
   Ala Lys Gly Arg Phe Thr Ile Ser Lys Thr Ser Ser Thr Thr Val Asp
                   85                  90                  95
   Leu Lys Ile Thr Ser Pro Thr Thr Glu Asp Thr Ala Thr Tyr Phe Cys
               100                 105                 110
   Ala Arg Asp Asp Pro Thr Thr Val Met Val Met Leu Ile Pro Phe Gly
           115                 120                 125
   Ala Gly Met Asp Leu
       130
<210> 220
   <211> 11
   <212> PRT
   <213> Oryctolagus cuniculus
<400> 220
   Gln Ala Ser Gln Ser Val Ser Ser Tyr Leu Asn
   1               5                   10
<210> 221
   <211> 7
   <212> PRT
   <213> Oryctolagus cuniculus
<400> 221
   Arg Ala Ser Thr Leu Glu Ser
   1               5
<210> 222
   <211> 13
   <212> PRT
   <213> Oryctolagus cuniculus
<400> 222
   Gln Cys Thr Tyr Gly Thr Ser Ser Ser Tyr Gly Ala Ala
   1               5                   10
<210> 223
   <211> 5
   <212> PRT
   <213> Oryctolagus cuniculus
<400> 223
   Ser Asn Ala Ile Ser
   1               5
<210> 224
   <211> 16
   <212> PRT
   <213> Oryctolagus cuniculus
<400> 224
   Ile Ile Ser Tyr Ser Gly Thr Thr Tyr Tyr Ala Ser Trp Ala Lys Gly
   1               5                   10                  15
<210> 225
   <211> 19
   <212> PRT
   <213> Oryctolagus cuniculus
<400> 225 Met Asp Leu
<210> 226
   <211> 369
   <212> DNA
   <213> Oryctolagus cuniculus
<400> 226
   atggacacga gggcccccac tcagctgctg gggctcctgc tgctctggct cccaggtgcc 60
   agatgtgatg ttgtgatgac ccagactcca gcctccgtgg aggcagctgt gggaggcaca 120
   gtcaccatca agtgccaggc cagtcagagc gttagtagct acttaaactg gtatcagcag 180
   aaaccagggc agcctcccaa gctcctgatc tacagggcat ccactctgga atctggggtc 240
   ccatcgcggt tcaaaggcag tggatctggg acagagttca ctctcaccat cagcgacctg 300
   gagtgtgccg atgctgccac ttactactgt caatgtactt atggtactag tagtagttat 360
   ggtgctgct 369
<210> 227
   <211> 399
   <212> DNA
   <213> Oryctolagus cuniculus
<400> 227
   atggagactg ggctgcgctg gcttctcctg gtcgctgtgc tcaaaggtgt ccagtgtcag 60
   tcggtggagg agtccggggg tcgcctggtc acgcctggga cacccctgac actcacctgc 120
   accgtctctg gtatctccct cagtagcaat gcaataagct gggtccgcca ggctccaggg 180
   aaggggctgg aatggatcgg aatcattagt tatagtggta ccacatacta cgcgagctgg 240
   gcgaaaggcc gattcaccat ctccaaaacc tcgtcgacca cggtggatct gaaaatcact 300
   agtccgacaa ccgaggacac ggccacctac ttctgtgcca gagatgaccc tacgacagtt 360
   atggttatgt tgataccttt tggagccggc atggacctc 399
<210> 228
   <211> 33
   <212> DNA
   <213> Oryctolagus cuniculus
<400> 228
   caggccagtc agagcgttag tagctactta aac 33
<210> 229
   <211> 21
   <212> DNA
   <213> Oryctolagus cuniculus
<400> 229
   agggcatcca ctctggaatc t 21
<210> 230
   <211> 39
   <212> DNA
   <213> Oryctolagus cuniculus
<400> 230
   caatgtactt atggtactag tagtagttat ggtgctgct 39
<210> 231
   <211> 15
   <212> DNA
   <213> Oryctolagus cuniculus
<400> 231
   agcaatgcaa taagc 15
<210> 232
   <211> 48
   <212> DNA
   <213> Oryctolagus cuniculus
<400> 232
   atcattagtt atagtggtac cacatactac gcgagctggg cgaaaggc 48
<210> 233
   <211> 57
   <212> DNA
   <213> Oryctolagus cuniculus
<400> 233
   gatgacccta cgacagttat ggttatgttg ataccttttg gagccggcat ggacctc 57
<210> 234
   <211> 125
   <212> PRT
   <213> Oryctolagus cuniculus
<400> 234
   Met Asp Thr Arg Ala Pro Thr Gln Leu Leu Gly Leu Leu Leu Leu Trp
   1               5                   10                  15
   Leu Pro Gly Ala Thr Phe Ala Gln Val Leu Thr Gln Thr Ala Ser Pro
               20                  25                  30
   Val Ser Ala Ala Val Gly Gly Thr Val Thr Ile Asn Cys Gln Ala Ser
           35                  40                  45
   Gln Ser Val Tyr Lys Asn Asn Tyr Leu Ser Trp Tyr Gln Gln Lys Pro
       50                  55                  60
   Gly Gln Pro Pro Lys Gly Leu Ile Tyr Ser Ala Ser Thr Leu Asp Ser
   65                  70                  75                  80
   Gly Val Pro Leu Arg Phe Ser Gly Ser Gly Ser Gly Thr Gln Phe Thr
                   85                  90                  95
   Leu Thr Ile Ser Asp Val Gln Cys Asp Asp Ala Ala Thr Tyr Tyr Cys
               100                 105                 110
   Leu Gly Ser Tyr Asp Cys Ser Ser Gly Asp Cys Tyr Ala
           115                 120                 125
<210> 235
   <211> 119
   <212> PRT
   <213> Oryctolagus cuniculus
<400> 235
   Met Glu Thr Gly Leu Arg Trp Leu Leu Leu Val Ala Val Leu Lys Gly
   1               5                   10                  15
   Val Gln Cys Gln Ser Leu Glu Glu Ser Gly Gly Asp Leu Val Lys Pro
               20                  25                  30
   Glu Gly Ser Leu Thr Leu Thr Cys Thr Ala Ser Gly Phe Ser Phe Ser
           35                  40                  45
   Ser Tyr Trp Met Cys Trp Val Arg Gln Ala Pro Gly Lys Gly Leu Glu
       50                  55                  60
   Trp Ile Ala Cys Ile Val Thr Gly Asn Gly Asn Thr Tyr Tyr Ala Asn
   65                  70                  75                  80
   Trp Ala Lys Gly Arg Phe Thr Ile Ser Lys Thr Ser Ser Thr Thr Val
                   85                  90                  95
   Thr Leu Gln Met Thr Ser Leu Thr Ala Ala Asp Thr Ala Thr Tyr Phe
               100                 105                 110
   Cys Ala Lys Ala Tyr Asp Leu
           115
<210> 236
   <211> 13
   <212> PRT
   <213> Oryctolagus cuniculus
<400> 236
   Gln Ala Ser Gln Ser Val Tyr Lys Asn Asn Tyr Leu Ser
   1               5                   10
<210> 237
   <211> 7
   <212> PRT
   <213> Oryctolagus cuniculus
<400> 237
   Ser Ala Ser Thr Leu Asp Ser
   1               5
<210> 238
   <211> 13
   <212> PRT
   <213> Oryctolagus cuniculus
<400> 238
   Leu Gly Ser Tyr Asp Cys Ser Ser Gly Asp Cys Tyr Ala
   1 5 10
<210> 239
   <211> 5
   <212> PRT
   <213> Oryctolagus cuniculus
<400> 239
   Ser Tyr Trp Met Cys
   1 5
<210> 240
   <211> 17
   <212> PRT
   <213> Oryctolagus cuniculus
<400> 240
   Cys Ile Val Thr Gly Asn Gly Asn Thr Tyr Tyr Ala Asn Trp Ala Lys
   1 5 10 15
   Gly
<210> 241
   <211> 4
   <212> PRT
   <213> Oryctolagus cuniculus
<400> 241
   Ala Tyr Asp Leu
   1
<210> 242
   <211> 375
   <212> DNA
   <213> Oryctolagus cuniculus
<400> 242
   atggacacga gggcccccac tcagctgctg gggctcctgc tgctctggct cccaggtgcc 60
   acatttgccc aagtgctgac ccagactgca tcgcccgtgt ctgcagctgt gggaggcaca 120
   gtcaccatca actgccaggc cagtcagagt gtttataaga acaactactt atcctggtat 180
   cagcagaaac cagggcagcc tcccaaaggc ctgatctatt ctgcatcgac tctagattct 240
   ggggtcccat tgcggttcag cggcagtgga tctgggacac agttcactct caccatcagc 300
   gacgtgcagt gtgacgatgc tgccacttac tactgtctag gcagttatga ttgtagtagt 360
   ggtgattgtt atgct 375
<210> 243
   <211> 357
   <212> DNA
   <213> Oryctolagus cuniculus
<400> 243
   atggagactg ggctgcgctg gcttctcctg gtcgctgtgc tcaaaggtgt ccagtgtcag     60
   tcgttggagg agtccggggg agacctggtc aagcctgagg gatccctgac actcacctgc    120
   acagcctctg gattctcctt cagtagctac tggatgtgct gggtccgcca ggctccaggg    180
   aaggggctgg agtggatcgc atgcattgtt actggtaatg gtaacactta ctacgcgaac    240
   tgggcgaaag gccgattcac catctccaaa acctcgtcga ccacggtgac tctgcaaatg    300
   accagtctga cagccgcgga cacggccacc tatttttgtg cgaaagccta tgacttg       357
<210> 244
   <211> 39
   <212> DNA
   <213> Oryctolagus cuniculus
<400> 244
   caggccagtc agagtgttta taagaacaac tacttatcc 39
<210> 245
   <211> 21
   <212> DNA
   <213> Oryctolagus cuniculus
<400> 245
   tctgcatcga ctctagattc t 21
<210> 246
   <211> 39
   <212> DNA
   <213> Oryctolagus cuniculus
<400> 246
   ctaggcagtt atgattgtag tagtggtgat tgttatgct 39
<210> 247
   <211> 15
   <212> DNA
   <213> Oryctolagus cuniculus
<400> 247
   agctactgga tgtgc 15
<210> 248
   <211> 51
   <212> DNA
   <213> Oryctolagus cuniculus
<400> 248
   tgcattgtta ctggtaatgg taacacttac tacgcgaact gggcgaaagg c 51
<210> 249
   <211> 12
   <212> DNA
   <213> Oryctolagus cuniculus
<400> 249
   gcctatgact tg 12
<210> 250
   <211> 123
   <212> PRT
   <213> Oryctolagus cuniculus
<400> 250
   Met Asp Thr Arg Ala Pro Thr Gln Leu Leu Gly Leu Leu Leu Leu Trp
   1               5                   10                  15
   Leu Pro Gly Ser Thr Phe Ala Ala Val Leu Thr Gln Thr Pro Ser Pro
               20                  25                  30
   Val Ser Ala Ala Val Gly Gly Thr Val Ser Ile Ser Cys Gln Ala Ser
           35                  40                  45
   Gln Ser Val Tyr Asp Asn Asn Tyr Leu Ser Trp Tyr Gln Gln Lys Pro
       50                  55                  60
   Gly Gln Pro Pro Lys Leu Leu Ile Tyr Gly Ala Ser Thr Leu Ala Ser
   65                  70                  75                  80
   Gly Val Pro Ser Arg Phe Lys Gly Thr Gly Ser Gly Thr Gln Phe Thr
                   85                  90                  95
   Leu Thr Ile Thr Asp Val Gln Cys Asp Asp Ala Ala Thr Tyr Tyr Cys
               100                 105                 110
   Ala Gly Val Phe Asn Asp Asp Ser Asp Asp Ala
           115                 120
<210> 251
   <211> 125
   <212> PRT
   <213> Oryctolagus cuniculus
<400> 251
   Met Glu Thr Gly Leu Arg Trp Leu Leu Leu Val Ala Val Pro Lys Gly
   1               5                   10                  15
   Val Gln Cys Gln Ser Leu Glu Glu Ser Gly Gly Arg Leu Val Thr Pro
               20                  25                  30
   Gly Thr Pro Leu Thr Leu Thr Cys Thr Leu Ser Gly Phe Ser Leu Ser
           35                  40                  45
   Ala Tyr Tyr Met Ser Trp Val Arg Gln Ala Pro Gly Lys Gly Leu Glu
       50                  55                  60
   Trp Ile Gly Phe Ile Thr Leu Ser Asp His Ile Ser Tyr Ala Arg Trp
   65                  70                  75                  80
   Ala Lys Gly Arg Phe Thr Ile Ser Lys Thr Ser Thr Thr Val Asp Leu
                   85                  90                  95
   Lys Met Thr Ser Pro Thr Thr Glu Asp Thr Ala Thr Tyr Phe Cys Ala
               100                 105                 110
   Arg Ser Arg Gly Trp Gly Ala Met Gly Arg Leu Asp Leu
           115                 120                 125
<210> 252
   <211> 13
   <212> PRT
   <213> Oryctolagus cuniculus
<400> 252
   Gln Ala Ser Gln Ser Val Tyr Asp Asn Asn Tyr Leu Ser
   1               5                   10
<210> 253
   <211> 7
   <212> PRT
   <213> Oryctolagus cuniculus
<400> 253
   Gly Ala Ser Thr Leu Ala Ser
   1               5
<210> 254
   <211> 11
   <212> PRT
   <213> Oryctolagus cuniculus
<400> 254
   Ala Gly Val Phe Asn Asp Asp Ser Asp Asp Ala
   1               5                   10
<210> 255
   <211> 5
   <212> PRT
   <213> Oryctolagus cuniculus
<400> 255
   Ala Tyr Tyr Met Ser
   1               5
<210> 256
   <211> 16
   <212> PRT
   <213> Oryctolagus cuniculus
<400> 256
   Phe Ile Thr Leu Ser Asp His Ile Ser Tyr Ala Arg Trp Ala Lys Gly
   1               5                   10                  15
<210> 257
   <211> 12
   <212> PRT
   <213> Oryctolagus cuniculus
<400> 257
   Ser Arg Gly Trp Gly Ala Met Gly Arg Leu Asp Leu
   1               5                   10
<210> 258
   <211> 369
   <212> DNA
   <213> Oryctolagus cuniculus
<400> 258
   atggacacga gggcccccac tcagctgctg gggctcctgc tgctctggct cccaggttcc 60
   acatttgccg ccgtgctgac ccagactcca tctcccgtgt ctgcagctgt gggaggcaca 120
   gtcagcatca gttgccaggc cagtcagagt gtttatgaca acaactattt atcctggtat 180
   cagcagaaac caggacagcc tcccaagctc ctgatctatg gtgcatccac tctggcatct 240
   ggggtcccat cgcggttcaa aggcacggga tctgggacac agttcactct caccatcaca 300
   gacgtgcagt gtgacgatgc tgccacttac tattgtgcag gcgtttttaa tgatgatagt 360
   gatgatgcc 369
<210> 259
   <211> 375
   <212> DNA
   <213> Oryctolagus cuniculus
<400> 259
   atggagactg ggctgcgctg gcttctcctg gtcgctgtgc ccaaaggtgt ccagtgtcag        60
   tcgctggagg agtccggggg tcgcctggtc acgcctggga cacccctgac actcacctgc       120
   acactctctg gattctccct cagtgcatac tatatgagct gggtccgcca ggctccaggg       180
   aaggggctgg aatggatcgg attcattact ctgagtgatc atatatctta cgcgaggtgg       240
   gcgaaaggcc gattcaccat ctccaaaacc tcgaccacgg tggatctgaa aatgaccagt       300
   ccgacaaccg aggacacggc cacctatttc tgtgccagga gtcgtggctg gggtgcaatg        360
   ggtcggttgg atctc                                                        375
<210> 260
   <211> 39
   <212> DNA
   <213> Oryctolagus cuniculus
<400> 260
   caggccagtc agagtgttta tgacaacaac tatttatcc 39
<210> 261
   <211> 21
   <212> DNA
   <213> Oryctolagus cuniculus
<400> 261
   ggtgcatcca ctctggcatc t 21
<210> 262
   <211> 33
   <212> DNA
   <213> Oryctolagus cuniculus
<400> 262
   gcaggcgttt ttaatgatga tagtgatgat gcc 33
<210> 263
   <211> 15
   <212> DNA
   <213> Oryctolagus cuniculus
<400> 263
   gcatactata tgagc 15
<210> 264
   <211> 48
   <212> DNA
   <213> Oryctolagus cuniculus
<400> 264
   ttcattactc tgagtgatca tatatcttac gcgaggtggg cgaaaggc 48
<210> 265
   <211> 36
   <212> DNA
   <213> Oryctolagus cuniculus
<400> 265
   agtcgtggct ggggtgcaat gggtcggttg gatctc 36
<210> 266
   <211> 123
   <212> PRT
   <213> Oryctolagus cuniculus
<400> 266
   Met Asp Thr Arg Ala Pro Thr Gln Leu Leu Gly Leu Leu Leu Leu Trp
   1               5                   10                  15
   Leu Pro Gly Ala Thr Phe Ala Ala Val Leu Thr Gln Thr Pro Ser Pro
               20                  25                  30
   Val Ser Ala Ala Val Gly Gly Thr Val Thr Ile Ser Cys Gln Ala Ser
           35                  40                  45
   Gln Ser Val Tyr Asn Asn Lys Asn Leu Ala Trp Tyr Gln Gln Lys Ser
       50                  55                  60
   Gly Gln Pro Pro Lys Leu Leu Ile Tyr Trp Ala Ser Thr Leu Ala Ser
   65                  70                  75                  80
   Gly Val Ser Ser Arg Phe Ser Gly Ser Gly Ser Gly Thr Gln Phe Thr
                   85                  90                  95
   Leu Thr Val Ser Gly Val Gln Cys Asp Asp Ala Ala Thr Tyr Tyr Cys
               100                 105                 110
   Leu Gly Val Phe Asp Asp Asp Ala Asp Asn Ala
           115                 120
<210> 267
   <211> 121
   <212> PRT
   <213> Oryctolagus cuniculus
<400> 267
   Met Glu Thr Gly Leu Arg Trp Leu Leu Leu Val Ala Val Leu Lys Gly
   1               5                   10                  15
   Val Gln Cys Gln Ser Val Glu Glu Ser Gly Gly Arg Leu Val Thr Pro
               20                  25                  30
   Gly Thr Pro Leu Thr Leu Thr Cys Thr Ala Ser Gly Phe Ser Leu Ser
   35 40 45
   Ser Tyr Ser Met Thr Trp Val Arg Gln Ala Pro Gly Lys Gly Leu Glu
   50 55 60
   Tyr Ile Gly Val Ile Gly Thr Ser Gly Ser Thr Tyr Tyr Ala Thr Trp
   65 70 75 80
   Ala Lys Gly Arg Phe Thr Ile Ser Arg Thr Ser Thr Thr Val Ala Leu
   85 90 95
   Lys Ile Thr Ser Pro Thr Thr Glu Asp Thr Ala Thr Tyr Phe Cys Val
   100 105 110
   Arg Ser Leu Ser Ser Ile Thr Phe Leu
   115 120
<210> 268
   <211> 13
   <212> PRT
   <213> Oryctolagus cuniculus
<400> 268
   Gln Ala Ser Gln Ser Val Tyr Asn Asn Lys Asn Leu Ala
   1 5 10
<210> 269
   <211> 7
   <212> PRT
   <213> Oryctolagus cuniculus
<400> 269
   Trp Ala Ser Thr Leu Ala Ser
   1 5
<210> 270
   <211> 11
   <212> PRT
   <213> Oryctolagus cuniculus
<400> 270
   Leu Gly Val Phe Asp Asp Asp Ala Asp Asn Ala
   1 5 10
<210> 271
   <211> 5
   <212> PRT
   <213> Oryctolagus cuniculus
<400> 271
   Ser Tyr Ser Met Thr
   1 5
<210> 272
   <211> 16
   <212> PRT
   <213> Oryctolagus cuniculus
<400> 272
   Val Ile Gly Thr Ser Gly Ser Thr Tyr Tyr Ala Thr Trp Ala Lys Gly
   1 5 10 15
<210> 273
   <211> 8
   <212> PRT
   <213> Oryctolagus cuniculus
<400> 273
   Ser Leu Ser Ser Ile Thr Phe Leu
   1 5
<210> 274
   <211> 369
   <212> DNA
   <213> Oryctolagus cuniculus
<400> 274
   atggacacga gggcccccac tcagctgctg gggctcctgc tgctctggct cccaggtgcc 60
   acattcgcag ccgtgctgac ccagacacca tcgcccgtgt ctgcggctgt gggaggcaca 120
   gtcaccatca gttgccaggc cagtcagagt gtttataaca acaaaaattt agcctggtat 180
   cagcagaaat cagggcagcc tcccaagctc ctgatctact gggcatccac tctggcatct 240
   ggggtctcat cgcggttcag cggcagtgga tctgggacac agttcactct caccgtcagc 300
   ggcgtgcagt gtgacgatgc tgccacttac tactgtctag gcgtttttga tgatgatgct 360
   gataatgct 369
<210> 275
   <211> 363
   <212> DNA
   <213> Oryctolagus cuniculus
<400> 275
   atggagactg ggctgcgctg gcttctcctg gtcgctgtgc tcaaaggtgt ccaatgtcag 60
   tcggtggagg agtccggggg tcgcctggtc acgcctggga cacccctgac actcacctgc 120
   acagcctctg gattctccct cagtagctac tccatgacct gggtccgcca ggctccaggg 180
   aaggggctgg aatatatcgg agtcattggt actagtggta gcacatacta cgcgacctgg 240
   gcgaaaggcc gattcaccat ctccagaacc tcgaccacgg tggctctgaa aatcaccagt 300
   ccgacaaccg aggacacggc cacctatttc tgtgtcagga gtctttcttc tattactttc 360
   ttg 363
<210> 276
   <211> 39
   <212> DNA
   <213> Oryctolagus cuniculus
<400> 276
   caggccagtc agagtgttta taacaacaaa aatttagcc 39
<210> 277
   <211> 21
   <212> DNA
   <213> Oryctolagus cuniculus
<400> 277
   tgggcatcca ctctggcatc t 21
<210> 278
   <211> 33
   <212> DNA
   <213> Oryctolagus cuniculus
<400> 278
   ctaggcgttt ttgatgatga tgctgataat gct 33
<210> 279
   <211> 15
   <212> DNA
   <213> Oryctolagus cuniculus
<400> 279
   agctactcca tgacc 15
<210> 280
   <211> 48
   <212> DNA
   <213> Oryctolagus cuniculus
<400> 280
   gtcattggta ctagtggtag cacatactac gcgacctggg cgaaaggc 48
<210> 281
   <211> 24
   <212> DNA
   <213> Oryctolagus cuniculus
<400> 281
   agtctttctt ctattacttt cttg 24
<210> 282
   <211> 120
   <212> PRT
   <213> Oryctolagus cuniculus
<400> 282
   Met Asp Thr Arg Ala Pro Thr Gln Leu Leu Gly Leu Leu Leu Leu Trp
   1               5                   10                  15
   Leu Pro Gly Ala Arg Cys Ala Phe Glu Leu Thr Gln Thr Pro Ala Ser
               20                  25                  30
   Val Glu Ala Ala Val Gly Gly Thr Val Thr Ile Asn Cys Gln Ala Ser
           35                  40                  45
   Gln Asn Ile Tyr Arg Tyr Leu Ala Trp Tyr Gln Gln Lys Pro Gly Gln
       50                  55                  60
   Pro Pro Lys Phe Leu Ile Tyr Leu Ala Ser Thr Leu Ala Ser Gly Val
   65                  70                  75                  80
   Pro Ser Arg Phe Lys Gly Ser Gly Ser Gly Thr Glu Phe Thr Leu Thr
                   85                  90                  95
   Ile Ser Asp Leu Glu Cys Ala Asp Ala Ala Thr Tyr Tyr Cys Gln Ser
               100                 105                 110
   Tyr Tyr Ser Ser Asn Ser Val Ala
           115                 120
<210> 283
   <211> 128
   <212> **PRT**
   <213> Oryctolagus cuniculus
<400> 283
   Met Glu Thr Gly Leu Arg Trp Leu Leu Leu Val Ala Val Leu Lys Gly
   1               5                   10                  15
   Val Gln Cys Gln Glu Gln Leu Val Glu Ser Gly Gly Asp Leu Val Gln
               20                  25                  30
   Pro Glu Gly Ser Leu Thr Leu Thr Cys Thr Ala Ser Glu Leu Asp Phe
           35                  40                  45
   Ser Ser Gly Tyr Trp Ile Cys Trp Val Arg Gln Val Pro Gly Lys Gly
       50                  55                  60
   Leu Glu Trp Ile Gly Cys Ile Tyr Thr Gly Ser Ser Gly Ser Thr Phe
   65                  70                  75                  80
   Tyr Ala Ser Trp Ala Lys Gly Arg Phe Thr Ile Ser Lys Thr Ser Ser
   85 90 95
   Thr Thr Val Thr Leu Gln Met Thr Ser Leu Thr Ala Ala Asp Thr Ala
   100 105 110
   Thr Tyr Phe Cys Ala Arg Gly Tyr Ser Gly Phe Gly Tyr Phe Lys Leu
   115 120 125
<210> 284
   <211> 11
   <212> PRT
   <213> Oryctolagus cuniculus
<400> 284
   Gln Ala Ser Gln Asn Ile Tyr Arg Tyr Leu Ala
   1 5 10
<210> 285
   <211> 7
   <212> PRT
   <213> Oryctolagus cuniculus
<400> 285
   Leu Ala Ser Thr Leu Ala Ser
   1 5
<210> 286
   <211> 10
   <212> PRT
   <213> Oryctolagus cuniculus
<400> 286
   Gln Ser Tyr Tyr Ser Ser Asn Ser Val Ala
   1 5 10
<210> 287
   <211> 6
   <212> PRT
   <213> Oryctolagus cuniculus
<400> 287
   Ser Gly Tyr Trp Ile Cys
   1 5
<210> 288
   <211> 18
   <212> PRT
   <213> Oryctolagus cuniculus
<400> 288
<210> 289
   <211> 10
   <212> PRT
   <213> Oryctolagus cuniculus
<400> 289
   Gly Tyr Ser Gly Phe Gly Tyr Phe Lys Leu
   1               5                   10
<210> 290
   <211> 360
   <212> DNA
   <213> Oryctolagus cuniculus
<400> 290
   atggacacga gggcccccac tcagctgctg gggctcctgc tgctctggct cccaggtgcc 60
   agatgtgcat tcgaattgac ccagactcca gcctccgtgg aggcagctgt gggaggcaca 120
   gtcaccatca attgccaggc cagtcagaac atttatagat acttagcctg gtatcagcag 180
   aaaccagggc agcctcccaa gttcctgatc tatctggcat ctactctggc atctggggtc 240
   ccatcgcggt ttaaaggcag tggatctggg acagagttca ctctcaccat cagcgacctg 300
   gagtgtgccg atgctgccac ttactactgt caaagttatt atagtagtaa tagtgtcgct 360
<210> 291
   <211> 384
   <212> DNA
   <213> Oryctolagus cuniculus
<400> 291
   atggagactg ggctgcgctg gcttctcctg gtcgctgtgc tcaaaggtgt ccagtgtcag 60
   gagcagctgg tggagtccgg gggagacctg gtccagcctg agggatccct gacactcacc 120
   tgcacagctt ctgagttaga cttcagtagc ggctactgga tatgctgggt ccgccaggtt 180
   ccagggaagg ggctggagtg gatcggatgc atttatactg gtagtagtgg tagcactttt 240
   tacgcgagtt gggcgaaagg ccgattcacc atctccaaaa cctcgtcgac cacggtgact 300
   ctgcaaatga ccagtctgac agccgcggac acggccacct atttctgtgc gagaggttat 360
   agtggctttg gttactttaa gttg 384
<210> 292
   <211> 33
   <212> DNA
   <213> Oryctolagus cuniculus
<400> 292
   caggccagtc agaacattta tagatactta gcc 33
<210> 293
   <211> 21
   <212> DNA
   <213> Oryctolagus cuniculus
<400> 293
   ctggcatcta ctctggcatc t 21
<210> 294
   <211> 30
   <212> DNA
   <213> Oryctolagus cuniculus
<400> 294
   caaagttatt atagtagtaa tagtgtcgct 30
<210> 295
   <211> 18
   <212> DNA
   <213> Oryctolagus cuniculus
<400> 295
   agcggctact ggatatgc 18
<210> 296
   <211> 54
   <212> DNA
   <213> Oryctolagus cuniculus
<400> 296
   tgcatttata ctggtagtag tggtagcact ttttacgcga gttgggcgaa aggc 54
<210> 297
   <211> 30
   <212> DNA
   <213> Oryctolagus cuniculus
<400> 297
   ggttatagtg gctttggtta ctttaagttg 30
<210> 298
   <211> 122
   <212> PRT
   <213> Oryctolagus cuniculus
<400> 298
   Met Asp Thr Arg Ala Pro Thr Gln Leu Leu Gly Leu Leu Leu Leu Trp
   1               5                   10                  15
   Leu Pro Gly Ala Arg Cys Ala Tyr Asp Met Thr Gln Thr Pro Ala Ser
   20 25 30
   Val Glu Val Ala Val Gly Gly Thr Val Thr Ile Lys Cys Gln Ala Ser
   35 40 45
   Glu Asp Ile Tyr Arg Leu Leu Ala Trp Tyr Gln Gln Lys Pro Gly Gln
   50 55 60
   Pro Pro Lys Leu Leu Ile Tyr Asp Ser Ser Asp Leu Ala Ser Gly Val
   65 70 75 80
   Pro Ser Arg Phe Lys Gly Ser Gly Ser Gly Thr Glu Phe Thr Leu Ala
   85 90 95
   Ile Ser Gly Val Gln Cys Asp Asp Ala Ala Thr Tyr Tyr Cys Gln Gln
   100 105 110
   Ala Trp Ser Tyr Ser Asp Ile Asp Asn Ala
   115 120
<210> 299
   <211> 123
   <212> PRT
   <213> Oryctolagus cuniculus
<400> 299
   Met Glu Thr Gly Leu Arg Trp Leu Leu Leu Val Ala Val Leu Lys Gly
   1 5 10 15
   Val Gln Cys Gln Ser Val Glu Glu Ser Gly Gly Arg Leu Val Thr Pro
   20 25 30
   Gly Thr Pro Leu Thr Leu Thr Cys Thr Ala Ser Gly Phe Ser Leu Ser
   35 40 45
   Ser Tyr Tyr Met Ser Trp Val Arg Gln Ala Pro Gly Lys Gly Leu Glu
   50 55 60
   Trp Ile Gly Ile Ile Thr Thr Ser Gly Asn Thr Phe Tyr Ala Ser Trp
   65 70 75 80
   Ala Lys Gly Arg Leu Thr Ile Ser Arg Thr Ser Thr Thr Val Asp Leu
   85 90 95
   Lys Ile Thr Ser Pro Thr Thr Glu Asp Thr Ala Thr Tyr Phe Cys Ala
   100 105 110
   Arg Thr Ser Asp Ile Phe Tyr Tyr Arg Asn Leu
   115 120
<210> 300
   <211> 11
   <212> PRT
   <213> Oryctolagus cuniculus
<400> 300
   Gln Ala Ser Glu Asp Ile Tyr Arg Leu Leu Ala
   1 5 10
<210> 301
   <211> 7
   <212> PRT
   <213> Oryctolagus cuniculus
<400> 301
   Asp Ser Ser Asp Leu Ala Ser
   1 5
<210> 302
   <211> 12
   <212> PRT
   <213> Oryctolagus cuniculus
<400> 302
   Gln Gln Ala Trp Ser Tyr Ser Asp Ile Asp Asn Ala
   1 5 10
<210> 303
   <211> 5
   <212> PRT
   <213> Oryctolagus cuniculus
<400> 303
   Ser Tyr Tyr Met Ser
   1 5
<210> 304
   <211> 16
   <212> PRT
   <213> Oryctolagus cuniculus
<400> 304
   Ile Ile Thr Thr Ser Gly Asn Thr Phe Tyr Ala Ser Trp Ala Lys Gly
   1 5 10 15
<210> 305
   <211> 10
   <212> PRT
   <213> Oryctolagus cuniculus
<400> 305
   Thr Ser Asp Ile Phe Tyr Tyr Arg Asn Leu
   1 5 10
<210> 306
   <211> 366
   <212> DNA
   <213> Oryctolagus cuniculus
<400> 306
   atggacacga gggcccccac tcagctgctg gggctcctgc tgctctggct cccaggtgcc 60
   agatgtgcct atgatatgac ccagactcca gcctctgtgg aggtagctgt gggaggcaca 120
   gtcaccatca agtgccaggc cagtgaggac atttataggt tattggcctg gtatcaacag 180
   aaaccagggc agcctcccaa gctcctgatc tatgattcat ccgatctggc atctggggtc 240
   ccatcgcggt tcaaaggcag tggatctggg acagagttca ctctcgccat cagcggtgtg 300
   cagtgtgacg atgctgccac ttactactgt caacaggctt ggagttatag tgatattgat 360
   aatgct 366
<210> 307
   <211> 369
   <212> DNA
   <213> Oryctolagus cuniculus
<400> 307
   atggagactg ggctgcgctg gcttctcctg gtcgctgtgc tcaaaggtgt ccagtgtcag 60
   tcggtggagg agtccggggg tcgcctggtc acgccgggga cacccctgac actcacctgc 120
   acagcctctg gattctccct cagtagctac tacatgagct gggtccgcca ggctccaggg 180
   aaggggctgg aatggatcgg aatcattact actagtggta atacatttta cgcgagctgg 240
   gcgaaaggcc ggctcaccat ctccagaacc tcgaccacgg tggatctgaa aatcaccagt 300
   ccgacaaccg aggacacggc cacctatttc tgtgccagaa cttctgatat tttttattat 360
   cgtaacttg 369
<210> 308
   <211> 33
   <212> DNA
   <213> Oryctolagus cuniculus
<400> 308
   caggccagtg aggacattta taggttattg gcc 33
<210> 309
   <211> 21
   <212> DNA
   <213> Oryctolagus cuniculus
<400> 309
   gattcatccg atctggcatc t 21
<210> 310
   <211> 36
   <212> DNA
   <213> Oryctolagus cuniculus
<400> 310
   caacaggctt ggagttatag tgatattgat aatgct 36
<210> 311
   <211> 15
   <212> DNA
   <213> Oryctolagus cuniculus
<400> 311
   agctactaca tgagc 15
<210> 312
   <211> 48
   <212> DNA
   <213> Oryctolagus cuniculus
<400> 312
   atcattacta ctagtggtaa tacattttac gcgagctggg cgaaaggc 48
<210> 313
   <211> 30
   <212> DNA
   <213> Oryctolagus cuniculus
<400> 313
   acttctgata ttttttatta tcgtaacttg 30
<210> 314
   <211> 123
   <212> PRT
   <213> Oryctolagus cuniculus
<400> 314
   Met Asp Thr Arg Ala Pro Thr Gln Leu Leu Gly Leu Leu Leu Leu Trp
   1               5                   10                  15
   Leu Pro Gly Ala Thr Phe Ala Ala Val Leu Thr Gln Thr Ala Ser Pro
               20                  25                  30
   Val Ser Ala Ala Val Gly Ala Thr Val Thr Ile Asn Cys Gln Ser Ser
           35                  40                  45
   Gln Ser Val Tyr Asn Asp Met Asp Leu Ala Trp Phe Gln Gln Lys Pro
       50                  55                  60
   Gly Gln Pro Pro Lys Leu Leu Ile Tyr Ser Ala Ser Thr Leu Ala Ser
   65                  70                  75                  80
   Gly Val Pro Ser Arg Phe Ser Gly Ser Gly Ser Gly Thr Glu Phe Thr
                   85                  90                  95
   Leu Thr Ile Ser Gly Val Gln Cys Asp Asp Ala Ala Thr Tyr Tyr Cys
               100                 105                 110
   Leu Gly Ala Phe Asp Asp Asp Ala Asp Asn Thr
           115                 120
<210> 315
   <211> 129
   <212> PRT
   <213> Oryctolagus cuniculus
<400> 315
   Met Glu Thr Gly Leu Arg Trp Leu Leu Leu Val Ala Val Leu Lys Gly
   1               5                   10                  15
   Val Gln Cys Gln Ser Val Glu Glu Ser Gly Gly Arg Leu Val Thr Pro
               20                  25                  30
   Gly Thr Pro Leu Thr Leu Thr Cys Thr Val Ser Gly Phe Ser Leu Thr
           35                  40                  45
   Arg His Ala Ile Thr Trp Val Arg Gln Ala Pro Gly Lys Gly Leu Glu
       50                  55                  60
   Trp Ile Gly Cys Ile Trp Ser Gly Gly Ser Thr Tyr Tyr Ala Thr Trp
   65                  70                  75                  80
   Ala Lys Gly Arg Phe Thr Ile Ser Lys Thr Ser Thr Thr Val Asp Leu
                   85                  90                  95
   Arg Ile Thr Ser Pro Thr Thr Glu Asp Thr Ala Thr Tyr Phe Cys Ala
               100                 105                 110
   Arg Val Ile Gly Asp Thr Ala Gly Tyr Ala Tyr Phe Thr Gly Leu Asp
           115                 120                 125
   Leu
<210> 316
   <211> 13
   <212> PRT
   <213> Oryctolagus cuniculus
<400> 316
   Gln Ser Ser Gln Ser Val Tyr Asn Asp Met Asp Leu Ala
   1 5 10
<210> 317
   <211> 7
   <212> PRT
   <213> Oryctolagus cuniculus
<400> 317
   Ser Ala Ser Thr Leu Ala Ser
   1 5
<210> 318
   <211> 11
   <212> PRT
   <213> Oryctolagus cuniculus
<400> 318
   Leu Gly Ala Phe Asp Asp Asp Ala Asp Asn Thr
   1 5 10
<210> 319
   <211> 5
   <212> **PRT**
   <213> Oryctolagus cuniculus
<400> 319
   Arg His Ala Ile Thr
   1 5
<210> 320
   <211> 16
   <212> PRT
   <213> Oryctolagus cuniculus
<400> 320
   Cys Ile Trp Ser Gly Gly Ser Thr Tyr Tyr Ala Thr Trp Ala Lys Gly
   1 5 10 15
<210> 321
   <211> 16
   <212> PRT
   <213> Oryctolagus cuniculus
<400> 321
   Val Ile Gly Asp Thr Ala Gly Tyr Ala Tyr Phe Thr Gly Leu Asp Leu
   1 5 10 15
<210> 322
   <211> 369
   <212> DNA
   <213> Oryctolagus cuniculus
<400> 322
   atggacacga gggcccccac tcagctgctg gggctcctgc tgctctggct cccaggtgcc 60
   acgtttgcag ccgtgctgac ccagactgca tcacccgtgt ctgccgctgt gggagccaca 120
   gtcaccatca actgccagtc cagtcagagt gtttataatg acatggactt agcctggttt 180
   cagcagaaac cagggcagcc tcccaagctc ctgatctatt ctgcatccac tctggcatct 240
   ggggtcccat cgcggttcag cggcagtgga tctgggacag agttcactct caccatcagc 300
   ggcgtgcagt gtgacgatgc tgccacttac tactgtctag gcgcttttga tgatgatgct 360
   gataatact 369
<210> 323
   <211> 387
   <212> DNA
   <213> Oryctolagus cuniculus
<400> 323
   atggagactg ggctgcgctg gcttctcctg gtcgctgtgc tcaaaggtgt ccagtgtcag 60
   tcggtggagg agtccggggg tcgcctggtc acgcctggga cacccctgac actcacctgc 120
   acagtctctg gattctccct cactaggcat gcaataacct gggtccgcca ggctccaggg 180
   aaggggctgg aatggatcgg atgcatttgg agtggtggta gcacatacta cgcgacctgg 240
   gcgaaaggcc gattcaccat ctccaaaacc tcgaccacgg tggatctcag aatcaccagt 300
   ccgacaaccg aggacacggc cacctacttc tgtgccagag tcattggcga tactgctggt 360
   tatgcttatt ttacggggct tgacttg 387
<210> 324
   <211> 39
   <212> DNA
   <213> Oryctolagus cuniculus
<400> 324
   cagtccagtc agagtgttta taatgacatg gacttagcc 39
<210> 325
   <211> 21
   <212> DNA
   <213> Oryctolagus cuniculus
<400> 325
   tctgcatcca ctctggcatc t 21
<210> 326
   <211> 33
   <212> DNA
   <213> Oryctolagus cuniculus
<400> 326
   ctaggcgctt ttgatgatga tgctgataat act 33
<210> 327
   <211> 15
   <212> DNA
   <213> Oryctolagus cuniculus
<400> 327
   aggcatgcaa taacc 15
<210> 328
   <211> 48
   <212> DNA
   <213> Oryctolagus cuniculus
<400> 328
   tgcatttgga gtggtggtag cacatactac gcgacctggg cgaaaggc 48
<210> 329
   <211> 48
   <212> DNA
   <213> Oryctolagus cuniculus
<400> 329
   gtcattggcg atactgctgg ttatgcttat tttacggggc ttgacttg 48
<210> 330
   <211> 121
   <212> PRT
   <213> Oryctolagus cuniculus
<400> 330
   Met Asp Thr Arg Ala Pro Thr Gln Leu Leu Gly Leu Leu Leu Leu Trp
   1               5                   10                  15
   Leu Pro Gly Ala Arg Cys Ala Tyr Asp Met Thr Gln Thr Pro Ala Ser
               20                  25                  30
   Val Glu Val Ala Val Gly Gly Thr Val Thr Ile Lys Cys Gln Ala Ser
           35                  40                  45
   Gln Ser Val Tyr Asn Trp Leu Ser Trp Tyr Gln Gln Lys Pro Gly Gln
       50                  55                  60
   Pro Pro Lys Leu Leu Ile Tyr Thr Ala Ser Ser Leu Ala Ser Gly Val
   65                  70                  75                  80
   Pro Ser Arg Phe Ser Gly Ser Gly Ser Gly Thr Glu Phe Thr Leu Thr
                   85                  90                  95
   Ile Ser Gly Val Glu Cys Ala Asp Ala Ala Thr Tyr Tyr Cys Gln Gln
   100 105 110
   Gly Tyr Thr Ser Asp Val Asp Asn Val
   115 120
<210> 331
   <211> 130
   <212> PRT
   <213> Oryctolagus cuniculus
<400> 331
   Met Glu Thr Gly Leu Arg Trp Leu Leu Leu Val Ala Val Leu Lys Gly
   1 5 10 15
   Val Gln Cys Gln Ser Leu Glu Glu Ala Gly Gly Arg Leu Val Thr Pro
   20 25 30
   Gly Thr Pro Leu Thr Leu Thr Cys Thr Val Ser Gly Ile Asp Leu Ser
   35 40 45
   Ser Tyr Ala Met Gly Trp Val Arg Gln Ala Pro Gly Lys Gly Leu Glu
   50 55 60
   Tyr Ile Gly Ile Ile Ser Ser Ser Gly Ser Thr Tyr Tyr Ala Thr Trp
   65 70 75 80
   Ala Lys Gly Arg Phe Thr Ile Ser Gln Ala Ser Ser Thr Thr Val Asp
   85 90 95
   Leu Lys Ile Thr Ser Pro Thr Thr Glu Asp Ser Ala Thr Tyr Phe Cys
   100 105 110
   Ala Arg Gly Gly Ala Gly Ser Gly Gly Val Trp Leu Leu Asp Gly Phe
   115 120 125
   Asp Pro
   130
<210> 332
   <211> 11
   <212> PRT
   <213> Oryctolagus cuniculus
<400> 332
   Gln Ala Ser Gln Ser Val Tyr Asn Trp Leu Ser
   1 5 10
<210> 333
   <211> 7
   <212> PRT
   <213> Oryctolagus cuniculus
<400> 333
   Thr Ala Ser Ser Leu Ala Ser
   1 5
<210> 334
   <211> 11
   <212> PRT
   <213> Oryctolagus cuniculus
<400> 334
   Gln Gln Gly Tyr Thr Ser Asp Val Asp Asn Val
   1 5 10
<210> 335
   <211> 5
   <212> PRT
   <213> Oryctolagus cuniculus
<400> 335
   Ser Tyr Ala Met Gly
   1 5
<210> 336
   <211> 16
   <212> PRT
   <213> Oryctolagus cuniculus
<400> 336
   Ile Ile Ser Ser Ser Gly Ser Thr Tyr Tyr Ala Thr Trp Ala Lys Gly
   1 5 10 15
<210> 337
   <211> 16
   <212> PRT
   <213> Oryctolagus cuniculus
<400> 337
   Gly Gly Ala Gly Ser Gly Gly Val Trp Leu Leu Asp Gly Phe Asp Pro
   1 5 10 15
<210> 338
   <211> 363
   <212> DNA
   <213> Oryctolagus cuniculus
<400> 338
   atggacacga gggcccccac tcagctgctg gggctcctgc tgctctggct cccaggtgcc 60
   agatgtgcct atgatatgac ccagactcca gcctctgtgg aggtagctgt gggaggcaca 120
   gtcaccatca agtgccaggc cagtcagagt gtttataatt ggttatcctg gtatcagcag 180
   aaaccagggc agcctcccaa gctcctgatc tatactgcat ccagtctggc atctggggtc 240
   ccatcgcggt tcagtggcag tggatctggg acagagttca ctctcaccat cagcggcgtg 300
   gagtgtgccg atgctgccac ttactactgt caacagggtt atactagtga tgttgataat 360
   gtt 363
<210> 339
   <211> 390
   <212> DNA
   <213> Oryctolagus cuniculus
<400> 339
   atggagactg ggctgcgctg gcttctcctg gtcgctgtgc tcaaaggtgt ccagtgtcag 60
   tcgctggagg aggccggggg tcgcctggtc acgcctggga cacccctgac actcacctgc 120
   acagtctctg gaatcgacct cagtagctat gcaatgggct gggtccgcca ggctccaggg 180
   aaggggctgg aatacatcgg aatcattagt agtagtggta gcacatacta cgcgacctgg 240
   gcgaaaggcc gattcaccat ctcacaagcc tcgtcgacca cggtggatct gaaaattacc 300
   agtccgacaa ccgaggactc ggccacatat ttctgtgcca gagggggtgc tggtagtggt 360
   ggtgtttggc tgcttgatgg ttttgatccc 390
<210> 340
   <211> 33
   <212> DNA
   <213> Oryctolagus cuniculus
<400> 340
   caggccagtc agagtgttta taattggtta tcc 33
<210> 341
   <211> 21
   <212> DNA
   <213> Oryctolagus cuniculus
<400> 341
   actgcatcca gtctggcatc t 21
<210> 342
   <211> 33
   <212> DNA
   <213> Oryctolagus cuniculus
<400> 342
   caacagggtt atactagtga tgttgataat gtt 33
<210> 343
   <211> 15
   <212> DNA
   <213> Oryctolagus cuniculus
<400> 343
   agctatgcaa tgggc 15
<210> 344
   <211> 48
   <212> DNA
   <213> Oryctolagus cuniculus
<400> 344
   atcattagta gtagtggtag cacatactac gcgacctggg cgaaaggc 48
<210> 345
   <211> 48
   <212> DNA
   <213> Oryctolagus cuniculus
<400> 345
   gggggtgctg gtagtggtgg tgtttggctg cttgatggtt ttgatccc 48
<210> 346
   <211> 123
   <212> PRT
   <213> Oryctolagus cuniculus
<400> 346
   Met Asp Thr Arg Ala Pro Thr Gln Leu Leu Gly Leu Leu Leu Leu Trp
   1               5                   10                  15
   Leu Pro Gly Ala Lys Cys Ala Asp Val Val Met Thr Gln Thr Pro Ala
               20                  25                  30
   Ser Val Ser Ala Ala Val Gly Gly Thr Val Thr Ile Asn Cys Gln Ala
           35                  40                  45
   Ser Glu Asn Ile Tyr Asn Trp Leu Ala Trp Tyr Gln Gln Lys Pro Gly
       50                  55                  60
   Gln Pro Pro Lys Leu Leu Ile Tyr Thr Val Gly Asp Leu Ala Ser Gly
   65                  70                  75                  80
   Val Ser Ser Arg Phe Lys Gly Ser Gly Ser Gly Thr Glu Phe Thr Leu
                   85                  90                  95
   Thr Ile Ser Asp Leu Glu Cys Ala Asp Ala Ala Thr Tyr Tyr Cys Gln
               100                 105                 110
   Gln Gly Tyr Ser Ser Ser Tyr Val Asp Asn Val
           115                 120
<210> 347
   <211> 130
   <212> PRT
   <213> Oryctolagus cuniculus
<400> 347
   Met Glu Thr Gly Leu Arg Trp Leu Leu Leu Val Ala Val Leu Lys Gly
   1               5                   10                  15
   Val Gln Cys Gln Glu Gln Leu Lys Glu Ser Gly Gly Arg Leu Val Thr
               20                  25                  30
   Pro Gly Thr Pro Leu Thr Leu Thr Cys Thr Val Ser Gly Phe Ser Leu
           35                  40                  45
   Asn Asp Tyr Ala Val Gly Trp Phe Arg Gln Ala Pro Gly Lys Gly Leu
       50                  55                  60
   Glu Trp Ile Gly Tyr Ile Arg Ser Ser Gly Thr Thr Ala Tyr Ala Thr
   65                  70                  75                  80
   Trp Ala Lys Gly Arg Phe Thr Ile Ser Ala Thr Ser Thr Thr Val Asp
                   85                  90                  95
   Leu Lys Ile Thr Ser Pro Thr Thr Glu Asp Thr Ala Thr Tyr Phe Cys
               100                 105                 110
   Ala Arg Gly Gly Ala Gly Ser Ser Gly Val Trp Ile Leu Asp Gly Phe
           115                 120                 125
   Ala Pro
       130
<210> 348
   <211> 11
   <212> PRT
   <213> Oryctolagus cuniculus
<400> 348
   Gln Ala Ser Glu Asn Ile Tyr Asn Trp Leu Ala
   1               5                   10
<210> 349
   <211> 7
   <212> PRT
   <213> Oryctolagus cuniculus
<400> 349
   Thr Val Gly Asp Leu Ala Ser
   1               5
<210> 350
   <211> 12
   <212> PRT
   <213> Oryctolagus cuniculus
<400> 350
   Gln Gln Gly Tyr Ser Ser Ser Tyr Val Asp Asn Val
   1 5 10
<210> 351
   <211> 5
   <212> PRT
   <213> Oryctolagus cuniculus
<400> 351
   Asp Tyr Ala Val Gly
   1 5
<210> 352
   <211> 16
   <212> PRT
   <213> Oryctolagus cuniculus
<400> 352
   Tyr Ile Arg Ser Ser Gly Thr Thr Ala Tyr Ala Thr Trp Ala Lys Gly
   1 5 10 15
<210> 353
   <211> 16
   <212> PRT
   <213> Oryctolagus cuniculus
<400> 353
   Gly Gly Ala Gly Ser Ser Gly Val Trp Ile Leu Asp Gly Phe Ala Pro
   1 5 10 15
<210> 354
   <211> 369
   <212> DNA
   <213> Oryctolagus cuniculus
<400> 354
   atggacacga gggcccccac tcagctgctg gggctcctgc tgctctggct cccaggtgcc 60
   aaatgtgccg atgttgtgat gacccagact ccagcctccg tgtctgcagc tgtgggaggc 120
   acagtcacca tcaattgcca ggccagtgag aacatttata attggttagc ctggtatcag 180
   cagaaaccag ggcagcctcc caagctcctg atctatactg taggcgatct ggcatctggg 240
   gtctcatcgc ggttcaaagg cagtggatct gggacagagt tcactctcac catcagcgac 300
   ctggagtgtg ccgatgctgc cacttactat tgtcaacagg gttatagtag tagttatgtt 360
   gataatgtt 369
<210> 355
   <211> 390
   <212> DNA
   <213> Oryctolagus cuniculus
<400> 355
   atggagactg ggctgcgctg gcttctcctg gtcgctgtgc tcaaaggtgt ccagtgtcag        60
   gagcagctga aggagtccgg gggtcgcctg gtcacgcctg ggacacccct gacactcacc       120
   tgcacagtct ctggattctc cctcaatgac tatgcagtgg gctggttccg ccaggctcca       180
   gggaaggggc tggaatggat cggatacatt cgtagtagtg gtaccacagc ctacgcgacc       240
   tgggcgaaag gccgattcac catctccgct acctcgacca cggtggatct gaaaatcacc       300
   agtccgacaa ccgaggacac ggccacctat ttctgtgcca gagggggtgc tggtagtagt       360
   ggtgtgtgga tccttgatgg ttttgctccc                                        390
<210> 356
   <211> 33
   <212> DNA
   <213> Oryctolagus cuniculus
<400> 356
   caggccagtg agaacattta taattggtta gcc 33
<210> 357
   <211> 21
   <212> DNA
   <213> Oryctolagus cuniculus
<400> 357
   actgtaggcg atctggcatc t 21
<210> 358
   <211> 36
   <212> DNA
   <213> Oryctolagus cuniculus
<400> 358
   caacagggtt atagtagtag ttatgttgat aatgtt 36
<210> 359
   <211> 15
   <212> DNA
   <213> Oryctolagus cuniculus
<400> 359
   gactatgcag tgggc 15
<210> 360
   <211> 48
   <212> DNA
   <213> Oryctolagus cuniculus
<400> 360
   tacattcgta gtagtggtac cacagcctac gcgacctggg cgaaaggc 48
<210> 361
   <211> 48
   <212> DNA
   <213> Oryctolagus cuniculus
<400> 361
   gggggtgctg gtagtagtgg tgtgtggatc cttgatggtt ttgctccc 48
<210> 362
   <211> 121
   <212> PRT
   <213> Oryctolagus cuniculus
<400> 362
   Met Asp Thr Arg Ala Pro Thr Gln Leu Leu Gly Leu Leu Leu Leu Trp
   1               5                   10                  15
   Leu Pro Gly Ala Thr Phe Ala Gln Val Leu Thr Gln Thr Pro Ser Ser
               20                  25                  30
   Val Ser Ala Ala Val Gly Gly Thr Val Thr Ile Asn Cys Gln Ala Ser
           35                  40                  45
   Gln Ser Val Tyr Gln Asn Asn Tyr Leu Ser Trp Phe Gln Gln Lys Pro
       50                  55                  60
   Gly Gln Pro Pro Lys Leu Leu Ile Tyr Gly Ala Ala Thr Leu Ala Ser
   65                  70                  75                  80
   Gly Val Pro Ser Arg Phe Lys Gly Ser Gly Ser Gly Thr Gln Phe Thr
                   85                  90                  95
   Leu Thr Ile Ser Asp Leu Glu Cys Asp Asp Ala Ala Thr Tyr Tyr Cys
               100                 105                 110
   Ala Gly Ala Tyr Arg Asp Val Asp Ser
           115                 120
<210> 363
   <211> 130
   <212> PRT
   <213> Oryctolagus cuniculus
<400> 363
   Met Glu Thr Gly Leu Arg Trp Leu Leu Leu Val Ala Val Leu Lys Gly
   1               5                   10                  15
   Val Gln Cys Gln Ser Leu Glu Glu Ser Gly Gly Asp Leu Val Lys Pro
               20                  25                  30
   Gly Ala Ser Leu Thr Leu Thr Cys Thr Ala Ser Gly Phe Ser Phe Thr
           35                  40                  45
   Ser Thr Tyr Tyr Ile Tyr Trp Val Arg Gln Ala Pro Gly Lys Gly Leu
       50                  55                  60
   Glu Trp Ile Ala Cys Ile Asp Ala Gly Ser Ser Gly Ser Thr Tyr Tyr
   65                  70                  75                  80
   Ala Thr Trp Val Asn Gly Arg Phe Thr Ile Ser Lys Thr Ser Ser Thr
                   85                  90                  95
   Thr Val Thr Leu Gln Met Thr Ser Leu Thr Ala Ala Asp Thr Ala Thr
               100                 105                 110
   Tyr Phe Cys Ala Lys Trp Asp Tyr Gly Gly Asn Val Gly Trp Gly Tyr
           115                 120                 125
   Asp Leu
       130
<210> 364
   <211> 13
   <212> PRT
   <213> Oryctolagus cuniculus
<400> 364
   Gln Ala Ser Gln Ser Val Tyr Gln Asn Asn Tyr Leu Ser
   1               5                   10
<210> 365
   <211> 7
   <212> PRT
   <213> Oryctolagus cuniculus
<400> 365
   Gly Ala Ala Thr Leu Ala Ser
   1               5
<210> 366
   <211> 9
   <212> PRT
   <213> Oryctolagus cuniculus
<400> 366
   Ala Gly Ala Tyr Arg Asp Val Asp Ser
   1               5
<210> 367
   <211> 6
   <212> PRT
   <213> Oryctolagus cuniculus
<400> 367
   Ser Thr Tyr Tyr Ile Tyr
   1               5
<210> 368
   <211> 18
   <212> PRT
   <213> Oryctolagus cuniculus
<400> 368
   Cys Ile Asp Ala Gly Ser Ser Gly Ser Thr Tyr Tyr Ala Thr Trp Val
   1               5                   10                  15
   Asn Gly
<210> 369
   <211> 13
   <212> PRT
   <213> Oryctolagus cuniculus
<400> 369
   Trp Asp Tyr Gly Gly Asn Val Gly Trp Gly Tyr Asp Leu
   1               5                   10
<210> 370
   <211> 363
   <212> DNA
   <213> Oryctolagus cuniculus
<400> 370
   atggacacga gggcccccac tcagctgctg gggctcctgc tgctctggct cccaggtgcc 60
   acatttgctc aagtgctgac ccagactcca tcctccgtgt ctgcagctgt gggaggcaca 120
   gtcaccatca attgccaggc cagtcagagt gtttatcaga acaactactt atcctggttt 180
   cagcagaaac cagggcagcc tcccaagctc ctgatctatg gtgcggccac tctggcatct 240
   ggggtcccat cgcggttcaa aggcagtgga tctgggacac agttcactct caccatcagc 300
   gacctggagt gtgacgatgc tgccacttac tactgtgcag gcgcttatag ggatgtggat 360
   tct 363
<210> 371
   <211> 390
   <212> DNA
   <213> Oryctolagus cuniculus
<400> 371
   atggagactg ggctgcgctg gcttctcctg gtcgctgtgc tcaaaggtgt ccagtgtcag 60
   tcgttggagg agtccggggg agacctggtc aagcctgggg catccctgac actcacctgc 120
   acagcctctg gattctcctt tactagtacc tactacatct actgggtccg ccaggctcca 180
   gggaaggggc tggagtggat cgcatgtatt gatgctggta gtagtggtag cacttactac 240
   gcgacctggg tgaatggccg attcaccatc tccaaaacct cgtcgaccac ggtgactctg 300
   caaatgacca gtctgacagc cgcggacacg gccacctatt tctgtgcgaa atgggattat 360
   ggtggtaatg ttggttgggg ttatgacttg 390
<210> 372
   <211> 39
   <212> DNA
   <213> Oryctolagus cuniculus
<400> 372
   caggccagtc agagtgttta tcagaacaac tacttatcc 39
<210> 373
   <211> 21
   <212> DNA
   <213> Oryctolagus cuniculus
<400> 373
   ggtgcggcca ctctggcatc t 21
<210> 374
   <211> 27
   <212> DNA
   <213> Oryctolagus cuniculus
<400> 374
   gcaggcgctt atagggatgt ggattct 27
<210> 375
   <211> 18
   <212> DNA
   <213> Oryctolagus cuniculus
<400> 375
   agtacctact acatctac 18
<210> 376
   <211> 54
   <212> DNA
   <213> Oryctolagus cuniculus
<400> 376
   tgtattgatg ctggtagtag tggtagcact tactacgcga cctgggtgaa tggc 54
<210> 377
   <211> 39
   <212> DNA
   <213> Oryctolagus cuniculus
<400> 377
   tgggattatg gtggtaatgt tggttggggt tatgacttg 39
<210> 378
   <211> 120
   <212> PRT
   <213> Oryctolagus cuniculus
<400> 378
   Met Asp Thr Arg Ala Pro Thr Gln Leu Leu Gly Leu Leu Leu Leu Trp
   1               5                   10                  15
   Leu Pro Gly Ala Arg Cys Ala Phe Glu Leu Thr Gln Thr Pro Ser Ser
               20                  25                  30
   Val Glu Ala Ala Val Gly Gly Thr Val Thr Ile Lys Cys Gln Ala Ser
           35                  40                  45
   Gln Ser Ile Ser Ser Tyr Leu Ala Trp Tyr Gln Gln Lys Pro Gly Gln
       50                  55                  60
   Pro Pro Lys Phe Leu Ile Tyr Arg Ala Ser Thr Leu Ala Ser Gly Val
   65                  70                  75                  80
   Pro Ser Arg Phe Lys Gly Ser Gly Ser Gly Thr Glu Phe Thr Leu Thr
                   85                  90                  95
   Ile Ser Asp Leu Glu Cys Ala Asp Ala Ala Thr Tyr Tyr Cys Gln Ser
               100                 105                 110
   Tyr Tyr Asp Ser Val Ser Asn Pro
           115                 120
<210> 379
   <211> 127
   <212> PRT
   <213> Oryctolagus cuniculus
<400> 379
   Met Glu Thr Gly Leu Arg Trp Leu Leu Leu Val Ala Val Leu Lys Gly
   1               5                   10                  15
   Val Gln Cys Gln Ser Leu Glu Glu Ser Gly Gly Asp Leu Val Lys Pro
               20                  25                  30
   Glu Gly Ser Leu Thr Leu Thr Cys Lys Ala Ser Gly Leu Asp Leu Gly
           35                  40                  45
   Thr Tyr Trp Phe Met Cys Trp Val Arg Gln Ala Pro Gly Lys Gly Leu
       50                  55                  60
   Glu Trp Ile Ala Cys Ile Tyr Thr Gly Ser Ser Gly Ser Thr Phe Tyr
   65                  70                  75                  80
   Ala Ser Trp Val Asn Gly Arg Phe Thr Ile Ser Lys Thr Ser Ser Thr
                   85                  90                  95
   Thr Val Thr Leu Gln Met Thr Ser Leu Thr Ala Ala Asp Thr Ala Thr
               100                 105                 110
   Tyr Phe Cys Ala Arg Gly Tyr Ser Gly Tyr Gly Tyr Phe Lys Leu
           115                 120                 125
<210> 380
   <211> 11
   <212> PRT
   <213> Oryctolagus cuniculus
<400> 380
   Gln Ala Ser Gln Ser Ile Ser Ser Tyr Leu Ala
   1               5                   10
<210> 381
   <211> 7
   <212> PRT
   <213> Oryctolagus cuniculus
<400> 381
   Arg Ala Ser Thr Leu Ala Ser
   1               5
<210> 382
   <211> 10
   <212> PRT
   <213> Oryctolagus cuniculus
<400> 382
   Gln Ser Tyr Tyr Asp Ser Val Ser Asn Pro
   1 5 10
<210> 383
   <211> 6
   <212> PRT
   <213> Oryctolagus cuniculus
<400> 383
   Thr Tyr Trp Phe Met Cys
   1 5
<210> 384
   <211> 18
   <212> PRT
   <213> Oryctolagus cuniculus
<400> 384
   Cys Ile Tyr Thr Gly Ser Ser Gly Ser Thr Phe Tyr Ala Ser Trp Val
   1 5 10 15
   Asn Gly
<210> 385
   <211> 10
   <212> PRT
   <213> Oryctolagus cuniculus
<400> 385
   Gly Tyr Ser Gly Tyr Gly Tyr Phe Lys Leu
   1 5 10
<210> 386
   <211> 360
   <212> DNA
   <213> Oryctolagus cuniculus
<400> 386
   atggacacga gggcccccac tcagctgctg gggctcctgc tgctctggct cccaggtgcc 60
   agatgtgcat tcgaattgac ccagactcca tcctccgtgg aggcagctgt gggaggcaca 120
   gtcaccatca agtgccaggc cagtcagagc attagtagtt acttagcctg gtatcagcag 180
   aaaccagggc agcctcccaa gttcctgatc tacagggcgt ccactctggc atctggggtc 240
   ccatcgcgat tcaaaggcag tggatctggg acagagttca ctctcaccat cagcgacctg 300
   gagtgtgccg atgctgccac ttactactgt caaagctatt atgatagtgt ttcaaatcct 360
<210> 387
   <211> 381
   <212> DNA
   <213> Oryctolagus cuniculus
<400> 387
   atggagactg ggctgcgctg gcttctcctg gtcgctgtgc tcaaaggtgt ccagtgtcag      60
   tcgttggagg agtccggggg agacctggtc aagcctgagg gatccctgac actcacctgc     120
   aaagcctctg gactcgacct cggtacctac tggttcatgt gctgggtccg ccaggctcca     180
   gggaaggggc tggagtggat cgcttgtatt tatactggta gtagtggttc cactttctac     240
   gcgagctggg tgaatggccg attcaccatc tccaaaacct cgtcgaccac ggtgactctg     300
   caaatgacca gtctgacagc cgcggacacg gccacttatt tttgtgcgag aggttatagt     360
   ggttatggtt attttaagtt g                                               381
<210> 388
   <211> 33
   <212> DNA
   <213> Oryctolagus cuniculus
<400> 388
   caggccagtc agagcattag tagttactta gcc 33
<210> 389
   <211> 21
   <212> DNA
   <213> Oryctolagus cuniculus
<400> 389
   agggcgtcca ctctggcatc t 21
<210> 390
   <211> 30
   <212> DNA
   <213> Oryctolagus cuniculus
<400> 390
   caaagctatt atgatagtgt ttcaaatcct 30
<210> 391
   <211> 18
   <212> DNA
   <213> Oryctolagus cuniculus
<400> 391
   acctactggt tcatgtgc 18
<210> 392
   <211> 54
   <212> DNA
   <213> Oryctolagus cuniculus
<400> 392
   tgtatttata ctggtagtag tggttccact ttctacgcga gctgggtgaa tggc 54
<210> 393
   <211> 30
   <212> DNA
   <213> Oryctolagus cuniculus
<400> 393
   ggttatagtg gttatggtta ttttaagttg 30
<210> 394
   <211> 124
   <212> PRT
   <213> Oryctolagus cuniculus
<400> 394
   Met Asp Thr Arg Ala Pro Thr Gln Leu Leu Gly Leu Leu Leu Leu Trp
   1               5                   10                  15
   Leu Pro Gly Val Thr Phe Ala Ile Glu Met Thr Gln Ser Pro Phe Ser
               20                  25                  30
   Val Ser Ala Ala Val Gly Gly Thr Val Ser Ile Ser Cys Gln Ala Ser
           35                  40                  45
   Gln Ser Val Tyr Lys Asn Asn Gln Leu Ser Trp Tyr Gln Gln Lys Ser
       50                  55                  60
   Gly Gln Pro Pro Lys Leu Leu Ile Tyr Gly Ala Ser Ala Leu Ala Ser
   65                  70                  75                  80
   Gly Val Pro Ser Arg Phe Lys Gly Ser Gly Ser Gly Thr Glu Phe Thr
                   85                  90                  95
   Leu Thr Ile Ser Asp Val Gln Cys Asp Asp Ala Ala Thr Tyr Tyr Cys
               100                 105                 110
   Ala Gly Ala Ile Thr Gly Ser Ile Asp Thr Asp Gly
           115                 120
<210> 395
   <211> 130
   <212> PRT
   <213> Oryctolagus cuniculus
<400> 395
   Met Glu Thr Gly Leu Arg Trp Leu Leu Leu Val Ala Val Leu Lys Gly
   1               5                   10                  15
   Val Gln Cys Gln Ser Leu Glu Glu Ser Gly Gly Asp Leu Val Lys Pro
               20                  25                  30
   Gly Ala Ser Leu Thr Leu Thr Cys Thr Thr Ser Gly Phe Ser Phe Ser
           35                  40                  45
   Ser Ser Tyr Phe Ile Cys Trp Val Arg Gln Ala Pro Gly Lys Gly Leu
       50                  55                  60
   Glu Trp Ile Ala Cys Ile Tyr Gly Gly Asp Gly Ser Thr Tyr Tyr Ala
   65                  70                  75                  80
   Ser Trp Ala Lys Gly Arg Phe Thr Ile Ser Lys Thr Ser Ser Thr Thr
                   85                  90                  95
   Val Thr Leu Gln Met Thr Ser Leu Thr Ala Ala Asp Thr Ala Thr Tyr
               100                 105                 110
   Phe Cys Ala Arg Glu Trp Ala Tyr Ser Gln Gly Tyr Phe Gly Ala Phe
           115                 120                 125
   Asp Leu
       130
<210> 396
   <211> 13
   <212> PRT
   <213> Oryctolagus cuniculus
<400> 396
   Gln Ala Ser Gln Ser Val Tyr Lys Asn Asn Gln Leu Ser
   1               5                   10
<210> 397
   <211> 7
   <212> PRT
   <213> Oryctolagus cuniculus
<400> 397
   Gly Ala Ser Ala Leu Ala Ser
   1               5
<210> 398
   <211> 12
   <212> PRT
   <213> Oryctolagus cuniculus
<400> 398
   Ala Gly Ala Ile Thr Gly Ser Ile Asp Thr Asp Gly
   1 5 10
<210> 399
   <211> 6
   <212> PRT
   <213> Oryctolagus cuniculus
<400> 399
   Ser Ser Tyr Phe Ile Cys
   1 5
<210> 400
   <211> 17
   <212> PRT
   <213> Oryctolagus cuniculus
<400> 400
   Cys Ile Tyr Gly Gly Asp Gly Ser Thr Tyr Tyr Ala Ser Trp Ala Lys
   1 5 10 15
   Gly
<210> 401
   <211> 14
   <212> PRT
   <213> Oryctolagus cuniculus
<400> 401
   Glu Trp Ala Tyr Ser Gln Gly Tyr Phe Gly Ala Phe Asp Leu
   1 5 10
<210> 402
   <211> 372
   <212> DNA
   <213> Oryctolagus cuniculus
<400> 402
   atggacacga gggcccccac tcagctgctg gggctcctgc tgctctggct cccaggtgtc 60
   acatttgcca tcgaaatgac ccagagtcca ttctccgtgt ctgcagctgt gggaggcaca 120
   gtcagcatca gttgccaggc cagtcagagt gtttataaga acaaccaatt atcctggtat 180
   cagcagaaat cagggcagcc tcccaagctc ctgatctatg gtgcatcggc tctggcatct 240
   ggggtcccat cgcggttcaa aggcagtgga tctgggacag agttcactct caccatcagc 300
   gacgtgcagt gtgacgatgc tgccacttac tactgtgcag gcgctattac tggtagtatt 360
   gatacggatg gt 372
<210> 403
   <211> 390
   <212> DNA
   <213> Oryctolagus cuniculus
<400> 403
   atggagactg ggctgcgctg gcttctcctg gtcgctgtgc tcaaaggtgt ccagtgtcag 60
   tcgttggagg agtccggggg agacctggtc aagcctgggg catccctgac actcacctgc 120
   acaacttctg gattctcctt cagtagcagc tacttcattt gctgggtccg ccaggctcca 180
   gggaaggggc tggagtggat cgcatgcatt tatggtggtg atggcagcac atactacgcg 240
   agctgggcga aaggccgatt caccatctcc aaaacctcgt cgaccacggt gacgctgcaa 300
   atgaccagtc tgacagccgc ggacacggcc acctatttct gtgcgagaga atgggcatat 360
   agtcaaggtt attttggtgc ttttgatctc 390
<210> 404
   <211> 39
   <212> DNA
   <213> Oryctolagus cuniculus
<400> 404
   caggccagtc agagtgttta taagaacaac caattatcc 39
<210> 405
   <211> 21
   <212> DNA
   <213> Oryctolagus cuniculus
<400> 405
   ggtgcatcgg ctctggcatc t 21
<210> 406
   <211> 36
   <212> DNA
   <213> Oryctolagus cuniculus
<400> 406
   gcaggcgcta ttactggtag tattgatacg gatggt 36
<210> 407
   <211> 18
   <212> DNA
   <213> Oryctolagus cuniculus
<400> 407
   agcagctact tcatttgc 18
<210> 408
   <211> 51
   <212> DNA
   <213> Oryctolagus cuniculus
<400> 408
   tgcatttatg gtggtgatgg cagcacatac tacgcgagct gggcgaaagg c 51
<210> 409
   <211> 42
   <212> DNA
   <213> Oryctolagus cuniculus
<400> 409
   gaatgggcat atagtcaagg ttattttggt gcttttgatc tc 42
<210> 410
   <211> 124 <212> PRT
   <213> Oryctolagus cuniculus
<400> 410
   Met Asp Thr Arg Ala Pro Thr Gln Leu Leu Gly Leu Leu Leu Leu Trp
   1               5                   10                  15
   Leu Pro Gly Ala Arg Cys Asp Val Val Met Thr Gln Thr Pro Ala Ser
               20                  25                  30
   Val Glu Ala Ala Val Gly Gly Thr Val Thr Ile Lys Cys Gln Ala Ser
           35                  40                  45
   Glu Asp Ile Ser Ser Tyr Leu Ala Trp Tyr Gln Gln Lys Pro Gly Gln
       50                  55                  60
   Pro Pro Lys Leu Leu Ile Tyr Ala Ala Ser Asn Leu Glu Ser Gly Val
   65                  70                  75                  80
   Ser Ser Arg Phe Lys Gly Ser Gly Ser Gly Thr Glu Tyr Thr Leu Thr
                   85                  90                  95
   Ile Ser Asp Leu Glu Cys Ala Asp Ala Ala Thr Tyr Tyr Cys Gln Cys
               100                 105                 110
   Thr Tyr Gly Thr Ile Ser Ile Ser Asp Gly Asn Ala
           115                 120
<210> 411
   <211> 124
   <212> PRT
   <213> Oryctolagus cuniculus
<400> 411
   Met Glu Thr Gly Leu Arg Trp Leu Leu Leu Val Ala Val Leu Lys Gly
   1               5                   10                  15
   Val Gln Cys Gln Ser Val Glu Glu Ser Gly Gly Arg Leu Val Thr Pro
               20                  25                  30
   Gly Thr Pro Leu Thr Leu Thr Cys Thr Val Ser Gly Phe Ser Leu Ser
           35                  40                  45
   Ser Tyr Phe Met Thr Trp Val Arg Gln Ala Pro Gly Glu Gly Leu Glu
       50                  55                  60
   Tyr Ile Gly Phe Ile Asn Pro Gly Gly Ser Ala Tyr Tyr Ala Ser Trp
   65                  70                  75                  80
   Val Lys Gly Arg Phe Thr Ile Ser Lys Ser Ser Thr Thr Val Asp Leu
                   85                  90                  95
   Lys Ile Thr Ser Pro Thr Thr Glu Asp Thr Ala Thr Tyr Phe Cys Ala
               100                 105                 110
   Arg Val Leu Ile Val Ser Tyr Gly Ala Phe Thr Ile
           115                 120
<210> 412
   <211> 11
   <212> PRT
   <213> Oryctolagus cuniculus
<400> 412
   Gln Ala Ser Glu Asp Ile Ser Ser Tyr Leu Ala
   1               5                   10
<210> 413
   <211> 7
   <212> PRT
   <213> Oryctolagus cuniculus
<400> 413
   Ala Ala Ser Asn Leu Glu Ser
   1               5
<210> 414
   <211> 14
   <212> PRT
   <213> Oryctolagus cuniculus
<400> 414
   Gln Cys Thr Tyr Gly Thr Ile Ser Ile Ser Asp Gly Asn Ala
   1               5                   10
<210> 415
   <211> 5
   <212> PRT
   <213> Oryctolagus cuniculus
<400> 415
   Ser Tyr Phe Met Thr
   1               5
<210> 416
   <211> 16
   <212> PRT
   <213> Oryctolagus cuniculus
<400> 416
   Phe Ile Asn Pro Gly Gly Ser Ala Tyr Tyr Ala Ser Trp Val Lys Gly
   1               5                   10                  15
<210> 417
   <211> 11
   <212> PRT
   <213> Oryctolagus cuniculus
<400> 417
   Val Leu Ile Val Ser Tyr Gly Ala Phe Thr Ile
   1               5                   10
<210> 418
   <211> 372
   <212> DNA
   <213> Oryctolagus cuniculus
<400> 418
   atggacacga gggcccccac tcagctgctg gggctcctgc tgctctggct cccaggtgcc      60
   agatgtgatg ttgtgatgac ccagactcca gcctccgtgg aggcagctgt gggaggcaca     120
   gtcaccatca agtgccaggc cagtgaggat attagtagct acttagcctg gtatcagcag     180
   aaaccagggc agcctcccaa gctcctgatc tatgctgcat ccaatctgga atctggggtc     240
   tcatcgcgat tcaaaggcag tggatctggg acagagtaca ctctcaccat cagcgacctg     300
   gagtgtgccg atgctgccac ctattactgt caatgtactt atggtactat ttctattagt     360
   gatggtaatg ct                                                         372
<210> 419
   <211> 372
   <212> DNA
   <213> Oryctolagus cuniculus
<400> 419
   atggagactg ggctgcgctg gcttctcctg gtcgctgtgc tcaaaggtgt ccaatgtcag 60
   tcggtggagg agtccggggg tcgcctggtc acgcctggga cacccctgac actcacctgc 120
   acagtctctg gattctccct cagtagctac ttcatgacct gggtccgcca ggctccaggg 180
   gaggggctgg aatacatcgg attcattaat cctggtggta gcgcttacta cgcgagctgg 240
   gtgaaaggcc gattcaccat ctccaagtcc tcgaccacgg tagatctgaa aatcaccagt 300
   ccgacaaccg aggacacggc cacctatttc tgtgccaggg ttctgattgt ttcttatgga 360
   gcctttacca tc 372
<210> 420
   <211> 33
   <212> DNA
   <213> Oryctolagus cuniculus
<400> 420
   caggccagtg aggatattag tagctactta gcc 33
<210> 421
   <211> 21
   <212> DNA
   <213> Oryctolagus cuniculus
<400> 421
   gctgcatcca atctggaatc t 21
<210> 422
   <211> 42
   <212> DNA
   <213> Oryctolagus cuniculus
<400> 422
   caatgtactt atggtactat ttctattagt gatggtaatg ct 42
<210> 423
   <211> 15
   <212> DNA
   <213> Oryctolagus cuniculus
<400> 423
   agctacttca tgacc 15
<210> 424
   <211> 48
   <212> DNA
   <213> Oryctolagus cuniculus
<400> 424
   ttcattaatc ctggtggtag cgcttactac gcgagctggg tgaaaggc 48
<210> 425
   <211> 33
   <212> DNA
   <213> Oryctolagus cuniculus
<400> 425
   gttctgattg tttcttatgg agcctttacc atc 33
<210> 426
   <211> 124
   <212> PRT
   <213> Oryctolagus cuniculus
<400> 426
   Met Asp Thr Arg Ala Pro Thr Gln Leu Leu Gly Leu Leu Leu Leu Trp
   1               5                   10                  15
   Leu Pro Gly Ala Arg Cys Asp Val Val Met Thr Gln Thr Pro Ala Ser
               20                  25                  30
   Val Ser Ala Ala Val Gly Gly Thr Val Thr Ile Lys Cys Gln Ala Ser
           35                  40                  45
   Glu Asp Ile Glu Ser Tyr Leu Ala Trp Tyr Gln Gln Lys Pro Gly Gln
       50                  55                  60
   Pro Pro Lys Leu Leu Ile Tyr Gly Ala Ser Asn Leu Glu Ser Gly Val
   65                  70                  75                  80
   Ser Ser Arg Phe Lys Gly Ser Gly Ser Gly Thr Glu Phe Thr Leu Thr
                   85                  90                  95
   Ile Ser Asp Leu Glu Cys Ala Asp Ala Ala Thr Tyr Tyr Cys Gln Cys
               100                 105                 110
   Thr Tyr Gly Ile Ile Ser Ile Ser Asp Gly Asn Ala
           115                 120
<210> 427
   <211> 124
   <212> PRT
   <213> Oryctolagus cuniculus
<400> 427
   Met Glu Thr Gly Leu Arg Trp Leu Leu Leu Val Ala Val Leu Lys Gly
   1               5                   10                  15
   Val Gln Cys Gln Ser Val Glu Glu Ser Gly Gly Arg Leu Val Thr Pro
               20                  25                  30
   Gly Thr Pro Leu Thr Leu Thr Cys Thr Val Ser Gly Phe Ser Leu Ser
           35                  40                  45
   Ser Tyr Phe Met Thr Trp Val Arg Gln Ala Pro Gly Glu Gly Leu Glu
       50                  55                  60
   Tyr Ile Gly Phe Met Asn Thr Gly Asp Asn Ala Tyr Tyr Ala Ser Trp
   65                  70                  75                  80
   Ala Lys Gly Arg Phe Thr Ile Ser Lys Thr Ser Thr Thr Val Asp Leu
                   85                  90                  95
   Lys Ile Thr Ser Pro Thr Thr Glu Asp Thr Ala Thr Tyr Phe Cys Ala
               100                 105                 110
   Arg Val Leu Val Val Ala Tyr Gly Ala Phe Asn Ile
           115                 120
<210> 428
   <211> 11
   <212> PRT
   <213> Oryctolagus cuniculus
<400> 428
   Gln Ala Ser Glu Asp Ile Glu Ser Tyr Leu Ala
   1               5                   10
<210> 429
   <211> 7
   <212> PRT
   <213> Oryctolagus cuniculus
<400> 429
   Gly Ala Ser Asn Leu Glu Ser
   1               5
<210> 430
   <211> 14
   <212> PRT
   <213> Oryctolagus cuniculus
<400> 430
   Gln Cys Thr Tyr Gly Ile Ile Ser Ile Ser Asp Gly Asn Ala
   1 5 10
<210> 431
   <211> 5
   <212> PRT
   <213> Oryctolagus cuniculus
<400> 431
   Ser Tyr Phe Met Thr
   1 5
<210> 432
   <211> 16
   <212> PRT
   <213> Oryctolagus cuniculus
<400> 432
   Phe Met Asn Thr Gly Asp Asn Ala Tyr Tyr Ala Ser Trp Ala Lys Gly
   1 5 10 15
<210> 433
   <211> 11
   <212> PRT
   <213> Oryctolagus cuniculus
<400> 433
   Val Leu Val Val Ala Tyr Gly Ala Phe Asn Ile
   1 5 10
<210> 434
   <211> 372
   <212> DNA
   <213> Oryctolagus cuniculus
<400> 434
   atggacacga gggcccccac tcagctgctg gggctcctgc tgctctggct cccaggtgcc 60
   agatgtgatg ttgtgatgac ccagactcca gcctccgtgt ctgcagctgt gggaggcaca 120
   gtcaccatca agtgccaggc cagtgaggac attgaaagct atctagcctg gtatcagcag 180
   aaaccagggc agcctcccaa gctcctgatc tatggtgcat ccaatctgga atctggggtc 240
   tcatcgcggt tcaaaggcag tggatctggg acagagttca ctctcaccat cagcgacctg 300
   gagtgtgccg atgctgccac ttactattgt caatgcactt atggtattat tagtattagt 360
   gatggtaatg ct 372
<210> 435
   <211> 372
   <212> DNA
   <213> Oryctolagus cuniculus
<400> 435
   atggagactg ggctgcgctg gcttctcctg gtcgctgtgc tcaaaggtgt ccagtgtcag 60
   tcggtggagg agtccggggg tcgcctggtc acgcctggga cacccctgac actcacctgc 120
   acagtgtctg gattctccct cagtagctac ttcatgacct gggtccgcca ggctccaggg 180
   gaggggctgg aatacatcgg attcatgaat actggtgata acgcatacta cgcgagctgg 240
   gcgaaaggcc gattcaccat ctccaaaacc tcgaccacgg tggatctgaa aatcaccagt 300
   ccgacaaccg aggacacggc cacctatttc tgtgccaggg ttcttgttgt tgcttatgga 360
   gcctttaaca tc 372
<210> 436
   <211> 33
   <212> DNA
   <213> Oryctolagus cuniculus
<400> 436
   caggccagtg aggacattga aagctatcta gcc 33
<210> 437
   <211> 21
   <212> DNA
   <213> Oryctolagus cuniculus
<400> 437
   ggtgcatcca atctggaatc t 21
<210> 438
   <211> 42
   <212> DNA
   <213> Oryctolagus cuniculus
<400> 438
   caatgcactt atggtattat tagtattagt gatggtaatg ct 42
<210> 439
   <211> 15
   <212> DNA
   <213> Oryctolagus cuniculus
<400> 439
   agctacttca tgacc 15
<210> 440
   <211> 48
   <212> DNA
   <213> Oryctolagus cuniculus
<400> 440
   ttcatgaata ctggtgataa cgcatactac gcgagctggg cgaaaggc 48
<210> 441
   <211> 33
   <212> DNA
   <213> Oryctolagus cuniculus
<400> 441
   gttcttgttg ttgcttatgg agcctttaac atc 33
<210> 442
   <211> 124
   <212> PRT
   <213> Oryctolagus cuniculus
<400> 442
   Met Asp Thr Arg Ala Pro Thr Gln Leu Leu Gly Leu Leu Leu Leu Trp
   1               5                   10                  15
   Leu Pro Gly Ala Thr Phe Ala Ala Val Leu Thr Gln Thr Pro Ser Pro
               20                  25                  30
   Val Ser Glu Pro Val Gly Gly Thr Val Ser Ile Ser Cys Gln Ser Ser
           35                  40                  45
   Lys Ser Val Met Asn Asn Asn Tyr Leu Ala Trp Tyr Gln Gln Lys Pro
       50                  55                  60
   Gly Gln Pro Pro Lys Leu Leu Ile Tyr Gly Ala Ser Asn Leu Ala Ser
   65                  70                  75                  80
   Gly Val Pro Ser Arg Phe Ser Gly Ser Gly Ser Gly Thr Gln Phe Thr
                   85                  90                  95
   Leu Thr Ile Ser Asp Val Gln Cys Asp Asp Ala Ala Thr Tyr Tyr Cys
               100                 105                 110
   Gln Gly Gly Tyr Thr Gly Tyr Ser Asp His Gly Thr
           115                 120
<210> 443
   <211> 127
   <212> PRT
   <213> Oryctolagus cuniculus
<400> 443
   Met Glu Thr Gly Leu Arg Trp Leu Leu Leu Val Ala Val Leu Lys Gly
   1               5                   10                  15
   Val Gln Cys Gln Ser Val Glu Glu Ser Gly Gly Arg Leu Val Lys Pro
               20                  25                  30
   Asp Glu Thr Leu Thr Leu Thr Cys Thr Val Ser Gly Ile Asp Leu Ser
           35                  40                  45
   Ser Tyr Pro Met Asn Trp Val Arg Gln Ala Pro Gly Lys Gly Leu Glu
       50                  55                  60
   Trp Ile Gly Phe Ile Asn Thr Gly Gly Thr Ile Val Tyr Ala Ser Trp
   65                  70                  75                  80
   Ala Lys Gly Arg Phe Thr Ile Ser Lys Thr Ser Thr Thr Val Asp Leu
                   85                  90                  95
   Lys Met Thr Ser Pro Thr Thr Glu Asp Thr Ala Thr Tyr Phe Cys Ala
               100                 105                 110
   Arg Gly Ser Tyr Val Ser Ser Gly Tyr Ala Tyr Tyr Phe Asn Val
           115                 120                 125
<210> 444
   <211> 13
   <212> PRT
   <213> Oryctolagus cuniculus
<400> 444
   Gln Ser Ser Lys Ser Val Met Asn Asn Asn Tyr Leu Ala
   1               5                   10
<210> 445
   <211> 7
   <212> PRT
   <213> Oryctolagus cuniculus
<400> 445
   Gly Ala Ser Asn Leu Ala Ser
   1               5
<210> 446
   <211> 12
   <212> PRT
   <213> Oryctolagus cuniculus
<400> 446
   Gln Gly Gly Tyr Thr Gly Tyr Ser Asp His Gly Thr
   1 5 10
<210> 447
   <211> 5
   <212> PRT
   <213> Oryctolagus cuniculus
<400> 447
   Ser Tyr Pro Met Asn
   1 5
<210> 448
   <211> 16
   <212> PRT
   <213> Oryctolagus cuniculus
<400> 448
   Phe Ile Asn Thr Gly Gly Thr Ile Val Tyr Ala Ser Trp Ala Lys Gly
   1 5 10 15
<210> 449
   <211> 14
   <212> PRT
   <213> Oryctolagus cuniculus
<400> 449
   Gly Ser Tyr Val Ser Ser Gly Tyr Ala Tyr Tyr Phe Asn Val
   1 5 10
<210> 450
   <211> 372
   <212> DNA
   <213> Oryctolagus cuniculus
<400> 450
   atggacacga gggcccccac tcagctgctg gggctcctgc tgctctggct cccaggtgcc 60
   acatttgccg ccgtgctgac ccagactcca tctcccgtgt ctgaacctgt gggaggcaca 120
   gtcagcatca gttgccagtc cagtaagagt gttatgaata acaactactt agcctggtat 180
   cagcagaaac cagggcagcc tcccaagctc ctgatctatg gtgcatccaa tctggcatct 240
   ggggtcccat cacggttcag cggcagtgga tctgggacac agttcactct caccatcagc 300
   gacgtgcagt gtgacgatgc tgccacttac tactgtcaag gcggttatac tggttatagt 360
   gatcatggga ct 372
<210> 451
   <211> 381
   <212> DNA
   <213> Oryctolagus cuniculus
<400> 451
   atggagactg ggctgcgctg gcttctcctg gtcgctgtgc tcaaaggtgt ccagtgtcag        60
   tcggtggagg agtccggggg tcgcctggtc aagcctgacg aaaccctgac actcacctgc       120
   acagtctctg gaatcgacct cagtagctat ccaatgaact gggtccgcca ggctccaggg       180
   aaggggctgg aatggatcgg attcattaat actggtggta ccatagtcta cgcgagctgg       240
   gcaaaaggcc gattcaccat ctccaaaacc tcgaccacgg tggatctgaa aatgaccagt       300
   ccgacaaccg aggacacggc cacctatttc tgtgccagag gcagttatgt ttcatctggt       360
   tatgcctact attttaatgt c                                                 381
<210> 452
   <211> 39
   <212> DNA
   <213> Oryctolagus cuniculus
<400> 452
   cagtccagta agagtgttat gaataacaac tacttagcc 39
<210> 453
   <211> 21
   <212> DNA
   <213> Oryctolagus cuniculus
<400> 453
   ggtgcatcca atctggcatc t 21
<210> 454
   <211> 36
   <212> DNA
   <213> Oryctolagus cuniculus
<400> 454
   caaggcggtt atactggtta tagtgatcat gggact 36
<210> 455
   <211> 15
   <212> DNA
   <213> Oryctolagus cuniculus
<400> 455
   agctatccaa tgaac 15
<210> 456
   <211> 48
   <212> DNA
   <213> Oryctolagus cuniculus
<400> 456
   ttcattaata ctggtggtac catagtctac gcgagctggg caaaaggc 48
<210> 457
   <211> 42
   <212> DNA
   <213> Oryctolagus cuniculus
<400> 457
   ggcagttatg tttcatctgg ttatgcctac tattttaatg tc 42
<210> 458
   <211> 121
   <212> PRT
   <213> Oryctolagus cuniculus
<400> 458
   Met Asp Thr Arg Ala Pro Thr Gln Leu Leu Gly Leu Leu Leu Leu Trp
   1               5                   10                  15
   Leu Pro Gly Ala Thr Phe Ala Ala Val Leu Thr Gln Thr Pro Ser Pro
               20                  25                  30
   Val Ser Ala Ala Val Gly Gly Thr Val Ser Ile Ser Cys Gln Ser Ser
           35                  40                  45
   Gln Ser Val Tyr Asn Asn Asn Trp Leu Ser Trp Phe Gln Gln Lys Pro
       50                  55                  60
   Gly Gln Pro Pro Lys Leu Leu Ile Tyr Lys Ala Ser Thr Leu Ala Ser
   65                  70                  75                  80
   Gly Val Pro Ser Arg Phe Lys Gly Ser Gly Ser Gly Thr Gln Phe Thr
                   85                  90                  95
   Leu Thr Ile Ser Asp Val Gln Cys Asp Asp Val Ala Thr Tyr Tyr Cys
               100                 105                 110
   Ala Gly Gly Tyr Leu Asp Ser Val Ile
           115                 120
<210> 459
   <211> 126
   <212> PRT
   <213> Oryctolagus cuniculus
<400> 459
   Met Glu Thr Gly Leu Arg Trp Leu Leu Leu Val Ala Val Leu Lys Gly
   1               5                   10                  15
   Val Gln Cys Gln Ser Val Glu Glu Ser Gly Gly Arg Leu Val Thr Pro
               20                  25                  30
   Gly Thr Pro Leu Thr Leu Thr Cys Thr Val Ser Gly Phe Ser Leu Ser
           35                  40                  45
   Thr Tyr Ser Ile Asn Trp Val Arg Gln Ala Pro Gly Lys Gly Leu Glu
       50                  55                  60
   Trp Ile Gly Ile Ile Ala Asn Ser Gly Thr Thr Phe Tyr Ala Asn Trp
   65                  70                  75                  80
   Ala Lys Gly Arg Phe Thr Val Ser Lys Thr Ser Thr Thr Val Asp Leu
                   85                  90                  95
   Lys Ile Thr Ser Pro Thr Thr Glu Asp Thr Ala Thr Tyr Phe Cys Ala
               100                 105                 110
   Arg Glu Ser Gly Met Tyr Asn Glu Tyr Gly Lys Phe Asn Ile
           115                 120                 125
<210> 460
   <211> 13
   <212> PRT
   <213> Oryctolagus cuniculus
<400> 460
   Gln Ser Ser Gln Ser Val Tyr Asn Asn Asn Trp Leu Ser
   1               5                   10
<210> 461
   <211> 7
   <212> PRT
   <213> Oryctolagus cuniculus
<400> 461
   Lys Ala Ser Thr Leu Ala Ser
   1               5
<210> 462
   <211> 9
   <212> PRT
   <213> Oryctolagus cuniculus
<400> 462
   Ala Gly Gly Tyr Leu Asp Ser Val Ile
   1               5
<210> 463
   <211> 5
   <212> PRT
   <213> Oryctolagus cuniculus
<400> 463
   Thr Tyr Ser Ile Asn
   1               5
<210> 464
   <211> 16
   <212> PRT
   <213> Oryctolagus cuniculus
<400> 464
   Ile Ile Ala Asn Ser Gly Thr Thr Phe Tyr Ala Asn Trp Ala Lys Gly
   1               5                   10                  15
<210> 465
   <211> 13
   <212> PRT
   <213> Oryctolagus cuniculus
<400> 465
   Glu Ser Gly Met Tyr Asn Glu Tyr Gly Lys Phe Asn Ile
   1               5                   10
<210> 466
   <211> 363
   <212> DNA
   <213> Oryctolagus cuniculus
<400> 466
   atggacacga gggcccccac tcagctgctg gggctcctgc tgctctggct cccaggtgcc 60
   acatttgccg ccgtgctgac ccagactcca tctcccgtgt ctgcagctgt gggaggcaca 120
   gtcagcatca gttgccagtc cagtcagagt gtttataata acaactggtt atcctggttt 180
   cagcagaaac cagggcagcc tcccaagctc ctgatctaca aggcatccac tctggcatct 240
   ggggtcccat cgcggttcaa aggcagtgga tctgggacac agttcactct caccatcagc 300
   gacgtgcagt gtgacgatgt tgccacttac tactgtgcgg gcggttatct tgatagtgtt 360
   att 363
<210> 467
   <211> 378
   <212> DNA
   <213> Oryctolagus cuniculus
<400> 467
   atggagactg ggctgcgctg gcttctcctg gtcgctgtgc tcaaaggtgt ccagtgtcag 60
   tcggtggagg agtccggggg tcgcctggtc acgcctggga cacccctgac actcacctgc 120
   acagtctctg gattctccct cagtacctat tcaataaact gggtccgcca ggctccaggg 180
   aagggcctgg aatggatcgg aatcattgct aatagtggta ccacattcta cgcgaactgg 240
   gcgaaaggcc gattcaccgt ctccaaaacc tcgaccacgg tggatctgaa aatcaccagt 300
   ccgacaaccg aggacacggc cacctatttc tgtgccagag agagtggaat gtacaatgaa 360
   tatggtaaat ttaacatc 378
<210> 468
   <211> 39
   <212> DNA
   <213> Oryctolagus cuniculus
<400> 468
   cagtccagtc agagtgttta taataacaac tggttatcc 39
<210> 469
   <211> 21
   <212> DNA
   <213> Oryctolagus cuniculus
<400> 469
   aaggcatcca ctctggcatc t 21
<210> 470
   <211> 27
   <212> DNA
   <213> Oryctolagus cuniculus
<400> 470
   gcgggcggtt atcttgatag tgttatt 27
<210> 471
   <211> 15
   <212> DNA
   <213> Oryctolagus cuniculus
<400> 471
   acctattcaa taaac 15
<210> 472
   <211> 48
   <212> DNA
   <213> Oryctolagus cuniculus
<400> 472
   atcattgcta atagtggtac cacattctac gcgaactggg cgaaaggc 48
<210> 473
   <211> 39
   <212> DNA
   <213> Oryctolagus cuniculus
<400> 473
   gagagtggaa tgtacaatga atatggtaaa tttaacatc 39
<210> 474
   <211> 122
   <212> PRT
   <213> Oryctolagus cuniculus
<400> 474
   Met Asp Thr Arg Ala Pro Thr Gln Leu Leu Gly Leu Leu Leu Leu Trp
   1               5                   10                  15
   Leu Pro Gly Ala Arg Cys Ala Ser Asp Met Thr Gln Thr Pro Ser Ser
               20                  25                  30
   Val Ser Ala Ala Val Gly Gly Thr Val Thr Ile Asn Cys Gln Ala Ser
           35                  40                  45
   Glu Asn Ile Tyr Ser Phe Leu Ala Trp Tyr Gln Gln Lys Pro Gly Gln
       50                  55                  60
   Pro Pro Lys Leu Leu Ile Phe Lys Ala Ser Thr Leu Ala Ser Gly Val
   65                  70                  75                  80
   Ser Ser Arg Phe Lys Gly Ser Gly Ser Gly Thr Gln Phe Thr Leu Thr
                   85                  90                  95
   Ile Ser Asp Leu Glu Cys Asp Asp Ala Ala Thr Tyr Tyr Cys Gln Gln
               100                 105                 110
   Gly Ala Thr Val Tyr Asp Ile Asp Asn Asn
           115                 120
<210> 475
   <211> 128
   <212> PRT
   <213> Oryctolagus cuniculus
<400> 475
   Met Glu Thr Gly Leu Arg Trp Leu Leu Leu Val Ala Val Leu Lys Gly
   1               5                   10                  15
   Val Gln Cys Gln Ser Leu Glu Glu Ser Gly Gly Arg Leu Val Thr Pro
   20 25 30
   Gly Thr Pro Leu Thr Leu Thr Cys Thr Val Ser Gly Ile Asp Leu Ser
   35 40 45
   Ala Tyr Ala Met Ile Trp Val Arg Gln Ala Pro Gly Glu Gly Leu Glu
   50 55 60
   Trp Ile Thr Ile Ile Tyr Pro Asn Gly Ile Thr Tyr Tyr Ala Asn Trp
   65 70 75 80
   Ala Lys Gly Arg Phe Thr Val Ser Lys Thr Ser Thr Ala Met Asp Leu
   85 90 95
   Lys Ile Thr Ser Pro Thr Thr Glu Asp Thr Ala Thr Tyr Phe Cys Ala
   100 105 110
   Arg Asp Ala Glu Ser Ser Lys Asn Ala Tyr Trp Gly Tyr Phe Asn Val
   115 120 125
<210> 476
   <211> 11
   <212> PRT
   <213> Oryctolagus cuniculus
<400> 476
   Gln Ala Ser Glu Asn Ile Tyr Ser Phe Leu Ala
   1 5 10
<210> 477
   <211> 7
   <212> PRT
   <213> Oryctolagus cuniculus
<400> 477
   Lys Ala Ser Thr Leu Ala Ser
   1 5
<210> 478
   <211> 12
   <212> PRT
   <213> Oryctolagus cuniculus
<400> 478
   Gln Gln Gly Ala Thr Val Tyr Asp Ile Asp Asn Asn
   1 5 10
<210> 479
   <211> 5
   <212> PRT
   <213> Oryctolagus cuniculus
<400> 479
   Ala Tyr Ala Met Ile
   1               5
<210> 480
   <211> 16
   <212> PRT
   <213> Oryctolagus cuniculus
<400> 480
   Ile Ile Tyr Pro Asn Gly Ile Thr Tyr Tyr Ala Asn Trp Ala Lys Gly
   1               5                   10                  15
<210> 481
   <211> 15
   <212> PRT
   <213> Oryctolagus cuniculus
<400> 481
   Asp Ala Glu Ser Ser Lys Asn Ala Tyr Trp Gly Tyr Phe Asn Val
   1               5                   10                  15
<210> 482
   <211> 366
   <212> DNA
   <213> Oryctolagus cuniculus
<400> 482
   atggacacga gggcccccac tcagctgctg gggctcctgc tgctctggct cccaggtgcc    60
   agatgtgcct ctgatatgac ccagactcca tcctccgtgt ctgcagctgt gggaggcaca   120
   gtcaccatca attgccaggc cagtgagaac atttatagct ttttggcctg gtatcagcag   180
   aaaccagggc agcctcccaa gctcctgatc ttcaaggctt ccactctggc atctggggtc   240
   tcatcgcggt tcaaaggcag tggatctggg acacagttca ctctcaccat cagcgacctg   300
   gagtgtgacg atgctgccac ttactactgt caacagggtg ctactgtgta tgatattgat   360
   aataat                                                              366
<210> 483
   <211> 384
   <212> DNA
   <213> Oryctolagus cuniculus
<400> 483
   atggagactg ggctgcgctg gcttctcctg gtcgctgtgc tcaaaggtgt ccagtgtcag 60
   tcgctggagg agtccggggg tcgcctggtc acgcctggga cacccctgac actcacctgc 120
   acagtttctg gaatcgacct cagtgcctat gcaatgatct gggtccgcca ggctccaggg 180
   gaggggctgg aatggatcac aatcatttat cctaatggta tcacatacta cgcgaactgg 240
   gcgaaaggcc gattcaccgt ctccaaaacc tcgaccgcga tggatctgaa aatcaccagt 300
   ccgacaaccg aggacacggc cacctatttc tgtgccagag atgcagaaag tagtaagaat 360
   gcttattggg gctactttaa cgtc 384
<210> 484
   <211> 33
   <212> DNA
   <213> Oryctolagus cuniculus
<400> 484
   caggccagtg agaacattta tagctttttg gcc 33
<210> 485
   <211> 21
   <212> DNA
   <213> Oryctolagus cuniculus
<400> 485
   aaggcttcca ctctggcatc t 21
<210> 486
   <211> 36
   <212> DNA
   <213> Oryctolagus cuniculus
<400> 486
   caacagggtg ctactgtgta tgatattgat aataat 36
<210> 487
   <211> 15
   <212> DNA
   <213> Oryctolagus cuniculus
<400> 487
   gcctatgcaa tgatc 15
<210> 488
   <211> 48
   <212> DNA
   <213> Oryctolagus cuniculus
<400> 488
   atcatttatc ctaatggtat cacatactac gcgaactggg cgaaaggc 48
<210> 489
   <211> 45
   <212> DNA
   <213> Oryctolagus cuniculus
<400> 489
   gatgcagaaa gtagtaagaa tgcttattgg ggctacttta acgtc 45
<210> 490
   <211> 122
   <212> PRT
   <213> Oryctolagus cuniculus
<400> 490
   Met Asp Thr Arg Ala Pro Thr Gln Leu Leu Gly Leu Leu Leu Leu Trp
   1               5                   10                  15
   Leu Pro Gly Ala Arg Cys Ala Ser Asp Met Thr Gln Thr Pro Ser Ser
               20                  25                  30
   Val Ser Ala Ala Val Gly Gly Thr Val Thr Ile Asn Cys Gln Ala Ser
           35                  40                  45
   Glu Asn Ile Tyr Ser Phe Leu Ala Trp Tyr Gln Gln Lys Pro Gly Gln
       50                  55                  60
   Pro Pro Lys Leu Leu Ile Phe Arg Ala Ser Thr Leu Ala Ser Gly Val
   65                  70                  75                  80
   Ser Ser Arg Phe Lys Gly Ser Gly Ser Gly Thr Gln Phe Thr Leu Thr
                   85                  90                  95
   Ile Ser Asp Leu Glu Cys Asp Asp Ala Ala Thr Tyr Tyr Cys Gln Gln
               100                 105                 110
   Gly Ala Thr Val Tyr Asp Ile Asp Asn Asn
           115                 120
<210> 491
   <211> 128
   <212> PRT
   <213> Oryctolagus cuniculus
<400> 491
   Met Glu Thr Gly Leu Arg Trp Leu Leu Leu Val Ala Val Leu Lys Gly
   1               5                   10                  15
   Val Gln Cys Gln Ser Leu Glu Glu Ser Gly Gly Arg Leu Val Thr Pro
               20                  25                  30
   Gly Thr Pro Leu Thr Leu Thr Cys Thr Val Ser Gly Ile Asp Leu Ser
           35                  40                  45
   Ala Tyr Ala Met Ile Trp Val Arg Gln Ala Pro Gly Glu Gly Leu Glu
       50                  55                  60
   Trp Ile Thr Ile Ile Tyr Pro Asn Gly Ile Thr Tyr Tyr Ala Asn Trp
   65                  70                  75                  80
   Ala Lys Gly Arg Phe Thr Val Ser Lys Thr Ser Thr Ala Met Asp Leu
                   85                  90                  95
   Lys Ile Thr Ser Pro Thr Thr Glu Asp Thr Ala Thr Tyr Phe Cys Ala
               100                 105                 110
   Arg Asp Ala Glu Ser Ser Lys Asn Ala Tyr Trp Gly Tyr Phe Asn Val
           115                 120                 125
<210> 492
   <211> 11
   <212> PRT
   <213> Oryctolagus cuniculus
<400> 492
   Gln Ala Ser Glu Asn Ile Tyr Ser Phe Leu Ala
   1               5                   10
<210> 493
   <211> 7
   <212> PRT
   <213> Oryctolagus cuniculus
<400> 493
   Arg Ala Ser Thr Leu Ala Ser
   1               5
<210> 494
   <211> 12
   <212> PRT
   <213> Oryctolagus cuniculus
<400> 494
   Gln Gln Gly Ala Thr Val Tyr Asp Ile Asp Asn Asn
   1 5 10
<210> 495
   <211> 5
   <212> PRT
   <213> Oryctolagus cuniculus
<400> 495
   Ala Tyr Ala Met Ile
   1 5
<210> 496
   <211> 16
   <212> PRT
   <213> Oryctolagus cuniculus
<400> 496
   Ile Ile Tyr Pro Asn Gly Ile Thr Tyr Tyr Ala Asn Trp Ala Lys Gly
   1 5 10 15
<210> 497
   <211> 15
   <212> PRT
   <213> Oryctolagus cuniculus
<400> 497
   Asp Ala Glu Ser Ser Lys Asn Ala Tyr Trp Gly Tyr Phe Asn Val
   1 5 10 15
<210> 498
   <211> 366
   <212> DNA
   <213> Oryctolagus cuniculus
<400> 498
   atggacacga gggcccccac tcagctgctg gggctcctgc tgctctggct cccaggtgcc 60
   agatgtgcct ctgatatgac ccagactcca tcctccgtgt ctgcagctgt gggaggcaca 120
   gtcaccatca attgccaggc cagtgagaac atttatagct ttttggcctg gtatcagcag 180
   aaaccagggc agcctcccaa gctcctgatc ttcagggctt ccactctggc atctggggtc 240
   tcatcgcggt tcaaaggcag tggatctggg acacagttca ctctcaccat cagcgacctg 300
   gagtgtgacg atgctgccac ttactactgt caacagggtg ctactgtgta tgatattgat 360
   aataat 366
<210> 499
   <211> 384
   <212> DNA
   <213> Oryctolagus cuniculus
<400> 499
   atggagactg ggctgcgctg gcttctcctg gtcgctgtgc tcaaaggtgt ccagtgtcag      60
   tcgctggagg agtccggggg tcgcctggtc acgcctggga cacccctgac actcacctgc     120
   acagtttctg gaatcgacct cagtgcctat gcaatgatct gggtccgcca ggctccaggg     180
   gaggggctgg aatggatcac aatcatttat cctaatggta tcacatacta cgcgaactgg     240
   gcgaaaggcc gattcaccgt ctccaaaacc tcgaccgcga tggatctgaa aatcaccagt     300
   ccgacaaccg aggacacggc cacctatttc tgtgccagag atgcagaaag tagtaagaat     360
   gcttattggg gctactttaa cgtc                                            384
<210> 500
   <211> 33
   <212> DNA
   <213> Oryctolagus cuniculus
<400> 500
   caggccagtg agaacattta tagctttttg gcc 33
<210> 501
   <211> 21
   <212> DNA
   <213> Oryctolagus cuniculus
<400> 501
   agggcttcca ctctggcatc t 21
<210> 502
   <211> 36
   <212> DNA
   <213> Oryctolagus cuniculus
<400> 502
   caacagggtg ctactgtgta tgatattgat aataat 36
<210> 503
   <211> 15
   <212> DNA
   <213> Oryctolagus cuniculus
<400> 503
   gcctatgcaa tgatc 15
<210> 504
   <211> 48
   <212> DNA
   <213> Oryctolagus cuniculus
<400> 504
   atcatttatc ctaatggtat cacatactac gcgaactggg cgaaaggc 48
<210> 505
   <211> 45
   <212> DNA
   <213> Oryctolagus cuniculus
<400> 505
   gatgcagaaa gtagtaagaa tgcttattgg ggctacttta acgtc 45
<210> 506
   <211> 124
   <212> PRT
   <213> Oryctolagus cuniculus
<400> 506
   Met Asp Thr Arg Ala Pro Thr Gln Leu Leu Gly Leu Leu Leu Leu Trp
   1               5                   10                  15
   Leu Pro Gly Ala Thr Phe Ala Ile Glu Met Thr Gln Thr Pro Ser Pro
               20                  25                  30
   Val Ser Ala Ala Val Gly Gly Thr Val Thr Ile Asn Cys Gln Ala Ser
           35                  40                  45
   Glu Ser Val Phe Asn Asn Met Leu Ser Trp Tyr Gln Gln Lys Pro Gly
       50                  55                  60
   His Ser Pro Lys Leu Leu Ile Tyr Asp Ala Ser Asp Leu Ala Ser Gly
   65                  70                  75                  80
   Val Pro Ser Arg Phe Lys Gly Ser Gly Ser Gly Thr Gln Phe Thr Leu
                   85                  90                  95
   Thr Ile Ser Gly Val Glu Cys Asp Asp Ala Ala Thr Tyr Tyr Cys Ala
               100                 105                 110
   Gly Tyr Lys Ser Asp Ser Asn Asp Gly Asp Asn Val
           115                 120
<210> 507
   <211> 123
   <212> PRT
   <213> Oryctolagus cuniculus
<400> 507
   Met Glu Thr Gly Leu Arg Trp Leu Leu Leu Val Ala Val Leu Lys Gly
   1               5                   10                  15
   Val Gln Cys Gln Ser Leu Glu Glu Ser Gly Gly Arg Leu Val Thr Pro
               20                  25                  30
   Gly Thr Pro Leu Thr Leu Thr Cys Thr Val Ser Gly Phe Ser Leu Asn
           35                  40                  45
   Arg Asn Ser Ile Thr Trp Val Arg Gln Ala Pro Gly Glu Gly Leu Glu
       50                  55                  60
   Trp Ile Gly Ile Ile Thr Gly Ser Gly Arg Thr Tyr Tyr Ala Asn Trp
   65                  70                  75                  80
   Ala Lys Gly Arg Phe Thr Ile Ser Lys Thr Ser Thr Thr Val Asp Leu
                   85                  90                  95
   Lys Met Thr Ser Pro Thr Thr Glu Asp Thr Ala Thr Tyr Phe Cys Ala
               100                 105                 110
   Arg Gly His Pro Gly Leu Gly Ser Gly Asn Ile
           115                 120
<210> 508
   <211> 12
   <212> PRT
   <213> Oryctolagus cuniculus
<400> 508
   Gln Ala Ser Glu Ser Val Phe Asn Asn Met Leu Ser
   1               5                   10
<210> 509
   <211> 7
   <212> PRT
   <213> Oryctolagus cuniculus
<400> 509
   Asp Ala Ser Asp Leu Ala Ser
   1               5
<210> 510
   <211> 13
   <212> PRT
   <213> Oryctolagus cuniculus
<400> 510
   Ala Gly Tyr Lys Ser Asp Ser Asn Asp Gly Asp Asn Val
   1               5                   10
<210> 511
   <211> 5
   <212> PRT
   <213> Oryctolagus cuniculus
<400> 511
   Arg Asn Ser Ile Thr
   1               5
<210> 512
   <211> 16
   <212> PRT
   <213> Oryctolagus cuniculus
<400> 512
   Ile Ile Thr Gly Ser Gly Arg Thr Tyr Tyr Ala Asn Trp Ala Lys Gly
   1               5                   10                  15
<210> 513
   <211> 10
   <212> PRT
   <213> Oryctolagus cuniculus
<400> 513
   Gly His Pro Gly Leu Gly Ser Gly Asn Ile
   1               5                   10
<210> 514
   <211> 372
   <212> DNA
   <213> Oryctolagus cuniculus
<400> 514
   atggacacga gggcccccac tcagctgctg gggctcctgc tgctctggct cccaggtgcc    60
   acatttgcca ttgaaatgac ccagactcca tcccccgtgt ctgccgctgt gggaggcaca   120
   gtcaccatca attgccaggc cagtgagagt gtttttaata atatgttatc ctggtatcag   180
   cagaaaccag ggcactctcc taagctcctg atctatgatg catccgatct ggcatctggg   240
   gtcccatcgc ggttcaaagg cagtggatct gggacacagt tcactctcac catcagtggc   300
   gtggagtgtg acgatgctgc cacttactat tgtgcagggt ataaaagtga tagtaatgat   360
   ggcgataatg tt                                                       372
<210> 515
   <211> 369
   <212> DNA
   <213> Oryctolagus cuniculus
<400> 515
   atggagactg ggctgcgctg gcttctcctg gtcgctgtgc tcaaaggtgt ccagtgtcag        60
   tcgctggagg agtccggggg tcgcctggtc acgcctggga cacccctgac actcacctgc       120
   acagtctctg gattctccct caacaggaat tcaataacct gggtccgcca ggctccaggg       180
   gaggggctgg aatggatcgg aatcattact ggtagtggta gaacgtacta cgcgaactgg       240
   gcaaaaggcc gattcaccat ctccaaaacc tcgaccacgg tggatctgaa aatgaccagt       300
   ccgacaaccg aggacacggc cacctatttc tgtgccagag gccatcctgg tcttggtagt       360
   ggtaacatc                                                               369
<210> 516
   <211> 36
   <212> DNA
   <213> Oryctolagus cuniculus
<400> 516
   caggccagtg agagtgtttt taataatatg ttatcc 36
<210> 517
   <211> 21
   <212> DNA
   <213> Oryctolagus cuniculus
<400> 517
   gatgcatccg atctggcatc t 21
<210> 518
   <211> 39
   <212> DNA
   <213> Oryctolagus cuniculus
<400> 518
   gcagggtata aaagtgatag taatgatggc gataatgtt 39
<210> 519
   <211> 15
   <212> DNA
   <213> Oryctolagus cuniculus
<400> 519
   aggaattcaa taacc 15
<210> 520
   <211> 48
   <212> DNA
   <213> Oryctolagus cuniculus
<400> 520
   atcattactg gtagtggtag aacgtactac gcgaactggg caaaaggc 48
<210> 521
   <211> 30
   <212> DNA
   <213> Oryctolagus cuniculus
<400> 521
   ggccatcctg gtcttggtag tggtaacatc 30
<210> 522
   <211> 121
   <212> PRT
   <213> Oryctolagus cuniculus
<400> 522
   Met Asp Thr Arg Ala Pro Thr Gln Leu Leu Gly Leu Leu Leu Leu Trp
   1               5                   10                  15
   Leu Pro Gly Ala Thr Phe Ala Gln Val Leu Thr Gln Thr Ala Ser Ser
               20                  25                  30
   Val Ser Ala Ala Val Gly Gly Thr Val Thr Ile Asn Cys Gln Ser Ser
           35                  40                  45
   Gln Ser Val Tyr Asn Asn Tyr Leu Ser Trp Tyr Gln Gln Lys Pro Gly
       50                  55                  60
   Gln Pro Pro Lys Leu Leu Ile Tyr Thr Ala Ser Ser Leu Ala Ser Gly
   65                  70                  75                  80
   Val Pro Ser Arg Phe Lys Gly Ser Gly Ser Gly Thr Gln Phe Thr Leu
                   85                  90                  95
   Thr Ile Ser Glu Val Gln Cys Asp Asp Ala Ala Thr Tyr Tyr Cys Gln
               100                 105                 110
   Gly Tyr Tyr Ser Gly Pro Ile Ile Thr
           115                 120
<210> 523
   <211> 122
   <212> PRT
   <213> Oryctolagus cuniculus
<400> 523
   Met Glu Thr Gly Leu Arg Trp Leu Leu Leu Val Ala Val Leu Lys Gly
   1               5                   10                  15
   Val Gln Cys Gln Ser Leu Glu Glu Ser Gly Gly Arg Leu Val Thr Pro
               20                  25                  30
   Gly Thr Pro Leu Thr Leu Thr Cys Thr Ala Ser Gly Phe Ser Leu Asn
           35                  40                  45
   Asn Tyr Tyr Ile Gln Trp Val Arg Gln Ala Pro Gly Glu Gly Leu Glu
       50                  55                  60
   Trp Ile Gly Ile Ile Tyr Ala Gly Gly Ser Ala Tyr Tyr Ala Thr Trp
   65                  70                  75                  80
   Ala Asn Gly Arg Phe Thr Ile Ala Lys Thr Ser Ser Thr Thr Val Asp
                   85                  90                  95
   Leu Lys Met Thr Ser Leu Thr Thr Glu Asp Thr Ala Thr Tyr Phe Cys
               100                 105                 110
   Ala Arg Gly Thr Phe Asp Gly Tyr Glu Leu
           115                 120
<210> 524
   <211> 12
   <212> PRT
   <213> Oryctolagus cuniculus
<400> 524
   Gln Ser Ser Gln Ser Val Tyr Asn Asn Tyr Leu Ser
       1               5                   10
<210> 525
   <211> 7
   <212> PRT
   <213> Oryctolagus cuniculus
<400> 525
   Thr Ala Ser Ser Leu Ala Ser
   1               5
<210> 526
   <211> 10
   <212> PRT
   <213> Oryctolagus cuniculus
<400> 526
   Gln Gly Tyr Tyr Ser Gly Pro Ile Ile Thr
   1               5                   10
<210> 527
   <211> 5
   <212> PRT
   <213> Oryctolagus cuniculus
<400> 527
   Asn Tyr Tyr Ile Gln
   1               5
<210> 528
   <211> 16
   <212> PRT
   <213> Oryctolagus cuniculus
<400> 528
   Ile Ile Tyr Ala Gly Gly Ser Ala Tyr Tyr Ala Thr Trp Ala Asn Gly
   1               5                   10                  15
<210> 529
   <211> 8
   <212> PRT
   <213> Oryctolagus cuniculus
<400> 529
   Gly Thr Phe Asp Gly Tyr Glu Leu
   1               5
<210> 530
   <211> 363
   <212> DNA
   <213> Oryctolagus cuniculus
<400> 530
   atggacacga gggcccccac tcagctgctg gggctcctgc tgctctggct cccaggtgcc 60
   acatttgcgc aagtgctgac ccagactgca tcgtccgtgt ctgcagctgt gggaggcaca 120
   gtcaccatca attgccagtc cagtcagagt gtttataata actacttatc ctggtatcag 180
   cagaaaccag ggcagcctcc caagctcctg atctatactg catccagcct ggcatctggg 240
   gtcccatcgc ggttcaaagg cagtggatct gggacacagt tcactctcac catcagcgaa 300
   gtgcagtgtg acgatgctgc cacttactac tgtcaaggct attatagtgg tcctataatt 360
   act 363
<210> 531
   <211> 366
   <212> DNA
   <213> Oryctolagus cuniculus
<400> 531
   atggagactg ggctgcgctg gcttctcctg gtcgctgtgc tcaaaggtgt ccagtgtcag        60
   tcgctggagg agtccggggg tcgcctggtc acgcctggga cacccctgac actcacctgc       120
   acagcctctg gattctccct caataactac tacatacaat gggtccgcca ggctccaggg       180
   gaggggctgg aatggatcgg gatcatttat gctggtggta gcgcatacta cgcgacctgg       240
   gcaaacggcc gattcaccat cgccaaaacc tcgtcgacca cggtggatct gaagatgacc       300
   agtctgacaa ccgaggacac ggccacctat ttctgtgcca gagggacatt tgatggttat       360
   gagttg                                                                  366
<210> 532
   <211> 36
   <212> DNA
   <213> Oryctolagus cuniculus
<400> 532
   cagtccagtc agagtgttta taataactac ttatcc 36
<210> 533
   <211> 21
   <212> DNA
   <213> Oryctolagus cuniculus
<400> 533
   actgcatcca gcctggcatc t 21
<210> 534
   <211> 30
   <212> DNA
   <213> Oryctolagus cuniculus
<400> 534
   caaggctatt atagtggtcc tataattact 30
<210> 535
   <211> 15
   <212> DNA
   <213> Oryctolagus cuniculus
<400> 535
   aactactaca tacaa 15
<210> 536
   <211> 48
   <212> DNA
   <213> Oryctolagus cuniculus
<400> 536
   atcatttatg ctggtggtag cgcatactac gcgacctggg caaacggc 48
<210> 537
   <211> 24
   <212> DNA
   <213> Oryctolagus cuniculus
<400> 537
   gggacatttg atggttatga gttg 24
<210> 538
   <211> 122
   <212> PRT
   <213> Oryctolagus cuniculus
<400> 538
   Met Asp Thr Arg Ala Pro Thr Gln Leu Leu Gly Leu Leu Leu Leu Trp
   1               5                   10                  15
   Leu Pro Gly Ala Thr Phe Ala Gln Val Leu Thr Gln Thr Pro Ser Pro
               20                  25                  30
   Val Ser Val Pro Val Gly Asp Thr Val Thr Ile Ser Cys Gln Ser Ser
           35                  40                  45
   Glu Ser Val Tyr Ser Asn Asn Leu Leu Ser Trp Tyr Gln Gln Lys Pro
       50                  55                  60
   Gly Gln Pro Pro Lys Leu Leu Ile Tyr Arg Ala Ser Asn Leu Ala Ser
   65                  70                  75                  80
   Gly Val Pro Ser Arg Phe Lys Gly Ser Gly Ser Gly Thr Gln Phe Thr
                   85                  90                  95
   Leu Thr Ile Ser Gly Ala Gln Cys Asp Asp Ala Ala Thr Tyr Tyr Cys
               100                 105                 110
   Gln Gly Tyr Tyr Ser Gly Val Ile Asn Ser
           115                 120
<210> 539
   <211> 124
   <212> PRT
   <213> Oryctolagus cuniculus
<400> 539
   Met Glu Thr Gly Leu Arg Trp Leu Leu Leu Val Ala Val Leu Lys Gly
   1               5                   10                  15
   Val Gln Cys Gln Ser Val Glu Glu Ser Gly Gly Arg Leu Val Thr Pro
               20                  25                  30
   Gly Thr Pro Leu Thr Leu Thr Cys Thr Val Ser Gly Phe Ser Leu Ser
           35                  40                  45
   Ser Tyr Phe Met Ser Trp Val Arg Gln Ala Pro Gly Glu Gly Leu Glu
       50                  55                  60
   Tyr Ile Gly Phe Ile Asn Pro Gly Gly Ser Ala Tyr Tyr Ala Ser Trp
   65                  70                  75                  80
   Ala Ser Gly Arg Leu Thr Ile Ser Lys Thr Ser Thr Thr Val Asp Leu
                   85                  90                  95
   Lys Ile Thr Ser Pro Thr Thr Glu Asp Thr Ala Thr Tyr Phe Cys Ala
               100                 105                 110
   Arg Ile Leu Ile Val Ser Tyr Gly Ala Phe Thr Ile
           115                 120
<210> 540
   <211> 13
   <212> PRT
   <213> Oryctolagus cuniculus
<400> 540
   Gln Ser Ser Glu Ser Val Tyr Ser Asn Asn Leu Leu Ser
   1               5                   10
<210> 541
   <211> 7
   <212> PRT
   <213> Oryctolagus cuniculus
<400> 541
   Arg Ala Ser Asn Leu Ala Ser
   1               5
<210> 542
   <211> 10
   <212> PRT
   <213> Oryctolagus cuniculus
<400> 542
   Gln Gly Tyr Tyr Ser Gly Val Ile Asn Ser
   1 5 10
<210> 543
   <211> 5
   <212> PRT
   <213> Oryctolagus cuniculus
<400> 543
   Ser Tyr Phe Met Ser
   1 5
<210> 544
   <211> 16
   <212> PRT
   <213> Oryctolagus cuniculus
<400> 544
   Phe Ile Asn Pro Gly Gly Ser Ala Tyr Tyr Ala Ser Trp Ala Ser Gly
   1 5 10 15
<210> 545
   <211> 11
   <212> PRT
   <213> Oryctolagus cuniculus
<400> 545
   Ile Leu Ile Val Ser Tyr Gly Ala Phe Thr Ile
   1 5 10
<210> 546
   <211> 366
   <212> DNA
   <213> Oryctolagus cuniculus
<400> 546
   atggacacga gggcccccac tcagctgctg gggctcctgc tgctctggct cccaggtgcc 60
   acatttgccc aagtgctgac ccagactcca tcccctgtgt ctgtccctgt gggagacaca 120
   gtcaccatca gttgccagtc cagtgagagc gtttatagta ataacctctt atcctggtat 180
   cagcagaaac cagggcagcc tcccaagctc ctgatctaca gggcatccaa tctggcatct 240
   ggtgtcccat cgcggttcaa aggcagtgga tctgggacac agttcactct caccatcagc 300
   ggcgcacagt gtgacgatgc tgccacttac tactgtcaag gctattatag tggtgtcatt 360
   aatagt 366
<210> 547
   <211> 372
   <212> DNA
   <213> Oryctolagus cuniculus
<400> 547
   atggagactg ggctgcgctg gcttctcctg gtcgctgtgc tcaaaggtgt ccagtgtcag        60
   tcggtggagg agtccggggg tcgcctggtc acgcctggga cacccctgac actcacctgc       120
   acagtgtctg gattctccct cagtagctac ttcatgagct gggtccgcca ggctccaggg       180
   gaggggctgg aatacatcgg attcattaat cctggtggta gcgcatacta cgcgagctgg       240
   gcgagtggcc gactcaccat ctccaaaacc tcgaccacgg tagatctgaa aatcaccagt       300
   ccgacaaccg aggacacggc cacctatttc tgtgccagga ttcttattgt ttcttatgga       360
   gcctttacca tc                                                           372
<210> 548
   <211> 39
   <212> DNA
   <213> Oryctolagus cuniculus
<400> 548
   cagtccagtg agagcgttta tagtaataac ctcttatcc 39
<210> 549
   <211> 21
   <212> DNA
   <213> Oryctolagus cuniculus
<400> 549
   agggcatcca atctggcatc t 21
<210> 550
   <211> 30
   <212> DNA
   <213> Oryctolagus cuniculus
<400> 550
   caaggctatt atagtggtgt cattaatagt 30
<210> 551
   <211> 15
   <212> DNA
   <213> Oryctolagus cuniculus
<400> 551
   agctacttca tgagc 15
<210> 552
   <211> 48
   <212> DNA
   <213> Oryctolagus cuniculus
<400> 552
   ttcattaatc ctggtggtag cgcatactac gcgagctggg cgagtggc 48
<210> 553
   <211> 33
   <212> DNA
   <213> Oryctolagus cuniculus
<400> 553
   attcttattg tttcttatgg agcctttacc atc 33
<210> 554
   <211> 122
   <212> PRT
   <213> Oryctolagus cuniculus
<400> 554
   Met Asp Thr Arg Ala Pro Thr Gln Leu Leu Gly Leu Leu Leu Leu Trp
   1               5                   10                  15
   Leu Pro Gly Ala Arg Cys Ala Tyr Asp Met Thr Gln Thr Pro Ala Ser
               20                  25                  30
   Val Glu Val Ala Val Gly Gly Thr Val Thr Ile Lys Cys Gln Ala Thr
           35                  40                  45
   Glu Ser Ile Gly Asn Glu Leu Ser Trp Tyr Gln Gln Lys Pro Gly Gln
       50                  55                  60
   Ala Pro Lys Leu Leu Ile Tyr Ser Ala Ser Thr Leu Ala Ser Gly Val
   65                  70                  75                  80
   Pro Ser Arg Phe Lys Gly Ser Gly Ser Gly Thr Gln Phe Thr Leu Thr
                   85                  90                  95
   Ile Thr Gly Val Glu Cys Asp Asp Ala Ala Thr Tyr Tyr Cys Gln Gln
               100                 105                 110
   Gly Tyr Ser Ser Ala Asn Ile Asp Asn Ala
           115                 120
<210> 555
   <211> 128
   <212> PRT
   <213> Oryctolagus cuniculus
<400> 555
   Met Glu Thr Gly Leu Arg Trp Leu Leu Leu Val Ala Val Leu Lys Gly
   1 5 10 15
   Val Gln Cys Gln Ser Leu Glu Glu Ser Gly Gly Arg Leu Val Thr Pro
   20 25 30
   Gly Thr Pro Leu Thr Leu Thr Cys Thr Val Ser Gly Phe Ser Leu Ser
   35 40 45
   Lys Tyr Tyr Met Ser Trp Val Arg Gln Ala Pro Glu Lys Gly Leu Lys
   50 55 60
   Tyr Ile Gly Tyr Ile Asp Ser Thr Thr Val Asn Thr Tyr Tyr Ala Thr
   65 70 75 80
   Trp Ala Arg Gly Arg Phe Thr Ile Ser Lys Thr Ser Thr Thr Val Asp
   85 90 95
   Leu Lys Ile Thr Ser Pro Thr Ser Glu Asp Thr Ala Thr Tyr Phe Cys
   100 105 110
   Ala Arg Gly Ser Thr Tyr Phe Thr Asp Gly Gly His Arg Leu Asp Leu
   115 120 125
<210> 556
   <211> 11
   <212> PRT
   <213> Oryctolagus cuniculus
<400> 556
   Gln Ala Thr Glu Ser Ile Gly Asn Glu Leu Ser
   1 5 10
<210> 557
   <211> 7
   <212> PRT
   <213> Oryctolagus cuniculus
<400> 557
   Ser Ala Ser Thr Leu Ala Ser
   1 5
<210> 558
   <211> 12
   <212> PRT
   <213> Oryctolagus cuniculus
<400> 558
   Gln Gln Gly Tyr Ser Ser Ala Asn Ile Asp Asn Ala
   1 5 10
<210> 559
   <211> 5
   <212> PRT
   <213> Oryctolagus cuniculus
<400> 559
   Lys Tyr Tyr Met Ser
   1               5
<210> 560
   <211> 17
   <212> PRT
   <213> Oryctolagus cuniculus
<400> 560
   Tyr Ile Asp Ser Thr Thr Val Asn Thr Tyr Tyr Ala Thr Trp Ala Arg
   1               5                   10                  15
   Gly
<210> 561
   <211> 14
   <212> PRT
   <213> Oryctolagus cuniculus
<400> 561
   Gly Ser Thr Tyr Phe Thr Asp Gly Gly His Arg Leu Asp Leu
   1               5                   10
<210> 562
   <211> 366
   <212> DNA
   <213> Oryctolagus cuniculus
<400> 562
   atggacacga gggcccccac tcagctgctg gggctcctgc tgctctggct cccaggtgcc     60
   agatgtgcct atgatatgac ccagactcca gcctctgtgg aggtagctgt gggaggcaca    120
   gtcaccatca agtgccaggc cactgagagc attggcaatg agttatcctg gtatcagcag    180
   aaaccagggc aggctcccaa gctcctgatc tattctgcat ccactctggc atctggggtc    240
   ccatcgcggt tcaaaggcag tggatctggg acacagttca ctctcaccat caccggcgtg    300
   gagtgtgatg atgctgccac ttactactgt caacagggtt atagtagtgc taatattgat    360
   aatgct                                                               366
<210> 563
   <211> 384
   <212> DNA
   <213> Oryctolagus cuniculus
<400> 563
   atggagactg ggctgcgctg gcttctcctg gtcgctgtgc tcaaaggtgt ccagtgtcag        60
   tcgctggagg agtccggggg tcgcctggtc acgcctggga cacccctgac actcacctgc       120
   accgtctctg gattctccct cagtaagtac tacatgagct gggtccgcca ggctccagag       180
   aaggggctga aatacatcgg atacattgat agtactactg ttaatacata ctacgcgacc       240
   tgggcgagag gccgattcac catctccaaa acctcgacca cggtggatct gaagatcacc       300
   agtccgacaa gtgaggacac ggccacctat ttctgtgcca gaggaagtac ttattttact       360
   gatggaggcc atcggttgga tctc                                              384
<210> 564
   <211> 33
   <212> DNA
   <213> Oryctolagus cuniculus
<400> 564
   caggccactg agagcattgg caatgagtta tcc 33
<210> 565
   <211> 21
   <212> DNA
   <213> Oryctolagus cuniculus
<400> 565
   tctgcatcca ctctggcatc t 21
<210> 566
   <211> 36
   <212> DNA
   <213> Oryctolagus cuniculus
<400> 566
   caacagggtt atagtagtgc taatattgat aatgct 36
<210> 567
   <211> 15
   <212> DNA
   <213> Oryctolagus cuniculus
<400> 567
   aagtactaca tgagc 15
<210> 568
   <211> 51
   <212> DNA
   <213> Oryctolagus cuniculus
<400> 568
   tacattgata gtactactgt taatacatac tacgcgacct gggcgagagg c 51
<210> 569
   <211> 42
   <212> DNA
   <213> Oryctolagus cuniculus
<400> 569
   ggaagtactt attttactga tggaggccat cggttggatc tc 42
<210> 570
   <211> 122
   <212> PRT
   <213> Oryctolagus cuniculus
<400> 570
   Met Asp Thr Arg Ala Pro Thr Gln Leu Leu Gly Leu Leu Leu Leu Trp
   1               5                   10                  15
   Leu Pro Gly Ala Arg Cys Ala Tyr Asp Met Thr Gln Thr Pro Ala Ser
               20                  25                  30
   Val Glu Val Ala Val Gly Gly Thr Val Thr Ile Lys Cys Gln Ala Thr
           35                  40                  45
   Glu Ser Ile Gly Asn Glu Leu Ser Trp Tyr Gln Gln Lys Pro Gly Gln
       50                  55                  60
   Ala Pro Lys Leu Leu Ile Tyr Ser Ala Ser Thr Leu Ala Ser Gly Val
   65                  70                  75                  80
   Pro Ser Arg Phe Lys Gly Ser Gly Ser Gly Thr Gln Phe Thr Leu Thr
                   85                  90                  95
   Ile Thr Gly Val Glu Cys Asp Asp Ala Ala Thr Tyr Tyr Cys Gln Gln
               100                 105                 110
   Gly Tyr Ser Ser Ala Asn Ile Asp Asn Ala
           115                 120
<210> 571
   <211> 124
   <212> PRT
   <213> Oryctolagus cuniculus
<400> 571
   Met Glu Thr Gly Leu Arg Trp Leu Leu Leu Val Ala Val Leu Lys Gly
   1               5                   10                  15
   Val Gln Cys Gln Ser Leu Glu Glu Ser Gly Gly Arg Leu Val Thr Pro
               20                  25                  30
   Gly Thr Pro Leu Thr Leu Thr Cys Thr Val Ser Gly Phe Ser Leu Ser
           35                  40                  45
   Thr Tyr Asn Met Gly Trp Val Arg Gln Ala Pro Gly Lys Gly Leu Glu
       50                  55                  60
   Trp Ile Gly Ser Ile Thr Ile Asp Gly Arg Thr Tyr Tyr Ala Ser Trp
   65                  70                  75                  80
   Ala Lys Gly Arg Phe Thr Val Ser Lys Ser Ser Thr Thr Val Asp Leu
                   85                  90                  95
   Lys Met Thr Ser Leu Thr Thr Gly Asp Thr Ala Thr Tyr Phe Cys Ala
               100                 105                 110
   Arg Ile Leu Ile Val Ser Tyr Gly Ala Phe Thr Ile
           115                 120
<210> 572
   <211> 11
   <212> PRT
   <213> Oryctolagus cuniculus
<400> 572
   Gln Ala Thr Glu Ser Ile Gly Asn Glu Leu Ser
   1               5                   10
<210> 573
   <211> 7
   <212> PRT
   <213> Oryctolagus cuniculus
<400> 573
   Ser Ala Ser Thr Leu Ala Ser
   1               5
<210> 574
   <211> 12
   <212> PRT
   <213> Oryctolagus cuniculus
<400> 574
   Gln Gln Gly Tyr Ser Ser Ala Asn Ile Asp Asn Ala
   1 5 10
<210> 575
   <211> 5
   <212> PRT
   <213> Oryctolagus cuniculus
<400> 575
   Thr Tyr Asn Met Gly
   1 5
<210> 576
   <211> 16
   <212> PRT
   <213> Oryctolagus cuniculus
<400> 576
   Ser Ile Thr Ile Asp Gly Arg Thr Tyr Tyr Ala Ser Trp Ala Lys Gly
   1 5 10 15
<210> 577
   <211> 11
   <212> PRT
   <213> Oryctolagus cuniculus
<400> 577
   Ile Leu Ile Val Ser Tyr Gly Ala Phe Thr Ile
   1 5 10
<210> 578
   <211> 366
   <212> DNA
   <213> Oryctolagus cuniculus
<400> 578
   atggacacga gggcccccac tcagctgctg gggctcctgc tgctctggct cccaggtgcc 60
   agatgtgcct atgatatgac ccagactcca gcctctgtgg aggtagctgt gggaggcaca 120
   gtcaccatca agtgccaggc cactgagagc attggcaatg agttatcctg gtatcagcag 180
   aaaccagggc aggctcccaa gctcctgatc tattctgcat ccactctggc atctggggtc 240
   ccatcgcggt tcaaaggcag tggatctggg acacagttca ctctcaccat caccggcgtg 300
   gagtgtgatg atgctgccac ttactactgt caacagggtt atagtagtgc taatattgat 360
   aatgct 366
<210> 579
   <211> 372
   <212> DNA
   <213> Oryctolagus cuniculus
<400> 579
   atggagactg ggctgcgctg gcttctcctg gtcgctgtgc tcaaaggtgt ccagtgtcag        60
   tcgctggagg agtccggggg tcgcctggta acgcctggga cacccctgac actcacctgc       120
   acagtctctg gattctccct cagtacctac aacatgggct gggtccgcca ggctccaggg       180
   aaggggctgg aatggatcgg aagtattact attgatggtc gcacatacta cgcgagctgg       240
   gcgaaaggcc gattcaccgt ctccaaaagc tcgaccacgg tggatctgaa aatgaccagt       300
   ctgacaaccg gggacacggc cacctatttc tgtgccagga ttcttattgt ttcttatggg       360
   gcctttacca tc                                                            372
<210> 580
   <211> 33
   <212> DNA
   <213> Oryctolagus cuniculus
<400> 580
   caggccactg agagcattgg caatgagtta tcc 33
<210> 581
   <211> 21
   <212> DNA
   <213> Oryctolagus cuniculus
<400> 581
   tctgcatcca ctctggcatc t 21
<210> 582
   <211> 36
   <212> DNA
   <213> Oryctolagus cuniculus
<400> 582
   caacagggtt atagtagtgc taatattgat aatgct 36
<210> 583
   <211> 15
   <212> DNA
   <213> Oryctolagus cuniculus
<400> 583
   acctacaaca tgggc 15
<210> 584
   <211> 48
   <212> DNA
   <213> Oryctolagus cuniculus
<400> 584
   agtattacta ttgatggtcg cacatactac gcgagctggg cgaaaggc 48
<210> 585
   <211> 33
   <212> DNA
   <213> Oryctolagus cuniculus
<400> 585
   attcttattg tttcttatgg ggcctttacc atc 33
<210> 586
   <211> 105
   <212> PRT
   <213> Artificial Sequence
<220>
   <223> Kappa constant domain
<400> 586
<210> 587
   <211> 315
   <212> DNA
   <213> Artificial Sequence
<220>
   <223> Kappa constant domain
<400> 587
   gtggctgcac catctgtctt catcttcccg ccatctgatg agcagttgaa atctggaact        60
   gcctctgttg tgtgcctgct gaataacttc tatcccagag aggccaaagt acagtggaag       120
   gtggataacg ccctccaatc gggtaactcc caggagagtg tcacagagca ggacagcaag       180
   gacagcacct acagcctcag cagcaccctg acgctgagca aagcagacta cgagaaacac       240
   aaagtctacg cctgcgaagt cacccatcag ggcctgagct cgcccgtcac aaagagcttc       300
   aacaggggag agtgt                                                        315
<210> 588
   <211> 330
   <212> PRT
   <213> Artificial Sequence
<220>
   <223> Gamma-1 constant domain
<400> 588
   Ala Ser Thr Lys Gly Pro Ser Val Phe Pro Leu Ala Pro Ser Ser Lys
   1               5                   10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Arg Val Glu Pro Lys Ser Cys Asp Lys Thr His Thr Cys Pro Pro Cys
               100                 105                 110
   Pro Ala Pro Glu Leu Leu Gly Gly Pro Ser Val Phe Leu Phe Pro Pro
           115                 120                 125
   Lys Pro Lys Asp Thr Leu Met Ile Ser Arg Thr Pro Glu Val Thr Cys
       130                 135                 140
   Val Val Val Asp Val Ser His Glu Asp Pro Glu Val Lys Phe Asn Trp
   145                 150                 155                 160
   Tyr Val Asp Gly Val Glu Val His Asn Ala Lys Thr Lys Pro Arg Glu
                   165                 170                 175
   Glu Gln Tyr Ala Ser Thr Tyr Arg Val Val Ser Val Leu Thr Val Leu
               180                 185                 190
   His Gln Asp Trp Leu Asn Gly Lys Glu Tyr Lys Cys Lys Val Ser Asn
           195                 200                 205
   Lys Ala Leu Pro Ala Pro Ile Glu Lys Thr Ile Ser Lys Ala Lys Gly
       210                 215                 220
   Gln Pro Arg Glu Pro Gln Val Tyr Thr Leu Pro Pro Ser Arg Glu Glu
   225                 230                 235                 240
   Met Thr Lys Asn Gln Val Ser Leu Thr Cys Leu Val Lys Gly Phe Tyr
                   245                 250                 255
   Pro Ser Asp Ile Ala Val Glu Trp Glu Ser Asn Gly Gln Pro Glu Asn
               260                 265                 270
   Asn Tyr Lys Thr Thr Pro Pro Val Leu Asp Ser Asp Gly Ser Phe Phe
           275                 280                 285
   Leu Tyr Ser Lys Leu Thr Val Asp Lys Ser Arg Trp Gln Gln Gly Asn
       290                 295                 300
   Val Phe Ser Cys Ser Val Met His Glu Ala Leu His Asn His Tyr Thr
   305                 310                 315                 320
   Gln Lys Ser Leu Ser Leu Ser Pro Gly Lys
                   325                 330
<210> 589
   <211> 990
   <212> DNA
   <213> Artificial Sequence
<220>
   <223> Gamma-1 constant domain
<400> 589
   gcctccacca agggcccatc ggtcttcccc ctggcaccct cctccaagag cacctctggg 60
   ggcacagcgg ccctgggctg cctggtcaag gactacttcc ccgaaccggt gacggtgtcg 120
   tggaactcag gcgccctgac cagcggcgtg cacaccttcc cggctgtcct acagtcctca 180
   ggactctact ccctcagcag cgtggtgacc gtgccctcca gcagcttggg cacccagacc 240
   tacatctgca acgtgaatca caagcccagc aacaccaagg tggacaagag agttgagccc 300
   aaatcttgtg acaaaactca cacatgccca ccgtgcccag cacctgaact cctgggggga 360
   ccgtcagtct tcctcttccc cccaaaaccc aaggacaccc tcatgatctc ccggacccct 420
   gaggtcacat gcgtggtggt ggacgtgagc cacgaagacc ctgaggtcaa gttcaactgg 480
   tacgtggacg gcgtggaggt gcataatgcc aagacaaagc cgcgggagga gcagtacgcc 540
   agcacgtacc gtgtggtcag cgtcctcacc gtcctgcacc aggactggct gaatggcaag 600
   gagtacaagt gcaaggtctc caacaaagcc ctcccagccc ccatcgagaa aaccatctcc 660
   aaagccaaag ggcagccccg agaaccacag gtgtacaccc tgcccccatc ccgggaggag 720
   atgaccaaga accaggtcag cctgacctgc ctggtcaaag gcttctatcc cagcgacatc 780
   gccgtggagt gggagagcaa tgggcagccg gagaacaact acaagaccac gcctcccgtg 840
   ctggactccg acggctcctt cttcctctac agcaagctca ccgtggacaa gagcaggtgg 900
   cagcagggga acgtcttctc atgctccgtg atgcatgagg ctctgcacaa ccactacacg 960
   cagaagagcc tctccctgtc tccgggtaaa 990
<210> 590
   <211> 15
   <212> PRT
   <213> Homo sapiens
<400> 590
   Val Pro Pro Gly Glu Asp Ser Lys Asp Val Ala Ala Pro His Arg
   1 5 10 15
<210> 591
   <211> 15
   <212> PRT
   <213> Homo sapiens
<400> 591
   Gly Glu Asp Ser Lys Asp Val Ala Ala Pro His Arg Gln Pro Leu
   1 5 10 15
<210> 592
   <211> 15
   <212> PRT
   <213> Homo sapiens
<400> 592
   Ser Lys Asp Val Ala Ala Pro His Arg Gln Pro Leu Thr Ser Ser
   1 5 10 15
<210> 593
   <211> 15
   <212> PRT
   <213> Homo sapiens
<400> 593
   Val Ala Ala Pro His Arg Gln Pro Leu Thr Ser Ser Glu Arg Ile
   1 5 10 15
<210> 594
   <211> 15
   <212> PRT
   <213> Homo sapiens
<400> 594
   Pro His Arg Gln Pro Leu Thr Ser Ser Glu Arg Ile Asp Lys Gln
   1 5 10 15
<210> 595
   <211> 15
   <212> PRT
   <213> Homo sapiens
<400> 595
   Gln Pro Leu Thr Ser Ser Glu Arg Ile Asp Lys Gln Ile Arg Tyr
   1 5 10 15
<210> 596
   <211> 15
   <212> PRT
   <213> Homo sapiens
<400> 596
   Thr Ser Ser Glu Arg Ile Asp Lys Gln Ile Arg Tyr Ile Leu Asp
   1 5 10 15
<210> 597
   <211> 15
   <212> PRT
   <213> Homo sapiens
<400> 597
   Glu Arg Ile Asp Lys Gln Ile Arg Tyr Ile Leu Asp Gly Ile Ser
   1 5 10 15
<210> 598
   <211> 15
   <212> PRT
   <213> Homo sapiens
<400> 598
   Asp Lys Gln Ile Arg Tyr Ile Leu Asp Gly Ile Ser Ala Leu Arg
   1               5                   10                  15
<210> 599
   <211> 15
   <212> PRT
   <213> Homo sapiens
<400> 599
   Ile Arg Tyr Ile Leu Asp Gly Ile Ser Ala Leu Arg Lys Glu Thr
   1               5                   10                  15
<210> 600
   <211> 15
   <212> PRT
   <213> Homo sapiens
<400> 600
   Ile Leu Asp Gly Ile Ser Ala Leu Arg Lys Glu Thr Cys Asn Lys
   1               5                   10                  15
<210> 601
   <211> 15
   <212> PRT
   <213> Homo sapiens
<400> 601
   Gly Ile Ser Ala Leu Arg Lys Glu Thr Cys Asn Lys Ser Asn Met
   1               5                   10                  15
<210> 602
   <211> 15
   <212> PRT
   <213> Homo sapiens
<400> 602
   Ala Leu Arg Lys Glu Thr Cys Asn Lys Ser Asn Met Cys Glu Ser
   1               5                   10                  15
<210> 603
   <211> 15
   <212> PRT
   <213> Homo sapiens
<400> 603
   Lys Glu Thr Cys Asn Lys Ser Asn Met Cys Glu Ser Ser Lys Glu
   1               5                   10                  15
<210> 604
   <211> 15
   <212> PRT
   <213> Homo sapiens
<400> 604
   Cys Asn Lys Ser Asn Met Cys Glu Ser Ser Lys Glu Ala Leu Ala
   1 5 10 15
<210> 605
   <211> 15
   <212> PRT
   <213> Homo sapiens
<400> 605
   Ser Asn Met Cys Glu Ser Ser Lys Glu Ala Leu Ala Glu Asn Asn
   1 5 10 15
<210> 606
   <211> 15
   <212> PRT
   <213> Homo sapiens
<400> 606
   Cys Glu Ser Ser Lys Glu Ala Leu Ala Glu Asn Asn Leu Asn Leu
   1 5 10 15
<210> 607
   <211> 15
   <212> PRT
   <213> Homo sapiens
<400> 607
   Ser Lys Glu Ala Leu Ala Glu Asn Asn Leu Asn Leu Pro Lys Met
   1 5 10 15
<210> 608
   <211> 15
   <212> PRT
   <213> Homo sapiens
<400> 608
   Ala Leu Ala Glu Asn Asn Leu Asn Leu Pro Lys Met Ala Glu Lys
   1 5 10 15
<210> 609
   <211> 15
   <212> PRT
   <213> Homo sapiens
<400> 609
   Glu Asn Asn Leu Asn Leu Pro Lys Met Ala Glu Lys Asp Gly Cys
   1               5                   10                  15
<210> 610
   <211> 15
   <212> PRT
   <213> Homo sapiens
<400> 610
   Leu Asn Leu Pro Lys Met Ala Glu Lys Asp Gly Cys Phe Gln Ser
   1               5                   10                  15
<210> 611
   <211> 15
   <212> PRT
   <213> Homo sapiens
<400> 611
   Pro Lys Met Ala Glu Lys Asp Gly Cys Phe Gln Ser Gly Phe Asn
   1               5                   10                  15
<210> 612
   <211> 15
   <212> PRT
   <213> Homo sapiens
<400> 612
   Ala Glu Lys Asp Gly Cys Phe Gln Ser Gly Phe Asn Glu Glu Thr
   1               5                   10                  15
<210> 613
   <211> 15
   <212> PRT
   <213> Homo sapiens
<400> 613
   Asp Gly Cys Phe Gln Ser Gly Phe Asn Glu Glu Thr Cys Leu Val
   1               5                   10                  15
<210> 614
   <211> 15
   <212> PRT
   <213> Homo sapiens
<400> 614
   Phe Gln Ser Gly Phe Asn Glu Glu Thr Cys Leu Val Lys Ile Ile
   1               5                   10                  15
<210> 615
   <211> 15
   <212> PRT
   <213> Homo sapiens
<400> 615
   Gly Phe Asn Glu Glu Thr Cys Leu Val Lys Ile Ile Thr Gly Leu
   1 5 10 15
<210> 616
   <211> 15
   <212> PRT
   <213> Homo sapiens
<400> 616
   Glu Glu Thr Cys Leu Val Lys Ile Ile Thr Gly Leu Leu Glu Phe
   1 5 10 15
<210> 617
   <211> 15
   <212> PRT
   <213> Homo sapiens
<400> 617
   Cys Leu Val Lys Ile Ile Thr Gly Leu Leu Glu Phe Glu Val Tyr
   1 5 10 15
<210> 618
   <211> 15
   <212> PRT
   <213> Homo sapiens
<400> 618
   Lys Ile Ile Thr Gly Leu Leu Glu Phe Glu Val Tyr Leu Glu Tyr
   1 5 10 15
<210> 619
   <211> 15
   <212> PRT
   <213> Homo sapiens
<400> 619
   Thr Gly Leu Leu Glu Phe Glu Val Tyr Leu Glu Tyr Leu Gln Asn
   1 5 10 15
<210> 620
   <211> 15
   <212> PRT
   <213> Homo sapiens
<400> 620
   Leu Glu Phe Glu Val Tyr Leu Glu Tyr Leu Gln Asn Arg Phe Glu
   1 5 10 15
<210> 621
   <211> 15
   <212> PRT
   <213> Homo sapiens
<400> 621
   Glu Val Tyr Leu Glu Tyr Leu Gln Asn Arg Phe Glu Ser Ser Glu
   1 5 10 15
<210> 622
   <211> 15
   <212> PRT
   <213> Homo sapiens
<400> 622
   Leu Glu Tyr Leu Gln Asn Arg Phe Glu Ser Ser Glu Glu Gln Ala
   1 5 10 15
<210> 623
   <211> 15
   <212> PRT
   <213> Homo sapiens
<400> 623
   Leu Gln Asn Arg Phe Glu Ser Ser Glu Glu Gln Ala Arg Ala Val
   1 5 10 15
<210> 624
   <211> 15
   <212> PRT
   <213> Homo sapiens
<400> 624
   Arg Phe Glu Ser Ser Glu Glu Gln Ala Arg Ala Val Gln Met Ser
   1 5 10 15
<210> 625
   <211> 15
   <212> PRT
   <213> Homo sapiens
<400> 625
   Ser Ser Glu Glu Gln Ala Arg Ala Val Gln Met Ser Thr Lys Val
   1 5 10 15
<210> 626
   <211> 15
   <212> PRT
   <213> Homo sapiens
<400> 626
   Glu Gln Ala Arg Ala Val Gln Met Ser Thr Lys Val Leu Ile Gln
   1 5 10 15
<210> 627
   <211> 15
   <212> PRT
   <213> Homo sapiens
<400> 627
   Arg Ala Val Gln Met Ser Thr Lys Val Leu Ile Gln Phe Leu Gln
   1 5 10 15
<210> 628
   <211> 15
   <212> PRT
   <213> Homo sapiens
<400> 628
   Gln Met Ser Thr Lys Val Leu Ile Gln Phe Leu Gln Lys Lys Ala
   1 5 10 15
<210> 629
   <211> 15
   <212> PRT
   <213> Homo sapiens
<400> 629
   Thr Lys Val Leu Ile Gln Phe Leu Gln Lys Lys Ala Lys Asn Leu
   1 5 10 15
<210> 630
   <211> 15
   <212> PRT
   <213> Homo sapiens
<400> 630
   Leu Ile Gln Phe Leu Gln Lys Lys Ala Lys Asn Leu Asp Ala Ile
   1 5 10 15
<210> 631
   <211> 15
   <212> PRT
   <213> Homo sapiens
<400> 631
   Phe Leu Gln Lys Lys Ala Lys Asn Leu Asp Ala Ile Thr Thr Pro
   1 5 10 15
<210> 632
   <211> 15
   <212> PRT
   <213> Homo sapiens
<400> 632
   Lys Lys Ala Lys Asn Leu Asp Ala Ile Thr Thr Pro Asp Pro Thr
   1 5 10 15
<210> 633
   <211> 15
   <212> PRT
   <213> Homo sapiens
<400> 633
   Lys Asn Leu Asp Ala Ile Thr Thr Pro Asp Pro Thr Thr Asn Ala
   1               5                   10                  15
<210> 634
   <211> 15
   <212> PRT
   <213> Homo sapiens
<400> 634
   Asp Ala Ile Thr Thr Pro Asp Pro Thr Thr Asn Ala Ser Leu Leu
   1               5                   10                  15
<210> 635
   <211> 15
   <212> PRT
   <213> Homo sapiens
<400> 635
   Thr Thr Pro Asp Pro Thr Thr Asn Ala Ser Leu Leu Thr Lys Leu
   1               5                   10                  15
<210> 636
   <211> 15
   <212> PRT
   <213> Homo sapiens
<400> 636
   Asp Pro Thr Thr Asn Ala Ser Leu Leu Thr Lys Leu Gln Ala Gln
   1               5                   10                  15
<210> 637
   <211> 15
   <212> PRT
   <213> Homo sapiens
<400> 637
   Thr Asn Ala Ser Leu Leu Thr Lys Leu Gln Ala Gln Asn Gln Trp
   1               5                   10                  15
<210> 638
   <211> 15
   <212> PRT
   <213> Homo sapiens
<400> 638
   Ser Leu Leu Thr Lys Leu Gln Ala Gln Asn Gln Trp Leu Gln Asp
   1 5 10 15
<210> 639
   <211> 15
   <212> PRT
   <213> Homo sapiens
<400> 639
   Thr Lys Leu Gln Ala Gln Asn Gln Trp Leu Gln Asp Met Thr Thr
   1 5 10 15
<210> 640
   <211> 15
   <212> PRT
   <213> Homo sapiens
<400> 640
   Gln Ala Gln Asn Gln Trp Leu Gln Asp Met Thr Thr His Leu Ile
   1 5 10 15
<210> 641
   <211> 15
   <212> PRT
   <213> Homo sapiens
<400> 641
   Asn Gln Trp Leu Gln Asp Met Thr Thr His Leu Ile Leu Arg Ser
   1 5 10 15
<210> 642
   <211> 15
   <212> PRT
   <213> Homo sapiens
<400> 642
   Leu Gln Asp Met Thr Thr His Leu Ile Leu Arg Ser Phe Lys Glu
   1 5 10 15
<210> 643
   <211> 15
   <212> PRT
   <213> Homo sapiens
<400> 643
   Met Thr Thr His Leu Ile Leu Arg Ser Phe Lys Glu Phe Leu Gln
   1 5 10 15
<210> 644
   <211> 15
   <212> PRT
   <213> Homo sapiens
<400> 644
   His Leu Ile Leu Arg Ser Phe Lys Glu Phe Leu Gln Ser Ser Leu
   1 5 10 15
<210> 645
   <211> 15
   <212> PRT
   <213> Homo sapiens
<400> 645
   Leu Arg Ser Phe Lys Glu Phe Leu Gln Ser Ser Leu Arg Ala Leu
   1 5 10 15
<210> 646
   <211> 15
   <212> PRT
   <213> Homo sapiens
<400> 646
   Phe Lys Glu Phe Leu Gln Ser Ser Leu Arg Ala Leu Arg Gln Met
   1 5 10 15
<210> 647
   <211> 111
   <212> PRT
   <213> Oryctolagus cuniculus
<400> 647
   Ala Tyr Asp Met Thr Gln Thr Pro Ala Ser Val Ser Ala Ala Val Gly
   1 5 10 15
   Gly Thr Val Thr Ile Lys Cys Gln Ala Ser Gln Ser Ile Asn Asn Glu
   20 25 30
   Leu Ser Trp Tyr Gln Gln Lys Pro Gly Gln Arg Pro Lys Leu Leu Ile
   35 40 45
   Tyr Arg Ala Ser Thr Leu Ala Ser Gly Val Ser Ser Arg Phe Lys Gly
   50 55 60
   Ser Gly Ser Gly Thr Glu Phe Thr Leu Thr Ile Ser Asp Leu Glu Cys
   65 70 75 80
   Ala Asp Ala Ala Thr Tyr Tyr Cys Gln Gln Gly Tyr Ser Leu Arg Asn
   85 90 95
   Ile Asp Asn Ala Phe Gly Gly Gly Thr Glu Val Val Val Lys Arg
   100 105 110
<210> 648
   <211> 88
   <212> PRT
   <213> Homo sapiens
<400> 648
   Ala Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn Asp
               20                  25                  30
   Leu Gly Trp Tyr Gln Gln Lys Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys
                   85
<210> 649
   <211> 88
   <212> PRT
   <213> Homo sapiens
<400> 649
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Asn Tyr
               20                  25                  30
   Leu Ala Trp Tyr Gln Gln Lys Pro Gly Lys Val Pro Lys Leu Leu Ile
           35                  40                  45
   Tyr Ala Ala Ser Thr Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys
                   85
<210> 650
   <211> 88
   <212> PRT
   <213> Homo sapiens
<400> 650
   Asp Ile Gln Met Thr Gln Ser Pro Ser Thr Leu Ser Ala Ser Val Gly
   1               5                   10                  15
   Asp Arg Val Thr Ile Thr Cys Arg Ala Ser Gln Ser Ile Ser Ser Trp
               20                  25                  30
   Leu Ala Trp Tyr Gln Gln Lys Pro Gly Lys Ala Pro Lys Leu Leu Ile
           35                  40                  45
   Tyr Lys Ala Ser Ser Leu Glu Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Asp Asp Phe Ala Thr Tyr Tyr Cys
                   85
<210> 651
   <211> 111
   <212> PRT
   <213> Artificial Sequence
<220>
   <223> Humanized antibody
<400> 651
   Ala Ile Gln Met Thr Gln Ser Pro Ser Ser Leu Ser Ala Ser Val Gly
   1               5                   10                  15
   Asp Arg Val Thr Ile Thr Cys Gln Ala Ser Gln Ser Ile Asn Asn Glu
               20                  25                  30
   Leu Ser Trp Tyr Gln Gln Lys Pro Gly Lys Ala Pro Lys Leu Leu Ile
           35                  40                  45
   Tyr Arg Ala Ser Thr Leu Ala Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Gly Tyr Ser Leu Arg Asn
                   85                  90                  95
   Ile Asp Asn Ala Phe Gly Gly Gly Thr Lys Val Glu Ile Lys Arg
               100                 105                 110
<210> 652
   <211> 117
   <212> PRT
   <213> Oryctolagus cuniculus
<400> 652
   Gln Ser Leu Glu Glu Ser Gly Gly Arg Leu Val Thr Pro Gly Thr Pro
   1               5                   10                  15
   Leu Thr Leu Thr Cys Thr Ala Ser Gly Phe Ser Leu Ser Asn Tyr Tyr
               20                  25                  30
   Val Thr Trp Val Arg Gln Ala Pro Gly Lys Gly Leu Glu Trp Ile Gly
           35                  40                  45
   Ile Ile Tyr Gly Ser Asp Glu Thr Ala Tyr Ala Thr Trp Ala Ile Gly
       50                  55                  60
   Arg Phe Thr Ile Ser Lys Thr Ser Thr Thr Val Asp Leu Lys Met Thr
   65 70 75 80
   Ser Leu Thr Ala Ala Asp Thr Ala Thr Tyr Phe Cys Ala Arg Asp Asp
   85 90 95
   Ser Ser Asp Trp Asp Ala Lys Phe Asn Leu Trp Gly Gln Gly Thr Leu
   100 105 110
   Val Thr Val Ser Ser
   115
<210> 653
   <211> 97
   <212> PRT
   <213> Homo sapiens
<400> 653
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Val Ser Ser Asn
   20 25 30
   Tyr Met Ser Trp Val Arg Gln Ala Pro Gly Lys Gly Leu Glu Trp Val
   35 40 45
   Ser Val Ile Tyr Ser Gly Gly Ser Thr Tyr Tyr Ala Asp Ser Val Lys
   50 55 60
   Gly Arg Phe Thr Ile Ser Arg Asp Asn Ser Lys Asn Thr Leu Tyr Leu
   65 70 75 80
   Gln Met Asn Ser Leu Arg Ala Glu Asp Thr Ala Val Tyr Tyr Cys Ala
   85 90 95
   Arg
<210> 654
   <211> 97
   <212> PRT
   <213> Homo sapiens
<400> 654
   Glu Val Gln Leu Val Glu Ser Gly Gly Gly Leu Ile Gln Pro Gly Gly
   1 5 10 15
   Ser Leu Arg Leu Ser Cys Ala Ala Ser Gly Phe Thr Val Ser Ser Asn
   20 25 30
   Tyr Met Ser Trp Val Arg Gln Ala Pro Gly Lys Gly Leu Glu Trp Val
   35 40 45
   Ser Val Ile Tyr Ser Gly Gly Ser Thr Tyr Tyr Ala Asp Ser Val Lys
   50 55 60
   Gly Arg Phe Thr Ile Ser Arg Asp Asn Ser Lys Asn Thr Leu Tyr Leu
   65 70 75 80
   Gln Met Asn Ser Leu Arg Ala Glu Asp Thr Ala Val Tyr Tyr Cys Ala
   85 90 95
   Arg
<210> 655
   <211> 98
   <212> PRT
   <213> Homo sapiens
<400> 655
   Glu Val Gln Leu Leu Glu Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Ala Met Ser Trp Val Arg Gln Ala Pro Gly Lys Gly Leu Glu Trp Val
   35 40 45
   Ser Val Ile Tyr Ser Gly Gly Ser Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Lys
<210> 656
   <211> 120
   <212> PRT
   <213> Artificial sequence
<220>
   <223> Humanized antibody
<400> 656
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Ser Leu Ser Asn Tyr
               20                  25                  30
   Tyr Val Thr Trp Val Arg Gln Ala Pro Gly Lys Gly Leu Glu Trp Val
           35                  40                  45
   Gly Ile Ile Tyr Gly Ser Asp Glu Thr Ala Tyr Ala Thr Trp Ala Ile
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Arg Asp Asp Ser Ser Asp Trp Asp Ala Lys Phe Asn Leu Trp Gly Gln
               100                 105                 110
   Gly Thr Leu Val Thr Val Ser Ser
           115                 120
<210> 657
   <211> 120
   <212> PRT
   <213> Artificial sequence
<220>
   <223> Humanized antibody
<400> 657
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Ser Leu Ser Asn Tyr
               20                  25                  30
   Tyr Val Thr Trp Val Arg Gln Ala Pro Gly Lys Gly Leu Glu Trp Val
           35                  40                  45
   Gly Ile Ile Tyr Gly Ser Asp Glu Thr Ala Tyr Ala Thr Ser Ala Ile
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Arg Asp Asp Ser Ser Asp Trp Asp Ala Lys Phe Asn Leu Trp Gly Gln
               100                 105                 110
   Gly Thr Leu Val Thr Val Ser Ser
           115                 120
<210> 658
   <211> 166
   <212> PRT
   <213> Oryctolagus cuniculus
<400> 658
   Met Glu Thr Gly Leu Arg Trp Leu Leu Leu Val Ala Val Leu Lys Gly
   1 5 10 15
   Val Gln Cys Gln Ser Leu Glu Glu Ser Gly Gly Arg Leu Val Thr Pro
   20 25 30
   Gly Thr Pro Leu Thr Leu Thr Cys Thr Ala Ser Gly Phe Ser Leu Ser
   35 40 45
   Asn Tyr Tyr Val Thr Trp Val Arg Gln Ala Pro Gly Lys Gly Leu Glu
   50 55 60
   Trp Ile Gly Ile Ile Tyr Gly Ser Asp Glu Thr Ala Tyr Ala Thr Ser
   65 70 75 80
   Ala Ile Gly Arg Phe Thr Ile Ser Lys Thr Ser Thr Thr Val Asp Leu
   85 90 95
   Lys Met Thr Ser Leu Thr Ala Ala Asp Thr Ala Thr Tyr Phe Cys Ala
   100 105 110
   Arg Asp Asp Ser Ser Asp Trp Asp Ala Lys Phe Asn Leu Trp Gly Gln
   115 120 125
   Gly Thr Leu Val Thr Val Ser Ser Ala Ser Thr Lys Gly Pro Ser Val
   130 135 140
   Phe Pro Leu Ala Pro Ser Ser Lys Ser Thr Ser Gly Gly Thr Ala Ala
   145 150 155 160
   Leu Gly Cys Leu Val Lys
   165
<210> 659
   <211> 16
   <212> PRT
   <213> Oryctolagus cuniculus
<400> 659
   Ile Ile Tyr Gly Ser Asp Glu Thr Ala Tyr Ala Thr Ser Ala Ile Gly
   1 5 10 15
<210> 660
   <211> 122
   <212> PRT
   <213> Oryctolagus cuniculus
<400> 660
   Met Asp Thr Arg Ala Pro Thr Gln Leu Leu Gly Leu Leu Leu Leu Trp
   1               5                   10                  15
   Leu Pro Gly Ala Arg Cys Ala Tyr Asp Met Thr Gln Thr Pro Ala Ser
               20                  25                  30
   Val Ser Ala Ala Val Gly Gly Thr Val Thr Ile Lys Cys Gln Ala Ser
           35                  40                  45
   Gln Ser Ile Asn Asn Glu Leu Ser Trp Tyr Gln Gln Lys Pro Gly Gln
       50                  55                  60
   Arg Pro Lys Leu Leu Ile Tyr Arg Ala Ser Thr Leu Ala Ser Gly Val
   65                  70                  75                  80
   Ser Ser Arg Phe Lys Gly Ser Gly Ser Gly Thr Glu Phe Thr Leu Thr
                   85                  90                  95
   Ile Ser Asp Leu Glu Cys Ala Asp Ala Ala Thr Tyr Tyr Cys Gln Gln
               100                 105                 110
   Gly Tyr Ser Leu Arg Asn Ile Asp Asn Ala
           115                 120
<210> 661
   <211> 125
   <212> PRT
   <213> Oryctolagus cuniculus
<400> 661
   Met Glu Thr Gly Leu Arg Trp Leu Leu Leu Val Ala Val Leu Lys Gly
   1               5                   10                  15
   Val Gln Cys Gln Ser Leu Glu Glu Ser Gly Gly Arg Leu Val Thr Pro
               20                  25                  30
   Gly Thr Pro Leu Thr Leu Thr Cys Thr Ala Ser Gly Phe Ser Leu Ser
           35                  40                  45
   Asn Tyr Tyr Val Thr Trp Val Arg Gln Ala Pro Gly Lys Gly Leu Glu
       50                  55                  60
   Trp Ile Gly Ile Ile Tyr Gly Ser Asp Glu Thr Ala Tyr Ala Thr Trp
   65                  70                  75                  80
   Ala Ile Gly Arg Phe Thr Ile Ser Lys Thr Ser Thr Thr Val Asp Leu
                   85                  90                  95
   Lys Met Thr Ser Leu Thr Ala Ala Asp Thr Ala Thr Tyr Phe Cys Ala
               100                 105                 110
   Arg Asp Asp Ser Ser Asp Trp Asp Ala Lys Phe Asn Leu
           115                 120                 125
<210> 662
   <211> 366
   <212> DNA
   <213> Oryctolagus cuniculus
<400> 662
   atggacacga gggcccccac tcagctgctg gggctcctgc tgctctggct cccaggtgcc      60
   agatgtgcct atgatatgac ccagactcca gcctcggtgt ctgcagctgt gggaggcaca     120
   gtcaccatca agtgccaggc cagtcagagc attaacaatg aattatcctg gtatcagcag     180
   aaaccagggc agcgtcccaa gctcctgatc tatagggcat ccactctggc atctggggtc     240
   tcatcgcggt tcaaaggcag tggatctggg acagagttca ctctcaccat cagcgacctg     300
   gagtgtgccg atgctgccac ttactactgt caacagggtt atagtctgag gaatattgat     360
   aatgct                                                                366
<210> 663
   <211> 375
   <212> DNA
   <213> Oryctolagus cuniculus
<400> 663
   atggagactg ggctgcgctg gcttctcctg gtcgctgtgc tcaaaggtgt ccagtgtcag      60
   tcgctggagg agtccggggg tcgcctggtc acgcctggga cacccctgac actcacctgc     120
   acagcctctg gattctccct cagtaactac tacgtgacct gggtccgcca ggctccaggg     180
   aaggggctgg aatggatcgg aatcatttat ggtagtgatg aaacggccta cgcgacctgg     240
   gcgataggcc gattcaccat ctccaaaacc tcgaccacgg tggatctgaa aatgaccagt     300
   ctgacagccg cggacacggc cacctatttc tgtgccagag atgatagtag tgactgggat     360
   gcaaaattta acttg                                                      375
<210> 664
   <211> 450
   <212> PRT
   <213> Oryctolagus cuniculus
<400> 664
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Ser Leu Ser Asn Tyr
               20                  25                  30
   Tyr Val Thr Trp Val Arg Gln Ala Pro Gly Lys Gly Leu Glu Trp Val
           35                  40                  45
   Gly Ile Ile Tyr Gly Ser Asp Glu Thr Ala Tyr Ala Thr Trp Ala Ile
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Arg Asp Asp Ser Ser Asp Trp Asp Ala Lys Phe Asn Leu Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
   180 185 190
   Ser Ser Ser Leu Gly Thr Gln Thr Tyr Ile Cys Asn Val Asn His Lys
   195 200 205
   Pro Ser Asn Thr Lys Val Asp Lys Arg Val Glu Pro Lys Ser Cys Asp
   210 215 220
   Lys Thr His Thr Cys Pro Pro Cys Pro Ala Pro Glu Leu Leu Gly Gly
   225 230 235 240
   Pro Ser Val Phe Leu Phe Pro Pro Lys Pro Lys Asp Thr Leu Met Ile
   245 250 255
   Ser Arg Thr Pro Glu Val Thr Cys Val Val Val Asp Val Ser His Glu
   260 265 270
   Asp Pro Glu Val Lys Phe Asn Trp Tyr Val Asp Gly Val Glu Val His
   275 280 285
   Asn Ala Lys Thr Lys Pro Arg Glu Glu Gln Tyr Ala Ser Thr Tyr Arg
   290 295 300
   Val Val Ser Val Leu Thr Val Leu His Gln Asp Trp Leu Asn Gly Lys
   305 310 315 320
   Glu Tyr Lys Cys Lys Val Ser Asn Lys Ala Leu Pro Ala Pro Ile Glu
   325 330 335
   Lys Thr Ile Ser Lys Ala Lys Gly Gln Pro Arg Glu Pro Gln Val Tyr
   340 345 350
   Thr Leu Pro Pro Ser Arg Asp Glu Leu Thr Lys Asn Gln Val Ser Leu
   355 360 365
   Thr Cys Leu Val Lys Gly Phe Tyr Pro Ser Asp Ile Ala Val Glu Trp
   370 375 380
   Glu Ser Asn Gly Gln Pro Glu Asn Asn Tyr Lys Thr Thr Pro Pro Val
   385 390 395 400
   Leu Asp Ser Asp Gly Ser Phe Phe Leu Tyr Ser Lys Leu Thr Val Asp
   405 410 415
   Lys Ser Arg Trp Gln Gln Gly Asn Val Phe Ser Cys Ser Val Met His
   420 425 430
   Glu Ala Leu His Asn His Tyr Thr Gln Lys Ser Leu Ser Leu Ser Pro
   435 440 445
   Gly Lys
   450
<210> 665
   <211> 450
   <212> PRT
   <213> Oryctolagus cuniculus
<400> 665
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Ser Leu Ser Asn Tyr
   20 25 30
   Tyr Val Thr Trp Val Arg Gln Ala Pro Gly Lys Gly Leu Glu Trp Val
   35 40 45
   Gly Ile Ile Tyr Gly Ser Asp Glu Thr Ala Tyr Ala Thr Ser Ala Ile
   50 55 60
   Gly Arg Phe Thr Ile Ser Arg Asp Asn Ser Lys Asn Thr Leu Tyr Leu
   65 70 75 80
   Gln Met Asn Ser Leu Arg Ala Glu Asp Thr Ala Val Tyr Tyr Cys Ala
   85 90 95
   Arg Asp Asp Ser Ser Asp Trp Asp Ala Lys Phe Asn Leu Trp Gly Gln
   100 105 110
   Gly Thr Leu Val Thr Val Ser Ser Ala Ser Thr Lys Gly Pro Ser Val
   115 120 125
   Phe Pro Leu Ala Pro Ser Ser Lys Ser Thr Ser Gly Gly Thr Ala Ala
   130 135 140
   Leu Gly Cys Leu Val Lys Asp Tyr Phe Pro Glu Pro Val Thr Val Ser
   145 150 155 160
   Trp Asn Ser Gly Ala Leu Thr Ser Gly Val His Thr Phe Pro Ala Val
   165 170 175
   Leu Gln Ser Ser Gly Leu Tyr Ser Leu Ser Ser Val Val Thr Val Pro
   180 185 190
   Ser Ser Ser Leu Gly Thr Gln Thr Tyr Ile Cys Asn Val Asn His Lys
           195                 200                 205
   Pro Ser Asn Thr Lys Val Asp Lys Arg Val Glu Pro Lys Ser Cys Asp
       210                 215                 220
   Lys Thr His Thr Cys Pro Pro Cys Pro Ala Pro Glu Leu Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Ala Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Pro Ala Pro Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Ser Arg Asp Glu Leu Thr Lys Asn Gln Val Ser Leu
           355                 360                 365
   Thr Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Tyr Ser Lys Leu Thr Val Asp
                   405                 410                 415
   Lys Ser Arg Trp Gln Gln Gly Asn Val Phe Ser Cys Ser Val Met His
               420                 425                 430
   Glu Ala Leu His Asn His Tyr Thr Gln Lys Ser Leu Ser Leu Ser Pro
           435                 440                 445
   Gly Lys
       450
<210> 666
   <211> 216
   <212> PRT
   <213> Oryctolagus cuniculus
<400> 666
   Ile Gln Met Thr Gln Ser Pro Ser Ser Leu Ser Ala Ser Val Gly Asp
   1               5                   10                  15
   Arg Val Thr Ile Thr Cys Gln Ala Ser Gln Ser Ile Asn Asn Glu Leu
               20                  25                  30
   Ser Trp Tyr Gln Gln Lys Pro Gly Lys Ala Pro Lys Leu Leu Ile Tyr
           35                  40                  45
   Arg Ala Ser Thr Leu Ala Ser Gly Val Pro Ser Arg Phe Ser Gly Ser
       50                  55                  60
   Gly Ser Gly Thr Asp Phe Thr Leu Thr Ile Ser Ser Leu Gln Pro Asp
   65                  70                  75                  80
   Asp Phe Ala Thr Tyr Tyr Cys Gln Gln Gly Tyr Ser Leu Arg Asn Ile
                   85                  90                  95
   Asp Asn Ala Phe Gly Gly Gly Thr Lys Val Glu Ile Lys Arg Thr Val
               100                 105                 110
   Ala Ala Pro Ser Val Phe Ile Phe Pro Pro Ser Asp Glu Gln Leu Lys
           115                 120                 125
   Ser Gly Thr Ala Ser Val Val Cys Leu Leu Asn Asn Phe Tyr Pro Arg
       130                 135                 140
   Glu Ala Lys Val Gln Trp Lys Val Asp Asn Ala Leu Gln Ser Gly Asn
   145                 150                 155                 160
   Ser Gln Glu Ser Val Thr Glu Gln Asp Ser Lys Asp Ser Thr Tyr Ser
                   165                 170                 175
   Leu Ser Ser Thr Leu Thr Leu Ser Lys Ala Asp Tyr Glu Lys His Lys
               180                 185                 190
   Val Tyr Ala Cys Glu Val Thr His Gln Gly Leu Ser Ser Pro Val Thr
           195                 200                 205
   Lys Ser Phe Asn Arg Gly Glu Cys
       210                 215
<210> 667
   <211> 122
   <212> PRT
   <213> Oryctolagus cuniculus
<400> 667
   Met Asp Thr Arg Ala Pro Thr Gln Leu Leu Gly Leu Leu Leu Leu Trp
   1               5                   10                  15
   Leu Pro Gly Ala Arg Cys Ala Tyr Asp Met Thr Gln Thr Pro Ala Ser
               20                  25                  30
   Val Glu Val Ala Val Gly Gly Thr Val Thr Ile Asn Cys Gln Ala Ser
           35                  40                  45
   Glu Thr Ile Tyr Ser Trp Leu Ser Trp Tyr Gln Gln Lys Pro Gly Gln
       50                  55                  60
   Pro Pro Lys Leu Leu Ile Tyr Gln Ala Ser Asp Leu Ala Ser Gly Val
   65                  70                  75                  80
   Pro Ser Arg Phe Ser Gly Ser Gly Ala Gly Thr Glu Tyr Thr Leu Thr
                   85                  90                  95
   Ile Ser Gly Val Gln Cys Asp Asp Ala Ala Thr Tyr Tyr Cys Gln Gln
               100                 105                 110
   Gly Tyr Ser Gly Ser Asn Val Asp Asn Val
           115                 120
<210> 668
   <211> 126
   <212> PRT
   <213> Oryctolagus cuniculus
<400> 668
   Met Glu Thr Gly Leu Arg Trp Leu Leu Leu Val Ala Val Leu Lys Gly
   1               5                   10                  15
   Val Gln Cys Gln Glu Gln Leu Lys Glu Ser Gly Gly Arg Leu Val Thr
               20                  25                  30
   Pro Gly Thr Pro Leu Thr Leu Thr Cys Thr Ala Ser Gly Phe Ser Leu
           35                  40                  45
   Asn Asp His Ala Met Gly Trp Val Arg Gln Ala Pro Gly Lys Gly Leu
       50                  55                  60
   Glu Tyr Ile Gly Phe Ile Asn Ser Gly Gly Ser Ala Arg Tyr Ala Ser
   65                  70                  75                  80
   Trp Ala Glu Gly Arg Phe Thr Ile Ser Arg Thr Ser Thr Thr Val Asp
                   85                  90                  95
   Leu Lys Met Thr Ser Leu Thr Thr Glu Asp Thr Ala Thr Tyr Phe Cys
               100                 105                 110
   Val Arg Gly Gly Ala Val Trp Ser Ile His Ser Phe Asp Pro
           115                 120                 125
<210> 669
   <211> 366
   <212> DNA
   <213> Oryctolagus cuniculus
<400> 669
   atggacacga gggcccccac tcagctgctg gggctcctgc tgctctggct cccaggtgcc      60
   agatgtgcct atgatatgac ccagactcca gcctctgtgg aggtagctgt gggaggcaca     120
   gtcaccatca attgccaggc cagtgagacc atttacagtt ggttatcctg gtatcagcag     180
   aagccagggc agcctcccaa gctcctgatc taccaggcat ccgatctggc atctggggtc     240
   ccatcgcgat tcagcggcag tggggctggg acagagtaca ctctcaccat cagcggcgtg     300
   cagtgtgacg atgctgccac ttactactgt caacagggtt atagtggtag taatgttgat     360
   aatgtt                                                                366
<210> 670
   <211> 378
   <212> DNA
   <213> Oryctolagus cuniculus
<400> 670
   atggagactg ggctgcgctg gcttctcctg gtcgctgtgc tcaaaggtgt ccagtgtcag       60
   gagcagctga aggagtccgg gggtcgcctg gtcacgcctg ggacacccct gacacttacc      120
   tgcacagcct ctggattctc cctcaatgac catgcaatgg gctgggtccg ccaggctcca      180
   gggaaggggc tggaatacat cggattcatt aatagtggtg gtagcgcacg ctacgcgagc      240
   tgggcagaag gccgattcac catctccaga acctcgacca cggtggatct gaaaatgacc      300
   agtctgacaa ccgaggacac ggccacctat ttctgtgtca gagggggtgc tgtttggagt      360
   attcatagtt ttgatccc                                                    378
<210> 671
   <211> 123
   <212> PRT
   <213> Oryctolagus cuniculus
<400> 671
   Met Asp Thr Arg Ala Pro Thr Gln Leu Leu Gly Leu Leu Leu Leu Trp
   1               5                   10                  15
   Leu Pro Gly Ala Thr Phe Ala Ala Val Leu Thr Gln Thr Pro Ser Pro
               20                  25                  30
   Val Ser Ala Ala Val Gly Gly Thr Val Ser Ile Ser Cys Gln Ala Ser
           35                  40                  45
   Gln Ser Val Tyr Asp Asn Asn Tyr Leu Ser Trp Phe Gln Gln Lys Pro
       50                  55                  60
   Gly Gln Pro Pro Lys Leu Leu Ile Tyr Gly Ala Ser Thr Leu Ala Ser
   65                  70                  75                  80
   Gly Val Pro Ser Arg Phe Val Gly Ser Gly Ser Gly Thr Gln Phe Thr
                   85                  90                  95
   Leu Thr Ile Thr Asp Val Gln Cys Asp Asp Ala Ala Thr Tyr Tyr Cys
               100                 105                 110
   Ala Gly Val Tyr Asp Asp Asp Ser Asp Asn Ala
           115                 120
<210> 672
   <211> 125
   <212> PRT
   <213> Oryctolagus cuniculus
<400> 672
   Met Glu Thr Gly Leu Arg Trp Leu Leu Leu Val Ala Val Leu Lys Gly
   1               5                   10                  15
   Val Gln Cys Gln Ser Leu Glu Glu Ser Gly Gly Arg Leu Val Thr Pro
               20                  25                  30
   Gly Thr Pro Leu Thr Leu Thr Cys Thr Ala Ser Gly Phe Ser Leu Ser
           35                  40                  45
   Val Tyr Tyr Met Asn Trp Val Arg Gln Ala Pro Gly Lys Gly Leu Glu
       50                  55                  60
   Trp Ile Gly Phe Ile Thr Met Ser Asp Asn Ile Asn Tyr Ala Ser Trp
   65                  70                  75                  80
   Ala Lys Gly Arg Phe Thr Ile Ser Lys Thr Ser Thr Thr Val Asp Leu
                   85                  90                  95
   Lys Met Thr Ser Pro Thr Thr Glu Asp Thr Ala Thr Tyr Phe Cys Ala
               100                 105                 110
   Arg Ser Arg Gly Trp Gly Thr Met Gly Arg Leu Asp Leu
           115                 120                 125
<210> 673
   <211> 369
   <212> DNA
   <213> Oryctolagus cuniculus
<400> 673
   atggacacga gggcccccac tcagctgctg gggctcctgc tgctctggct cccaggtgcc      60
   acatttgccg ccgtgctgac ccagactcca tctcccgtgt ctgcagctgt gggaggcaca     120
   gtcagcatca gttgccaggc cagtcagagt gtttatgaca acaactactt atcctggttt     180
   cagcagaaac cagggcagcc tcccaagctc ctgatctatg gtgcatccac tctggcatct     240
   ggggtcccat cgcggttcgt gggcagtgga tctgggacac agttcactct caccatcaca     300
   gacgtgcagt gtgacgatgc tgccacttac tattgtgcag gcgtttatga tgatgatagt     360
   gataatgcc                                                             369
<210> 674
   <211> 375
   <212> DNA
   <213> Oryctolagus cuniculus
<400> 674
   atggagactg ggctgcgctg gcttctcctg gtggctgtgc tcaaaggtgt ccagtgtcag        60
   tcgctggagg agtccggggg tcgcctggtc acccctggga cacccctgac actcacctgc       120
   acagcctctg gattctccct cagtgtctac tacatgaact gggtccgcca ggctccaggg       180
   aaggggctgg aatggatcgg attcattaca atgagtgata atataaatta cgcgagctgg       240
   gcgaaaggcc gattcaccat ctccaaaacc tcgaccacgg tggatctgaa aatgaccagt       300
   ccgacaaccg aggacacggc cacctatttc tgtgccagga gtcgtggctg gggtacaatg        360
   ggtcggttgg atctc                                                        375
<210> 675
   <211> 123
   <212> PRT
   <213> Oryctolagus cuniculus
<400> 675
   Met Asp Thr Arg Ala Pro Thr Gln Leu Leu Gly Leu Leu Leu Leu Trp
   1               5                   10                  15
   Leu Pro Gly Ala Ile Cys Asp Pro Val Leu Thr Gln Thr Pro Ser Pro
               20                  25                  30
   Val Ser Ala Pro Val Gly Gly Thr Val Ser Ile Ser Cys Gln Ala Ser
           35                  40                  45
   Gln Ser Val Tyr Glu Asn Asn Tyr Leu Ser Trp Phe Gln Gln Lys Pro
       50                  55                  60
   Gly Gln Pro Pro Lys Leu Leu Ile Tyr Gly Ala Ser Thr Leu Asp Ser
   65                  70                  75                  80
   Gly Val Pro Ser Arg Phe Lys Gly Ser Gly Ser Gly Thr Gln Phe Thr
                   85                  90                  95
   Leu Thr Ile Thr Asp Val Gln Cys Asp Asp Ala Ala Thr Tyr Tyr Cys
               100                 105                 110
   Ala Gly Val Tyr Asp Asp Asp Ser Asp Asp Ala
           115                 120
<210> 676
   <211> 126
   <212> PRT
   <213> Oryctolagus cuniculus
<400> 676
   Met Glu Thr Gly Leu Arg Trp Leu Leu Leu Val Ala Val Leu Lys Gly
   1               5                   10                  15
   Val Gln Cys Gln Glu Gln Leu Lys Glu Ser Gly Gly Gly Leu Val Thr
               20                  25                  30
   Pro Gly Gly Thr Leu Thr Leu Thr Cys Thr Ala Ser Gly Phe Ser Leu
           35                  40                  45
   Asn Ala Tyr Tyr Met Asn Trp Val Arg Gln Ala Pro Gly Lys Gly Leu
       50                  55                  60
   Glu Trp Ile Gly Phe Ile Thr Leu Asn Asn Asn Val Ala Tyr Ala Asn
   65                  70                  75                  80
   Trp Ala Lys Gly Arg Phe Thr Phe Ser Lys Thr Ser Thr Thr Val Asp
                   85                  90                  95
   Leu Lys Met Thr Ser Pro Thr Pro Glu Asp Thr Ala Thr Tyr Phe Cys
               100                 105                 110
   Ala Arg Ser Arg Gly Trp Gly Ala Met Gly Arg Leu Asp Leu
           115                 120                 125
<210> 677
   <211> 369
   <212> DNA
   <213> Oryctolagus cuniculus
<400> 677
   atggacacga gggcccccac tcagctgctg gggctcctgc tgctctggct cccaggtgcc      60
   atatgtgacc ctgtgctgac ccagactcca tctcccgtat ctgcacctgt gggaggcaca     120
   gtcagcatca gttgccaggc cagtcagagt gtttatgaga acaactattt atcctggttt     180
   cagcagaaac cagggcagcc tcccaagctc ctgatctatg gtgcatccac tctggattct     240
   ggggtcccat cgcggttcaa aggcagtgga tctgggacac agttcactct caccattaca     300
   gacgtgcagt gtgacgatgc tgccacttac tattgtgcag gcgtttatga tgatgatagt     360
   gatgatgcc                                                             369
<210> 678
   <211> 378
   <212> DNA
   <213> Oryctolagus cuniculus
<400> 678
   atggagactg ggctgcgctg gcttctcctg gtggctgtgc tcaaaggtgt ccagtgtcag      60
   gagcagctga aggagtccgg aggaggcctg gtaacgcctg gaggaaccct gacactcacc     120
   tgcacagcct ctggattctc cctcaatgcc tactacatga actgggtccg ccaggctcca     180
   gggaaggggc tggaatggat cggattcatt actctgaata ataatgtagc ttacgcgaac     240
   tgggcgaaag gccgattcac cttctccaaa acctcgacca cggtggatct gaaaatgacc     300
   agtccgacac ccgaggacac ggccacctat ttctgtgcca ggagtcgtgg ctggggtgca     360
   atgggtcggt tggatctc                                                   378
<210> 679
   <211> 122
   <212> PRT
   <213> Oryctolagus cuniculus
<400> 679
   Met Asp Thr Arg Ala Pro Thr Gln Leu Leu Gly Leu Leu Leu Leu Trp
   1               5                   10                  15
   Leu Pro Gly Ala Thr Phe Ala Gln Val Leu Thr Gln Thr Pro Ser Pro
               20                  25                  30
   Val Ser Ala Ala Val Gly Gly Thr Val Thr Ile Asn Cys Gln Ala Ser
           35                  40                  45
   Gln Ser Val Asp Asp Asn Asn Trp Leu Gly Trp Tyr Gln Gln Lys Arg
       50                  55                  60
   Gly Gln Pro Pro Lys Tyr Leu Ile Tyr Ser Ala Ser Thr Leu Ala Ser
   65                  70                  75                  80
   Gly Val Pro Ser Arg Phe Lys Gly Ser Gly Ser Gly Thr Gln Phe Thr
                   85                  90                  95
   Leu Thr Ile Ser Asp Leu Glu Cys Asp Asp Ala Ala Thr Tyr Tyr Cys
               100                 105                 110
   Ala Gly Gly Phe Ser Gly Asn Ile Phe Ala
           115                 120
<210> 680
   <211> 122
   <212> PRT
   <213> Oryctolagus cuniculus
<400> 680
   Met Glu Thr Gly Leu Arg Trp Leu Leu Leu Val Ala Val Leu Lys Gly
   1 5 10 15
   Val Gln Cys Gln Ser Val Glu Glu Ser Gly Gly Arg Leu Val Thr Pro
   20 25 30
   Gly Thr Pro Leu Thr Leu Thr Cys Thr Val Ser Gly Phe Ser Leu Ser
   35 40 45
   Ser Tyr Ala Met Ser Trp Val Arg Gln Ala Pro Gly Lys Gly Leu Glu
   50 55 60
   Trp Ile Gly Ile Ile Gly Gly Phe Gly Thr Thr Tyr Tyr Ala Thr Trp
   65 70 75 80
   Ala Lys Gly Arg Phe Thr Ile Ser Lys Thr Ser Thr Thr Val Asp Leu
   85 90 95
   Arg Ile Thr Ser Pro Thr Thr Glu Asp Thr Ala Thr Tyr Phe Cys Ala
   100 105 110
   Arg Gly Gly Pro Gly Asn Gly Gly Asp Ile
   115 120
<210> 681
   <211> 366
   <212> DNA
   <213> Oryctolagus cuniculus
<400> 681
   atggacacga gggcccccac tcagctgctg gggctcctgc tgctctggct cccaggtgcc 60
   acatttgccc aagtgctgac ccagactcca tcgcctgtgt ctgcagctgt gggaggcaca 120
   gtcaccatca actgccaggc cagtcagagt gttgatgata acaactggtt aggctggtat 180
   cagcagaaac gagggcagcc tcccaagtac ctgatctatt ctgcatccac tctggcatct 240
   ggggtcccat cgcggttcaa aggcagtgga tctgggacac agttcactct caccatcagc 300
   gacctggagt gtgacgatgc tgccacttac tactgtgcag gcggttttag tggtaatatc 360
   tttgct 366
<210> 682
   <211> 366
   <212> DNA
   <213> Oryctolagus cuniculus
<400> 682
   atggagactg ggctgcgctg gcttctcctg gtcgctgtgc tcaaaggtgt ccagtgtcag       60
   tcggtggagg agtccggggg tcgcctggtc acgcctggga cacccctgac actcacctgc      120
   acagtctctg gcttctccct cagtagctat gcaatgagct gggtccgcca ggctccagga      180
   aaggggctgg agtggatcgg aatcattggt ggttttggta ccacatacta cgcgacctgg      240
   gcgaaaggcc gattcaccat ctccaaaacc tcgaccacgg tggatctgag aatcaccagt      300
   ccgacaaccg aggacacggc cacctatttc tgtgccagag gtggtcctgg taatggtggt      360
   gacatc                                                                 366
<210> 683
   <211> 122
   <212> PRT
   <213> Oryctolagus cuniculus
<400> 683
   Met Asp Thr Arg Ala Pro Thr Gln Leu Leu Gly Leu Leu Leu Leu Trp
   1               5                   10                  15
   Leu Pro Gly Ala Thr Phe Ala Ala Val Leu Thr Gln Thr Pro Ser Pro
               20                  25                  30
   Val Ser Val Pro Val Gly Gly Thr Val Thr Ile Lys Cys Gln Ser Ser
           35                  40                  45
   Gln Ser Val Tyr Asn Asn Phe Leu Ser Trp Tyr Gln Gln Lys Pro Gly
       50                  55                  60
   Gln Pro Pro Lys Leu Leu Ile Tyr Gln Ala Ser Lys Leu Ala Ser Gly
   65                  70                  75                  80
   Val Pro Asp Arg Phe Ser Gly Ser Gly Ser Gly Thr Gln Phe Thr Leu
                   85                  90                  95
   Thr Ile Ser Gly Val Gln Cys Asp Asp Ala Ala Thr Tyr Tyr Cys Leu
               100                 105                 110
   Gly Gly Tyr Asp Asp Asp Ala Asp Asn Ala
           115                 120
<210> 684
   <211> 128
   <212> PRT
   <213> Oryctolagus cuniculus
<400> 684
   Met Glu Thr Gly Leu Arg Trp Leu Leu Leu Val Ala Val Leu Lys Gly
   1               5                   10                  15
   Val Gln Cys Gln Ser Val Glu Glu Ser Gly Gly Arg Leu Val Thr Pro
               20                  25                  30
   Gly Thr Pro Leu Thr Leu Thr Cys Thr Val Ser Gly Ile Asp Leu Ser
           35                  40                  45
   Asp Tyr Ala Met Ser Trp Val Arg Gln Ala Pro Gly Lys Gly Leu Glu
       50                  55                  60
   Trp Ile Gly Ile Ile Tyr Ala Gly Ser Gly Ser Thr Trp Tyr Ala Ser
   65                  70                  75                  80
   Trp Ala Lys Gly Arg Phe Thr Ile Ser Lys Thr Ser Thr Thr Val Asp
                   85                  90                  95
   Leu Lys Ile Thr Ser Pro Thr Thr Glu Asp Thr Ala Thr Tyr Phe Cys
               100                 105                 110
   Ala Arg Asp Gly Tyr Asp Asp Tyr Gly Asp Phe Asp Arg Leu Asp Leu
           115                 120                 125
<210> 685
   <211> 366
   <212> DNA
   <213> Oryctolagus cuniculus
<400> 685
   atggacacga gggcccccac tcagctgctg gggctcctgc tgctctggct cccaggtgcc      60
   acatttgcag ccgtgctgac ccagacacca tcgcccgtgt ctgtacctgt gggaggcaca     120
   gtcaccatca agtgccagtc cagtcagagt gtttataata atttcttatc gtggtatcag     180
   cagaaaccag ggcagcctcc caagctcctg atctaccagg catccaaact ggcatctggg     240
   gtcccagata ggttcagcgg cagtggatct gggacacagt tcactctcac catcagcggc     300
   gtgcagtgtg acgatgctgc cacttactac tgtctaggcg gttatgatga tgatgctgat     360
   aatgct                                                                366
<210> 686
   <211> 384
   <212> DNA
   <213> Oryctolagus cuniculus
<400> 686
   atggagactg ggctgcgctg gcttctcctg gtcgctgtgc tcaaaggtgt ccagtgtcag 60
   tcggtggagg agtccggggg tcgcctggtc acgcctggga cacccctgac gctcacctgc 120
   acagtctctg gaatcgacct cagtgactat gcaatgagct gggtccgcca ggctccaggg 180
   aaggggctgg aatggatcgg aatcatttat gctggtagtg gtagcacatg gtacgcgagc 240
   tgggcgaaag gccgattcac catctccaaa acctcgacca cggtggatct gaaaatcacc 300
   agtccgacaa ccgaggacac ggccacctat ttctgtgcca gagatggata cgatgactat 360
   ggtgatttcg atcgattgga tctc 384
<210> 687
   <211> 122
   <212> PRT
   <213> Oryctolagus cuniculus
<400> 687
   Met Asp Thr Arg Ala Pro Thr Gln Leu Leu Gly Leu Leu Leu Leu Trp
   1 5 10 15
   Leu Pro Gly Ala Arg Cys Ala Tyr Asp Met Thr Gln Thr Pro Ala Ser
   20 25 30
   Val Ser Ala Ala Val Gly Gly Thr Val Thr Ile Lys Cys Gln Ala Ser
   35 40 45
   Gln Ser Ile Asn Asn Glu Leu Ser Trp Tyr Gln Gln Lys Ser Gly Gln
   50 55 60
   Arg Pro Lys Leu Leu Ile Tyr Arg Ala Ser Thr Leu Ala Ser Gly Val
   65 70 75 80
   Ser Ser Arg Phe Lys Gly Ser Gly Ser Gly Thr Glu Phe Thr Leu Thr
   85 90 95
   Ile Ser Asp Leu Glu Cys Ala Asp Ala Ala Thr Tyr Tyr Cys Gln Gln
   100 105 110
   Gly Tyr Ser Leu Arg Asn Ile Asp Asn Ala
   115 120
<210> 688
   <211> 125
   <212> PRT
   <213> Oryctolagus cuniculus
<400> 688
   Met Glu Thr Gly Leu Arg Trp Leu Leu Leu Val Ala Val Leu Ser Gly
   1               5                   10                  15
   Val Gln Cys Gln Ser Leu Glu Glu Ser Gly Gly Arg Leu Val Thr Pro
               20                  25                  30
   Gly Thr Pro Leu Thr Leu Thr Cys Thr Ala Ser Gly Phe Ser Leu Ser
           35                  40                  45
   Asn Tyr Tyr Met Thr Trp Val Arg Gln Ala Pro Gly Lys Gly Leu Glu
       50                  55                  60
   Trp Ile Gly Met Ile Tyr Gly Ser Asp Glu Thr Ala Tyr Ala Asn Trp
   65                  70                  75                  80
   Ala Ile Gly Arg Phe Thr Ile Ser Lys Thr Ser Thr Thr Val Asp Leu
                   85                  90                  95
   Lys Met Thr Ser Leu Thr Ala Ala Asp Thr Ala Thr Tyr Phe Cys Ala
               100                 105                 110
   Arg Asp Asp Ser Ser Asp Trp Asp Ala Lys Phe Asn Leu
           115                 120                 125
<210> 689
   <211> 366
   <212> DNA
   <213> Oryctolagus cuniculus
<400> 689
   atggacacga gggcccccac tcagctgctg gggctcctgc tgctctggct cccaggtgcc       60
   agatgtgcct atgatatgac ccagactcca gcctcggtgt ctgcagctgt gggaggcaca      120
   gtcaccatca aatgccaggc cagtcagagc attaacaatg aattatcctg gtatcagcag      180
   aaatcagggc agcgtcccaa gctcctgatc tatagggcat ccactctggc atctggggtc      240
   tcatcgcggt tcaaaggcag tggatctggg acagagttca ctctcaccat cagcgacctg      300
   gagtgtgccg atgctgccac ttactactgt caacagggtt atagtctgag gaatattgat      360
   aatgct                                                                 366
<210> 690
   <211> 375
   <212> DNA
   <213> Oryctolagus cuniculus
<400> 690
   atggagactg ggctgcgctg gcttctcctg gtcgctgtgc tctcaggtgt ccagtgtcag        60
   tcgctggagg agtccggggg tcgcctggtc acgcctggga cacccctgac actcacctgc       120
   acagcctctg gattctccct cagtaactac tacatgacct gggtccgcca ggctccaggg       180
   aaggggctgg aatggatcgg aatgatttat ggtagtgatg aaacagccta cgcgaactgg       240
   gcgataggcc gattcaccat ctccaaaacc tcgaccacgg tggatctgaa aatgaccagt       300
   ctgacagccg cggacacggc cacctatttc tgtgccagag atgatagtag tgactgggat       360
   gcaaaattta acttg                                                        375
<210> 691
   <211> 450
   <212> PRT
   <213> Oryctolagus cuniculus
<400> 691
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Ser Leu Ser Asn Tyr
               20                  25                  30
   Tyr Met Thr Trp Val Arg Gln Ala Pro Gly Lys Gly Leu Glu Trp Val
           35                  40                  45
   Gly Met Ile Tyr Gly Ser Asp Glu Thr Ala Tyr Ala Asn Trp Ala Ile
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Arg Asp Asp Ser Ser Asp Trp Asp Ala Lys Phe Asn Leu Trp Gly Gln
   100 105 110
   Gly Thr Leu Val Thr Val Ser Ser Ala Ser Thr Lys Gly Pro Ser Val
   115 120 125
   Phe Pro Leu Ala Pro Ser Ser Lys Ser Thr Ser Gly Gly Thr Ala Ala
   130 135 140
   Leu Gly Cys Leu Val Lys Asp Tyr Phe Pro Glu Pro Val Thr Val Ser
   145 150 155 160
   Trp Asn Ser Gly Ala Leu Thr Ser Gly Val His Thr Phe Pro Ala Val
   165 170 175
   Leu Gln Ser Ser Gly Leu Tyr Ser Leu Ser Ser Val Val Thr Val Pro
   180 185 190
   Ser Ser Ser Leu Gly Thr Gln Thr Tyr Ile Cys Asn Val Asn His Lys
   195 200 205
   Pro Ser Asn Thr Lys Val Asp Lys Arg Val Glu Pro Lys Ser Cys Asp
   210 215 220
   Lys Thr His Thr Cys Pro Pro Cys Pro Ala Pro Glu Leu Leu Gly Gly
   225 230 235 240
   Pro Ser Val Phe Leu Phe Pro Pro Lys Pro Lys Asp Thr Leu Met Ile
   245 250 255
   Ser Arg Thr Pro Glu Val Thr Cys Val Val Val Asp Val Ser His Glu
   260 265 270
   Asp Pro Glu Val Lys Phe Asn Trp Tyr Val Asp Gly Val Glu Val His
   275 280 285
   Asn Ala Lys Thr Lys Pro Arg Glu Glu Gln Tyr Ala Ser Thr Tyr Arg
   290 295 300
   Val Val Ser Val Leu Thr Val Leu His Gln Asp Trp Leu Asn Gly Lys
   305 310 315 320
   Glu Tyr Lys Cys Lys Val Ser Asn Lys Ala Leu Pro Ala Pro Ile Glu
   325 330 335
   Lys Thr Ile Ser Lys Ala Lys Gly Gln Pro Arg Glu Pro Gln Val Tyr
   340 345 350
   Thr Leu Pro Pro Ser Arg Asp Glu Leu Thr Lys Asn Gln Val Ser Leu
   355 360 365
   Thr Cys Leu Val Lys Gly Phe Tyr Pro Ser Asp Ile Ala Val Glu Trp
   370 375 380
   Glu Ser Asn Gly Gln Pro Glu Asn Asn Tyr Lys Thr Thr Pro Pro Val
   385 390 395 400
   Leu Asp Ser Asp Gly Ser Phe Phe Leu Tyr Ser Lys Leu Thr Val Asp
   405 410 415
   Lys Ser Arg Trp Gln Gln Gly Asn Val Phe Ser Cys Ser Val Met His
   420 425 430
   Glu Ala Leu His Asn His Tyr Thr Gln Lys Ser Leu Ser Leu Ser Pro
   435 440 445
   Gly Lys
   450
<210> 692
   <211> 450
   <212> PRT
   <213> Oryctolagus cuniculus
<400> 692
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Ser Leu Ser Asn Tyr
   20 25 30
   Tyr Met Thr Trp Val Arg Gln Ala Pro Gly Lys Gly Leu Glu Trp Val
   35 40 45
   Gly Met Ile Tyr Gly Ser Asp Glu Thr Ala Tyr Ala Asn Ser Ala Ile
   50 55 60
   Gly Arg Phe Thr Ile Ser Arg Asp Asn Ser Lys Asn Thr Leu Tyr Leu
   65 70 75 80
   Gln Met Asn Ser Leu Arg Ala Glu Asp Thr Ala Val Tyr Tyr Cys Ala
   85 90 95
   Arg Asp Asp Ser Ser Asp Trp Asp Ala Lys Phe Asn Leu Trp Gly Gln
   100 105 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Arg Val Glu Pro Lys Ser Cys Asp
       210                 215                 220
   Lys Thr His Thr Cys Pro Pro Cys Pro Ala Pro Glu Leu Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Ala Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Pro Ala Pro Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Ser Arg Asp Glu Leu Thr Lys Asn Gln Val Ser Leu
           355                 360                 365
   Thr Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Tyr Ser Lys Leu Thr Val Asp
                   405                 410                 415
   Lys Ser Arg Trp Gln Gln Gly Asn Val Phe Ser Cys Ser Val Met His
               420                 425                 430
   Glu Ala Leu His Asn His Tyr Thr Gln Lys Ser Leu Ser Leu Ser Pro
           435                 440                 445
   Gly Lys
       450
<210> 693
   <211> 217
   <212> PRT
   <213> Oryctolagus cuniculus
<400> 693
   Asp Ile Gln Met Thr Gln Ser Pro Ser Thr Leu Ser Ala Ser Val Gly
   1               5                   10                  15
   Asp Arg Val Thr Ile Thr Cys Gln Ala Ser Gln Ser Ile Asn Asn Glu
               20                  25                  30
   Leu Ser Trp Tyr Gln Gln Lys Pro Gly Lys Ala Pro Lys Leu Leu Ile
           35                  40                  45
   Tyr Arg Ala Ser Thr Leu Ala Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Asp Asp Phe Ala Thr Tyr Tyr Cys Gln Gln Gly Tyr Ser Leu Arg Asn
                   85                  90                  95
   Ile Asp Asn Ala Phe Gly Gly Gly Thr Lys Val Glu Ile Lys Arg Thr
               100                 105                 110
   Val Ala Ala Pro Ser Val Phe Ile Phe Pro Pro Ser Asp Glu Gln Leu
           115                 120                 125
   Lys Ser Gly Thr Ala Ser Val Val Cys Leu Leu Asn Asn Phe Tyr Pro
       130                 135                 140
   Arg Glu Ala Lys Val Gln Trp Lys Val Asp Asn Ala Leu Gln Ser Gly
   145                 150                 155                 160
   Asn Ser Gln Glu Ser Val Thr Glu Gln Asp Ser Lys Asp Ser Thr Tyr
                   165                 170                 175
   Ser Leu Ser Ser Thr Leu Thr Leu Ser Lys Ala Asp Tyr Glu Lys His
               180                 185                 190
   Lys Val Tyr Ala Cys Glu Val Thr His Gln Gly Leu Ser Ser Pro Val
           195                 200                 205
   Thr Lys Ser Phe Asn Arg Gly Glu Cys
       210                 215
<210> 694
   <211> 33
   <212> DNA
   <213> Oryctolagus cuniculus
<400> 694
   caggccagtc agagcattaa caatgagtta tcc 33
<210> 695
   <211> 36
   <212> DNA
   <213> Oryctolagus cuniculus
<400> 695
   caacagggtt atagtctgag gaacattgat aatgct 36
<210> 696
   <211> 48
   <212> DNA
   <213> Oryctolagus cuniculus
<400> 696
   atcatctatg gtagtgatga aaccgcctac gctacctccg ctataggc 48
<210> 697
   <211> 36
   <212> DNA
   <213> Oryctolagus cuniculus
<400> 697
   gatgatagta gtgactggga tgcaaagttc aacttg 36
<210> 698
   <211> 336
   <212> DNA
   <213> Oryctolagus cuniculus
<400> 698
   gctatccaga tgacccagtc tccttcctcc ctgtctgcat ctgtaggaga cagagtcacc 60
   atcacttgcc aggccagtca gagcattaac aatgagttat cctggtatca gcagaaacca 120
   gggaaagccc ctaagctcct gatctatagg gcatccactc tggcatctgg ggtcccatca 180
   aggttcagcg gcagtggatc tgggacagac ttcactctca ccatcagcag cctgcagcct 240
   gatgattttg caacttatta ctgccaacag ggttatagtc tgaggaacat tgataatgct 300
   ttcggcggag ggaccaaggt ggaaatcaaa cgtacg 336
<210> 699
   <211> 112
   <212> PRT
   <213> Oryctolagus cuniculus
<400> 699
   Ala Ile Gln Met Thr Gln Ser Pro Ser Ser Leu Ser Ala Ser Val Gly
   1 5 10 15
   Asp Arg Val Thr Ile Thr Cys Gln Ala Ser Gln Ser Ile Asn Asn Glu
   20 25 30
   Leu Ser Trp Tyr Gln Gln Lys Pro Gly Lys Ala Pro Lys Leu Leu Ile
   35 40 45
   Tyr Arg Ala Ser Thr Leu Ala Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Asp Asp Phe Ala Thr Tyr Tyr Cys Gln Gln Gly Tyr Ser Leu Arg Asn
   85 90 95
   Ile Asp Asn Ala Phe Gly Gly Gly Thr Lys Val Glu Ile Lys Arg Thr
   100 105 110
<210> 700
   <211> 360
   <212> DNA
   <213> Oryctolagus cuniculus
<400> 700
   gaggtgcagc tggtggagtc tgggggaggc ttggtccagc ctggggggtc cctgagactc 60
   tcctgtgcag cctctggatt ctccctcagt aactactacg tgacctgggt ccgtcaggct 120
   ccagggaagg ggctggagtg ggtcggcatc atctatggta gtgatgaaac cgcctacgct 180
   acctccgcta taggccgatt caccatctcc agagacaatt ccaagaacac cctgtatctt 240
   caaatgaaca gcctgagagc tgaggacact gctgtgtatt actgtgctag agatgatagt 300
   agtgactggg atgcaaagtt caacttgtgg ggccaaggga ccctcgtcac cgtctcgagc 360
<210> 701
   <211> 651
   <212> DNA
   <213> Oryctolagus cuniculus
<400> 701
   gctatccaga tgacccagtc tccttcctcc ctgtctgcat ctgtaggaga cagagtcacc 60
   atcacttgcc aggccagtca gagcattaac aatgagttat cctggtatca gcagaaacca 120
   gggaaagccc ctaagctcct gatctatagg gcatccactc tggcatctgg ggtcccatca 180
   aggttcagcg gcagtggatc tgggacagac ttcactctca ccatcagcag cctgcagcct 240
   gatgattttg caacttatta ctgccaacag ggttatagtc tgaggaacat tgataatgct 300
   ttcggcggag ggaccaaggt ggaaatcaaa cgtacggtgg ctgcaccatc tgtcttcatc 360
   ttcccgccat ctgatgagca gttgaaatct ggaactgcct ctgttgtgtg cctgctgaat 420
   aacttctatc ccagagaggc caaagtacag tggaaggtgg ataacgccct ccaatcgggt 480
   aactcccagg agagtgtcac agagcaggac agcaaggaca gcacctacag cctcagcagc 540
   accctgacgc tgagcaaagc agactacgag aaacacaaag tctacgcctg cgaagtcacc 600
   catcagggcc tgagctcgcc cgtcacaaag agcttcaaca ggggagagtg t 651
<210> 702
   <211> 217
   <212> PRT
   <213> Oryctolagus cuniculus
<400> 702
   Ala Ile Gln Met Thr Gln Ser Pro Ser Ser Leu Ser Ala Ser Val Gly
   1               5                   10                  15
   Asp Arg Val Thr Ile Thr Cys Gln Ala Ser Gln Ser Ile Asn Asn Glu
               20                  25                  30
   Leu Ser Trp Tyr Gln Gln Lys Pro Gly Lys Ala Pro Lys Leu Leu Ile
           35                  40                  45
   Tyr Arg Ala Ser Thr Leu Ala Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Asp Asp Phe Ala Thr Tyr Tyr Cys Gln Gln Gly Tyr Ser Leu Arg Asn
                   85                  90                  95
   Ile Asp Asn Ala Phe Gly Gly Gly Thr Lys Val Glu Ile Lys Arg Thr
               100                 105                 110
   Val Ala Ala Pro Ser Val Phe Ile Phe Pro Pro Ser Asp Glu Gln Leu
           115                 120                 125
   Lys Ser Gly Thr Ala Ser Val Val Cys Leu Leu Asn Asn Phe Tyr Pro
       130                 135                 140
   Arg Glu Ala Lys Val Gln Trp Lys Val Asp Asn Ala Leu Gln Ser Gly
   145                 150                 155                 160
   Asn Ser Gln Glu Ser Val Thr Glu Gln Asp Ser Lys Asp Ser Thr Tyr
                   165                 170                 175
   Ser Leu Ser Ser Thr Leu Thr Leu Ser Lys Ala Asp Tyr Glu Lys His
               180                 185                 190
   Lys Val Tyr Ala Cys Glu Val Thr His Gln Gly Leu Ser Ser Pro Val
           195                 200                 205
   Thr Lys Ser Phe Asn Arg Gly Glu Cys
       210                 215
<210> 703
   <211> 1350
   <212> DNA
   <213> Oryctolagus cuniculus
<400> 703
   gaggtgcagc tggtggagtc tgggggaggc ttggtccagc ctggggggtc cctgagactc      60
   tcctgtgcag cctctggatt ctccctcagt aactactacg tgacctgggt ccgtcaggct     120
   ccagggaagg ggctggagtg ggtcggcatc atctatggta gtgatgaaac cgcctacgct     180
   acctccgcta taggccgatt caccatctcc agagacaatt ccaagaacac cctgtatctt     240
   caaatgaaca gcctgagagc tgaggacact gctgtgtatt actgtgctag agatgatagt     300
   agtgactggg atgcaaagtt caacttgtgg ggccaaggga ccctcgtcac cgtctcgagc     360
   gcctccacca agggcccatc ggtcttcccc ctggcaccct cctccaagag cacctctggg     420
   ggcacagcgg ccctgggctg cctggtcaag gactacttcc ccgaaccggt gacggtgtcg     480
   tggaactcag gcgccctgac cagcggcgtg cacaccttcc cggctgtcct acagtcctca     540
   ggactctact ccctcagcag cgtggtgacc gtgccctcca gcagcttggg cacccagacc     600
   tacatctgca acgtgaatca caagcccagc aacaccaagg tggacaagag agttgagccc     660
   aaatcttgtg acaaaactca cacatgccca ccgtgcccag cacctgaact cctgggggga     720
   ccgtcagtct tcctcttccc cccaaaaccc aaggacaccc tcatgatctc ccggacccct     780
   gaggtcacat gcgtggtggt ggacgtgagc cacgaagacc ctgaggtcaa gttcaactgg     840
   tacgtggacg gcgtggaggt gcataatgcc aagacaaagc cgcgggagga gcagtacgcc     900
   agcacgtacc gtgtggtcag cgtcctcacc gtcctgcacc aggactggct gaatggcaag     960
   gagtacaagt gcaaggtctc caacaaagcc ctcccagccc ccatcgagaa aaccatctcc    1020
   aaagccaaag ggcagccccg agaaccacag gtgtacaccc tgcccccatc ccgggaggag    1080
   atgaccaaga accaggtcag cctgacctgc ctggtcaaag gcttctatcc cagcgacatc    1140
   gccgtggagt gggagagcaa tgggcagccg gagaacaact acaagaccac gcctcccgtg    1200
   ctggactccg acggctcctt cttcctctac agcaagctca ccgtggacaa gagcaggtgg    1260
   cagcagggga acgtcttctc atgctccgtg atgcatgagg ctctgcacaa ccactacacg    1320
   cagaagagcc tctccctgtc tccgggtaaa                                     1350
<210> 704
   <211> 450
   <212> PRT
   <213> Oryctolagus cuniculus
<400> 704
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Ser Leu Ser Asn Tyr
               20                  25                  30
   Tyr Val Thr Trp Val Arg Gln Ala Pro Gly Lys Gly Leu Glu Trp Val
           35                  40                  45
   Gly Ile Ile Tyr Gly Ser Asp Glu Thr Ala Tyr Ala Thr Ser Ala Ile
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Arg Asp Asp Ser Ser Asp Trp Asp Ala Lys Phe Asn Leu Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Arg Val Glu Pro Lys Ser Cys Asp
       210                 215                 220
   Lys Thr His Thr Cys Pro Pro Cys Pro Ala Pro Glu Leu Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Ala Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Pro Ala Pro Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Ser Arg Glu Glu Met Thr Lys Asn Gln Val Ser Leu
           355                 360                 365
   Thr Cys Leu Val Lys Gly Phe Tyr Pro Ser Asp Ile Ala Val Glu Trp
   370 375 380
   Glu Ser Asn Gly Gln Pro Glu Asn Asn Tyr Lys Thr Thr Pro Pro Val
   385 390 395 400
   Leu Asp Ser Asp Gly Ser Phe Phe Leu Tyr Ser Lys Leu Thr Val Asp
   405 410 415
   Lys Ser Arg Trp Gln Gln Gly Asn Val Phe Ser Cys Ser Val Met His
   420 425 430
   Glu Ala Leu His Asn His Tyr Thr Gln Lys Ser Leu Ser Leu Ser Pro
   435 440 445
   Gly Lys
   450
<210> 705
   <211> 705
   <212> DNA
   <213> Oryctolagus cuniculus
<400> 705
   atgaagtggg taacctttat ttcccttctg tttctcttta gcagcgctta ttccgctatc 60
   cagatgaccc agtctccttc ctccctgtct gcatctgtag gagacagagt caccatcact 120
   tgccaggcca gtcagagcat taacaatgag ttatcctggt atcagcagaa accagggaaa 180
   gcccctaagc tcctgatcta tagggcatcc actctggcat ctggggtccc atcaaggttc 240
   agcggcagtg gatctgggac agacttcact ctcaccatca gcagcctgca gcctgatgat 300
   tttgcaactt attactgcca acagggttat agtctgagga acattgataa tgctttcggc 360
   ggagggacca aggtggaaat caaacgtacg gtggctgcac catctgtctt catcttcccg 420
   ccatctgatg agcagttgaa atctggaact gcctctgttg tgtgcctgct gaataacttc 480
   tatcccagag aggccaaagt acagtggaag gtggataacg ccctccaatc gggtaactcc 540
   caggagagtg tcacagagca ggacagcaag gacagcacct acagcctcag cagcaccctg 600
   acgctgagca aagcagacta cgagaaacac aaagtctacg cctgcgaagt cacccatcag 660
   ggcctgagct cgcccgtcac aaagagcttc aacaggggag agtgt 705
<210> 706
   <211> 235
   <212> PRT
   <213> Oryctolagus cuniculus
<400> 706
   Met Lys Trp Val Thr Phe Ile Ser Leu Leu Phe Leu Phe Ser Ser Ala
   1               5                   10                  15
   Tyr Ser Ala Ile Gln Met Thr Gln Ser Pro Ser Ser Leu Ser Ala Ser
               20                  25                  30
   Val Gly Asp Arg Val Thr Ile Thr Cys Gln Ala Ser Gln Ser Ile Asn
           35                  40                  45
   Asn Glu Leu Ser Trp Tyr Gln Gln Lys Pro Gly Lys Ala Pro Lys Leu
       50                  55                  60
   Leu Ile Tyr Arg Ala Ser Thr Leu Ala Ser Gly Val Pro Ser Arg Phe
   65                  70                  75                  80
   Ser Gly Ser Gly Ser Gly Thr Asp Phe Thr Leu Thr Ile Ser Ser Leu
                   85                  90                  95
   Gln Pro Asp Asp Phe Ala Thr Tyr Tyr Cys Gln Gln Gly Tyr Ser Leu
               100                 105                 110
   Arg Asn Ile Asp Asn Ala Phe Gly Gly Gly Thr Lys Val Glu Ile Lys
           115                 120                 125
   Arg Thr Val Ala Ala Pro Ser Val Phe Ile Phe Pro Pro Ser Asp Glu
       130                 135                 140
   Gln Leu Lys Ser Gly Thr Ala Ser Val Val Cys Leu Leu Asn Asn Phe
   145                 150                 155                 160
   Tyr Pro Arg Glu Ala Lys Val Gln Trp Lys Val Asp Asn Ala Leu Gln
                   165                 170                 175
   Ser Gly Asn Ser Gln Glu Ser Val Thr Glu Gln Asp Ser Lys Asp Ser
               180                 185                 190
   Thr Tyr Ser Leu Ser Ser Thr Leu Thr Leu Ser Lys Ala Asp Tyr Glu
           195                 200                 205
   Lys His Lys Val Tyr Ala Cys Glu Val Thr His Gln Gly Leu Ser Ser
       210                 215                 220
   Pro Val Thr Lys Ser Phe Asn Arg Gly Glu Cys
   225                 230                 235
<210> 707
   <211> 1404
   <212> DNA
   <213> Oryctolagus cuniculus
<400> 707
   atgaagtggg taacctttat ttcccttctg tttctcttta gcagcgctta ttccgaggtg      60
   cagctggtgg agtctggggg aggcttggtc cagcctgggg ggtccctgag actctcctgt     120
   gcagcctctg gattctccct cagtaactac tacgtgacct gggtccgtca ggctccaggg     180
   aaggggctgg agtgggtcgg catcatctat ggtagtgatg aaaccgccta cgctacctcc     240
   gctataggcc gattcaccat ctccagagac aattccaaga acaccctgta tcttcaaatg     300
   aacagcctga gagctgagga cactgctgtg tattactgtg ctagagatga tagtagtgac     360
   tgggatgcaa agttcaactt gtggggccaa gggaccctcg tcaccgtctc gagcgcctcc     420
   accaagggcc catcggtctt ccccctggca ccctcctcca agagcacctc tgggggcaca     480
   gcggccctgg gctgcctggt caaggactac ttccccgaac cggtgacggt gtcgtggaac     540
   tcaggcgccc tgaccagcgg cgtgcacacc ttcccggctg tcctacagtc ctcaggactc     600
   tactccctca gcagcgtggt gaccgtgccc tccagcagct tgggcaccca gacctacatc     660
   tgcaacgtga atcacaagcc cagcaacacc aaggtggaca agagagttga gcccaaatct     720
   tgtgacaaaa ctcacacatg cccaccgtgc ccagcacctg aactcctggg gggaccgtca     780
   gtcttcctct tccccccaaa acccaaggac accctcatga tctcccggac ccctgaggtc     840
   acatgcgtgg tggtggacgt gagccacgaa gaccctgagg tcaagttcaa ctggtacgtg     900
   gacggcgtgg aggtgcataa tgccaagaca aagccgcggg aggagcagta cgccagcacg     960
   taccgtgtgg tcagcgtcct caccgtcctg caccaggact ggctgaatgg caaggagtac    1020
   aagtgcaagg tctccaacaa agccctccca gcccccatcg agaaaaccat ctccaaagcc    1080
   aaagggcagc cccgagaacc acaggtgtac accctgcccc catcccggga ggagatgacc    1140
   aagaaccagg tcagcctgac ctgcctggtc aaaggcttct atcccagcga catcgccgtg    1200
   gagtgggaga gcaatgggca gccggagaac aactacaaga ccacgcctcc cgtgctggac    1260
   tccgacggct ccttcttcct ctacagcaag ctcaccgtgg acaagagcag gtggcagcag    1320
   gggaacgtct tctcatgctc cgtgatgcat gaggctctgc acaaccacta cacgcagaag    1380
   agcctctccc tgtctccggg taaa                                           1404
<210> 708
   <211> 468
   <212> PRT
   <213> Oryctolagus cuniculus
<400> 708
   Met Lys Trp Val Thr Phe Ile Ser Leu Leu Phe Leu Phe Ser Ser Ala
   1               5                   10                  15
   Tyr Ser Glu Val Gln Leu Val Glu Ser Gly Gly Gly Leu Val Gln Pro
               20                  25                  30
   Gly Gly Ser Leu Arg Leu Ser Cys Ala Ala Ser Gly Phe Ser Leu Ser
           35                  40                  45
   Asn Tyr Tyr Val Thr Trp Val Arg Gln Ala Pro Gly Lys Gly Leu Glu
       50                  55                  60
   Trp Val Gly Ile Ile Tyr Gly Ser Asp Glu Thr Ala Tyr Ala Thr Ser
   65                  70                  75                  80
   Ala Ile Gly Arg Phe Thr Ile Ser Arg Asp Asn Ser Lys Asn Thr Leu
                   85                  90                  95
   Tyr Leu Gln Met Asn Ser Leu Arg Ala Glu Asp Thr Ala Val Tyr Tyr
               100                 105                 110
   Cys Ala Arg Asp Asp Ser Ser Asp Trp Asp Ala Lys Phe Asn Leu Trp
           115                 120                 125
   Gly Gln Gly Thr Leu Val Thr Val Ser Ser Ala Ser Thr Lys Gly Pro
       130                 135                 140
   Ser Val Phe Pro Leu Ala Pro Ser Ser Lys Ser Thr Ser Gly Gly Thr
   145                 150                 155                 160
   Ala Ala Leu Gly Cys Leu Val Lys Asp Tyr Phe Pro Glu Pro Val Thr
                   165                 170                 175
   Val Ser Trp Asn Ser Gly Ala Leu Thr Ser Gly Val His Thr Phe Pro
               180                 185                 190
   Ala Val Leu Gln Ser Ser Gly Leu Tyr Ser Leu Ser Ser Val Val Thr
           195                 200                 205
   Val Pro Ser Ser Ser Leu Gly Thr Gln Thr Tyr Ile Cys Asn Val Asn
       210                 215                 220
   His Lys Pro Ser Asn Thr Lys Val Asp Lys Arg Val Glu Pro Lys Ser
   225                 230                 235                 240
   Cys Asp Lys Thr His Thr Cys Pro Pro Cys Pro Ala Pro Glu Leu Leu
                   245                 250                 255
   Gly Gly Pro Ser Val Phe Leu Phe Pro Pro Lys Pro Lys Asp Thr Leu
               260                 265                 270
   Met Ile Ser Arg Thr Pro Glu Val Thr Cys Val Val Val Asp Val Ser
   275 280 285
   His Glu Asp Pro Glu Val Lys Phe Asn Trp Tyr Val Asp Gly Val Glu
   290 295 300
   Val His Asn Ala Lys Thr Lys Pro Arg Glu Glu Gln Tyr Ala Ser Thr
   305 310 315 320
   Tyr Arg Val Val Ser Val Leu Thr Val Leu His Gln Asp Trp Leu Asn
   325 330 335
   Gly Lys Glu Tyr Lys Cys Lys Val Ser Asn Lys Ala Leu Pro Ala Pro
   340 345 350
   Ile Glu Lys Thr Ile Ser Lys Ala Lys Gly Gln Pro Arg Glu Pro Gln
   355 360 365
   Val Tyr Thr Leu Pro Pro Ser Arg Glu Glu Met Thr Lys Asn Gln Val
   370 375 380
   Ser Leu Thr Cys Leu Val Lys Gly Phe Tyr Pro Ser Asp Ile Ala Val
   385 390 395 400
   Glu Trp Glu Ser Asn Gly Gln Pro Glu Asn Asn Tyr Lys Thr Thr Pro
   405 410 415
   Pro Val Leu Asp Ser Asp Gly Ser Phe Phe Leu Tyr Ser Lys Leu Thr
   420 425 430
   Val Asp Lys Ser Arg Trp Gln Gln Gly Asn Val Phe Ser Cys Ser Val
   435 440 445
   Met His Glu Ala Leu His Asn His Tyr Thr Gln Lys Ser Leu Ser Leu
   450 455 460
   Ser Pro Gly Lys
   465
<210> 709
   <211> 111
   <212> PRT
   <213> Oryctolagus cuniculus
<400> 709
   Ala Ile Gln Met Thr Gln Ser Pro Ser Ser Leu Ser Ala Ser Val Gly
   1 5 10 15
   Asp Arg Val Thr Ile Thr Cys Gln Ala Ser Gln Ser Ile Asn Asn Glu
   20 25 30
   Leu Ser Trp Tyr Gln Gln Lys Pro Gly Lys Ala Pro Lys Leu Leu Ile
   35 40 45
   Tyr Arg Ala Ser Thr Leu Ala Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Asp Asp Phe Ala Thr Tyr Tyr Cys Gln Gln Gly Tyr Ser Leu Arg Asn
   85 90 95
   Ile Asp Asn Ala Phe Gly Gly Gly Thr Lys Val Glu Ile Lys Arg
   100 105 110
<210> 710
   <211> 11
   <212> PRT
   <213> Homo sapiens
<400> 710
   Arg Ala Ser Gln Gly Ile Arg Asn Asp Leu Gly
   1 5 10
<210> 711
   <211> 11
   <212> PRT
   <213> Homo sapiens
<400> 711
   Arg Ala Ser Gln Gly Ile Ser Asn Tyr Leu Ala
   1 5 10
<210> 712
   <211> 11
   <212> PRT
   <213> Homo sapiens
<400> 712
   Arg Ala Ser Gln Ser Ile Ser Ser Trp Leu Ala
   1 5 10
<210> 713
   <211> 7
   <212> PRT
   <213> Homo sapiens
<400> 713
   Ala Ala Ser Ser Leu Gln Ser
   1 5
<210> 714
   <211> 7
   <212> PRT
   <213> Homo sapiens
<400> 714
   Ala Ala Ser Thr Leu Gln Ser
   1 5
<210> 715
   <211> 7
   <212> PRT
   <213> Homo sapiens
<400> 715
   Lys Ala Ser Ser Leu Glu Ser
   1 5
<210> 716
   <211> 5
   <212> PRT
   <213> Homo sapiens
<400> 716
   Ser Asn Tyr Met Ser
   1 5
<210> 717
   <211> 16
   <212> PRT
   <213> Homo sapiens
<400> 717
   Val Ile Tyr Ser Gly Gly Ser Thr Tyr Tyr Ala Asp Ser Val Lys Gly
   1 5 10 15
<210> 718
   <211> 17
   <212> PRT
   <213> Homo sapiens
<400> 718
   Val Ile Tyr Ser Gly Gly Ser Ser Thr Tyr Tyr Ala Asp Ser Val Lys
   1 5 10 15
   Gly
<210> 719
   <211> 330
   <212> PRT
   <213> Artificial Sequence
<220>
   <223> Gamma-1 constant domain
<400> 719
   Ala Ser Thr Lys Gly Pro Ser Val Phe Pro Leu Ala Pro Ser Ser Lys
   1               5                   10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Arg Val Glu Pro Lys Ser Cys Asp Lys Thr His Thr Cys Pro Pro Cys
               100                 105                 110
   Pro Ala Pro Glu Leu Leu Gly Gly Pro Ser Val Phe Leu Phe Pro Pro
           115                 120                 125
   Lys Pro Lys Asp Thr Leu Met Ile Ser Arg Thr Pro Glu Val Thr Cys
       130                 135                 140
   Val Val Val Asp Val Ser His Glu Asp Pro Glu Val Lys Phe Asn Trp
   145                 150                 155                 160
   Tyr Val Asp Gly Val Glu Val His Asn Ala Lys Thr Lys Pro Arg Glu
                   165                 170                 175
   Glu Gln Tyr Ala Ser Thr Tyr Arg Val Val Ser Val Leu Thr Val Leu
               180                 185                 190
   His Gln Asp Trp Leu Asn Gly Lys Glu Tyr Lys Cys Lys Val Ser Asn
           195                 200                 205
   Lys Ala Leu Pro Ala Pro Ile Glu Lys Thr Ile Ser Lys Ala Lys Gly
   210 215 220
   Gln Pro Arg Glu Pro Gln Val Tyr Thr Leu Pro Pro Ser Arg Asp Glu
   225 230 235 240
   Leu Thr Lys Asn Gln Val Ser Leu Thr Cys Leu Val Lys Gly Phe Tyr
   245 250 255
   Pro Ser Asp Ile Ala Val Glu Trp Glu Ser Asn Gly Gln Pro Glu Asn
   260 265 270
   Asn Tyr Lys Thr Thr Pro Pro Val Leu Asp Ser Asp Gly Ser Phe Phe
   275 280 285
   Leu Tyr Ser Lys Leu Thr Val Asp Lys Ser Arg Trp Gln Gln Gly Asn
   290 295 300
   Val Phe Ser Cys Ser Val Met His Glu Ala Leu His Asn His Tyr Thr
   305 310 315 320
   Gln Lys Ser Leu Ser Leu Ser Pro Gly Lys
   325 330
<210> 720
   <211> 297
   <212> DNA
   <213> Oryctolagus cuniculus
<400> 720
   atccagatga cccagtctcc ttcctccctg tctgcatctg taggagacag agtcaccatc 60
   acttgccagg ccagtcagag cattaacaat gagttatcct ggtatcagca gaaaccaggg 120
   aaagccccta agctcctgat ctatagggca tccactctgg catctggggt cccatcaagg 180
   ttcagcggca gtggatctgg gacagacttc actctcacca tcagcagcct gcagcctgat 240
   gattttgcaa cttattactg ccaacagggt tatagtctga ggaacattga taatgct 297
<210> 721
   <211> 333
   <212> DNA
   <213> Oryctolagus cuniculus
<400> 721
   gcctatgata tgacccagac tccagcctcg gtgtctgcag ctgtgggagg cacagtcacc 60
   atcaagtgcc aggccagtca gagcattaac aatgaattat cctggtatca gcagaaacca 120
   gggcagcgtc ccaagctcct gatctatagg gcatccactc tggcatctgg ggtctcatcg 180
   cggttcaaag gcagtggatc tgggacagag ttcactctca ccatcagcga cctggagtgt 240
   gccgatgctg ccacttacta ctgtcaacag ggttatagtc tgaggaatat tgataatgct 300
   ttcggcggag ggaccgaggt ggtggtcaaa cgt 333
<210> 722
   <211> 648
   <212> DNA
   <213> Oryctolagus cuniculus
<400> 722
   atccagatga cccagtctcc ttcctccctg tctgcatctg taggagacag agtcaccatc 60
   acttgccagg ccagtcagag cattaacaat gagttatcct ggtatcagca gaaaccaggg 120
   aaagccccta agctcctgat ctatagggca tccactctgg catctggggt cccatcaagg 180
   ttcagcggca gtggatctgg gacagacttc actctcacca tcagcagcct gcagcctgat 240
   gattttgcaa cttattactg ccaacagggt tatagtctga ggaacattga taatgctttc 300
   ggcggaggga ccaaggtgga aatcaaacgt acggtggctg caccatctgt cttcatcttc 360
   ccgccatctg atgagcagtt gaaatctgga actgcctctg ttgtgtgcct gctgaataac 420
   ttctatccca gagaggccaa agtacagtgg aaggtggata acgccctcca atcgggtaac 480
   tcccaggaga gtgtcacaga gcaggacagc aaggacagca cctacagcct cagcagcacc 540
   ctgacgctga gcaaagcaga ctacgagaaa cacaaagtct acgcctgcga agtcacccat 600
   cagggcctga gctcgcccgt cacaaagagc ttcaacaggg gagagtgt 648
<210> 723
   <211> 333
   <212> DNA
   <213> Oryctolagus cuniculus
<400> 723
   gctatccaga tgacccagtc tccttcctcc ctgtctgcat ctgtaggaga cagagtcacc 60
   atcacttgcc aggccagtca gagcattaac aatgagttat cctggtatca gcagaaacca 120
   gggaaagccc ctaagctcct gatctatagg gcatccactc tggcatctgg ggtcccatca 180
   aggttcagcg gcagtggatc tgggacagac ttcactctca ccatcagcag cctgcagcct 240
   gatgattttg caacttatta ctgccaacag ggttatagtc tgaggaacat tgataatgct 300
   ttcggcggag ggaccaaggt ggaaatcaaa cgt 333
<210> 724
   <211> 327
   <212> DNA
   <213> Oryctolagus cuniculus
<400> 724
   gaggtgcagc tggtggagtc tgggggaggc ttggtccagc ctggggggtc cctgagactc 60
   tcctgtgcag cctctggatt ctccctcagt aactactacg tgacctgggt ccgtcaggct 120
   ccagggaagg ggctggagtg ggtcggcatc atctatggta gtgatgaaac cgcctacgct 180
   acctccgcta taggccgatt caccatctcc agagacaatt ccaagaacac cctgtatctt 240
   caaatgaaca gcctgagagc tgaggacact gctgtgtatt actgtgctag agatgatagt 300
   agtgactggg atgcaaagtt caacttg 327
<210> 725
   <211> 351
   <212> DNA
   <213> Oryctolagus cuniculus
<400> 725
   cagtcgctgg aggagtccgg gggtcgcctg gtcacgcctg ggacacccct gacactcacc 60
   tgcacagcct ctggattctc cctcagtaac tactacgtga cctgggtccg ccaggctcca 120
   gggaaggggc tggaatggat cggaatcatt tatggtagtg atgaaacggc ctacgcgacc 180
   tgggcgatag gccgattcac catctccaaa acctcgacca cggtggatct gaaaatgacc 240
   agtctgacag ccgcggacac ggccacctat ttctgtgcca gagatgatag tagtgactgg 300
   gatgcaaaat ttaacttgtg gggccaaggc accctggtca ccgtctcgag c 351
<210> 726
   <211> 224
   <212> PRT
   <213> Homo sapiens
<400> 726
   Met Glu Lys Leu Leu Cys Phe Leu Val Leu Thr Ser Leu Ser His Ala
   1               5                   10                  15
   Phe Gly Gln Thr Asp Met Ser Arg Lys Ala Phe Val Phe Pro Lys Glu
               20                  25                  30
   Ser Asp Thr Ser Tyr Val Ser Leu Lys Ala Pro Leu Thr Lys Pro Leu
           35                  40                  45
   Lys Ala Phe Thr Val Cys Leu His Phe Tyr Thr Glu Leu Ser Ser Thr
       50                  55                  60
   Arg Gly Tyr Ser Ile Phe Ser Tyr Ala Thr Lys Arg Gln Asp Asn Glu
   65                  70                  75                  80
   Ile Leu Ile Phe Trp Ser Lys Asp Ile Gly Tyr Ser Phe Thr Val Gly
                   85                  90                  95
   Gly Ser Glu Ile Leu Phe Glu Val Pro Glu Val Thr Val Ala Pro Val
               100                 105                 110
   His Ile Cys Thr Ser Trp Glu Ser Ala Ser Gly Ile Val Glu Phe Trp
           115                 120                 125
   Val Asp Gly Lys Pro Arg Val Arg Lys Ser Leu Lys Lys Gly Tyr Thr
       130                 135                 140
   Val Gly Ala Glu Ala Ser Ile Ile Leu Gly Gln Glu Gln Asp Ser Phe
   145                 150                 155                 160
   Gly Gly Asn Phe Glu Gly Ser Gln Ser Leu Val Gly Asp Ile Gly Asn
                   165                 170                 175
   Val Asn Met Trp Asp Phe Val Leu Ser Pro Asp Glu Ile Asn Thr Ile
               180                 185                 190
   Tyr Leu Gly Gly Pro Phe Ser Pro Asn Val Leu Asn Trp Arg Ala Leu
           195                 200                 205
   Lys Tyr Glu Val Gln Gly Glu Val Phe Thr Lys Pro Gln Leu Trp Pro
       210                 215                 220
<210> 727
   <211> 468
   <212> PRT
   <213> Homo sapiens
<400> 727
   Met Leu Ala Val Gly Cys Ala Leu Leu Ala Ala Leu Leu Ala Ala Pro
   1               5                   10                  15
   Gly Ala Ala Leu Ala Pro Arg Arg Cys Pro Ala Gln Glu Val Ala Arg
               20                  25                  30
   Gly Val Leu Thr Ser Leu Pro Gly Asp Ser Val Thr Leu Thr Cys Pro
           35                  40                  45
   Gly Val Glu Pro Glu Asp Asn Ala Thr Val His Trp Val Leu Arg Lys
       50                  55                  60
   Pro Ala Ala Gly Ser His Pro Ser Arg Trp Ala Gly Met Gly Arg Arg
   65                  70                  75                  80
   Leu Leu Leu Arg Ser Val Gln Leu His Asp Ser Gly Asn Tyr Ser Cys
                   85                  90                  95
   Tyr Arg Ala Gly Arg Pro Ala Gly Thr Val His Leu Leu Val Asp Val
               100                 105                 110
   Pro Pro Glu Glu Pro Gln Leu Ser Cys Phe Arg Lys Ser Pro Leu Ser
           115                 120                 125
   Asn Val Val Cys Glu Trp Gly Pro Arg Ser Thr Pro Ser Leu Thr Thr
       130                 135                 140
   Lys Ala Val Leu Leu Val Arg Lys Phe Gln Asn Ser Pro Ala Glu Asp
   145                 150                 155                 160
   Phe Gln Glu Pro Cys Gln Tyr Ser Gln Glu Ser Gln Lys Phe Ser Cys
                   165                 170                 175
   Gln Leu Ala Val Pro Glu Gly Asp Ser Ser Phe Tyr Ile Val Ser Met
               180                 185                 190
   Cys Val Ala Ser Ser Val Gly Ser Lys Phe Ser Lys Thr Gln Thr Phe
           195                 200                 205
   Gln Gly Cys Gly Ile Leu Gln Pro Asp Pro Pro Ala Asn Ile Thr Val
       210                 215                 220
   Thr Ala Val Ala Arg Asn Pro Arg Trp Leu Ser Val Thr Trp Gln Asp
   225                 230                 235                 240
   Pro His Ser Trp Asn Ser Ser Phe Tyr Arg Leu Arg Phe Glu Leu Arg
                   245                 250                 255
   Tyr Arg Ala Glu Arg Ser Lys Thr Phe Thr Thr Trp Met Val Lys Asp
               260                 265                 270
   Leu Gln His His Cys Val Ile His Asp Ala Trp Ser Gly Leu Arg His
           275                 280                 285
   Val Val Gln Leu Arg Ala Gln Glu Glu Phe Gly Gln Gly Glu Trp Ser
       290                 295                 300
   Glu Trp Ser Pro Glu Ala Met Gly Thr Pro Trp Thr Glu Ser Arg Ser
   305                 310                 315                 320
   Pro Pro Ala Glu Asn Glu Val Ser Thr Pro Met Gln Ala Leu Thr Thr
                   325                 330                 335
   Asn Lys Asp Asp Asp Asn Ile Leu Phe Arg Asp Ser Ala Asn Ala Thr
               340                 345                 350
   Ser Leu Pro Val Gln Asp Ser Ser Ser Val Pro Leu Pro Thr Phe Leu
           355                 360                 365
   Val Ala Gly Gly Ser Leu Ala Phe Gly Thr Leu Leu Cys Ile Ala Ile
       370                 375                 380
   Val Leu Arg Phe Lys Lys Thr Trp Lys Leu Arg Ala Leu Lys Glu Gly
   385                 390                 395                 400
   Lys Thr Ser Met His Pro Pro Tyr Ser Leu Gly Gln Leu Val Pro Glu
                   405                 410                 415
   Arg Pro Arg Pro Thr Pro Val Leu Val Pro Leu Ile Ser Pro Pro Val
               420                 425                 430
   Ser Pro Ser Ser Leu Gly Ser Asp Asn Thr Ser Ser His Asn Arg Pro
           435                 440                 445
   Asp Ala Arg Asp Pro Arg Ser Pro Tyr Asp Ile Ser Asn Thr Asp Tyr
       450                 455                 460
   Phe Phe Pro Arg
   465
<210> 728
   <211> 918
   <212> PRT
   <213> Homo sapiens
<400> 728
<210> 729
   <211> 32
   <212> DNA
   <213> Artificial sequence
<220>
   <223> Primer
<400> 729
   AGCGCTTATT CCGCTATCCA GATGACCCAG TC 32
<210> 730
   <211> 22
   <212> DNA
   <213> Artificial sequence
<220>
   <223> Primer
<400> 730
   CGTACGTTTG ATTTCCACCT TG 22
<210> 731
   <211> 32
   <212> DNA
   <213> Artificial sequence
<220>
   <223> Primer
<400> 731
   AGCGCTTATT CCGAGGTGCA GCTGGTGGAG TC 32
<210> 732
   <211> 20
   <212> DNA
   <213> Artificial sequence
<220>
   <223> Primer
<400> 732
   CTCGAGACGG TGACGAGGGT 20
<210> 733
   <211> 111
   <212> PRT
   <213> Oryctolagus cuniculus
<400> 733
   Ala Tyr Asp Met Thr Gln Thr Pro Ala Ser Val Glu Val Ala Val Gly
   1               5                   10                  15
   Gly Thr Val Thr Ile Asn Cys Gln Ala Ser Glu Thr Ile Tyr Ser Trp
               20                  25                  30
   Leu Ser Trp Tyr Gln Gln Lys Pro Gly Gln Pro Pro Lys Leu Leu Ile
           35                  40                  45
   Tyr Gln Ala Ser Asp Leu Ala Ser Gly Val Pro Ser Arg Phe Ser Gly
       50                  55                  60
   Ser Gly Ala Gly Thr Glu Tyr Thr Leu Thr Ile Ser Gly Val Gln Cys
   65                  70                  75                  80
   Asp Asp Ala Ala Thr Tyr Tyr Cys Gln Gln Gly Tyr Ser Gly Ser Asn
                   85                  90                  95
   Val Asp Asn Val Phe Gly Gly Gly Thr Glu Val Val Val Lys Arg
               100                 105                 110
<210> 734
   <211> 88
   <212> PRT
   <213> Homo Sapiens
<400> 734
   Asp Ile Gln Met Thr Gln Ser Pro Ser Thr Leu Ser Ala Ser Val Gly
   1 5 10 15
   Asp Arg Val Thr Ile Thr Cys Arg Ala Ser Gln Ser Ile Ser Ser Trp
   20 25 30
   Leu Ala Trp Tyr Gln Gln Lys Pro Gly Lys Ala Pro Lys Leu Leu Ile
   35 40 45
   Tyr Lys Ala Ser Ser Leu Glu Ser Gly Val Pro Ser Arg Phe Ser Gly
   50 55 60
   Ser Gly Ser Gly Thr Glu Phe Thr Leu Thr Ile Ser Ser Leu Gln Pro
   65 70 75 80
   Asp Asp Phe Ala Thr Tyr Tyr Cys
   85
<210> 735
   <211> 88
   <212> PRT
   <213> Homo Sapiens
<400> 735
   Asp Ile Gln Met Thr Gln Ser Pro Ser Thr Leu Ser Ala Ser Val Gly
   1 5 10 15
   Asp Arg Val Thr Ile Thr Cys Arg Ala Ser Gln Ser Ile Ser Ser Trp
   20 25 30
   Leu Ala Trp Tyr Gln Gln Lys Pro Gly Lys Ala Pro Lys Leu Leu Ile
   35 40 45
   Tyr Asp Ala Ser Ser Leu Glu Ser Gly Val Pro Ser Arg Phe Ser Gly
   50 55 60
   Ser Gly Ser Gly Thr Glu Phe Thr Leu Thr Ile Ser Ser Leu Gln Pro
   65 70 75 80
   Asp Asp Phe Ala Thr Tyr Tyr Cys
   85
<210> 736
   <211> 88
   <212> PRT
   <213> Homo Sapiens
<400> 736
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ser Ile Ser Ser Tyr
               20                  25                  30
   Leu Asn Trp Tyr Gln Gln Lys Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys
                   85
<210> 737
   <211> 111
   <212> PRT
   <213> Artificial sequence
<220>
   <223> Humanised variable light chain antibody sequence
<400> 737
   Asp Ile Gln Met Thr Gln Ser Pro Ser Thr Leu Ser Ala Ser Val Gly
   1               5                   10                  15
   Asp Arg Val Thr Ile Thr Cys Gln Ala Ser Glu Thr Ile Tyr Ser Trp
               20                  25                  30
   Leu Ser Trp Tyr Gln Gln Lys Pro Gly Lys Ala Pro Lys Leu Leu Ile
           35                  40                  45
   Tyr Gln Ala Ser Asp Leu Ala Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Asp Asp Phe Ala Thr Tyr Tyr Cys Gln Gln Gly Tyr Ser Gly Ser Asn
                   85                  90                  95
   Val Asp Asn Val Phe Gly Gly Gly Thr Lys Val Glu Ile Lys Arg
               100                 105                 110
<210> 738
   <211> 118
   <212> PRT
   <213> Oryctolagus cuniculus
<400> 738
   Gln Glu Gln Leu Lys Glu Ser Gly Gly Arg Leu Val Thr Pro Gly Thr
   1               5                   10                  15
   Pro Leu Thr Leu Thr Cys Thr Ala Ser Gly Phe Ser Leu Asn Asp His
               20                  25                  30
   Ala Met Gly Trp Val Arg Gln Ala Pro Gly Lys Gly Leu Glu Tyr Ile
           35                  40                  45
   Gly Phe Ile Asn Ser Gly Gly Ser Ala Arg Tyr Ala Ser Trp Ala Glu
       50                  55                  60
   Gly Arg Phe Thr Ile Ser Arg Thr Ser Thr Thr Val Asp Leu Lys Met
   65                  70                  75                  80
   Thr Ser Leu Thr Thr Glu Asp Thr Ala Thr Tyr Phe Cys Val Arg Gly
                   85                  90                  95
   Gly Ala Val Trp Ser Ile His Ser Phe Asp Pro Trp Gly Pro Gly Thr
   100 105 110
   Leu Val Thr Val Ser Ser
   115
<210> 739
   <211> 98
   <212> PRT
   <213> Homo Sapiens
<400> 739
   Gln Val Gln Leu Val Glu Ser Gly Gly Gly Leu Val Gln Pro Gly Gly
   1 5 10 15
   Ser Leu Arg Leu Ser Cys Ser Ala Ala Gly Phe Thr Phe Ser Ser Tyr
   20 25 30
   Ala Met His Trp Val Arg Gln Ala Pro Gly Lys Gly Leu Glu Tyr Val
   35 40 45
   Ser Ala Ile Ser Ser Asn Gly Gly Ser Thr Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg
<210> 740
   <211> 97
   <212> PRT
   <213> Homo Sapiens
<400> 740
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Val Ser Ser Asn
   20 25 30
   Tyr Met Ser Trp Val Arg Gln Ala Pro Gly Lys Gly Leu Glu Trp Val
   35 40 45
   Ser Val Ile Tyr Ser Gly Gly Ser Thr Tyr Tyr Ala Asp Ser Val Lys
   50 55 60
   Gly Arg Phe Thr Ile Ser Arg Asp Asn Ser Lys Asn Thr Leu Tyr Leu
   65 70 75 80
   Gln Met Asn Ser Leu Arg Ala Glu Asp Thr Ala Val Tyr Tyr Cys Ala
   85 90 95
   Arg
<210> 741
   <211> 97
   <212> PRT
   <213> Homo Sapiens
<400> 741
   Glu Val Gln Leu Val Glu Thr Gly Gly Gly Leu Ile Gln Pro Gly Gly
   1 5 10 15
   Ser Leu Arg Leu Ser Cys Ala Ala Ser Gly Phe Thr Val Ser Ser Asn
   20 25 30
   Tyr Met Ser Trp Val Arg Gln Ala Pro Gly Lys Gly Leu Glu Trp Val
   35 40 45
   Ser Val Ile Tyr Ser Gly Gly Ser Thr Tyr Tyr Ala Asp Ser Val Lys
   50 55 60
   Gly Arg Phe Thr Ile Ser Arg Asp Asn Ser Lys Asn Thr Leu Tyr Leu
   65 70 75 80
   Gln Met Asn Ser Leu Arg Ala Glu Asp Thr Ala Val Tyr Tyr Cys Ala
   85 90 95
   Arg
<210> 742
   <211> 120
   <212> PRT
   <213> Artificial sequence
<220>
   <223> Humanised variable heavy chain antibody sequence
<400> 742
   Gln Val Gln Leu Val Glu Ser Gly Gly Gly Leu Val Gln Pro Gly Gly
   1               5                   10                  15
   Ser Leu Arg Leu Ser Cys Ser Ala Ser Gly Phe Ser Leu Asn Asp His
               20                  25                  30
   Ala Met Gly Trp Val Arg Gln Ala Pro Gly Lys Gly Leu Glu Tyr Val
           35                  40                  45
   Gly Phe Ile Asn Ser Gly Gly Ser Ala Arg Tyr Ala Ser Ser Ala Glu
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Arg Gly Gly Ala Val Trp Ser Ile His Ser Phe Asp Pro Trp Gly Gln
               100                 105                 110
   Gly Thr Leu Val Thr Val Ser Ser
           115

## Description

### BACKGROUND OF THE INVENTION

### Field of the Invention

This invention is an extension of Applicants' prior invention relating to novel anti-IL-6 antibodies, novel therapies and therapeutic protocols utilizing anti-IL-6 antibodies, and pharmaceutical formulations containing anti-IL-6 antibodies. The anti-IL-6 antibody as defined in the claims is Ab1, and comprises a light chain polypeptide that comprises the light chain variable region of SEQ ID NO:709 and the light chain constant region of SEQ ID NO:586; and a heavy chain polypeptide that comprises the heavy chain variable region of SEQ ID NO:657 and the heavy chain constant region of SEQ ID NO:588, wherein the anti-IL-6 antibody is aglycosylated, The subject application pertains in particular to preferred formulations and therapeutic uses of an exemplary humanized antibody referred to herein as Ab1. In preferred embodiments, the anti-IL-6 antibody has an *in vivo* half-life of at least about 25 days, an *in vivo* effect of raising albumin, has an *in vivo* effect of lowering C-reactive protein, has an *in vivo* effect of restoring a normal coagulation profile, possesses a binding affinity (Kd) for IL-6 of less than about 50 picomolar, and/or has a rate of dissociation (Kₒff) from IL-6 of less than or equal to 10⁻⁴ S⁻¹.

The invention provides a composition comprising an anti-IL-6 antibody as defined in the claims for use in methods of preventing or treating a condition in a human subject associated with elevated IL-6, wherein the condition is further defined in the claims.

This invention pertains to methods of improving survivability or quality of life of a patient in need thereof, comprising administering to the patient an anti-IL-6 antibody as defined in the claims, whereby the patient's C-reactive protein ("CRP") level is lowered, and/or the patient's albumin level is raised, and optionally monitoring the patient to determine the patient's CRP and/or albumin level.

This invention relates to methods of lowering the C-reactive protein level in a patient in need thereof, comprising administering to the patient an IL-6 antagonist as defined in the claims, whereby the patient's CRP level is lowered, and monitoring the patient to assess the CRP level. This invention may relate to methods of raising the albumin level in a patient in need thereof, comprising administering to the patient an IL-6 antagonist as defined in the claims, whereby the patient's serum albumin level is raised, and monitoring the patient to assess the albumin level.

Described herein are methods of preventing or treating cachexia, weakness, fatigue, and/or fever in a patient in need thereof, e.g., a patient showing elevated CRP levels, comprising administering to the patient an anti-IL-6 antibody or antibody fragment or variant thereof, whereby the patient's cachexia, weakness, fatigue, and/or fever is improved or restored to a normal condition, and optionally monitoring the patient to assess cachexia, weakness, fatigue, and/or fever.

Described herein are methods of preventing or treating thrombosis in a patient in a state of hypercoagulation, comprising administering to the patient an anti-IL-6 antibody, such as Ab1 or a fragment or variant thereof, whereby the patient's coagulation profile is improved or restored to a normal condition, and optionally monitoring the patient to assess coagulation profile.

Described herein are novel pharmaceutical compositions and their use in novel combination therapies and comprising administration of an anti-IL-6 antibody, such as Ab1 or a fragment or variant thereof, and at least one other therapeutic compound such as a statin, anti-coagulant, anti-emetic, anti-nausea agent, anti-cachexia agent, chemotherapy agent, anti-cytokine agent, etc.

Weight loss, fatigue, and muscular weakness are very common symptoms of patients with advanced forms of cancer, and these symptoms can worsen as the cancer continues to progress. Fatigue, weight loss and muscular weakness can have significant negative effects on the recovery of patients with advanced forms of cancer, for example by disrupting lifestyles and relationships and affecting the willingness or ability of patients to continue cancer treatments. Known methods of addressing fatigue, weight loss and muscular weakness include regular routines of fitness and exercise, methods of conserving the patient's energy, and treatments that address anemia-induced fatigue and muscular weakness. Nevertheless, there remains a need in the art for methods and/or treatments that improve fatigue, weight loss and muscular weakness in cancer patients.

Thrombosis is a significant cause of mortality in cancer patients. Bick, N Engl J Med 349:109-111 (2003). For example, serious, life-threatening thrombotic events occur in approximately 6% of lung cancer patients. Alguire et al., J Clin Oncol 2004 Vol 22 (July 15th Supplement) No. 14S: 8082. Cancer patients often exhibit hypercoagulation, in which the coagulation system has an increased clotting tendency. Rickles and Edwards, Blood 62:14-31 (1983). Markers of hypercoagulation correlate with poor patient outcome for at least some cancers. Bick, Semin Thromb Hemostat 18:353-372 (1992); Buccheri et al., Cancer 97:3044-3052 (2003); Wojtukiewicz, Blood Coagul Fibrinolysis 3:429-437 (1992). Causes of hypercoagulation include the cancer itself and the cancer treatments (e.g., chemotherapy). Hypercoagulation results in an increased risk of thrombotic events, which can be further exacerbated when patients become bed-ridden. When not contraindicated, anticoagulant therapy has conferred survival benefit in some cancers. Lebeau et al., Cancer 74:38-45 (1994); Chahinian et al., J Clin Oncol 7:993-1002 (1989). However, therapeutic options are often limited because many cancer patients are at an elevated risk of major bleeding, precluding administration of anticoagulants that could otherwise be given prophylactically to reduce the risk of thrombosis. In summary, the available methods for prevention of thrombosis in cancer patients are unsatisfactory, and thus there is a need for new therapies. Such therapies would enhance cancer patient survival and promote better quality of life.

Thrombosis can also be a significant cause of adverse events and mortality in other patient groups, including those with chronic illness or chronic inflammation, surgical patients, bed-ridden individuals, and orthopedic patients. When they are not otherwise contraindicated, preventative methods include calf compression and anticoagulants (e.g. low molecular weight heparin). These preventative methods can reduce - but not eliminate - the risk of thrombosis. Because these preventative methods are not always effective and are contraindicated for some patients, and because anticoagulants can cause potentially lethal side-effects such as major bleeding, there is a need for alternative methods to prevent thrombosis in these patients. Such methods should improve patient outcomes.

Interleukin-6 (hereinafter "IL-6") (also known as interferon-β₂; B-cell differentiation factor; B-cell stimulatory factor-2; hepatocyte stimulatory factor; hybridoma growth factor; and plasmacytoma growth factor) is a multifunctional cytokine involved in numerous biological processes such as the regulation of the acute inflammatory response, the modulation of specific immune responses including B- and T-cell differentiation, bone metabolism, thrombopoiesis, epidermal proliferation, menses, neuronal cell differentiation, neuroprotection, aging, cancer, and the inflammatory reaction occurring in Alzheimer's disease. *See* A. Papassotiropoulos et al, Neurobiology of Aging, 22:863-871 (2001).

IL-6 is a member of a family of cytokines that promote cellular responses through a receptor complex consisting of at least one subunit of the signal-transducing glycoprotein gp130 and the IL-6 receptor ("IL-6R") (also known as gp80). The IL-6R may also be present in a soluble form ("sIL-6R"). IL-6 binds to IL-6R, which then dimerizes the signal-transducing receptor gp130. *See* Jones, SA, J. Immunology, 175:3463-3468 (2005).

In humans, the gene encoding IL-6 is organized in five exons and four introns, and maps to the short arm of chromosome 7 at 7p21. Translation of IL-6 RNA and post-translational processing result in the formation of a 21 to 28 kDa protein with 184 amino acids in its mature form. *See* A. Papassotiropoulos, et al, Neurobiology of Aging, 22:863-871 (2001).

As set forth in greater detail herein IL-6 is believed to play a role in the development of a multitude of diseases and disorders, including but not limited to fatigue, cachexia, autoimmune diseases, diseases of the skeletal system, cancer, heart disease, obesity, diabetes, asthma, Alzheimer's disease and multiple sclerosis. Due to the perceived involvement of IL-6 in a wide range of diseases and disorders, there remains a need in the art for compositions and methods useful for preventing or treating diseases associated with IL-6, as well as methods of screening to identify patients having diseases or disorders associated with IL-6. Particularly preferred anti-IL-6 compositions are those having minimal or minimizing adverse reactions when administered to the patient. Compositions or methods that reduce or inhibit diseases or disorders associated with IL-6 are beneficial to the patient in need thereof.

The function of IL-6 is not restricted to the immune response as it acts in hematopoiesis, thrombopoiesis, osteoclast formation, elicitation of hepatic acute phase response resulting in the elevation of C-reactive protein (CRP) and serum amyloid A (SAA) protein. It is known to be a growth factor for epidermal keratinocytes, renal mesangial cells, myeloma and plasmacytoma cells (Grossman et al., 1989 Prot Natl Acad Sci., 86, (16) 6367-6371; Horii et al., 1989, J Immunol, 143, 12, 3949-3955; Kawano et al., 1988, Nature 332, 6159, 83-85). IL-6 is produced by a wide range of cell types including monocytes/macrophages, fibroblasts, epidermal keratinocytes, vascular endothelial cells, renal messangial cells, glial cells, condrocytes, T and B-cells and some tumor cells (Akira et al, 1990, FASEB J., 4, 11, 2860-2867). Except for tumor cells that constitutively produce IL-6, normal cells do not express IL-6 unless appropriately stimulated.

Elevated IL-6 levels have been observed in many types of cancer, including breast cancer, leukemia, ovarian cancer, prostate cancer, pancreatic cancer, lymphoma, lung cancer, renal cell carcinoma, colorectal cancer, and multiple myeloma (*e.g.,* Chopra et al., 2004, MJAFI 60:45-49; Songur et al., 2004, Tumori 90:196-200; Blay et al., 1992, Cancer Research 52:3317-3322; Nikiteas et al., 2005, World J. Gasterenterol. 11:1639-1643; reviewed in Heikkila et al., 2008, Eur J Cancer, 44:937-945). As noted above, IL-6 is known or suspected to play a role in promoting proliferation or survival of at least some types of cancer. Moreover, some of these studies have demonstrated correlation between IL-6 levels and patient outcome. Together, these results suggest the possibility that inhibition of IL-6 can be therapeutically beneficial. Indeed, clinical studies (reviewed in Trikha et al., 2003, Clinical Cancer Research 9:4653-4665) have shown some improvement in patient outcomes due to administration of various anti-IL-6 antibodies, particularly in those cancers in which IL-6 plays a direct role promoting cancer cell proliferation or survival.

As noted above, IL-6 stimulates the hepatic acute phase response, resulting in increased production of CRP and elevated serum CRP levels. For this reason, C-reactive protein (CRP) has been reported to comprise a surrogate marker of IL-6 activity. Thus, elevated IL-6 activity can be detected through measurement of serum CRP. Conversely, effective suppression of IL-6 activity, e.g., through administration of a neutralizing anti-IL-6 antibody, can be detected by the resulting decrease in serum CRP levels.

A recent clinical trial demonstrated that administration of rosuvastatin to apparently healthy individuals having elevated CRP (greater than 2.0 mg/l) reduced their CRP levels by 37% and greatly decreased the incidence of myocardial infarction, stroke, arterial revascularization, hospitalization for unstable angina, or death from cardiovascular causes. Ridker et al., N Engl J Med. 2008 Nov 9 [Epub ahead of print].

In addition to its direct role in pathogenesis of some cancers and other diseases, chronically elevated IL-6 levels appear to adversely affect patient well-being and quality of life. For example, elevated IL-6 levels have been reported to be associated with cachexia and fever, and reduced serum albumin. Gauldie et al., 1987, PNAS 84:7251-7253; Heinric *et al.,* 1990, 265:621-636; Zamir et al., 1993, Metabolism 42:204-208; Zamir et al., 1992, Arch Surg, 127:170-174. Inhibition of IL-6 by a neutralizing antibody has been reported to ameliorate fever and cachexia in cancer patients, though improvement in these patients' serum albumin level has not been reported (Emille et al., 1994, Blood, 84:2472-2479; Blay et al., 1992, Cancer Research 52:3317-3322; Bataille et al., 1995, Blood, 86: 685-691).

Numerous studies have suggested that CRP is a valuable prognostic factor in cancer patients, with elevated CRP levels predicting poor outcome. *See, e.g.,* Hefler et al, Clin Cancer Res, 2008 Feb 1;14(3):710-4; Nagaoka et al, Liver Int, 2007 Oct;27(8):1091-7; Heikkilä et al, J Epidemiol Community Health, 2007 Sep;61(9):824-33, Review; Hara et al, Anticancer Res, 2007 Jul-Aug;27(4C):3001-4; Polterauer et al, Gynecol Oncol, 2007 Oct; 107(1):114-7, Epub 2007 Jul 6; Tingstedt et al, Scand J Gastroenterol, 2007 Jun;42(6):754-9; Suh et al, Support Care Cancer, 2007 Jun;15(6):613-20, Epub 2007 Jan 18; Gerhardt et al, World J Gastroenterol, 2006 Sep 14;12(34):5495-500; McArdle et al, Urol Int, 2006;77(2):127-9; Guillem et al, Dis Esophagus, 2005;18(3):146-50; Brown et al, Cancer, 2005 Jan 15;103(2):377-82. Decreased serum albumin (hypoalbuminemia) is also associated with increased morbidity and mortality in many critical illnesses, including cancers (*e.g.,* Vigano et al., Arch Intern Med, 2000 Mar 27;160(6):861-8; Hauser et al., Support Care Cancer, 2006 Oct;14(10):999-1011; Seve et al., Cancer, 2006 Dec 1;107(11):2698-705). The apparent link between hypoalbuminemia and poor patient outcome might suggest that restoring albumin levels through direct albumin infusion could promote patient survival, however, albumin infusion alone has not improved survival of patients with advanced cancer (Demirkazik et al., Proc Am Soc Clin Oncol 21: 2002 (abstr 2892)) or other critically ill patients groups (reviewed in Wilkes et al., Ann Intern Med, 2001 Aug 7;135(3):149-64).

The Glasgow Prognostic Score (GPS) is an inflammation-based prognostic score that combines levels of albumin (< 35 mg/L = 1 point) and CRP (> 10 mg/L = 1 point) (Forrest et al., Br J Cancer, 2004 May 4;90(9):1704-6). Since its introduction in 2004, the Glasgow Prognostic Score has already been shown to have prognostic value as a predictor of mortality in numerous cancers, including gastro-esophageal cancer, non-small-cell lung cancer, colorectal cancer, breast cancer, ovarian cancer, bronchogenic cancer, and metastatic renal cancer (Forrest et al., Br J Cancer, 2004 May 4;90(9):1704-6; Sharma et al., Clin Colorectal Cancer, 2008 Sep;7(5):331-7; Sharma et al., Eur J Cancer, 2008 Jan;44(2):251-6; McMillan et al., Nutr Cancer, 2001;41(1-2):64-9; McMillan, Proc Nutr Soc, 2008 Aug;67(3):257-62; Ramsey et al., Cancer, 2007 Jan 15;109(2):205-12).

U.S. patent application publication no. 20080081041 (relating to treatment of cancer using an anti-IL-6 antibody) discloses that since IL-6 is associated with disease activity and since CRP is a surrogate marker of IL-6 activity, sustained suppression of CRP by neutralization of IL-6 by their anti-IL-6 antibody (CNTO 328, Zaki et al., Int J Cancer, 2004 Sep 10;111(4):592-5) may be assumed necessary to achieve biological activity. The same patent application indicates that the relationship between IL-6 and CRP in patients with benign and malignant prostate disease was previously examined by McArdle (McArdle et al. 2004 Br J Cancer 91(10): 1755-1757). McArdle reportedly found no significant differences between the concentrations of IL-6 and CRP in the patients with benign disease compared with prostate cancer patients, in the cancer patients there was a significant increase in both IL-6 and CRP concentration with increasing tumor grade. The median serum CRP value for the 86 subjects with prostate cancer was 1.8 mg/L. Based thereon the inventors in the above-referenced patent application postulate a proposed dose and schedule wherein 6 mg/kg of an anti-IL-6 antibody (CNTO 328) is administered every 2 weeks and allege that this is likely to achieve sustained suppression of CRP in subjects with metastatic HRPC.

IL-6 signaling is mediated by the Jak-Tyk family of cytoplasmic tyrosine kinases, including JAK1, JAK2, and JAK3 (reviewed in Murray J Immunol. 2007 Mar 1;178(5):2623-9). Sivash et al. report abrogation of IL-6-mediated JAK signaling by the cyclopentenone prostaglandin 15d-PGJ2 in oral squamous carcinoma cells. British Journal of Cancer (2004) 91, 1074-1080. These results suggest that inhibitors of JAK1, JAK2, or JAK3 could be employed as antagonists of IL-6.

Ulanova et al. report that inhibition of the nonreceptor protein tyrosine kinase Syk (using siRNA) decreased production of IL-6 by epithelial cells. Am J Physiol Lung Cell Mol Physiol. 2005 Mar;288(3):L497-507. These results suggest that an inhibitor of Syk could be employed as an antagonist of IL-6.

Kedar et al. report that treatment with thalidomide significantly reduced serum levels of CRP and IL-6 to normal or near normal levels in a substantial fraction of renal cell carcinoma patients. Int J Cancer. 2004 Jun 10;110(2):260-5. These results suggest that thalidomide, and possibly derivatives thereof, such as lenalidomide, may be useful antagonists of IL-6.

In addition, another published patent application, US 20070292420 teaches a Phase I dose escalating study using an anti-IL-6 (cCLB-8) antibody for treating refractory patients with advanced stage multiple myeloma (N=12) and indicate that this study demonstrated that some patients had disease stabilization. The application also reports that after discontinuation of treatment there was acceleration in the increase of M protein levels, suggesting disease rebound after the withdrawal of therapy. Anti-IL-6 cCLB-8 antibody inhibited free circulating IL-6.

The application also indicates that this antibody trial resulted in no toxicity (except transient thrombocytopenia in two heavily pretreated patients) or allergic reactions were observed and that C-reactive protein (*CRP*) decreased below detection level in all patients. Their antibody (cCLB-8 antibody) reportedly possessed a circulating half-life of 17.8 days, and that there was no human anti-chimeric antibody (HACA) immune response observed (van Zaanen et al., British Journal of Haematology, 1998, 102, 783-790). They allege that the administration of CNTO 328 did not cause changes in blood pressure, pulse rate, temperature, hemoglobin, liver functions and renal functions. Except for transient thrombocytopenia in two heavily pretreated patients, no toxicity or allergic reactions allegedly were observed, and there was no human anti-chimeric antibody (HACA) immune response observed. Three patients in their study reportedly developed infection-related complications during therapy, however, a possible relation with anti-IL-6 cCLB-8 antibody was concluded by the inventors to be unlikely because infectious complications are reportedly common in end stage multiple myeloma and are a major cause of death. They conclude based on their results that this anti-IL-6 cCLB-8 antibody was safe in multiple myeloma patients.

Certain of the anti-IL-6 antibodies disclosed herein have also been disclosed in the following published and unpublished patent applications, which are co-owned by the assignee of the present application: U.S. 2009/0028784, WO 2008/144763, U.S. 2009/0297513and U.S. 2009/0297436.

Other anti-IL-6 antibodies have been disclosed in the following U.S. patents and published patent applications: 7,482,436; 7,291,721; 6,121,423; 2008/0075726; 2007/0178098; 2007/0154481; 2006/0257407; and 2006/0188502.

As noted above, elevated IL-6 has been implicated in pathogenesis of cachexia, weakness, fatigue, and fever. Diseases and disorders associated with fatigue include, but are not limited to, general fatigue, stress-related fatigue, exercise-induced fatigue, cancer-related fatigue, inflammatory disease-related fatigue and chronic fatigue syndrome. *See,* for example, Esper DH, et al, The cancer cachexia syndrome: a review of metabolic and clinical manifestations, Nutr Clin Pract., 2005 Aug;20 (4):369-76; Vgontzas AN, et al, IL-6 and its circadian secretion in humans, Neuroimmunomodulation, 2005;12(3):131-40; Robson-Ansley, PJ, et al, Acute interleukin-6 administration impairs athletic performance in healthy, trained male runners, Can J Appl Physiol., 2004 Aug;29(4):411-8; Shephard RJ., Cytokine responses to physical activity, with particular reference to IL-6: sources, actions, and clinical implications, Crit Rev Immunol., 2002;22(3):165-82; Arnold, MC, et al, Using an interleukin-6 challenge to evaluate neuropsychological performance in chronic fatigue syndrome, Psychol Med., 2002 Aug;32(6): 1075-89; Kurzrock R., The role of cytokines in cancer-related fatigue, Cancer, 2001 Sep 15;92(6 Suppl):1684-8; Nishimoto N, et al, Improvement in Castleman's disease by humanized anti-interleukin-6 receptor antibody therapy, Blood, 2000 Jan 1; 95 (1):56-61; Vgontzas AN, et al, Circadian interleukin-6 secretion and quantity and depth of sleep, J Clin Endocrinol Metab., 1999 Aug;84(8):2603-7; and Spath-Schwalbe E, et al, Acute effects of recombinant human interleukin 6 on endocrine and central nervous sleep functions in healthy men, J Clin Endocrinol Metab., 1998 May;83(5): 1573-9.

Diseases and disorders associated with cachexia include, but are not limited to, cancer-related cachexia, cardiac-related cachexia, respiratory-related cachexia, renal-related cachexia and age-related cachexia. *See,* for example, Barton, BE., Interleukin-6 and new strategies for the treatment of cancer, hyperproliferative diseases and paraneoplastic syndromes, Expert Opin Ther Targets, 2005 Aug;9(4):737-52; Zaki MH, et al, CNTO 328, a monoclonal antibody to IL-6, inhibits human tumor-induced cachexia in nude mice, Int J Cancer, 2004 Sep 10;111(4):592-5; Trikha M, et al, Targeted anti-interleukin-6 monoclonal antibody therapy for cancer: a review of the rationale and clinical evidence, Clin Cancer Res., 2003 Oct 15;9(13):4653-65; Lelli G, et al, Treatment of the cancer anorexia-cachexia syndrome: a critical reappraisal, J Chemother., 2003 Jun;15(3):220-5; Argiles JM, et al, Cytokines in the pathogenesis of cancer cachexia, Curr Opin Clin Nutr Metab Care, 2003 Jul;6(4):401-6; Barton BE., IL-6-like cytokines and cancer cachexia: consequences of chronic inflammation, Immunol Res., 2001;23(1):41-58; Yamashita JI, et al, Medroxyprogesterone acetate and cancer cachexia: interleukin-6 involvement, Breast Cancer, 2000;7(2): 130-5; Yeh SS, et al, Geriatric cachexia: the role of cytokines, Am J Clin Nutr., 1999 Aug;70(2):183-97; Strassmann G, et al, Inhibition of experimental cancer cachexia by anti-cytokine and anti-cytokine-receptor therapy, Cytokines Mol Ther., 1995 Jun;1(2):107-13; Fujita J, et al, Anti-interleukin-6 receptor antibody prevents muscle atrophy in colon-26 adenocarcinoma-bearing mice with modulation of lysosomal and ATP-ubiquitin-dependent proteolytic pathways, Int J Cancer, 1996 Nov 27;68(5):637-43; Tsujinaka T, et al, Interleukin 6 receptor antibody inhibits muscle atrophy and modulates proteolytic systems in interleukin 6 transgenic mice, J Clin Invest., 1996 Jan 1;97(1):244-9; Emilie D, et al, Administration of an anti-interleukin-6 monoclonal antibody to patients with acquired immunodeficiency syndrome and lymphoma: effect on lymphoma growth and on B clinical Symptoms, Blood, 1994 Oct 15;84 (8):2472-9; and Strassmann G, et al, Evidence for the involvement of interleukin 6 in experimental cancer cachexia, J Clin Invest., 1992 May;89(5):1681-4.

Another cachexia-related disease is failure to thrive, also known as faltering growth, in which a child exhibits a rate of weight gain less than expected. Failure to thrive is typically defined as weight below the third percentile or a decrease in the percentile rank of 2 major growth parameters in a short period. Failure to thrive results from heterogeneous medical and psychosocial causes, and the cause sometimes eludes diagnosis. One recent study (totaling 34 patients) reported a statistically significant elevation in IL-6 levels in patients diagnosed with failure to thrive. Shaoul et al. J Pediatr Gastroenterol Nutr., 2003 Oct;37(4):487-91.

WO2008/065378 describes antibodies and fragments thereof having binding specificity for IL-6 which neutralize its biological effects as well as their use for treating inflammatory diseases. Hennigan et al. (2008, Therapeutics and Clinical Risk Management; 4(4): 767-775) describes IL-6 inhibitors specifically in the treatment of rheumatoid arthritis, and WO2004/039826 describes chimeric, humanized and/or CDR-grafted anti-IL-6 antibodies and their use for treating various immune disorders.

### BRIEF SUMMARY OF THE INVENTION

The present invention provides a composition comprising an anti-IL-6 antibody that comprises a light chain polypeptide that comprises the light chain variable region of SEQ ID NO:709 and the light chain constant region of SEQ ID NO:586; and a heavy chain polypeptide that comprises the heavy chain variable region of SEQ ID NO:657 and the heavy chain constant region of SEQ ID NO:588, wherein the anti-IL-6 antibody is aglycosylated, for use in a method of preventing or treating a condition in a human subject associated with elevated IL-6 selected from rheumatoid arthritis, cancer, advanced cancer, liver disease, renal disease, inflammatory bowel disease, celiac disease, trauma, burns, other diseases associated with reduced serum albumin, juvenile rheumatoid arthritis, psoriasis, psoriatic arthropathy, ankylosing spondylitis, systemic lupus erythematosus, Crohn's disease, ulcerative colitis, pemphigus, dermatomyositis, polymyositis, polymyalgia rheumatica, giant cell arteritis, vasculitis, polyarteritis nodosa, Wegener's granulomatosis, Kawasaki disease, isolated CNS vasculitis, Churg-Strauss arteritis, microscopic polyarteritis, microscopic polyangiitis, Henoch-Schönlein purpura, essential cryoglobulinemic vasculitis, rheumatoid vasculitis, cryoglobulinemia, relapsing polychondritis, Behçet's disease, Takayasu's arteritis, ischemic heart disease, stroke, multiple sclerosis, sepsis, vasculitis secondary to viral infection (e.g., hepatitis B, hepatitis C, HIV, cytomegalovirus, Epstein-Barr virus, Parvo B 19 virus, etc.), Buerger's Disease, osteoarthritis, systemic sclerosis, CREST syndrome, Reiter's disease, Paget's disease of bone, Sjogren's syndrome, diabetes type 1, diabetes type 2, familial Mediterranean fever, autoimmune thrombocytopenia, autoimmune hemolytic anemia, autoimmune thyroid diseases, pernicious anemia, vitiligo, alopecia areata, primary biliary cirrhosis, autoimmune chronic active hepatitis, alcoholic cirrhosis, viral hepatitis including hepatitis B and C, idiopathic pulmonary fibrosis, chronic obstructive pulmonary disease, allergic asthma, or any combination thereof.

The present invention is an extension of Applicants' previous invention which is directed to specific antibodies, humanized or chimeric or single chain antibodies and fragments and variants thereof having binding specificity for IL-6, in particular antibodies having specific epitopic specificity and/or functional properties and novel therapies using these and other anti-IL-6 antibodies. The invention relates to specific humanized antibodies and fragments and variants thereof capable of binding to IL-6 and/or the IL-6/IL-6R complex. These antibodies may bind soluble IL-6 or cell surface expressed IL-6. Also, these antibodies may inhibit the formation or the biological effects of one or more of IL-6, IL-6/IL-6R complexes, IL-6/IL-6R/gp130 complexes and/or multimers of IL-6/IL-6R/gp130. Described herein are novel therapies and therapeutic protocols using anti-IL-6 antibodies, preferably those described herein. Also described herein are methods of preventing or treating thrombosis in a patient in need thereof, e.g., a patient showing elevated D-dimer and/or CRP levels prior to treatment, comprising administering to the patient an IL-6 antagonist, such as those identified infra, e.g., an anti-IL-6 antibody (such as Ab1) or antibody fragment or variant thereof, whereby the patient's coagulation profile is improved or restored to a normal condition. These methods may further include the administration of other actives such as statins that may further help (synergize) with the IL-6 antagonist such as Ab1 and thereby more effectively treat or prevent thrombosis.

The present invention also pertains to methods of improving survivability or quality of life of a patient in need thereof, *e.g.,* a patient showing elevated CRP levels and/or lowered albumin levels, comprising administering to the patient an IL-6 antagonist as defined in the claims, whereby the patient's C-reactive protein ("CRP") level is lowered, and/or the patient's albumin level is raised. These methods may further include the administration of other actives such as statins that may further help (synergize) with the IL-6 antagonist such as Ab1 and thereby more effectively treat the patient.

The invention relates to Ab1. In the human clinical trial data presented, a humanized form of Ab1 was administered.

This is effected by the administration of an antibody as defined in the claims. In accordance with the claims, these antibodies will be aglycosylated. In more specific embodiments of the invention these antibodies will block gp130 activation and/or possess binding affinities (Kds) less than 50 picomolar and/or K_{off} values less than or equal to 10⁻⁴ S⁻¹.

Also described herein are antibodies and humanized versions derived from rabbit immune cells (B lymphocytes) which may be selected based on their homology (sequence identity) to human germ line sequences. These antibodies may require minimal or no sequence modifications, thereby facilitating retention of functional properties after humanizationThese humanized antibodies may comprise human frameworks which are highly homologous (possess high level of sequence identity) to that of a parent (e.g. rabbit) antibody as described infra.

The subject antibodies may be selected based on their activity in functional assays such as IL-6 driven T1165 proliferation assays, and IL-6 simulated HepG2 haptoglobin production assays. Also described herein are fragments from anti-IL-6 antibodies encompassing V_{H}, V_{L} and CDR polypeptides or variants or fragments thereof, e.g., derived from rabbit immune cells and the polynucleotides encoding the same, as well as the use of these antibody fragments and the polynucleotides encoding them in the creation of novel antibodies and polypeptide compositions capable of recognizing IL-6 and/or IL-6/IL-6R complexes or IL-6/IL-6R/gp130 complexes and/or multimers thereof.

Described herein is the administration of conjugates of anti-IL-6 antibodies and humanized, chimeric or single chain versions thereof and other binding fragments and variants thereof conjugated to one or more functional or detectable moieties. Further described herein are methods of making said humanized anti-IL-6 or anti-IL-6/IL-6R complex antibodies and binding fragments and variants thereof. Binding fragments include, but are not limited to, Fab, Fab', F(ab')₂, Fv and scFv fragments.

The invention provides a composition comprising anti-IL-6 antibodies as defined in the claims for the prevention and treatment of a condition associated with elevated IL-6, wherein the condition is further defined in the claims. Also described is the use of fragments or variants of anti-IL-6 antibodies for the diagnosis, assessment and treatment of diseases and disorders associated with IL-6 or aberrant expression thereof. A preferred usage of the subject antibodies, is the treatment and prevention of rheumatoid arthritis.

Described herein is the production of anti-IL-6 antibodies in recombinant host cells, preferably diploid yeast such as diploid Pichia and other yeast strains.

The invention relates to methods of improving survivability or quality of life of a patient diagnosed with cancer, comprising administering to the patient an anti-IL-6 antibody as defined in the claims, whereby the patient's serum C-reactive protein ("CRP") level is stabilized and preferably reduced, and monitoring the patient to assess the reduction in the patient's serum CRP level.

The invention may relate to methods of improving muscular strength in a patient diagnosed with cancer, comprising administering to the patient an anti-IL-6 antibody as defined in the claims, whereby the patient's muscular strength is improved, and monitoring the patient to assess muscular strength. In such methods preferably the patient's muscular strength is improved by at least about 15% within approximately 4 weeks of administering the anti-IL-6 antibody or antibody fragment or variant thereof, as measured by the Hand Grip Strength test and more preferably the patient's muscular strength is improved by at least about 20% within approximately 4 weeks of administering the anti-IL-6 antibody or antibody fragment or variant thereof, as measured by the Hand Grip Strength test.

The invention may relate to methods of increasing serum albumin in a patient in need thereof, comprising administering to the patient an anti-IL-6 antibody as defined in the claims, whereby the patient's serum albumin level is improved, and monitoring the patient to assess serum albumin level. Preferably, these methods are effected under conditions whereby the patient's survivability is improved, and/or under conditions wherein the serum albumin level is increased by about 5-10 g/L, preferably 7-8 g/L, within approximately 6 weeks of administering the anti-IL-6 antibody or antibody fragment or variant thereof. These patients will include those diagnosed with rheumatoid arthritis, cancer, advanced cancer, liver disease, renal disease, inflammatory bowel disease, celiac disease, trauma, burns, or any combination thereof, provided the condition is associated with elevated IL-6.

The present invention provides a composition comprising an anti-IL-6 antibody as defined in the claims for use in a method of preventing or treating a condition in a human subject associated with elevated IL-6. In accordance with the claims the condition may be selected from rheumatoid arthritis, juvenile rheumatoid arthritis, psoriasis, psoriatic arthropathy, ankylosing spondylitis, systemic lupus erythematosus, Crohn's disease, ulcerative colitis, pemphigus, dermatomyositis, polymyositis, polymyalgia rheumatica, giant cell arteritis, vasculitis, polyarteritis nodosa, Wegener's granulomatosis, Kawasaki disease, isolated CNS vasculitis, Churg-Strauss arteritis, microscopic polyarteritis, microscopic polyangiitis, Henoch-Schönlein purpura, essential cryoglobulinemic vasculitis, rheumatoid vasculitis, cryoglobulinemia, relapsing polychondritis, Behcet's disease, Takayasu's arteritis, ischemic heart disease, stroke, multiple sclerosis, sepsis, vasculitis secondary to viral infection (e.g., hepatitis B, hepatitis C, HIV, cytomegalovirus, Epstein-Barr virus, Parvo B19 virus, etc.), Buerger's Disease, cancer, advanced cancer, Osteoarthritis, systemic sclerosis, CREST syndrome, Reiter's disease, Paget's disease of bone, Sjogran's syndrome, diabetes type 1, diabetes type 2, familial Mediterranean fever, autoimmune thrombocytopenia, autoimmune hemolytic anemia, autoimmune thyroid diseases, pernicious anemia, vitiligo, alopecia areata, primary biliary cirrhosis, autoimmune chronic active hepatitis, alcoholic cirrhosis, viral hepatitis including hepatitis B and C, other organ specific autoimmune diseases, burns, idiopathic pulmonary fibrosis, chronic obstructive pulmonary disease, allergic asthma, other allergic conditions or any combination thereof.

In an embodiment of the invention, the patient may have an elevated C-reactive protein (CRP) level prior to treatment.

The invention relates to methods of improving survivability or quality of life of a patient in need thereof, comprising administering to the patient an IL-6 antagonist as defined in the claims, whereby the patient's serum C-reactive protein ("CRP") level is reduced, and monitoring the patient to assess the reduction in the patient's serum CRP level.

The invention may relate to methods of improving survivability or quality of life of a patient in need thereof, comprising administering to the patient an IL-6 antagonist as defined in the claims, whereby the patient's serum albumin level is increased, and monitoring the patient to assess the increase in the patient's serum albumin level.

The invention may relate to methods of improving survivability or quality of life of a patient in need thereof, comprising administering to the patient an IL-6 antagonist as defined in the claims, whereby the patient's serum CRP level is reduced and the patient's serum albumin level is increased, and monitoring the patient to assess the reduction in the patient's serum CRP level and the increase in the patient's serum albumin level.

Described herein are methods of preventing or treating thrombosis in a patient in a state of hypercoagulation, comprising administering to the patient an IL-6 antagonist, e.g. an anti-IL-6 antibody (e.g., Ab1) and chimeric, humanized, single chain antibodies and fragments thereof (containing one or more CDRs of the afore-identified antibodies) that specifically bind IL-6, which preferably are aglycosylated, whereby the patient's coagulation profile is improved or restored to a normal condition, and monitoring the patient to assess coagulation profile. The anti-IL-6 antibody may comprise a humanized antibody containing the CDRs of Ab1 and more preferably may comprise the variable heavy and light chain in SEQ ID NO: 657 and SEQ ID NO: 709 respectively and the constant regions in SEQ ID NO: 588 and 586 respectively or variants thereof wherein one or more amino acids are modified by substitution or deletion without substantially disrupting IL-6 binding affinity.

In such methods if the IL-6 antagonist is an anti-IL-6 antibody or antibody fragment or variant thereof preferably this antibody may specifically bind to the same linear or conformational epitope(s) and/or compete for binding to the same linear or conformational epitope(s) on an intact human IL-6 polypeptide or fragment thereof as an anti-IL-6 antibody comprising Ab1 and fragments and variants thereof. The patient's coagulation profile may be assessed by measurement of the patient's serum level of one or more of D-dimer, Factor II, Factor V, Factor VIII, Factor IX, Factor XI, Factor XII, F/fibrin degradation products, thrombin-antithrombin III complex, fibrinogen, plasminogen, prothrombin, and von Willebrand factor and preferably by a method including measuring the patient's serum D-dimer level prior to administration of the anti-IL-6 antibody, and administering the anti-IL-6 antibody or antibody fragment or variant thereof if the patient's serum D-dimer level is elevated. In addition, the levels of C reactive protein may also be assessed in the patient prior to treatment and, if elevated, this may be used as a further indicator as to an increased risk of thrombosis in the patient.

Described herein are methods of treating a patient having a disease or condition associated with hypercoagulation, which may comprise administering to the patient an IL-6 antagonist such as Ab1, whereby the patient's coagulation profile is improved or restored to normal, and monitoring the patient to assess coagulation profile.

The patient may have elevated serum D-dimer levels prior to treatment.

The patient may have a reduced serum albumin level prior to treatment.

The patient's Glasgow Prognostic Score (GPS) may be improved following the treatment.

In an embodiment of the invention, the patient may have an elevated serum CRP level prior to treatment.

The method may further comprise the administration of at least one statin.

The IL-6 antagonist may target IL-6, IL-6 receptor alpha, gp130, p38 MAP kinase, JAK1, JAK2, JAK3, SYK, or any combination thereof.

The IL-6 antagonist may comprise an antibody, an antibody fragment, a peptide, a glycoalkoid, an antisense nucleic acid, a ribozyme, a retinoid, an avemir, a small molecule, or any combination thereof.

The IL-6 antagonist may comprise an anti-IL-6R, anti-gp130, anti-p38 MAP kinase, anti-JAK1, anti-JAK2, anti-JAK3, or anti-SYK antibody or antibody fragment.

The IL-6 antagonist may comprise a small molecule comprising thalidomide, lenalidomide, or any combination thereof.

The antagonist may comprise an anti-IL-6 antibody (e.g., Ab1) or antibody fragment or variant thereof.

The anti-IL-6 antibody or antibody fragment or variant thereof may specifically bind to the same linear or conformational epitope(s) and/or compete for binding to the same linear or conformational epitope(s) on an intact human IL-6 polypeptide or fragment thereof as an anti-IL-6 antibody comprising Ab1 and chimeric, humanized, single chain antibodies and fragments thereof (containing one or more CDRs of the afore-identified antibodies) that specifically bind IL-6, which preferably are aglycosylated.

The anti-IL-6 antibody may bind to the same linear or conformational epitope(s) and/or compete for binding to the same linear or conformational epitope(s) on an intact human IL-6 polypeptide or a fragment thereof as Ab1.

The anti-IL-6 antibody or antibody fragment or variant thereof may specifically bind to the same linear or conformational epitope(s) on an intact human IL-6 polypeptide or fragment thereof as an anti-IL-6 antibody comprising Ab1 and chimeric, humanized, single chain antibodies and fragments thereof (containing one or more CDRs of the afore-identified antibodies) that specifically bind IL-6, which preferably are aglycosylated.

The anti-IL-6 antibody or antibody fragment or variant thereof may specifically bind to the same linear or conformational epitope(s) on an intact human IL-6 polypeptide or a fragment thereof as Ab1.

The anti-IL-6 antibody or antibody fragment or variant thereof may specifically bind to the same linear or conformational epitopes on an intact IL-6 polypeptide or fragment thereof that is (are) specifically bound by Ab1 and wherein said epitope(s) when ascertained by epitopic mapping using overlapping linear peptide fragments which span the full length of the native human IL-6 polypeptide includes one or more residues comprised in IL-6 fragments selected from those respectively encompassing amino acid residues 37-51, amino acid residues 70-84, amino acid residues 169-183, amino acid residues 31-45 and/or amino acid residues 58-72.

The anti-IL-6 antibody or antibody fragment or variant thereof may comprise at least 2 complementarity determining regions (CDRs) in each the variable light and the variable heavy regions which are identical to those contained in an anti-IL-6 antibody comprising Ab1 and chimeric, humanized, single chain antibodies and fragments thereof (containing one or more CDRs of the afore-identified antibodies) that specifically bind IL-6, which preferably are aglycosylated. Antibodies containing these CDRs may be constructed using appropriate human frameworks based on the humanization methods disclosed herein.

The anti-IL-6 antibody or antibody fragment or variant thereof may comprise at least 2 complementarity determining regions (CDRs) in each the variable light and the variable heavy regions which are identical to those contained in Ab1.

All of the CDRs in the anti-IL-6 antibody or antibody fragment or variant thereof may be identical to the CDRs contained in an anti-IL-6 antibody comprising Ab1 and chimeric, humanized, single chain antibodies and fragments thereof (containing one or more CDRs of the afore-identified antibodies) that specifically bind IL-6, which preferably are aglycosylated.

All of the CDRs in the anti-IL-6 antibody or antibody fragment or variant thereof may be identical to one or more of the CDRs contained in Ab1.

In accordance with the claims, the anti-IL-6 antibody comprises all the CDRs in Ab1. In accordance with the claims, the anti-IL-6 antibody comprises the variable heavy and light chain sequences in SEQ ID NO: 657 and SEQ ID NO: 709.

In accordance with the claims, the humanized anti-IL-6 antibody comprises the variable heavy and variable light chain sequences respectively contained in SEQ ID NO: 657 and SEQ ID NO: 709, and further comprises the heavy chain and light chain constant regions respectively contained in SEQ ID NO: 588 and SEQ ID NO: 586. Further described herein are polynucleotides comprising, or alternatively consisting of, one or more of the nucleic acids encoding the variable heavy chain (SEQ ID NO: 700) and variable light chain (SEQ ID NO: 723) sequences and the constant region heavy chain (SEQ ID NO: 589) and constant region light chain (SEQ ID NO: 587) sequences. Described herein are nucleic acids encoding variants comprising one or more amino acid substitutions or deletions to the variable heavy and variable light chain sequences respectively contained in SEQ ID NO: 657 and SEQ ID NO: 709 and the heavy chain and light chain constant regions respectively contained in SEQ ID NO: 588 and SEQ ID NO: 586, that do not substantially affect IL-6 binding and/or desired effector function.

In accordance with the claims, the anti-IL-6 antibody is aglycosylated.

The anti-IL-6 antibody or antibody fragment or variant thereof may contain an Fc region that has been modified to alter effector function, half-life, proteolysis, and/or glycosylation. Preferably the Fc region is modified to eliminate glycosylation.

The anti-IL-6 antibody defined in the claims is a humanized antibody derived from a rabbit (parent) anti-IL-6 antibody.

The framework regions (FRs) in the variable light region and the variable heavy regions of described anti-IL-6 antibodies or antibody fragments or variants thereof respectively may be human FRs which are unmodified or which have been modified by the substitution of at most 2 or 3 human FR residues in the variable light or heavy chain region with the corresponding FR residues of the parent rabbit antibody, and the human FRs may have been derived from human variable heavy and light chain antibody sequences which have been selected from a library of human germline antibody sequences based on their high level of homology to the corresponding rabbit variable heavy or light chain regions relative to other human germline antibody sequences contained in the library. As disclosed in detail the antibody may comprise human FRs which are selected based on their high level of homology (degree of sequence identity) to that of the parent antibody that is humanized.

In an embodiment of the invention, the anti-IL-6 antibody as defined in the claims may be administered to the patient with a frequency at most once per period of approximately four weeks, approximately eight weeks, approximately twelve weeks, approximately sixteen weeks, approximately twenty weeks, or approximately twenty-four weeks.

The patient's coagulation profile may remain improved for an entire period intervening two consecutive anti-IL-6 antibody administrations.

In an embodiment of the invention, the patient's serum CRP level may remain decreased and/or serum albumin level may remain raised for an entire period intervening two consecutive anti-IL-6 antibody administrations.

The patient's cachexia, weakness, fatigue, and/or fever may remain improved for an entire period intervening two consecutive anti-IL-6 antibody administrations.

In an embodiment of the invention, the patient may have been diagnosed with cancer selected from Acanthoma, Acinic cell carcinoma, Acoustic neuroma, Acral lentiginous melanoma, Acrospiroma, Acute eosinophilic leukemia, Acute lymphoblastic leukemia, Acute megakaryoblastic leukemia, Acute monocytic leukemia, Acute myeloblastic leukemia with maturation, Acute myeloid dendritic cell leukemia, Acute myeloid leukemia, Acute promyelocytic leukemia, Adamantinoma, Adenocarcinoma, Adenoid cystic carcinoma, Adenoma, Adenomatoid odontogenic tumor, Adrenocortical carcinoma, Adult T-cell leukemia, Aggressive NK-cell leukemia, AIDS-Related Cancers, AIDS-related lymphoma, Alveolar soft part sarcoma, Ameloblastic fibroma, Anal cancer, Anaplastic large cell lymphoma, Anaplastic thyroid cancer, Angioimmunoblastic T-cell lymphoma, Angiomyolipoma, Angiosarcoma, Appendix cancer, Astrocytoma, Atypical teratoid rhabdoid tumor, Basal cell carcinoma, Basal-like carcinoma, B-cell leukemia, B-cell lymphoma, Bellini duct carcinoma, Biliary tract cancer, Bladder cancer, Blastoma, Bone Cancer, Bone tumor, Brain Stem Glioma, Brain Tumor, Breast Cancer, Brenner tumor, Bronchial Tumor, Bronchioloalveolar carcinoma, Brown tumor, Burkitt's lymphoma, Cancer of Unknown Primary Site, Carcinoid Tumor, Carcinoma, Carcinoma in situ, Carcinoma of the penis, Carcinoma of Unknown Primary Site, Carcinosarcoma, Castleman's Disease, Central Nervous System Embryonal Tumor, Cerebellar Astrocytoma, Cerebral Astrocytoma, Cervical Cancer, Cholangiocarcinoma, Chondroma, Chondrosarcoma, Chordoma, Choriocarcinoma, Choroid plexus papilloma, Chronic Lymphocytic Leukemia, Chronic monocytic leukemia, Chronic myelogenous leukemia, Chronic Myeloproliferative Disorder, Chronic neutrophilic leukemia, Clear-cell tumor, Colon Cancer, Colorectal cancer, Craniopharyngioma, Cutaneous T-cell lymphoma, Degos disease, Dermatofibrosarcoma protuberans, Dermoid cyst, Desmoplastic small round cell tumor, Diffuse large B cell lymphoma, Dysembryoplastic neuroepithelial tumor, Embryonal carcinoma, Endodermal sinus tumor, Endometrial cancer, Endometrial Uterine Cancer, Endometrioid tumor, Enteropathy-associated T-cell lymphoma, Ependymoblastoma, Ependymoma, Epithelioid sarcoma, Erythroleukemia, Esophageal cancer, Esthesioneuroblastoma, Ewing Family of Tumor, Ewing Family Sarcoma, Ewing's sarcoma, Extracranial Germ Cell Tumor, Extragonadal Germ Cell Tumor, Extrahepatic Bile Duct Cancer, Extramammary Paget's disease, Fallopian tube cancer, Fetus in fetu, Fibroma, Fibrosarcoma, Follicular lymphoma, Follicular thyroid cancer, Gallbladder Cancer, Gallbladder cancer, Ganglioglioma, Ganglioneuroma, Gastric Cancer, Gastric lymphoma, Gastrointestinal cancer, Gastrointestinal Carcinoid Tumor, Gastrointestinal Stromal Tumor, Gastrointestinal stromal tumor, Germ cell tumor, Germinoma, Gestational choriocarcinoma, Gestational Trophoblastic Tumor, Giant cell tumor of bone, Glioblastoma multiforme, Glioma, Gliomatosis cerebri, Glomus tumor, Glucagonoma, Gonadoblastoma, Granulosa cell tumor, Hairy Cell Leukemia, Hairy cell leukemia, Head and Neck Cancer, Head and neck cancer, Heart cancer, Hemangioblastoma, Hemangiopericytoma, Hemangiosarcoma, Hematological malignancy, Hepatocellular carcinoma, Hepatosplenic T-cell lymphoma, Hereditary breast-ovarian cancer syndrome, Hodgkin Lymphoma, Hodgkin's lymphoma, Hypopharyngeal Cancer, Hypothalamic Glioma, Inflammatory breast cancer, Intraocular Melanoma, Islet cell carcinoma, Islet Cell Tumor, Juvenile myelomonocytic leukemia, Kaposi Sarcoma, Kaposi's sarcoma, Kidney Cancer, Klatskin tumor, Krukenberg tumor, Laryngeal Cancer, Laryngeal cancer, Lentigo maligna melanoma, Leukemia, Leukemia, Lip and Oral Cavity Cancer, Liposarcoma, Lung cancer, Luteoma, Lymphangioma, Lymphangiosarcoma, Lymphoepithelioma, Lymphoid leukemia, Lymphoma, Macroglobulinemia, Malignant Fibrous Histiocytoma, Malignant fibrous histiocytoma, Malignant Fibrous Histiocytoma of Bone, Malignant Glioma, Malignant Mesothelioma, Malignant peripheral nerve sheath tumor, Malignant rhabdoid tumor, Malignant triton tumor, MALT lymphoma, Mantle cell lymphoma, Mast cell leukemia, Mediastinal germ cell tumor, Mediastinal tumor, Medullary thyroid cancer, Medulloblastoma, Medulloblastoma, Medulloepithelioma, Melanoma, Melanoma, Meningioma, Merkel Cell Carcinoma, Mesothelioma, Mesothelioma, Metastatic Squamous Neck Cancer with Occult Primary, Metastatic urothelial carcinoma, Mixed Müllerian tumor, Monocytic leukemia, Mouth Cancer, Mucinous tumor, Multiple Endocrine Neoplasia Syndrome, Multiple Myeloma, Multiple myeloma, Mycosis Fungoides, Mycosis fungoides, Myelodysplastic Disease, Myelodysplastic Syndromes, Myeloid leukemia, Myeloid sarcoma, Myeloproliferative Disease, Myxoma, Nasal Cavity Cancer, Nasopharyngeal Cancer, Nasopharyngeal carcinoma, Neoplasm, Neurinoma, Neuroblastoma, Neuroblastoma, Neurofibroma, Neuroma, Nodular melanoma, Non-Hodgkin Lymphoma, Non-Hodgkin lymphoma, Nonmelanoma Skin Cancer, Non-Small Cell Lung Cancer, Ocular oncology, Oligoastrocytoma, Oligodendroglioma, Oncocytoma, Optic nerve sheath meningioma, Oral Cancer, Oral cancer, Oropharyngeal Cancer, Osteosarcoma, Osteosarcoma, Ovarian Cancer, Ovarian cancer, Ovarian Epithelial Cancer, Ovarian Germ Cell Tumor, Ovarian Low Malignant Potential Tumor, Paget's disease of the breast, Pancoast tumor, Pancreatic Cancer, Pancreatic cancer, Papillary thyroid cancer, Papillomatosis, Paraganglioma, Paranasal Sinus Cancer, Parathyroid Cancer, Penile Cancer, Perivascular epithelioid cell tumor, Pharyngeal Cancer, Pheochromocytoma, Pineal Parenchymal Tumor of Intermediate Differentiation, Pineoblastoma, Pituicytoma, Pituitary adenoma, Pituitary tumor, Plasma Cell Neoplasm, Pleuropulmonary blastoma, Polyembryoma, Precursor T-lymphoblastic lymphoma, Primary central nervous system lymphoma, Primary effusion lymphoma, Primary Hepatocellular Cancer, Primary Liver Cancer, Primary peritoneal cancer, Primitive neuroectodermal tumor, Prostate cancer, Pseudomyxoma peritonei, Rectal Cancer, Renal cell carcinoma, Respiratory Tract Carcinoma Involving the NUT Gene on Chromosome 15, Retinoblastoma, Rhabdomyoma, Rhabdomyosarcoma, Richter's transformation, Sacrococcygeal teratoma, Salivary Gland Cancer, Sarcoma, Schwannomatosis, Sebaceous gland carcinoma, Secondary neoplasm, Seminoma, Serous tumor, Sertoli-Leydig cell tumor, Sex cord-stromal tumor, Sézary Syndrome, Signet ring cell carcinoma, Skin Cancer, Small blue round cell tumor, Small cell carcinoma, Small Cell Lung Cancer, Small cell lymphoma, Small intestine cancer, Soft tissue sarcoma, Somatostatinoma, Soot wart, Spinal Cord Tumor, Spinal tumor, Splenic marginal zone lymphoma, Squamous cell carcinoma, Stomach cancer, Superficial spreading melanoma, Supratentorial Primitive Neuroectodermal Tumor, Surface epithelial-stromal tumor, Synovial sarcoma, T-cell acute lymphoblastic leukemia, T-cell large granular lymphocyte leukemia, T-cell leukemia, T-cell lymphoma, T-cell prolymphocytic leukemia, Teratoma, Terminal lymphatic cancer, Testicular cancer, Thecoma, Throat Cancer, Thymic Carcinoma, Thymoma, Thyroid cancer, Transitional Cell Cancer of Renal Pelvis and Ureter, Transitional cell carcinoma, Urachal cancer, Urethral cancer, Urogenital neoplasm, Uterine sarcoma, Uveal melanoma, Vaginal Cancer, Verner Morrison syndrome, Verrucous carcinoma, Visual Pathway Glioma, Vulvar Cancer, Waldenström's macroglobulinemia, Warthin's tumor, Wilms' tumor, or any combination thereof.

In an embodiment of the invention, the patient may have been diagnosed with a cancer selected from Colorectal Cancer, Non-Small Cell Lung Cancer, Cholangiocarcinoma, Mesothelioma, Castleman's disease, Renal Cell Carcinoma, or any combination thereof.

The anti-IL-6 antibody defined in the claims is humanized.

In an embodiment of the invention, said anti-IL-6 antibody defined in the claims comprises a human Fc, e.g., an Fc region comprised of the variable heavy and light chain constant regions contained in SEQ ID NO: 704 and 702.

A human Fc may be derived from IgG1, IgG2, IgG3, IgG4, IgG5, IgG6, IgG7, IgG8, IgG9, IgG10, IgG11, IgG12, IgG13, IgG14, IgG15, IgG16, IgG17, IgG18 or IgG19.

Described herein is anti-IL-6 antibody or antibody fragment or variant thereof may comprise a polypeptide having at least 90% sequence homology to one or more of the polypeptide sequences of SEQ ID NO: **3, 18, 19, 652, 656, 657, 658, 661, 664, 665, 704, 708,** 2, 20, 647, 651, 660, 666, 699, 702, 706, and 709.

In an embodiment of the invention, the anti-IL-6 antibody as defined in the claims may have an elimination half-life of at least about 22 days, at least about 25 days, or at least about 30 days.

In an embodiment of the invention, the IL-6 antagonist as defined in the claims may be co-administered with a chemotherapy agent. In an embodiment of the invention, the chemotherapy agent include: VEGF antagonists, EGFR antagonists, platins, taxols, irinotecan, 5-fluorouracil, gemcytabine, leucovorine, steroids, cyclophosphamide, melphalan, vinca alkaloids (e.g., vinblastine, vincristine, vindesine and vinorelbine), mustines, tyrosine kinase inhibitors, radiotherapy, sex hormone antagonists, selective androgen receptor modulators, selective estrogen receptor modulators, PDGF antagonists, TNF antagonists, IL-1 antagonists, interleukins (e.g. IL-12 or IL-2), IL-12R antagonists, Toxin conjugated monoclonal antibodies, tumor antigen specific monoclonal antibodies, Erbitux™, Avastin™, Pertuzumab, anti-CD20 antibodies, Rituxan®, ocrelizumab, ofatumumab, DXL625, Herceptin®, or any combination thereof.

In an embodiment of the invention, the another therapeutic compound may be a statin.

In an embodiment of the invention, the anti-IL-6 antibody as defined in the claimsmay be directly or indirectly attached to a detectable label or therapeutic agent.

Described herein is the administration of an antagonist of a cachexia-associated factor, weakness-associated factor, fatigue-associated factor, and/or fever-associated factor. The cachexia-associated factor, weakness-associated factor, fatigue-associated factor, and/or fever-associated factor may be selected from tumor necrosis factor-alpha, Interferon gamma, Interleukin 1 alpha, Interleukin 1 beta, Interleukin 6, proteolysis inducing factor, leukemia-inhibitory factor, or any combination thereof.

Described herein is the administration of an anti-cachexia agent selected from cannabis, dronabinol (Marinol™), nabilone (Cesamet), cannabidiol, cannabichromene, tetrahydrocannabinol, Sativex, megestrol acetate, or any combination thereof.

Described herein is the administration of an anti-nausea or antiemetic agent selected from 5-HT3 receptor antagonists, ajwain, alizapride, anticholinergics, antihistamines, aprepitant, benzodiazepines, cannabichromene, cannabidiol, cannabinoids, cannabis, casopitant, chlorpromazine, cyclizine, dexamethasone, dexamethasone, dimenhydrinate (Gravol™), diphenhydramine, dolasetron, domperidone, dopamine antagonists, doxylamine, dronabinol (Marinol™), droperidol, emetrol, ginger, granisetron, haloperidol, hydroxyzine, hyoscine, lorazepam, meclizine, metoclopramide, midazolam, muscimol, nabilone (Cesamet), nk1 receptor antagonists, ondansetron, palonosetron, peppermint, Phenergan, prochlorperazine, Promacot, promethazine, Pentazine, propofol, sativex, tetrahydrocannabinol, trimethobenzamide, tropisetron, nandrolone, stilbestrol, thalidomide, lenalidomide, ghrelin agonists, myostatin antagonists, anti-myostatin antibodies, selective androgen receptor modulators, selective estrogen receptor modulators, angiotensin All antagonists, beta two adenergic receptor agonists, beta three adenergic receptor agonists, or any combination thereof.

Described herein is the administration of an anti-nausea or antiemetic agent selected from 5-HT3 receptor antagonists, ajwain, alizapride, anticholinergics, antihistamines, aprepitant, benzodiazepines, cannabichromene, cannabidiol, cannabinoids, cannabis, casopitant, chlorpromazine, cyclizine, dexamethasone, dexamethasone, dimenhydrinate (Gravol™), diphenhydramine, dolasetron, domperidone, dopamine antagonists, doxylamine, dronabinol (Marinol™), droperidol, emetrol, ginger, granisetron, haloperidol, hydroxyzine, hyoscine, lorazepam, meclizine, metoclopramide, midazolam, muscimol, nabilone (Cesamet), nk1 receptor antagonists, ondansetron, palonosetron, peppermint, Phenergan, prochlorperazine, Promacot, promethazine, Pentazine, propofol, sativex, tetrahydrocannabinol, trimethobenzamide, tropisetron, nandrolone, stilbestrol, thalidomide, lenalidomide, ghrelin agonists, myostatin antagonists, anti-myostatin antibodies, selective androgen receptor modulators, selective estrogen receptor modulators, angiotensin All antagonists, beta two adenergic receptor agonists, beta three adenergic receptor agonists, or any combination thereof.

The patient's fever may be assessed by measurement of patient's body temperature.

The method may include measuring the patient's body temperature prior to administration of the anti-IL-6 antibody, and administering the anti-IL-6 antibody or antibody fragment or variant thereof if the patient's body temperature is higher than about 38 °C.

The method may include measuring the patient's body temperature within 24 hours prior to administration of the anti-IL-6 antibody, and administering the anti-IL-6 antibody or antibody fragment or variant thereof if the patient's body temperature measurement indicates that a fever was present.

The method may further include measuring the patient's body weight prior to administration of the anti-IL-6 antibody, and administering the anti-IL-6 antibody or antibody fragment or variant thereof if the patient's weight has declined by greater than approximately 5% within approximately 30 days, or if the patient's lean body mass index is less than about 17 kg / m² (male patient) or less than about 14 kg / m² (female patient).

The method may include measuring the patient's muscular strength prior to administration of the anti-IL-6 antibody, and administering the anti-IL-6 antibody or antibody fragment or variant thereof if the patient's muscular strength has declined by greater than approximately 20% within approximately 30 days.

The method may result in a prolonged improvement in cachexia, weakness, fatigue, and/or fever in the patient.

The patient's body mass may be raised by approximately 1 kilogram within approximately 4 weeks of administration of the anti-IL-6 antibody or antibody fragment or variant thereof.

The patient's cachexia may be measurably improved within about 4 weeks of anti-IL-6 antibody administration.

The patient's cachexia may be assessed by measurement of the patient's total body mass, lean body mass, lean body mass index, and/or appendicular lean body mass.

The measurement of the patient's body mass may discount (subtract) the estimated weight of the patient's tumor(s) and/or extravascular fluid collection(s).

The patient's cachexia may remain measurably improved approximately 8 weeks after anti-IL-6 antibody administration.

The patient's weakness may be measurably improved within about 4 weeks of anti-IL-6 antibody administration.

The patient's weakness may be measured by the hand grip strength test.

The patient's hand grip strength may be improved by at least about 15%, or at least about 20%.

The patient's weakness may remain measurably improved approximately 8 weeks after anti-IL-6 antibody administration.

The patient's fatigue may be measurably improved within about 1 week of anti-IL-6 antibody administration.

The patient's fatigue may be measured by the FACIT-F FS test.

The patient's FACIT-F FS score may be improved by at least about 10 points.

The patient's fatigue may remain measurably improved approximately 8 weeks after anti-IL-6 antibody administration.

The patient's fever may be measurably improved within about 1 week of anti-IL-6 antibody administration.

The patient's fever may remain measurably improved approximately 8 weeks after anti-IL-6 antibody administration.

The patient's quality of life may be improved.

Described herein is the administration of one or more anti-coagulants or statins.

The one or more anti-coagulants may be selected from abciximab (ReoPro™), acenocoumarol, antithrombin III, argatroban, aspirin, bivalirudin (Angiomax™), clopidogrel, dabigatran, dabigatran etexilate (Pradaxa™/Pradax™), desirudin (Revasc™/Iprivask™), dipyridamole, eptifibatide (Integrilin™), fondaparinux, heparin, hirudin, idraparinux, lepirudin (Refludan™), low molecular weight heparin, melagatran, phenindione, phenprocoumon, ticlopidine, tirofiban (Aggrastat™), warfarin, ximelagatran, ximelagatran (Exanta™/ Exarta™), or any combination thereof.

The one or more statins may be selected from atorvastatin, cerivastatin, fluvastatin, lovastatin, mevastatin, pitavastatin, pravastatin, rosuvastatin, simvastatin, or any combination thereof.

The patient's coagulation profile may be assessed by measurement of the patient's serum level of one or more of D-dimer, Factor II, Factor V, Factor VIII, Factor IX, Factor XI, Factor XII, F/fibrin degradation products, thrombin-antithrombin III complex, fibrinogen, plasminogen, prothrombin, and von Willebrand factor.

The patient's coagulation profile may be assessed by a functional measurement of clotting ability.

The functional measurement of clotting ability may be selected from prothrombin time (PT), prothrombin ratio (PR), international normalized ratio (INR), or any combination thereof.

The method may include measuring the patient's international normalized ratio (INR) prior to administration of the IL-6 antagonist, and administering to the patient an IL-6 antagonist such as Ab1 if the patient's INR is less than about 0.9.

The method may include measuring the patient's international normalized ratio (INR) prior to administration of the IL-6 antagonist, and administering to the patient an IL-6 antagonist such as Ab1 if the patient's INR is less than about 0.5. 54.) The patient's INR may be raised to more than approximately 0.9 within 4 weeks of administering to the patient an IL-6 antagonist.

The method may include measuring the patient's serum D-dimer level prior to administration of the IL-6 antagonist, and administering the IL-6 antagonist such as Ab1 if the patient's serum D-dimer level is above the normal reference range.

The patient's serum D-dimer level may be lowered to less than the upper limit of the normal reference range within 4 weeks of administering to the patient an IL-6 antagonist.

The method may result in a prolonged improvement in the patient's coagulation profile.

The patient's coagulation profile may be measurably improved within about 2 weeks of administration of the IL-6 antagonist.

The patient's coagulation profile may remain measurably improved approximately 12 weeks after administering to the patient an IL-6 antagonist.

The patient's survivability may be improved.

The IL-6 antagonist is an anti-IL-6 antibody as defined in the claims.

The IL-6 antagonist may be coupled to a half-life increasing moiety.

The present invention provides a composition comprising an anti-IL-6 antibody as defined in the claims for use in a method of preventing or treating a condition in a human subject associated with elevanted IL-6, wherein the condition is further defined in the claims. In an embodiment of the invention, the method may include measuring the patient's serum CRP level prior to administration of the anti-IL-6 antibody, and administering the anti-IL-6 antibody if the patient's serum CRP level is at least approximately 5 mg/L.

In an embodiment of the invention, the patient's serum CRP level may be reduced to less than approximately 5 mg/L within 1 week of administration of the IL-6 antagonist.

In an embodiment of the invention, the patient's serum CRP level may be reduced to below 1 mg/L within 1 week of administration of the IL-6 antagonist.

In an embodiment of the invention, treatment may result in a prolonged reduction in serum CRP level of the patient.

In an embodiment of the invention, the patient's serum CRP level may be reduced to below 10 mg/L within about 1 week of IL-6 antagonist administration.

In an embodiment of the invention, 14 days after IL-6 antagonist administration the patient's serum CRP level may remain below 10 mg/L.

In an embodiment of the invention, 21 days after IL-6 antagonist administration the patient's serum CRP level may remain below 10 mg/L.

In an embodiment of the invention, 28 days after IL-6 antagonist administration the patient's serum CRP level may remain below 10 mg/L.

In an embodiment of the invention, 35 days after IL-6 antagonist administration the patient's serum CRP level may remain below 10 mg/L.

In an embodiment of the invention, 42 days after IL-6 antagonist administration the patient's serum CRP level may remain below 10 mg/L.

In an embodiment of the invention, 49 days after IL-6 antagonist administration the patient's serum CRP level may remain below 10 mg/L.

In an embodiment of the invention, 56 days after IL-6 antagonist administration the patient's serum CRP level may remain below 10 mg/L.

In an embodiment of the invention, the patient's survivability is improved.

The method may include measuring the patient's serum albumin level prior to administration of the IL-6 antagonist, and administering the IL-6 antagonist such as Ab1 if the patient's serum albumin level is less than approximately 35 g/L.

The patient's serum albumin level may be increased to more than approximately 35 g/L within about 5 weeks of administration of the IL-6 antagonist.

Treatment may result in a prolonged increase in serum albumin level of the patient.

42 days after IL-6 antagonist administration the patient's serum albumin level may remain above 35 g/L.

49 days after IL-6 antagonist administration the patient's serum albumin level may remain above 35 g/L.

56 days after IL-6 antagonist administration the patient's serum albumin level may remain above 35 g/L.

The patient's serum albumin level may be increased by about 5 g/L within approximately 5 weeks of administering the IL-6 antagonist.

The patient may have been diagnosed with rheumatoid arthritis, cancer, advanced cancer, liver disease, renal disease, inflammatory bowel disease, celiac disease, trauma, burns, other diseases associated with reduced serum albumin, or any combination thereof.

The method may further comprise administration of one or more statins to the patient, including without limitation thereto atorvastatin, cerivastatin, fluvastatin, lovastatin, mevastatin, pitavastatin, pravastatin, rosuvastatin, simvastatin, or any combination thereof.

Described herein is a composition comprising an IL-6 antagonist such as Ab1, and an anti-coagulant. The one or more anti-coagulants may be selected from abciximab (ReoPro™), acenocoumarol, antithrombin III, argatroban, aspirin, bivalirudin (Angiomax™), clopidogrel, dabigatran, dabigatran etexilate (Pradaxa™/Pradax™), desirudin (Revasc™/Iprivask™), dipyridamole, eptifibatide (Integrilin™), fondaparinux, heparin, hirudin, idraparinux, lepirudin (Refludan™), low molecular weight heparin, melagatran, phenindione, phenprocoumon, ticlopidine, tirofiban (Aggrastat™), warfarin, ximelagatran, ximelagatran (Exanta™/ Exarta™), or any combination thereof.

Described herein is a composition comprising an IL-6 antagonist such as Ab1, and a chemotherapy agent. The chemotherapy agent may be selected from VEGF antagonists, EGFR antagonists, platins, taxols, irinotecan, 5-fluorouracil, gemcytabine, leucovorine, steroids, cyclophosphamide, melphalan, vinca alkaloids (e.g., vinblastine, vincristine, vindesine and vinorelbine), mustines, tyrosine kinase inhibitors, radiotherapy, sex hormone antagonists, selective androgen receptor modulators, selective estrogen receptor modulators, PDGF antagonists, TNF antagonists, IL-1 antagonists, interleukins (e.g. IL-12 or IL-2), IL-12R antagonists, Toxin conjugated monoclonal antibodies, tumor antigen specific monoclonal antibodies, Erbitux™, Avastin™, Pertuzumab, anti-CD20 antibodies, Rituxan®, ocrelizumab, ofatumumab, DXL625, Herceptin®, or any combination thereof.

### BRIEF DESCRIPTION OF THE DRAWINGS

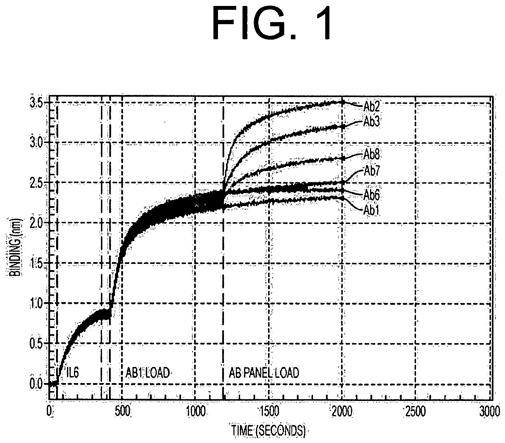

FIG. 1 shows that a variety of unique epitopes were recognized by the collection of anti-IL-6 antibodies prepared by the antibody selection protocol. Epitope variability was confirmed by antibody-IL-6 binding competition studies (ForteBio Octet).

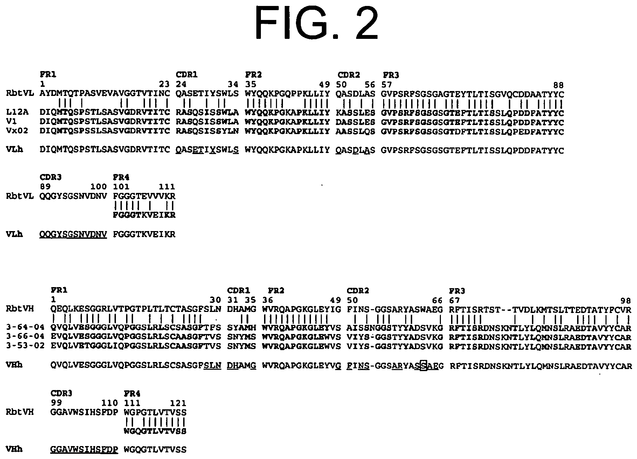

FIG. 2 shows alignments of variable light and variable heavy sequences between a rabbit antibody variable light and variable heavy sequences and homologous human sequences and the humanized sequences. Framework regions are identified FR1-FR4. Complementarity determining regions are identified as CDR1-CDR3. Amino acid residues are numbered as shown. The initial rabbit sequences are called RbtVL and RbtVH for the variable light and variable heavy sequences respectively. Three of the most similar human germline antibody sequences, spanning from Framework 1 through to the end of Framework 3, are aligned below the rabbit sequences. The human sequence that is considered the most similar to the rabbit sequence is shown first. In this example those most similar sequences are L12A for the light chain and 3-64-04 for the heavy chain. Human CDR3 sequences are not shown. The closest human Framework 4 sequence is aligned below the rabbit Framework 4 sequence. The vertical dashes indicate a residue where the rabbit residue is identical with one or more of the human residues at the same position. The bold residues indicate that the human residue at that position is identical to the rabbit residue at the same position. The final humanized sequences are called VLh and VHh for the variable light and variable heavy sequences respectively. The underlined residues indicate that the residue is the same as the rabbit residue at that position but different than the human residues at that position in the three aligned human sequences.
FIG. 3 demonstrates the high correlation between the IgG produced and antigen specificity for an exemplary IL-6 protocol. 9 of 11 wells showed specific IgG correlation with antigen recognition.
FIG. 4 provides the *alpha*-2-macroglobulin (A2M) dose response curve for antibody Ab1 administered intravenously at different doses one hour after a 100µg/kg s.c. dose of human IL-6.
FIG. 5 provides survival data for the antibody Ab1 progression groups versus control groups.
FIG. 6 provides additional survival data for the antibody Ab1 regression groups versus control groups.
FIG. 7 provides survival data for polyclonal human IgG at 10 mg/kg i.v. every three days (270-320 mg tumor size) versus antibody Ab1 at 10 mg/kg i.v. every three days (270-320 mg tumor size).
FIG. 8 provides survival data for polyclonal human IgG at 10 mg/kg i.v. every three days (400-527 mg tumor size) versus antibody Ab1 at 10 mg/kg i.v. every three days (400-527 mg tumor size).
FIG. 9 provides a pharmacokinetic profile of antibody Ab1 in cynomolgus monkey. Plasma levels of antibody Ab1 were quantitated through antigen capture ELISA. This protein displays a half life of between 12 and 17 days consistent with other full length humanized antibodies.
FIG. 10 (A-D) provides binding data for antibodies Ab4, Ab3, Ab8 and Ab2, respectively. FIG. 10E provides binding data for antibodies Ab1, Ab6 and Ab7.
FIG. 11 summarizes the binding data of FIG. 10 (A-E) in tabular form.
FIG. 12 presents the sequences of the 15 amino acid peptides used in the peptide mapping experiment of Example 14.
FIG. 13 presents the results of the blots prepared in Example 14.
FIG. 14 presents the results of the blots prepared in Example 14.
FIG. 15A shows affinity and binding kinetics of Ab1 for IL-6 of various species.
FIG. 15B demonstrates inhibition of IL-6 by Ab1 in the T1165 cell proliferation assay.
FIG. 16 shows the mean plasma concentration of Ab1 resulting from a single administration of Ab1 to healthy male subjects in several dosage groups.
FIG. 17 shows mean area under the plasma Ab1 concentration time curve (AUC) for the dosage groups shown in FIG. 16.
FIG. 18 shows mean peak plasma Ab1 concentration (Cmax) for the dosage groups shown in FIG. 16.
FIG. 19 summarizes Ab1 pharmacokinetic measurements of the dosage groups shown in FIG. 16.
FIG. 20 shows the mean plasma concentration of Ab1 resulting from a single administration of Ab1 to patients with advanced cancer.
FIG. 21 illustrates the unprecedented elimination half-life of Ab1 compared with other anti-IL-6 antibodies.
FIG. 22 shows increased hemoglobin concentration following administration of Ab1 to patients with advanced cancer.
FIG. 23 shows mean plasma lipid concentrations following administration of Ab1 to patients with advanced cancer.
FIG. 24 shows mean neutrophil counts following administration of Ab1 to patients with advanced cancer.
FIG. 25 demonstrates suppression of serum CRP levels in healthy individuals.
FIG. 26 (A-B) demonstrates suppression of serum CRP levels in advanced cancer patients.
FIG. 27 shows prevention of weight loss by Ab1 in a mouse cancer cachexia model.
FIG. 28 shows the physical appearance of representative Ab1-treated and control mice in a cancer cachexia model.
FIG. 29 demonstrates that Ab1 promotes weight gain in advanced cancer patients.
FIG. 30 demonstrates that Ab1 reduces fatigue in advanced cancer patients.
FIG. 31 demonstrates that Ab1 promotes hand grip strength in advanced cancer patients.
FIG. 32 demonstrates that Ab1 suppresses an acute phase protein (Serum Amyloid A) in mice.
FIG. 33 demonstrates that Ab1 increase plasma albumin concentration in advanced cancer patients.
Figs. 34 and 35 show alignments between a rabbit antibody light and variable heavy sequences and homologous human sequences and the humanized sequences. Framework regions are identified as FR1-FR4. Complementarity determining regions are identified as CDR1-CDR3.
FIGS. 36 and 37 show alignments between light and variable heavy sequences, respectively, of different forms of Ab1. Framework regions are identified as FR1-FR4. Complementarity determining regions are identified as CDR1-CDR3. Sequence differences within the CDR regions highlighted.
Fig. 38 shows the mean CRP values for each dosage concentrations (placebo, 80 mg, 160 mg, and 320 mg) of the Ab1 monoclonal antibody.
Fig. 39 shows the change in median values of CRP from each dosage concentration group corresponding to Fig. 38.
Fig. 40 shows a reduction in serum CRP levels in patients with various cancers after dosing at 80, 160 or 320 mg for 12 weeks.
Fig. 41 shows a reduction in serum CRP levels in the patient population with rheumatoid arthritis after dosing at 80, 160 and 320 mg for 12 weeks.
Fig. 42 demonstrates that Ab1 increases mean hemoglobin at 80, 160 and 320 mg after 12 weeks of dosing.
Figure 43 demonstrates mean change from baseline hemoglobin for the data presented in Figure 42.
Figure 44 demonstrates that Ab1 increases mean hemoglobin at 160 and 320 mg after 12 weeks of dosing in patients having baseline hemoglobin below 11 g/l.
Figure 45 demonstrates that Ab1 increases mean hemoglobin at 80, 160 and 320 mg after 16 weeks of dosing.
Figure 46 demonstrates that Ab1 increases mean albumin concentration at 80, 160 and 320 mg after 12 weeks of dosing.
Figure 47 demonstrates the change from baseline for mean albumin concentration from each dosage concentration group corresponding to Figure 46.
Figure 48 demonstrates that Ab1 provides sustained increases in mean albumin concentration at 160 and 320 mg after 12 weeks of dosing in patients having baseline albumin below 35 g/l.
Figure 49 demonstrates the averaged weight change data from each dosage concentration group (placebo, 80 mg, 160 mg, and 320 mg) of the Ab1 monoclonal antibody over 12 weeks.
Fig. 50 demonstrates the averaged percent change in body weight from each dosage concentration group corresponding to Fig. 49.
Figure 51 demonstrates the change in averaged lean body mass data for the dosage concentration groups corresponding to Figure 49.
Figure 52 demonstrates increases in the mean Facit-F FS subscale score for some of the dosage concentration groups in the patient population after dosing at 80, 160 and 320 mg after 8 weeks.
Fig. 53 demonstrates the change from baseline Facit-F FS subscale score corresponding to Fig. 52.
Figure 54 demonstrates that Ab1 drops D-dimer levels over placebo at 80, 160 and 320 mg after 16 weeks of dosing.
Figure 55 demonstrates the percent change from baseline in D-dimer concentration from each dosage concentration group corresponding to Figure 54.
Figure 56 demonstrating that treatment of patients with rheumatoid arthritis produced significant improvement over placebo based upon ACR metrics.
Figure 57 demonstrates patients achieving ACR 20 over placebo at 80, 160, and 320 mg after 16 weeks of dosing.
Figure 58 demonstrates patients achieving ACR 50 over placebo at 80, 160, and 320 mg after 16 weeks of dosing.
Figure 59 demonstrates patients achieving ACR 70 over placebo at 80, 160, and 320 mg after 16 weeks of dosing.
Figure 60 demonstrates the change from baseline for the components of the ACR metric for placebo, 80, 160, and 320 mg dosage concentration groups.
Figure 61 demonstrates the change in HAQ-DI scores for placebo, 80, 160, and 320 mg dosage concentration groups.
Figure 62 demonstrates the change in DAS28 scores for placebo, 80, 160, and 320 mg dosage concentration groups.
Figure 63 demonstrates the change in percentage of patients achieving EULAR good or moderate responses for placebo, 80, 160, and 320 mg dosage concentration groups.

### DETAILED DESCRIPTION

### Definitions

It is to be understood that this invention is not limited to the particular methodology, protocols, cell lines, animal species or genera, and reagents described, as such may vary. It is also to be understood that the terminology used herein is for the purpose of describing particular embodiments only, and is not intended to limit the scope of the present invention which will be limited only by the appended claims.

The term "variants" (as applied to antibodies including Ab1) includes single-chain antibodies, dimers, multimers, sequence variants, domain substitution variants, etc. Single-chain antibodies such as SMIPs, shark antibodies, nanobodies (e.g., Camelidiae antibodies). Sequence variants can be specified by percentage identity (or similarity) e.g., 99%, 95%, 90%, 85%, 80%, 70%, 60%, etc. or by numbers of permitted conservative or non-conservative substitutions. Domain substitution variants include replacement of a domain of one protein with a similar domain of a related protein. A similar domain may be identified by similarity of sequence, structure (actual or predicted), or function. For example, domain substitution variants include the substitution of one or more CDRs and/or framework regions.

As used herein the singular forms "a", "and", and "the" include plural referents unless the context clearly dictates otherwise. Thus, for example, reference to "a cell" includes a plurality of such cells and reference to "the protein" includes reference to one or more proteins and equivalents thereof known to those skilled in the art, and so forth. All technical and scientific terms used herein have the same meaning as commonly understood to one of ordinary skill in the art to which this invention belongs unless clearly indicated otherwise.

*Interleukin-6* (*IL-6*)*:* As used herein, interleukin-6 (IL-6) encompasses not only the following 212 amino acid sequence available as GenBank Protein Accession No. NP_000591: MNSFSTSAFGPVAFSLGLLLVLPAAFPAPVPPGEDSKDVAAPHRQPLTSSERIDKQIRY ILDGISALRKETCNKSNMCESSKEALAENNLNLPKMAEKDGCFQSGFNEETCLVKIIT GLLEFEVYLEYLQNRFESSEEQARAVQMSTKVLIQFLQKKAKNLDAITTPDPTTNASL LTKLQAQNQWLQDMTTHLILRSFKEFLQSSLRALRQM (SEQ ID NO: 1), but also any pre-pro, pro- and mature forms of this IL-6 amino acid sequence, as well as mutants and variants including allelic variants of this sequence.

*IL-6 antagonist:* As used herein, the terms "IL-6 antagonist," and grammatical variants thereof include any composition that prevents, inhibits, or lessens the effect(s) of IL-6 signaling. Generally, such antagonists may reduce the levels or activity of IL-6, IL-6 receptor alpha, gp130, or a molecule involved in IL-6 signal transduction, or may reduce the levels or activity complexes between the foregoing (e.g., reducing the activity of an IL-6 / IL-6 receptor complex). Antagonists include antisense nucleic acids, including DNA, RNA, or a nucleic acid analogue such as a peptide nucleic acid, locked nucleic acid, morpholino (phosphorodiamidate morpholino oligo), glycerol nucleic acid, or threose nucleic acid. See Heasman, Dev Biol. 2002 Mar 15;243(2):209-14; Hannon and Rossi, Nature. 2004 Sep 16;431(7006):371-8; Paul et al., Nat Biotechnol. 2002 May;20(5):505-8; Zhang et al., J Am Chem Soc. 2005 Mar 30;127(12):4174-5; Wahlestedt et al., Proc Natl Acad Sci U S A. 2000 May 9;97(10):5633-8; Hanvey *et al.,* 1992 Nov 27;258(5087):1481-5; Braasch et al., Biochemistry. 2002 Apr 9;41(14):4503-10; Schoning et al., Science. 2000 Nov 17;290(5495):1347-51. In addition IL-6 antagonists specifically include peptides that block IL-6 signaling such as those described in any of US Patent No. 6,599,875; 6,172, 042; 6,838,433; 6,841,533; 5,210,075 *et al.* Also, IL-6 antagonists according to the invention may include p38 MAP kinase inhibitors such as those reported in US20070010529 *et al.* given this kinase's role in cytokine production and more particularly IL-6 production. Further, IL-6 antagonists according to the invention include the glycoalkaloid compounds reported in US20050090453 as well as other IL-6 antagonist compounds isolatable using the IL-6 antagonist screening assays reported therein. Other IL-6 antagonists include antibodies, such as anti-IL-6 antibodies, anti-IL-6 receptor alpha antibodies, anti-gp130 antibodies, and anti-p38 MAP kinase antibodies including (but not limited to) the anti-IL-6 antibodies disclosed herein, Actemra™ (Tocilizumab), Remicade®, Zenapax™ (daclizumab), or any combination thereof. Other IL-6 antagonists include portions or fragments of molecules involved in IL-6 signaling, such as IL-6, IL-6 receptor alpha, and gp130, which may be native, mutant, or variant sequence, and may optionally be coupled to other moieties (such as half-life-increasing moieties, e.g. an Fc domain). For example, an IL-6 antagonist may be a soluble IL-6 receptor or fragment, a soluble IL-6 receptor:Fc fusion protein, a small molecule inhibitor of IL-6, an anti-IL-6 receptor antibody or antibody fragment or variant thereof, antisense nucleic acid, etc. Other IL-6 antagonists include avemirs, such as C326 (Silverman et al., Nat Biotechnol. 2005 Dec;23(12):1556-61) and small molecules, such as synthetic retinoid AM80 (tamibarotene) (Takeda et al., Arterioscler Thromb Vasc Biol. 2006 May;26(5):1177-83). Such IL-6 antagonists may be administered by any means known in the art, including contacting a subject with nucleic acids which encode or cause to be expressed any of the foregoing polypeptides or antisense sequences.

*Thrombosis:* As used herein, thrombosis refers to a thrombus (blood clot) inside a blood vessel. The term encompasses, without limitation, arterial and venous thrombosis, including deep vein thrombosis, portal vein thrombosis, jugular vein thrombosis, renal vein thrombosis, stroke, myocardial infarction, Budd-Chiari syndrome, Paget-Schroetter disease, and cerebral venous sinus thrombosis. Diseases and conditions associated with thrombosis include, without limitation, acute venous thrombosis, pulmonary embolism, thrombosis during pregnancy, hemorrhagic skin necrosis, acute or chronic disseminated intravascular coagulation (DIC), clot formation from surgery, long bed rest, long periods of immobilization, venous thrombosis, fulminant meningococcemia, acute thrombotic stroke, acute coronary occlusion, acute peripheral arterial occlusion, massive pulmonary embolism, axillary vein thrombosis, massive iliofemoral vein thrombosis, occluded arterial cannulae, occluded venous cannulae, cardiomyopathy, venoocclusive disease of the liver, hypotension, decreased cardiac output, decreased vascular resistance, pulmonary hypertension, diminished lung compliance, leukopenia, and thrombocytopenia.

*D-Dimer:* As used herein, D-dimer refers to a fibrin degradation product produced during the break down of blood clots by the enzyme plasmin. Monoclonal antibodies specifically reactive against D-dimer are readily available, e.g. DD-3B6/22 (Elms et al., 1986, Am J Clin Pathol. 85:360-4). Clinical measurements of D-dimer levels are routinely performed, *e.g.,* using a red blood cell agglutination test, ELISA, etc. (reviewed in Dempfle, Semin Vasc Med, 2005 Nov;5(4):315-20). Measurements of D-dimer may vary depending on the measurement method and testing lab; nonetheless, a normal "reference range" may be readily established for any particular method and testing lab, e.g. by taking measurements from healthy individuals. Accordingly, an elevated D-dimer level is understood by persons skilled in the art to refer to a D-dimer level that is above the reference range for the particular method and testing lab.

*Coagulation profile:* As used herein, coagulation profile refers generally to the functioning of the coagulation system. Both the tissue factor (extrinsic) and contact activation (intrinsic) pathways of clotting are components of the coagulation profile. A normal coagulation profile refers to coagulation functioning as in a normal, healthy individual, *i.e*., maintaining balance between ability to control bleeding and tendency towards excessive clotting (thrombotic tendency). An abnormal coagulation profile may be a decrease or an increase in coagulation tendency. One particularly abnormal coagulation profile is *hypercoagulation,* which refers to a greatly increased risk of excessive clot formation, resulting in high risk of thrombosis. Coagulation profile may be assessed by various tests and assays known in the art, such as: the activated partial thromboplastin time (aPTT) test; prothrombin time (PT) test (typical reference range of 12 to 15 second); measurements derived from the PT test, such as prothrombin ratio (PR) and international normalized ratio (INR) (typical reference range 0.8 to 1.2); fibrinogen testing (e.g. the Clauss method (Clauss A, "Rapid Physiological Coagulation Method for the Determination of Fibrinogen [German],"Acta Haematol, 1957, 17:237-46) or the Ellis method (Ellis BC and Stransky A, "A Quick and Accurate Method for the Determination of Fibrinogen in Plasma,"J Lab Clin Med, 1961, 58:477-88); assays for activated protein C resistance, protein C, protein S, and antithrombin; assays for antiphospholipid antibodies (lupus anticoagulant and anticardiolipin antibodies); elevated homocysteine; assays for plasminogen, dysfibrinogenemia, heparin cofactor II, or platelet hyperaggregability. Other assays useful to assess coagulation profile include measurement of clotting factors and/or indicators of clotting, such as serum levels of D-dimer, Factor II, Factor V, Factor VIII, Factor IX, Factor XI, Factor XII, F/fibrin degradation products, thrombin-antithrombin III complex, thrombocytosis, fibrinogen, plasminogen, prothrombin, and von Willebrand factor. *Worsening in coagulation profile* refers to a measureable change in an indicator of coagulation, *e.g.,* any of the aforementioned assays, that reflects a deterioration of the normal coagulation tendency, such that the measured value becomes abnormal or deviates farther from the normal range than previously. *Improvement in coagulation profile* refers to a measureable change in an indicator of coagulation, e.g., any of the aforementioned assays, that reflects a partial or full restoration of the normal coagulation tendency, i.e., after a therapeutic intervention, such as administration of an anti-IL-6 antibody, the measured value is in the normal range or closer to the normal range than prior to the therapeutic intervention.

*Disease or condition:* As used herein, "disease or condition" refers to a disease or condition that a patient has been diagnosed with or is suspected of having, particularly a disease or condition associated with elevated IL-6. A disease or condition encompasses, without limitation thereto, the side-effects of medications or treatments (such as radiation therapy), as well as idiopathic conditions characterized by symptoms that include elevated IL-6.

*Cachexia:* As used herein, cachexia, also known as wasting disease, refers to any disease marked especially by progressive emaciation, weakness, general ill health, malnutrition, loss of body mass, loss of muscle mass, or an accelerated loss of skeletal muscle in the context of a chronic inflammatory response (reviewed in Kotler, Ann Intern Med. 2000 Oct 17;133(8):622-34). Diseases and conditions in which cachexia is frequently observed include cancer, rheumatoid arthritis, AIDS, heart disease, dehydration, malnutrition, lead exposure, malaria, respiratory disease, old age, hypothyroidism, tuberculosis, hypopituitarism, neurasthenia, hypernatremia, hyponatremia, renal disease, splenica, ankylosing spondylitis, failure to thrive (faltering growth) and other diseases, particularly chronic diseases. Cachexia may also be idiopathic (arising from an uncertain cause). Weight assessment in a patient is understood to exclude growths or fluid accumulations, e.g. tumor weight, extravascular fluid accumulation, etc. Cachexia may be assessed by measurement of a patient's total body mass (exclusive of growths or fluid accumulations), total lean (fat-free) body mass, lean mass of the arms and legs (appendicular lean mass, e.g. measured using dual-energy x-ray absorptiometry or bioelectric impedance spectroscopy), and/or lean body mass index (lean body mass divided by the square of the patient's height). See Kotler, Ann Intern Med. 2000 Oct 17;133(8):622-34; Marcora et al., Rheumatology (Oxford). 2006 Nov;45(11):1385-8.

*Weakness:* As used herein, weakness refers physical fatigue, which typically manifests as a loss of muscle strength and/or endurance. Weakness may be central (affecting most or all of the muscles in the body) or peripheral (affecting a subset of muscles). Weakness includes "true weakness," in which a patient's muscles have a decrease in some measure of peak and/or sustained force output, and "perceived weakness," in which a patient perceives that a greater effort is required for performance of a task even though objectively measured strength remains nearly the same, and may be objectively measured or self-reported by the patient. For example, weakness may be objectively measured using the hand grip strength test (a medically recognized test for evaluating muscle strength), typically employing a handgrip dynamometer.

*Fatigue:* As used herein, fatigue refers to mental fatigue (for physical fatigue see "weakness"). Fatigue includes drowsiness (somnolence) and/or decreased attention. Fatigue may be measured using a variety of tests known in the art, such as the FACIT-F (Functional Assessment of Chronic Illness Therapy-Fatigue) test. See, e.g., Cella, D., Lai, J.S., Chang, C.H., Peterman, A., & Slavin, M. (2002). Fatigue in cancer patients compared with fatigue in the general population. Cancer, 94(2), 528-538; Cella, D., Eton, D.T., Lai,F J-S., Peterman, A.H & Merkel, D.E. (2002). Combining anchor and distribution based methods to derive minimal clinically important differences on the Functional Assessment of Cancer Therapy anemia and fatigue scales. Journal of Pain & Symptom Management, 24 (6) 547-561.

*Fever:* As used herein, "fever" refers to a body temperature set-point that is elevated by at least 1 to 2 degrees Celsius. Fever is often associated with a subjective feeling of hypothermia exhibited as a cold sensation, shivering, increased heart rate and respiration rate by which the individual's body reaches the increased set-point. As is well understood in the medical arts, normal body temperature typically varies with activity level and time of day, with highest temperatures observed in the afternoon and early evening hours, and lowest temperatures observed during the second half of the sleep cycle, and temperature measurements may be influenced by external factors such as mouth breathing, consumption of food or beverage, smoking, or ambient temperature (depending on the type of measurement). Moreover, the normal temperature set point for individuals may vary by up to about 0.5 degrees Celsius, thus a medical professional may interpret an individual's temperature in view of these factors to diagnose whether a fever is present. Generally speaking, a fever is typically diagnosed by a core body temperature above 38.0 degrees Celsius, an oral temperature above 37.5 degrees Celsius, or an axillary temperature above 37.2 degrees Celsius.

*Improved:* As used herein, "improved," "improvement," and other grammatical variants, includes any beneficial change resulting from a treatment. A beneficial change is any way in which a patient's condition is better than it would have been in the absence of the treatment. "Improved" includes prevention of an undesired condition, slowing the rate at which a condition worsens, delaying the development of an undesired condition, and restoration to an essentially normal condition. For example, improvement in cachexia encompasses any increase in patient's mass, such as total body mass (excluding weight normally excluded during assessment of cachexia, e.g. tumor weight, extravascular fluid accumulation, etc.), lean body mass, and/or appendicular lean mass, as well as any delay or slowing in the rate of loss of mass, or prevention or slowing of loss of mass associated with a disease or condition with which the patient has been diagnosed. For another example, improvement in weakness encompasses any increase in patient's strength, as well as any delay or slowing in the rate of loss of strength, or prevention or slowing of loss of strength associated with a disease or condition with which the patient has been diagnosed. For yet another example, improvement in fatigue encompasses any decrease in patient's fatigue, as well as any delay or slowing in the rate of increase of fatigue, or prevention or slowing of increase in fatigue associated with a disease or condition with which the patient has been diagnosed. For still another example, improvement in fever encompasses any decrease in patient's fever, as well as any delay or slowing in the rate of increase in fever, or prevention or slowing of increase in fever associated with a disease or condition with which the patient has been diagnosed.

*C-Reactive Protein (CRP):* As used herein, C-Reactive Protein (CRP) encompasses not only the following 224 amino acid sequence available as GenBank Protein Accession No. NP_000558:

MEKLLCFLVLTSLSHAFGQTDMSRKAFVFPKESDTSYVSLKAPLTKPLKAF TVCLHFYTELSSTRGYSIFSYATKRQDNEILIFWSKDIGYSFTVGGSEILFEVPEVTVAP VHICTSWESASGIVEFWVDGKPRVRKSLKKGYTVGAEASIILGQEQDSFGGNFEGSQS LVGDIGNVNMWDFVLSPDEINTIYLGGPFSPNVLNWRALKYEVQGEVFTKPQLWP (SEQ ID NO: 726), but also any pre-pro, pro- and mature forms of this CRP amino acid sequence, as well as mutants and variants including allelic variants of this sequence. CRP levels, e.g. in the serum, liver, tumor, or elsewhere in the body, can be readily measured using routine methods and commercially available reagents, e.g. ELISA, antibody test strip, immunoturbidimetry, rapid immunodiffusion, visual agglutination, Western blot, Northern blot, etc. As mentioned above CRP levels may in addition be measured in patients having or at risk of developing thrombosis according to the invention.

*Interleukin-6 receptor (IL-6R); also called IL-6 receptor alpha (IL-6RA):* As used herein, "interleukin-6 receptor" ("IL-6R"; also "IL-6 receptor alpha" or "IL-6RA") encompasses not only the following 468 amino acid sequence available as Swiss-Prot Protein Accession No. P08887: MLAVGCALLAALLAAPGAALAPRRCPAQEVARGVLTSLPGDSVTLTCPGVEPEDNA TVHWVLRKPAAGSHPSRWAGMGRRLLLRSVQLHDSGNYSCYRAGRPAGTVHLLVD VPPEEPQLSCFRKSPLSNVVCEWGPRSTPSLTTKAVLLVRKFQNSPAEDFQEPCQYSQ ESQKFSCQLAVPEGDSSFYIVSMCVASSVGSKFSKTQTFQGCGILQPDPPANITVTAV ARNPRWLSVTWQDPHSWNSSFYRLRFELRYRAERSKTFTTWMVKDLQHHCVIHDA WSGLRHVVQLRAQEEFGQGEWSEWSPEAMGTPWTESRSPPAENEVSTPMQALTTN KDDDNILFRDSANATSLPVQDSSSVPLPTFLVAGGSLAFGTLLCIAIVLRFKKTWKLR ALKEGKTSMHPPYSLGQLVPERPRPTPVLVPLISPPVSPSSLGSDNTSSHNRPDARDPR SPYDISNTDYFFPR (SEQ ID NO: 727), but also any pre-pro, pro- and mature forms of this amino acid sequence, as well as mutants and variants including allelic variants of this sequence.

*gp130:* As used herein, gp130 (also called Interleukin-6 receptor subunit beta) encompasses not only the following 918 precursor amino acid sequence available as Swiss-Prot Protein Accession No. P40189: MLTLQTWVVQALFIFLTTESTGELLDPCGYISPESPVVQLHSNFTAVCVLKEKCMDY FHVNANYIVWKTNHFTIPKEQYTIINRTASSVTFTDIASLNIQLTCNILTFGQLEQNVY GITIISGLPPEKPKNLSCIVNEGKKMRCEWDGGRETHLETNFTLKSEW ATHKFADCK AKRDTPTSCTVDYSTVYFVNIEVWVEAENALGKVTSDHINFDPVYKVKPNPPHNLSV INSEELSSILKLTWTNPSIKSVIILKYNIQYRTKDASTWSQIPPEDTASTRSSFTVQDLKP FTEYVFRIRCMKEDGKGYWSDWSEEASGITYEDRPSKAPSFWYKIDPSHTQGYRTVQ LVWKTLPPFEANGKILDYEVTLTRWKSHLQNYTVNATKLTVNLTNDRYLATLTVRN LVGKSDAAVLTIPACDFQATHPVMDLKAFPKDNMLWVEWTTPRESVKKYILEWCV LSDKAPCITDWQQEDGTVHRTYLRGNLAESKCYLITVTPVYADGPGSPESIKAYLKQ APPSKGPTVRTKKVGKNEAVLEWDQLPVDVQNGFIRNYTIFYRTIIGNETAVNVDSS HTEYTLSSLTSDTLYMVRMAAYTDEGGKDGPEFTFTTPKFAQGEIEAIVVPVCLAFLL TTLLGVLFCFNKRDLIKKHIWPNVPDPSKSHIAQWSPHTPPRHNFNSKDQMYSDGNF TDVSVVEIEANDKKPFPEDLKSLDLFKKEKINTEGHSSGIGGSSCMSSSRPSISSSDENE SSQNTSSTVQYSTVVHSGYRHQVPSVQVFSRSESTQPLLDSEERPEDLQLVDHVDGG DGILPRQQYFKQNCSQHESSPDISHFERSKQVSSVNEEDFVRLKQQISDHISQSCGSGQ MKMFQEVSAADAFGPGTEGQVERFETVGMEAATDEGMPKSYLPQTVRQGGYMPQ (SEQ ID NO: 728), but also any pre-pro, pro- and mature forms of this amino acid sequence, such as the mature form encoded by amino acids 23 through 918 of the sequence shown, as well as mutants and variants including allelic variants of this sequence.

*Glasgow Prognostic Score* (*GPS*)*:* As used herein, Glasgow Prognostic Score (GPS) refers to an inflammation-based prognostic score that awards one point for a serum albumin level less than < 35 mg/L and one point for a CRP level above 10 mg/L. Thus, a GPS of 0 indicates normal albumin and CRP, a GPS of 1 indicates reduced albumin or elevated CRP, and a GPS of 2 indicates both reduced albumin and elevated CRP.

*Effective amount:* As used herein, "effective amount," "amount effective to," "amount of X effective to" and the like, refer to an amount of an active ingredient that is effective to relieve or reduce to some extent one or more of the symptoms of the disease in need of treatment, or to retard initiation of clinical markers or symptoms of a disease in need of prevention, when the compound is administered. Thus, an effective amount refers to an amount of the active ingredient which exhibit effects such as (i) reversing the rate of progress of a disease; (ii) inhibiting to some extent further progress of the disease; and/or, (iii) relieving to some extent (or, preferably, eliminating) one or more symptoms associated with the disease. The effective amount may be empirically determined by experimenting with the compounds concerned in known in vivo and in vitro model systems for a disease in need of treatment. The context in which the phrase "effective amount" is used may indicate a particular desired effect. For example, "an amount of an anti-IL-6 antibody effective to prevent or treat a hypercoagulable state" and similar phrases refer to an amount of anti-IL-6 antibody that, when administered to a subject, will cause a measurable improvement in the subject's coagulation profile, or prevent, slow, delay, or arrest, a worsening of the coagulation profile for which the subject is at risk. Similarly, "an amount of an anti-IL-6 antibody effective to reduce serum CRP levels" and similar phrases refer to an amount of anti-IL-6 antibody that, when administered to a subject, will cause a measurable decrease in serum CRP levels, or prevent, slow, delay, or arrest, an increase in serum CRP levels for which the subject is at risk. Similarly, "an amount of an anti-IL-6 antibody effective to increase serum albumin levels" and similar phrases refer to an amount of anti-IL-6 antibody that, when administered to a subject, will cause a measurable increase in serum albumin levels, or prevent, slow, delay, or arrest, a decrease in serum albumin levels for which the subject is at risk. Similarly, "an amount of an anti-IL-6 antibody effective to reduce weakness" and similar phrases refer to an amount of anti-IL-6 antibody that, when administered to a subject, will cause a measurable decrease in weakness as determined by the hand grip strength test. Similarly, "an amount of an anti-IL-6 antibody effective to increase weight" and similar phrases refer to an amount of anti-IL-6 antibody that, when administered to a subject, will cause a measurable increase in a patient's weight. An effective amount will vary according to the weight, sex, age and medical history of the individual, as well as the severity of the patient's condition(s), the type of disease(s), mode of administration, and the like. An effective amount may be readily determined using routine experimentation, e.g., by titration (administration of increasing dosages until an effective dosage is found) and/or by reference to amounts that were effective for prior patients. Generally, the anti-IL-6 antibodies of the present invention will be administered in dosages ranging between about 0.1 mg/kg and about 20 mg/kg of the patient's body-weight.

*Prolonged improvement in coagulation profile:* As used herein, "prolonged improvement in coagulation profile" and similar phrases refer to a measurable improvement in the subject's coagulation profile relative to the initial coagulation profile (i.e. the coagulation profile at a time before treatment begins) that is detectable within about a week from when treatment begins (e.g. administration of an IL-6 antagonist such as Ab1) and remains improved for a prolonged duration, e.g., at least about 14 days, at least about 21 days, at least about 28 days, at least about 35 days, at least about 40 days, at least about 50 days, at least about 60 days, at least about 70 days, at least about 11 weeks, or at least about 12 weeks from when the treatment begins.

*Prolonged reduction in serum CRP:* As used herein, "prolonged reduction in serum CRP" and similar phrases refer to a measurable decrease in serum CRP level relative to the initial serum CRP level (i.e. the serum CRP level at a time before treatment begins) that is detectable within about a week from when a treatment begins (e.g. administration of an anti-IL-6 antibody) and remains below the initial serum CRP level for an prolonged duration, e.g. at least about 14 days, at least about 21 days, at least about 28 days, at least about 35 days, at least about 40 days, at least about 50 days, at least about 60 days, at least about 70 days, at least about 11 weeks, or at least about 12 weeks from when the treatment begins.

*Prolonged increase in serum albumin:* As used herein, "prolonged increase in serum albumin" and similar phrases refer to a measurable decrease in serum albumin level relative to the initial serum albumin level (i.e. the serum albumin level at a time before treatment begins) that is detectable within about a week from when a treatment begins (e.g. administration of an anti-IL-6 antibody) and remains above the initial serum albumin level for an prolonged duration, e.g. at least about 14 days, at least about 21 days, at least about 28 days, at least about 35 days, at least about 40 days, at least about 50 days, at least about 60 days, at least about 70 days, at least about 11 weeks, or at least about 12 weeks from when the treatment begins.

*Prolonged improvement in cachexia:* As used herein, "prolonged improvement in cachexia" refers to a measureable improvement patient's body mass, lean body mass, appendicular lean body mass, and/or lean body mass index, relative to the initial level (i.e. the level at a time before treatment begins) that is detectable within about 4 weeks and remains improved for a prolonged duration, e.g. at least about 35 days, at least about 40 days, at least about 50 days, at least about 60 days, at least about 70 days, at least about 11 weeks, or at least about 12 weeks from when the treatment begins.

*Prolonged improvement in weakness:* As used herein, "prolonged improvement in weakness" refers to a measureable improvement in muscular strength, relative to the initial level (i.e. the level at a time before treatment begins) that is detectable within about 2 weeks and remains improved for a prolonged duration, e.g. at least about 21 days, at least about 28 days, at least about 35 days, at least about 40 days, at least about 50 days, at least about 60 days, at least about 70 days, at least about 11 weeks, or at least about 12 weeks from when the treatment begins.

*Prolonged improvement in fatigue:* As used herein, "prolonged improvement in fatigue" refers to a measureable improvement in fatigue, relative to the initial level (i.e. the level at a time before treatment begins) that is detectable within about 1 week and remains improved for a prolonged duration, e.g. at least about 14 days, at least about 21 days, at least about 28 days, at least about 35 days, at least about 40 days, at least about 50 days, at least about 60 days, at least about 70 days, at least about 11 weeks, or at least about 12 weeks from when the treatment begins.

*Prolonged improvement in fever:* As used herein, "prolonged improvement in fever" refers to a measureable decrease in fever (e.g. peak temperature or amount of time that temperature is elevated), relative to the initial level (i.e. the level at a time before treatment begins) that is detectable within about 1 week and remains improved for a prolonged duration, e.g. at least about 14 days, at least about 21 days, at least about 28 days, at least about 35 days, at least about 40 days, at least about 50 days, at least about 60 days, at least about 70 days, at least about 11 weeks, or at least about 12 weeks from when the treatment begins.

*Mating competent yeast species:* This is intended to broadly encompass any diploid or tetraploid yeast which can be grown in culture. Such species of yeast may exist in a haploid, diploid, or tetraploid form. The cells of a given ploidy may, under appropriate conditions, proliferate for indefinite number of generations in that form. Diploid cells can also sporulate to form haploid cells. Sequential mating can result in tetraploid strains through further mating or fusion of diploid strains. The diploid or polyploidal yeast cells are preferably produced by mating or spheroplast fusion.

The mating competent yeast is a member of the *Saccharomycetaceae* family, which includes the genera *Arxiozyma*; *Ascobotryozyma*; *Citeromyces*; *Debaryomyces*; *Dekkera*; *Eremothecium*; *Issatchenkia*; *Kazachstania*; *Kluyveromyces*; *Kodamaea*; *Lodderomyces*; *Pachysolen*; *Pichia*; *Saccharomyces*; *Saturnispora*; *Tetrapisispora*; *Torulaspora*; *Williopsis*; and *Zygosaccharomyces.* Other types of yeast potentially useful in the invention include *Yarrowia, Rhodosporidium, Candida, Hansenula, Filobasium, Filobasidellla, Sporidiobolus, Bullera, Leucosporidium* and *Filobasidella.*

The mating competent yeast is a member of the genus *Pichia.* The mating competent yeast of the genus *Pichia* is one of the following species: *Pichia pastoris, Pichia methanolica,* and *Hansenula polymorpha* (*Pichia angusta*). The mating competent yeast of the genus *Pichia* is the species *Pichia pastoris.*

*Haploid Yeast Cell:* A cell having a single copy of each gene of its normal genomic (chromosomal) complement.

*Polyploid Yeast Cell:* A cell having more than one copy of its normal genomic (chromosomal) complement.

*Diploid Yeast Cell:* A cell having two copies (alleles) of essentially every gene of its normal genomic complement, typically formed by the process of fusion (mating) of two haploid cells.

*Tetraploid Yeast Cell:* A cell having four copies (alleles) of essentially every gene of its normal genomic complement, typically formed by the process of fusion (mating) of two haploid cells. Tetraploids may carry two, three, four, or more different expression cassettes. Such tetraploids might be obtained in *S*. *cerevisiae* by selective mating homozygotic heterothallic a/a and alpha/alpha diploids and in *Pichia* by sequential mating of haploids to obtain auxotrophic diploids. For example, a [met his] haploid can be mated with [ade his] haploid to obtain diploid [his]; and a [met arg] haploid can be mated with [ade arg] haploid to obtain diploid [arg]; then the diploid [his] x diploid [arg] to obtain a tetraploid prototroph. It will be understood by those of skill in the art that reference to the benefits and uses of diploid cells may also apply to tetraploid cells.

*Yeast Mating:* The process by which two haploid yeast cells naturally fuse to form one diploid yeast cell.

*Meiosis:* The process by which a diploid yeast cell undergoes reductive division to form four haploid spore products. Each spore may then germinate and form a haploid vegetatively growing cell line.

*Selectable Marker*: A selectable marker is a gene or gene fragment that confers a growth phenotype (physical growth characteristic) on a cell receiving that gene as, for example through a transformation event. The selectable marker allows that cell to survive and grow in a selective growth medium under conditions in which cells that do not receive that selectable marker gene cannot grow. Selectable marker genes generally fall into several types, including positive selectable marker genes such as a gene that confers on a cell resistance to an antibiotic or other drug, temperature when two ts mutants are crossed or a ts mutant is transformed; negative selectable marker genes such as a biosynthetic gene that confers on a cell the ability to grow in a medium without a specific nutrient needed by all cells that do not have that biosynthetic gene, or a mutagenized biosynthetic gene that confers on a cell inability to grow by cells that do not have the wild type gene; and the like. Suitable markers include but are not limited to: ZEO; G418; LYS3; MET1; MET3a; ADE1; ADE3; URA3; and the like.

*Expression Vector:* These DNA vectors contain elements that facilitate manipulation for the expression of a foreign protein within the target host cell. Conveniently, manipulation of sequences and production of DNA for transformation is first performed in a bacterial host, *e.g. E. coli,* and usually vectors will include sequences to facilitate such manipulations, including a bacterial origin of replication and appropriate bacterial selection marker. Selection markers encode proteins necessary for the survival or growth of transformed host cells grown in a selective culture medium. Host cells not transformed with the vector containing the selection gene will not survive in the culture medium. Typical selection genes encode proteins that (a) confer resistance to antibiotics or other toxins, (b) complement auxotrophic deficiencies, or (c) supply critical nutrients not available from complex media. Exemplary vectors and methods for transformation of yeast are described, for example, in Burke, D., Dawson, D., & Stearns, T. (2000). Methods in yeast genetics: a Cold Spring Harbor Laboratory course manual. Plainview, N.Y.: Cold Spring Harbor Laboratory Press.

Expression vectors will further include yeast specific sequences, including a selectable auxotrophic or drug marker for identifying transformed yeast strains. A drug marker may further be used to amplify copy number of the vector in a yeast host cell.

The polypeptide coding sequence of interest is operably linked to transcriptional and translational regulatory sequences that provide for expression of the polypeptide in yeast cells. These vector components may include, but are not limited to, one or more of the following: an enhancer element, a promoter, and a transcription termination sequence. Sequences for the secretion of the polypeptide may also be included, e.g. a signal sequence, and the like. A yeast origin of replication is optional, as expression vectors are often integrated into the yeast genome.

The polypeptide of interest may be operably linked, or fused, to sequences providing for optimized secretion of the polypeptide from yeast diploid cells.

Nucleic acids are "operably linked" when placed into a functional relationship with another nucleic acid sequence. For example, DNA for a signal sequence is operably linked to DNA for a polypeptide if it is expressed as a preprotein that participates in the secretion of the polypeptide; a promoter or enhancer is operably linked to a coding sequence if it affects the transcription of the sequence. Generally, "operably linked" means that the DNA sequences being linked are contiguous, and, in the case of a secretory leader, contiguous and in reading frame. However, enhancers do not have to be contiguous. Linking is accomplished by ligation at convenient restriction sites or alternatively via a PCR/recombination method familiar to those skilled in the art (Gateway^{R} Technology; Invitrogen, Carlsbad California). If such sites do not exist, the synthetic oligonucleotide adapters or linkers are used in accordance with conventional practice.

Promoters are untranslated sequences located upstream (5') to the start codon of a structural gene (generally within about 100 to 1000 bp) that control the transcription and translation of particular nucleic acid sequences to which they are operably linked. Such promoters fall into several classes: inducible, constitutive, and repressible promoters (that increase levels of transcription in response to absence of a repressor). Inducible promoters may initiate increased levels of transcription from DNA under their control in response to some change in culture conditions, *e.g.,* the presence or absence of a nutrient or a change in temperature.

The yeast promoter fragment may also serve as the site for homologous recombination and integration of the expression vector into the same site in the yeast genome; alternatively a selectable marker is used as the site for homologous recombination. *Pichia* transformation is described in Cregg et al. (1985) Mol. Cell. Biol. 5:3376-3385.

Examples of suitable promoters from *Pichia* include the AOX1 and promoter (Cregg et al. (1989) Mol. Cell. Biol. 9:1316-1323); ICL1 promoter (Menendez et al. (2003) Yeast 20(13):1097-108); glyceraldehyde-3-phosphate dehydrogenase promoter (GAP) (Waterham et al. (1997) Gene 186(1):37-44); and FLD1 promoter (Shen et al. (1998) Gene 216(1):93-102). The *GAP* promoter is a strong constitutive promoter and the AOX and FLD1 promoters are inducible.

Other yeast promoters include ADH1, alcohol dehydrogenase II, GAL4, PHO3, PHO5, Pyk, and chimeric promoters derived therefrom. Additionally, non-yeast promoters may be used such as mammalian, insect, plant, reptile , amphibian, viral, and avian promoters. Most typically the promoter will comprise a mammalian promoter (potentially endogenous to the expressed genes) or will comprise a yeast or viral promoter that provides for efficient transcription in yeast systems.

The polypeptides of interest may be produced recombinantly not only directly, but also as a fusion polypeptide with a heterologous polypeptide, *e.g.* a signal sequence or other polypeptide having a specific cleavage site at the N-terminus of the mature protein or polypeptide. In general, the signal sequence may be a component of the vector, or it may be a part of the polypeptide coding sequence that is inserted into the vector. The heterologous signal sequence selected preferably is one that is recognized and processed through one of the standard pathways available within the host cell. The *S*. *cerevisiae* alpha factor pre-pro signal has proven effective in the secretion of a variety of recombinant proteins from *P. pastoris.* Other yeast signal sequences include the alpha mating factor signal sequence, the invertase signal sequence, and signal sequences derived from other secreted yeast polypeptides. Additionally, these signal peptide sequences may be engineered to provide for enhanced secretion in diploid yeast expression systems. Other secretion signals of interest also include mammalian signal sequences, which may be heterologous to the protein being secreted, or may be a native sequence for the protein being secreted. Signal sequences include pre-peptide sequences, and in some instances may include propeptide sequences. Many such signal sequences are known in the art, including the signal sequences found on immunoglobulin chains, *e.g.,* K28 preprotoxin sequence, PHA-E, FACE, human MCP-1, human serum albumin signal sequences, human Ig heavy chain, human Ig light chain, and the like. For example, see Hashimoto et. al. Protein Eng 11(2) 75 (1998); and Kobayashi et. al. Therapeutic Apheresis 2(4) 257 (1998).

Transcription may be increased by inserting a transcriptional activator sequence into the vector. These activators are cis-acting elements of DNA, usually about from 10 to 300 bp, which act on a promoter to increase its transcription. Transcriptional enhancers are relatively orientation and position independent, having been found 5' and 3' to the transcription unit, within an intron, as well as within the coding sequence itself. The enhancer may be spliced into the expression vector at a position 5' or 3' to the coding sequence, but is preferably located at a site 5' from the promoter.

Expression vectors used in eukaryotic host cells may also contain sequences necessary for the termination of transcription and for stabilizing the mRNA. Such sequences are commonly available from 3' to the translation termination codon, in untranslated regions of eukaryotic or viral DNAs or cDNAs. These regions contain nucleotide segments transcribed as polyadenylated fragments in the untranslated portion of the mRNA.

Construction of suitable vectors containing one or more of the above-listed components employs standard ligation techniques or PCR/recombination methods. Isolated plasmids or DNA fragments are cleaved, tailored, and re-ligated in the form desired to generate the plasmids required or via recombination methods. For analysis to confirm correct sequences in plasmids constructed, the ligation mixtures are used to transform host cells, and successful transformants selected by antibiotic resistance (e.g. ampicillin or Zeocin™ (phleomycin)) where appropriate. Plasmids from the transformants are prepared, analyzed by restriction endonuclease digestion and/or sequenced.

As an alternative to restriction and ligation of fragments, recombination methods based on att sites and recombination enzymes may be used to insert DNA sequences into a vector. Such methods are described, for example, by Landy (1989) Ann.Rev.Biochem. 58:913-949; and are known to those of skill in the art. Such methods utilize intermolecular DNA recombination that is mediated by a mixture of lambda and *E.coli* -encoded recombination proteins. Recombination occurs between specific attachment (*att*) sites on the interacting DNA molecules. For a description of att sites see Weisberg and Landy (1983) Site-Specific Recombination in Phage Lambda, in Lambda II, Weisberg, ed.(Cold Spring Harbor, NY:Cold Spring Harbor Press), pp.211-250. The DNA segments flanking the recombination sites are switched, such that after recombination, the *att* sites are hybrid sequences comprised of sequences donated by each parental vector. The recombination can occur between DNAs of any topology.

*Att* sites may be introduced into a sequence of interest by ligating the sequence of interest into an appropriate vector; generating a PCR product containing *att* B sites through the use of specific primers; generating a cDNA library cloned into an appropriate vector containing att sites; and the like.

*Folding,* as used herein, refers to the three-dimensional structure of polypeptides and proteins, where interactions between amino acid residues act to stabilize the structure. While non-covalent interactions are important in determining structure, usually the proteins of interest will have intra- and/or intermolecular covalent disulfide bonds formed by two cysteine residues. For naturally occurring proteins and polypeptides or derivatives and variants thereof, the proper folding is typically the arrangement that results in optimal biological activity, and can conveniently be monitored by assays for activity, e.g. ligand binding, enzymatic activity, *etc.*

In some instances, for example where the desired product is of synthetic origin, assays based on biological activity will be less meaningful. The proper folding of such molecules may be determined on the basis of physical properties, energetic considerations, modeling studies, and the like.

The expression host may be further modified by the introduction of sequences encoding one or more enzymes that enhance folding and disulfide bond formation, *i.e.* foldases, chaperonins, *etc.* Such sequences may be constitutively or inducibly expressed in the yeast host cell, using vectors, markers, *etc.* as known in the art. Preferably the sequences, including transcriptional regulatory elements sufficient for the desired pattern of expression, are stably integrated in the yeast genome through a targeted methodology.

For example, the eukaryotic PDI is not only an efficient catalyst of protein cysteine oxidation and disulfide bond isomerization, but also exhibits chaperone activity. Co-expression of PDI can facilitate the production of active proteins having multiple disulfide bonds. Also of interest is the expression of BIP (immunoglobulin heavy chain binding protein); cyclophilin; and the like. Each of the haploid parental strains may express a distinct folding enzyme, *e.g.* one strain may express BIP, and the other strain may express PDI or combinations thereof.

The terms *"desired protein"* or *"target protein"* are used interchangeably and refer generally to a humanized antibody or a binding portion thereof described herein. The term "antibody" is intended to include any polypeptide chain-containing molecular structure with a specific shape that fits to and recognizes an epitope, where one or more non-covalent binding interactions stabilize the complex between the molecular structure and the epitope. The archetypal antibody molecule is the immunoglobulin, and all types of immunoglobulins, IgG, IgM, IgA, IgE, IgD, etc., from all sources, e.g. human, rodent, rabbit, cow, sheep, pig, dog, other mammals, chicken, other avians, etc., are considered to be "antibodies." A preferred source for producing antibodies useful as starting material is rabbits. Numerous antibody coding sequences have been described; and others may be raised by methods well-known in the art. Examples thereof include chimeric antibodies, human antibodies and other non-human mammalian antibodies, humanized antibodies, single chain antibodies such as scFvs, camelbodies, nanobodies, IgNAR (single-chain antibodies derived from sharks), small-modular immunopharmaceuticals (SMIPs), and antibody fragments such as Fabs, Fab', F(ab')2 and the like. *See* Streltsov VA, et al., Structure of a shark IgNAR antibody variable domain and modeling of an early-developmental isotype, Protein Sci. 2005 Nov;14(11):2901-9. Epub 2005 Sep 30; Greenberg AS, et al., A new antigen receptor gene family that undergoes rearrangement and extensive somatic diversification in sharks, Nature. 1995 Mar 9;374(6518):168-73; Nuttall SD, et al., Isolation of the new antigen receptor from wobbegong sharks, and use as a scaffold for the display of protein loop libraries, Mol Immunol. 2001 Aug;38(4):313-26; Hamers-Casterman C, et al., Naturally occurring antibodies devoid of light chains, Nature. 1993 Jun 3;363(6428):446-8; Gill DS, et al., Biopharmaceutical drug discovery using novel protein scaffolds, Curr Opin Biotechnol. 2006 Dec;17(6):653-8. Epub 2006 Oct 19.

For example, antibodies or antigen binding fragments or variants thereof may be produced by genetic engineering. In this technique, as with other methods, antibody-producing cells are sensitized to the desired antigen or immunogen. The messenger RNA isolated from antibody producing cells is used as a template to make cDNA using PCR amplification. A library of vectors, each containing one heavy chain gene and one light chain gene retaining the initial antigen specificity, is produced by insertion of appropriate sections of the amplified immunoglobulin cDNA into the expression vectors. A combinatorial library is constructed by combining the heavy chain gene library with the light chain gene library. This results in a library of clones which co-express a heavy and light chain (resembling the Fab fragment or antigen binding fragment of an antibody molecule). The vectors that carry these genes are co-transfected into a host cell. When antibody gene synthesis is induced in the transfected host, the heavy and light chain proteins self-assemble to produce active antibodies that can be detected by screening with the antigen or immunogen.

Antibody coding sequences of interest include those encoded by native sequences, as well as nucleic acids that, by virtue of the degeneracy of the genetic code, are not identical in sequence to the disclosed nucleic acids, and variants thereof. Variant polypeptides can include amino acid (aa) substitutions, additions or deletions. The amino acid substitutions can be conservative amino acid substitutions or substitutions to eliminate non-essential amino acids, such as to alter a glycosylation site, or to minimize misfolding by substitution or deletion of one or more cysteine residues that are not necessary for function. Variants can be designed so as to retain or have enhanced biological activity of a particular region of the protein (*e.g.,* a functional domain, catalytic amino acid residues, *etc*)*.* Variants also include fragments of the polypeptides disclosed herein, particularly biologically active fragments and/or fragments corresponding to functional domains. Techniques for in vitro mutagenesis of cloned genes are known. Also described herein are polypeptides that have been modified using ordinary molecular biological techniques so as to improve their resistance to proteolytic degradation or to optimize solubility properties or to render them more suitable as a therapeutic agent.

Chimeric antibodies may be made by recombinant means by combining the variable light and heavy chain regions (V_{L} and V_{H}), obtained from antibody producing cells of one species with the constant light and heavy chain regions from another. Typically chimeric antibodies utilize rodent or rabbit variable regions and human constant regions, in order to produce an antibody with predominantly human domains. The production of such chimeric antibodies is well known in the art, and may be achieved by standard means (as described, e.g., in U.S. Patent No. 5,624,659). It is further contemplated that the human constant regions of chimeric antibodies of the invention may be selected from IgG1, IgG2, IgG3, IgG4, IgG5, IgG6, IgG7, IgG8, IgG9, IgG10, IgG11, IgG12, IgG13, IgG14, IgG15, IgG16, IgG17, IgG18 or IgG19 constant regions.

Humanized antibodies are engineered to contain even more human-like immunoglobulin domains, and incorporate only the complementarity-determining regions of the animal-derived antibody. This is accomplished by carefully examining the sequence of the hyper-variable loops of the variable regions of the monoclonal antibody, and fitting them to the structure of the human antibody chains. Although facially complex, the process is straightforward in practice. See, e.g., U.S. Patent No. 6,187,287. In a preferred embodiment, humanization may be effected as disclosed in detail infra. This scheme grafts CDRs onto human FRs highly homologous to the parent antibody that is being humanized.

In addition to entire immunoglobulins (or their recombinant counterparts), immunoglobulin fragments comprising the epitope binding site (e.g., Fab', F(ab')2, or other fragments) may be synthesized. "Fragment," or minimal immunoglobulins may be designed utilizing recombinant immunoglobulin techniques. For instance "Fv" immunoglobulins may be produced by synthesizing a fused variable light chain region and a variable heavy chain region. Combinations of antibodies are also of interest, *e.g.* diabodies, which comprise two distinct Fv specificities. SMIPs (small molecule immunopharmaceuticals), camelbodies, nanobodies, and IgNAR are encompassed by immunoglobulin fragments.

Immunoglobulins and fragments thereof may be modified post-translationally, *e.g.* to add effector moieties such as chemical linkers, detectable moieties, such as fluorescent dyes, enzymes, toxins, substrates, bioluminescent materials, radioactive materials, chemiluminescent moieties and the like, or specific binding moieties, such as streptavidin, avidin, or biotin, and the like may be utilized in the methods and compositions of the present invention. Examples of additional effector molecules are provided *infra.*

The term "polyploid yeast that stably expresses or expresses a desired secreted heterologous polypeptide for prolonged time" refers to a yeast culture that secretes said polypeptide for at least several days to a week, more preferably at least a month, still more preferably at least 1-6 months, and even more preferably for more than a year at threshold expression levels, typically at least 10-25 mg/liter and preferably substantially greater.

The term "polyploidal yeast culture that secretes desired amounts of recombinant polypeptide" refers to cultures that stably or for prolonged periods secrete at least 10-25 mg/liter of heterologous polypeptide, more preferably at least 50-500 mg/liter, and most preferably 500-1000 mg/liter or more.

A polynucleotide sequence "corresponds" to a polypeptide sequence if translation of the polynucleotide sequence in accordance with the genetic code yields the polypeptide sequence (i.e., the polynucleotide sequence "encodes" the polypeptide sequence), one polynucleotide sequence "corresponds" to another polynucleotide sequence if the two sequences encode the same polypeptide sequence.

A "heterologous" region or domain of a DNA construct is an identifiable segment of DNA within a larger DNA molecule that is not found in association with the larger molecule in nature. Thus, when the heterologous region encodes a mammalian gene, the gene will usually be flanked by DNA that does not flank the mammalian genomic DNA in the genome of the source organism. Another example of a heterologous region is a construct where the coding sequence itself is not found in nature (e.g., a cDNA where the genomic coding sequence contains introns, or synthetic sequences having codons different than the native gene). Allelic variations or naturally-occurring mutational events do not give rise to a heterologous region of DNA as defined herein.

A "coding sequence" is an in-frame sequence of codons that (in view of the genetic code) correspond to or encode a protein or peptide sequence. Two coding sequences correspond to each other if the sequences or their complementary sequences encode the same amino acid sequences. A coding sequence in association with appropriate regulatory sequences may be transcribed and translated into a polypeptide. A polyadenylation signal and transcription termination sequence will usually be located 3' to the coding sequence. A "promoter sequence" is a DNA regulatory region capable of binding RNA polymerase in a cell and initiating transcription of a downstream (3' direction) coding sequence. Promoter sequences typically contain additional sites for binding of regulatory molecules (e.g., transcription factors) which affect the transcription of the coding sequence. A coding sequence is "under the control" of the promoter sequence or "operatively linked" to the promoter when RNA polymerase binds the promoter sequence in a cell and transcribes the coding sequence into mRNA, which is then in turn translated into the protein encoded by the coding sequence.

Vectors are used to introduce a foreign substance, such as DNA, RNA or protein, into an organism or host cell. Typical vectors include recombinant viruses (for polynucleotides) and liposomes or other lipid aggregates (for polypeptides and/or polynucleotides). A "DNA vector" is a replicon, such as plasmid, phage or cosmid, to which another polynucleotide segment may be attached so as to bring about the replication of the attached segment. An "expression vector" is a DNA vector which contains regulatory sequences which will direct polypeptide synthesis by an appropriate host cell. This usually means a promoter to bind RNA polymerase and initiate transcription of mRNA, as well as ribosome binding sites and initiation signals to direct translation of the mRNA into a polypeptide(s). Incorporation of a polynucleotide sequence into an expression vector at the proper site and in correct reading frame, followed by transformation of an appropriate host cell by the vector, enables the production of a polypeptide encoded by said polynucleotide sequence. Exemplary expression vectors and techniques for their use are described in the following publications: Old et al., Principles of Gene Manipulation: An Introduction to Genetic Engineering, Blackwell Scientific Publications, 4th edition, 1989; Sambrook et al., Molecular Cloning: A Laboratory Manual, 2nd Edition, Cold Spring Harbor Laboratory Press, 1989; Sambrook et al., Molecular Cloning: A Laboratory Manual, 3rd Edition, Cold Spring Harbor Laboratory Press, 2001; Gorman, "High Efficiency Gene Transfer into Mammalian Cells," in DNA Cloning, Volume II, Glover, D. M., Ed., IRL Press, Washington, D.C., pp. 143 190 (1985).

For example, a liposomes or other lipid aggregate may comprise a lipid such as phosphatidylcholines (lecithins) (PC), phosphatidylethanolamines (PE), lysolecithins, lysophosphatidylethanolamines, phosphatidylserines (PS), phosphatidylglycerols (PG), phosphatidylinositol (PI), sphingomyelins, cardiolipin, phosphatidic acids (PA), fatty acids, gangliosides, glucolipids, glycolipids, mono-, di or triglycerides, ceramides, cerebrosides and combinations thereof; a cationic lipid (or other cationic amphiphile) such as 1,2-dioleyloxy-3-(trimethylamino) propane (DOTAP); N-cholesteryloxycarbaryl-3,7,12-triazapentadecane-1,15-diamine (CTAP); N-[1-(2,3, -ditetradecyloxy)propyl]-N,N-dimethyl-N-hydroxyethylammonium bromide (DMRIE); N-[1-(2,3,-dioleyloxy)propyl]-N,N-dimethyl-N-hydroxy ethylammonium bromide (DORIE); N-[1-(2,3-dioleyloxy) propyl]-N,N,N-trimethylammonium chloride (DOTMA); 3 beta [N-(N',N'-dimethylaminoethane)carbamoly] cholesterol (DC-Choi); and dimethyldioctadecylammonium (DDAB); dioleoylphosphatidyl ethanolamine (DOPE), cholesterol-containing DOPC; and combinations thereof; and/or a hydrophilic polymer such as polyvinylpyrrolidone, polyvinylmethylether, polymethyloxazoline, polyethyloxazoline, polyhydroxypropyloxazoline, polyhydroxypropylmethacrylamide, polymethacrylamide, polydimethylacrylamide, polyhydroxypropylmethacrylate, polyhydroxyethylacrylate, hydroxymethylcellulose, hydroxyethylcellulose, polyethyleneglycol, polyaspartamide and combinations thereof. Other suitable cationic lipids are described in Miller, Angew. Chem. Int. Ed. 37:1768 1785 (1998), and Cooper et al., Chem. Eur. J. 4(1): 137 151 (1998). Liposomes can be crosslinked, partially crosslinked, or free from crosslinking. Crosslinked liposomes can include crosslinked as well as non-crosslinked components. Suitable cationic liposomes or cytofectins are commercially available and can also be prepared as described in Sipkins et al., Nature Medicine, 1998, 4(5):(1998), 623 626 or as described in Miller, supra. Exemplary liposomes include a polymerizable zwitterionic or neutral lipid, a polymerizable integrin targeting lipid and a polymerizable cationic lipid suitable for binding a nucleic acid. Liposomes can optionally include peptides that provide increased efficiency, for example as described in U.S. Pat. No. 7,297,759. Additional exemplary liposomes and other lipid aggregates are described in U.S. Pat. No. 7,166,298.

"Amplification" of polynucleotide sequences is the *in vitro* production of multiple copies of a particular nucleic acid sequence. The amplified sequence is usually in the form of DNA. A variety of techniques for carrying out such amplification are described in a review article by Van Brunt (1990, Bio/Technol., 8(4):291-294). Polymerase chain reaction or PCR is a prototype of nucleic acid amplification, and use of PCR herein should be considered exemplary of other suitable amplification techniques.

The general structure of antibodies in vertebrates now is well understood (Edelman, G. M., Ann. N.Y. Acad. Sci., 190: 5 (1971)). Antibodies consist of two identical light polypeptide chains of molecular weight approximately 23,000 daltons (the "light chain"), and two identical heavy chains of molecular weight 53,000-70,000 (the "heavy chain"). The four chains are joined by disulfide bonds in a "Y" configuration wherein the light chains bracket the heavy chains starting at the mouth of the "Y" configuration. The "branch" portion of the "Y" configuration is designated the Fab region; the stem portion of the "Y" configuration is designated the Fc region. The amino acid sequence orientation runs from the N-terminal end at the top of the "Y" configuration to the C-terminal end at the bottom of each chain. The N-terminal end possesses the variable region having specificity for the antigen that elicited it, and is approximately 100 amino acids in length, there being slight variations between light and heavy chain and from antibody to antibody.

The variable region is linked in each chain to a constant region that extends the remaining length of the chain and that within a particular class of antibody does not vary with the specificity of the antibody (i.e., the antigen eliciting it). There are five known major classes of constant regions that determine the class of the immunoglobulin molecule (IgG, IgM, IgA, IgD, and IgE corresponding to γ, µ, α, δ, and ε (gamma, mu, alpha, delta, or epsilon) heavy chain constant regions). The constant region or class determines subsequent effector function of the antibody, including activation of complement (Kabat, E. A., Structural Concepts in Immunology and Immunochemistry, 2nd Ed., p. 413-436, Holt, Rinehart, Winston (1976)), and other cellular responses (Andrews, D. W., et al., Clinical Immunobiology, pp 1-18, W. B. Sanders (1980); Kohl, S., et al., Immunology, 48: 187 (1983)); while the variable region determines the antigen with which it will react. Light chains are classified as either κ (kappa) or λ (lambda). Each heavy chain class can be paired with either kappa or lambda light chain. The light and heavy chains are covalently bonded to each other, and the "tail" portions of the two heavy chains are bonded to each other by covalent disulfide linkages when the immunoglobulins are generated either by hybridomas or by B cells.

The expression "variable region" or "VR" refers to the domains within each pair of light and heavy chains in an antibody that are involved directly in binding the antibody to the antigen. Each heavy chain has at one end a variable domain (V_{H}) followed by a number of constant domains. Each light chain has a variable domain (V_{L}) at one end and a constant domain at its other end; the constant domain of the light chain is aligned with the first constant domain of the heavy chain, and the light chain variable domain is aligned with the variable domain of the heavy chain.

The expressions "complementarity determining region," "hypervariable region," or "CDR" refer to one or more of the hyper-variable or complementarity determining regions (CDRs) found in the variable regions of light or heavy chains of an antibody (*See* Kabat, E. A. et al., Sequences of Proteins of Immunological Interest, National Institutes of Health, Bethesda, Md., (1987)). These expressions include the hypervariable regions as defined by Kabat *et al.* ("Sequences of Proteins of Immunological Interest," Kabat E., et al., US Dept. of Health and Human Services, 1983) or the hypervariable loops in 3-dimensional structures of antibodies (Chothia and Lesk, J Mol. Biol. 196 901-917 (1987)). The CDRs in each chain are held in close proximity by framework regions and, with the CDRs from the other chain, contribute to the formation of the antigen binding site. Within the CDRs there are select amino acids that have been described as the selectivity determining regions (SDRs) which represent the critical contact residues used by the CDR in the antibody-antigen interaction (Kashmiri, S., Methods, 36:25-34 (2005)). CDRs for exemplary anti-IL-6 antibodies are provided herein.

The expressions "framework region" or "FR" refer to one or more of the framework regions within the variable regions of the light and heavy chains of an antibody (*See* Kabat, E. A. et al., Sequences of Proteins of Immunological Interest, National Institutes of Health, Bethesda, Md., (1987)). These expressions include those amino acid sequence regions interposed between the CDRs within the variable regions of the light and heavy chains of an antibody. As mentioned in the preferred embodiments, the FRs will comprise human FRs highly homologous to the parent antibody (e.g., rabbit antibody).

### Ab1 Anti-IL-6 Antibodies and Binding Fragments Thereof

The invention includes compositions comprising antibodies as defined in the claims for use in accordance with the claims, wherein the antibodies have binding specificity to IL-6 and possess a variable light chain sequence comprising the sequence set forth below: AIQMTQSPSSLSASVGDRVTITCQASQSINNELSWYQQKPGKAPKLLIYRASTLASGV PSRFSGSGSGTDFTLTISSLQPDDFATYYCQQGYSLRNIDNAFGGGTKVEIKR (SEQ ID NO: 709) and a variable heavy chain sequence comprising the sequence set forth below: EVQLVESGGGLVQPGGSLRLSCAASGFSLSNYYVTWVRQAPGKGLEWVGIIYGSDE TAYATSAIGRFTISRDNSKNTLYLQMNSLRAEDTAVYYCARDDSSDWDAKFNLWGQ GTLVTVSS (SEQ ID NO: 657).

In one embodiment of the invention, the antibody comprises the polypeptide sequence of SEQ ID NO: 702 and SEQ ID NO: 704.

The anti-IL-6 antibody defined in the claims is Ab1, comprising SEQ ID NO: 657 and SEQ ID NO: 709 (which are respectively encoded by the nucleic acid sequences in SEQ ID NO: 700 and SEQ ID NO: 723).

Variable light and heavy chains of the anti-IL-6 antibody defined in the claims respectively will be encoded by SEQ ID NO: 701 and SEQ ID NO: 703.

Described herein are nucleic acid constructs containing any of the foregoing nucleic acid sequences and combinations thereof as well as recombinant cells containing these nucleic acid sequences and constructs containing wherein these nucleic acid sequences or constructs may be extrachromosomal or integrated into the host cell genome

In accordace with the claims, the invention covers polypeptides containing the variable light polypeptides comprised in SEQ ID NO: 702, or 709 and the variable heavy polypeptides comprised in SEQ ID NO: 657, or 704. These polypeptides optionally may be attached directly or indirectly to other immunoglobulin polypeptides or effector moieties such as therapeutic or detectable entities.

The anti-IL-6 antibody defined in the claims is one comprising a variable light chain encoded by the sequence in SEQ ID NO: 701 and a variable chain encoded by the sequence in SEQ ID NO: 703.

The *in vitro* and *in vivo* properties of the antibody defined in the claims are described in more detail in the examples below and include: competing with Ab1 for binding to IL-6 and/or peptides thereof; having a binding affinity (Kd) for IL-6 of less than about 50 picomolar, and/or a rate of dissociation (K_{off}) from IL-6 of less than or equal to 10⁻⁴ S⁻¹; having an *in-vivo* half-life of at least about 22 days in a healthy human subject; ability to prevent or treat hypoalbunemia; ability to prevent or treat elevated CRP; ability to prevent or treat abnormal coagulation; and/or ability to decrease the risk of thrombosis in an individual having a disease or condition associated with increased risk of thrombosis. Additional non-limiting examples of anti-IL-6 activity are set forth herein, for example, under the heading "Anti-IL-6 Activity."

**Table 1. Sequences of exemplary anti-IL-6 antibodies.**

| | Antibody chains | | CDR1 | | CDR2 | | CDR3 | |
|---|---|---|---|---|---|---|---|---|
| Antibody | PRT. | Nuc. | PRT. | Nuc. | PRT. | Nuc. | PRT. | Nuc. |
| Ab1 light chains * | 2 | 10 | 4 | 12 | 5 | 13 | 6 | 14 |
| | 20 | 720 | 4 | 12 | 5 | 13 | 6 | 14 |
| | 647 | 721 | 4 | 12 | 5 | 13 | 6 | 14 |
| | 651 | | 4 | 12 | 5 | 13 | 6 | 14 |
| | 660 | 662 | 4 | 12 | 5 | 13 | 6 | 14 |
| | 666 | 722 | 4 | 12 | 5 | 13 | 6 | 14 |
| | 699 | 698 | 4 | 694 | 5 | 13 | 6 | 695 |
| | 702 | 701 | 4 | 694 | 5 | 13 | 6 | 695 |
| | 706 | 705 | 4 | 694 | 5 | 13 | 6 | 695 |
| | 709 | 723 | 4 | 12 | 5 | 13 | 6 | 14 |
| Human light chains used in Ab1 humanization | 648 | | 710 | | 713 | | | |
| | 649 | | 711 | | 714 | | | |
| | 650 | | 712 | | 715 | | | |
| Ab1 heavy chains | 3 | 11 | 7 | 15 | 8 | 16 | 9 | 17 |
| | 18 | | 7 | 15 | 8 | 16 | 9 | 17 |
| | 19 | 724 | 7 | 15 | 120 | 696 | 9 | 17 |
| | 652 | 725 | 7 | 15 | 8 | 16 | 9 | 17 |
| | 656 | | 7 | 15 | 8 | 16 | 9 | 17 |
| | 657 | 700 | 7 | 15 | 659 | 696 | 9 | 697 |
| | 658 | | 7 | 15 | 120 | 696 | 9 | 17 |
| | 661 | 663 | 7 | 15 | 8 | 16 | 9 | 17 |
| | 664 | | 7 | 15 | 8 | 16 | 9 | 17 |
| | 665 | | 7 | 15 | 120 | 696 | 9 | 17 |
| | 704 | 703 | 7 | 15 | 120 | 696 | 9 | 697 |
| | 708 | 707 | 7 | 15 | 120 | 696 | 9 | 697 |
| Human heavy chains used in Ab1 humanization | 653 | | 716 | | 717 | | | |
| | 654 | | 716 | | 717 | | | |
| | 655 | | 74 | 82 | 718 | | | |
| Ab2 light chains | 21 | 29 | 23 | 31 | 24 | 32 | 25 | 33 |
| | 667 | 669 | 23 | 31 | 24 | 32 | 25 | 33 |
| Ab2 heavy chains | 22 | 30 | 26 | 34 | 27 | 35 | 28 | 36 |
| | 668 | 670 | 26 | 34 | 27 | 35 | 28 | 36 |
| Ab3 light chains | 37 | 45 | 39 | 47 | 40 | 48 | 41 | 49 |
| | 671 | 673 | 39 | 47 | 40 | 48 | 41 | 49 |
| Ab3 heavy chains | 38 | 46 | 42 | 50 | 43 | 51 | 44 | 52 |
| | 672 | 674 | 42 | 50 | 43 | 51 | 44 | 52 |
| Ab4 light chains | 53 | 61 | 55 | 63 | 56 | 64 | 57 | 65 |
| | 675 | 677 | 55 | 63 | 56 | 64 | 57 | 65 |
| Ab4 heavy chains | 54 | 62 | 58 | 66 | 59 | 67 | 60 | 68 |
| | 676 | 678 | 58 | 66 | 59 | 67 | 60 | 68 |
| Ab5 light chains | 69 | 77 | 71 | 79 | 72 | 80 | 73 | 81 |
| | 679 | 681 | 71 | 79 | 72 | 80 | 73 | 81 |
| Ab5 heavy chains | 70 | 78 | 74 | 82 | 75 | 83 | 76 | 84 |
| | 680 | 682 | 74 | 82 | 75 | 83 | 76 | 84 |
| Ab6 light chains | 85 | 93 | 87 | 95 | 88 | 96 | 89 | 97 |
| | 683 | 685 | 87 | 95 | 88 | 96 | 89 | 97 |
| Ab6 heavy chains | 86 | 94 | 90 | 98 | 91 | 99 | 92 | 100 |
| | 684 | 686 | 90 | 98 | 91 | 99 | 92 | 100 |
| Ab7 light chains | 101 | 109 | 103 | 111 | 104 | 112 | 105 | 113 |
| | 119 | | 103 | 111 | 104 | 112 | 105 | 113 |
| | 687 | 689 | 103 | 111 | 104 | 112 | 105 | 113 |
| | 693 | | 103 | 111 | 104 | 112 | 105 | 113 |
| Ab7 heavy chains | 102 | 110 | 106 | 114 | 107 | 115 | 108 | 116 |
| | 117 | | 106 | 114 | 107 | 115 | 108 | 116 |
| | 118 | | 106 | 114 | 121 | | 108 | 116 |
| | 688 | 690 | 106 | 114 | 107 | 115 | 108 | 116 |
| | 691 | | 106 | 114 | 107 | 115 | 108 | 116 |
| | 692 | | 106 | 114 | 121 | | 108 | 116 |
| Ab8 light chain | 122 | 130 | 124 | 132 | 125 | 133 | 126 | 134 |
| Ab8 heavy chain | 123 | 131 | 127 | 135 | 128 | 136 | 129 | 137 |
| Ab9 light chain | 138 | 146 | 140 | 148 | 141 | 149 | 142 | 150 |
| Ab9 heavy chain | 139 | 147 | 143 | 151 | 144 | 152 | 145 | 153 |
| Ab10 light chain | 154 | 162 | 156 | 164 | 157 | 165 | 158 | 166 |
| Ab10 heavy chain | 155 | 163 | 159 | 167 | 160 | 168 | 161 | 169 |
| Ab11 light chain | 170 | 178 | 172 | 180 | 173 | 181 | 174 | 182 |
| Ab11 heavy chain | 171 | 179 | 175 | 183 | 176 | 184 | 177 | 185 |
| Ab12 light chain | 186 | 194 | 188 | 196 | 189 | 197 | 190 | 198 |
| Ab12 heavy chain | 187 | 195 | 191 | 199 | 192 | 200 | 193 | 201 |
| Ab13 light chain | 202 | 210 | 204 | 212 | 205 | 213 | 206 | 214 |
| Ab13 heavy chain | 203 | 211 | 207 | 215 | 208 | 216 | 209 | 217 |
| Ab14 light chain | 218 | 226 | 220 | 228 | 221 | 229 | 222 | 230 |
| Ab14 heavy chain | 219 | 227 | 223 | 231 | 224 | 232 | 225 | 233 |
| Ab15 light chain | 234 | 242 | 236 | 244 | 237 | 245 | 238 | 246 |
| Ab15 heavy chain | 235 | 243 | 239 | 247 | 240 | 248 | 241 | 249 |
| Ab16 light chain | 250 | 258 | 252 | 260 | 253 | 261 | 254 | 262 |
| Ab16 heavy chain | 251 | 259 | 255 | 263 | 256 | 264 | 257 | 265 |
| Ab17 light chain | 266 | 274 | 268 | 276 | 269 | 277 | 270 | 278 |
| Ab17 heavy chain | 267 | 275 | 271 | 279 | 272 | 280 | 273 | 281 |
| Ab18 light chain | 282 | 290 | 284 | 292 | 285 | 293 | 286 | 294 |
| Ab18 heavy chain | 283 | 291 | 287 | 295 | 288 | 296 | 289 | 297 |
| Ab19 light chain | 298 | 306 | 300 | 308 | 301 | 309 | 302 | 310 |
| Ab19 heavy chain | 299 | 307 | 303 | 311 | 304 | 312 | 305 | 313 |
| Ab20 light chain | 314 | 322 | 316 | 324 | 317 | 325 | 318 | 326 |
| Ab20 heavy chain | 315 | 323 | 319 | 327 | 320 | 328 | 321 | 329 |
| Ab21 light chain | 330 | 338 | 332 | 340 | 333 | 341 | 334 | 342 |
| Ab21 heavy chain | 331 | 339 | 335 | 343 | 336 | 344 | 337 | 345 |
| Ab22 light chain | 346 | 354 | 348 | 356 | 349 | 357 | 350 | 358 |
| Ab22 heavy chain | 347 | 355 | 351 | 359 | 352 | 360 | 353 | 361 |
| Ab23 light chain | 362 | 370 | 364 | 372 | 365 | 373 | 366 | 374 |
| Ab23 heavy chain | 363 | 371 | 367 | 375 | 368 | 376 | 369 | 377 |
| Ab24 light chain | 378 | 386 | 380 | 388 | 381 | 389 | 382 | 390 |
| Ab24 heavy chain | 379 | 387 | 383 | 391 | 384 | 392 | 385 | 393 |
| Ab25 light chain | 394 | 402 | 396 | 404 | 397 | 405 | 398 | 406 |
| Ab25 heavy chain | 395 | 403 | 399 | 407 | 400 | 408 | 401 | 409 |
| Ab26 light chain | 410 | 418 | 412 | 420 | 413 | 421 | 414 | 422 |
| Ab26 heavy chain | 411 | 419 | 415 | 423 | 416 | 424 | 417 | 425 |
| Ab27 light chain | 426 | 434 | 428 | 436 | 429 | 437 | 430 | 438 |
| Ab27 heavy chain | 427 | 435 | 431 | 439 | 432 | 440 | 433 | 441 |
| Ab28 light chain | 442 | 450 | 444 | 452 | 445 | 453 | 446 | 454 |
| Ab28 heavy chain | 443 | 451 | 447 | 455 | 448 | 456 | 449 | 457 |
| Ab29 light chain | 458 | 466 | 460 | 468 | 461 | 469 | 462 | 470 |
| Ab29 heavy chain | 459 | 467 | 463 | 471 | 464 | 472 | 465 | 473 |
| Ab30 light chain | 474 | 482 | 476 | 484 | 477 | 485 | 478 | 486 |
| Ab30 heavy chain | 475 | 483 | 479 | 487 | 480 | 488 | 481 | 489 |
| Ab31 light chain | 490 | 498 | 492 | 500 | 493 | 501 | 494 | 502 |
| Ab31 heavy chain | 491 | 499 | 495 | 503 | 496 | 504 | 497 | 505 |
| Ab32 light chain | 506 | 514 | 508 | 516 | 509 | 517 | 510 | 518 |
| Ab32 heavy chain | 507 | 515 | 511 | 519 | 512 | 520 | 513 | 521 |
| Ab33 light chain | 522 | 530 | 524 | 532 | 525 | 533 | 526 | 534 |
| Ab33 heavy chain | 523 | 531 | 527 | 535 | 528 | 536 | 529 | 537 |
| Ab34 light chain | 538 | 546 | 540 | 548 | 541 | 549 | 542 | 550 |
| Ab34 heavy chain | 539 | 547 | 543 | 551 | 544 | 552 | 545 | 553 |
| Ab35 light chain | 554 | 562 | 556 | 564 | 557 | 565 | 558 | 566 |
| Ab35 heavy chain | 555 | 563 | 559 | 567 | 560 | 568 | 561 | 569 |
| Ab36 light chain | 570 | 578 | 572 | 580 | 573 | 581 | 574 | 582 |
| Ab36 heavy chain | 571 | 579 | 575 | 583 | 576 | 584 | 577 | 585 |

| | | | | | | | | |
|---|---|---|---|---|---|---|---|---|
| * Exemplary sequence variant forms of heavy and light chains are shown on separate lines. PRT.: Polypeptide sequence. Nuc.: Exemplary coding sequence. | | | | | | | | |

For reference, sequence identifiers other than those included in Table 1 are summarized in Table 2.

**Table 2. Summary of sequence identifiers in this application.**

| **SEQ ID** | **Description** |
|---|---|
| 1 | Human IL-6 |
| 586 | kappa constant light chain polypeptide sequence |
| 587 | kappa constant light chain polynucleotide sequence |
| 588 | gamma-1 constant heavy chain polypeptide sequence |
| 589 | gamma-1 constant heavv chain polynucleotide sequence |
| 590 - 646 | Human IL-6 peptides (see FIG. 12 and Example 14) |
| 719 | gamma-1 constant heavy chain polypeptide sequence (differs from SEQ ID NO: 518 at two positions) |
| 726 | C-reactive protein polypeptide sequence |
| 727 | IL-6 receptor alpha |
| 728 | IL-6 receptor beta / gp130 |

In accordance with the claims, the anti-IL-6 antibodies comprise the kappa constant light chain sequence comprising the sequence set forth below: VAAPSVFIFPPSDEQLKSGTASVVCLLNNFYPREAKVQWKVDNALQSGNSQESVTEQ DSKDSTYSLSSTLTLSKADYEKHKVYACEVTHQGLSSPVTKSFNRGEC (SEQ ID NO: 586) and the gamma-1 constant heavy chain polypeptide sequence comprising one of the sequences set forth below:

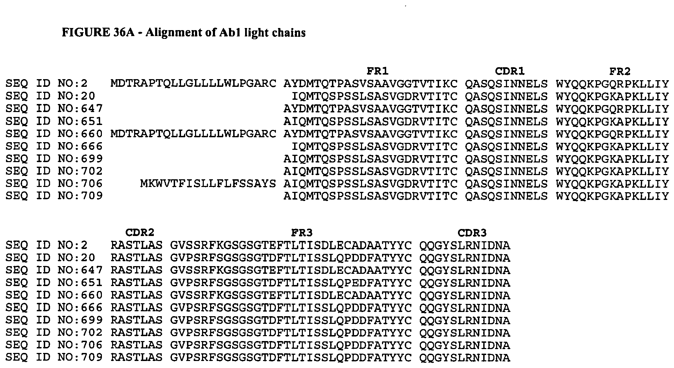

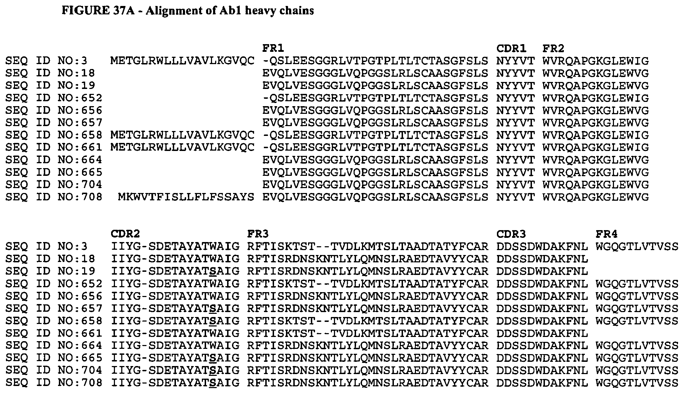

Embodiments of antibodies described herein may include a leader sequence, such as a rabbit Ig leader, albumin pre-peptide, a yeast mating factor pre pro secretion leader sequence (such as *P. pastoris* or *Saccharomyces cerevisiae* a or alpha factor), or human HAS leader. Exemplary leader sequences are shown offset from FR1 at the N-terminus of polypeptides shown in Figs. 36A and 37A as follows: rabbit Ig leader sequences in SEQ ID NOs: 2 and 660 (MD...) and SEQ ID NOs: 3 and 661 (ME...); and an albumin prepeptide in SEQ ID NOs: 706 and 708, which facilitates secretion. Other leader sequences known in the art to confer desired properties, such as secretion, improved stability or half-life, etc. may also be used, either alone or in combinations with one another, on the heavy and/or light chains, which may optionally be cleaved prior to administration to a subject. For example, a polypeptide may be expressed in a cell or cell-free expression system that also expresses or includes (or is modified to express or include) a protease, e.g., a membrane-bound signal peptidase, that cleaves a leader sequence.

The invention contemplates an isolated anti-IL-6 antibody as defined in the claims comprising a V_{H} polypeptide sequence comprising: SEQ ID NO: 657; and further comprising a V_{L} polypeptide sequence comprising: SEQ ID NO: 709 and a variable heavy and light chain constant region as contained in SEQ ID NO: 588 and SEQ ID NO: 586.

The anti-IL-6 antibodies and fragments and variants thereof may have binding specificity for primate homologs of the human IL-6 protein. Non-limiting examples of primate homologs of the human IL-6 protein are IL-6 obtained from *Macaca fascicularis* (also known as the cynomolgus monkey) and the Rhesus monkey. The anti-IL-6 antibodies and fragments and variants thereof may inhibit the association of IL-6 with IL-6R, and/or the production of IL-6/IL-6R/gp130 complexes and/or the production of IL-6/IL-6R/gp130 multimers and/or antagonizes the biological effects of one or more of the foregoing.

As stated above, antibodies and fragments and variants thereof may be modified post-translationally to add effector moieties such as chemical linkers, detectable moieties such as for example fluorescent dyes, enzymes, substrates, bioluminescent materials, radioactive materials, and chemiluminescent moieties, or functional moieties such as for example streptavidin, avidin, biotin, a cytotoxin, a cytotoxic agent, and radioactive materials.

Regarding detectable moieties, further exemplary enzymes include, but are not limited to, horseradish peroxidase, acetylcholinesterase, alkaline phosphatase, *beta-*galactosidase and luciferase. Further exemplary fluorescent materials include, but are not limited to, rhodamine, fluorescein, fluorescein isothiocyanate, umbelliferone, dichlorotriazinylamine, phycoerythrin and dansyl chloride. Further exemplary chemiluminescent moieties include, but are not limited to, luminol. Further exemplary bioluminescent materials include, but are not limited to, luciferin and aequorin. Further exemplary radioactive materials include, but are not limited to, Iodine 125 (¹²⁵I), Carbon 14 (¹⁴C), Sulfur 35 (³⁵S), Tritium (³H) and Phosphorus 32 (³²P).

Regarding functional moieties, exemplary cytotoxic agents include, but are not limited to, methotrexate, aminopterin, 6-mercaptopurine, 6-thioguanine, cytarabine, 5-fluorouracil decarbazine; alkylating agents such as mechlorethamine, thioepa chlorambucil, melphalan, carmustine (BSNU), mitomycin C, lomustine (CCNU), 1-methylnitrosourea, cyclothosphamide, mechlorethamine, busulfan, dibromomannitol, streptozotocin, mitomycin C, cis-dichlorodiamine platinum (II) (DDP) cisplatin and carboplatin (paraplatin); anthracyclines include daunorubicin (formerly daunomycin), doxorubicin (adriamycin), detorubicin, carminomycin, idarubicin, epirubicin, mitoxantrone and bisantrene; antibiotics include dactinomycin (actinomycin D), bleomycin, calicheamicin, mithramycin, and anthramycin (AMC); and antimytotic agents such as the vinca alkaloids, vincristine and vinblastine. Other cytotoxic agents include paclitaxel (taxol), ricin, pseudomonas exotoxin, gemcitabine, cytochalasin B, gramicidin D, ethidium bromide, emetine, etoposide, tenoposide, colchicin, dihydroxy anthracin dione, 1-dehydrotestosterone, glucocorticoids, procaine, tetracaine, lidocaine, propranolol, puromycin, procarbazine, hydroxyurea, asparaginase, corticosteroids, mytotane (O,P'-(DDD)), interferons, and mixtures of these cytotoxic agents.

Further cytotoxic agents include, but are not limited to, chemotherapeutic agents such as carboplatin, cisplatin, paclitaxel, gemcitabine, calicheamicin, doxorubicin, 5-fluorouracil, mitomycin C, actinomycin D, cyclophosphamide, vincristine, bleomycin, VEGF antagonists, EGFR antagonists, platins, taxols, irinotecan, 5-fluorouracil, gemcytabine, leucovorine, steroids, cyclophosphamide, melphalan, vinca alkaloids (e.g., vinblastine, vincristine, vindesine and vinorelbine), mustines, tyrosine kinase inhibitors, radiotherapy, sex hormone antagonists, selective androgen receptor modulators, selective estrogen receptor modulators, PDGF antagonists, TNF antagonists, IL-1 antagonists, interleukins (e.g. IL-12 or IL-2), IL-12R antagonists, Toxin conjugated monoclonal antibodies, tumor antigen specific monoclonal antibodies, Erbitux™, Avastin™, Pertuzumab, anti-CD20 antibodies, Rituxan®, ocrelizumab, ofatumumab, DXL625, Herceptin®, or any combination thereof. Toxic enzymes from plants and bacteria such as ricin, diphtheria toxin and *Pseudomonas* toxin may be conjugated to the humanized antibodies, or binding fragments thereof, to generate cell-type-specific-killing reagents (Youle, et al., Proc. Nat'1 Acad. Sci. USA 77:5483 (1980); Gilliland, et al., Proc. Nat'1 Acad. Sci. USA 77:4539 (1980); Krolick, et al., Proc. Nat'1 Acad. Sci. USA 77:5419 (1980)).

Other cytotoxic agents include cytotoxic ribonucleases as described by Goldenberg in U.S. Pat. No. 6,653,104. Described herein are radioimmunoconjugates where a radionuclide that emits alpha or beta particles is stably coupled to the antibody, or binding fragments thereof, with or without the use of a complex-forming agent. Such radionuclides include beta-emitters such as Phosphorus-32 (³²P), Scandium-47 (⁴⁷Sc), Copper-67 (⁶⁷Cu), Gallium-67 (⁶⁷Ga), Yttrium-88 (⁸⁸Y), Yttrium-90 (⁹⁰Y), Iodine-125 (¹²⁵I), Iodine-131 (¹³¹I), Samarium-153 (¹⁵³Sm), Lutetium-177 (¹⁷⁷Lu), Rhenium-186 (¹⁸⁶Re) or Rhenium-188 (¹⁸⁸Re), and alpha-emitters such as Astatine-211 (²¹¹At), Lead-212 (²¹²Pb), Bismuth-212 (²¹²Bi) or - 213 (²¹³Bi) or Actinium-225 (²²⁵Ac).

Methods are known in the art for conjugating an antibody or binding fragment thereof to a detectable moiety and the like, such as for example those methods described by Hunter et al, Nature 144:945 (1962); David et al, Biochemistry 13:1014 (1974); Pain et al, J. Immunol. Meth. 40:219 (1981); and Nygren, J., Histochem. and Cytochem. 30:407 (1982).

Described herein is the generation and use of anti-idiotypic antibodies that bind any of the foregoing sequences. In an exemplary embodiment, such an anti-idiotypic antibody could be administered to a subject who has received an anti-IL-6 antibody to modulate, reduce, or neutralize, the effect of the anti-IL-6 antibody. Such anti-idiotypic antibodies could also be useful for treatment of an autoimmune disease characterized by the presence of anti-IL-6 antibodies. A further exemplary use of such anti-idiotypic antibodies is for detection of the anti-IL-6 antibodies of the present invention, for example to monitor the levels of the anti-IL-6 antibodies present in a subject's blood or other bodily fluids.

### Polynucleotides Encoding Anti-IL-6 Antibody Polypeptides

Described herein are polynucleotides encoding polypeptides of the antibodies having binding specificity to IL-6. Polynucleotides may comprise, or alternatively consist of, the following polynucleotide sequence encoding the variable light chain polypeptide sequence of SEQ ID NO: 2:
ATGGACACGAGGGCCCCCACTCAGCTGCTGGGGCTCCTGCTGCTCTGGC TCCCAGGTGCCAGATGTGCCTATGATATGACCCAGACTCCAGCCTCGGTGTCTGC AGCTGTGGGAGGCACAGTCACCATCAAGTGCCAGGCCAGTCAGAGCATTAACAA TGAATTATCCTGGTATCAGCAGAAACCAGGGCAGCGTCCCAAGCTCCTGATCTAT AGGGCATCCACTCTGGCATCTGGGGTCTCATCGCGGTTCAAAGGCAGTGGATCTG GGACAGAGTTCACTCTCACCATCAGCGACCTGGAGTGTGCCGATGCTGCCACTTA CTACTGTCAACAGGGTTATAGTCTGAGGAATATTGATAATGCTTTCGGCGGAGGG ACCGAGGTGGTGGTCAAACGTACGGTAGCGGCCCCATCTGTCTTCATCTTCCCGC CATCTGATGAGCAGTTGAAATCTGGAACTGCCTCTGTTGTGTGCCTGCTGAATAA CTT (SEQ ID NO: 10) or the polynucleotide sequence of SEQ ID NO: 662, 698, 701, or 705.

Polynucleotides may comprise, or alternatively consist of, the following polynucleotide sequence encoding the variable heavy chain polypeptide sequence of SEQ ID NO: 3:
ATGGAGACTGGGCTGCGCTGGCTTCTCCTGGTCGCTGTGCTCAAAGGTG TCCAGTGTCAGTCGCTGGAGGAGTCCGGGGGTCGCCTGGTCACGCCTGGGACAC CCCTGACACTCACCTGCACAGCCTCTGGATTCTCCCTCAGTAACTACTACGTGAC CTGGGTCCGCCAGGCTCCAGGGAAGGGGCTGGAATGGATCGGAATCATTTATGG TAGTGATGAAACGGCCTACGCGACCTGGGCGATAGGCCGATTCACCATCTCCAA AACCTCGACCACGGTGGATCTGAAAATGACCAGTCTGACAGCCGCGGACACGGC CACCTATTTCTGTGCCAGAGATGATAGTAGTGACTGGGATGCAAAATTTAACTTG TGGGGCCAAGGCACCCTGGTCACCGTCTCGAGCGCCTCCACCAAGGGCCCATCG GTCTTCCCCCTGGCACCCTCCTCCAAGAGCACCTCTGGGGGCACAGCGGCCCTGG GCTGCCTGGTCAAGG (SEQ ID NO: 11) or the polynucleotide sequence of SEQ ID NO: 663, 700, 703, or 707.

Polynucleotides encoding fragments or variants of the antibody having binding specificity to IL-6 may comprise, or alternatively consist of, one or more of the polynucleotide sequences of SEQ ID NO: 12 or 694; SEQ ID NO: 13; and SEQ ID NO: 14 or 695 which correspond to polynucleotides encoding the complementarity-determining regions (CDRs, or hypervariable regions) of the light chain variable sequence of SEQ ID NO: 2.

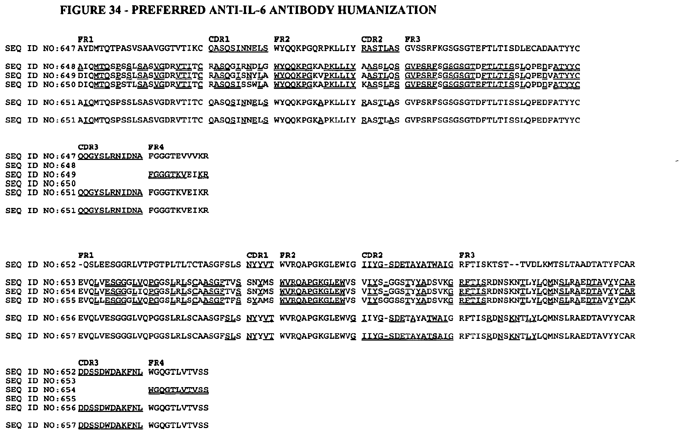

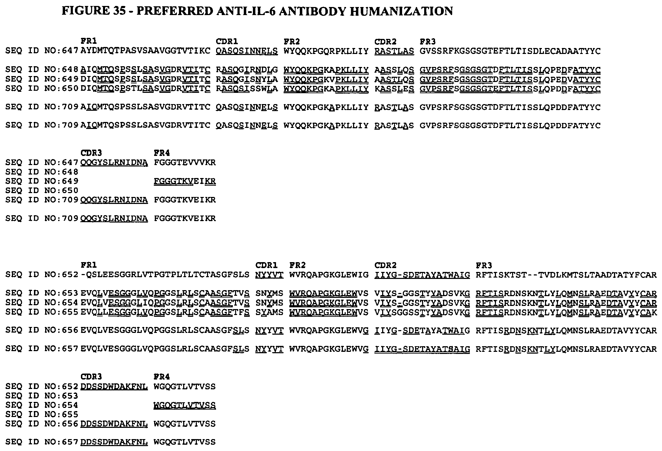

Polynucleotides encoding fragments or variants of the antibody having binding specificity to IL-6 may comprise, or alternatively consist of, one or more of the polynucleotide sequences of SEQ ID NO: 15; SEQ ID NO: 16 or 696; and SEQ ID NO: 17 or 697 which correspond to polynucleotides encoding the complementarity-determining regions (CDRs, or hypervariable regions) of the heavy chain variable sequence of SEQ ID NO: 3 or SEQ ID NO: 661 or SEQ ID NO: 657 or others depicted in Figs. 34 or 35.

Also described herein are polynucleotide sequences including one or more of the polynucleotide sequences encoding antibody fragments or variants described herein. Polynucleotides encoding fragments or variants of the antibody having binding specificity to IL-6 may comprise, or alternatively consist of, one, two, three or more, including all of the following polynucleotides encoding antibody fragments: the polynucleotide SEQ ID NO: 10 encoding the light chain variable region of SEQ ID NO: 2; the polynucleotide SEQ ID NO: 11 encoding the heavy chain variable region of SEQ ID NO: 3; the polynucleotide SEQ ID NO: 720 encoding the light chain polypeptide of SEQ ID NO: 20; the polynucleotide SEQ ID NO: 721 encoding the light chain polypeptide of SEQ ID NO: 647; the polynucleotide SEQ ID NO: 662 encoding the light chain polypeptide of SEQ ID NO: 660; the polynucleotide SEQ ID NO: 722 encoding the light chain polypeptide of SEQ ID NO: 666; the polynucleotide SEQ ID NO: 698 encoding the light chain polypeptide of SEQ ID NO: 699; the polynucleotide SEQ ID NO: 701 encoding the light chain polypeptide of SEQ ID NO: 702; the polynucleotide SEQ ID NO: 705 encoding the light chain polypeptide of SEQ ID NO: 706; the polynucleotide SEQ ID NO: 723 encoding the light chain polypeptide of SEQ ID NO: 709; the polynucleotide SEQ ID NO: 724 encoding the heavy chain polypeptide of SEQ ID NO: 19; the polynucleotide SEQ ID NO: 725 encoding the heavy chain polypeptide of SEQ ID NO: 652; the polynucleotide SEQ ID NO: 700 encoding the heavy chain polypeptide of SEQ ID NO: 657; the polynucleotide SEQ ID NO: 663 encoding the heavy chain polypeptide of SEQ ID NO: 661; the polynucleotide SEQ ID NO: 703 encoding the heavy chain polypeptide of SEQ ID NO: 704; the polynucleotide SEQ ID NO: 707 encoding the heavy chain polypeptide of SEQ ID NO: 708; the polynucleotides of SEQ ID NO: 12, 13, 14, 694 and 695 encoding the complementarity-determining regions of the aforementioned light chain polypeptides; and the polynucleotides of SEQ ID NO: 15, 16, 17, 696 and 697 encoding the complementarity-determining regions of the aforementioned heavy chain polypeptides, and polynucleotides encoding the variable heavy and light chain sequences in SEQ ID NO: 657 and SEQ ID NO: 709 respectively, e.g., the nucleic acid sequences in SEQ ID NO: 700 and SEQ ID NO: 723 and fragments or variants thereof , e.g., based on codon degeneracy. These nucleic acid sequences encoding variable heavy and light chain sequences may be expressed alone or in combination and these sequences preferably are fused to suitable variable constant sequences, e.g., those in SEQ ID NO: 589 and SEQ ID NO: 587.

Exemplary nucleotide sequences encoding anti-IL-6 antibodies are identified in Table 1, above. The polynucleotide sequences shown are to be understood to be illustrative, rather than limiting. One of skill in the art can readily determine the polynucleotide sequences that would encode a given polypeptide and can readily generate coding sequences suitable for expression in a given expression system, such as by adapting the polynucleotide sequences provided and/or by generating them *de novo,* and can readily produce codon-optimized expression sequences, for example as described in published U.S. Patent Application no. 2008/0120732 or using other methods known in the art.

Polynucleotides may further comprise, the following polynucleotide sequence encoding the kappa constant light chain sequence of SEQ ID NO: 586:

Polynucleotides may further comprise, the following polynucleotide sequence encoding the gamma-1 constant heavy chain polypeptide sequence of SEQ ID NO: 588:

Described herein is an isolated polynucleotide comprising a polynucleotide encoding an anti-IL-6 V_{H} antibody amino acid sequence selected from SEQ ID NO: 3, 18, 19, 652, 656, 657, 658, 661, 664, 665, 704, and 708 or encoding a variant thereof wherein at least one framework residue (FR residue) has been substituted with an amino acid present at the corresponding position in a rabbit anti-IL-6 antibody V_{H} polypeptide or a conservative amino acid substitution. In addition, the invention specifically encompasses humanized anti-I1-6 antibodies or humanized antibody binding fragments or variants thereof and nucleic acid sequences encoding the foregoing comprising the humanized variable heavy chain and/or light chain polypeptides depicted in the sequences contained in FIG. 2 or 34-37, or those identified in Table 1, or variants thereof wherein one or more framework or CDR residues may be modified. Preferably, if any modifications are introduced they will not affect adversely the binding affinity of the resulting anti-IL-6 antibody or fragment or variant thereof.

Also described herein is an isolated polynucleotide comprising the polynucleotide sequence encoding an anti-IL-6 V_{L} antibody amino acid sequence selected from SEQ ID NO: 2, 20, 647, 651, 660, 666, 699, 702, 706, and 709 or encoding a variant thereof wherein at least one framework residue (FR residue) has been substituted with an amino acid present at the corresponding position in a rabbit anti-IL-6 antibody V_{L} polypeptide or a conservative amino acid substitution.

Described herein is one or more heterologous polynucleotides comprising a sequence encoding the polypeptides contained in SEQ ID NO: 2 and SEQ ID NO: 3; SEQ ID NO: 2 and SEQ ID NO: 18; SEQ ID NO: 2 and SEQ ID NO: 19; SEQ ID NO: 20 and SEQ ID NO: 3; SEQ ID NO: 20 and SEQ ID NO: 18; or SEQ ID NO: 20 and SEQ ID NO: 19.

Described herein is an isolated polynucleotide that expresses a polypeptide containing at least one CDR polypeptide derived from an anti-IL-6 antibody wherein said expressed polypeptide alone specifically binds IL-6 or specifically binds IL-6 when expressed in association with another polynucleotide sequence that expresses a polypeptide containing at least one CDR polypeptide derived from an anti-IL-6 antibody wherein said at least one CDR is selected from those contained in the V_{L} or V_{H} polypeptides contained in SEQ ID NO: **3, 18, 19, 652, 656, 657, 658, 661, 664, 665, 704, 708,** 2, 20, 647, 651, 660, 666, 699, 702, 706, or 709.

Host cells and vectors comprising said polynucleotides are also described.

Described herein are nucleic acid constructs containing any of the foregoing nucleic acid sequences and combinations thereof as well as recombinant cells containing these nucleic acid sequences and constructs containing wherein these nucleic acid sequences or constructs may be extrachromosomal or integrated into the host cell genome.

Described herein are vectors comprising the polynucleotide sequences encoding the variable heavy and light chain polypeptide sequences, as well as the individual complementarity determining regions (CDRs, or hypervariable regions) set forth herein, as well as host cells comprising said sequences. The host cell may be a yeast cell. The yeast host cell may belongs to the genus *Pichia.*

**Table 3. Multiple exemplary polynucleotides encoding particular polypeptides.**

| **Polypeptide SEQ ID NO** | **Exemplary coding SEQ ID NOs** |
|---|---|
| 4 | 12, 111,694 |
| 5 | 13,112,389,501 |
| 6 | 14, 113,695 |
| 9 | 17,116,697 |
| 39 | 47, 260 |
| 40 | 48,261 |
| 60 | 68, 265 |
| 72 | 80, 325, 565, 581 |
| 89 | 97, 134, 166 |
| 103 | 12, 111,694 |
| 104 | 13, 112, 389, 501 |
| 105 | 14, 113, 695 |
| 108 | 17, 116, 697 |
| 126 | 97, 134, 166 |
| 158 | 97, 134, 166 |
| 190 | 198, 214 |
| 191 | 199, 215 |
| 205 | 213, 469, 485 |
| 206 | 198, 214 |
| 207 | 199, 215 |
| 252 | 47, 260 |
| 253 | 48, 261 |
| 257 | 68, 265 |
| 317 | 80, 325, 565, 581 |
| 333 | 341, 533 |
| 381 | 13, 112, 389, 501 |
| 415 | 423, 439 |
| 431 | 423, 439 |
| 461 | 213, 469, 485 |
| 475 | 483, 499 |
| 476 | 484, 500 |
| 477 | 213, 469, 485 |
| 478 | 486, 502 |
| 479 | 487, 503 |
| 480 | 488, 504 |
| 481 | 489, 505 |
| 491 | 483, 499 |
| 492 | 484, 500 |
| 493 | 13, 112, 389, 501 |
| 494 | 486, 502 |
| 495 | 487, 503 |
| 496 | 488, 504 |
| 497 | 489, 505 |
| 525 | 341,533 |
| 545 | 553, 585 |
| 554 | 562, 578 |
| 556 | 564, 580 |
| 557 | 80, 325, 565, 581 |
| 558 | 566, 582 |
| 570 | 562, 578 |
| 572 | 564, 580 |
| 573 | 80, 325, 565, 581 |
| 574 | 566, 582 |
| 577 | 553, 585 |

In some instances, multiple sequence identifiers refer to the same polypeptide or polynucleotide sequence, as summarized in Table 4. References to these sequence identifiers are understood to be interchangeable, except where context indicates otherwise.

**Table 4. Repeated sequences. Each cell lists a group of repeated sequences included in the sequence listing.**

| **SEQ ID NOs of repeated sequences** |
|---|
| 4, 103 |
| 5, 104, 381, 493 |
| 6, 105 |
| 9, 108 |
| 12, 111 |
| 13, 112 |
| 14, 113 |
| 17, 116 |
| 39, 252 |
| 40, 253 |
| 48, 261 |
| 60, 257 |
| 68, 265 |
| 72, 317, 557, 573 |
| 80, 325, 565, 581 |
| 89, 126, 158 |
| 97, 134, 166 |
| 120, 659 |
| 190, 206 |
| 191, 207 |
| 198, 214 |
| 199, 215 |
| 205, 461, 477 |
| 213, 469 |
| 333, 525 |
| 415, 431 |
| 423, 439 |
| 475,491 |
| 476,492 |
| 478, 494 |
| 479, 495 |
| 480, 496 |
| 481, 497 |
| 483, 499 |
| 484, 500 |
| 486, 502 |
| 487, 503 |
| 488, 504 |
| 489, 505 |
| 545,577 |
| 554, 570 |
| 556, 572 |
| 558, 574 |
| 562, 578 |
| 564, 580 |
| 566, 582 |

Further described herein are polynucleotides that hybridize under moderately or highly stringent hybridization conditions to a polynucleotide having one of the exemplary coding sequences recited in Table 1, and also include polynucleotides that hybridize under moderately or highly stringent hybridization conditions to a polynucleotide encoding the same polypeptide as a polynucleotide having one of the exemplary coding sequences recited in Table 1, or polypeptide encoded by any of the foregoing polynucleotides.

The phrase "high stringency hybridization conditions" refers to conditions under which a probe will hybridize to its target subsequence, typically in a complex mixture of nucleic acid, but to no other sequences. High stringency conditions are sequence dependent and will be different in different circumstances. Longer sequences hybridize specifically at higher temperatures. An extensive guide to the hybridization of nucleic acids is found in Tijssen, Techniques in Biochemistry and Molecular Biology--Hybridization with Nucleic Probes, "Overview of principles of hybridization and the strategy of nucleic acid assays" (1993). Generally, high stringency conditions are selected to be about 5-10 °C lower than the thermal melting point (Tm) for the specific sequence at a defined ionic strength pH. The Tm is the temperature (under defined ionic strength, pH, and nucleic concentration) at which 50% of the probes complementary to the target hybridize to the target sequence at equilibrium (as the target sequences are present in excess, at Tm, 50% of the probes are occupied at equilibrium). High stringency conditions will be those in which the salt concentration is less than about 1.0 M sodium ion, typically about 0.01 to 1.0 M sodium ion concentration (or other salts) at pH 7.0 to 8.3 and the temperature is at least about 30 °C for short probes (e.g., 10 to 50 nucleotides) and at least about 60 °C for long probes (e.g., greater than 50 nucleotides). High stringency conditions may also be achieved with the addition of destabilizing agents such as formamide. For selective or specific hybridization, a positive signal is at least two times background, optionally 10 times background hybridization. Exemplary high stringency hybridization conditions can be as following: 50% formamide, 5xSSC, and 1% SDS, incubating at 42 °C, or, 5xSSC, 1% SDS, incubating at 65 °C, with wash in 0.2xSSC, and 0.1% SDS at 65 °C. Such hybridizations and wash steps can be carried out for, e.g., 1, 2, 5, 10, 15, 30, 60; or more minutes.

Nucleic acids that do not hybridize to each other under high stringency conditions are still substantially related if the polypeptides that they encode are substantially related. This occurs, for example, when a copy of a nucleic acid is created using the maximum codon degeneracy permitted by the genetic code. In such cases, the nucleic acids typically hybridize under moderate stringency hybridization conditions. Exemplary "moderate stringency hybridization conditions" include a hybridization in a buffer of 40% formamide, 1 M NaCl, 1% SDS at 37 °C., and a wash in 1×SSC at 45 °C. Such hybridizations and wash steps can be carried out for, e.g., 1, 2, 5, 10, 15, 30, 60, or more minutes. A positive hybridization is at least twice background. Those of ordinary skill will readily recognize that alternative hybridization and wash conditions can be utilized to provide conditions of similar stringency.

### Additional Exemplary Embodiments and Disclosures

Exemplary constant regions that provide for the production of aglycosylated antibodies in Pichia are comprised in SEQ ID NO: 588 and SEQ ID NO: 586 which respectively are encoded by the nucleic acid sequences in SEQ ID NO: 589 and SEQ ID NO: 587.

In one embodiment of the invention, the anti-IL-6 antibody as defined in the claims may specifically bind to IL-6 expressing human cells and/or to circulating soluble IL-6 molecules in vivo, including IL-6 expressed on or by human cells in a patient with a disease associated with cells that express IL-6.

Described herein are anti-IL-6 antibodies or fragments or variants thereof directly or indirectly attached to a detectable label or therapeutic agent.

Described herein are one or more nucleic acid sequences which result in the expression of an anti-IL-6 antibody or antibody fragment or variant thereof as set forth above, including those comprising, or alternatively consisting of, yeast or human preferred codons. The invention also contemplates vectors (including plasmids or recombinant viral vectors) comprising said nucleic acid sequence(s). The invention also contemplates host cells or recombinant host cells expressing at least one of the antibodies set forth above, including a mammalian, yeast, bacterial, and insect cells. The host cell may be a yeast cell. Tthe yeast cell may be a diploidal yeast cell. The yeast cell may be a Pichia yeast.

The invention provides a composition comprising an antibody as defined in the claims for use in the prevention or treatment of a condition in a human subject associated with elevated IL-6. The method of treatment may comprise administering to a patient with a disease or condition associated with IL-6 expressing cells a therapeutically effective amount of at least one anti-IL-6 antibody as defined in the claims. The diseases that may be treated are presented in the claims. In a preferred embodiment, the disease is selected from a cancer, autoimmune disease, or inflammatory condition. In a particularly preferred embodiment, the disease is cancer or viral infection. In another embodiment the treatment further includes the administration of another therapeutic agent or regimen selected from chemotherapy, radiotherapy, cytokine administration or gene therapy.

Also described herein is a method of in vivo imaging which detects the presence of cells which express IL-6 comprising administering a diagnostically effective amount of at least one anti-IL-6 antibody. Said administration may further include the administration of a radionuclide or fluorophore that facilitates detection of the antibody at IL-6 expressing disease sites. The method of in vivo imaging may be used to detect IL-6 expressing tumors or metastases or is used to detect the presence of sites of autoimmune disorders associated with IL-6 expressing cells. The results of said in vivo imaging method may be used to facilitate design of an appropriate therapeutic regimen, including therapeutic regimens including radiotherapy, chemotherapy or a combination thereof.

### Anti-IL-6 Activity

As stated previously, IL-6 is a member of a family of cytokines that promote cellular responses through a receptor complex consisting of at least one subunit of the signal-transducing glycoprotein gp130 and the IL-6 receptor (IL-6R). The IL-6R may also be present in a soluble form (sIL-6R). IL-6 binds to IL-6R, which then dimerizes the signal-transducing receptor gp130.

It is believed that the anti-IL-6 antibodies of the invention, or IL-6 binding fragments or variants thereof, are useful by exhibiting anti-IL-6 activity. The anti-IL-6 antibodies of the invention, or IL-6 binding fragments or variants thereof, exhibit anti-IL-6 activity by binding to IL-6 which may be soluble IL-6 or cell surface expressed IL-6 and/or may prevent or inhibit the binding of IL-6 to IL-6R and/or activation (dimerization) of the gp130 signal-transducing glycoprotein and the formation of IL-6/IL-6R/gp130 multimers and the biological effects of any of the foregoing. The subject anti-IL-6 antibodies may possess different antagonistic activities based on where (i.e., epitope) the particular antibody binds IL-6 and/or how it affects the formation of the foregoing IL-6 complexes and/or multimers and the biological effects thereof. Consequently, different anti-IL-6 antibodies according to the invention e.g., may be better suited for preventing or treating conditions involving the formation and accumulation of substantial soluble IL-6 such as rheumatoid arthritis whereas other antibodies may be favored in treatments wherein the prevention of IL-6/IL-6R/gp130 or IL-6/IL-6R/gp130 multimers is a desired therapeutic outcome. This can be determined in binding and other assays.

The anti-IL-6 activity of the anti-IL-6 antibody defined in the claims may also be described by their strength of binding or their affinity for IL-6. This also may affect their therapeutic properties. In one embodiment of the invention, the anti-IL-6 antibodies defined in the claims bind to IL-6 with a dissociation constant (K_{D}) of less than or equal to 5x10⁻⁷, 10⁻⁷, 5x10⁻⁸, 10⁻⁸ 5x10⁻⁹, 10⁻⁹ 5x10⁻¹⁰, 10⁻¹⁰ , 5x10⁻¹¹ , 10⁻¹¹, 5x10⁻¹² , 10⁻¹² 5x10⁻¹³, 10⁻¹³ , 5x10⁻¹⁴, 10⁻¹⁴, 5x10⁻¹⁵ or 10⁻¹⁵. Preferably, the anti-IL-6 antibodies bind IL-6 with a dissociation constant of less than or equal to 5x10⁻¹⁰.

In another embodiment of the invention, the anti-IL-6 activity of the anti-IL-6 antibodies defined in the claims, bind to IL-6 with an off-rate of less than or equal to 10⁻⁴ S⁻¹, 5x10⁻⁵ S⁻¹, 10⁻⁵ S⁻¹, 5x10⁻⁶ S⁻¹, 10⁻⁶ S⁻¹, 5x10⁻⁷ S⁻¹, or 10⁻⁷ S⁻¹. In one embodiment of the invention, the anti-IL-6 antibodies bind to a linear or conformational IL-6 epitope.

In a further embodiment of the invention, the anti-IL-6 activity of the anti-IL-6 antibodies defined in the claims exhibit anti-IL-6 activity by ameliorating or reducing the symptoms of, or alternatively treating, or preventing, diseases and disorders associated with elevated IL-6. Limiting examples of diseases and disorders associated with elevated IL-6 are set forth in the claims. As noted rheumatoid arthritis is one preferred indication for the subject anti-IL-6 antibodies.

In another embodiment of the invention, the anti-IL-6 antibodies may not have binding specificity for IL-6R or the gp-130 signal-transducing glycoprotein.

### B-cell Screening and Isolation

Described herein are methods of isolating a clonal population of antigen-specific B cells that may be used for isolating at least one antigen-specific cell. As described and exemplified infra, these methods contain a series of culture and selection steps that can be used separately, in combination, sequentially, repetitively, or periodically. Preferably, these methods are used for isolating at least one antigen-specific cell, which can be used to produce a monoclonal antibody, which is specific to a desired antigen, or a nucleic acid sequence corresponding to such an antibody.

Described herein is a method comprising the steps of:
a. preparing a cell population comprising at least one antigen-specific B cell;
b. enriching the cell population, e.g., by chromatography, to form an enriched cell population comprising at least one antigen-specific B cell;
c. isolating a single B cell from the enriched B cell population; and
d. determining whether the single B cell produces an antibody specific to the antigen.

Described herein is an improvement to a method of isolating a single, antibody-producing B cell, the improvement comprising enriching a B cell population obtained from a host that has been immunized or naturally exposed to an antigen, wherein the enriching step precedes any selection steps, comprises at least one culturing step, and results in a clonal population of B cells that produces a single monoclonal antibody specific to said antigen.

Throughout this application, a "clonal population of B cells" refers to a population of B cells that only secrete a single antibody specific to a desired antigen. That is to say that these cells produce only one type of monoclonal antibody specific to the desired antigen.

In the present application, "enriching" a cell population cells means increasing the frequency of desired cells, typically antigen-specific cells, contained in a mixed cell population, e.g., a B cell-containing isolate derived from a host that is immunized against a desired antigen. Thus, an enriched cell population encompasses a cell population having a higher frequency of antigen-specific cells as a result of an enrichment step, but this population of cells may contain and produce different antibodies.

The general term "cell population" encompasses pre- and a post-enrichment cell populations, keeping in mind that when multiple enrichment steps are performed, a cell population can be both pre- and post-enrichment. For example, described herein is a method comprising:
a. harvesting a cell population from an immunized host to obtain a harvested cell population;
b. creating at least one single cell suspension from the harvested cell population;
c. enriching at least one single cell suspension to form a first enriched cell population;
d. enriching the first enriched cell population to form a second enriched cell population;
e. enriching the second enriched cell population to form a third enriched cell population; and
f. selecting an antibody produced by an antigen-specific cell of the third enriched cell population.

Each cell population may be used directly in the next step, or it can be partially or wholly frozen for long- or short- term storage or for later steps. Also, cells from a cell population can be individually suspended to yield single cell suspensions. The single cell suspension can be enriched, such that a single cell suspension serves as the pre-enrichment cell population. Then, one or more antigen-specific single cell suspensions together form the enriched cell population; the antigen-specific single cell suspensions can be grouped together, e.g., re-plated for further analysis and/or antibody production.

Described herein is a method of enriching a cell population to yield an enriched cell population having an antigen-specific cell frequency that is about 50% to about 100%, or increments therein. Preferably, the enriched cell population has an antigen-specific cell frequency greater than or equal to about 50%, 60%, 70%, 75%, 80%, 90%, 95%, 99%, or 100%.

Described herein isa method of enriching a cell population whereby the frequency of antigen-specific cells is increased by at least about 2-fold, 5-fold, 10-fold, 20-fold, 50-fold, 100-fold, or increments therein.

Throughout this application, the term "increment" is used to define a numerical value in varying degrees of precision, e.g., to the nearest 10, 1, 0.1, 0.01, etc. The increment can be rounded to any measurable degree of precision, and the increment need not be rounded to the same degree of precision on both sides of a range. For example, the range 1 to 100 or increments therein includes ranges such as 20 to 80, 5 to 50, and 0.4 to 98. When a range is open-ended, e.g., a range of less than 100, increments therein means increments between 100 and the measurable limit. For example, less than 100 or increments therein means 0 to 100 or increments therein unless the feature, e.g., temperature, is not limited by 0.

Antigen-specificity can be measured with respect to any antigen. The antigen can be any substance to which an antibody can bind including, but not limited to, peptides, proteins or fragments thereof; carbohydrates; organic and inorganic molecules; receptors produced by animal cells, bacterial cells, and viruses; enzymes; agonists and antagonists of biological pathways; hormones; and cytokines. Exemplary antigens include, but are not limited to, IL-2, IL-4, IL-6, IL-10, IL-12, IL-13, IL-18, IFN-*alpha,* IFN-*gamma,* BAFF, CXCL13, IP-10, VEGF, EPO, EGF, HRG, Hepatocyte Growth Factor (HGF) and Hepcidin. Preferred antigens include IL-6, IL-13, TNF-*alpha,* VEGF-*alpha,* Hepatocyte Growth Factor (HGF) and Hepcidin. In a method utilizing more than one enrichment step, the antigen used in each enrichment step can be the same as or different from one another. Multiple enrichment steps with the same antigen may yield a large and/or diverse population of antigen-specific cells; multiple enrichment steps with different antigens may yield an enriched cell population with cross-specificity to the different antigens.

Enriching a cell population can be performed by any cell-selection means known in the art for isolating antigen-specific cells. For example, a cell population can be enriched by chromatographic techniques, e.g., Miltenyi bead or magnetic bead technology. The beads can be directly or indirectly attached to the antigen of interest. The method of enriching a cell population may include at least one chromatographic enrichment step.

A cell population can also be enriched by performed by any antigen-specificity assay technique known in the art, e.g., an ELISA assay or a halo assay. ELISA assays include, but are not limited to, selective antigen immobilization (e.g., biotinylated antigen capture by streptavidin, avidin, or neutravidin coated plate), non-specific antigen plate coating, and through an antigen build-up strategy (e.g., selective antigen capture followed by binding partner addition to generate a heteromeric protein-antigen complex). The antigen can be directly or indirectly attached to a solid matrix or support, e.g., a column. A halo assay comprises contacting the cells with antigen-loaded beads and labeled anti-host antibody specific to the host used to harvest the B cells. The label can be, e.g., a fluorophore. In one embodiment, at least one assay enrichment step is performed on at least one single cell suspension. The method of enriching a cell population may include at least one chromatographic enrichment step and at least one assay enrichment step.

Methods of "enriching" a cell population by size or density are known in the art. See, e.g., U.S. Patent 5,627,052. These steps can be used in the present method in addition to enriching the cell population by antigen-specificity.

The cell populations described herein contain at least one cell capable of recognizing an antigen. Antigen-recognizing cells include, but are not limited to, B cells, plasma cells, and progeny thereof. Described herein isa clonal cell population containing a single type of antigen-specific B-cell, i.e., the cell population produces a single monoclonal antibody specific to a desired antigen.

It is believed that the clonal antigen-specific population of B cells consists predominantly of antigen-specific, antibody-secreting cells, which are obtained by the novel culture and selection protocol provided herein. Accordingly, described herein are methods for obtaining an enriched cell population containing at least one antigen-specific, antibody-secreting cell. Described herein isan enriched cell population containing about 50% to about 100%, or increments therein, or greater than or equal to about 60%, 70%, 80%, 90%, or 100% of antigen-specific, antibody-secreting cells.

Described herein isa method of isolating a single B cell by enriching a cell population obtained from a host before any selection steps, e.g., selecting a particular B cell from a cell population and/or selecting an antibody produced by a particular cell. The enrichment step can be performed as one, two, three, or more steps. A single B cell is isolated from an enriched cell population before confirming whether the single B cell secretes an antibody with antigen-specificity and/or a desired property.

A method of enriching a cell population is used in a method for antibody production and/or selection. Thus, described herein is a method comprising enriching a cell population before selecting an antibody. The method can include the steps of: preparing a cell population comprising at least one antigen-specific cell, enriching the cell population by isolating at least one antigen-specific cell to form an enriched cell population, and inducing antibody production from at least one antigen-specific cell. The enriched cell population may contain more than one antigen-specific cell. Each antigen-specific cell of the enriched population may be cultured under conditions that yield a clonal antigen-specific B cell population before isolating an antibody producing cell therefrom and/or producing an antibody using said B cell, or a nucleic acid sequence corresponding to such an antibody. In contrast to prior techniques where antibodies are produced from a cell population with a low frequency of antigen-specific cells, the described method allows antibody selection from among a high frequency of antigen-specific cells. Because an enrichment step is used prior to antibody selection, the majority of the cells, preferably virtually all of the cells, used for antibody production are antigen-specific. By producing antibodies from a population of cells with an increased frequency of antigen specificity, the quantity and variety of antibodies are increased.

In the antibody selection methods described herein, an antibody is preferably selected after an enrichment step and a culture step that results in a clonal population of antigen-specific B cells. The methods can further comprise a step of sequencing a selected antibody or portions thereof from one or more isolated, antigen-specific cells. Any method known in the art for sequencing can be employed and can include sequencing the heavy chain, light chain, variable region(s), and/or complementarity determining region(s) (CDR).

In addition to the enrichment step, the method for antibody selection can also include one or more steps of screening a cell population for antigen recognition and/or antibody functionality. For example, the desired antibodies may have specific structural features, such as binding to a particular epitope or mimicry of a particular structure; antagonist or agonist activity; or neutralizing activity, e.g., inhibiting binding between the antigen and a ligand. The antibody functionality screen may be ligand-dependent. Screening for antibody functionality includes, but is not limited to, an in vitro protein-protein interaction assay that recreates the natural interaction of the antigen ligand with recombinant receptor protein; and a cell-based response that is ligand dependent and easily monitored (e.g., proliferation response). The method for antibody selection may include a step of screening the cell population for antibody functionality by measuring the inhibitory concentration (IC50). At least one of the isolated, antigen-specific cells produces an antibody having an IC50 of less than about 100, 50, 30, 25, 10 µg/mL, or increments therein.

In addition to the enrichment step, the method for antibody selection can also include one or more steps of screening a cell population for antibody binding strength. Antibody binding strength can be measured by any method known in the art (e.g., Biacore™). At least one of the isolated, antigen-specific cells may produce an antibody having a high antigen affinity, e.g., a dissociation constant (Kd) of less than about 5x10⁻¹⁰ M-1, preferably about 1x10⁻¹³ to 5x10⁻¹⁰, 1x10⁻¹² to 1x10⁻¹⁰, 1x10⁻¹² to 7.5x10⁻¹¹, 1x10⁻¹¹ to 2x10⁻¹¹, about 1.5x10⁻¹¹ or less, or increments therein. In this embodiment, the antibodies are said to be affinity mature. The affinity of the antibodies may be comparable to or higher than the affinity of any one of Panorex® (edrecolomab), Rituxan® (rituximab), Herceptin® (traztuzumab), Mylotarg® (gentuzumab), Campath® (alemtuzumab), Zevalin™ (ibritumomab), Erbitux™ (cetuximab), Avastin™ (bevicizumab), Raptiva™ (efalizumab), Remicade® (infliximab), Humira™ (adalimumab), and Xolair™ (omalizumab). Preferably, the affinity of the antibodies is comparable to or higher than the affinity of Humira™. The affinity of an antibody can also be increased by known affinity maturation techniques. At least one cell population may be screened for at least one of, preferably both, antibody functionality and antibody binding strength.

In addition to the enrichment step, the method for antibody selection can also include one or more steps of screening a cell population for antibody sequence homology, especially human homology. At least one of the isolated, antigen-specific cells may produce an antibody that has a homology to a human antibody of about 50% to about 100%, or increments therein, or greater than about 60%, 70%, 80%, 85%, 90%, or 95% homologous. The antibodies can be humanized to increase the homology to a human sequence by techniques known in the art such as CDR grafting or selectivity determining residue grafting (SDR).

The B cell selection protocol disclosed herein has a number of intrinsic advantages versus other methods for obtaining antibody-secreting B cells and monoclonal antibodies specific to desired target antigens. These advantages include, but are not restricted to, the following:

First, it has been found that when these selection procedures are utilized with a desired antigen such as IL-6 or TNF*-alpha,* the methods reproducibly result in antigen-specific B cells capable of generating what appears to be a substantially comprehensive complement of antibodies, i.e., antibodies that bind to the various different epitopes of the antigen. Without being bound by theory, it is hypothesized that the comprehensive complement is attributable to the antigen enrichment step that is performed prior to initial B cell recovery. Moreover, this advantage allows for the isolation and selection of antibodies with different properties as these properties may vary depending on the epitopic specificity of the particular antibody.

Second, it has been found that the B cell selection protocol reproducibly yields a clonal B cell culture containing a single B cell, or its progeny, secreting a single monoclonal antibody that generally binds to the desired antigen with a relatively high binding affinity, i.e. picomolar or better antigen binding affinities. By contrast, prior antibody selection methods tend to yield relatively few high affinity antibodies and therefore require extensive screening procedures to isolate an antibody with therapeutic potential. Without being bound by theory, it is hypothesized that the protocol results in both in vivo B cell immunization of the host (primary immunization) followed by a second in vitro B cell stimulation (secondary antigen priming step) that may enhance the ability and propensity of the recovered clonal B cells to secrete a single high affinity monoclonal antibody specific to the antigen target.

Third, it has been observed (as shown herein with IL-6 specific B cells) that the B cell selection protocol reproducibly yields enriched B cells producing IgG's that are, on average, highly selective (antigen specific) to the desired target. Antigen-enriched B cells recovered by these methods are believed to contain B cells capable of yielding the desired full complement of epitopic specificities as discussed above.

Fourth, it has been observed that the B cell selection protocols, even when used with small antigens, i.e., peptides of 100 amino acids or less, e.g., 5-50 amino acids long, reproducibly give rise to a clonal B cell culture that secretes a single high affinity antibody to the small antigen, e.g., a peptide. This is highly surprising as it is generally quite difficult, labor intensive, and sometimes not even feasible to produce high affinity antibodies to small peptides. Accordingly, the method can be used to produce therapeutic antibodies to desired peptide targets, e.g., viral, bacterial or autoantigen peptides, thereby allowing for the production of monoclonal antibodies with very discrete binding properties or even the production of a cocktail of monoclonal antibodies to different peptide targets, e.g., different viral strains. This advantage may especially be useful in the context of the production of a therapeutic or prophylactic vaccine having a desired valency, such as an HPV vaccine that induces protective immunity to different HPV strains.

Fifth, the B cell selection protocol, particularly when used with B cells derived from rabbits, tends to reproducibly yield antigen-specific antibody sequences that are very similar to endogenous human immunoglobulins (around 90% similar at the amino acid level) and that contain CDRs that possess a length very analogous to human immunoglobulins and therefore require little or no sequence modification (typically at most only a few CDR residues may be modified in the parent antibody sequence and no framework exogenous residues introduced) in order to eliminate potential immunogenicity concerns. In particular, preferably the recombinant antibody will contain only the host (rabbit) CDR1 and CDR2 residues required for antigen recognition and the entire CDR3. Thereby, the high antigen binding affinity of the recovered antibody sequences produced according to the B cell and antibody selection protocol remains intact or substantially intact even with humanization.

In sum, these methods can be used to produce antibodies exhibiting higher binding affinities to more distinct epitopes by the use of a more efficient protocol than was previously known.

Described herein isa method for identifying a single B cell that secretes an antibody specific to a desired antigen and that optionally possesses at least one desired functional property such as affinity, avidity, cytolytic activity, and the like by a process including the following steps:
a. immunizing a host against an antigen;
b. harvesting B cells from the host;
c. enriching the harvested B cells to increase the frequency of antigen-specific cells;
d. creating at least one single cell suspension;
e. culturing a sub-population from the single cell suspension under conditions that favor the survival of a single antigen-specific B cell per culture well;
f. isolating B cells from the sub-population; and
g. determining whether the single B cell produces an antibody specific to the antigen.

Typically, these methods will further comprise an additional step of isolating and sequencing, in whole or in part, the polypeptide and nucleic acid sequences encoding the desired antibody. These sequences or modified versions or portions thereof can be expressed in desired host cells in order to produce recombinant antibodies to a desired antigen.

As noted previously, it is believed that the clonal population of B cells predominantly comprises antibody-secreting B cells producing antibody against the desired antigen. It is also believed based on experimental results obtained with several antigens and with different B cell populations that the clonally produced B cells and the isolated antigen-specific B cells derived therefrom produced secrete a monoclonal antibody that is typically of relatively high affinity and moreover is capable of efficiently and reproducibly producing a selection of monoclonal antibodies of greater epitopic variability as compared to other methods of deriving monoclonal antibodies from cultured antigen-specific B cells. The population of immune cells used in such B cell selection methods may be derived from a rabbit. However, other hosts that produce antibodies, including non-human and human hosts, can alternatively be used as a source of immune B cells. It is believed that the use of rabbits as a source of B cells may enhance the diversity of monoclonal antibodies that may be derived by the methods. Also, the antibody sequences derived from rabbits typically possess sequences having a high degree of sequence identity to human antibody sequences making them favored for use in humans since they should possess little antigenicity. In the course of humanization, the final humanized antibody contains a much lower foreign/host residue content, usually restricted to a subset of the host CDR residues that differ dramatically due to their nature versus the human target sequence used in the grafting. This enhances the probability of complete activity recovery in the humanized antibody protein.

The methods of antibody selection using an enrichment step disclosed herein include a step of obtaining an immune cell-containing cell population from an immunized host. Methods of obtaining an immune cell-containing cell population from an immunized host are known in the art and generally include inducing an immune response in a host and harvesting cells from the host to obtain one or more cell populations. The response can be elicited by immunizing the host against a desired antigen. Alternatively, the host used as a source of such immune cells can be naturally exposed to the desired antigen such as an individual who has been infected with a particular pathogen such as a bacterium or virus or alternatively has mounted a specific antibody response to a cancer that the individual is afflicted with.

Host animals are well-known in the art and include, but are not limited to, guinea pig, rabbit, mouse, rat, non-human primate, human, as well as other mammals and rodents, chicken, cow, pig, goat, and sheep. Preferably the host is a mammal, more preferably, rabbit, mouse, rat, or human. When exposed to an antigen, the host produces antibodies as part of the native immune response to the antigen. As mentioned, the immune response can occur naturally, as a result of disease, or it can be induced by immunization with the antigen. Immunization can be performed by any method known in the art, such as, by one or more injections of the antigen with or without an agent to enhance immune response, such as complete or incomplete Freund's adjuvant. Also described herein is intrasplenic immunization. As an alternative to immunizing a host animal in vivo, the method can comprise immunizing a host cell culture in vitro.

After allowing time for the immune response (e.g., as measured by serum antibody detection), host animal cells are harvested to obtain one or more cell populations. A harvested cell population may be screened for antibody binding strength and/or antibody functionality. A harvested cell population is preferably from at least one of the spleen, lymph nodes, bone marrow, and/or peripheral blood mononuclear cells (PBMCs). The cells can be harvested from more than one source and pooled. Certain sources may be preferred for certain antigens. For example, the spleen, lymph nodes, and PBMCs are preferred for IL-6; and the lymph nodes are preferred for TNF. The cell population is harvested about 20 to about 90 days or increments therein after immunization, preferably about 50 to about 60 days. A harvested cell population and/or a single cell suspension therefrom can be enriched, screened, and/or cultured for antibody selection. The frequency of antigen-specific cells within a harvested cell population is usually about 1% to about 5%, or increments therein.

A single cell suspension from a harvested cell population may be enriched, preferably by using Miltenyi beads. From the harvested cell population having a frequency of antigen-specific cells of about 1% to about 5%, an enriched cell population is thus derived having a frequency of antigen-specific cells approaching 100%.

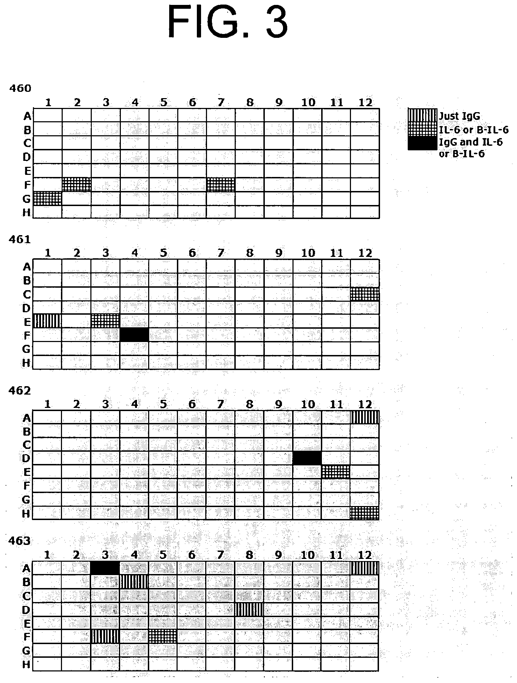

The method of antibody selection using an enrichment step includes a step of producing antibodies from at least one antigen-specific cell from an enriched cell population. Methods of producing antibodies in vitro are well known in the art, and any suitable method can be employed. An enriched cell population, such as an antigen-specific single cell suspension from a harvested cell population, may be plated at various cell densities, such as 50, 100, 250, 500, or other increments between 1 and 1000 cells per well. Preferably, the sub-population comprises no more than about 10,000 antigen-specific, antibody-secreting cells, more preferably about 50-10,000, about 50-5,000, about 50-1,000, about 50-500, about 50-250 antigen-specific, antibody-secreting cells, or increments therein. Then, these sub-populations are cultured with suitable medium (e.g., an activated T cell conditioned medium, particularly 1-5% activated rabbit T cell conditioned medium) on a feeder layer, preferably under conditions that favor the survival of a single proliferating antibody-secreting cell per culture well. The feeder layer, generally comprised of irradiated cell matter, e.g., EL4B cells, does not constitute part of the cell population. The cells are cultured in a suitable media for a time sufficient for antibody production, for example about 1 day to about 2 weeks, about 1 day to about 10 days, at least about 3 days, about 3 to about 5 days, about 5 days to about 7 days, at least about 7 days, or other increments therein. In one embodiment, more than one sub-population is cultured simultaneously. Preferably, a single antibody-producing cell and progeny thereof survives in each well, thereby providing a clonal population of antigen-specific B cells in each well. At this stage, the immunoglobulin G (IgG) produced by the clonal population is highly correlative with antigen specificity. The IgGs may exhibit a correlation with antigen specificity that is greater than about 50%, more preferably greater than 70%, 85%, 90%, 95%, 99%, or increments therein. See Fig. 3, which demonstrates an exemplary correlation for IL-6. The correlations were demonstrated by setting up B cell cultures under limiting conditions to establish single antigen-specific antibody products per well. Antigen-specific versus general IgG synthesis was compared. Three populations were observed: IgG that recognized a single format of antigen (biotinylated and direct coating), detectable IgG and antigen recognition irrespective of immobilization, and IgG production alone. IgG production was highly correlated with antigen-specificity.

A supernatant containing the antibodies is optionally collected, which can be enriched, screened, and/or cultured for antibody selection according to the steps described above. The supernatant may be enriched (preferably by an antigen-specificity assay, especially an ELISA assay) and/or screened for antibody functionality.

The enriched, preferably clonal, antigen-specific B cell population from which a supernatant described above may be optionally screened in order to detect the presence of the desired secreted monoclonal antibody is used for the isolation of a few B cells, preferably a single B cell, which is then tested in an appropriate assay in order to confirm the presence of a single antibody-producing B cell in the clonal B cell population. In one embodiment about 1 to about 20 cells are isolated from the clonal B cell population, preferably less than about 15, 12, 10, 5, or 3 cells, or increments therein, most preferably a single cell. The screen is preferably effected by an antigen-specificity assay, especially a halo assay. The halo assay can be performed with the full length protein, or a fragment thereof. The antibody-containing supernatant can also be screened for at least one of: antigen binding affinity; agonism or antagonism of antigen-ligand binding, induction or inhibition of the proliferation of a specific target cell type; induction or inhibition of lysis of a target cell, and induction or inhibition of a biological pathway involving the antigen.

The identified antigen-specific cell can be used to derive the corresponding nucleic acid sequences encoding the desired monoclonal antibody. (An AluI digest can confirm that only a single monoclonal antibody type is produced per well.) As mentioned above, these sequences can be mutated, such as by humanization, in order to render them suitable for use in human medicaments.

As mentioned, the enriched B cell population used in the process can also be further enriched, screened, and/or cultured for antibody selection according to the steps described above which can be repeated or performed in a different order. At least one cell of an enriched, preferably clonal, antigen-specific cell population may be isolated, cultured, and used for antibody selection.

Thus, described herein isa method comprising:
a. harvesting a cell population from an immunized host to obtain a harvested cell population;
b. creating at least one single cell suspension from a harvested cell population;
c. enriching at least one single cell suspension, preferably by chromatography, to form a first enriched cell population;
d. enriching the first enriched cell population, preferably by ELISA assay, to form a second enriched cell population which preferably is clonal, i.e., it contains only a single type of antigen-specific B cell;
e. enriching the second enriched cell population, preferably by halo assay, to form a third enriched cell population containing a single or a few number of B cells that produce an antibody specific to a desired antigen; and
f. selecting an antibody produced by an antigen-specific cell isolated from the third enriched cell population.

The method can further include one or more steps of screening the harvested cell population for antibody binding strength (affinity, avidity) and/or antibody functionality. Suitable screening steps include, but are not limited to, assay methods that detect: whether the antibody produced by the identified antigen-specific B cell produces an antibody possessing a minimal antigen binding affinity, whether the antibody agonizes or antagonizes the binding of a desired antigen to a ligand; whether the antibody induces or inhibits the proliferation of a specific cell type; whether the antibody induces or elicits a cytolytic reaction against target cells; whether the antibody binds to a specific epitope; and whether the antibody modulates (inhibits or agonizes) a specific biological pathway or pathways involving the antigen.

Similarly, the method can include one or more steps of screening the second enriched cell population for antibody binding strength and/or antibody functionality.

The method can further include a step of sequencing the polypeptide sequence or the corresponding nucleic acid sequence of the selected antibody. The method can also include a step of producing a recombinant antibody using the sequence, a fragment thereof, or a genetically modified version of the selected antibody. Methods for mutating antibody sequences in order to retain desired properties are well known to those skilled in the art and include humanization, chimerisation, production of single chain antibodies; these mutation methods can yield recombinant antibodies possessing desired effector function, immunogenicity, stability, removal or addition of glycosylation, and the like. The recombinant antibody can be produced by any suitable recombinant cell, including, but not limited to mammalian cells such as CHO, COS, BHK, HEK-293, bacterial cells, yeast cells, plant cells, insect cells, and amphibian cells. The antibodies may be expressed in polyploidal yeast cells, i.e., diploid yeast cells, particularly Pichia.

Described herein is a method comprising:
a. immunizing a host against an antigen to yield host antibodies;
b. screening the host antibodies for antigen specificity and neutralization;
c. harvesting B cells from the host;
d. enriching the harvested B cells to create an enriched cell population having an increased frequency of antigen-specific cells;
e. culturing one or more sub-populations from the enriched cell population under conditions that favor the survival of a single B cell to produce a clonal population in at least one culture well;
f. determining whether the clonal population produces an antibody specific to the antigen;
g. isolating a single B cell; and
h. sequencing the nucleic acid sequence of the antibody produced by the single B cell.

### Methods of Humanizing Antibodies

Described herein isa method for humanizing antibody heavy and light chains. The following method may be followed for the humanization of the heavy and light chains:

### Light Chain

1. Identify the amino acid that is the first one following the signal peptide sequence. This is the start of Framework 1. The signal peptide starts at the first initiation methionine and is typically, but not necessarily 22 amino acids in length for rabbit light chain protein sequences. The start of the mature polypeptide can also be determined experimentally by N-terminal protein sequencing, or can be predicted using a prediction algorithm. This is also the start of Framework 1 as classically defined by those in the field.
   Example: RbtVL Amino acid residue 1 in Fig. 2, starting 'AYDM...'
2. Identify the end of Framework 3. This is typically 86-90 amino acids following the start of Framework 1 and is typically a cysteine residue preceded by two tyrosine residues. This is the end of the Framework 3 as classically defined by those in the field.
   Example: RbtVL amino acid residue 88 in Fig. 2, ending as 'TYYC'
3. Use the rabbit light chain sequence of the polypeptide starting from the beginning of Framework 1 to the end of Framework 3 as defined above and perform a sequence homology search for the most similar human antibody protein sequences. This will typically be a search against human germline sequences prior to antibody maturation in order to reduce the possibility of immunogenicity, however any human sequences can be used. Typically a program like BLAST can be used to search a database of sequences for the most homologous. Databases of human antibody sequences can be found from various sources such as NCBI (National Center for Biotechnology Information).
   Example: RbtVL amino acid sequence from residues numbered 1 through 88 in Fig. 2 is BLASTed against a human antibody germline database. The top three unique returned sequences are shown in Fig. 2 as L12A, V1 and Vx02.
4. Generally the most homologous human germline variable light chain sequence is then used as the basis for humanization. However those skilled in the art may decide to use another sequence that wasn't the highest homology as determined by the homology algorithm, based on other factors including sequence gaps and framework similarities.
   Example: In Fig. 2, L12A was the most homologous human germline variable light chain sequence and is used as the basis for the humanization of RbtVL.
5. Determine the framework and CDR arrangement (FR1, FR2, FR3, CDR1 & CDR2) for the human homolog being used for the light chain humanization. This is using the traditional layout as described in the field. Align the rabbit variable light chain sequence with the human homolog, while maintaining the layout of the framework and CDR regions.
   Example: In Fig. 2, the RbtVL sequence is aligned with the human homologous sequence L12A, and the framework and CDR domains are indicated.
6. Replace the human homologous light chain sequence CDR1 and CDR2 regions with the CDR1 and CDR2 sequences from the rabbit sequence. If there are differences in length between the rabbit and human CDR sequences then use the entire rabbit CDR sequences and their lengths. It is possible that the specificity, affinity and/or immunogenicity of the resulting humanized antibody may be unaltered if smaller or larger sequence exchanges are performed, or if specific residue(s) are altered, however the exchanges as described have been used successfully, but do not exclude the possibility that other changes may be permitted.
   Example: In Fig. 2, the CDR1 and CDR2 amino acid residues of the human homologous variable light chain L12A are replaced with the CDR1 and CDR2 amino acid sequences from the RbtVL rabbit antibody light chain sequence. The human L12A frameworks 1, 2 and 3 are unaltered. The resulting humanized sequence is shown below as VLh from residues numbered 1 through 88. Note that the only residues that are different from the L12A human sequence are underlined, and are thus rabbit-derived amino acid residues. In this example only 8 of the 88 residues are different than the human sequence.
7. After framework 3 of the new hybrid sequence created in Step 6, attach the entire CDR3 of the rabbit light chain antibody sequence. The CDR3 sequence can be of various lengths, but is typically 9 to 15 amino acid residues in length. The CDR3 region and the beginning of the following framework 4 region are defined classically and identifiable by those skilled in the art. Typically the beginning of Framework 4, and thus after the end of CDR3 consists of the sequence 'FGGG...', however some variation may exist in these residues.
   Example: In Fig. 2, the CDR3 of RbtVL (amino acid residues numbered 89-100) is added after the end of framework 3 in the humanized sequence indicated as VLh.
8. The rabbit light chain framework 4, which is typically the final 11 amino acid residues of the variable light chain and begins as indicated in Step 7 above and typically ends with the amino acid sequence '...VVKR' is replaced with the nearest human light chain framework 4 homolog, usually from germline sequence. Frequently this human light chain framework 4 is of the sequence 'FGGGTKVEIKR'. It is possible that other human light chain framework 4 sequences that are not the most homologous or otherwise different may be used without affecting the specificity, affinity and/or immunogenicity of the resulting humanized antibody. This human light chain framework 4 sequence is added to the end of the variable light chain humanized sequence immediately following the CDR3 sequence from Step 7 above. This is now the end of the variable light chain humanized amino acid sequence.

Example: In Fig. 2, Framework 4 (FR4) of the RbtVL rabbit light chain sequence is shown above a homologous human FR4 sequence. The human FR4 sequence is added to the humanized variable light chain sequence (VLh) right after the end of the CD3 region added in Step 7 above.

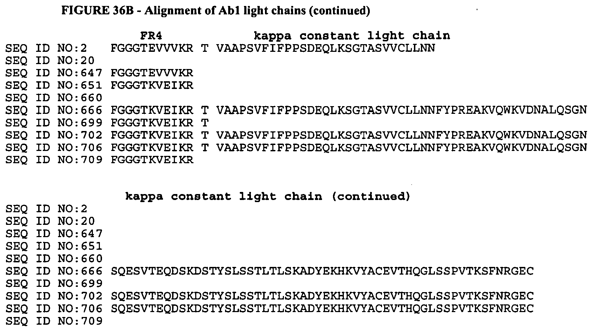

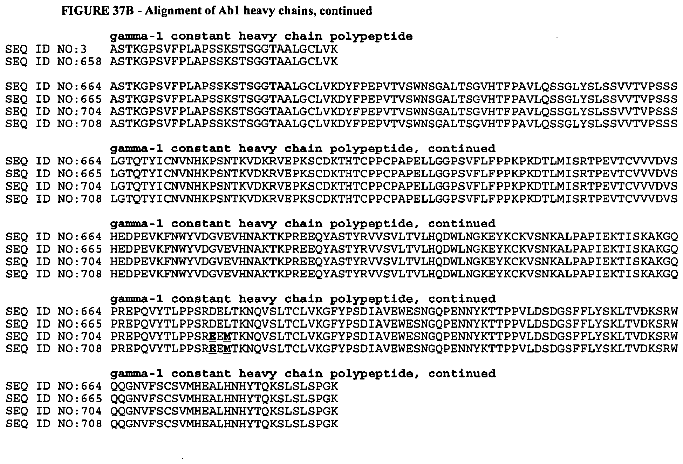

In addition, Figs. 34 and 35 depict preferred humanized anti-IL-6 variable heavy and variable light chain sequences humanized from the variable heavy and light regions in Ab1. These humanized light and heavy chain regions are respectively contained in the polypeptides contained in SEQ ID NO: 647, or 651 and in SEQ ID NO: 652, 656, 657 or 658. The CDR2 of the humanized variable heavy region in SEQ ID NO: 657 (containing a serine substitution in CDR2) is contained in SEQ ID NO: 658. Alignments illustrating variants of the light and heavy chains are shown in Figs. 36 and 37, respectively, with sequence differences within the CDR regions highlighted. Sequence identifiers of CDR sequences and of exemplary coding sequences are summarized in Table 1, above.

### Heavy Chain

1. Identify the amino acid that is the first one following the signal peptide sequence. This is the start of Framework 1. The signal peptide starts at the first initiation methionine and is typically 19 amino acids in length for rabbit heavy chain protein sequences. Typically, but not necessarily always, the final 3 amino acid residues of a rabbit heavy chain signal peptide are '...VQC', followed by the start of Framework 1. The start of the mature polypeptide can also be determined experimentally by N-terminal protein sequencing, or can be predicted using a prediction algorithm. This is also the start of Framework 1 as classically defined by those in the field.
   Example: RbtVH Amino acid residue 1 in Fig. 2, starting 'QEQL...'
2. Identify the end of Framework 3. This is typically 95-100 amino acids following the start of Framework 1 and typically has the final sequence of '...CAR' (although the alanine can also be a valine). This is the end of the Framework 3 as classically defined by those in the field.
   Example: RbtVH amino acid residue 98 in Fig. 2, ending as '...FCVR'.
3. Use the rabbit heavy chain sequence of the polypeptide starting from the beginning of Framework 1 to the end of Framework 3 as defined above and perform a sequence homology search for the most similar human antibody protein sequences. This will typically be against a database of human germline sequences prior to antibody maturation in order to reduce the possibility of immunogenicity, however any human sequences can be used. Typically a program like BLAST can be used to search a database of sequences for the most homologous. Databases of human antibody sequences can be found from various sources such as NCBI (National Center for Biotechnology Information).
   Example: RbtVH amino acid sequence from residues numbered 1 through 98 in Fig. 2 is BLASTed against a human antibody germline database. The top three unique returned sequences are shown in Fig. 2 as 3-64-04, 3-66-04, and 3-53-02.
4. Generally the most homologous human germline variable heavy chain sequence is then used as the basis for humanization. However those skilled in the art may decide to use another sequence that wasn't the most homologous as determined by the homology algorithm, based on other factors including sequence gaps and framework similarities.
   Example: 3-64-04 in Fig. 2 was the most homologous human germline variable heavy chain sequence and is used as the basis for the humanization of RbtVH.
5. Determine the framework and CDR arrangement (FR1, FR2, FR3, CDR1 & CDR2) for the human homolog being used for the heavy chain humanization. This is using the traditional layout as described in the field. Align the rabbit variable heavy chain sequence with the human homolog, while maintaining the layout of the framework and CDR regions.
   Example: In Fig. 2, the RbtVH sequence is aligned with the human homologous sequence 3-64-04, and the framework and CDR domains are indicated.
6. Replace the human homologous heavy chain sequence CDR1 and CDR2 regions with the CDR1 and CDR2 sequences from the rabbit sequence. If there are differences in length between the rabbit and human CDR sequences then use the entire rabbit CDR sequences and their lengths. In addition, it may be necessary to replace the final three amino acids of the human heavy chain Framework 1 region with the final three amino acids of the rabbit heavy chain Framework 1. Typically but not always, in rabbit heavy chain Framework 1 these three residues follow a Glycine residue preceded by a Serine residue. In addition, it may be necessary replace the final amino acid of the human heavy chain Framework 2 region with the final amino acid of the rabbit heavy chain Framework 2. Typically, but not necessarily always, this is a Glycine residue preceded by an Isoleucine residue in the rabbit heavy chain Framework 2. It is possible that the specificity, affinity and/or immunogenicity of the resulting humanized antibody may be unaltered if smaller or larger sequence exchanges are performed, or if specific residue(s) are altered, however the exchanges as described have been used successfully, but do not exclude the possibility that other changes may be permitted. For example, a tryptophan amino acid residue typically occurs four residues prior to the end of the rabbit heavy chain CDR2 region, whereas in human heavy chain CDR2 this residue is typically a Serine residue. Changing this rabbit tryptophan residue to a the human Serine residue at this position has been demonstrated to have minimal to no effect on the humanized antibody's specificity or affinity, and thus further minimizes the content of rabbit sequence-derived amino acid residues in the humanized sequence.
   Example: In Fig. 2, The CDR1 and CDR2 amino acid residues of the human homologous variable heavy chain are replaced with the CDR1 and CDR2 amino acid sequences from the RbtVH rabbit antibody light chain sequence, except for the boxed residue, which is tryptophan in the rabbit sequence (position number 63) and Serine at the same position in the human sequence, and is kept as the human Serine residue. In addition to the CDR1 and CDR2 changes, the final three amino acids of Framework 1 (positions 28-30) as well as the final residue of Framework 2 (position 49) are retained as rabbit amino acid residues instead of human. The resulting humanized sequence is shown below as VHh from residues numbered 1 through 98. Note that the only residues that are different from the 3-64-04 human sequence are underlined, and are thus rabbit-derived amino acid residues. In this example only 15 of the 98 residues are different than the human sequence.
7. After framework 3 of the new hybrid sequence created in Step 6, attach the entire CDR3 of the rabbit heavy chain antibody sequence. The CDR3 sequence can be of various lengths, but is typically 5 to 19 amino acid residues in length. The CDR3 region and the beginning of the following framework 4 region are defined classically and are identifiable by those skilled in the art. Typically the beginning of framework 4, and thus after the end of CDR3 consists of the sequence WGXG...(where X is usually Q or P), however some variation may exist in these residues.
   Example: The CDR3 of RbtVH (amino acid residues numbered 99-110) is added after the end of framework 3 in the humanized sequence indicated as VHh.
8. The rabbit heavy chain framework 4, which is typically the final 11 amino acid residues of the variable heavy chain and begins as indicated in Step 7 above and typically ends with the amino acid sequence '...TVSS' is replaced with the nearest human heavy chain framework 4 homolog, usually from germline sequence. Frequently this human heavy chain framework 4 is of the sequence 'WGQGTLVTVSS'. It is possible that other human heavy chain framework 4 sequences that are not the most homologous or otherwise different may be used without affecting the specificity, affinity and/or immunogenicity of the resulting humanized antibody. This human heavy chain framework 4 sequence is added to the end of the variable heavy chain humanized sequence immediately following the CDR3 sequence from Step 7 above. This is now the end of the variable heavy chain humanized amino acid sequence.

Example: In Fig. 2, framework 4 (FR4) of the RbtVH rabbit heavy chain sequence is shown above a homologous human heavy FR4 sequence. The human FR4 sequence is added to the humanized variable heavy chain sequence (VHh) right after the end of the CD3 region added in Step 7 above.

### Methods of Producing Antibodies and Fragments thereof

Also described herein is the production of the antibodies described herein or fragments thereof. Recombinant polypeptides corresponding to the antibodies described herein or fragments thereof are secreted from polyploidal, preferably diploid or tetraploid strains of mating competent yeast. Described herein are methods for producing these recombinant polypeptides in secreted form for prolonged periods using cultures comprising polyploid yeast, i.e., at least several days to a week, more preferably at least a month or several months, and even more preferably at least 6 months to a year or longer. These polyploid yeast cultures will express at least 10-25 mg/liter of the polypeptide, more preferably at least 50-250 mg/liter, still more preferably at least 500-1000 mg/liter, and most preferably a gram per liter or more of the recombinant polypeptide(s).

A pair of genetically marked yeast haploid cells may be transformed with expression vectors comprising subunits of a desired heteromultimeric protein. One haploid cell comprises a first expression vector, and a second haploid cell comprises a second expression vector. Diploid yeast cells may be be transformed with one or more expression vectors that provide for the expression and secretion of one or more of the recombinant polypeptides. A single haploid cell may be transformed with one or more vectors and used to produce a polyploidal yeast by fusion or mating strategies. A diploid yeast culture may be transformed with one or more vectors providing for the expression and secretion of a desired polypeptide or polypeptides. These vectors may comprise vectors e.g., linearized plasmids or other linear DNA products that integrate into the yeast cell's genome randomly, through homologous recombination, or using a recombinase such as Cre/Lox or Flp/Frt. Optionally, additional expression vectors may be introduced into the haploid or diploid cells; or the first or second expression vectors may comprise additional coding sequences; for the synthesis of heterotrimers; heterotetramers; etc. The expression levels of the non-identical polypeptides may be individually calibrated, and adjusted through appropriate selection, vector copy number, promoter strength and/or induction and the like. The transformed haploid cells are genetically crossed or fused. The resulting diploid or tetraploid strains are utilized to produce and secrete fully assembled and biologically functional proteins, humanized antibodies described herein or fragments thereof.

The use of diploid or tetraploid cells for protein production provides for unexpected benefits. The cells can be grown for production purposes, i.e. scaled up, and for extended periods of time, in conditions that can be deleterious to the growth of haploid cells, which conditions may include high cell density; growth in minimal media; growth at low temperatures; stable growth in the absence of selective pressure; and which may provide for maintenance of heterologous gene sequence integrity and maintenance of high level expression over time. Without wishing to be bound thereby, the inventors theorize that these benefits may arise, at least in part, from the creation of diploid strains from two distinct parental haploid strains. Such haploid strains can comprise numerous minor autotrophic mutations, which mutations are complemented in the diploid or tetraploid, enabling growth and enhanced production under highly selective conditions.

Transformed mating competent haploid yeast cells provide a genetic method that enables subunit pairing of a desired protein. Haploid yeast strains are transformed with each of two expression vectors, a first vector to direct the synthesis of one polypeptide chain and a second vector to direct the synthesis of a second, non-identical polypeptide chain. The two haploid strains are mated to provide a diploid host where optimized target protein production can be obtained.

Optionally, additional non-identical coding sequence(s) are provided. Such sequences may be present on additional expression vectors or in the first or the second expression vectors. As is known in the art, multiple coding sequences may be independently expressed from individual promoters; or may be coordinately expressed through the inclusion of an "internal ribosome entry site" or "IRES", which is an element that promotes direct internal ribosome entry to the initiation codon, such as ATG, of a cistron (a protein encoding region), thereby leading to the cap-independent translation of the gene. IRES elements functional in yeast are described by Thompson et al. (2001) P.N.A.S. 98:12866-12868.

Antibody sequences may be produced in combination with a secretory J chain, which provides for enhanced stability of IgA (see U.S. Patent Nos. 5,959,177; and 5,202,422).

The two haploid yeast strains may each be auxotrophic, and require supplementation of media for growth of the haploid cells. The pair of auxotrophs are complementary, such that the diploid product will grow in the absence of the supplements required for the haploid cells. Many such genetic markers are known in yeast, including requirements for amino acids (e.g. met, lys, his, arg, etc.), nucleosides (e.g. ura3, ade1, etc.); and the like. Amino acid markers may be preferred for the methods described herein. Alternatively diploid cells which contain the desired vectors can be selected by other means, e.g., by use of other markers, such as green fluorescent protein, antibiotic resistance genes, various dominant selectable markers, and the like.

Two transformed haploid cells may be genetically crossed and diploid strains arising from this mating event selected by their hybrid nutritional requirements and/or antibiotic resistance spectra. Alternatively, populations of the two transformed haploid strains are spheroplasted and fused, and diploid progeny regenerated and selected. By either method, diploid strains can be identified and selectively grown based on their ability to grow in different media than their parents. For example, the diploid cells may be grown in minimal medium that may include antibiotics. The diploid synthesis strategy has certain advantages. Diploid strains have the potential to produce enhanced levels of heterologous protein through broader complementation to underlying mutations, which may impact the production and/or secretion of recombinant protein. Furthermore, once stable strains have been obtained, any antibiotics used to select those strains do not necessarily need to be continuously present in the growth media.

As noted above, a haploid yeast may be transformed with a single or multiple vectors and mated or fused with a non-transformed cell to produce a diploid cell containing the vector or vectors. A diploid yeast cell may be transformed with one or more vectors that provide for the expression and secretion of a desired heterologous polypeptide by the diploid yeast cell.

Two haploid strains may be transformed with a library of polypeptides, e.g. a library of antibody heavy or light chains. Transformed haploid cells that synthesize the polypeptides are mated with the complementary haploid cells. The resulting diploid cells are screened for functional protein. The diploid cells provide a means of rapidly, conveniently and inexpensively bringing together a large number of combinations of polypeptides for functional testing. This technology is especially applicable for the generation of heterodimeric protein products, where optimized subunit synthesis levels are critical for functional protein expression and secretion.

The expression level ratio of the two subunits may be regulated in order to maximize product generation. Heterodimer subunit protein levels have been shown previously to impact the final product generation (Simmons LC, J Immunol Methods. 2002 May 1;263(1-2):133-47). Regulation can be achieved prior to the mating step by selection for a marker present on the expression vector. By stably increasing the copy number of the vector, the expression level can be increased. In some cases, it may be desirable to increase the level of one chain relative to the other, so as to reach a balanced proportion between the subunits of the polypeptide. Antibiotic resistance markers are useful for this purpose, e.g. Zeocin™ (phleomycin) resistance marker, G418 resistance, etc. and provide a means of enrichment for strains that contain multiple integrated copies of an expression vector in a strain by selecting for transformants that are resistant to higher levels of Zeocin™ (phleomycin) or G418. The proper ratio, e.g. 1:1; 1:2; etc. of the subunit genes may be important for efficient protein production. Even when the same promoter is used to transcribe both subunits, many other factors contribute to the final level of protein expressed and therefore, it can be useful to increase the number of copies of one encoded gene relative to the other. Alternatively, diploid strains that produce higher levels of a polypeptide, relative to single copy vector strains, are created by mating two haploid strains, both of which have multiple copies of the expression vectors.

Host cells are transformed with the above-described expression vectors, mated to form diploid strains, and cultured in conventional nutrient media modified as appropriate for inducing promoters, selecting transformants or amplifying the genes encoding the desired sequences. A number of minimal media suitable for the growth of yeast are known in the art. Any of these media may be supplemented as necessary with salts (such as sodium chloride, calcium, magnesium, and phosphate), buffers (such as phosphate, HEPES), nucleosides (such as adenosine and thymidine), antibiotics, trace elements, and glucose or an equivalent energy source. Any other necessary supplements may also be included at appropriate concentrations that would be known to those skilled in the art. The culture conditions, such as temperature, pH and the like, are those previously used with the host cell selected for expression, and will be apparent to the ordinarily skilled artisan.

Secreted proteins are recovered from the culture medium. A protease inhibitor, such as phenyl methyl sulfonyl fluoride (PMSF) may be useful to inhibit proteolytic degradation during purification, and antibiotics may be included to prevent the growth of adventitious contaminants. The composition may be concentrated, filtered, dialyzed, etc., using methods known in the art.

The diploid cells may be grown for production purposes. Such production purposes desirably include growth in minimal media, which media lacks pre-formed amino acids and other complex biomolecules, e.g., media comprising ammonia as a nitrogen source, and glucose as an energy and carbon source, and salts as a source of phosphate, calcium and the like. Preferably such production media lacks selective agents such as antibiotics, amino acids, purines, pyrimidines, etc. The diploid cells can be grown to high cell density, for example at least about 50 g/L; more usually at least about 100 g/L; and may be at least about 300, about 400, about 500 g/L or more.

The growth of the subject cells for production purposes may be performed at low temperatures, which temperatures may be lowered during log phase, during stationary phase, or both. The term "low temperature" refers to temperatures of at least about 15 °C, more usually at least about 17 °C, and may be about 20 °C, and is usually not more than about 25 °C, more usually not more than about 22 °C. In another embodiment of the invention, the low temperature is usually not more than about 28 °C. Growth temperature can impact the production of full-length secreted proteins in production cultures, and decreasing the culture growth temperature can strongly enhance the intact product yield. The decreased temperature appears to assist intracellular trafficking through the folding and post-translational processing pathways used by the host to generate the target product, along with reduction of cellular protease degradation.

The methods provide for expression of secreted, active protein, preferably a mammalian protein. Secreted, "active antibodies", as used herein, refers to a correctly folded multimer of at least two properly paired chains, which accurately binds to its cognate antigen. Expression levels of active protein are usually at least about 10-50 mg/liter culture, more usually at least about 100 mg/liter, preferably at least about 500 mg/liter, and may be 1000 mg/liter or more.

The methods decribed herein can provide for increased stability of the host and heterologous coding sequences during production. The stability is evidenced, for example, by maintenance of high levels of expression of time, where the starting level of expression is decreased by not more than about 20%, usually not more than 10%, and may be decreased by not more than about 5% over about 20 doublings, 50 doublings, 100 doublings, or more.

The strain stability also provides for maintenance of heterologous gene sequence integrity over time, where the sequence of the active coding sequence and requisite transcriptional regulatory elements are maintained in at least about 99% of the diploid cells, usually in at least about 99.9% of the diploid cells, and preferably in at least about 99.99% of the diploid cells over about 20 doublings, 50 doublings, 100 doublings, or more. Preferably, substantially all of the diploid cells maintain the sequence of the active coding sequence and requisite transcriptional regulatory elements.

Other methods of producing antibodies are well known to those of ordinary skill in the art. For example, methods of producing chimeric antibodies are now well known in the art (See, for example, U.S. Patent No. 4,816,567 to Cabilly et al.*;* Morrison et al., P.N.A.S. USA, 81:8651-55 (1984); Neuberger, M.S. et al., Nature, 314:268-270 (1985); Boulianne, G.L. et al., Nature, 312:643-46 (1984)).

Likewise, other methods of producing humanized antibodies are now well known in the art (See, for example, U.S. Patent Nos. 5,530,101, 5,585,089, 5,693,762, and 6,180,370 to Queen et al; U.S. Patent Nos. 5,225,539 and 6,548,640 to Winter; U.S. Patent Nos. 6,054,297, 6,407,213 and 6,639,055 to Carter et al; U.S. Patent No. 6,632,927 to Adair; Jones, P.T. et al, Nature, 321:522-525 (1986); Reichmann, L., et al, Nature, 332:323-327 (1988); Verhoeyen, M, et al, Science, 239:1534-36 (1988)).

Antibody polypeptides of the invention having IL-6 binding specificity may also be produced by constructing, using conventional techniques well known to those of ordinary skill in the art, an expression vector containing an operon and a DNA sequence encoding an antibody heavy chain in which the DNA sequence encoding the CDRs required for antibody specificity is derived from a non-human cell source, preferably a rabbit B-cell source, while the DNA sequence encoding the remaining parts of the antibody chain is derived from a human cell source.

A second expression vector is produced using the same conventional means well known to those of ordinary skill in the art, said expression vector containing an operon and a DNA sequence encoding an antibody light chain in which the DNA sequence encoding the CDRs required for antibody specificity is derived from a non-human cell source, preferably a rabbit B-cell source, while the DNA sequence encoding the remaining parts of the antibody chain is derived from a human cell source.

The expression vectors are transfected into a host cell by convention techniques well known to those of ordinary skill in the art to produce a transfected host cell, said transfected host cell cultured by conventional techniques well known to those of ordinary skill in the art to produce said antibody polypeptides.

The host cell may be co-transfected with the two expression vectors described above, the first expression vector containing DNA encoding an operon and a light chain-derived polypeptide and the second vector containing DNA encoding an operon and a heavy chain-derived polypeptide. The two vectors contain different selectable markers, but preferably achieve substantially equal expression of the heavy and light chain polypeptides. Alternatively, a single vector may be used, the vector including DNA encoding both the heavy and light chain polypeptides. The coding sequences for the heavy and light chains may comprise cDNA.

The host cells used to express the antibody polypeptides may be either a bacterial cell such as E. coli, or a eukaryotic cell. A mammalian cell of a well-defined type for this purpose, such as a myeloma cell or a Chinese hamster ovary (CHO) cell line may be used.

The general methods by which the vectors may be constructed, transfection methods required to produce the host cell and culturing methods required to produce the antibody polypeptides from said host cells all include conventional techniques. Although preferably the cell line used to produce the antibody is a mammalian cell line, any other suitable cell line, such as a bacterial cell line such as an E. coli-derived bacterial strain, or a yeast cell line, may alternatively be used.

Similarly, once produced the antibody polypeptides may be purified according to standard procedures in the art, such as for example cross-flow filtration, ammonium sulphate precipitation, affinity column chromatography and the like.

The antibody polypeptides described herein may also be used for the design and synthesis of either peptide or non-peptide mimetics that would be useful for the same therapeutic applications as the antibody polypeptides of the invention. See, for example, Saragobi et al, Science, 253:792-795 (1991).

### Examples of Heavy and Light Chain Polypeptides and Polynucleotides

This section recites examples of heavy and light chain polypeptides, as well as exemplary polynucleotides encoding such polypeptides. These exemplary polynucleotides are suitable for expression in the disclosed Pichia expression system.

Described herein are polynucleotides having at least 70%, such as at least 75%, at least 80%, at least 85%, at least 90%, at least 91%, at least 92%, at least 93%, at least 94%, at least 95%, at least 96%, at least 97%, at least 98%, at least 99%, or 100% identity to the polynucleotides recited in this application or that encode polypeptides recited in this application, or that hybridize to said polynucleotides under conditions of low-stringency, moderate-stringency, or high-stringency conditions, preferably those that encode polypeptides (e.g. an immunoglobulin heavy and light chain, a single-chain antibody, an antibody fragment, etc.) that have at least one of the biological activities set forth herein, including without limitation thereto specific binding to an IL-6 polypeptide. Described herein isa composition comprising such a polynucleotide and/or a polypeptide encoded by such a polynucleotide. Described herein isa method of treatment of a disease or condition associated with IL-6 or that may be prevented, treated, or ameliorated with an IL-6 antagonist such as Ab1 (e.g. cachexia, cancer fatigue, arthritis, etc.) comprising administration of a composition comprising such a polynucleotide and/or polypeptide.

A heavy chain polypeptide may comprise one or more of the CDR sequences of the heavy and/or light chain polypeptides recited herein (including those contained in the heavy and light chain polypeptides recited herein) and one or more of the framework region polypeptides recited herein, including those depicted in Figs. 2 and 34-37 or Table 1, and contained in the heavy and light chain polypeptide sequences recited herein. A heavy chain polypeptide may comprise one or more Framework 4 region sequences as depicted in Figs. 2 and 34-37 or Table 1, or as contained in a heavy or light chain polypeptide recited herein.

A light chain polypeptide may comprise one or more of the CDR sequences of the heavy and/or light chain polypeptides recited herein (including those contained in the heavy and light chain polypeptides recited herein) and one or more of the Framework region polypeptides recited herein, including those depicted in Figs. 2 and 34-37 or Table 1, and contained in the heavy and light chain polypeptide sequences recited herein. A light chain polypeptide may comprise one or more Framework 4 region sequences as depicted in Figs. 2 and 34-37 or Table 1, or as contained in a heavy or light chain polypeptide recited herein.

Certain of the sequences recited may be substituted for each other, unless the context indicates otherwise. The recitation that particular sequences may be substituted for one another, where such recitations are made, are understood to be illustrative rather than limiting, and it is also understood that such substitutions are encompassed even when no illustrative examples of substitutions are recited, For example, wherever one or more of the Ab1 light chain polypeptides is recited, e.g. any of SEQ ID NO: 2, 20, 647, 651, 660, 666, 699, 702, 706, or 709, another Ab1 light chain polypeptide may be substituted unless the context indicates otherwise. Similarly, wherever one of the Ab1 heavy chain polypeptides is recited, e.g. any of SEQ ID NO: 3, 18, 19, 652, 656, 657, 658, 661, 664, 665, 704, or 708, another Ab1 heavy chain polypeptide may be substituted unless the context indicates otherwise. Likewise, wherever one of the Ab1 light chain polynucleotides is recited, e.g. any of SEQ ID NO: 10, 662, 698, 701, or 705, another Ab1 light chain polynucleotide may be substituted unless the context indicates otherwise. Similarly, wherever one of the Ab1 heavy chain polynucleotides is recited, e.g. any of SEQ ID NO: 11, 663, 700, 703, or 707, another Ab1 heavy chain polynucleotide may be substituted unless the context indicates otherwise. Additionally, recitation of any member of any of the following groups is understood to encompass substitution by any other member of the group, as follows: Ab2 Light chain polypeptides (SEQ ID NO: 21 and 667); Ab2 Light chain polynucleotides (SEQ ID NO: 29 and 669); Ab2 Heavy chain polypeptides (SEQ ID NO: 22 and 668); Ab2 Heavy chain polynucleotides (SEQ ID NO: 30 and 670); Ab3 Light chain polypeptides (SEQ ID NO: 37 and 671); Ab3 Light chain polynucleotides (SEQ ID NO: 45 and 673); Ab3 Heavy chain polypeptides (SEQ ID NO: 38 and 672); Ab3 Heavy chain polynucleotides (SEQ ID NO: 46 and 674); Ab4 Light chain polypeptides (SEQ ID NO: 53 and 675); Ab4 Light chain polynucleotides (SEQ ID NO: 61 and 677); Ab4 Heavy chain polypeptides (SEQ ID NO: 54 and 676); Ab4 Heavy chain polynucleotides (SEQ ID NO: 62 and 678); Ab5 Light chain polypeptides (SEQ ID NO: 69 and 679); Ab5 Light chain polynucleotides (SEQ ID NO: 77 and 681); Ab5 Heavy chain polypeptides (SEQ ID NO: 70 and 680); Ab5 Heavy chain polynucleotides (SEQ ID NO: 78 and 682); Ab6 Light chain polypeptides (SEQ ID NO: 85 and 683); Ab6 Light chain polynucleotides (SEQ ID NO: 93 and 685); Ab6 Heavy chain polypeptides (SEQ ID NO: 86 and 684); Ab6 Heavy chain polynucleotides (SEQ ID NO: 94 and 686); Ab7 Light chain polypeptides (SEQ ID NO: 101, 119, 687, 693); Ab7 Light chain polynucleotides (SEQ ID NO: 109 and 689); Ab7 Heavy chain polypeptides (SEQ ID NO: 102, 117, 118, 688, 691, and 692); Ab7 Heavy chain polynucleotides (SEQ ID NO: 110 and 690); Ab1 Light Chain CDR1 polynucleotides (SEQ ID NO: 12 and 694); Ab1 Light Chain CDR3 polynucleotides (SEQ ID NO: 14 and 695); Ab1 Heavy Chain CDR2 polynucleotides (SEQ ID NO: 16 and 696) and Ab1 Heavy Chain CDR3 polynucleotides (SEQ ID NO: 17 and 697).

Exemplary Ab1-encoding polynucleotide sequences are recited as follows:

### Screening Assays

Also described herein are screening assays designed to assist in the identification of diseases and disorders associated with IL-6 in patients exhibiting symptoms of an IL-6 associated disease or disorder.

The anti-IL-6 antibodies described herein, or IL-6 binding fragments or variants thereof, may be used to detect the presence of IL-6 in a biological sample obtained from a patient exhibiting symptoms of a disease or disorder associated with IL-6. The presence of IL-6, or elevated levels thereof when compared to pre-disease levels of IL-6 in a comparable biological sample, may be beneficial in diagnosing a disease or disorder associated with IL-6.

Described herein isa diagnostic or screening assay to assist in diagnosis of diseases or disorders associated with IL-6 in patients exhibiting symptoms of an IL-6 associated disease or disorder identified herein, comprising assaying the level of IL-6 expression in a biological sample from said patient using a post-translationally modified anti-IL-6 antibody or binding fragment or variant thereof. The anti-IL-6 antibody or binding fragment or variant thereof may be post-translationally modified to include a detectable moiety such as set forth previously in the disclosure.

The IL-6 level in the biological sample is determined using a modified anti-IL-6 antibody or binding fragment or variant thereof as set forth herein, and comparing the level of IL-6 in the biological sample against a standard level of IL-6 (e.g., the level in normal biological samples). The skilled clinician would understand that some variability may exist between normal biological samples, and would take that into consideration when evaluating results.

The above-recited assay may also be useful in monitoring a disease or disorder, where the level of IL-6 obtained in a biological sample from a patient believed to have an IL-6 associated disease or disorder is compared with the level of IL-6 in prior biological samples from the same patient, in order to ascertain whether the IL-6 level in said patient has changed with, for example, a treatment regimen.

Described herein isa method of *in vivo* imaging which detects the presence of cells which express IL-6 comprising administering a diagnostically effective amount of a diagnostic composition. Said in vivo imaging is useful for the detection and imaging of IL-6 expressing tumors or metastases and IL-6 expressing inflammatory sites, for example, and can be used as part of a planning regimen for design of an effective cancer or arthritis treatment protocol. The treatment protocol may include, for example, one or more of radiation, chemotherapy, cytokine therapy, gene therapy, and antibody therapy, as well as an anti-IL-6 antibody or fragment or variant thereof.

A skilled clinician would understand that a biological sample includes, but is not limited to, sera, plasma, urine, saliva, mucous, pleural fluid, synovial fluid and spinal fluid.

### Methods of Ameliorating or Reducing Symptoms of, or Treating, or Preventing, Diseases and Disorders Associated with, IL-6

In accordance with the claims, IL-6 antibodies as defined in the claims are useful for ameliorating or reducing the symptoms of, or treating, or preventing, conditions associated with elevated IL-6. The conditions are defined in the claims. IL-6 antagonists described herein (e.g., Ab1) can also be administered in a therapeutically effective amount to patients in need of treatment of conditions associated with elevated IL-6 in the form of a pharmaceutical composition as described in greater detail below.

Described herein are IL-6 antagonists (e.g., Ab1) which are useful for ameliorating or reducing the symptoms of, or treating, or preventing, diseases and disorders associated with elevated C-reactive protein (CRP). Such diseases include any disease that exhibits chronic inflammation, e.g., rheumatoid arthritis, juvenile rheumatoid arthritis, psoriasis, psoriatic arthropathy, ankylosing spondylitis, systemic lupus erythematosis, Crohn's disease, ulcerative colitis, pemphigus, dermatomyositis, polymyositis, polymyalgia rheumatica, giant cell arteritis, vasculitis, polyarteritis nodosa, Wegener's granulomatosis, Kawasaki disease, isolated CNS vasculitis, Churg-Strauss arteritis, microscopic polyarteritis, microscopic polyangiitis, Henoch-Schonlein purpura, essential cryoglobulinemic vasculitis, rheumatoid vasculitis, cryoglobulinemia, relapsing polychondritis, Behcet's disease, Takayasu's arteritis, ischemic heart disease, stroke, multiple sclerosis, sepsis, vasculitis secondary to viral infection (e.g., hepatitis B, hepatitis C, HIV, cytomegalovirus, Epstein-Barr virus, Parvo B19 virus, etc.), Buerger's Disease, cancer, advanced cancer, Osteoarthritis, systemic sclerosis, CREST syndrome, Reiter's disease, Paget's disease of bone, Sjogran's syndrome, diabetes type 1, diabetes type 2, familial Mediterranean fever, autoimmune thrombocytopenia, autoimmune hemolytic anemia, autoimmune thyroid diseases, pernicious anemia, vitiligo, alopecia areata, primary biliary cirrhosis, autoimmune chronic active hepatitis, alcoholic cirrhosis, viral hepatitis including hepatitis B and C, other organ specific autoimmune diseases, burns, idiopathic pulmonary fibrosis, chronic obstructive pulmonary disease, allergic asthma, other allergic conditions or any combination thereof.

Described herein are IL-6 antagonists, such as anti-IL-6 antibodies (e.g., Ab1), variants thereof, or fragments thereof, are useful for ameliorating or reducing the symptoms of, or treating, or preventing, diseases and disorders associated with reduced serum albumin, e.g. rheumatoid arthritis, cancer, advanced cancer, liver disease, renal disease, inflammatory bowel disease, celiac's disease, trauma, burns, other diseases associated with reduced serum albumin, or any combination thereof.

Described herein are IL-6 antagonists which are administered to a patient in combination with another active agent. For example, an IL-6 antagonist such as Ab1 may be co-administered with one or more chemotherapy agents, such as VEGF antagonists, EGFR antagonists, platins, taxols, irinotecan, 5-fluorouracil, gemcytabine, leucovorine, steroids, cyclophosphamide, melphalan, vinca alkaloids (e.g., vinblastine, vincristine, vindesine and vinorelbine), mustines, tyrosine kinase inhibitors, radiotherapy, sex hormone antagonists, selective androgen receptor modulators, selective estrogen receptor modulators, PDGF antagonists, TNF antagonists, IL-1 antagonists, interleukins (e.g. IL-12 or IL-2), IL-12R antagonists, Toxin conjugated monoclonal antibodies, tumor antigen specific monoclonal antibodies, Erbitux™, Avastin™, Pertuzumab, anti-CD20 antibodies, Rituxan®, ocrelizumab, ofatumumab, DXL625, Herceptin®, or any combination thereof.

Described herein are anti-IL-6 antibodies, or fragments or variants thereof, which are useful for ameliorating or reducing the symptoms of, or treating, or preventing, diseases and disorders associated with fatigue. Diseases and disorders associated with fatigue include, but are not limited to, general fatigue, exercise-induced fatigue, cancer-related fatigue, fibromyalgia, inflammatory disease-related fatigue and chronic fatigue syndrome. *See,* for example, Esper DH, et al, The cancer cachexia syndrome: a review of metabolic and clinical manifestations, Nutr Clin Pract., 2005 Aug;20 (4):369-76; Vgontzas AN, et al, IL-6 and its circadian secretion in humans, Neuroimmunomodulation, 2005;12(3):131-40; Robson-Ansley, PJ, et al, Acute interleukin-6 administration impairs athletic performance in healthy, trained male runners, Can J Appl Physiol., 2004 Aug;29(4):411-8; Shephard RJ., Cytokine responses to physical activity, with particular reference to IL-6: sources, actions, and clinical implications, Crit Rev Immunol., 2002;22(3):165-82; Arnold, MC, et al, Using an interleukin-6 challenge to evaluate neuropsychological performance in chronic fatigue syndrome, Psychol Med., 2002 Aug;32(6): 1075-89; Kurzrock R., The role of cytokines in cancer-related fatigue, Cancer, 2001 Sep 15;92(6 Suppl):1684-8; Nishimoto N, et al, Improvement in Castleman's disease by humanized anti-interleukin-6 receptor antibody therapy, Blood, 2000 Jan 1; 95 (1):56-61; Vgontzas AN, et al, Circadian interleukin-6 secretion and quantity and depth of sleep, J Clin Endocrinol Metab., 1999 Aug;84(8):2603-7; and Spath-Schwalbe E, et al, Acute effects of recombinant human interleukin 6 on endocrine and central nervous sleep functions in healthy men, J Clin Endocrinol Metab., 1998 May;83(5):1573-9.

Described herein are anti-IL-6 antibodies, or fragments or variants thereof, which are useful for ameliorating or reducing the symptoms of, or treating, or preventing, cachexia. Diseases and disorders associated with cachexia include, but are not limited to, cancer-related cachexia, cardiac-related cachexia, respiratory-related cachexia, renal-related cachexia and age-related cachexia. *See,* for example, Barton, BE., Interleukin-6 and new strategies for the treatment of cancer, hyperproliferative diseases and paraneoplastic syndromes, Expert Opin Ther Targets, 2005 Aug;9(4):737-52; Zaki MH, et al, CNTO 328, a monoclonal antibody to IL-6, inhibits human tumor-induced cachexia in nude mice, Int J Cancer, 2004 Sep 10;111(4):592-5; Trikha M, et al, Targeted anti-interleukin-6 monoclonal antibody therapy for cancer: a review of the rationale and clinical evidence, Clin Cancer Res., 2003 Oct 15;9(13):4653-65; Lelli G, et al, Treatment of the cancer anorexia-cachexia syndrome: a critical reappraisal, J Chemother., 2003 Jun;15(3):220-5; Argiles JM, et al, Cytokines in the pathogenesis of cancer cachexia, Curr Opin Clin Nutr Metab Care, 2003 Jul;6(4):401-6; Barton BE., IL-6-like cytokines and cancer cachexia: consequences of chronic inflammation, Immunol Res., 2001;23(1):41-58; Yamashita JI, et al, Medroxyprogesterone acetate and cancer cachexia: interleukin-6 involvement, Breast Cancer, 2000;7(2): 130-5; Yeh SS, et al, Geriatric cachexia: the role of cytokines, Am J Clin Nutr., 1999 Aug;70(2):183-97; Strassmann G, et al, Inhibition of experimental cancer cachexia by anti-cytokine and anti-cytokine-receptor therapy, Cytokines Mol Ther., 1995 Jun;1(2):107-13; Fujita J, et al, Anti-interleukin-6 receptor antibody prevents muscle atrophy in colon-26 adenocarcinoma-bearing mice with modulation of lysosomal and ATP-ubiquitin-dependent proteolytic pathways, Int J Cancer, 1996 Nov 27;68(5):637-43; Tsujinaka T, et al, Interleukin 6 receptor antibody inhibits muscle atrophy and modulates proteolytic systems in interleukin 6 transgenic mice, J Clin Invest., 1996 Jan 1;97(1):244-9; Emilie D, et al, Administration of an anti-interleukin-6 monoclonal antibody to patients with acquired immunodeficiency syndrome and lymphoma: effect on lymphoma growth and on B clinical Symptoms, Blood, 1994 Oct 15;84 (8):2472-9; and Strassmann G, et al, Evidence for the involvement of interleukin 6 in experimental cancer cachexia, J Clin Invest., 1992 May;89(5):1681-4.

In another embodiment of the invention, anti-IL-6 antibodies defined in the claims, are useful for ameliorating or reducing the symptoms of, or treating, or preventing, autoimmune diseases and disorders. Diseases and disorders associated with autoimmunity include rheumatoid arthritis, systemic lupus erythematosus (SLE), systemic juvenile idiopathic arthritis, psoriasis, psoriatic arthropathy, ankylosing spondylitis, inflammatory bowel disease (IBD), polymyalgia rheumatica, giant cell arteritis, autoimmune vasculitis, and Sjogren's syndrome. In a preferred embodiment of the invention, humanized anti-IL-6 antibodies as defined in the claims, are useful for ameliorating or reducing the symptoms of, or treating, or preventing, rheumatoid arthritis and systemic juvenile idiopathic arthritis. *See,* for example, Nishimoto N., Clinical studies in patients with Castleman's disease, Crohn's disease, and rheumatoid arthritis in Japan, Clin Rev Allergy Immunol., 2005 Jun;28(3):221-30; Nishimoto N, et al, Treatment of rheumatoid arthritis with humanized anti-interleukin-6 receptor antibody: a multicenter, double-blind, placebo-controlled trial, Arthritis Rheum., 2004 Jun;50(6):1761-9; Choy E., Interleukin 6 receptor as a target for the treatment of rheumatoid arthritis, Ann Rheum Dis., 2003 Nov;62 Suppl 2:ii68-9; Nishimoto N, et al, Toxicity, pharmacokinetics, and dose-finding study of repetitive treatment with the humanized anti-interleukin 6 receptor antibody MRA in rheumatoid arthritis. Phase I/II clinical study, J Rheumatol., 2003 Jul;30(7): 1426-35; Mihara M, et al, Humanized antibody to human interleukin-6 receptor inhibits the development of collagen arthritis in cynomolgus monkeys, Clin Immunol., 2001 Mar;98(3):319-26; Nishimoto N, et al, Anti-interleukin 6 receptor antibody treatment in rheumatic disease, Ann Rheum Dis., 2000 Nov;59 Suppl 1:i21-7; Tackey E, et al, Rationale for interleukin-6 blockade in systemic lupus erythematosus, Lupus, 2004;13(5):339-43; Finck BK, et al, Interleukin 6 promotes murine lupus in NZB/NZW Fl mice, J Clin Invest., 1994 Aug;94 (2):585-91; Kitani A, et al, Autostimulatory effects of IL-6 on excessive B cell differentiation in patients with systemic lupus erythematosus: analysis of IL-6 production and IL-6R expression, Clin Exp Immunol., 1992 Apr;88(1):75-83; Stuart RA, et al, Elevated serum interleukin-6 levels associated with active disease in systemic connective tissue disorders, Clin Exp Rheumatol., 1995 Jan-Feb;13 (1): 17-22; Mihara M, et al, IL-6 receptor blockage inhibits the onset of autoimmune kidney disease in NZB/W Fl mice, Clin Exp Immunol., 1998 Jun;12(3):397-402; Woo P, et al, Open label phase II trial of single, ascending doses of MRA in Caucasian children with severe systemic juvenile idiopathic arthritis: proof of principle of the efficacy of IL-6 receptor blockade in this type of arthritis and demonstration of prolonged clinical improvement, Arthritis Res Ther., 2005;7(6):RI281-8. Epub 2005 Sep 15; Yokota S, et al, Clinical study of tocilizumab in children with systemic-onset juvenile idiopathic arthritis, Clin Rev Allergy Immunol., 2005 Jun;28(3):231-8; Yokota S, et al, Therapeutic efficacy of humanized recombinant anti-interleukin-6 receptor antibody in children with systemic-onset juvenile idiopathic arthritis, Arthritis Rheum., 2005 Mar;52(3):818-25; de Benedetti F, et al, Targeting the interleukin-6 receptor: a new treatment for systemic juvenile idiopathic arthritis?, Arthritis Rheum., 2005 Mar;52(3):687-93; De Benedetti F, et al, Is systemic juvenile rheumatoid arthritis an interleukin 6 mediated disease?, J Rheumatol., 1998 Feb;25(2):203-7; Ishihara K, et al, IL-6 in autoimmune disease and chronic inflammatory proliferative disease, Cytokine Growth Factor Rev., 2002 Aug-Oct;13 (4-5):357- 68; Gilhar A, et al, In vivo effects of cytokines on psoriatic skin grafted on nude mice :involvement of the tumor necrosis factor (TNF) receptor, Clin Exp Immunol., 1996 Oct;106(1): 134-42; Spadaro A, et al, Interleukin-6 and soluble interleukin-2 receptor in psoriatic arthritis: correlations with clinical and laboratory parameters, Clin Exp Rheumatol., 1996 Jul-Aug;14 (4):413-6; Ameglio F, et al, Interleukin-6 and tumor necrosis factor levels decrease in the suction blister fluids of psoriatic patients during effective therapy, Dermatology, 1994;189(4):359-63; Wendling D, et al, Combination therapy of anti-CD4 and anti-IL-6 monoclonal antibodies in a case of severe spondylarthropathy, Br J Rheumatol., 1996 Dec;35(12):1330; Gratacos J, et al, Serum cytokines (IL-6, TNF-alpha, IL-1 beta and IFN-gamma) in ankylosing spondylitis: a close correlation between serum IL-6 and disease activity and severity, Br J Rheumatol., 1994 Oct;33(10):927-31; Ito H., Treatment of Crohn's disease with anti-IL-6 receptor antibody, J Gastroenterol., 2005 Mar;40 Suppl 16:32-4; Ito H, et al, A pilot randomized trial of a human anti-interleukin-6 receptor monoclonal antibody in active Crohn's disease, Gastroenterology, 2004 Apr;126(4):989-96; discussion 947; Ito H., IL-6 and Crohn's disease, Curr Drug Targets Inflamm Allergy, 2003 Jun;2(2):12530; Ito H, et al, Anti-IL-6 receptor monoclonal antibody inhibits leukocyte recruitment and promotes T-cell apoptosis in a murine model of Crohn's disease, J Gastroenterol., 2002 Nov;37 Suppl 14:56-61; Ito H., Anti-interleukin-6 therapy for Crohn's disease, Curr Pharm Des., 2003;9(4):295-305; Salvarani C, et al, Acute-phase reactants and the risk of relapse/recurrence in polymyalgia rheumatica: a prospective follow-up study, Arthritis Rheum., 2005 Feb 15;53(1):33-8; Roche NE, et al, Correlation of interleukin-6 production and disease activity in polymyalgia rheumatica and giant cell arteritis, Arthritis Rheum., 1993 Sep;36(9): 1286-94; Gupta M, et al, Cytokine modulation with immune gamma-globulin in peripheral blood of normal children and its implications in Kawasaki disease treatment, J Clin Immunol., 2001 May;21(3):193-9; Noris M, et al, Interleukin-6 and RANTES in Takayasu arteritis: a guide for therapeutic decisions?, Circulation, 1999 Jul 6;100(1):55-60; Besbas N, et al, The role of cytokines in Henoch Schonlein purpura, Scand J Rheumatol., 1997;26(6):456-60; Hirohata S, et al, Cerebrospinal fluid interleukin-6 in progressive Neuro-Behcet's syndrome, Clin Immunol Immunopathol., 1997 Jan;82(1):12-7; Yamakawa Y, et al, Interleukin-6 (IL-6) in patients with Behcet's disease, J Dermatol Sci., 1996 Mar;11(3):189-95; Kim DS., Serum interleukin-6 in Kawasaki disease, Yonsei Med J., 1992 Jun;33(2):183-8; Lange, A., et al, Cytokines, adhesion molecules (E-selectin and VCAM-1) and graft-versus-host disease, Arch. Immunol Ther Exp., 1995, 43(2):99-105; Tanaka, J., et al, Cytokine gene expression after allogeneic bone marrow transplantation, Leuk. Lymphoma, 1995 16(5-6):413-418; Dickenson, AM, et al, Predicting outcome in hematological stem cell transplantation, Arch Immunol Ther Exp., 2002 50(6):371-8; Zeiser, R, et al, Immunopathogenesis of acute graft-versus-host disease: implications for novel preventive and therapeutic strategies, Ann Hematol., 2004 83(9):551-65; Dickinson, AM, et al, Genetic polymorphisms predicting the outcome of bone marrow transplants, Br. J Haematol., 2004 127(5):479-90; and Scheinberg MA, et al, Interleukin 6: a possible marker of disease activity in adult onset Still's disease, Clin Exp Rheumatol., 1996 Nov-Dec;14 (6):653-5.

In another embodiment of the invention, anti-IL-6 antibodies as defined in the claims, are useful for ameliorating or reducing the symptoms of, or treating, or preventing, diseases and disorders associated with the skeletal system. Diseases and disorders associated with the skeletal system include osteoarthritis, and Paget's disease of bone. In a preferred embodiment of the invention, humanized anti-IL-6 antibodies as defined in the claims, are useful for ameliorating or reducing the symptoms of, or treating, or preventing, osteoarthritis. *See,* for example, Malemud CJ., Cytokines as therapeutic targets for osteoarthritis, BioDrugs, 2004;18(1):23-35; Westacott CI, et al, Cytokines in osteoarthritis: mediators or markers of joint destruction?, Semin Arthritis Rheum., 1996 Feb;25(4):254-72; Sugiyama T., Involvement of interleukin-6 and prostaglandin E2 in particular osteoporosis of postmenopausal women with rheumatoid arthritis, J Bone Miner Metab., 2001;19(2):89-96; Abrahamsen B, et al, Cytokines and bone loss in a 5-year longitudinal study - hormone replacement therapy suppresses serum soluble interleukin-6 receptor and increases interleukin-1-receptor antagonist: the Danish Osteoporosis Prevention Study, J Bone Miner Res., 2000 Aug;15(8): 1545-54; Straub RH, et al, Hormone replacement therapy and interrelation between serum interleukin-6 and body mass index in postmenopausal women: a population-based study, J Clin Endocrinol Metab., 2000 Mar;85(3): 1340-4; Manolagas SC, The role of IL-6 type cytokines and their receptors in bone, Ann N Y Acad Sci., 1998 May 1;840:194-204; Ershler WB, et al, Immunologic aspects of osteoporosis, Dev Comp Immunol., 1997 Nov-Dec;21(6):487-99; Jilka RL, et al, Increased osteoclast development after estrogen loss: mediation by interleukin-6, Science, 1992 Jul 3;257(5066):88-91; Kallen KJ, et al, New developments in IL-6 dependent biology and therapy: where do we stand and what are the options?, Expert Opin Investig Drugs, 1999 Sep;8(9): 1327-49; Neale SD, et al, The influence of serum cytokines and growth factors on osteoclast formation in Paget's disease, QJM, 2002 Apr;95 (4):233 - 40; Roodman GD, Osteoclast function In Paget's disease and multiple myeloma, Bone, 1995 Aug;17(2 Suppl):57S-61S; Hoyland JA, et al, Interleukin-6, IL-6 receptor, and IL-6 nuclear factor gene expression in Paget's disease, J Bone Miner Res., 1994 Jan;9(1):75-80; and Roodman GD, et al, Interleukin 6. A potential autocrine/paracrine factor in Paget's disease of bone, J Clin Invest., 1992 Jan;89(1):46-52;.

In another embodiment of the invention, anti-IL-6 antibodies as defined the claims, are useful for ameliorating or reducing the symptoms of, or treating, or preventing, diseases and disorders associated with cancer. Diseases and disorders associated with cancer include, but are not limited to, Acanthoma, Acinic cell carcinoma, Acoustic neuroma, Acral lentiginous melanoma, Acrospiroma, Acute eosinophilic leukemia, Acute lymphoblastic leukemia, Acute megakaryoblastic leukemia, Acute monocytic leukemia, Acute myeloblastic leukemia with maturation, Acute myeloid dendritic cell leukemia, Acute myeloid leukemia, Acute promyelocytic leukemia, Adamantinoma, Adenocarcinoma, Adenoid cystic carcinoma, Adenoma, Adenomatoid odontogenic tumor, Adrenocortical carcinoma, Adult T-cell leukemia, Aggressive NK-cell leukemia, AIDS-Related Cancers, AIDS-related lymphoma, Alveolar soft part sarcoma, Ameloblastic fibroma, Anal cancer, Anaplastic large cell lymphoma, Anaplastic thyroid cancer, Angioimmunoblastic T-cell lymphoma, Angiomyolipoma, Angiosarcoma, Appendix cancer, Astrocytoma, Atypical teratoid rhabdoid tumor, Basal cell carcinoma, Basal-like carcinoma, B-cell leukemia, B-cell lymphoma, Bellini duct carcinoma, Biliary tract cancer, Bladder cancer, Blastoma, Bone Cancer, Bone tumor, Brain Stem Glioma, Brain Tumor, Breast Cancer, Brenner tumor, Bronchial Tumor, Bronchioloalveolar carcinoma, Brown tumor, Burkitt's lymphoma, Cancer of Unknown Primary Site, Carcinoid Tumor, Carcinoma, Carcinoma in situ, Carcinoma of the penis, Carcinoma of Unknown Primary Site, Carcinosarcoma, Castleman's Disease, Central Nervous System Embryonal Tumor, Cerebellar Astrocytoma, Cerebral Astrocytoma, Cervical Cancer, Cholangiocarcinoma, Chondroma, Chondrosarcoma, Chordoma, Choriocarcinoma, Choroid plexus papilloma, Chronic Lymphocytic Leukemia, Chronic monocytic leukemia, Chronic myelogenous leukemia, Chronic Myeloproliferative Disorder, Chronic neutrophilic leukemia, Clear-cell tumor, Colon Cancer, Colorectal cancer, Craniopharyngioma, Cutaneous T-cell lymphoma, Degos disease, Dermatofibrosarcoma protuberans, Dermoid cyst, Desmoplastic small round cell tumor, Diffuse large B cell lymphoma, Dysembryoplastic neuroepithelial tumor, Embryonal carcinoma, Endodermal sinus tumor, Endometrial cancer, Endometrial Uterine Cancer, Endometrioid tumor, Enteropathy-associated T-cell lymphoma, Ependymoblastoma, Ependymoma, Epithelioid sarcoma, Erythroleukemia, Esophageal cancer, Esthesioneuroblastoma, Ewing Family of Tumor, Ewing Family Sarcoma, Ewing's sarcoma, Extracranial Germ Cell Tumor, Extragonadal Germ Cell Tumor, Extrahepatic Bile Duct Cancer, Extramammary Paget's disease, Fallopian tube cancer, Fetus in fetu, Fibroma, Fibrosarcoma, Follicular lymphoma, Follicular thyroid cancer, Gallbladder Cancer, Gallbladder cancer, Ganglioglioma, Ganglioneuroma, Gastric Cancer, Gastric lymphoma, Gastrointestinal cancer, Gastrointestinal Carcinoid Tumor, Gastrointestinal Stromal Tumor, Gastrointestinal stromal tumor, Germ cell tumor, Germinoma, Gestational choriocarcinoma, Gestational Trophoblastic Tumor, Giant cell tumor of bone, Glioblastoma multiforme, Glioma, Gliomatosis cerebri, Glomus tumor, Glucagonoma, Gonadoblastoma, Granulosa cell tumor, Hairy Cell Leukemia, Hairy cell leukemia, Head and Neck Cancer, Head and neck cancer, Heart cancer, Hemangioblastoma, Hemangiopericytoma, Hemangiosarcoma, Hematological malignancy, Hepatocellular carcinoma, Hepatosplenic T-cell lymphoma, Hereditary breast-ovarian cancer syndrome, Hodgkin Lymphoma, Hodgkin's lymphoma, Hypopharyngeal Cancer, Hypothalamic Glioma, Inflammatory breast cancer, Intraocular Melanoma, Islet cell carcinoma, Islet Cell Tumor, Juvenile myelomonocytic leukemia, Kaposi Sarcoma, Kaposi's sarcoma, Kidney Cancer, Klatskin tumor, Krukenberg tumor, Laryngeal Cancer, Laryngeal cancer, Lentigo maligna melanoma, Leukemia, Leukemia, Lip and Oral Cavity Cancer, Liposarcoma, Lung cancer, Luteoma, Lymphangioma, Lymphangiosarcoma, Lymphoepithelioma, Lymphoid leukemia, Lymphoma, Macroglobulinemia, Malignant Fibrous Histiocytoma, Malignant fibrous histiocytoma, Malignant Fibrous Histiocytoma of Bone, Malignant Glioma, Malignant Mesothelioma, Malignant peripheral nerve sheath tumor, Malignant rhabdoid tumor, Malignant triton tumor, MALT lymphoma, Mantle cell lymphoma, Mast cell leukemia, Mediastinal germ cell tumor, Mediastinal tumor, Medullary thyroid cancer, Medulloblastoma, Medulloblastoma, Medulloepithelioma, Melanoma, Melanoma, Meningioma, Merkel Cell Carcinoma, Mesothelioma, Mesothelioma, Metastatic Squamous Neck Cancer with Occult Primary, Metastatic urothelial carcinoma, Mixed Müllerian tumor, Monocytic leukemia, Mouth Cancer, Mucinous tumor, Multicentric Castleman's disease, Multiple Endocrine Neoplasia Syndrome, Multiple Myeloma, Multiple myeloma, Mycosis Fungoides, Mycosis fungoides, Myelodysplastic Disease, Myelodysplastic Syndromes, Myeloid leukemia, Myeloid sarcoma, Myeloproliferative Disease, Myxoma, Nasal Cavity Cancer, Nasopharyngeal Cancer, Nasopharyngeal carcinoma, Neoplasm, Neurinoma, Neuroblastoma, Neuroblastoma, Neurofibroma, Neuroma, Nodular melanoma, Non-Hodgkin Lymphoma, Non-Hodgkin lymphoma, Nonmelanoma Skin Cancer, Non-Small Cell Lung Cancer, Ocular oncology, Oligoastrocytoma, Oligodendroglioma, Oncocytoma, Optic nerve sheath meningioma, Oral Cancer, Oral cancer, Oropharyngeal Cancer, Osteosarcoma, Osteosarcoma, Ovarian Cancer, Ovarian cancer, Ovarian Epithelial Cancer, Ovarian Germ Cell Tumor, Ovarian Low Malignant Potential Tumor, Paget's disease of the breast, Pancoast tumor, Pancreatic Cancer, Pancreatic cancer, Papillary thyroid cancer, Papillomatosis, Paraganglioma, Paranasal Sinus Cancer, Parathyroid Cancer, Penile Cancer, Perivascular epithelioid cell tumor, Pharyngeal Cancer, Pheochromocytoma, Pineal Parenchymal Tumor of Intermediate Differentiation, Pineoblastoma, Pituicytoma, Pituitary adenoma, Pituitary tumor, Plasma Cell Neoplasm, Pleuropulmonary blastoma, Polyembryoma, Precursor T-lymphoblastic lymphoma, Primary central nervous system lymphoma, Primary effusion lymphoma, Primary Hepatocellular Cancer, Primary Liver Cancer, Primary peritoneal cancer, Primitive neuroectodermal tumor, Prostate cancer, Pseudomyxoma peritonei, Rectal Cancer, Renal cell carcinoma, Respiratory Tract Carcinoma Involving the NUT Gene on Chromosome 15, Retinoblastoma, Rhabdomyoma, Rhabdomyosarcoma, Richter's transformation, Sacrococcygeal teratoma, Salivary Gland Cancer, Sarcoma, Schwannomatosis, Sebaceous gland carcinoma, Secondary neoplasm, Seminoma, Serous tumor, Sertoli-Leydig cell tumor, Sex cord-stromal tumor, Sézary Syndrome, Signet ring cell carcinoma, Skin Cancer, Small blue round cell tumor, Small cell carcinoma, Small Cell Lung Cancer, Small cell lymphoma, Small intestine cancer, Soft tissue sarcoma, Somatostatinoma, Soot wart, Spinal Cord Tumor, Spinal tumor, Splenic marginal zone lymphoma, Squamous cell carcinoma, Stomach cancer, Superficial spreading melanoma, Supratentorial Primitive Neuroectodermal Tumor, Surface epithelial-stromal tumor, Synovial sarcoma, T-cell acute lymphoblastic leukemia, T-cell large granular lymphocyte leukemia, T-cell leukemia, T-cell lymphoma, T-cell prolymphocytic leukemia, Teratoma, Terminal lymphatic cancer, Testicular cancer, Thecoma, Throat Cancer, Thymic Carcinoma, Thymoma, Thyroid cancer, Transitional Cell Cancer of Renal Pelvis and Ureter, Transitional cell carcinoma, Urachal cancer, Urethral cancer, Urogenital neoplasm, Uterine sarcoma, Uveal melanoma, Vaginal Cancer, Verner Morrison syndrome, Verrucous carcinoma, Visual Pathway Glioma, Vulvar Cancer, Waldenström's macroglobulinemia, Warthin's tumor, Wilms' tumor, or any combination thereof, as well as drug resistance in cancer chemotherapy and cancer chemotherapy toxicity. *See,* for example, Hirata T, et al, Humanized anti-interleukin-6 receptor monoclonal antibody induced apoptosis of fresh and cloned human myeloma cells in vitro, Leuk Res., 2003 Apr;27(4):343-9, Bataille R, et al, Biologic effects of anti-interleukin-6 murine monoclonal antibody in advanced multiple myeloma, Blood, 1995 Jul 15;86 (2):685-91; Goto H, et al, Mouse anti-human interleukin-6 receptor monoclonal antibody inhibits proliferation of fresh human myeloma cells in vitro, Jpn J Cancer Res., 1994 Sep;85(9):958-65; Klein B, et al, Murine anti-interleukin-6 monoclonal antibody therapy for a patient with plasma cell leukemia, Blood, 1991 Sep 1;78(5):1198-204; Mauray S, et al, Epstein-Barr virus-dependent lymphoproliferative disease: critical role of IL-6, Eur J Immunol., 2000 Jul;30(7):2065-73; Tsunenari T, et al, New xenograft model of multiple myeloma and efficacy of a humanized antibody against human interleukin-6 receptor, Blood, 1997 Sep 15;90(6):2437-44; Emilie D, et al, Interleukin-6 production in high-grade B lymphomas: correlation with the presence of malignant immunoblasts in acquired immunodeficiency syndrome and in human immunodeficiency virus-seronegative patients, Blood, 1992 Jul 15;80(2):498-504; Emilie D, et al, Administration of an anti-interleukin-6 monoclonal antibody to patients with acquired immunodeficiency syndrome and lymphoma: effect on lymphoma growth and on B clinical Symptoms, Blood, 1994 Oct 15; 84(8):2472-9; Smith PC, et al, Anti-interleukin-6 monoclonal antibody induces regression of human prostate cancer xenografts in nude mice, Prostate, 2001 Jun 15;48(1):47-53; Smith PC, et al, Interleukin-6 and prostate cancer progression, Cytokine Growth Factor Rev., 2001 Mar;12(l):33-40; Chung TD, et al, Characterization of the role of IL-6 in the progression of prostate cancer, Prostate, 1999 Feb 15;38(3):199-207; Okamoto M, et al, Interleukin-6 as a paracrine and autocrine growth factor in human prostatic carcinoma cells in vitro, Cancer Res., 1997 Jan 1;57(1):141-6; Reittie JE, et al, Interleukin-6 inhibits apoptosis and tumor necrosis factor induced proliferation of B-chronic lymphocytic leukemia, Leuk Lymphoma, 1996 Jun;22(1-2):83-90, follow 186, color plate VI; Sugiyama H, et al, The expression of IL-6 and its related genes in acute leukemia, Leuk Lymphoma, 1996 Mar;21(1-2):49-52; Bataille R, et al, Effects of an anti-interleukin-6 (IL-6) murine monoclonal antibody in a patient with acute monoblastic leukemia, Med Oncol Tumor Pharmacother., 1993;10(4):185-8; Kedar I, et al, Thalidomide reduces serum C-reactive protein and interleukin-6 and induces response to IL-2 in a fraction of metastatic renal cell cancer patients who failed IL-2-based therapy, Int J Cancer, 2004 Jun 10;110(2):260-5; Angelo LS, Talpaz M, Kurzrock R, Autocrine interleukin-6 production in renal cell carcinoma: evidence for the involvement of p53, Cancer Res., 2002 Feb 1;62(3):932-40; Nishimoto N, Humanized anti-interleukin-6 receptor antibody treatment of multicentric Castleman disease, Blood, 2005 Oct 15;106(8):2627-32, Epub 2005 Jul 5; Katsume A, et al, Anti-interleukin 6 (IL-6) receptor antibody suppresses Castleman's disease like symptoms emerged in IL-6 transgenic mice, Cytokine, 2002 Dec 21;20(6):304-11; Nishimoto N, et al, Improvement in Castleman's disease by humanized anti-interleukin-6 receptor antibody therapy, Blood, 2000 Jan 1;95(1):56-61; Screpanti I, Inactivation of the IL-6 gene prevents development of multicentric Castleman's disease in C/EBP beta-deficient mice, J Exp Med., 1996 Oct 1;184(4):1561-6; Hsu SM, et al, Expression of interleukin-6 in Castleman's disease, Hum Pathol., 1993 Aug;24(8):833-9; Yoshizaki K, et al, Pathogenic significance of interleukin-6 (IL 6/BSF-2) in Castleman's disease, Blood, 1989 Sep;74(4):1360-7; Nilsson MB, et al, Interleukin-6, secreted by human ovarian carcinoma cells, is a potent proangiogenic cytokine, Cancer Res., 2005 Dec 1;65(23): 10794-800; Toutirais O, et al, Constitutive expression of TGF-betal, interleukin-6 and interleukin-8 by tumor cells as a major component of immune escape in human ovarian carcinoma, Eur Cytokine Netw., 2003 Oct-Dec;14(4):246-55; Obata NH, et al, Effects of interleukin 6 on in vitro cell attachment, migration and invasion of human ovarian carcinoma, Anticancer Res., 1997 Jan-Feb;17 (1A):337-42; Dedoussis GV, et al, Endogenous interleukin 6 conveys resistance to cis-diamminedichloroplatinum-mediated apoptosis of the K562 human leukemic cell line, Exp Cell Res., 1999 Jun 15;249(2):269-78; Borsellino N, et al, Blocking signaling through the Gp130 receptor chain by interleukin-6 and oncostatin M inhibits PC-3 cell growth and sensitizes the tumor cells to etoposide and cisplatin-mediated cytotoxicity, Cancer, 1999 Jan 1;85(1):134-44; Borsellino N, et al, Endogenous interleukin 6 is a resistance factor for cis-diamminedichloroplatinum and etoposide-mediated cytotoxicity of human prostate carcinoma cell lines, Cancer Res., 1995 Oct 15;55(20):4633-9; Mizutani Y, et al, Sensitization of human renal cell carcinoma cells to cis-diamminedichloroplatinum(II) by anti-interleukin 6 monoclonal antibody or anti-interleukin 6 receptor monoclonal antibody; Cancer Res., 1995 Feb 1;55(3):590-6; Yusuf RZ, et al, Paclitaxel resistance: molecular mechanisms and pharmacologic manipulation, Curr Cancer Drug Targets, 2003 Feb;3(1):1-19; Duan Z, et al, Overexpression of IL-6 but not IL-8 increases paclitaxel resistance of U-20S human osteosarcoma cells, Cytokine, 2002 Mar 7;17(5):234-42; Conze D, et al, Autocrine production of interleukin 6 causes multidrug resistance in breast cancer cells, Cancer Res., 2001 Dec 15;61(24):8851-8; Rossi JF, et al, Optimizing the use of anti-interleukin-6 monoclonal antibody with dexamethasone and 140 mg/m2 of melphalan in multiple myeloma: results of a pilot study including biological aspects, Bone Marrow Transplant, 2005 Nov;36(9):771-9; and Tonini G, et al, Oxaliplatin may induce cytokine-release syndrome in colorectal cancer patients, J Biol Regul Homeost Agents, 2002 Apr-Jun;16 (2):105-9.

Described herein are anti-IL-6 antibodies, or fragments or variants thereof, which are useful for ameliorating or reducing the symptoms of, or treating, or preventing, ischemic heart disease, atherosclerosis, obesity, diabetes, asthma, multiple sclerosis, Alzheimer's disease, cerebrovascular disease, fever, acute phase response, allergies, anemia, anemia of inflammation (anemia of chronic disease), hypertension, depression, depression associated with a chronic illness, thrombosis, thrombocytosis, acute heart failure, metabolic syndrome, miscarriage, obesity, chronic prostatitis, glomerulonephritis, pelvic inflammatory disease, reperfusion injury, and transplant rejection. *See,* for example, Tzoulaki I, et al, C-reactive protein, interleukin-6, and soluble adhesion molecules as predictors of progressive peripheral atherosclerosis in the general population: Edinburgh Artery Study, Circulation, 2005 Aug 16;112(7):976-83, Epub 2005 Aug 8; Rattazzi M, et al, C-reactive protein and interleukin-6 in vascular disease: culprits or passive bystanders?, J Hypertens., 2003 Oct;21(10):1787-803; Ito T, et al, HMG-CoA reductase inhibitors reduce interleukin-6 synthesis in human vascular smooth muscle cells, Cardiovasc Drugs Ther., 2002 Mar;16(2):121-6; Stenvinkel P, et al, Mortality, malnutrition, and atherosclerosis in ESRD: what is the role of interleukin-6?, Kidney Int Suppl., 2002 May;(80): 103-8; Yudkin JS, et al, Inflammation, obesity, stress and coronary heart disease: is interleukin-6 the link?, Atherosclerosis, 2000 Feb;148(2):209-14; Huber SA, et al, Interleukin-6 exacerbates early atherosclerosis in mice, Arterioscler Thromb Vasc Biol., 1999 Oct;19(10):2364-7; Kado S, et al, Circulating levels of interleukin-6, its soluble receptor and interleukin-6/interleukin-6 receptor complexes in patients with type 2 diabetes mellitus, Acta Diabetol.,1999 Jun;36(1-2):67-72; Sukovich DA, et al, Expression of interleukin-6 in atherosclerotic lesions of male ApoE-knockout mice: inhibition by 17beta-estradiol, Arterioscler Thromb Vasc Biol.,1998 Sept;8(9):1498-505; Klover PJ, et al, Interleukin-6 depletion selectively improves hepatic insulin action in obesity, Endocrinology, 2005 Aug;146(8):3417-27, Epub 2005 Apr 21; Lee YH, et al, The evolving role of inflammation in obesity and the metabolic syndrome, Curr Diab Rep., 2005 Feb;5(1):70-5; Diamant M, et al, The association between abdominal visceral fat and carotid stiffness is mediated by circulating inflammatory markers in uncomplicated type 2 diabetes, J Clin Endocrinol Metab., 2005 Mar;90(3):1495-501, Epub 2004 Dec 21; Bray GA, Medical consequences of obesity, J Clin Endocrinol Metab., 2004 Jun;89(6):2583 9; Klover PJ, et al, Chronic exposure to interleukin-6 causes hepatic insulin resistance in mice, Diabetes, 2003 Nov;52 (11):2784-9; Yudkin JS, et al, Inflammation, obesity, stress and coronary heart disease: is interleukin-6 the link?, Atherosclerosis, 2000 Feb;148(2):209-14; Doganci A, et al, Pathological role of IL-6 in the experimental allergic bronchial asthma in mice, Clin Rev Allergy Immunol., 2005 Jun;28(3):257-70; Doganci A, et al, The IL-6R alpha chain controls lung CD4+CD25+ Treg development and function during allergic airway inflammation in vivo, J Clin Invest., 2005 Feb;115(2):313 25, (Erratum in: J Clin Invest., 2005 May;115(5):1388, Lehr, Hans A [added]); Stelmasiak Z, et al, IL 6 and sIL-6R concentration in the cerebrospinal fluid and serum of MS patients, Med Sci Monit., 2001 Sep-Oct;7(5):914-8; Tilgner J, et al, Continuous interleukin-6 application in vivo via macroencapsulation of interleukin-6-expressing COS-7 cells induces massive gliosis, Glia, 2001 Sep;35(3):234-45, Brunello AG, et al, Astrocytic alterations in interleukin-6 Soluble interleukin-6 receptor alpha double-transgenic mice, Am J Pathol., 2000 Nov;157(5):1485-93; Hampel H, et al, Pattern of interleukin-6 receptor complex immunoreactivity between cortical regions of rapid autopsy normal and Alzheimer's disease brain, Eur Arch Psychiatry Clin Neurosci., 2005 Aug;255(4):269-78, Epub 2004 Nov 26; Cacquevel M, et al, Cytokines in neuroinflammation and Alzheimer's disease, Curr Drug Targets, 2004 Aug;5(6):529-34; Quintanilla RA, et al, Interleukin 6 induces Alzheimer-type phosphorylation of tau protein by deregulating the cdk5/p35 pathway, Exp Cell Res., 2004 Apr 15; 295 (1):245-57; Gadient RA, et al, Interleukin-6 (IL-6)--a molecule with both beneficial and destructive potentials, Prog Neurobiol., 1997 Aug;52(5):379-90; Hull M, et al, Occurrence of interleukin-6 in cortical plaques of Alzheimer's disease patients may precede transformation of diffuse into neuritic plaques, Ann N Y Acad Sci., 1996 Jan 17;777:205-12; Rallidis LS, et al, Inflammatory markers and in-hospital mortality in acute ischaemic stroke, Atherosclerosis, 2005 Dec 30; Emsley HC, et al, Interleukin-6 and acute ischaemic stroke, Acta Neurol Scand., 2005 Oct;112(4):273-4; Smith CJ, et al, Peak plasma interleukin-6 and other peripheral markers of inflammation in the first week of ischaemic stroke correlate with brain infarct volume, stroke severity and long-term outcome, BMC Neurol., 2004 Jan 15;4:2; Vila N, et al, Proinflammatory cytokines and early neurological worsening in ischemic stroke, Stroke, 2000 Oct;31(10):2325-9; and Tarkowski E, et al, Early intrathecal production of interleukin-6 predicts the size of brain lesion in stroke, Stroke, 1995 Aug;26(8): 1393-8.

Described herein are anti-IL-6 antibodies, or fragments or variants thereof, which are useful for ameliorating or reducing the symptoms of, or treating, or preventing, diseases and disorders associated with cytokine storm. Diseases and disorders associated with cytokine storm include, but are not limited to, graft versus host disease (GVHD), avian influenza, smallpox, pandemic influenza, adult respiratory distress syndrome (ARDS), severe acute respiratory syndrome (SARS), sepsis, and systemic inflammatory response syndrome (SIRS). *See,* for example, Cecil, R. L., Goldman, L., & Bennett, J. C. (2000). Cecil textbook of medicine. Philadelphia: W.B. Saunders; Ferrara JL, et al., Cytokine storm of graft-versus-host disease: a critical effector role for interleukin-1, Transplant Proc. 1993 Feb;25(1 Pt 2):1216-7; Osterholm MT, Preparing for the Next Pandemic, N Engl J Med. 2005 May 5;352(18):1839-42; Huang KJ, et al., An interferon-gamma-related cytokine storm in SARS patients, J Med Virol. 2005 Feb;75(2):185-94; and Cheung CY, et al., Induction of proinflammatory cytokines in human macrophages by influenza A (H5N1) viruses: a mechanism for the unusual severity of human disease? Lancet. 2002 Dec 7;360(9348):1831-7.

Described herein are anti-IL-6 antibodies, or fragments or variants thereof, which are useful as a wakefulness aid.

### Administration

The invention provides compositions comprising anti-IL-6 antibodies as defined in the claims for use in a method of preventing or treating a condition in a human subject associated with elevated IL-6, where the conditions are defined in the claims. In one embodiment of the invention, the anti-IL-6 antibodies are administered to a subject at a concentration of between about 0.1 and 20 mg/kg, such as about 0.4 mg/kg, about 0.8 mg/kg, about 1.6 mg/kg, or about 4 mg/kg, of body weight of recipient subject. In a preferred embodiment of the invention, the anti-IL-6 antibodies are administered to a subject at a concentration of about 0.4 mg/kg of body weight of recipient subject. In a preferred embodiment of the invention, the anti-IL-6 antibodies are administered to a recipient subject with a frequency of once every twenty-six weeks or less, such as once every sixteen weeks or less, once every eight weeks or less, or once every four weeks, or less. In another preferred embodiment of the invention, the anti-IL-6 antibodies are administered to a recipient subject with a frequency at most once per period of approximately one week, such as at most once per period of approximately two weeks, such as at most once per period of approximately four weeks, such as at most once per period of approximately eight weeks, such as at most once per period of approximately twelve weeks, such as at most once per period of approximately sixteen weeks, such as at most once per period of approximately twenty-four weeks.

It is understood that the effective dosage may depend on recipient subject attributes, such as, for example, age, gender, pregnancy status, body mass index, lean body mass, condition or conditions for which the composition is given, other health conditions of the recipient subject that may affect metabolism or tolerance of the composition, levels of IL-6 in the recipient subject, and resistance to the composition (for example, arising from the patient developing antibodies against the composition). A person of skill in the art would be able to determine an effective dosage and frequency of administration through routine experimentation, for example guided by the disclosure herein and the teachings in Goodman, L. S., Gilman, A., Brunton, L. L., Lazo, J. S., & Parker, K. L. (2006). Goodman & Gilman's the pharmacological basis of therapeutics. New York: McGraw-Hill; Howland, R. D., Mycek, M. J., Harvey, R. A., Champe, P. C., & Mycek, M. J. (2006). Pharmacology. Lippincott's illustrated reviews. Philadelphia: Lippincott Williams & Wilkins; and Golan, D. E. (2008). Principles of pharmacology: the pathophysiologic basis of drug therapy. Philadelphia, Pa., [etc.]: Lippincott Williams & Wilkins.

In another embodiment of the invention, the anti-IL-6 antibodies are administered to a subject in a pharmaceutical formulation.

A "pharmaceutical composition" refers to a chemical or biological composition suitable for administration to a mammal. Such compositions may be specifically formulated for administration via one or more of a number of routes, including but not limited to buccal, epicutaneous, epidural, inhalation, intraarterial, intracardial, intracerebroventricular, intradermal, intramuscular, intranasal, intraocular, intraperitoneal, intraspinal, intrathecal, intravenous, oral, parenteral, rectally via an enema or suppository, subcutaneous, subdermal, sublingual, transdermal, and transmucosal. In addition, administration can occur by means of injection, powder, liquid, gel, drops, or other means of administration.

In one embodiment of the invention, the anti-IL-6 antibodies may be optionally administered in combination with one or more active agents. Such active agents include analgesic, antipyretic, anti-inflammatory, antibiotic, antiviral, and anti-cytokine agents. Active agents include agonists, antagonists, and modulators of TNF-*alpha,* IL-2, IL-4, IL-6, IL-10, IL-12, IL-13, IL-18, IFN*-alpha,* IFN*-gamma,* BAFF, CXCL13, IP-10, VEGF, EPO, EGF, HRG, Hepatocyte Growth Factor (HGF), Hepcidin, including antibodies reactive against any of the foregoing, and antibodies reactive against any of their receptors. Active agents also include 2-Arylpropionic acids, Aceclofenac, Acemetacin, Acetylsalicylic acid (Aspirin), Alclofenac, Alminoprofen, Amoxiprin, Ampyrone, Arylalkanoic acids, Azapropazone, Benorylate/Benorilate, Benoxaprofen, Bromfenac, Carprofen, Celecoxib, Choline magnesium salicylate, Clofezone, COX-2 inhibitors, Dexibuprofen, Dexketoprofen, Diclofenac, Diflunisal, Droxicam, Ethenzamide, Etodolac, Etoricoxib, Faislamine, fenamic acids, Fenbufen, Fenoprofen, Flufenamic acid, Flunoxaprofen, Flurbiprofen, Ibuprofen, Ibuproxam, Indometacin, Indoprofen, Kebuzone, Ketoprofen, Ketorolac, Lornoxicam, Loxoprofen, Lumiracoxib, Magnesium salicylate, Meclofenamic acid, Mefenamic acid, Meloxicam, Metamizole, Methyl salicylate, Mofebutazone, Nabumetone, Naproxen, N-Arylanthranilic acids, Oxametacin, Oxaprozin, Oxicams, Oxyphenbutazone, Parecoxib, Phenazone, Phenylbutazone, Phenylbutazone, Piroxicam, Pirprofen, profens, Proglumetacin, Pyrazolidine derivatives, Rofecoxib, Salicyl salicylate, Salicylamide, Salicylates, Sulfinpyrazone, Sulindac, Suprofen, Tenoxicam, Tiaprofenic acid, Tolfenamic acid, Tolmetin, and Valdecoxib. Antibiotics include Amikacin, Aminoglycosides, Amoxicillin, Ampicillin, Ansamycins, Arsphenamine, Azithromycin, Azlocillin, Aztreonam, Bacitracin, Carbacephem, Carbapenems, Carbenicillin, Cefaclor, Cefadroxil, Cefalexin, Cefalothin, Cefalotin, Cefamandole, Cefazolin, Cefdinir, Cefditoren, Cefepime, Cefixime, Cefoperazone, Cefotaxime, Cefoxitin, Cefpodoxime, Cefprozil, Ceftazidime, Ceftibuten, Ceftizoxime, Ceftobiprole, Ceftriaxone, Cefuroxime, Cephalosporins, Chloramphenicol, Cilastatin, Ciprofloxacin, Clarithromycin, Clindamycin, Cloxacillin, Colistin, Co-trimoxazole, Dalfopristin, Demeclocycline, Dicloxacillin, Dirithromycin, Doripenem, Doxycycline, Enoxacin, Ertapenem, Erythromycin, Ethambutol, Flucloxacillin, Fosfomycin, Furazolidone, Fusidic acid, Gatifloxacin, Geldanamycin, Gentamicin, Glycopeptides, Herbimycin, Imipenem, Isoniazid, Kanamycin, Levofloxacin, Lincomycin, Linezolid, Lomefloxacin, Loracarbef, Macrolides, Mafenide, Meropenem, Meticillin, Metronidazole, Mezlocillin, Minocycline, Monobactams, Moxifloxacin, Mupirocin, Nafcillin, Neomycin, Netilmicin, Nitrofurantoin, Norfloxacin, Ofloxacin, Oxacillin, Oxytetracycline, Paromomycin, Penicillin, Penicillins, Piperacillin, Platensimycin, Polymyxin B, Polypeptides, Prontosil, Pyrazinamide, Quinolones, Quinupristin, Rifampicin, Rifampin, Roxithromycin, Spectinomycin, Streptomycin, Sulfacetamide, Sulfamethizole, Sulfanilimide, Sulfasalazine, Sulfisoxazole, Sulfonamides, Teicoplanin, Telithromycin, Tetracycline, Tetracyclines, Ticarcillin, Tinidazole, Tobramycin, Trimethoprim, Trimethoprim-Sulfamethoxazole, Troleandomycin, Trovafloxacin, and Vancomycin. Active agents also include Aldosterone, Beclometasone, Betamethasone, Corticosteroids, Cortisol, Cortisone acetate, Deoxycorticosterone acetate, Dexamethasone, Fludrocortisone acetate, Glucocorticoids, Hydrocortisone, Methylprednisolone, Prednisolone, Prednisone, Steroids, and Triamcinolone. Antiviral agents include abacavir, aciclovir, acyclovir, adefovir, amantadine, amprenavir, an antiretroviral fixed dose combination, an antiretroviral synergistic enhancer, arbidol, atazanavir, atripla, brivudine, cidofovir, combivir, darunavir, delavirdine, didanosine, docosanol, edoxudine, efavirenz, emtricitabine, enfuvirtide, entecavir, entry inhibitors, famciclovir, fomivirsen, fosamprenavir, foscarnet, fosfonet, fusion inhibitor, ganciclovir, gardasil, ibacitabine, idoxuridine, imiquimod, imunovir, indinavir, inosine, integrase inhibitor, interferon, interferon type I, interferon type II, interferon type III, lamivudine, lopinavir, loviride, maraviroc, MK-0518, moroxydine, nelfinavir, nevirapine, nexavir, nucleoside analogues, oseltamivir, penciclovir, peramivir, pleconaril, podophyllotoxin, protease inhibitor, reverse transcriptase inhibitor, ribavirin, rimantadine, ritonavir, saquinavir, stavudine, tenofovir, tenofovir disoproxil, tipranavir, trifluridine, trizivir, tromantadine, truvada, valaciclovir, valganciclovir, vicriviroc, vidarabine, viramidine, zalcitabine, zanamivir, and zidovudine. Any suitable combination of these active agents is also contemplated.

A "pharmaceutical excipient" or a "pharmaceutically acceptable excipient" is a carrier, usually a liquid, in which an active therapeutic agent is formulated. The active therapeutic agent may be a humanized antibody described herein, or one or more fragments or variants thereof. The excipient generally does not provide any pharmacological activity to the formulation, though it may provide chemical and/or biological stability, and release characteristics. Exemplary formulations can be found, for example, in Remington's Pharmaceutical Sciences, 19th Ed., Grennaro, A., Ed., 1995.

As used herein "pharmaceutically acceptable carrier" or "excipient" includes any and all solvents, dispersion media, coatings, antibacterial and antifungal agents, isotonic and absorption delaying agents that are physiologically compatible. The carrier may be suitable for parenteral administration. Alternatively, the carrier can be suitable for intravenous, intraperitoneal, intramuscular, or sublingual administration. Pharmaceutically acceptable carriers include sterile aqueous solutions or dispersions and sterile powders for the extemporaneous preparation of sterile injectable solutions or dispersions. The use of such media and agents for pharmaceutically active substances is well known in the art. Except insofar as any conventional media or agent is incompatible with the active compound, use thereof in the pharmaceutical compositions of the invention is contemplated. Supplementary active compounds can also be incorporated into the compositions.

There is described intravenous administration of antibodies, including Ab1, for cancer indications, wherein the administration formulation comprises, or alternatively consists of, about 10.5 mg/mL of antibody, 25 mM Histidine base, Phosphoric acid q.s. to pH 6, and 250 mM sorbitol.

Described herein is the intravenous administration of antibodies, including Ab1, for cancer indications, wherein the administration formulation comprises, or alternatively consists of, about 10.5 mg/mL of antibody, 12.5 mM Histidine base, 12.5 mM Histidine HCl (or 25 mM Histidine base and Hydrochloric acid q.s. to pH 6), 250 mM sorbitol, and 0.015% (w/w) Polysorbate 80.

Described herein is the subcutaneous administration of antibodies, including Ab1, for rheumatoid arthritis indications, wherein the administration formulation comprises, or alternatively consists of, about 50 or 100 mg/mL of antibody, about 5 mM Histidine base, about 5 mM Histidine HCl to make final pH 6, 250 mM sorbitol, and 0.015% (w/w) Polysorbate 80.

Described herein is the subcutaneous administration of antibodies, including Ab1, for rheumatoid arthritis indications, wherein the administration formulation comprises, or alternatively consists of, about 20 or 100 mg/mL of antibody, about 5 mM Histidine base, about 5 mM Histidine HCl to make final pH 6, 250 to 280 mM sorbitol (or sorbitol in combination with sucrose), and 0.015% (w/w) Polysorbate 80, said formulation having a nitrogen headspace in the shipping vials.

Pharmaceutical compositions typically must be sterile and stable under the conditions of manufacture and storage. The pharmaceutical composition may be present in lyophilized form. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol), and suitable mixtures thereof. A stabilizer may be included in the pharmaceutical composition.

In many cases, it will be preferable to include isotonic agents, for example, sugars, polyalcohols such as mannitol, sorbitol, or sodium chloride in the composition. Prolonged absorption of the injectable compositions can be brought about by including in the composition an agent which delays absorption, for example, monostearate salts and gelatin. Moreover, the alkaline polypeptide can be formulated in a time release formulation, for example in a composition which includes a slow release polymer. The active compounds can be prepared with carriers that will protect the compound against rapid release, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, polylactic acid and polylactic, polyglycolic copolymers (PLG). Many methods for the preparation of such formulations are known to those skilled in the art.

The compounds can be administered by a variety of dosage forms. Any biologically-acceptable dosage form known to persons of ordinary skill in the art, and combinations thereof, are contemplated. Examples of such dosage forms include, without limitation, reconstitutable powders, elixirs, liquids, solutions, suspensions, emulsions, powders, granules, particles, microparticles, dispersible granules, cachets, inhalants, aerosol inhalants, patches, particle inhalants, implants, depot implants, injectables (including subcutaneous, intramuscular, intravenous, and intradermal), infusions, and combinations thereof.
The invention is not limited by the disclosure, but instead the scope of the invention is to be determined entirely by the appended claims.

The invention may be practiced in ways other than those particularly described in the foregoing description and examples.

Certain teachings related to methods for obtaining a clonal population of antigen-specific B cells were disclosed in U.S. Provisional patent application no. 60/801,412, filed May 19, 2006.

Certain teachings related to humanization of rabbit-derived monoclonal antibodies and preferred sequence modifications to maintain antigen binding affinity were disclosed in International Application No. 12/124,723, corresponding to Attorney Docket No. 67858.704001, entitled "Novel Rabbit Antibody Humanization Method and Humanized Rabbit Antibodies", filed May 21, 2008.

Certain teachings related to producing antibodies or fragments thereof using mating competent yeast and corresponding methods were disclosed in U.S. Patent application no. 11/429,053, filed May 8, 2006, (U.S. Patent Application Publication No. US2006/0270045).

Certain teachings related to anti-IL-6 antibodies, methods of producing antibodies or fragments thereof using mating competent yeast and corresponding methods were disclosed in U.S. provisional patent application no. 60/924,550, filed May 21, 2007.

Certain teachings related to anti-IL-6 antibodies and methods of using those antibodies or fragments thereof to address certain diseases and/or disorders were disclosed in U.S. provisional patent application nos. 61/117,839, 61/117,861, and 61/117,811, all filed on November 25, 2008.

Certain anti-IL-6 antibody polynucleotides and polypeptides are disclosed in the sequence listing accompanying this patent application filing.

The following examples are put forth so as to provide those of ordinary skill in the art with a complete disclosure and description of how to make and use the subject invention, and are not intended to limit the scope of what is regarded as the invention. Efforts have been made to ensure accuracy with respect to the numbers used (*e.g.* amounts, temperature, concentrations, *etc.*) but some experimental errors and deviations should be allowed for. Unless otherwise indicated, parts are parts by weight, molecular weight is average molecular weight, temperature is in degrees centigrade; and pressure is at or near atmospheric.

### EXAMPLES

In the following examples, the term "Ab1" refers to an antibody containing the light chain sequence of SEQ ID NO: 702 and the heavy chain sequence of SEQ ID NO: 704, except where the context indicates otherwise.

### Example 1 Production of Enriched Antigen-Specific B Cell Antibody Culture

Panels of antibodies are derived by immunizing traditional antibody host animals to exploit the native immune response to a target antigen of interest. Typically, the host used for immunization is a rabbit or other host that produces antibodies using a similar maturation process and provides for a population of antigen-specific B cells producing antibodies of comparable diversity, e.g., epitopic diversity. The initial antigen immunization can be conducted using complete Freund's adjuvant (CFA), and the subsequent boosts effected with incomplete adjuvant. At about 50-60 days after immunization, preferably at day 55, antibody titers are tested, and the Antibody Selection (ABS) process is initiated if appropriate titers are established. The two key criteria for ABS initiation are potent antigen recognition and function-modifying activity in the polyclonal sera.

At the time positive antibody titers are established, animals are sacrificed and B cell sources isolated. These sources include: the spleen, lymph nodes, bone marrow, and peripheral blood mononuclear cells (PBMCs). Single cell suspensions are generated, and the cell suspensions are washed to make them compatible for low temperature long term storage. The cells are then typically frozen.

To initiate the antibody identification process, a small fraction of the frozen cell suspensions are thawed, washed, and placed in tissue culture media. These suspensions are then mixed with a biotinylated form of the antigen that was used to generate the animal immune response, and antigen-specific cells are recovered using the Miltenyi magnetic bead cell selection methodology. Specific enrichment is conducted using streptavidin beads. The enriched population is recovered and progressed in the next phase of specific B cell isolation.

### Example 2 Production of Clonal, Antigen-Specific B Cell-Containing Culture

Enriched B cells produced according to Example 1 are then plated at varying cell densities per well in a 96 well microtiter plate. Generally, this is at 50, 100, 250, or 500 cells per well with 10 plates per group. The media is supplemented with 4% activated rabbit T cell conditioned media along with 50K frozen irradiated EL4B feeder cells. These cultures are left undisturbed for 5-7 days at which time supernatant-containing secreted antibody is collected and evaluated for target properties in a separate assay setting. The remaining supernatant is left intact, and the plate is frozen at -70 °C. Under these conditions, the culture process typically results in wells containing a mixed cell population that comprises a clonal population of antigen-specific B cells, i.e., a single well will only contain a single monoclonal antibody specific to the desired antigen.

### Example 3 Screening of Antibody Supernatants for Monoclonal Antibody of Desired Specificity and/or Functional Properties

Antibody-containing supernatants derived from the well containing a clonal antigen-specific B cell population produced according to Example 2 are initially screened for antigen recognition using ELISA methods. This includes selective antigen immobilization (e.g., biotinylated antigen capture by streptavidin coated plate), non-specific antigen plate coating, or alternatively, through an antigen build-up strategy (e.g., selective antigen capture followed by binding partner addition to generate a heteromeric protein-antigen complex). Antigen-positive well supernatants are then optionally tested in a function-modifying assay that is strictly dependant on the ligand. One such example is an in vitro protein-protein interaction assay that recreates the natural interaction of the antigen ligand with recombinant receptor protein. Alternatively, a cell-based response that is ligand dependent and easily monitored (e.g., proliferation response) is utilized. Supernatant that displays significant antigen recognition and potency is deemed a positive well. Cells derived from the original positive well are then transitioned to the antibody recovery phase.

### Example 4 Recovery of Single, Antibody-Producing B Cell of Desired Antigen Specificity

Cells are isolated from a well that contains a clonal population of antigen-specific B cells (produced according to Example 2 or 3), which secrete a single antibody sequence. The isolated cells are then assayed to isolate a single, antibody-secreting cell. Dynal streptavidin beads are coated with biotinylated target antigen under buffered medium to prepare antigen-containing microbeads compatible with cell viability. Next antigen-loaded beads, antibody-producing cells from the positive well, and a fluorescein isothiocyanate (FITC)-labeled anti-host H&L IgG antibody (as noted, the host can be any mammalian host, e.g., rabbit, mouse, rat, etc.) are incubated together at 37 °C. This mixture is then re-pipetted in aliquots onto a glass slide such that each aliquot has on average a single, antibody-producing B-cell. The antigen-specific, antibody-secreting cells are then detected through fluorescence microscopy. Secreted antibody is locally concentrated onto the adjacent beads due to the bound antigen and provides localization information based on the strong fluorescent signal. Antibody-secreting cells are identified via FITC detection of antibody-antigen complexes formed adjacent to the secreting cell. The single cell found in the center of this complex is then recovered using a micromanipulator. The cell is snap-frozen in an eppendorf PCR tube for storage at -80 °C until antibody sequence recovery is initiated.

### Example 5 Isolation of Antibody Sequences From Antigen-Specific B Cell

Antibody sequences are recovered using a combined RT-PCR based method from a single isolated B-cell produced according to Example 4 or an antigenic specific B cell isolated from the clonal B cell population obtained according to Example 2. Primers are designed to anneal in conserved and constant regions of the target immunoglobulin genes (heavy and light), such as rabbit immunoglobulin sequences, and a two-step nested PCR recovery step is used to obtain the antibody sequence. Amplicons from each well are analyzed for recovery and size integrity. The resulting fragments are then digested with AluI to fingerprint the sequence clonality. Identical sequences display a common fragmentation pattern in their electrophoretic analysis. Significantly, this common fragmentation pattern which proves cell clonality is generally observed even in the wells originally plated up to 1000 cells/well. The original heavy and light chain amplicon fragments are then restriction enzyme digested with HindIII and XhoI or HindIII and BsiWI to prepare the respective pieces of DNA for cloning. The resulting digestions are then ligated into an expression vector and transformed into bacteria for plasmid propagation and production. Colonies are selected for sequence characterization.

### Example 6 Recombinant Production of Monoclonal Antibody of Desired Antigen Specificity and/or Functional Properties

Correct full-length antibody sequences for each well containing a single monoclonal antibody is established and miniprep DNA is prepared using Qiagen solid-phase methodology. This DNA is then used to transfect mammalian cells to produce recombinant full-length antibody. Crude antibody product is tested for antigen recognition and functional properties to confirm the original characteristics are found in the recombinant antibody protein. Where appropriate, large-scale transient mammalian transfections are completed, and antibody is purified through Protein A affinity chromatography. Kd is assessed using standard methods (e.g., Biacore™) as well as IC50 in a potency assay.

### Example 7 Preparation of Antibodies that Bind Human IL-6

By using the antibody selection protocol described herein, one can generate an extensive panel of antibodies. The antibodies have high affinity towards IL-6 (single to double digit pM Kd) and demonstrate potent antagonism of IL-6 in multiple cell-based screening systems (T1165 and HepG2). Furthermore, the collection of antibodies displays distinct modes of antagonism toward IL-6-driven processes.

### Immunization Strategy

Rabbits were immunized with huIL-6 (R&R). Immunization consisted of a first subcutaneous (sc) injection of 100 µg in complete Freund's adjuvant (CFA) (Sigma) followed by two boosts, two weeks apart, of 50 µg each in incomplete Freund's adjuvant (IFA) (Sigma). Animals were bled on day 55, and serum titers were determined by ELISA (antigen recognition) and by non-radioactive proliferation assay (Promega) using the T1165 cell line.

### Antibody Selection Titer Assessment

Antigen recognition was determined by coating Immulon 4 plates (Thermo) with 1 µg/mL of huIL-6 (50 µL/well) in phosphate buffered saline (PBS, Hyclone) overnight at 4 °C. On the day of the assay, plates were washed 3 times with PBS /Tween 20 (PBST tablets, Calbiochem). Plates were then blocked with 200 µL/well of 0.5% fish skin gelatin (FSG, Sigma) in PBS for 30 minutes at 37 °C. Blocking solution was removed, and plates were blotted. Serum samples were made (bleeds and pre-bleeds) at a starting dilution of 1:100 (all dilutions were made in FSG 50 µL/well) followed by 1:10 dilutions across the plate (column 12 was left blank for background control). Plates were incubated for 30 minutes at 37 °C. Plates were washed 3 times with PBS/Tween 20. Goat anti-rabbit Fc-HRP (Pierce) diluted 1:5000 was added to all wells (50 µL/well), and plates were incubated for 30 minutes at 37 °C. Plates were washed as described above. 50 µL/well of TMB-Stable stop (Fitzgerald Industries) was added to plates, and color was allowed to develop, generally for 3 to 5 minutes. The development reaction was stopped with 50 µL/well 0.5 M HCl. Plates were read at 450 nm. Optical density (OD) versus dilution was plotted using Graph Pad Prizm software, and titers were determined.

### Functional Titer Assessment

The functional activity of the samples was determined by a T1165 proliferation assay. T1165 cells were routinely maintained in modified RPMI medium (Hyclone) supplemented with HEPES, sodium pyruvate, sodium bicarbonate, L-glutamine, high glucose, penicillin/streptomycin, 10% heat inactivated fetal bovine serum (FBS) (all supplements from Hyclone), 2-mercaptoethanol (Sigma), and 10 ng/mL of huIL-6 (R&D). On the day of the assay, cell viability was determined by trypan blue (Invitrogen), and cells were seeded at a fixed density of 20,000 cells/well. Prior to seeding, cells were washed twice in the medium described above without human-IL-6 (by centrifuging at 13000 rpm for 5 minutes and discarding the supernatant). After the last wash, cells were resuspended in the same medium used for washing in a volume equivalent to 50 µL/well. Cells were set aside at room temperature.

In a round-bottom, 96-well plate (Costar), serum samples were added starting at 1:100, followed by a 1:10 dilution across the plate (columns 2 to 10) at 30 µL/well in replicates of 5 (rows B to F: dilution made in the medium described above with no huIL-6). Column 11 was medium only for IL-6 control. 30 µL/well of huIL-6 at 4x concentration of the final EC50 (concentration previously determined) were added to all wells (huIL-6 was diluted in the medium described above). Wells were incubated for 1 hour at 37 °C to allow antibody binding to occur. After 1 hour, 50 µL/well of antibody-antigen (Ab-Ag) complex were transferred to a flat-bottom, 96-well plate (Costar) following the plate map format laid out in the round-bottom plate. On Row G, 50 µL/well of medium were added to all wells (columns 2 to 11) for background control. 50 µL/well of the cell suspension set aside were added to all wells (columns 2 to 11, rows B to G). On Columns 1 and 12 and on rows A and H, 200 µL/well of medium was added to prevent evaporation of test wells and to minimize edge effect. Plates were incubated for 72 h at 37 °C in 4% CO₂. At 72 h, 20 µL/well of CellTiter96 (Promega) reagents was added to all test wells per manufacturer protocol, and plates were incubated for 2 h at 37 °C. At 2 h, plates were gently mixed on an orbital shaker to disperse cells and to allow homogeneity in the test wells. Plates were read at 490 nm wavelength. Optical density (OD) versus dilution was plotted using Graph Pad Prizm software, and functional titer was determined. A positive assay control plate was conducted as described above using MAB2061 (R&D Systems) at a starting concentration of 1 µg/mL (final concentration) followed by 1:3 dilutions across the plate.

### Tissue Harvesting

Once acceptable titers were established, the rabbit(s) were sacrificed. Spleen, lymph nodes, and whole blood were harvested and processed as follows:
Spleen and lymph nodes were processed into a single cell suspension by disassociating the tissue and pushing through sterile wire mesh at 70 µm (Fisher) with a plunger of a 20 cc syringe. Cells were collected in the modified RPMI medium described above without huIL-6, but with low glucose. Cells were washed twice by centrifugation. After the last wash, cell density was determined by trypan blue. Cells were centrifuged at 1500 rpm for 10 minutes; the supernatant was discarded. Cells were resuspended in the appropriate volume of 10% dimethyl sulfoxide (DMSO, Sigma) in FBS (Hyclone) and dispensed at 1 mL/vial. Vials were then stored at -70 °C for 24 h prior to being placed in a liquid nitrogen (LN2) tank for long-term storage.

Peripheral blood mononuclear cells (PBMCs) were isolated by mixing whole blood with equal parts of the low glucose medium described above without FBS. 35 mL of the whole blood mixture was carefully layered onto 8 mL of Lympholyte Rabbit (Cedarlane) into a 45 mL conical tube (Corning) and centrifuged 30 minutes at 2500 rpm at room temperature without brakes. After centrifugation, the PBMC layers were carefully removed using a glass Pasteur pipette (VWR), combined, and placed into a clean 50 mL vial. Cells were washed twice with the modified medium described above by centrifugation at 1500 rpm for 10 minutes at room temperature, and cell density was determined by trypan blue staining. After the last wash, cells were resuspended in an appropriate volume of 10% DMSO/FBS medium and frozen as described above.

### B cell culture

On the day of setting up B cell culture, PBMC, splenocyte, or lymph node vials were thawed for use. Vials were removed from LN2 tank and placed in a 37 °C water bath until thawed. Contents of vials were transferred into 15 mL conical centrifuge tube (Corning) and 10 mL of modified RPMI described above was slowly added to the tube. Cells were centrifuged for 5 minutes at 1.5K rpm, and the supernatant was discarded. Cells were resuspended in 10 mL of fresh media. Cell density and viability was determined by trypan blue. Cells were washed again and resuspended at 1E07 cells/80 µL medium. Biotinylated huIL-6 (B huIL-6) was added to the cell suspension at the final concentration of 3 µg/mL and incubated for 30 minutes at 4 °C. Unbound B huIL-6 was removed with two 10 mL washes of phosphate-buffered (PBF):Ca/Mg free PBS (Hyclone), 2 mM ethylenediamine tetraacetic acid (EDTA), 0.5% bovine serum albumin (BSA) (Sigma-biotin free). After the second wash, cells were resuspended at 1E07 cells/80 µL PBF. 20 µL of MACS® streptavidin beads (Milteni)/10E7 cells were added to the cell suspension. Cells were incubated at 4 °C for 15 minutes. Cells were washed once with 2 mL of PBF/10E7 cells. After washing, the cells were resuspended at 1E08 cells/500 µL of PBF and set aside. A MACS® MS column (Milteni) was pre-rinsed with 500 mL of PBF on a magnetic stand (Milteni). Cell suspension was applied to the column through a pre-filter, and unbound fraction was collected. The column was washed with 1.5 mL of PBF buffer. The column was removed from the magnet stand and placed onto a clean, sterile 5 mL Polypropylene Falcon tube. 1 mL of PBF buffer was added to the top of the column, and positive selected cells were collected. The yield and viability of positive and negative cell fraction was determined by trypan blue staining. Positive selection yielded an average of 1% of the starting cell concentration.

A pilot cell screen was established to provide information on seeding levels for the culture. Three 10-plate groups (a total of 30 plates) were seeded at 50, 100, and 200 enriched B cells/well. In addition, each well contained 50K cells/well of irradiated EL-4.B5 cells (5,000 Rads) and an appropriate level of T cell supernatant (ranging from 1-5% depending on preparation) in high glucose modified RPMI medium at a final volume of 250 µL/well. Cultures were incubated for 5 to 7 days at 37 °C in 4% CO₂ .

### Identification of Selective Antibody Secreting B Cells

Cultures were tested for antigen recognition and functional activity between days 5 and 7.

### Antigen Recognition Screening

The ELISA format used is as described above except 50 µL of supernatant from the B cell cultures (BCC) wells (all 30 plates) was used as the source of the antibody. The conditioned medium was transferred to antigen-coated plates. After positive wells were identified, the supernatant was removed and transferred to a 96-well master plate(s). The original culture plates were then frozen by removing all the supernatant except 40 µL/well and adding 60 µL/well of 16% DMSO in FBS. Plates were wrapped in paper towels to slow freezing and placed at -70 °C.

### Functional Activity Screening

Master plates were then screened for functional activity in the T1165 proliferation assay as described before, except row B was media only for background control, row C was media + IL-6 for positive proliferation control, and rows D-G and columns 2-11 were the wells from the BCC (50 µL/well, single points). 40 µL of IL-6 was added to all wells except the media row at 2.5 times the EC50 concentration determined for the assay. After 1 h incubation, the Ab/Ag complex was transferred to a tissue culture (TC) treated, 96-well, flat-bottom plate. 20 µL of cell suspension in modified RPMI medium without huIL-6 (T1165 at 20,000 cells/well) was added to all wells (100 µL final volume per well). Background was subtracted, and observed OD values were transformed into % of inhibition.

### B cell recovery

Plates containing wells of interest were removed from -70 °C, and the cells from each well were recovered with 5-200 µL washes of medium/well. The washes were pooled in a 1.5 mL sterile centrifuge tube, and cells were pelleted for 2 minutes at 1500 rpm.

The tube was inverted, the spin repeated, and the supernatant carefully removed. Cells were resuspended in 100 µL/tube of medium. 100 µL biotinylated IL-6 coated streptavidin M280 dynabeads (Invitrogen) and 16 µL of goat anti-rabbit H&L IgG-FITC diluted 1:100 in medium was added to the cell suspension.

20 µL of cell/beads/FITC suspension was removed, and 5 µL droplets were prepared on a glass slide (Corning) previously treated with Sigmacote (Sigma), 35 to 40 droplets/slide. An impermeable barrier of paraffin oil (JT Baker) was added to submerge the droplets, and the slide was incubated for 90 minutes at 37 °C, 4% CO₂ in the dark.

Specific B cells that produce antibody can be identified by the fluorescent ring around them due to antibody secretion, recognition of the bead-associated biotinylated antigen, and subsequent detection by the fluorescent-IgG detection reagent. Once a cell of interest was identified, the cell in the center of the fluorescent ring was recovered via a micromanipulator (Eppendorf). The single cell synthesizing and exporting the antibody was transferred into a 250 µL microcentrifuge tube and placed in dry ice. After recovering all cells of interest, these were transferred to -70 °C for long-term storage.

### Example 8 Yeast Cell Expression

*Antibody genes:* Genes were cloned and constructed that directed the synthesis of a chimeric humanized rabbit monoclonal antibody.

*Expression vector:* The vector contains the following functional components: 1) a mutant ColE1 origin of replication, which facilitates the replication of the plasmid vector in cells of the bacterium *Escherichia coli*; 2) a bacterial *Sh ble* gene, which confers resistance to the antibiotic Zeocin™ (phleomycin) and serves as the selectable marker for transformations of both *E. coli* and *P. pastoris*; 3) an expression cassette composed of the glyceraldehyde dehydrogenase gene (*GAP* gene) promoter, fused to sequences encoding the *Saccharomyces cerevisiae* alpha mating factor pre pro secretion leader sequence, followed by sequences encoding a *P. pastoris* transcriptional termination signal from the *P. pastoris* alcohol oxidase I gene (*AOX1*)*.* The Zeocin™ (phleomycin) resistance marker gene provides a means of enrichment for strains that contain multiple integrated copies of an expression vector in a strain by selecting for transformants that are resistant to higher levels of Zeocin™ (phleomycin).

*P. pastoris* strains: *P. pastoris* strains *met1, lys3, ura3* and *ade1* may be used. Although any two complementing sets of auxotrophic strains could be used for the construction and maintenance of diploid strains, these two strains are especially suited for this method for two reasons. First, they grow more slowly than diploid strains that are the result of their mating or fusion. Thus, if a small number of haploid *ade1* or *ura3* cells remain present in a culture or arise through meiosis or other mechanism, the diploid strain should outgrow them in culture.

The second is that it is easy to monitor the sexual state of these strains since diploid Ade+ colonies arising from their mating are a normal white or cream color, whereas cells of any strains that are haploid *ade1* mutants will form a colony with a distinct pink color. In addition, any strains that are haploid *ura3* mutants are resistant to the drug 5-fluoro-orotic acid (FOA) and can be sensitively identified by plating samples of a culture on minimal medium + uracil plates with FOA. On these plates, only uracil-requiring *ura3* mutant (presumably haploid) strains can grow and form colonies. Thus, with haploid parent strains marked with *ade1* and *ura3,* one can readily monitor the sexual state of the resulting antibody-producing diploid strains (haploid versus diploid).

### Methods

*Construction of pGAPZ-alpha expression vectors for transcription of light and heavy chain antibody genes.* The humanized light and heavy chain fragments were cloned into the pGAPZ expression vectors through a PCR directed process. The recovered humanized constructs were subjected to amplification under standard KOD polymerase (Novagen) kit conditions ((1) 94 °C, 2 minutes; (2) 94 °C, 30 seconds (3) 55 °C, 30 seconds; (4) 72 °C, 30 seconds-cycling through steps 2-4 for 35 times; (5) 72 °C 2 minutes) employing the following primers (1) light chain forward AGCGCTTATTCCGCTATCCAGATGACCCAGTC-the AfeI site is single underlined. The end of the HSA signal sequence is double underlined, followed by the sequence for the mature variable light chain (not underlined); the reverse CGTACGTTTGATTTCCACCTTG.

Variable light chain reverse primer. BsiWI site is underlined, followed by the reverse complement for the 3' end of the variable light chain. Upon restriction enzyme digest with AfeI and BsiWI this enable insertion in-frame with the pGAPZ vector using the human HAS leader sequence in frame with the human kapp light chain constant region for export. (2) A similar strategy is performed for the heavy chain. The forward primer employed is AGCGCTTATTCCGAGGTGCAGCTGGTGGAGTC. The AfeI site is single underlined. The end of the HSA signal sequence is double underlined, followed by the sequence for the mature variable heavy chain (not underlined). The reverse heavy chain primer is CTCGAGACGGTGACGAGGGT. The XhoI site is underlined, followed by the reverse complement for the 3' end of the variable heavy chain. This enables cloning of the heavy chain in-frame with IgG-γ1 CH1-CH2-CH3 region previous inserted within pGAPZ using a comparable directional cloning strategy.

*Transformation of expression vectors into haploid ade1 ura3, met1 and lys3 host strains of P. pastoris.* All methods used for transformation of haploid *P. pastoris* strains and genetic manipulation of the *P. pastoris* sexual cycle are as described in Higgins, D. R., and Cregg, J. M., Eds. 1998. Pichia Protocols. Methods in Molecular Biology. Humana Press, Totowa, NJ.

Prior to transformation, each expression vector is linearized within the *GAP* promoter sequences with AvrII to direct the integration of the vectors into the *GAP* promoter locus of the *P. pastoris* genome. Samples of each vector are then individually transformed into electrocompetent cultures of the *ade1, ura3, met1* and *lys3* strains by electroporation and successful transformants are selected on YPD Zeocin™ (phleomycin) plates by their resistance to this antibiotic. Resulting colonies are selected, streaked for single colonies on YPD Zeocin™ (phleomycin) plates and then examined for the presence of the antibody gene insert by a PCR assay on genomic DNA extracted from each strain for the proper antibody gene insert and/or by the ability of each strain to synthesize an antibody chain by a colony lift/immunoblot method (Wung et al. Biotechniques 21 808-812 (1996). Haploid *ade1, met1* and *lys3* strains expressing one of the three heavy chain constructs are collected for diploid constructions along with haploid *ura3* strain expressing light chain gene. The haploid expressing heavy chain genes are mated with the appropriate light chain haploid *ura3* to generate diploid secreting protein.

Mating of haploid strains synthesizing a single antibody chain and selection of diploid derivatives synthesizing tetrameric functional antibodies. To mate P. pastoris haploid strains, each ade1 (or met1 or lys3) heavy chain producing strain to be crossed is streaked across a rich YPD plate and the ura3 light chain producing strain is streaked across a second YPD plate (-10 streaks per plate). After one or two days incubation at 30 °C, cells from one plate containing heavy chain strains and one plate containing ura3 light chain strains are transferred to a sterile velvet cloth on a replica-plating block in a cross hatched pattern so that each heavy chain strain contain a patch of cells mixed with each light chain strain. The cross-streaked replica plated cells are then transferred to a mating plate and incubated at 25 °C to stimulate the initiation of mating between strains. After two days, the cells on the mating plates are transferred again to a sterile velvet on a replica-plating block and then transferred to minimal medium plates. These plates are incubated at 30 °C for three days to allow for the selective growth of colonies of prototrophic diploid strains. Colonies that arose are picked and streaked onto a second minimal medium plate to single colony isolate and purify each diploid strain. The resulting diploid cell lines are then examined for antibody production.

Putative diploid strains are tested to demonstrate that they are diploid and contain both expression vectors for antibody production. For diploidy, samples of a strain are spread on mating plates to stimulate them to go through meiosis and form spores. Haploid spore products are collected and tested for phenotype. If a significant percentage of the resulting spore products are single or double auxotrophs it may be concluded that the original strain must have been diploid. Diploid strains are examined for the presence of both antibody genes by extracting genomic DNA from each and utilizing this DNA in PCR reactions specific for each gene.

Fusion of haploid strains synthesizing a single antibody chain and selection of diploid derivatives synthesizing tetrameric functional antibodies. As an alternative to the mating procedure described above, individual cultures of single-chain antibody producing haploid ade1 and ura3 strains are spheroplasted and their resulting spheroplasts fused using polyethylene glycol/CaCl₂. The fused haploid strains are then embedded in agar containing 1 M sorbitol and minimal medium to allow diploid strains to regenerate their cell wall and grow into visible colonies. Resulting colonies are picked from the agar, streaked onto a minimal medium plate, and the plates are incubated for two days at 30 °C to generate colonies from single cells of diploid cell lines. The resulting putative diploid cell lines are then examined for diploidy and antibody production as described above.

Purification and analysis of antibodies. A diploid strain for the production of full length antibody is derived through the mating of met1 light chain and lys3 heavy chain using the methods described above. Culture media from shake-flask or fermenter cultures of diploid P. pastoris expression strains are collected and examined for the presence of antibody protein via SDS-PAGE and immunoblotting using antibodies directed against heavy and light chains of human IgG, or specifically against the heavy chain of IgG.

To purify the yeast secreted antibodies, clarified media from antibody producing cultures are passed through a protein A column and after washing with 20 mM sodium phosphate, pH 7.0, binding buffer, protein A bound protein is eluted using 0.1 M glycine HCl buffer, pH 3.0. Fractions containing the most total protein are examined by Coomasie blue strained SDS-PAGE and immunoblotting for antibody protein. Antibody is characterized using the ELISA described above for IL-6 recognition.

*Assay for antibody activity.* The recombinant yeast-derived humanized antibody is evaluated for functional activity through the IL-6 driven T1165 cell proliferation assay and IL-6 stimulated HepG2 haptoglobin assay described above.

### Example 9 Acute Phase Response Neutralization by Intravenous Administration of Anti-IL-6 Antibody Ab1.

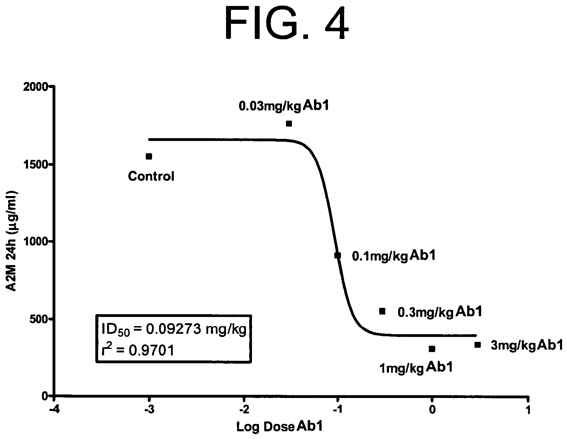

Human IL-6 can provoke an acute phase response in rats, and one of the major acute phase proteins that is stimulated in the rat is *alpha*-2 macroglobulin (A2M). A study was designed to assess the dose of antibody Ab1 required to ablate the A2M response to a single s.c. injection of 100 µg of human IL-6 given one hour after different doses (0.03, 0.1, 0.3, 1, and 3 mg/kg) of antibody Ab1 administered intravenously (n=10 rats/dose level) or polyclonal human IgG1 as the control (n=10 rats). Plasma was recovered and the A2M was quantitated via a commercial sandwich ELISA kit (ICL Inc., Newberg OR; cat. no.- E-25A2M). The endpoint was the difference in the plasma concentration of A2M at the 24 hour time point (post-Abl). The results are presented in Fig. 4.

The ID50 for antibody Ab1 was 0.1 mg/kg with complete suppression of the A2M response at the 0.3 mg/kg. This firmly establishes *in vivo* neutralization of human IL-6 can be accomplished by antibody Ab1.

### Example 10 RXF393 Cachexia Model Study 1.

### Introduction

The human renal cell cancer cell line, RXF393 produces profound weight loss when transplanted into athymic nude mice. Weight loss begins around day 15 after transplantation with 80% of all animals losing at least 30% of their total body weight by day 18 - 20 after transplantation. RXF393 secretes human IL-6 and the plasma concentration of human IL-6 in these animals is very high at around 10 ng/mL. Human IL-6 can bind murine soluble IL-6 receptor and activate IL-6 responses in the mouse. Human IL-6 is approximately 10 times less potent than murine IL-6 at activating IL-6 responses in the mouse. The objectives of this study were to determine the effect of antibody Ab1, on survival, body weight, serum amyloid A protein, hematology parameters, and tumor growth in athymic nude mice transplanted with the human renal cell cancer cell line, RXF393.

### Methods

Eighty, 6 week old, male athymic nude mice were implanted with RXF393 tumor fragments (30-40 mg) subcutaneously in the right flank. Animals were then divided into eight groups of ten mice. Three groups were given either antibody Ab1 at 3 mg/kg, 10 mg/kg, or 30 mg/kg intravenously weekly on day 1, day 8, day 15 and day 22 after transplantation (progression groups). Another three groups were given either antibody Ab1 at 3 mg/kg, or 10 mg/kg, or 30 mg/kg intravenously weekly on day 8, day 15 and day 22 after transplantation (regression groups). Finally, one control group was given polyclonal human IgG 30 mg/kg and a second control group was given phosphate buffered saline intravenously weekly on day 1, day 8, day 15 and day 22 after transplantation.

Animals were euthanized at either day 28, when the tumor reached 4,000 mm³ or if they became debilitated (>30% loss of body weight). Animals were weighed on days 1, 6 and then daily from days 9 to 28 after transplantation. Mean Percent Body Weight (MPBW) was used as the primary parameter to monitor weight loss during the study. It was calculated as follows: (Body Weight - Tumor Weight)/Baseline Body Weight x 100. Tumor weight was measured on days 1, 6, 9, 12, 15, 18, 22, 25 and 28 after transplantation. Blood was taken under anesthesia from five mice in each group on days 5 and 13 and all ten mice in each group when euthanized (day 28 in most cases). Blood was analyzed for hematology and serum amyloid A protein (SAA) concentration. An additional group of 10 non-tumor bearing 6 week old, athymic nude male mice had blood samples taken for hematology and SAA concentration estimation to act as a baseline set of values.

### Results - Survival

No animals were euthanized or died in any of the antibody Ab1 groups prior to the study termination date of day 28. In the two control groups, 15 animals (7/9 in the polyclonal human IgG group and 8/10 in the phosphate buffered saline group) were found dead or were euthanized because they were very debilitated (>30% loss of body weight). Median survival time in both control groups was 20 days.

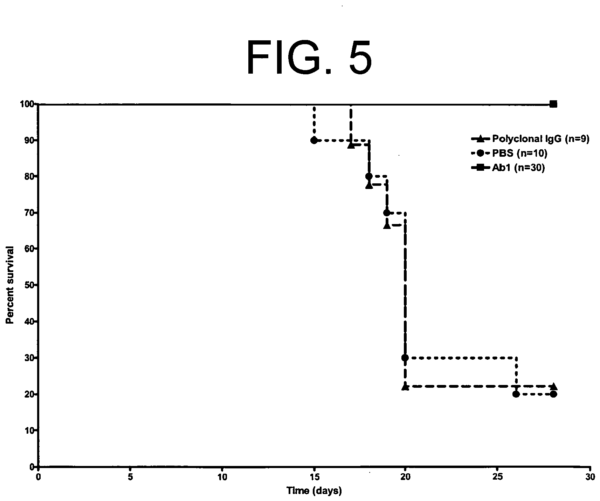

The survival curves for the two control groups and the antibody Ab1 progression (dosed from day 1 of the study) groups are presented in Fig. 5.

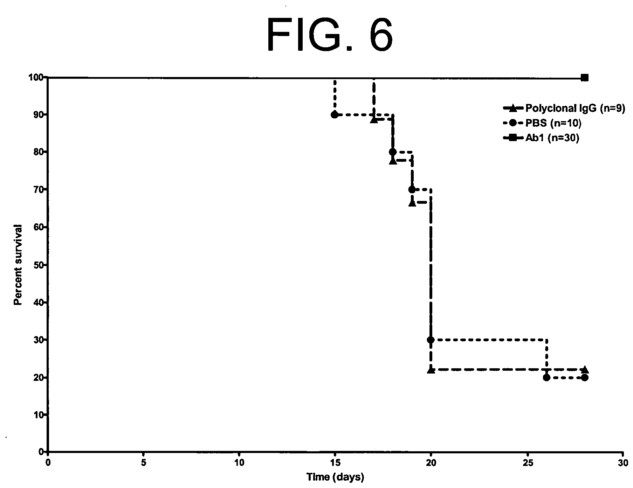

The survival curves for the two control groups and the antibody Ab1 regression (dosed from day 8 of the study) groups are presented in Fig. 6.

There was a statistically significant difference between the survival curves for the polyclonal human IgG (p=0.0038) and phosphate buffered saline (p=0.0003) control groups and the survival curve for the six antibody Ab1 groups. There was no statistically significant difference between the two control groups (p=0.97).

### Results - Tumor Size

Tumor size in surviving mice was estimated by palpation. For the first 15 days of the study, none of the mice in any group were found dead or were euthanized, and so comparison of tumor sizes between groups on these days was free from sampling bias. No difference in tumor size was observed between the antibody Ab1 progression or regression groups and the control groups through day 15. Comparison of the tumor size between surviving mice in the control and treatment groups subsequent to the onset of mortality in the controls (on day 15) was not undertaken because tumor size the surviving control mice was presumed to be biased and accordingly the results of such comparison would not be meaningful.

As administration of antibody Ab1 promoted survival without any apparent reduction in tumor size, elevated serum IL-6 may contribute to mortality through mechanisms independent of tumor growth. These observations support the hypothesis that antibody Ab1 can promote cancer patient survivability without directly affecting tumor growth, possibly by enhancing general patient well-being.

### Results - Weight Loss

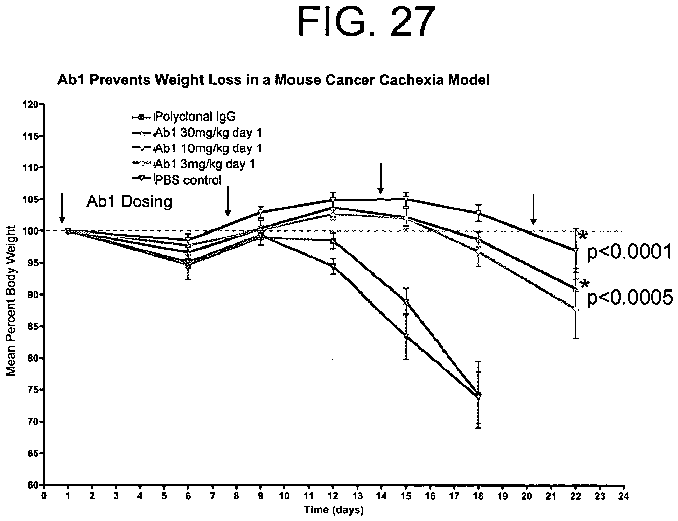

Mean Percent Body Weight (MPBW) (± SEM) versus time is shown in Fig. 27. Compared to controls, mice dosed with Ab1 were protected from weight loss. On day 18, MPBW in control mice was 75%, corresponding to an average weight loss of 25%. In contrast, on the same day, MPBW in Ab-1 treatment groups was minimally changed (between 97% and 103%). There was a statistically significant difference between the MPBW curves for the controls (receiving polyclonal human IgG or PBS) and the 10 mg/kg dosage group (p<0.0001) or 3 mg/kg and 30 mg/kg dosage groups (p<0.0005). There was no statistically significant difference between the two control groups.

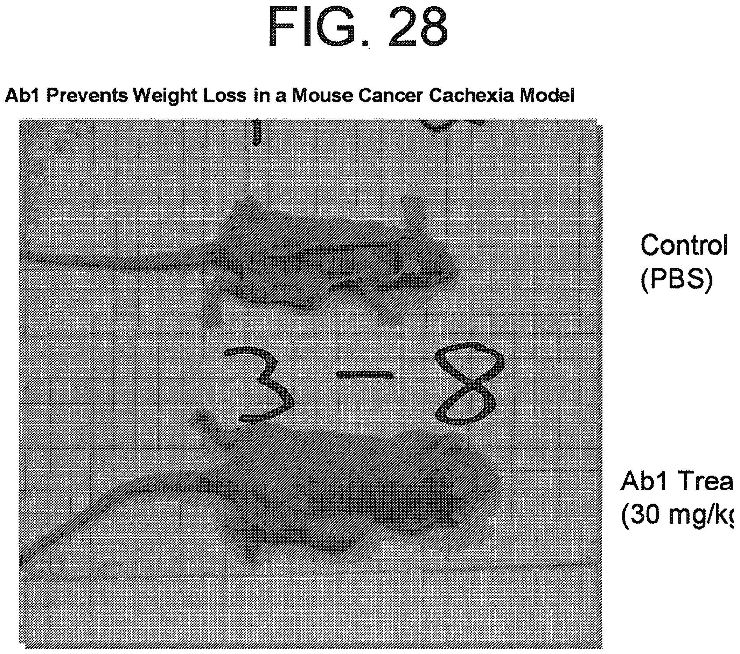

Representative photographs of control and Ab1-treated mice (Fig. 28) illustrate the emaciated condition of the control mice, compared to the normal appearance of the Ab1-treated mouse, at the end of the study (note externally visible tumor sites in right flank).

These results suggest that Ab1 may be useful to prevent or treat cachexia caused by elevated IL-6 in humans.

### Results - Plasma Serum Amyloid A

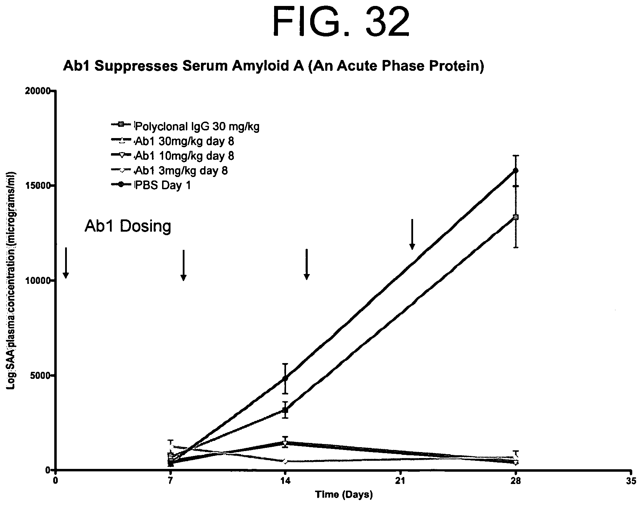

The mean (± SEM) plasma serum amyloid A concentration versus time for the two control groups and the antibody Ab1 progression (dosed from day 1 of the study) and regression (dosed from day 8 of the study) groups are presented in Table 5 and graphically in Fig. 32.

**Table 5: Mean Plasma SAA - antibody Ab1, all groups versus control groups**

| | Mean Plasma SAA±SEM Day 5 (µg/mL) | Mean Plasma SAA±SEM Day 13 (µg/mL) | Mean Plasma SAA±SEM Terminal Bleed (µg/mL) |
|---|---|---|---|
| Polyclonal IgG iv weekly from day 1 | 675 ± 240 (n=5) | 3198 ± 628 (n=4) | 13371 ± 2413 (n=4) |
| PBS iv weekly from day 1 | 355 ± 207 (n=5) | 4844 ± 1126 (n=5) | 15826 ± 802 (n=3) |
| Ab1 30 mg/kg iv weekly from day 1 | 246 ± 100 (n=5) | 2979 ± 170(n=5) | 841 ± 469 (n=10) |
| Ab1 10 mg/kg iv weekly from day 1 | 3629 ± 624 (n=5) | 3096 ± 690 (n=5) | 996 ± 348 (n=10) |
| Ab1 3 mg/kg iv weekly from day 1 | 106 ± 9 (n=5) | 1623 ± 595 (n=4) | 435 ± 70 (n=9) |
| Ab1 30 mg/kg iv weekly from day 8 | 375 ± 177 (n=5) | 1492 ± 418 (n=4) | 498 ± 83 (n=9) |
| Ab1 10 mg/kg iv weekly from day 8 | 487 ± 170 (n=5) | 1403 ± 187 (n=5) | 396 ± 58 (n=10) |
| Ab1 3 mg/kg iv weekly from day 8 | 1255 ± 516 (n=5) | 466 ± 157 (n=5) | 685 ± 350 (n=5) |

SAA is up-regulated via the stimulation of hIL-6 and this response is directly correlated with circulating levels of hIL-6 derived from the implanted tumor. The surrogate marker provides an indirect readout for active hIL-6. Thus in the two treatment groups described above there are significantly decreased levels of SAA due to the neutralization of tumor-derived hIL-6. This further supports the contention that antibody Ab1 displays *in vivo* efficacy.

### Example 11 RXF393 Cachexia Model Study 2.

### Introduction

A second study was performed in the RXF-393 cachexia model where treatment with antibody Ab1 was started at a later stage (days 10 and 13 post-transplantation) and with a more prolonged treatment phase (out to 49 days post transplantation). The dosing interval with antibody Ab1 was shortened to 3 days from 7 and also daily food consumption was measured. There was also an attempt to standardize the tumor sizes at the time of initiating dosing with antibody Ab1.

### Methods

Eighty, 6 week old, male athymic nude mice were implanted with RXF393 tumor fragments (30-40 mg) subcutaneously in the right flank. 20 mice were selected whose tumors had reached between 270 - 320 mg in size and divided into two groups. One group received antibody Ab1 at 10 mg/kg i.v. every three days and the other group received polyclonal human IgG 10 mg/kg every 3 days from that time-point (day 10 after transplantation). Another 20 mice were selected when their tumor size had reached 400 - 527 mg in size and divided into two groups. One group received antibody Ab1 at 10 mg/kg i.v. every three days and the other group received polyclonal human IgG 10 mg/kg every 3 days from that time-point (day 13 after transplantation). The remaining 40 mice took no further part in the study and were euthanized at either day 49, when the tumor reached 4,000 mm³ or if they became very debilitated (>30% loss of body weight).

Animals were weighed every 3-4 days from day 1 to day 49 after transplantation. Mean Percent Body Weight (MPBW) was used as the primary parameter to monitor weight loss during the study. It was calculated as follows: ((Body Weight - Tumor Weight)/Baseline Body Weight) x 100. Tumor weight was measured every 3-4 days from day 5 to day 49 after transplantation. Food consumption was measured (amount consumed in 24 hours by weight (g) by each treatment group) every day from day 10 for the 270-320 mg tumor groups and day 13 for the 400-527 mg tumor groups.

### Results -survival

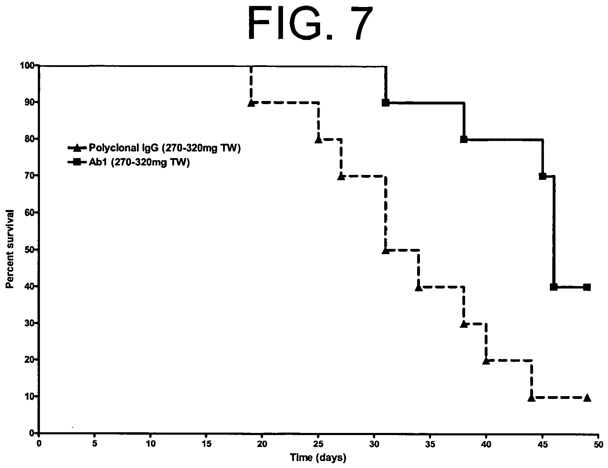

The survival curves for antibody Ab1 at 10 mg/kg i.v. every three days (270-320 mg tumor size) and for the polyclonal human IgG 10 mg/kg i.v. every three days (270-320 mg tumor size) are presented in Fig. 7.

Median survival for the antibody Ab1 at 10 mg/kg i.v. every three days (270-320 mg tumor size) was 46 days and for the polyclonal human IgG at 10 mg/kg i.v. every three days (270-320 mg tumor size) was 32.5 days (p=0.0071).

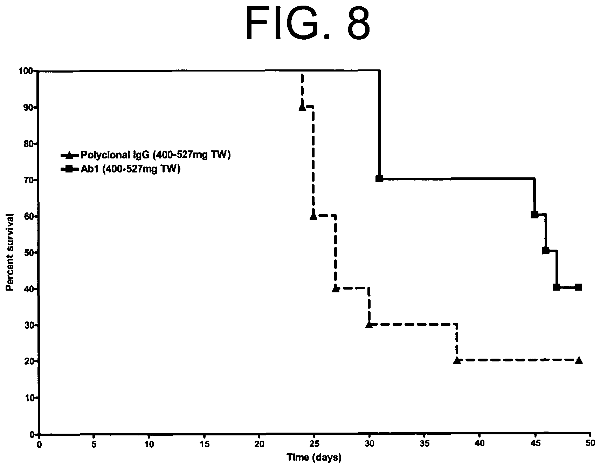

The survival curves for the antibody Ab1 at 10 mg/kg i.v. every three days (400-527 mg tumor size) and for the polyclonal human IgG at 10 mg/kg i.v. every three days (400-527 mg tumor size) are presented in Fig. 8. Median survival for the antibody Ab1 at 10 mg/kg i.v. every three days (400-527 mg tumor size) was 46.5 days and for the polyclonal human IgG at 10 mg/kg i.v. every three days (400-527 mg tumor size) was 27 days (p=0.0481).

### Example 12 Multi-dose Pharmacokinetic Evaluation of Antibody Ab1 in Non-human Primates.

Antibody Ab1 was dosed in a single bolus infusion to a single male and single female cynomologus monkey in phosphate buffered saline. Plasma samples were removed at fixed time intervals and the level of antibody Ab1 was quantitated through of the use of an antigen capture ELISA assay. Biotinylated IL-6 (50 µl of 3 µg/mL) was captured on Streptavidin coated 96 well microtiter plates. The plates were washed and blocked with 0.5% Fish skin gelatin. Appropriately diluted plasma samples were added and incubated for 1 hour at room temperature. The supernatants removed and an anti-hFc-HRP conjugated secondary antibody applied and left at room temperature.

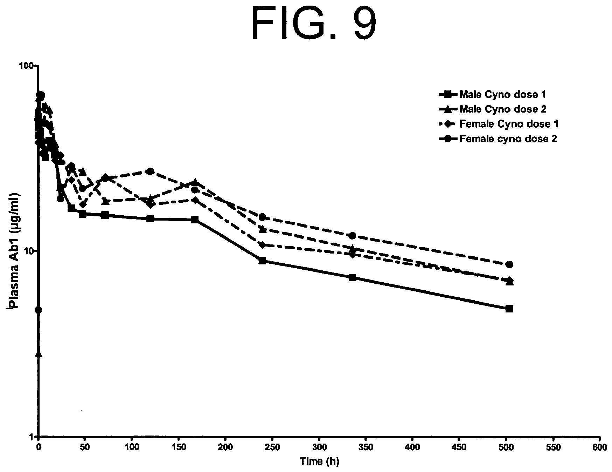

The plates were then aspirated and TMB added to visualize the amount of antibody. The specific levels were then determined through the use of a standard curve. A second dose of antibody Ab1 was administered at day 35 to the same two cynomologus monkeys and the experiment replicated using an identical sampling plan. The resulting concentrations are then plot vs. time as show in Fig. 9.

This humanized full length aglycosylated antibody expressed and purified *Pichia pastoris* displays comparable characteristics to mammalian expressed protein. In addition, multiple doses of this product display reproducible half-lives inferring that this production platform does not generate products that display enhanced immunogenicity.

### Example 13 Octet Mechanistic Characterization of Antibody Proteins.

IL-6 signaling is dependent upon interactions between IL-6 and two receptors, IL-6R1 (CD126) and gp130 (IL-6 signal transducer). To determine the antibody mechanism of action, mechanistic studies were performed using bio-layer interferometry with an Octet QK instrument (ForteBio; Menlo Park, CA). Studies were performed in two different configurations. In the first orientation, biotinylated IL-6 (R&D systems part number 206-IL-001MG/CF, biotinylated using Pierce EZ-link sulfo-NHS-LC-LC-biotin product number 21338 according to manufacturer's protocols) was initially bound to a streptavidin coated biosensor (ForteBio part number 18-5006). Binding is monitored as an increase in signal.

The IL-6 bound to the sensor was then incubated either with the antibody in question or diluent solution alone. The sensor was then incubated with soluble IL-6R1 (R&D systems product number 227-SR-025/CF) molecule. If the IL-6R1 molecule failed to bind, the antibody was deemed to block IL-6/IL-6R1 interactions. These complexes were incubated with gp130 (R&D systems 228-GP-010/CF) in the presence of IL-6R1 for stability purposes. If gp130 did not bind, it was concluded that the antibody blocked gp130 interactions with IL-6.

In the second orientation, the antibody was bound to a biosensor coated with an anti-human IgG1 Fc-specific reagent (ForteBio part number 18-5001). The IL-6 was bound to the immobilized antibody and the sensor was incubated with IL-6R1. If the IL-6R1 did not interact with the IL-6, then it was concluded that the IL-6 binding antibody blocked IL-6/IL-6R1 interactions. In those situations where antibody/IL-6/IL-6R1 was observed, the complex was incubated with gp130 in the presence of IL-6R1. If gp130 did not interact, then it was concluded that the antibody blocked IL-6/gp130 interactions. All studies were performed in a 200 µL final volume, at 30C and 1000 rpm. For these studies, all proteins were diluted using ForteBio's sample diluent buffer (part number 18-5028).

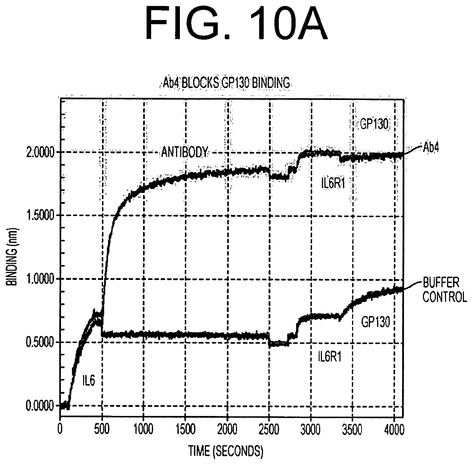

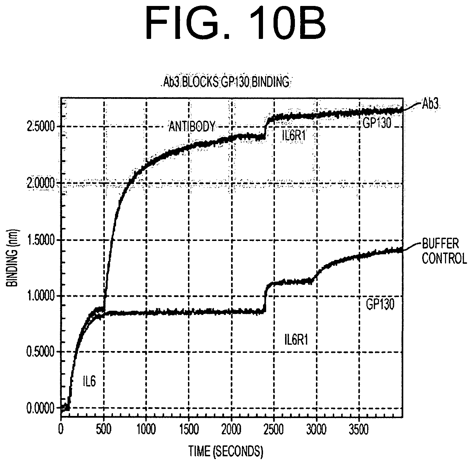

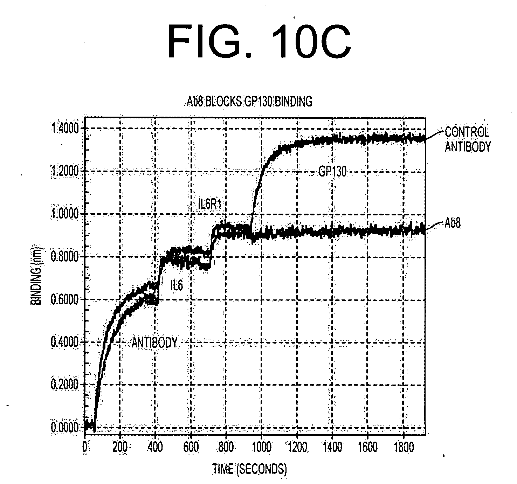

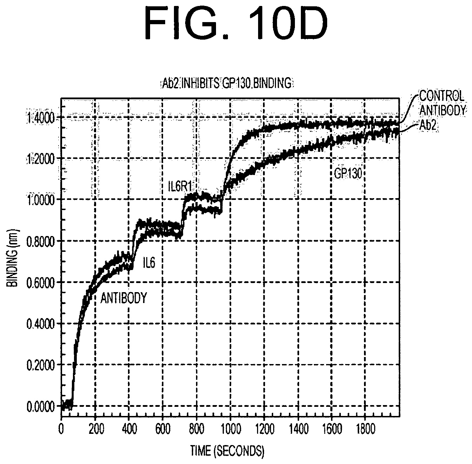

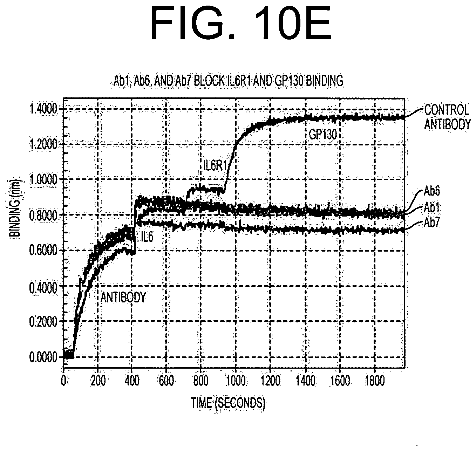

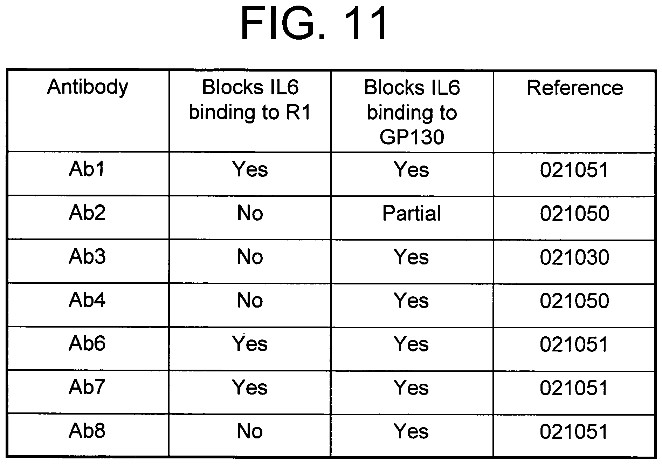

Results are presented in Fig. 10 (A-E) and Fig. 11.

### Example 14 Peptide Mapping.

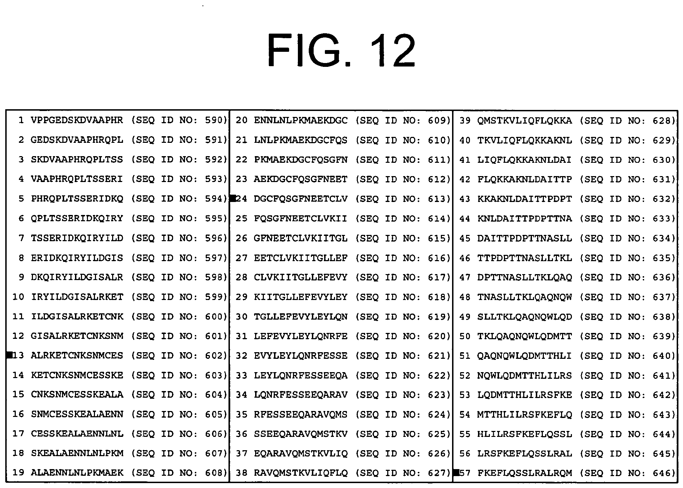

In order to determine the epitope recognized by Ab1 on human IL-6, the antibody was employed in a western-blot based assay. The form of human IL-6 utilized in this example had a sequence of 183 amino acids in length (shown below). A 57-member library of overlapping 15 amino acid peptides encompassing this sequence was commercially synthesized and covalently bound to a PepSpots nitrocellulose membrane (JPT Peptide technologies, Berlin, Germany). The sequences of the overlapping 15 amino acid peptides is shown in Fig. 12 and correspond to SEQ ID NOs: 590-646. Blots were prepared and probed according to the manufacturer's recommendations.

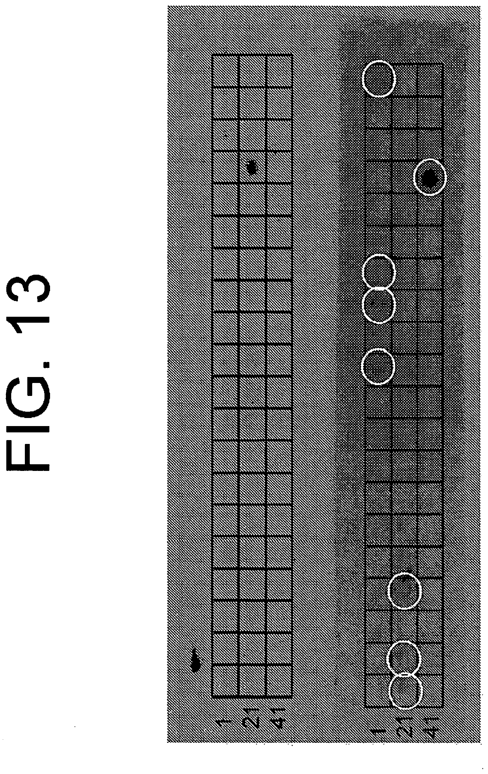

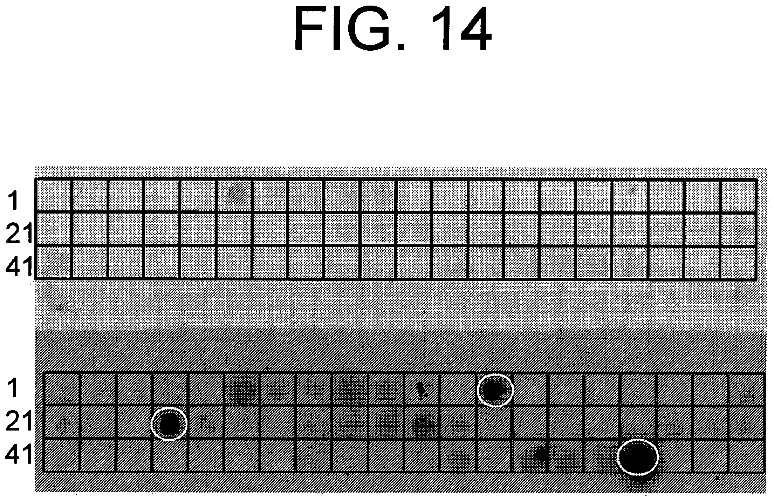

Briefly, blots were pre-wet in methanol, rinsed in PBS, and blocked for over 2 hours in 10% non-fat milk in PBS/0.05% Tween (Blocking Solution). The Ab1 antibody was used at 1 mg/mL final dilution, and the HRP-conjugated Mouse Anti-Human-Kappa secondary antibody (Southern BioTech #9220-05) was used at a 1:5000 dilution. Antibody dilutions/incubations were performed in blocking solution. Blots were developed using Amersham ECL advance reagents (GE# RPN2135) and chemiluminescent signal documented using a CCD camera (AlphaInnotec). The results of the blots is shown in Fig. 13 and Fig. 14.

The sequence of the form of human IL-6 utilized to generate peptide library is set forth:

### Example 15 Ab1 has high affinity for IL-6.

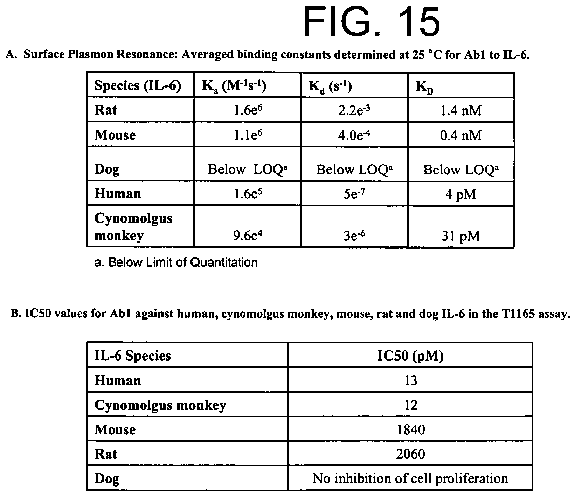

Surface plasmon resonance was used to measure association rate (Ka), dissociation rate (K_{d}) and dissociation constant (K_{D}) for Ab1 to IL-6 from rat, mouse, dog, human, and cynomolgus monkey at 25 °C (Fig. 15A). The dissociation constant for human IL-6 was 4 pM, indicating very high affinity. As expected, affinity generally decreased with phylogenetic distance from human. The dissociation constants of Ab1 for IL-6 of cynomolgus monkey, rat, and mouse were 31 pM, 1.4 nM, and 0.4 nM, respectively. Ab1 affinity for dog IL-6 below the limit of quantitation of the experiment.

The high affinity of Ab1 for mouse, rat, and cynomolgus monkey IL-6 suggest that Ab1 may be used to inhibit IL-6 of these species. This hypothesis was tested using a cell proliferation assay. In brief, each species's IL-6 was used to stimulate proliferation of T1165 cells, and the concentration at which Ab1 could inhibit 50% of proliferation (IC50) was measured. Inhibition was consistent with the measured dissociation constants (Fig. 15B). These results demonstrate that Ab1 can inhibit the native IL-6 of these species, and suggest the use of these organisms for *in vitro* or *in vivo* modeling of IL-6 inhibition by Ab1.

### Example 16 Multi-dose Pharmacokinetic Evaluation of Antibody Ab1 in Healthy Human Volunteers.

Antibody Ab1 was dosed in a single bolus infusion in histidine and sorbitol to healthy human volunteers. Dosages of 1 mg, 3 mg, 10 mg, 30 mg or 100 mg were administered to each individual in dosage groups containing five to six individuals. Plasma samples were removed at fixed time intervals for up to twelve weeks. Human plasma was collected via venipuncture into a vacuum collection tube containing EDTA. Plasma was separated and used to assess the circulating levels of Ab1 using a monoclonal antibody specific for Ab1, as follows. A 96 well microtiter plate was coated overnight with the monoclonal antibody specific for Ab1 in 1X PBS overnight at 4 °C. The remaining steps were conducted at room temperature. The wells were aspirated and subsequently blocked using 0.5% Fish Skin Gelatin (FSG) (Sigma) in 1X PBS for 60 minutes. Human plasma samples were then added and incubated for 60 minutes, then aspirated, then 50 µL of 1 µg/mL biotinylated IL-6 was then added to each well and incubated for 60 minutes. The wells were aspirated, and 50 µL streptavidin-HRP (Pharmingen), diluted 1:5,000 in 0.5% FSG/PBS, was added and incubated for 45 minutes. Development was conducted using standard methods employing TMB for detection. Levels were then determined via comparison to a standard curve prepared in a comparable format.

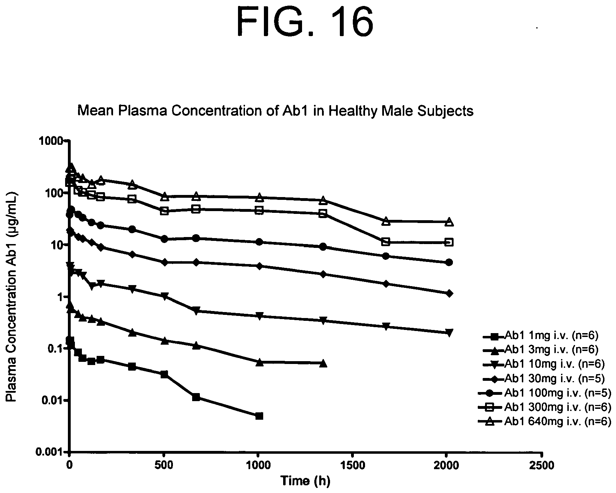

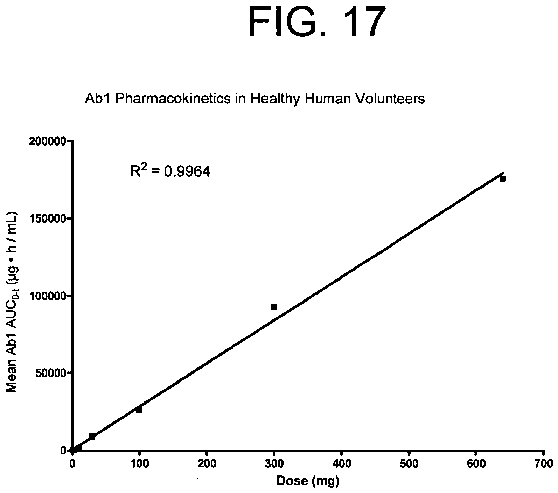

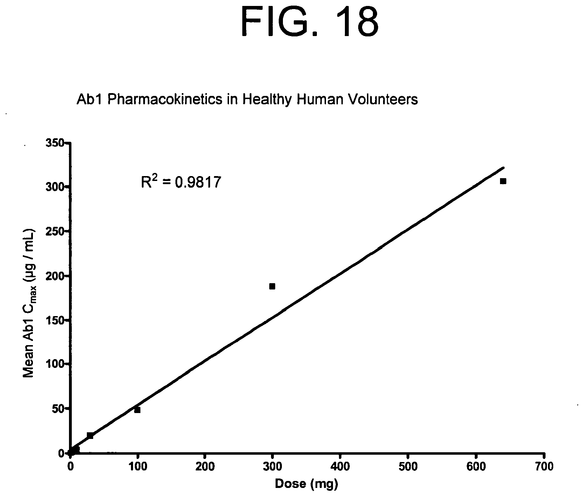

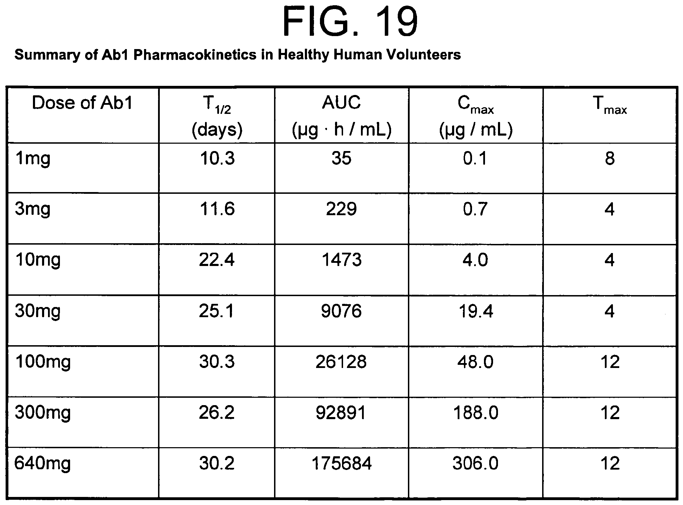

Average plasma concentration of Ab1 for each dosage group versus time is shown in Fig. 16. Mean AUC and Cₘₐₓ increased linearly with dosage (Fig. 17 and Fig. 18, respectively). For dosages of 30 mg and above, the average Ab1 half-life in each dosage group was between approximately 25 and 30 days (Fig. 19).

### Example 17 Pharmacokinetics of Ab1 in patients with advanced cancer.

Antibody Ab1 was dosed in a single bolus infusion in phosphate buffered saline to five individuals with advanced cancer. Each individual received a dosage of 80 mg (n=2) or 160 mg (n=3) of Ab1. Plasma samples were drawn weekly, and the level of antibody Ab1 was quantitated as in Example 16.

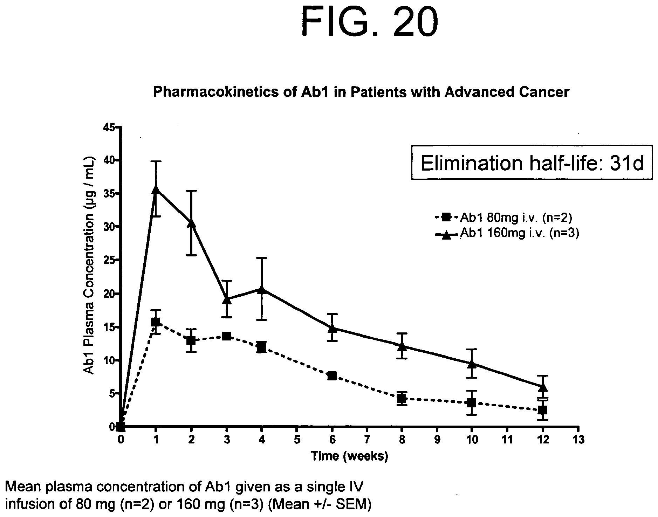

Average plasma concentration of Ab1 in these individuals as a function of time is shown in Fig. 20. The average Ab1 half-life was approximately 31 days.

### Example 18 Unprecedented half-life of Ab1.

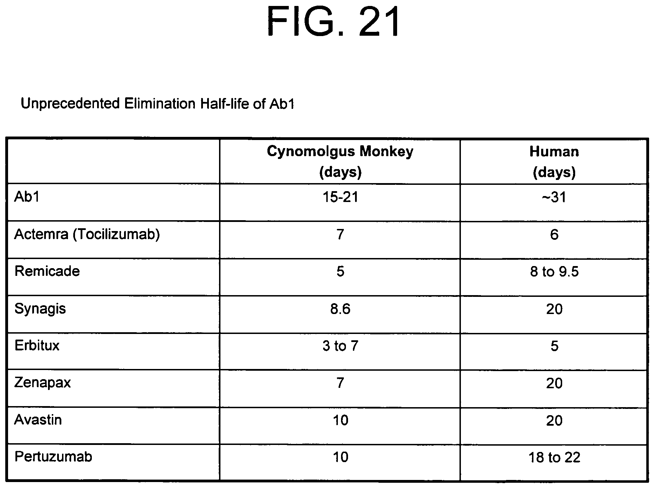

Overall, the average half-life of Ab1 was approximately 31 days in humans (for dosages of 10 mg and above), and approximately 15-21 days in cynomolgus monkey. The Ab1 half-life in humans and cynomolgus monkeys are unprecedented when compared with the half-lives of other anti-IL-6 antibodies (Fig. 21). As described above, Ab1 was derived from humanization of a rabbit antibody, and is produced from Pichia pastoris in an aglycosylated form. These characteristics results in an antibody with very low immunogenicity in humans. Moreover, the lack of glycosylation prevents Ab1 from interacting with the Fc receptor or complement. Without intent to be limited by theory, it is believed that the unprecedented half-life of Ab1 is at least partially attributable to the humanization and/or the lack of glycosylation. The particular sequence and/or structure of the antigen binding surfaces may also contribute to Abl's half-life.

### Example 19 Ab1 Effect on Hemoglobin Concentration, Plasma Lipid Concentration, and Neutrophil Counts in Patients with Advanced Cancer.

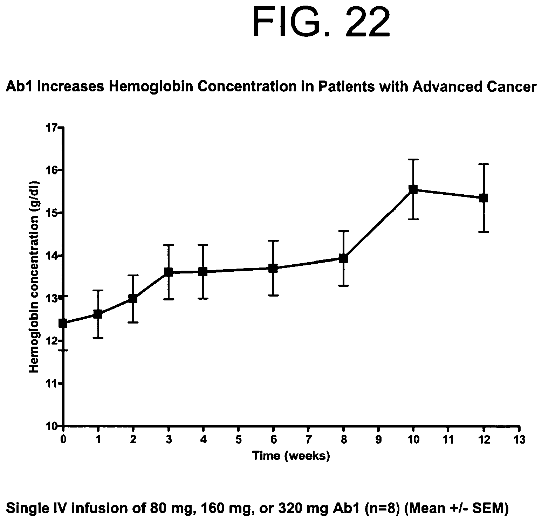

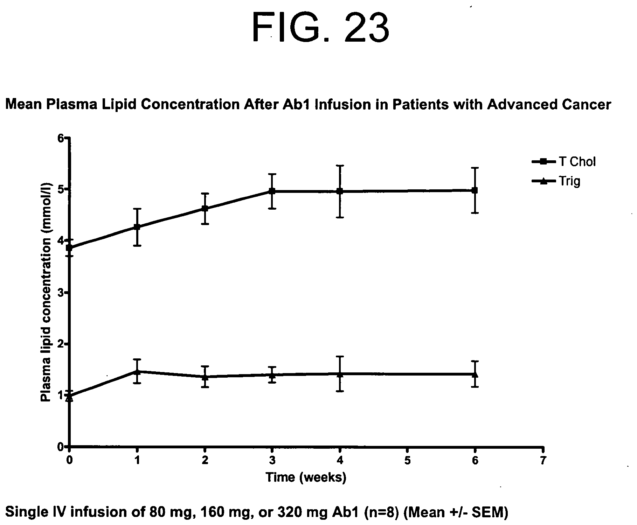

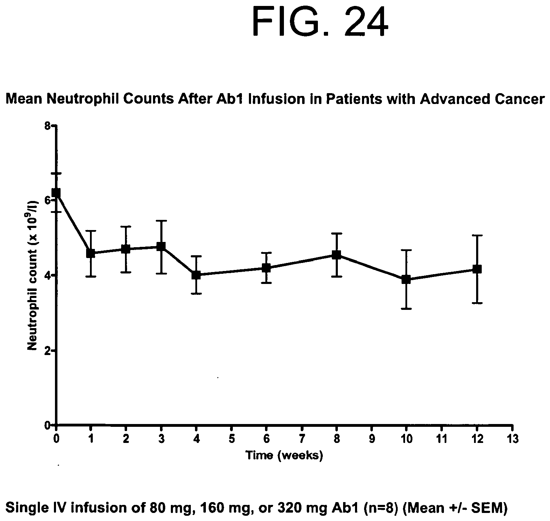

Antibody Ab1 was dosed in a single bolus infusion in phosphate buffered saline to eight individuals with advanced cancer (NSCLC, colorectal cancer, cholangiocarcinoma, or mesothelioma). Each individual received a dosage of 80 mg, 160 mg, or 320 mg of Ab1. Blood samples were removed just prior to infusion and at fixed time intervals for six weeks, and the hemoglobin concentration, plasma lipid concentration, and neutrophil counts were determined. Average hemoglobin concentration rose slightly (Fig. 22), as did total cholesterol and triglycerides (Fig. 23), while mean neutrophil counts fell slightly (Fig. 24).

These results further demonstrate some of the beneficial effects of administration of Ab1 to chronically ill individuals. Because IL-6 is the main cytokine responsible for the anemia of chronic disease (including cancer-related anemia), neutralization of IL-6 by Ab1 increases hemoglobin concentration in these individuals. Similarly, as IL-6 is centrally important in increasing neutrophil counts in inflammation, the observed slight reduction in neutrophil counts further confirms that Ab1 inhibits IL-6. Finally, IL-6 causes anorexia as well as cachexia in these patients; neutralization of IL-6 by Ab1 results in the return of appetite and reversal of cachexia. The increase in plasma lipid concentrations reflect the improved nutritional status of the patients. Taken together, these results further demonstrate that Ab1 effectively reverses these adverse consequences of IL-6 in these patients.

### Example 20 Ab1 Suppresses Serum CRP in Healthy Volunteers and in Patients with Advanced Cancer.

### Introduction

Serum CRP concentrations have been identified as a strong prognostic indicator in patients with certain forms of cancer. For example, Hashimoto *et al.* performed univariate and multivariate analysis of preoperative serum CRP concentrations in patients with hepatocellular carcinoma in order to identify factors affecting survival and disease recurrence (Hashimoto, K., et al., Cancer, 103(9):1856-1864 (2005)). Patients were classified into two groups, those with serum CRP levels > 1.0 mg/dL ("the CRP positive group") and those with serum CRP levels < 1.0 mg/dL ("the CRP negative group"). The authors identified "a significant correlation between preoperative serum CRP level and tumor size." *Id.* Furthermore, the authors found that "[t]he overall survival and recurrence-free survival rates in the CRP-positive group were significantly lower compared with the rates in the CRP-negative group." *Id.* The authors concluded that the preoperative CRP level of patients is an independent and significant predictive indicator or poor prognosis and early recurrence in patients with hepatocellular carcinoma.

Similar correlations have been identified by other investigators. For example, Karakiewicz *et al.* determined that serum CRP was an independent and informative predictor of renal cell carcinoma-specific mortality (Karakiewicz, P.I., et al., Cancer, 110(6):1241-1247 (2007)). Accordingly, there remains a need in the art for methods and/or treatments that reduce serum C-Reactive Protein (CRP) concentrations in cancer patients, and particularly those with advanced cancers.

### Methods

Healthy volunteers received a single 1-hour intravenous (IV) infusion of either 100 mg (5 patients), 30 mg (5 patients), 10 mg (6 patients), 3 mg (6 patients) or 1 mg (6 patients) of the Ab1 monoclonal antibody, while another 14 healthy volunteers received intravenous placebo. Comparatively, 2 patients with advanced forms of colorectal cancer received a single 1-hour intravenous (IV) infusion of 80 mg of the Ab1 monoclonal antibody. No further dosages of the Ab1 monoclonal antibody were administered to the test population.

Patients were evaluated prior to administration of the dosage, and thereafter on a weekly basis for at least 5 weeks post dose. At the time of each evaluation, patients were screened for serum CRP concentration.

### Results

### Healthy Volunteers

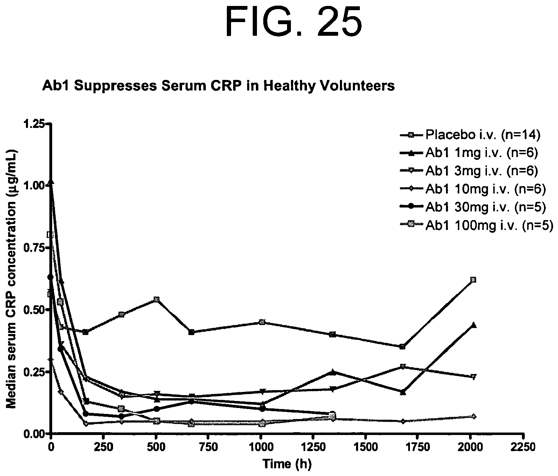

As noted above, serum CRP levels are a marker of inflammation; accordingly, baseline CRP levels are typically low in healthy individuals. The low baseline CRP levels can make a further reduction in CRP levels difficult to detect. Nonetheless, a substantial reduction in serum CRP concentrations was detectable in healthy volunteers receiving all concentrations of the Ab1 monoclonal antibody, compared to controls (Fig. 25). The reduction in serum CRP levels was rapid, occurring within one week of antibody administration, and prolonged, continuing at least through the final measurement was taken (8 or 12 weeks from antibody administration).

### Cancer Patients

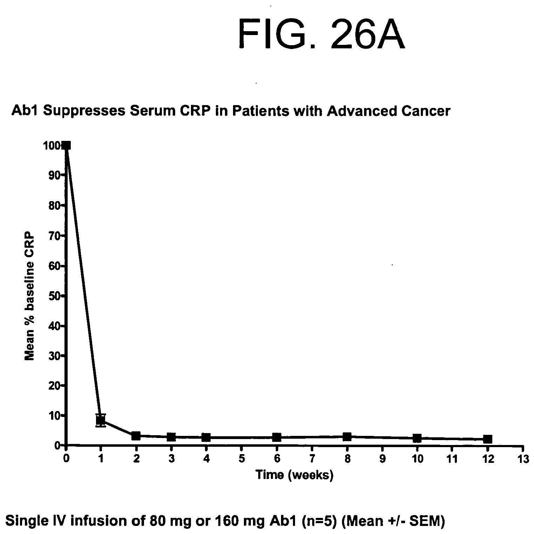

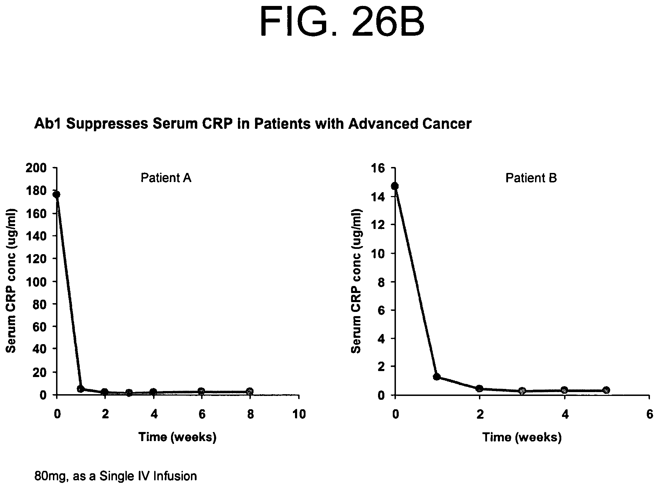

Five advanced cancer patients (colorectal cancer, cholangiocarcinoma, or NSCLC) having elevated serum CRP levels were dosed with 80 mg or 160 mg of Ab1. Serum CRP levels were greatly reduced in these patients (Fig. 26A). The reduction in serum CRP levels was rapid, with 90% of the decrease occurring within one week of Ab1 administration, and prolonged, continuing at least until the final measurement was taken (up to twelve weeks). The CRP levels of two representative individuals are shown in Fig. 26B. In those individuals, the CRP levels were lowered to below the normal reference range (less than 5 - 6 mg/l) within one week. Thus, administration of Ab1 to advanced cancer patients can cause a rapid and sustained suppression of serum CRP levels.

### Example 21 Ab1 Improved Muscular Strength, Improved Weight, and Reduced Fatigue in Patients with Advanced Cancer

### Introduction

Weight loss and fatigue (and accompanying muscular weakness) are very common symptoms of patients with advanced forms of cancer, and these symptoms can worsen as the cancer continues to progress. Fatigue, weight loss and muscular weakness can have significant negative effects on the recovery of patients with advanced forms of cancer, for example by disrupting lifestyles and relationships and affecting the willingness or ability of patients to continue cancer treatments. Known methods of addressing fatigue, weight loss and muscular weakness include regular routines of fitness and exercise, methods of conserving the patient's energy, and treatments that address anemia-induced fatigue and muscular weakness. Nevertheless, there remains a need in the art for methods and/or treatments that improve fatigue, weight loss and muscular weakness in cancer patients.

### Methods

Four patients with advanced forms of cancer (colorectal cancer (2), NSCLC (1), cholangiocarcinoma (1) received a single 1-hour intravenous (IV) infusion of either 80 mg or 160 mg of the Ab1 monoclonal antibody. No further dosages of the Ab1 monoclonal antibody were administered to the test population.

Patients were evaluated prior to administration of the dosage, and thereafter for at least 6 weeks post dose. At the time of each evaluation, patients were screened for the following: a.) any change in weight; b.) fatigue as measured using the Facit-F Fatigue Subscale questionnaire a medically recognized test for evaluating fatigue (See, e.g., Cella, D., Lai, J.S., Chang, C.H., Peterman, A., & Slavin, M. (2002). Fatigue in cancer patients compared with fatigue in the general population. Cancer, 94(2), 528-538; Cella, D., Eton, D.T., Lai,F J-S., Peterman, A.H & Merkel, D.E. (2002). Combining anchor and distribution based methods to derive minimal clinically important differences on the Functional Assessment of Cancer Therapy anemia and fatigue scales. Journal of Pain & Symptom Management, 24 (6) 547-561.); and hand-grip strength (a medically recognized test for evaluating muscle strength, typically employing a handgrip dynamometer).

### Results

### Weight Change

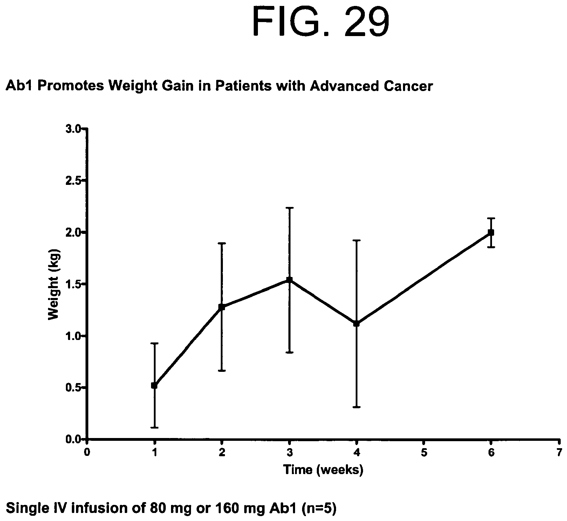

The averaged data for both dosage concentrations (80 mg and 160 mg) of the Ab1 monoclonal antibody demonstrated an increase of about 2 kilograms of weight per patient over the period of 6 weeks (Fig. 29).

### Fatigue

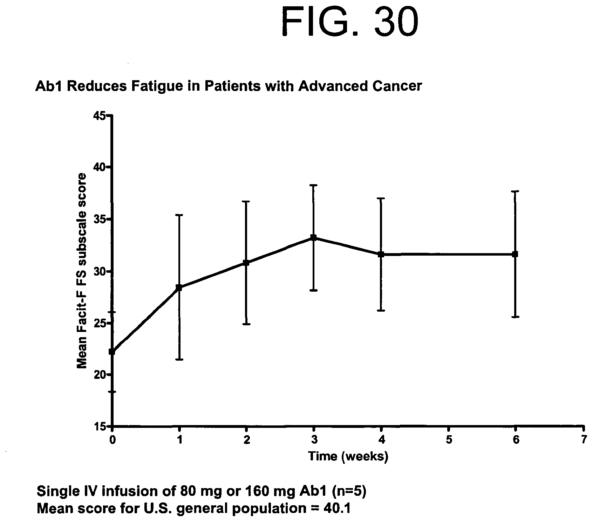

The averaged data for both dosage concentrations (80 mg and 160 mg) of the Ab1 monoclonal antibody demonstrated an increase in the mean Facit-F FS subscale score of at least about 10 points in the patient population over the period of 6 weeks (Fig. 30).

### Hand-Grip Strength

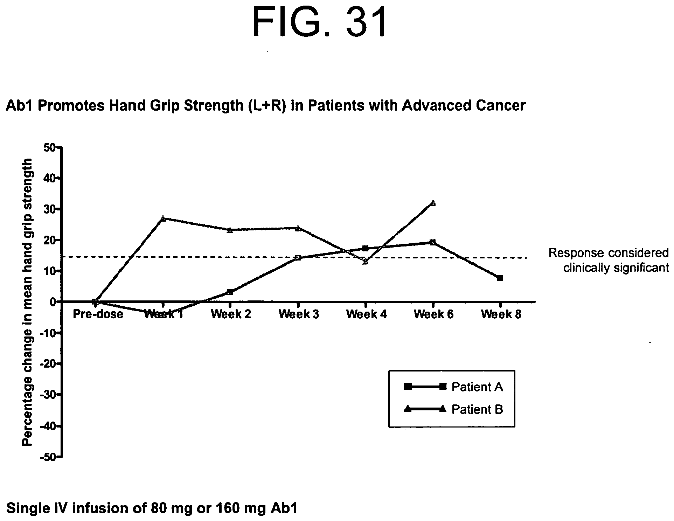

The averaged data for both dosage concentrations (80 mg and 160 mg) of the Ab1 monoclonal antibody demonstrated an increase in the mean hand-grip strength of at least about 10 percent in the patient population over the period of 6 weeks (Fig. 31).

### Example 22 Ab1 For Prevention of Thrombosis

Prior studies have shown that administration of an anti-IL-6 antibody can cause decreased platelet counts. Emilie, D. et al., Blood, 84(8):2472-9 (1994); Blay et al., Int J Cancer, 72(3):424-30 (1997). These results have apparently been viewed as an indicator of potential danger, because further decreases in platelet counts could cause complications such as bleeding. However, Applicants have now discerned that inhibiting IL-6 restores a normal coagulation profile, which Applicants predict will prevent thrombosis. Decreased platelet counts resulting from inhibition of IL-6 is not a sign of potential danger but rather reflects the beneficial restoration of normal coagulation.

The mechanism by which normal coagulation is restored is believed to result from the interplay between IL-6 and the acute phase reaction. In response to elevated IL-6 levels, as for example in a cancer patient, the liver produces acute phase proteins. These acute phase proteins include coagulation factors, such as Factor II, Factor V, Factor VIII, Factor IX, Factor XI, Factor XII, F/fibrin degradation products, thrombin-antithrombin III complex, fibrinogen, plasminogen, prothrombin, and von Willebrand factor. This increase in coagulation factors may be measured directly, or may be inferred from functional measurements of clotting ability. Antagonists of IL-6, such as Ab1, suppresses acute phase proteins, e.g., Serum Amyloid A (see Fig. 32 and Example 10). Applicants now predict that this suppression of acute phase proteins will restore the normal coagulation profile, and thereby prevent thrombosis. The restoration of normal coagulation may cause a slight drop in platelet counts, but the patient will nonetheless retain normal coagulation ability and thus will not have an increased risk of bleeding. Such a treatment will represent a vast improvement over the available anticoagulation therapies whose usefulness is limited by the risk of adverse side-effects, such as major bleeding.

Applicants contemplate that the same beneficial effects of inhibiting IL-6 will be obtained regardless of the method of inhibition. Suitable methods of inhibiting IL-6 include administration of anti-IL-6 antibodies, antisense therapy, soluble IL-6 receptor, etc. either individually or in combinations.

### Example 23 Ab1 Increases Plasma Albumin Concentration in Patients with Advanced Cancer

### Introduction

Serum albumin concentrations are recognized as predictive indicators of survival and/or recovery success of cancer patients. Hypoalbumenia correlates strongly with poor patient performance in numerous forms of cancer. For example, in one study no patients undergoing systemic chemotherapy for metastatic pancreatic adenocarcinoma and having serum albumin levels less than 3.5 g/dL successfully responded to systemic chemotherapy (Fujishiro, M., et al., Hepatogastroenterology, 47(36): 1744-46 (2000)). The authors conclude that "[p]atients with ... hypoalbuminemia ... might be inappropriate candidates for systemic chemotherapy and might be treated with other experimental approaches or supportive care." *Id.*

Similarly, Senior and Maroni state that "[t]he recent appreciation that hypoalbuminemia is the most powerful predictor of mortality in end-stage renal disease highlights the critical importance of ensuring adequate protein intake in this patient population." (J.R. Senior and B.J. Maroni, Am. Soc. Nutr. Sci., 129:313S-314S (1999)).

In at least one study, attempts to rectify hypoalbuminemia in 27 patients with metastatic cancer by daily intravenous albumin infusion of 20 g until normal serum albumin levels (>3.5 g/dL) were achieved had little success. The authors note that "[a]lbumin infusion for the advanced stage cancer patients has limited value in clinical practice. Patients with PS 4 and hypoalbuminemia have poorer prognosis." (Demirkazik, A., et al., Proc. Am. Soc. Clin. Oncol., 21:Abstr 2892 (2002)).

Accordingly, there remains a need in the art for methods and/or treatments that improve serum albumin concentrations in cancer patients and address hypoalbuminemic states in cancer patients, particularly those with advanced cancers.

### Methods

Four patients with advanced forms of cancer (colorectal cancer (2), NSCLC (1), cholangiocarcinoma (1) received a single 1-hour intravenous (IV) infusion of either 80 mg or 160 mg of the Ab1 monoclonal antibody. No further dosages of the Ab1 monoclonal antibody were administered to the test population.

Patients were evaluated prior to administration of the dosage, and thereafter for at least 6 weeks post dose. At the time of each evaluation, patients were screened for plasma albumin concentration.

### Results

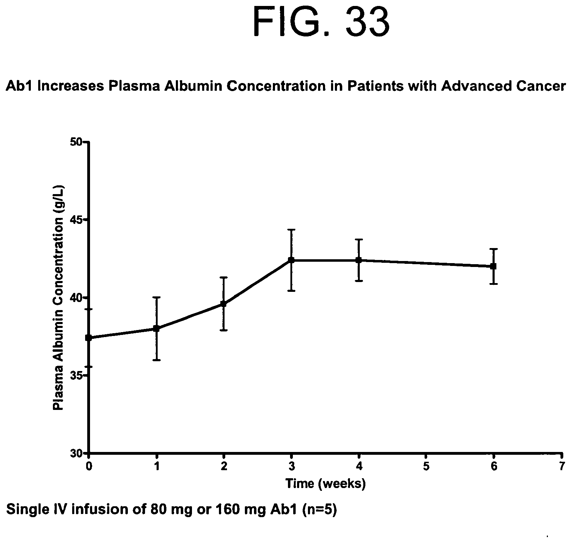

The averaged data for both dosage concentrations (80 mg and 160 mg) of the Ab1 monoclonal antibody demonstrated an increase of about 5 g/L of plasma albumin concentration per patient over the period of 6 weeks (Fig. 33).

### Example 24 Ab1 Suppresses Serum CRP in Patients with Advanced Cancer.

### Introduction

Serum CRP concentrations have been identified as a strong prognostic indicator in patients with certain forms of cancer. For example, Hashimoto *et al.* performed univariate and multivariate analysis of preoperative serum CRP concentrations in patients with hepatocellular carcinoma in order to identify factors affecting survival and disease recurrence (Hashimoto, K., et al., Cancer, 103(9):1856-1864 (2005)). Patients were classified into two groups, those with serum CRP levels > 1.0 mg/dL ("the CRP positive group") and those with serum CRP levels < 1.0 mg/dL ("the CRP negative group"). The authors identified "a significant correlation between preoperative serum CRP level and tumor size." *Id.* Furthermore, the authors found that "[t]he overall survival and recurrence-free survival rates in the CRP-positive group were significantly lower compared with the rates in the CRP-negative group." *Id.* The authors concluded that the preoperative CRP level of patients is an independent and significant predictive indicator of poor prognosis and early recurrence in patients with hepatocellular carcinoma.

Similar correlations have been identified by other investigators. For example, Karakiewicz *et al.* determined that serum CRP was an independent and informative predictor of renal cell carcinoma-specific mortality (Karakiewicz, P.I., et al., Cancer, 110(6):1241-1247 (2007)). Accordingly, there remains a need in the art for methods and/or treatments that reduce serum C-Reactive Protein (CRP) concentrations in cancer patients, and particularly those with advanced cancers.

### Methods

One-hundred twenty-four patients with non-small cell lung cancer (NSCLC) were divided into 4 treatment groups. Patients in one group received one 1-hour intravenous (IV) infusion of either placebo (n=31), 80 mg (n=29), 160 mg (n=32), or 320 mg (n=32) of the Ab1 monoclonal antibody every 8 weeks over a 24 week duration for a total of 3 doses. CRP concentration was quantitated by a C-reactive protein particle-enhanced immunoturbidimetric assay using latex-attached anti-CRP antibodies (i.e. Roche CRP Tinaquant®). Briefly, about 1.0 mL of patient sample serum was collected and stored in a plastic collection tube. Sample was placed into appropriate buffer, and anti-CRP antibody coupled to latex microparticles was added to the sample to start the reaction. These anti-CRP antibodies with conjugated latex microparticles react with antigen in the sample to form an antigen/antibody complex. Following agglutination, this was measured turbidimetrically using a Roche/Hitachi Modular P analizer.

Patients were evaluated prior to administration of the dosage, and thereafter at weeks 2, 4, 8, and 12. At the time of each evaluation, patients were screened for serum CRP concentration.

### Results

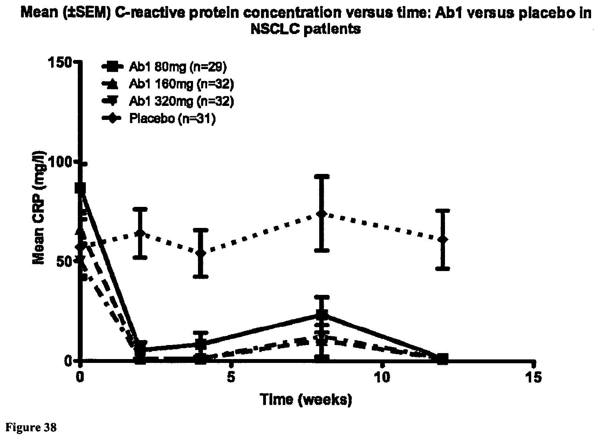

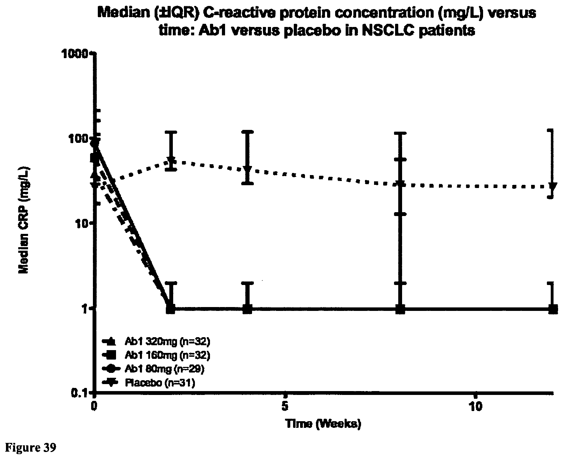

The averaged data for each dosage concentrations (placebo, 80 mg, 160 mg, and 320 mg) of the Ab1 monoclonal antibody are plotted in Figure 38. All dosage levels of Ab1 antibody demonstrated an immediate drop in CRP concentrations relative to placebo over the period of 12 weeks. CRP levels displayed breakthrough at 8 weeks post-dosing. The CRP levels fell below 5 mg/L by week 12. Median values of CRP demonstrated rapid and sustained decreases for all dosage concentrations relative to placebo (Fig. 39). Thus, administration of Ab1 to advanced cancer patients can cause a rapid and sustained suppression of serum CRP levels.

### Example 25 Ab1 Suppresses Serum CRP in Patients with Advanced Cancers.

### Introduction

Serum CRP concentrations have been identified as a strong prognostic indicator in patients with certain forms of cancer. For example, Hashimoto *et al.* performed univariate and multivariate analysis of preoperative serum CRP concentrations in patients with hepatocellular carcinoma in order to identify factors affecting survival and disease recurrence (Hashimoto, K., et al., Cancer, 103(9):1856-1864 (2005)). Patients were classified into two groups, those with serum CRP levels > 1.0 mg/dL ("the CRP positive group") and those with serum CRP levels < 1.0 mg/dL ("the CRP negative group"). The authors identified "a significant correlation between preoperative serum CRP level and tumor size." *Id.* Furthermore, the authors found that "[t]he overall survival and recurrence-free survival rates in the CRP-positive group were significantly lower compared with the rates in the CRP-negative group." *Id.* The authors concluded that the preoperative CRP level of patients is an independent and significant predictive indicator of poor prognosis and early recurrence in patients with hepatocellular carcinoma.

Similar correlations have been identified by other investigators. For example, Karakiewicz *et al.* determined that serum CRP was an independent and informative predictor of renal cell carcinoma-specific mortality (Karakiewicz, P.I., et al., Cancer, 110(6):1241-1247 (2007)). Accordingly, there remains a need in the art for methods and/or treatments that reduce serum C-Reactive Protein (CRP) concentrations in cancer patients, and particularly those with advanced cancers.

### Methods

Eight patients with various forms of advanced cancer (colorectal (3), NSCLC (1), cholangio (1), and mesothelioma (2)) received a single 1-hour intravenous infusion of either 80 mg (2 patients), 160 mg (3 patients) or 320 mg (3 patients) of the Ab1 monoclonal antibody. No further dosages of the Ab1 monoclonal antibody were administered to the test population.

Patients were evaluated prior to administration of the dosage and thereafter on a weekly basis for at least 8 weeks post dose. At the time of each evaluation, patients were screened for serum CRP concentration. CRP concentration was quantitated by a C-reactive protein particle-enhanced immunoturbidimetric assay using latex-attached anti-CRP antibodies (i.e. Roche CRP Tinaquant®). Briefly, about 1.0 mL of patient sample serum was collected and stored in a plastic collection tube. Sample was placed into appropriate buffer, and anti-CRP antibody coupled to latex microparticles was added to the sample to start the reaction. These anti-CRP antibodies with conjugated latex microparticles react with antigen in the sample to form an antigen/antibody complex. Following agglutination, this was measured turbidimetrically using a Roche/Hitachi Modular P analizer.

### Results

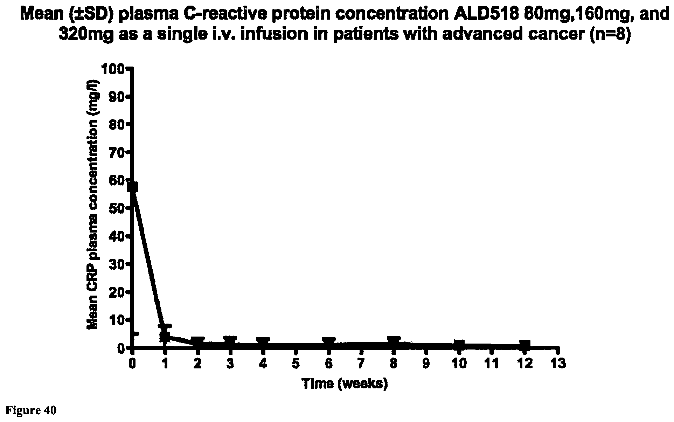

Serum CRP levels were greatly reduced in all patients studied (Fig. 40). The reduction in serum CRP levels was rapid, with approximately 90% of the decrease occurring within one week of Ab1 administration, and prolonged diminished levels continued at least until the final measurement was taken (up to twelve weeks). In all cases except one patient with colorectal cancer, CRP levels fell to at or below the normal reference range (less than 5-6 mg/L) within one week. The colorectal cancer patient achieved similar normal levels by week 4 of the study. Thus, administration of Ab1 to advanced cancer patients can cause a rapid and sustained suppression of serum CRP levels.

### Example 26 Ab1 Suppresses Serum CRP in Patients with Rheumatoid Arthritis.

### Introduction

Serum CRP concentrations have been identified as a strong prognostic indicator in patients with rheumatoid arthritis. Patients suffering from rheumatoid arthritis with high levels of CRP demonstrated almost universal deterioration. Amos et al., 1 Br. Med. J. 195-97 (1977). Conversely, patients with low CRP levels showed no disease progression, suggesting that sustaining low levels of CRP is necessary for effectively treating rheumatoid arthritis. *Id.* Tracking of CRP during rheumatoid arthritis treatment regimes of gold, D-penicillamine, chloroquine, or dapsone indicated that radiological deterioration was impeded after the first 6 months of treatment when CRP levels were consistently controlled. Dawes et al., 25 Rheumatology 44-49 (1986). A highly significant correlation between CRP production and radiological progression was identified, van Leeuwen et al., 32 (Supp. 3) Rheumatology 9-13 (1997). Another study revealed that for patients with active rheumatoid arthritis, suppression of abnormally elevated CRP led to improvement in functional testing metrics, whereas sustained CRP elevation associated with deterioration in the same metrics. Devlin et al., 24 J. Rheumatol. 9-13 (1997). No further deterioration was observed without CRP re-elevation, indicating CRP suppression as a viable candidate for rheumatoid arthritis treatment. *Id.* Accordingly, there remains a need in the art for methods and/or treatments that reduce serum C-Reactive Protein (CRP) concentrations in rheumatoid arthritis patients.

### Methods

One-hundred twenty-seven patients with active rheumatoid arthritis and CRP ≥10 mg/L were divided into 4 treatment groups. Patients in one group received one 1-hour intravenous (IV) infusion of either placebo (n=33), 80 mg (n=32), 160 mg (n=34), or 320 mg (n=28) of the Ab1 monoclonal antibody, once at the start of the 16 week trial and again at week 8. CRP concentration was quantitated by a C-reactive protein particle-enhanced immunoturbidimetric assay using latex-attached anti-CRP antibodies (i.e. Roche CRP Tinaquant®). Briefly, about 1.0 mL of patient sample serum was collected and stored in a plastic collection tube. Sample was placed into appropriate buffer, and anti-CRP antibody coupled to latex microparticles was added to the sample to start the reaction. These anti-CRP antibodies with conjugated latex microparticles react with antigen in the sample to form an antigen/antibody complex. Following agglutination, this was measured turbidimetrically using a Roche/Hitachi Modular P analizer. Data on CRP concentration was collected every week for the first 4 weeks, every two weeks between weeks 4 and 12, and at the conclusion of the test at week 16.

### Results

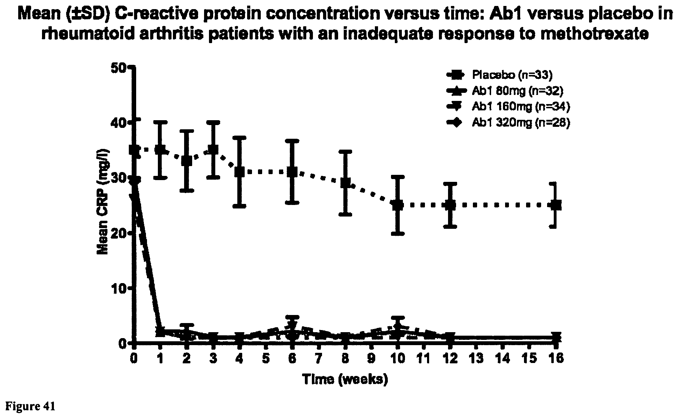

Serum CRP levels were greatly reduced in all patients studied (Fig. 41). The reduction in serum CRP levels was rapid, with immediate reduction in CRP levels relative to placebo within one week of Ab1 administration, and prolonged diminished levels continued at least until the final measurement was taken (up to sixteen weeks). In all cases, CRP levels fell to at or below the normal reference range (less than 5-6 mg/L) within one week. Thus, administration of Ab1 to rheumatoid arthritis patients can cause a rapid and sustained suppression of serum CRP levels and presents an effective treatment regime.

### Example 27 Ab1 Increases Hemoglobin in Patients with Advanced Cancer

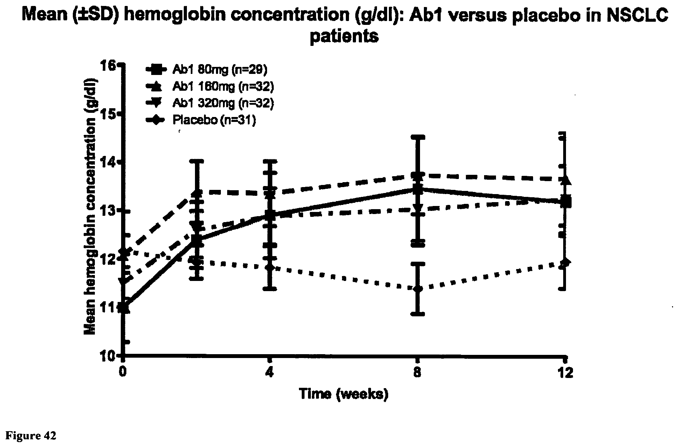

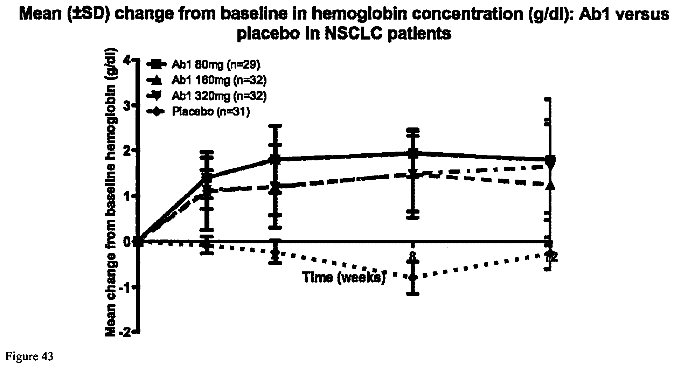

Antibody Ab1 was dosed at 80 mg, 160 mg, or 320 mg of Ab1 in phosphate buffered saline to 93 individuals with non-small cell lung carcinoma. The placebo group of 31 individuals with non-small cell lung carcinoma was dosed with with phosphate buffered saline only. Blood samples were removed just prior to dosing (zero week), and at two, four, eight and twelve weeks, and the hemoglobin concentration was determined. Mean hemoglobin concentration rose for those receiving antibody Ab1, while mean hemoglobin concentration of those receiving placebo did not rise after twelve weeks when compared to the concentration just prior to dosing (zero week) (Figs. 42 and 43).

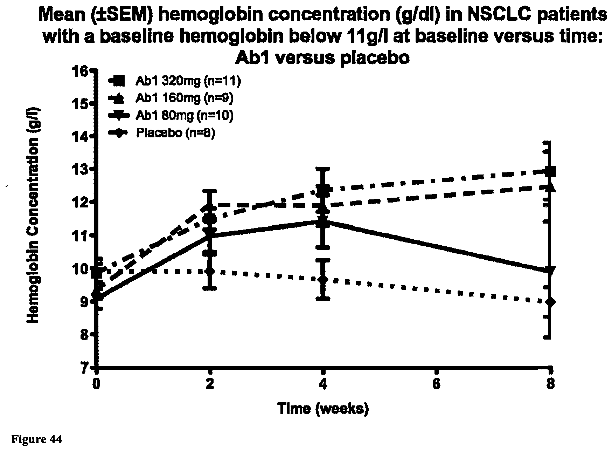

A subset of the study population began the study with low levels of hemoglobin, defined as a baseline hemoglobin concentration below 11 g/l. Mean hemoglobin concentration rose above 11 g/l after eight weeks for those receiving antibody Ab1 at dosages of 160 mg and 320 mg, while mean hemoglobin concentration of those receiving antibody Ab1 at dosages of 80 mg or placebo did not rise above 11 g/l after eight weeks (Fig. 44).

These results further demonstrate some of the beneficial effects of administration of Ab1 to chronically ill individuals. Because IL-6 is the main cytokine responsible for the anemia of chronic disease (including cancer-related anemia), neutralization of IL-6 by Ab1 increases hemoglobin concentration in these individuals.

### Example 28 Ab1 Increases Hemoglobin in Patients with Rheumatoid Arthritis.

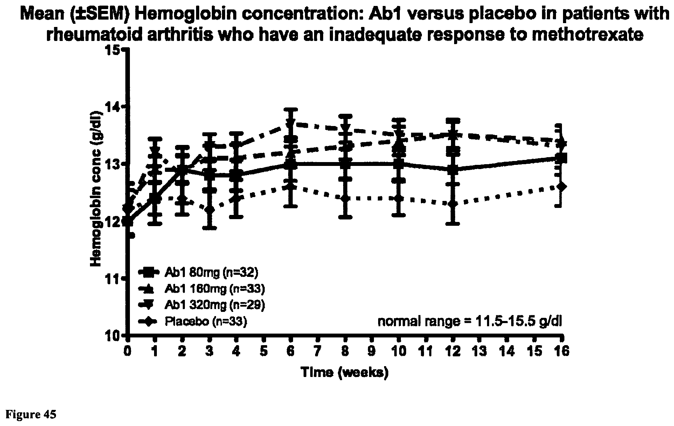

Hemoglobin levels were analyzed in patients with rheumatoid arthritis during treatment with Ab1 antibody. Ab1 antibody was dosed at 80 mg, 160 mg, or 320 mg in phosphate buffered saline to 94 individuals with rheumatoid arthritis. The placebo group of 33 individuals with rheumatoid arthritis was dosed with phosphate buffered saline only. Blood samples were removed just prior to dosing (zero week), and at one, two, three, four, six, eight, ten, twelve, and sixteen weeks, and the hemoglobin concentration was determined. Mean hemoglobin concentration rose for those receiving antibody Ab1, while mean hemoglobin concentration of those receiving placebo did not appreciably rise after sixteen weeks when compared to the concentration just prior to dosing (zero week) (Fig. 45).

These results further demonstrate some of the beneficial effects of administration of Ab1 to chronically ill individuals. Because IL-6 is the main cytokine responsible for the anemia of chronic disease (including cancer-related anemia), neutralization of IL-6 by Ab1 increases hemoglobin concentration.

### Example 29 Ab1 Increases Albumin in Patients with Advanced Cancer

### Introduction

Serum albumin concentrations are recognized as predictive indicators of survival and/or recovery success of cancer patients. Hypoalbumenia correlates strongly with poor patient performance in numerous forms of cancer. For example, in one study no patients undergoing systemic chemotherapy for metastatic pancreatic adenocarcinoma and having serum albumin levels less than 3.5 g/dL successfully responded to systemic chemotherapy (Fujishiro, M., et al., Hepatogastroenterology, 47(36):1744-46 (2000)). The authors conclude that "[p]atients with ... hypoalbuminemia ... might be inappropriate candidates for systemic chemotherapy and might be treated with other experimental approaches or supportive care." *Id.*

Similarly, Senior and Maroni state that "[t]he recent appreciation that hypoalbuminemia is the most powerful predictor of mortality in end-stage renal disease highlights the critical importance of ensuring adequate protein intake in this patient population." (J.R. Senior and B.J. Maroni, Am. Soc. Nutr. Sci., 129:313S-314S (1999)).

In at least one study, attempts to rectify hypoalbuminemia in 27 patients with metastatic cancer by daily intravenous albumin infusion of 20 g until normal serum albumin levels (>3.5 g/dL) were achieved had little success. The authors note that "[a]lbumin infusion for the advanced stage cancer patients has limited value in clinical practice. Patients with PS 4 and hypoalbuminemia have poorer prognosis." (Demirkazik, A., et al., Proc. Am. Soc. Clin. Oncol., 21:Abstr 2892 (2002)).

Accordingly, there remains a need in the art for methods and/or treatments that improve serum albumin concentrations in cancer patients and address hypoalbuminemic states in cancer patients, particularly those with advanced cancers.

### Methods

Antibody Ab1 was dosed at 80 mg, 160 mg, or 320 mg of Ab1 in phosphate buffered saline to 93 individuals with non-small cell lung carcinoma. Each individual received a dosage of. The placebo group of 31 individuals with non-small cell lung carcinoma was dosed with phosphate buffered saline only. Blood samples were removed just prior to dosing (zero week), and at two, four, eight and twelve weeks, and the albumin concentration was determined.

### Results

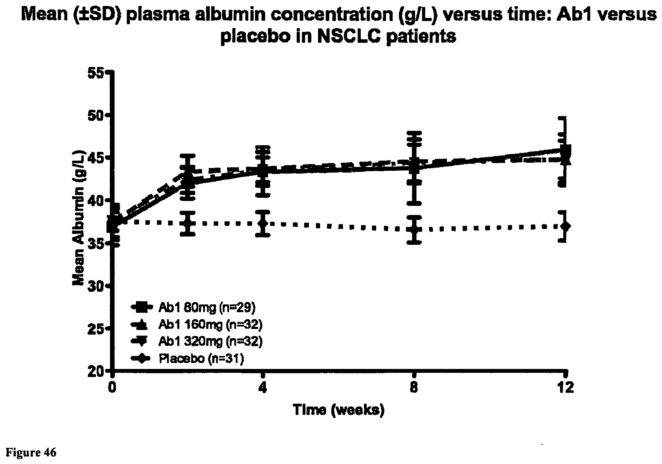

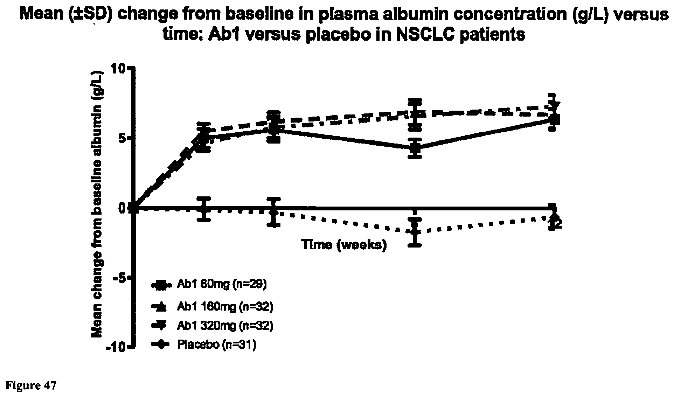

Mean albumin concentration rose for those receiving antibody Ab1, while mean albumin concentration of those receiving placebo did not rise after twelve weeks when compared to the concentration just prior to dosing (zero week) (Fig. 46). The change from baseline albumin values for all dosage concentration groups is plotted in Figure 47.

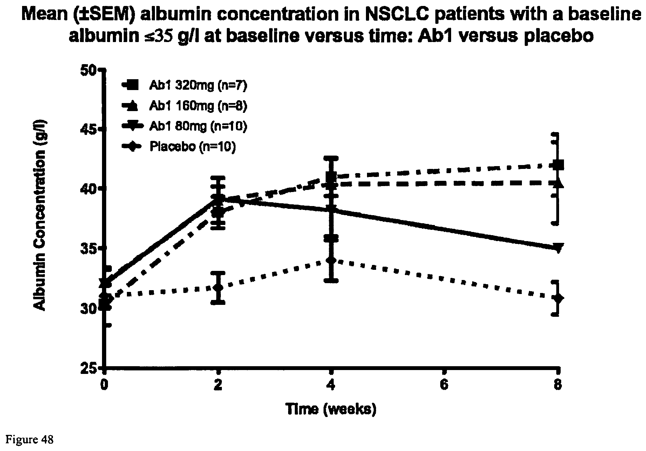

A subset of the study population began the study with low levels of albumin, defined as a baseline albumin concentration less than or equal to 35 g/L. Mean albumin concentration initially rose with all dosages of antibody Ab1 over placebo, but only patients receiving 160 mg or 320 mg demonstrated sustained albumin levels above 35 g/L over 8 weeks of the study (Fig. 48). The 80 mg dosage group demonstrated an initial increase, but gradually declined after week 2 and never rose above 35 g/L during the 8 weeks where data was available (*Id.*).

### Example 30 Ab1 Improved Weight and Reduced Fatigue in Patients with Advanced Cancer

### Introduction

Weight loss and fatigue are very common symptoms of patients with advanced forms of cancer, and these symptoms can worsen as the cancer continues to progress. Fatigue and weight loss can have significant negative effects on the recovery of patients with advanced forms of cancer, for example by disrupting lifestyles and relationships and affecting the willingness or ability of patients to continue cancer treatments. Known methods of addressing fatigue and weight loss include regular routines of fitness and exercise, methods of conserving the patient's energy, and treatments that address anemia-induced fatigue. Nevertheless, there remains a need in the art for methods and/or treatments that improve fatigue and weight loss in cancer patients.

### Methods

One-hundred twenty-four patients with non-small cell lung cancer (NSCLC) were divided into 4 treatment groups. Patients in one group received one 1-hour intravenous (IV) infusion of either placebo (n=31), 80 mg (n=29), 160 mg (n=32), or 320 mg (n=32) of the Ab1 monoclonal antibody every 8 weeks over a 24 week duration for a total of 3 doses.

Patients were evaluated prior to administration of the dosage, and thereafter for at least 12 weeks post dose. At the time of each evaluation, patients were screened for the following: a.) any change in weight; and b.) fatigue as measured using the Facit-F Fatigue Subscale questionnaire a medically recognized test for evaluating fatigue (See, e.g., Cella, D., Lai, J.S., Chang, C.H., Peterman, A., & Slavin, M. (2002). Fatigue in cancer patients compared with fatigue in the general population. Cancer, 94(2), 528-538; Cella, D., Eton, D.T., Lai,F J-S., Peterman, A.H & Merkel, D.E. (2002). Combining anchor and distribution based methods to derive minimal clinically important differences on the Functional Assessment of Cancer Therapy anemia and fatigue scales. Journal of Pain & Symptom Management, 24 (6) 547-561.).

### Results

### Weight Change

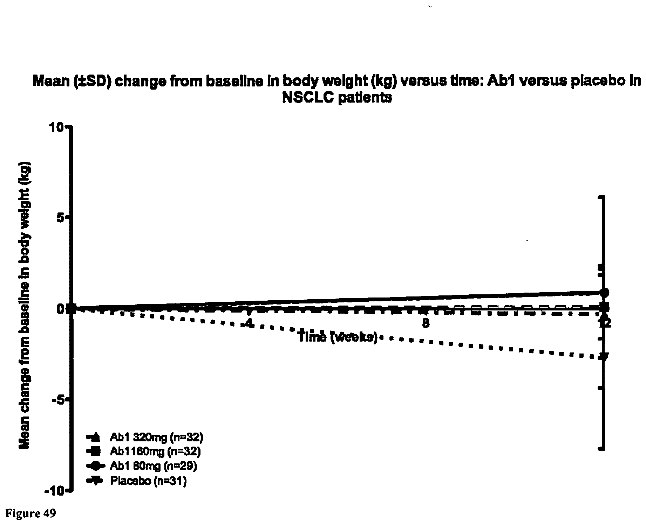

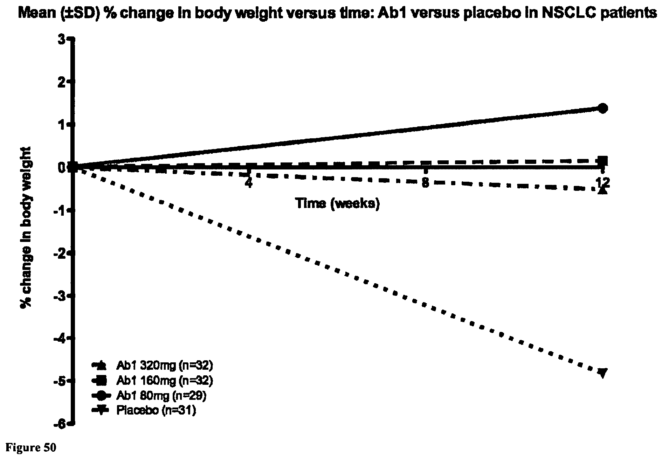

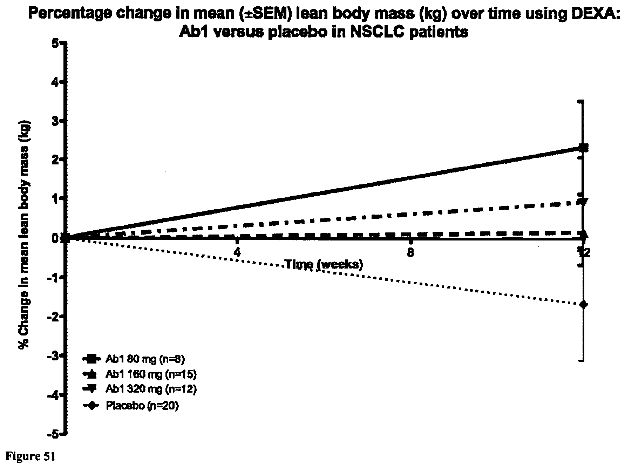

The averaged weight change data from each dosage concentration group (placebo, 80 mg, 160 mg, and 320 mg) of the Ab1 monoclonal antibody over 12 weeks is plotted in Figure 49. The average percent change in body weight from each dosage concentration is plotted in Fig. 50. The averaged lean body mass data for the dosage concentration groups is plotted in Figure 51.

### Fatigue

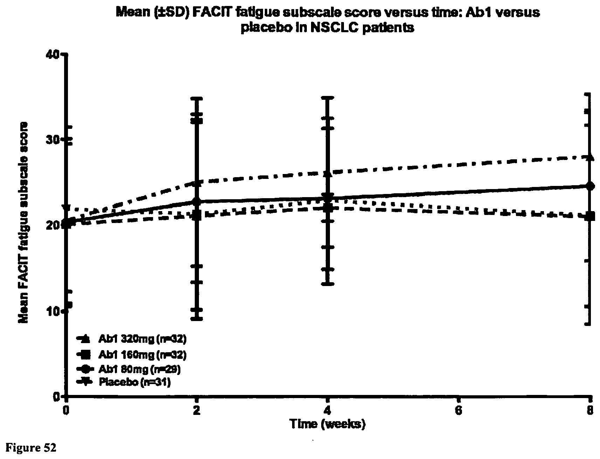

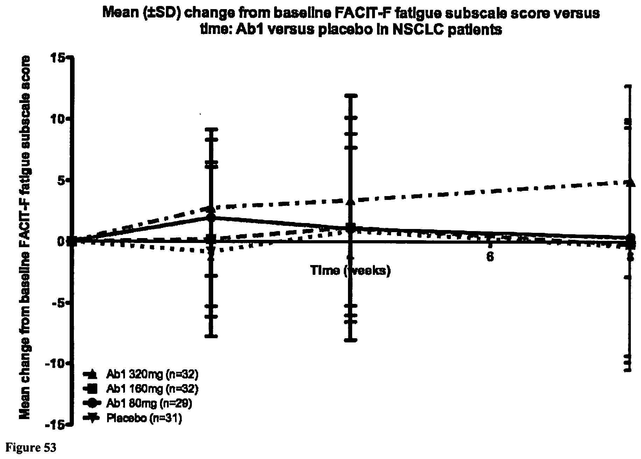

The averaged fatigue from each dosage concentration group (placebo, 80 mg, 160 mg, and 320 mg) of the Ab1 monoclonal antibody demonstrated increases in the mean Facit-F FS subscale score for some of the dosage concentration groups in the patient population over the period of 8 weeks (Fig. 52). The change from baseline Facit-F subscale score is plotted in Figure 53.

### Example 31 Ab1 Decreases D-dimer Levels in Patients with Advanced Cancer

### Introduction

D-dimer concentrations are recognized as useful diagnostic tools in predicting risks of thrombotic events in patients. (Adam et al., 113 Blood 2878-87 (2009)) Patients that are negative for D-dimer have a low probability for thrombosis. For example, D-dimer analysis can rule out suspected lower-extremity deep-vein thrombosis in patients. (Wells et al., 349 N. Engl. J. Med. 1227-35 (2003)) Clinical evaluation in combination with negative D-dimer test can effectively lower the instance of pulmonary embolism to 0.5%. (Van Belle et al., 295 JAMA 172-79 (2006); Kruip et al., 162 Arch. Intern. Med. 1631-35 (2002); Wells et al., 135 Ann. Intern. Med. 98-107 (2001))

D-dimer analysis may have utility in tracking the progress of treating coagulation disorders. One study indicated that anticoagulation treatment for acute venous thromboembolism resulted in a gradual decline in D-dimer concentrations. (Adam et al., 113 Blood 2878-87 (2009); Schutgens et al., 144 J. Lab. Clin. Med. 100-07 (2004)) This discovery led to the conclusion that D-dimer levels monitoring could be used to assess treatment responsiveness. (Adam et al., 113 Blood at 2883)

For patients with cancer, D-dimer analysis may have additional significance, as cancer increases the prevalence of thrombosis. (Adam et al., 113 Blood 2878-87 (2009)) One study with oncology patients indicated that D-dimer concentrations have a high negative predictive value and high sensitivity in diagnosing pulmonary embolism. (King et al., 247 Radiology 854-61 (2008)) Deep-vein thrombosis can similarly be excluded for cancer patients with low probability of developing deep-vein thrombosis and a negative test for D-dimer, although such a combination is less likely for oncology patients. (Lee et al., 123 Thromb. Res. 177-83 (2008)) A higher threshold for a negative D-dimer result may be necessary in cancer patients. (Righini et al., 95 Haemost. 715-19 (2006))

Accordingly, there remains a need in the art for methods and/or treatments of thrombosis that improve D-dimer concentrations in cancer patients and address elevated D-dimer states in cancer patients, particularly those with advanced cancers.

### Methods

One-hundred twenty-four patients with non-small cell lung cancer (NSCLC) were divided into 4 treatment groups. Patients in one group received one 1-hour intravenous (IV) infusion of either placebo (n=31), 80 mg (n=29), 160 mg (n=32), or 320 mg (n=32) of the Ab1 monoclonal antibody every 8 weeks over a 24 week duration for a total of 3 doses. Data on D-dimer concentration was collected for the first 8 weeks of treatment. D-dimer data concentration was quantitated by a D-dimer immunoturbidimetric assay. Briefly, the assay is based on the change in turbidity of a microparticle suspension that is measured by photometry. About 1.5 mL of patient sample sodium citrate plasma was collected and stored in a plastic collection tube. A suspension of latex microparticles, coated by covalent bonding with monoclonal antibodies specific for D-dimer, was mixed with the test plasma whose D-dimer level was to be assayed. Antigen-antibody reactions leading to an agglutination of the latex microparticles induced an increase in turbidity of the reaction medium. This increase in turbidity was reflected by an increase in absorbance, the latter being measured photometrically using a STAGO STA analyzer. The increase in absorbance was a function of the D-dimer level present in the test sample.

### Results

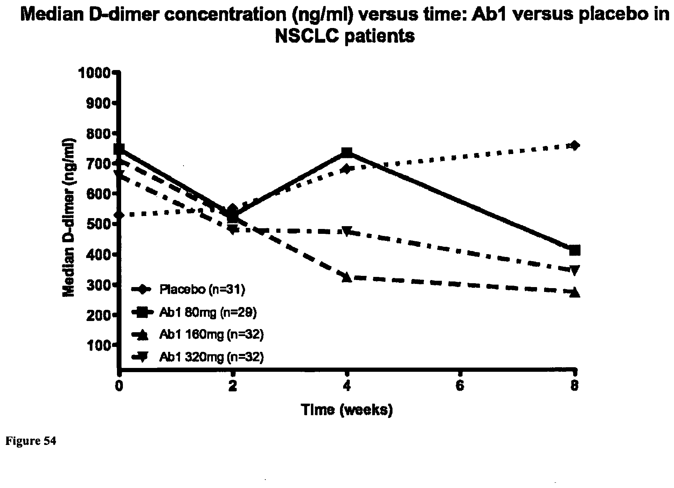

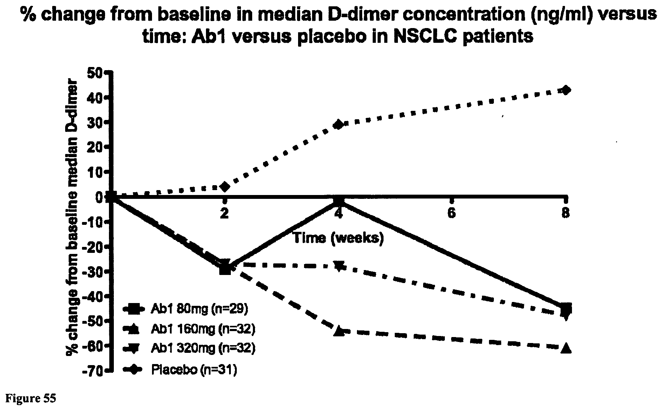

The averaged data for each dosage concentrations (placebo, 80 mg, 160 mg, and 320 mg) of the Ab1 monoclonal antibody are plotted in Figure 54. Error bars were omitted from the graph for clarity purposes. The percent change from baseline in D-dimer concentration is plotted in Figure 55. All dosage levels of Ab1 antibody demonstrated a drop in D-dimer levels over placebo over the period of 8 weeks.

### Example 32 Ab1 achieved ACR 20/50/70 in Patients with Rheumatoid Arthritis.

### Introduction

Rheumatoid arthritis is a chronic, systemic inflammatory disorder that principally attack synovium of joints. The disease causes painful and potentially disabling inflammation, with onset typically occurring between 40 and 50 years of age. Interpretation of drug treatment efficacy in rheumatoid arthritis is made difficult by the myriad of subjective and objective assessment tools made available over the years. The American College of Rheumatology ("ACR") released a standardized set of rheumatoid arthritis measures to facilitate evaluation of improvement of the disease in clinical trials. Felson et al., 36 Arthritis & Rheumatism 729-40 (1993).

### Methods

One-hundred twenty-seven patients with active rheumatoid arthritis and CRP ≥10 mg/L were divided into 4 treatment groups. Patients in one group received one 1-hour intravenous (IV) infusion of either placebo (n=33), 80 mg (n=32), 160 mg (n=34), or 320 mg (n=28) of the Ab1 monoclonal antibody, once at the start of the 16 week trial and again at week 8. Data on CRP concentration was collected every week for the first 4 weeks, every two weeks between weeks 4 and 12, and at the conclusion of the test at week 16.

Assessment under the standardized protocols from the American College of Rheumatology were employed in determining the percentage of improvement of patients during the clinical trial and conducted by a person trained in the ordinary art of evaluating rheumatoid arthritis. The evaluation was based upon activity measures, including tender joint count, swollen joint count, the patient's assessment of pain, the patient's and physician's global assessments of disease activity, and laboratory evaluation of either erythrocyte sedimentation rate or CRP level. *Id.* The patient's assessment of pain was based upon the Stanford Health Assessment Questionnaire Disability Index (HAQ DI). Patients that achieve a 20% increase in activity measures for rheumatoid arthritis during a clinical trial are categorized as achieving ACR 20. Similarly, patients achieving 50% and 70% improvements are categorized as ACR 50 and ACR 70, respectively.

### Results

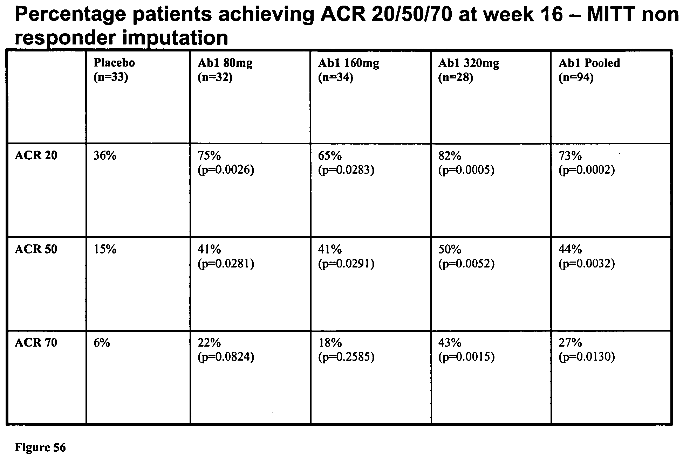

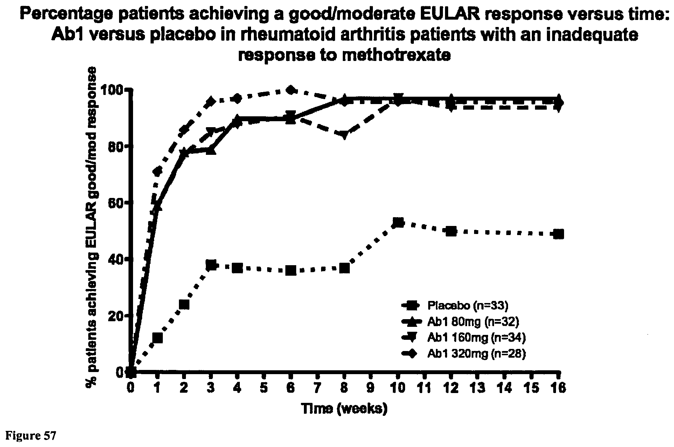

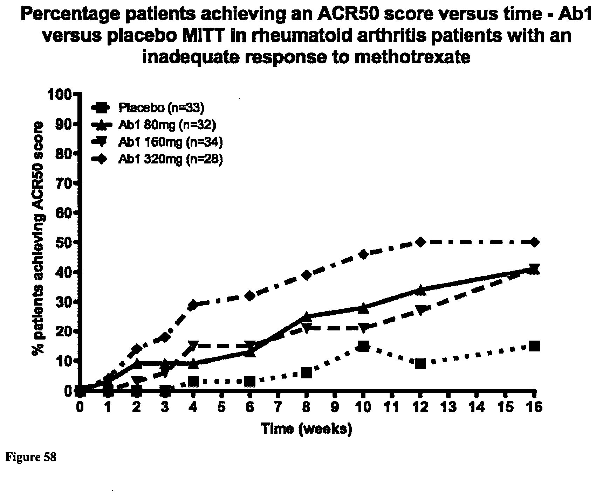

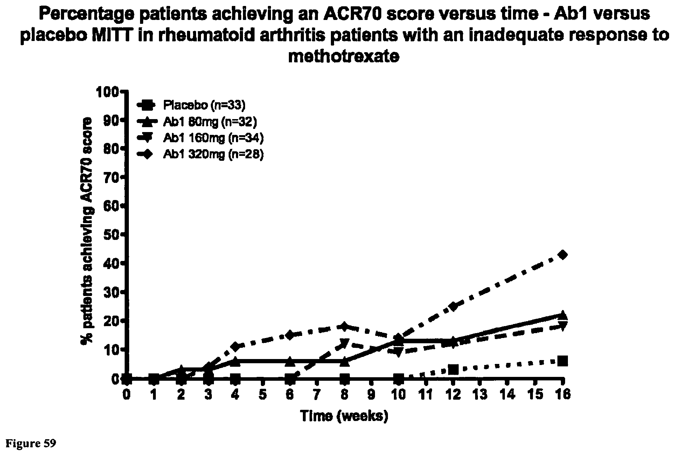

A significant portion of patients suffering from rheumatoid arthritis achieved ACR 20 or greater during the course of the study (Fig. 56). Patients observed rapid improvement in systems within the first 4 weeks of the study, as well as continued, steady improvement throughout the course of the 16 week evaluation (Figs. 57, 58, and 59). The greatest results where exhibited by patients receiving the 320 mg dosage level, with 43% achieving ACR 70 status during the study (Fig. 59).

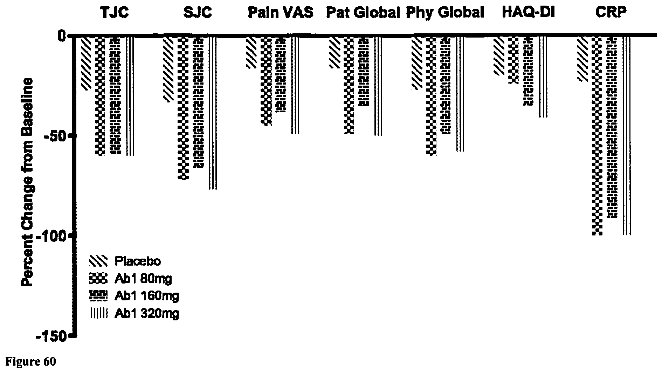

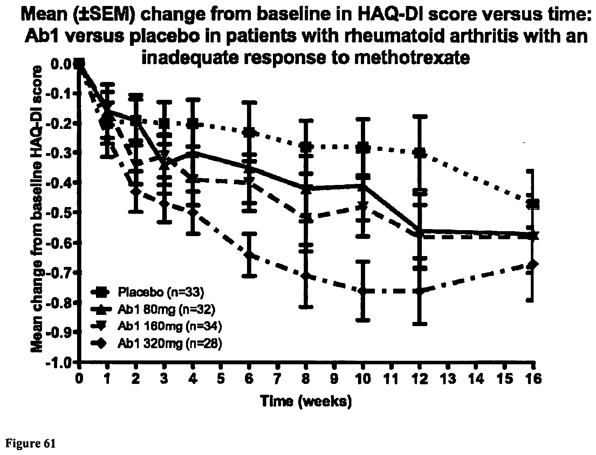

Analysis of the individual components of the ACR evaluation demonstrated gains in every component (Fig. 60). HAQ DI scores demonstrated clinically meaningful change over placebo during the course of the evaluation (Fig. 61). Serum CRP levels were greatly reduced in all patients studied (Fig. 41). The reduction in serum CRP levels was rapid, with immediate reduction in CRP levels relative to placebo within one week of Ab1 administration, and prolonged diminished levels continued at least until the final measurement was taken (up to sixteen weeks). In all cases, CRP levels fell to at or below the normal reference range (less than 5-6 mg/L) within one week. Thus, administration of Ab1 can cause a rapid and sustained improvement rheumatoid arthritis patients, as evidenced by the significant improvement in ACR scores during clinical evaluation, and presents an effective treatment regime.

### Example 33 Ab1 Achieved Improved DAS28 and EULAR Scores in Patients with Rheumatoid Arthritis

### Introduction

Rheumatoid arthritis is a chronic, systemic inflammatory disorder that principally attack synovium of joints. The disease causes painful and potentially disabling inflammation, with onset typically occurring between 40 and 50 years of age. Interpretation of drug treatment efficacy in rheumatoid arthritis is made difficult by the myriad of subjective and objective assessment tools made available over the years. The American College of Rheumatology ("ACR") released a standardized set of rheumatoid arthritis measures to facilitate evaluation of improvement of the disease in clinical trials. Felson et al., 36 Arthritis & Rheumatism 729-40 (1993).

Inflammatory activity associated with rheumatoid arthritis is measured using numerous variables through validated response criteria such as Disease Activity Score (DAS), DAS28 and EULAR. The DAS is a clinical index of rheumatoid arthritis disease activity that combines information from swollen joints, tender joints, the acute phase response, and general health. Fransen, J., et al., Clin. Exp. Rheumatol., 23 (Suppl. 39): S93-S99 (2005). The DAS 28 is an index similar to the original DAS, but utilizes a 28 tender joint count (range 0-28), a 28 swollen joint count (range 0-28), ESR (erythrocyte sedimentation rate), and an optional general health assessment on a visual analogue scale (range 0-100). *Id.* The European League against Rheumatism (EULAR) response criteria classify patients using the individual amount of change in the DAS and the DAS value (low, moderate, high) reached into one of the following classifications: Good; Moderate; or Non-Responders. *Id.*

### Methods

One-hundred twenty-seven patients with active rheumatoid arthritis were divided into 4 treatment groups. Patients in one group received one 1-hour intravenous (IV) infusion of either placebo (n=33), 80 mg (n=32), 160 mg (n=34), or 320 mg (n=28) of the Ab1 monoclonal antibody, once at the start of the 16 week trial and again at week 8. Data on the DAS28 and EULAR scores was collected every week for the first 4 weeks, every two weeks between weeks 4 and 12, and at the conclusion of the test at week 16. Assessment under the standardized DAS28 and EULAR protocols were employed in determining the respective scores of patients during the clinical trial and conducted by a person trained in the ordinary art of evaluating rheumatoid arthritis.

### Results

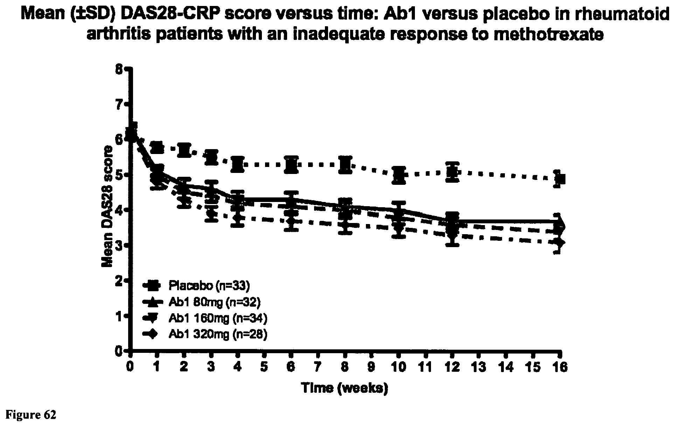

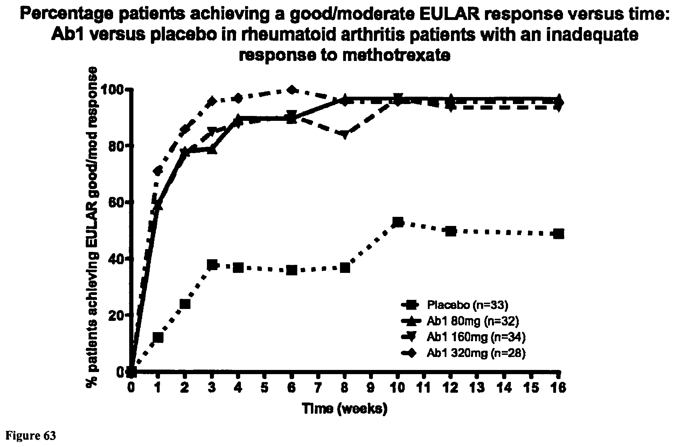

Patients receiving 80 mg, 160 mg or 320 mg of Ab1 demonstrated improved DAS28 scores relative to those patients receiving placebo over the course of 16 weeks, as presented in Fig. 62 as a mean change from the baseline DAS28 score. Furthermore, a significant percentage of patients receiving 80 mg, 160 mg or 320 mg of Ab1 achieved "Good" or "Moderate" classifications relative to those patients receiving placebo over the course of 16 weeks. (Fig. 63).

Thus, administration of Ab1 can result in improved DAS28 and EULAR scores in rheumatoid arthritis when compared to those patients receiving placebo.

### SEQUENCE LISTING

The biological sequences referenced herein are provided below:

**SEQ ID NO: 4**
   QASQSINNELS
**SEQ ID NO: 5**
   RASTLAS
**SEQ ID NO: 6**
   QQGYSLRNIDNA
**SEQ ID NO: 7**
   NYYVT
**SEQ ID NO: 8**
   IIYGSDETAYATWAIG
**SEQ ID NO: 9**
   DDSSDWDAKFNL
**SEQ ID NO: 12**
   CAGGCCAGTCAGAGCATTAACAATGAATTATCC
**SEQ ID NO: 13**
   AGGGCATCCACTCTGGCATCT
**SEQ ID NO: 14**
   CAACAGGGTTATAGTCTGAGGAATATTGATAATGCT
**SEQ ID NO: 15**
   AACTACTACGTGACC
**SEQ ID NO: 16**
   ATCATTTATGGTAGTGATGAAACGGCCTACGCGACCTGGGCGATAGGC
**SEQ ID NO: 17**
   GATGATAGTAGTGACTGGGATGCAAAATTTAACTTG
**SEQ ID NO: 23**
   QASETIYSWLS
**SEQ ID NO: 24**
   QASDLAS
**SEQ ID NO: 25**
   QQGYSGSNVDNV
**SEQ ID NO: 26**
   DHAMG
**SEQ ID NO: 27**
   FINSGGSARYASWAEG
**SEQ ID NO: 28**
   GGAVWSIHSFDP
**SEQ ID NO: 31**
   CAGGCCAGTGAGACCATTTACAGTTGGTTATCC
**SEQ ID NO: 32**
   CAGGCATCCGATCTGGCATCT
**SEQ ID NO: 33**
   CAACAGGGTTATAGTGGTAGTAATGTTGATAATGTT
**SEQ ID NO: 34**
   GACCATGCAATGGGC
**SEQ ID NO: 35**
   TTCATTAATAGTGGTGGTAGCGCACGCTACGCGAGCTGGGCAGAAGGC
**SEQ ID NO: 36**
   GGGGGTGCTGTTTGGAGTATTCATAGTTTTGATCCC
**SEQ ID NO: 39**
   QASQSVYDNNYLS
**SEQ ID NO: 40**
   GASTLAS
**SEQ ID NO: 41**
   AGVYDDDSDNA
**SEQ ID NO: 42**
   VYYMN
**SEQ ID NO: 43**
   FITMSDNINYASWAKG
**SEQ ID NO: 44**
   SRGWGTMGRLDL
**SEQ ID NO: 47**
   CAGGCCAGTCAGAGTGTTTATGACAACAACTACTTATCC
**SEQ ID NO: 48**
   GGTGCATCCACTCTGGCATCT
**SEQ ID NO: 49**
   GCAGGCGTTTATGATGATGATAGTGATAATGCC
**SEQ ID NO: 50**
   GTCTACTACATGAAC
**SEQ ID NO: 51**
   TTCATTACAATGAGTGATAATATAAATTACGCGAGCTGGGCGAAAGGC
**SEQ ID NO: 52**
   AGTCGTGGCTGGGGTACAATGGGTCGGTTGGATCTC
**SEQ ID NO: 55**
   QASQSVYENNYLS
**SEQ ID NO: 56**
   GASTLDS
**SEQ ID NO: 57**
   AGVYDDDSDDA
**SEQ ID NO: 58**
   AYYMN
**SEQ ID NO: 59**
   FITLNNNVAYANWAKG
**SEQ ID NO: 60**
   SRGWGAMGRLDL
**SEQ ID NO: 63**
   CAGGCCAGTCAGAGTGTTTATGAGAACAACTATTTATCC
**SEQ ID NO: 64**
   GGTGCATCCACTCTGGATTCT
**SEQ ID NO: 65**
   GCAGGCGTTTATGATGATGATAGTGATGATGCC
**SEQ ID NO: 66**
   GCCTACTACATGAAC
**SEQ ID NO: 67**
   TTCATTACTCTGAATAATAATGTAGCTTACGCGAACTGGGCGAAAGGC
**SEQ ID NO: 68**
   AGTCGTGGCTGGGGTGCAATGGGTCGGTTGGATCTC
**SEQ ID NO: 71**
   QASQSVDDNNWLG
**SEQ ID NO: 72**
   SASTLAS
**SEQ ID NO: 73**
   AGGFSGNIFA
**SEQ ID NO: 74**
   SYAMS
**SEQ ID NO: 75**
   IIGGFGTTYYATWAKG
**SEQ ID NO: 76**
   GGPGNGGDI
**SEQ ID NO: 79**
   CAGGCCAGTCAGAGTGTTGATGATAACAACTGGTTAGGC
**SEQ ID NO: 80**
   TCTGCATCCACTCTGGCATCT
**SEQ ID NO: 81**
   GCAGGCGGTTTTAGTGGTAATATCTTTGCT
**SEQ ID NO: 82**
   AGCTATGCAATGAGC
**SEQ ID NO: 83**
   ATCATTGGTGGTTTTGGTACCACATACTACGCGACCTGGGCGAAAGGC
**SEQ ID NO: 84**
   GGTGGTCCTGGTAATGGTGGTGACATC
**SEQ ID NO: 87**
   QSSQSVYNNFLS
**SEQ ID NO: 88**
   QASKLAS
**SEQ ID NO: 89**
   LGGYDDDADNA
**SEQ ID NO: 90**
   DYAMS
**SEQ ID NO: 91**
   IIYAGSGSTWYASWAKG
**SEQ ID NO: 92**
   DGYDDYGDFDRLDL
**SEQ ID NO: 95**
   CAGTCCAGTCAGAGTGTTTATAATAATTTCTTATCG
**SEQ ID NO: 96**
   CAGGCATCCAAACTGGCATCT
**SEQ ID NO: 97**
   CTAGGCGGTTATGATGATGATGCTGATAATGCT
**SEQ ID NO: 98**
   GACTATGCAATGAGC
**SEQ ID NO: 99**
   ATCATTTATGCTGGTAGTGGTAGCACATGGTACGCGAGCTGGGCGAAAGGC
**SEQ ID NO: 100**
   GATGGATACGATGACTATGGTGATTTCGATCGATTGGATCTC
**SEQ ID NO: 103**
   QASQSINNELS
**SEQ ID NO: 104**
   RASTLAS
**SEQ ID NO: 105**
   QQGYSLRNIDNA
**SEQ ID NO: 106**
   NYYMT
**SEQ ID NO: 107**
   MIYGSDETAYANWAIG
**SEQ ID NO: 108**
   DDSSDWDAKFNL
**SEQ ID NO: 111**
   CAGGCCAGTCAGAGCATTAACAATGAATTATCC
**SEQ ID NO: 112**
   AGGGCATCCACTCTGGCATCT
**SEQ ID NO: 113**
   CAACAGGGTTATAGTCTGAGGAATATTGATAATGCT
**SEQ ID NO: 114**
   AACTACTACATGACC
**SEQ ID NO: 115**
   ATGATTTATGGTAGTGATGAAACAGCCTACGCGAACTGGGCGATAGGC
**SEQ ID NO: 116**
   GATGATAGTAGTGACTGGGATGCAAAATTTAACTTG
**SEQ ID NO: 120**
   IIYGSDETAYATSAIG
**SEQ ID NO: 121**
   MIYGSDETAYANSAIG
**SEQ ID NO: 124**
   QSSQSVGNNQDLS
**SEQ ID NO: 125**
   EISKLES
**SEQ ID NO: 126**
   LGGYDDDADNA
**SEQ ID NO: 127**
   SRTMS
**SEQ ID NO: 128**
   YIWSGGSTYYATWAKG
**SEQ ID NO: 129**
   LGDTGGHAYATRLNL
**SEQ ID NO: 132**
   CAGTCCAGTCAGAGTGTTGGTAATAACCAGGACTTATCC
**SEQ ID NO: 133**
   GAAATATCCAAACTGGAATCT
**SEQ ID NO: 134**
   CTAGGCGGTTATGATGATGATGCTGATAATGCT
**SEQ ID NO: 135**
   AGTCGTACAATGTCC
**SEQ ID NO: 136**
   TACATTTGGAGTGGTGGTAGCACATACTACGCGACCTGGGCGAAAGGC
**SEQ ID NO: 137**
   TTGGGCGATACTGGTGGTCACGCTTATGCTACTCGCTTAAATCTC
**SEQ ID NO: 140**
   QSSQSVYSNKYLA
**SEQ ID NO: 141**
   WTSKLAS
**SEQ ID NO: 142**
   LGAYDDDADNA
**SEQ ID NO: 143**
   GGYMT
**SEQ ID NO: 144**
   ISYDSGSTYYASWAKG
**SEQ ID NO: 145**
   SLKYPTVTSDDL
**SEQ ID NO: 148**
   CAGTCCAGTCAGAGTGTTTATAGTAATAAGTACCTAGCC
**SEQ ID NO: 149**
   TGGACATCCAAACTGGCATCT
**SEQ ID NO: 150**
   CTAGGCGCTTATGATGATGATGCTGATAATGCT
**SEQ ID NO: 151**
   GGCGGCTACATGACC
**SEQ ID NO: 152**
   ATCAGTTATGATAGTGGTAGCACATACTACGCGAGCTGGGCGAAAGGC
**SEQ ID NO: 153**
   TCACTAAAATATCCTACTGTTACTTCTGATGACTTG
**SEQ ID NO: 156**
   QSSQSVYNNNDLA
**SEQ ID NO: 157**
   YASTLAS
**SEQ ID NO: 158**
   LGGYDDDADNA
**SEQ ID NO: 159**
   SNTIN
**SEQ ID NO: 160**
   YIWSGGSTYYASWVNG
**SEQ ID NO: 161**
   GGYASGGYPYATRLDL
**SEQ ID NO: 164**
   CAGTCCAGTCAGAGTGTTTATAATAATAACGACTTAGCC
**SEQ ID NO: 165**
   TATGCATCCACTCTGGCATCT
**SEQ ID NO: 166**
   CTAGGCGGTTATGATGATGATGCTGATAATGCT
**SEQ ID NO: 167**
   AGCAATACAATAAAC
**SEQ ID NO: 168**
   TACATTTGGAGTGGTGGTAGTACATACTACGCGAGCTGGGTGAATGGT
**SEQ ID NO: 169**
   GGGGGTTACGCTAGTGGTGGTTATCCTTATGCCACTCGGTTGGATCTC
**SEQ ID NO: 172**
   QSSQSVYNNDYLS
**SEQ ID NO: 173**
   GASKLAS
**SEQ ID NO: 174**
   LGDYDDDADNT
**SEQ ID NO: 175**
   TNYYLS
**SEQ ID NO: 176**
   IIYPSGNTYCAKWAKG
**SEQ ID NO: 177**
   NYGGDESL
**SEQ ID NO: 180**
   CAGTCCAGTCAGAGTGTTTATAATAACGACTACTTATCC
**SEQ ID NO: 181**
   GGTGCTTCCAAACTGGCATCT
**SEQ ID NO: 182**
   CTGGGCGATTATGATGATGATGCTGATAATACT
**SEQ ID NO: 183**
   ACCAACTACTACCTGAGC
**SEQ ID NO: 184**
   ATCATTTATCCTAGTGGTAACACATATTGCGCGAAGTGGGCGAAAGGC
**SEQ ID NO: 185**
   AATTATGGTGGTGATGAAAGTTTG
**SEQ ID NO: 188**
   QASETIGNALA
**SEQ ID NO: 189**
   KASKLAS
**SEQ ID NO: 190**
   QWCYFGDSV
**SEQ ID NO: 191**
   SGYYMC
**SEQ ID NO: 192**
   CIFTITTNTYYASWAKG
**SEQ ID NO: 193**
   GIYSDNNYYAL
**SEQ ID NO: 196**
   CAGGCCAGTGAGACCATTGGCAATGCATTAGCC
**SEQ ID NO: 197**
   AAGGCATCCAAACTGGCATCT
**SEQ ID NO: 198**
   CAATGGTGTTATTTTGGTGATAGTGTT
**SEQ ID NO: 199**
   AGCGGCTACTACATGTGC
**SEQ ID NO: 200**
   TGTATTTTCACTATTACTACTAACACTTACTACGCGAGCTGGGCGAAAGGC
**SEQ ID NO: 201**
   GGGATTTATTCTGATAATAATTATTATGCCTTG
**SEQ ID NO: 204**
   QASESIGNALA
**SEQ ID NO: 205**
   KASTLAS
**SEQ ID NO: 206**
   QWCYFGDSV
**SEQ ID NO: 207**
   SGYYMC
**SEQ ID NO: 208**
   CIFTITDNTYYANWAKG
**SEQ ID NO: 209**
   GIYSTDNYYAL
**SEQ ID NO: 212**
   CAGGCCAGTGAGAGCATTGGCAATGCATTAGCC
**SEQ ID NO: 213**
   AAGGCATCCACTCTGGCATCT
**SEQ ID NO: 214**
   CAATGGTGTTATTTTGGTGATAGTGTT
**SEQ ID NO: 215**
   AGCGGCTACTACATGTGC
**SEQ ID NO: 216**
   TGCATTTTTACTATTACTGATAACACTTACTACGCGAACTGGGCGAAAGGC
**SEQ ID NO: 217**
   GGGATTTATTCTACTGATAATTATTATGCCTTG
**SEQ ID NO: 220**
   QASQSVSSYLN
**SEQ ID NO: 221**
   RASTLES
**SEQ ID NO: 222**
   QCTYGTSSSYGAA
**SEQ ID NO: 223**
   SNAIS
**SEQ ID NO: 224**
   IISYSGTTYYASWAKG
**SEQ ID NO: 225**
   DDPTTVMVMLIPFGAGMDL
**SEQ ID NO: 228**
   CAGGCCAGTCAGAGCGTTAGTAGCTACTTAAAC
**SEQ ID NO: 229**
   AGGGCATCCACTCTGGAATCT
**SEQ ID NO: 230**
   CAATGTACTTATGGTACTAGTAGTAGTTATGGTGCTGCT
**SEQ ID NO: 231**
   AGCAATGCAATAAGC
**SEQ ID NO: 232**
   ATCATTAGTTATAGTGGTACCACATACTACGCGAGCTGGGCGAAAGGC
**SEQ ID NO: 233**
   GATGACCCTACGACAGTTATGGTTATGTTGATACCTTTTGGAGCCGGCATGGACCTC
**SEQ ID NO: 236**
   QASQSVYKNNYLS
**SEQ ID NO: 237**
   SASTLDS
**SEQ ID NO: 238**
   LGSYDCSSGDCYA
**SEQ ID NO: 239**
   SYWMC
**SEQ ID NO: 240**
   CIVTGNGNTYYANWAKG
**SEQ ID NO: 241**
   AYDL
**SEQ ID NO: 244**
   CAGGCCAGTCAGAGTGTTTATAAGAACAACTACTTATCC
**SEQ ID NO: 245**
   TCTGCATCGACTCTAGATTCT
**SEQ ID NO: 246**
   CTAGGCAGTTATGATTGTAGTAGTGGTGATTGTTATGCT
**SEQ ID NO: 247**
   AGCTACTGGATGTGC
**SEQ ID NO: 248**
   TGCATTGTTACTGGTAATGGTAACACTTACTACGCGAACTGGGCGAAAGGC
**SEQ ID NO: 249**
   GCCTATGACTTG
**SEQ ID NO: 252**
   QASQSVYDNNYLS
**SEQ ID NO: 253**
   GASTLAS
**SEQ ID NO: 254**
   AGVFNDDSDDA
**SEQ ID NO: 255**
   AYYMS
**SEQ ID NO: 256**
   FITLSDHISYARWAKG
**SEQ ID NO: 257**
   SRGWGAMGRLDL
**SEQ ID NO: 260**
   CAGGCCAGTCAGAGTGTTTATGACAACAACTATTTATCC
**SEQ ID NO: 261**
   GGTGCATCCACTCTGGCATCT
**SEQ ID NO: 262**
   GCAGGCGTTTTTAATGATGATAGTGATGATGCC
**SEQ ID NO: 263**
   GCATACTATATGAGC
**SEQ ID NO: 264**
   TTCATTACTCTGAGTGATCATATATCTTACGCGAGGTGGGCGAAAGGC
**SEQ ID NO: 265**
   AGTCGTGGCTGGGGTGCAATGGGTCGGTTGGATCTC
**SEQ ID NO: 268**
   QASQSVYNNKNLA
**SEQ ID NO: 269**
   WASTLAS
**SEQ ID NO: 270**
   LGVFDDDADNA
**SEQ ID NO: 271**
   SYSMT
**SEQ ID NO: 272**
   VIGTSGSTYYATWAKG
**SEQ ID NO: 273**
   SLSSITFL
**SEQ ID NO: 276**
   CAGGCCAGTCAGAGTGTTTATAACAACAAAAATTTAGCC
**SEQ ID NO: 277**
   TGGGCATCCACTCTGGCATCT
**SEQ ID NO: 278**
   CTAGGCGTTTTTGATGATGATGCTGATAATGCT
**SEQ ID NO: 279**
   AGCTACTCCATGACC
**SEQ ID NO: 280**
   GTCATTGGTACTAGTGGTAGCACATACTACGCGACCTGGGCGAAAGGC
**SEQ ID NO: 281**
   AGTCTTTCTTCTATTACTTTCTTG
**SEQ ID NO: 284**
   QASQNIYRYLA
**SEQ ID NO: 285**
   LASTLAS
**SEQ ID NO: 286**
   QSYYSSNSVA
**SEQ ID NO: 287**
   SGYWIC
**SEQ ID NO: 288**
   CIYTGSSGSTFYASWAKG
**SEQ ID NO: 289**
   GYSGFGYFKL
**SEQ ID NO: 290**
**SEQ ID NO: 292**
   CAGGCCAGTCAGAACATTTATAGATACTTAGCC
**SEQ ID NO: 293**
   CTGGCATCTACTCTGGCATCT
**SEQ ID NO: 294**
   CAAAGTTATTATAGTAGTAATAGTGTCGCT
**SEQ ID NO: 295**
   AGCGGCTACTGGATATGC
**SEQ ID NO: 296**
   TGCATTTATACTGGTAGTAGTGGTAGCACTTTTTACGCGAGTTGGGCGAAAGGC
**SEQ ID NO: 297**
   GGTTATAGTGGCTTTGGTTACTTTAAGTTG
**SEQ ID NO: 300**
   QASEDIYRLLA
**SEQ ID NO: 301**
   DSSDLAS
**SEQ ID NO: 302**
   QQAWSYSDIDNA
**SEQ ID NO: 303**
   SYYMS
**SEQ ID NO: 304**
   IITTSGNTFYASWAKG
**SEQ ID NO: 305**
   TSDIFYYRNL
**SEQ ID NO: 308**
   CAGGCCAGTGAGGACATTTATAGGTTATTGGCC
**SEQ ID NO: 309**
   GATTCATCCGATCTGGCATCT
**SEQ ID NO: 310**
   CAACAGGCTTGGAGTTATAGTGATATTGATAATGCT
**SEQ ID NO: 311**
   AGCTACTACATGAGC
**SEQ ID NO: 312**
   ATCATTACTACTAGTGGTAATACATTTTACGCGAGCTGGGCGAAAGGC
**SEQ ID NO: 313**
   ACTTCTGATATTTTTTATTATCGTAACTTG
**SEQ ID NO: 316**
   QSSQSVYNDMDLA
**SEQ ID NO: 317**
   SASTLAS
**SEQ ID NO: 318**
   LGAFDDDADNT
**SEQ ID NO: 319**
   RHAIT
**SEQ ID NO: 320**
   CIWSGGSTYYATWAKG
**SEQ ID NO: 321**
   VIGDTAGYAYFTGLDL
**SEQ ID NO: 324**
   CAGTCCAGTCAGAGTGTTTATAATGACATGGACTTAGCC
**SEQ ID NO: 325**
   TCTGCATCCACTCTGGCATCT
**SEQ ID NO: 326**
   CTAGGCGCTTTTGATGATGATGCTGATAATACT
**SEQ ID NO: 327**
   AGGCATGCAATAACC
**SEQ ID NO: 328**
   TGCATTTGGAGTGGTGGTAGCACATACTACGCGACCTGGGCGAAAGGC
**SEQ ID NO: 329**
   GTCATTGGCGATACTGCTGGTTATGCTTATTTTACGGGGCTTGACTTG
**SEQ ID NO: 332**
   QASQSVYNWLS
**SEQ ID NO: 333**
   TASSLAS
**SEQ ID NO: 334**
   QQGYTSDVDNV
**SEQ ID NO: 335**
   SYAMG
**SEQ ID NO: 336**
   IISSSGSTYYATWAKG
**SEQ ID NO: 337**
   GGAGSGGVWLLDGFDP
**SEQ ID NO: 340**
   CAGGCCAGTCAGAGTGTTTATAATTGGTTATCC
**SEQ ID NO: 341**
   ACTGCATCCAGTCTGGCATCT
**SEQ ID NO: 342**
   CAACAGGGTTATACTAGTGATGTTGATAATGTT
**SEQ ID NO: 343**
   AGCTATGCAATGGGC
**SEQ ID NO: 344**
   ATCATTAGTAGTAGTGGTAGCACATACTACGCGACCTGGGCGAAAGGC
**SEQ ID NO: 345**
   GGGGGTGCTGGTAGTGGTGGTGTTTGGCTGCTTGATGGTTTTGATCCC
**SEQ ID NO: 348**
   QASENIYNWLA
**SEQ ID NO: 349**
   TVGDLAS
**SEQ ID NO: 350**
   QQGYSSSYVDNV
**SEQ ID NO: 351**
   DYAVG
**SEQ ID NO: 352**
   YIRSSGTTAYATWAKG
**SEQ ID NO: 353**
   GGAGSSGVWILDGFAP
**SEQ ID NO: 356**
   CAGGCCAGTGAGAACATTTATAATTGGTTAGCC
**SEQ ID NO: 357**
   ACTGTAGGCGATCTGGCATCT
**SEQ ID NO: 358**
   CAACAGGGTTATAGTAGTAGTTATGTTGATAATGTT
**SEQ ID NO: 359**
   GACTATGCAGTGGGC
**SEQ ID NO: 360**
   TACATTCGTAGTAGTGGTACCACAGCCTACGCGACCTGGGCGAAAGGC
**SEQ ID NO: 361**
   GGGGGTGCTGGTAGTAGTGGTGTGTGGATCCTTGATGGTTTTGCTCCC
**SEQ ID NO: 364**
   QASQSVYQNNYLS
**SEQ ID NO: 365**
   GAATLAS
**SEQ ID NO: 366**
   AGAYRDVDS
**SEQ ID NO: 367**
   STYYIY
**SEQ ID NO: 368**
   CIDAGSSGSTYYATWVNG
**SEQ ID NO: 369**
   WDYGGNVGWGYDL
**SEQ ID NO: 372**
   CAGGCCAGTCAGAGTGTTTATCAGAACAACTACTTATCC
**SEQ ID NO: 373**
   GGTGCGGCCACTCTGGCATCT
**SEQ ID NO: 374**
   GCAGGCGCTTATAGGGATGTGGATTCT
**SEQ ID NO: 375**
   AGTACCTACTACATCTAC
**SEQ ID NO: 376**
   TGTATTGATGCTGGTAGTAGTGGTAGCACTTACTACGCGACCTGGGTGAATGGC
**SEQ ID NO: 377**
   TGGGATTATGGTGGTAATGTTGGTTGGGGTTATGACTTG
**SEQ ID NO: 380**
   QASQSISSYLA
**SEQ ID NO: 381**
   RASTLAS
**SEQ ID NO: 382**
   QSYYDSVSNP
**SEQ ID NO: 383**
   TYWFMC
**SEQ ID NO: 384**
   CIYTGSSGSTFYASWVNG
**SEQ ID NO: 385**
   GYSGYGYFKL
**SEQ ID NO: 388**
   CAGGCCAGTCAGAGCATTAGTAGTTACTTAGCC
**SEQ ID NO: 389**
   AGGGCGTCCACTCTGGCATCT
**SEQ ID NO: 390**
   CAAAGCTATTATGATAGTGTTTCAAATCCT
**SEQ ID NO: 391**
   ACCTACTGGTTCATGTGC
**SEQ ID NO: 392**
   TGTATTTATACTGGTAGTAGTGGTTCCACTTTCTACGCGAGCTGGGTGAATGGC
**SEQ ID NO: 393**
   GGTTATAGTGGTTATGGTTATTTTAAGTTG
**SEQ ID NO: 396**
   QASQSVYKNNQLS
**SEQ ID NO: 397**
   GASALAS
**SEQ ID NO: 398**
   AGAITGSIDTDG
**SEQ ID NO: 399**
   SSYFIC
**SEQ ID NO: 400**
   CIYGGDGSTYYASW AKG
**SEQ ID NO: 401**
   EWAYSQGYFGAFDL
**SEQ ID NO: 404**
   CAGGCCAGTCAGAGTGTTTATAAGAACAACCAATTATCC
**SEQ ID NO: 405**
   GGTGCATCGGCTCTGGCATCT
**SEQ ID NO: 406**
   GCAGGCGCTATTACTGGTAGTATTGATACGGATGGT
**SEQ ID NO: 407**
   AGCAGCTACTTCATTTGC
**SEQ ID NO: 408**
   TGCATTTATGGTGGTGATGGCAGCACATACTACGCGAGCTGGGCGAAAGGC
**SEQ ID NO: 409**
   GAATGGGCATATAGTCAAGGTTATTTTGGTGCTTTTGATCTC
**SEQ ID NO: 412**
   QASEDISSYLA
**SEQ ID NO: 413**
   AASNLES
**SEQ ID NO: 414**
   QCTYGTISISDGNA
**SEQ ID NO: 415**
   SYFMT
**SEQ ID NO: 416**
   FINPGGSAYYASWVKG
**SEQ ID NO: 417**
   VLIVSYGAFTI
**SEQ ID NO: 420**
   CAGGCCAGTGAGGATATTAGTAGCTACTTAGCC
**SEQ ID NO: 421**
   GCTGCATCCAATCTGGAATCT
**SEQ ID NO: 422**
   CAATGTACTTATGGTACTATTTCTATTAGTGATGGTAATGCT
**SEQ ID NO: 423**
   AGCTACTTCATGACC
**SEQ ID NO: 424**
   TTCATTAATCCTGGTGGTAGCGCTTACTACGCGAGCTGGGTGAAAGGC
**SEQ ID NO: 425**
   GTTCTGATTGTTTCTTATGGAGCCTTTACCATC
**SEQ ID NO: 428**
   QASEDIESYLA
**SEQ ID NO: 429**
   GASNLES
**SEQ ID NO: 430**
   QCTYGIISISDGNA
**SEQ ID NO: 431**
   SYFMT
**SEQ ID NO: 432**
   FMNTGDNAYYASWAKG
**SEQ ID NO: 433**
   VLVVAYGAFNI
**SEQ ID NO: 436**
   CAGGCCAGTGAGGACATTGAAAGCTATCTAGCC
**SEQ ID NO: 437**
   GGTGCATCCAATCTGGAATCT
**SEQ ID NO: 438**
   CAATGCACTTATGGTATTATTAGTATTAGTGATGGTAATGCT
**SEQ ID NO: 439**
   AGCTACTTCATGACC
**SEQ ID NO: 440**
   TTCATGAATACTGGTGATAACGCATACTACGCGAGCTGGGCGAAAGGC
**SEQ ID NO: 441**
   GTTCTTGTTGTTGCTTATGGAGCCTTTAACATC
**SEQ ID NO: 444**
   QSSKSVMNNNYLA
**SEQ ID NO: 445**
   GASNLAS
**SEQ ID NO: 446**
   QGGYTGYSDHGT
**SEQ ID NO: 447**
   SYPMN
**SEQ ID NO: 448**
   FINTGGTIVYASWAKG
**SEQ ID NO: 449**
   GSYVSSGYAYYFNV
**SEQ ID NO: 452**
   CAGTCCAGTAAGAGTGTTATGAATAACAACTACTTAGCC
**SEQ ID NO: 453**
   GGTGCATCCAATCTGGCATCT
**SEQ ID NO: 454**
   CAAGGCGGTTATACTGGTTATAGTGATCATGGGACT
**SEQ ID NO: 455**
   AGCTATCCAATGAAC
**SEQ ID NO: 456**
   TTCATTAATACTGGTGGTACCATAGTCTACGCGAGCTGGGCAAAAGGC
**SEQ ID NO: 457**
   GGCAGTTATGTTTCATCTGGTTATGCCTACTATTTTAATGTC
**SEQ ID NO: 460**
   QSSQSVYNNNWLS
**SEQ ID NO: 461**
   KASTLAS
**SEQ ID NO: 462**
   AGGYLDSVI
**SEQ ID NO: 463**
   TYSIN
**SEQ ID NO: 464**
   IIANSGTTFYANWAKG
**SEQ ID NO: 465**
   ESGMYNEYGKFNI
**SEQ ID NO: 468**
   CAGTCCAGTCAGAGTGTTTATAATAACAACTGGTTATCC
**SEQ ID NO: 469**
   AAGGCATCCACTCTGGCATCT
**SEQ ID NO: 470**
   GCGGGCGGTTATCTTGATAGTGTTATT
**SEQ ID NO: 471**
   ACCTATTCAATAAAC
**SEQ ID NO: 472**
   ATCATTGCTAATAGTGGTACCACATTCTACGCGAACTGGGCGAAAGGC
**SEQ ID NO: 473**
   GAGAGTGGAATGTACAATGAATATGGTAAATTTAACATC
**SEQ ID NO: 476**
   QASENIYSFLA
**SEQ ID NO: 477**
   KASTLAS
**SEQ ID NO: 478**
   QQGATVYDIDNN
**SEQ ID NO: 479**
   AYAMI
**SEQ ID NO: 480**
   IIYPNGITYYANWAKG
**SEQ ID NO: 481**
   DAESSKNAYWGYFNV
**SEQ ID NO: 484**
   CAGGCCAGTGAGAACATTTATAGCTTTTTGGCC
**SEQ ID NO: 485**
   AAGGCTTCCACTCTGGCATCT
**SEQ ID NO: 486**
   CAACAGGGTGCTACTGTGTATGATATTGATAATAAT
**SEQ ID NO: 487**
   GCCTATGCAATGATC
**SEQ ID NO: 488**
   ATCATTTATCCTAATGGTATCACATACTACGCGAACTGGGCGAAAGGC
**SEQ ID NO: 489**
   GATGCAGAAAGTAGTAAGAATGCTTATTGGGGCTACTTTAACGTC
**SEQ ID NO: 492**
   QASENIYSFLA
**SEQ ID NO: 493**
   RASTLAS
**SEQ ID NO: 494**
   QQGATVYDIDNN
**SEQ ID NO: 495**
   AYAMI
**SEQ ID NO: 496**
   IIYPNGITYYANWAKG
**SEQ ID NO: 497**
   DAESSKNAYWGYFNV
**SEQ ID NO: 500**
   CAGGCCAGTGAGAACATTTATAGCTTTTTGGCC
**SEQ ID NO: 501**
   AGGGCTTCCACTCTGGCATCT
**SEQ ID NO: 502**
   CAACAGGGTGCTACTGTGTATGATATTGATAATAAT
**SEQ ID NO: 503**
   GCCTATGCAATGATC
**SEQ ID NO: 504**
   ATCATTTATCCTAATGGTATCACATACTACGCGAACTGGGCGAAAGGC
**SEQ ID NO: 505**
   GATGCAGAAAGTAGTAAGAATGCTTATTGGGGCTACTTTAACGTC
**SEQ ID NO: 508**
   QASESVFNNMLS
**SEQ ID NO: 509**
   DASDLAS
**SEQ ID NO: 510**
   AGYKSDSNDGDNV
**SEQ ID NO: 511**
   RNSIT
**SEQ ID NO: 512**
   IITGSGRTYYANWAKG
**SEQ ID NO: 513**
   GHPGLGSGNI
**SEQ ID NO: 516**
   CAGGCCAGTGAGAGTGTTTTTAATAATATGTTATCC
**SEQ ID NO: 517**
   GATGCATCCGATCTGGCATCT
**SEQ ID NO: 518**
   GCAGGGTATAAAAGTGATAGTAATGATGGCGATAATGTT
**SEQ ID NO: 519**
   AGGAATTCAATAACC
**SEQ ID NO: 520**
   ATCATTACTGGTAGTGGTAGAACGTACTACGCGAACTGGGCAAAAGGC
**SEQ ID NO: 521**
   GGCCATCCTGGTCTTGGTAGTGGTAACATC
**SEQ ID NO: 524**
   QSSQSVYNNYLS
**SEQ ID NO: 525**
   TASSLAS
**SEQ ID NO: 526**
   QGYYSGPIIT
**SEQ ID NO: 527**
   NYYIQ
**SEQ ID NO: 528**
   IIYAGGSAYYATWANG
**SEQ ID NO: 529**
   GTFDGYEL
**SEQ ID NO: 532**
   CAGTCCAGTCAGAGTGTTTATAATAACTACTTATCC
**SEQ ID NO: 533**
   ACTGCATCCAGCCTGGCATCT
**SEQ ID NO: 534**
   CAAGGCTATTATAGTGGTCCTATAATTACT
**SEQ ID NO: 535**
   AACTACTACATACAA
**SEQ ID NO: 536**
   ATCATTTATGCTGGTGGTAGCGCATACTACGCGACCTGGGCAAACGGC
**SEQ ID NO: 537**
   GGGACATTTGATGGTTATGAGTTG
**SEQ ID NO: 540**
   QSSESVYSNNLLS
**SEQ ID NO: 541**
   RASNLAS
**SEQ ID NO: 542**
   QGYYSGVINS
**SEQ ID NO: 543**
   SYFMS
**SEQ ID NO: 544**
   FINPGGSAYYASWASG
**SEQ ID NO: 545**
   ILIVSYGAFTI
**SEQ ID NO: 548**
   CAGTCCAGTGAGAGCGTTTATAGTAATAACCTCTTATCC
**SEQ ID NO: 549**
   AGGGCATCCAATCTGGCATCT
**SEQ ID NO: 550**
   CAAGGCTATTATAGTGGTGTCATTAATAGT
**SEQ ID NO: 551**
   AGCTACTTCATGAGC
**SEQ ID NO: 552**
   TTCATTAATCCTGGTGGTAGCGCATACTACGCGAGCTGGGCGAGTGGC
**SEQ ID NO: 553**
   ATTCTTATTGTTTCTTATGGAGCCTTTACCATC
**SEQ ID NO: 556**
   QATESIGNELS
**SEQ ID NO: 557**
   SASTLAS
**SEQ ID NO: 558**
   QQGYSSANIDNA
**SEQ ID NO: 559**
   KYYMS
**SEQ ID NO: 560**
   YIDSTTVNTYYATWARG
**SEQ ID NO: 561**
   GSTYFTDGGHRLDL
**SEQ ID NO: 564**
   CAGGCCACTGAGAGCATTGGCAATGAGTTATCC
**SEQ ID NO: 565**
   TCTGCATCCACTCTGGCATCT
**SEQ ID NO: 566**
   CAACAGGGTTATAGTAGTGCTAATATTGATAATGCT
**SEQ ID NO: 567**
   AAGTACTACATGAGC
**SEQ ID NO: 568**
   TACATTGATAGTACTACTGTTAATACATACTACGCGACCTGGGCGAGAGGC
**SEQ ID NO: 569**
   GGAAGTACTTATTTTACTGATGGAGGCCATCGGTTGGATCTC
**SEQ ID NO: 572**
   QATESIGNELS
**SEQ ID NO: 573**
   SASTLAS
**SEQ ID NO: 574**
   QQGYSSANIDNA
**SEQ ID NO: 575**
   TYNMG
**SEQ ID NO: 576**
   SITIDGRTYYAS WAKG
**SEQ ID NO: 577**
   ILIVSYGAFTI
**SEQ ID NO: 580**
   CAGGCCACTGAGAGCATTGGCAATGAGTTATCC
**SEQ ID NO: 581**
   TCTGCATCCACTCTGGCATCT
**SEQ ID NO: 582**
   CAACAGGGTTATAGTAGTGCTAATATTGATAATGCT
**SEQ ID NO: 583**
   ACCTACAACATGGGC
**SEQ ID NO: 584**
   AGTATTACTATTGATGGTCGCACATACTACGCGAGCTGGGCGAAAGGC
**SEQ ID NO: 585**
   ATTCTTATTGTTTCTTATGGGGCCTTTACCATC
**SEQ ID NO: 590**
   VPPGEDSKDVAAPHR
**SEQ ID NO: 591**
   GEDSKDVAAPHRQPL
**SEQ ID NO: 592**
   SKDVAAPHRQPLTSS
**SEQ ID NO: 593**
   VAAPHRQPLTSSERI
**SEQ ID NO: 594**
   PHRQPLTSSERIDKQ
**SEQ ID NO: 595**
   QPLTSSERIDKQIRY
**SEQ ID NO: 596**
   TSSERIDKQIRYILD
**SEQ ID NO: 597**
   ERIDKQIRYILDGIS
**SEQ ID NO: 598**
   DKQIRYILDGISALR
**SEQ ID NO: 599**
   IRYILDGISALRKET
**SEQ ID NO: 600**
   ILDGISALRKETCNK
**SEQ ID NO: 601**
   GISALRKETCNKSNM
**SEQ ID NO: 602**
   ALRKETCNKSNMCES
**SEQ ID NO: 603**
   KETCNKSNMCESSKE
**SEQ ID NO: 604**
   CNKSNMCESSKEALA
**SEQ ID NO: 605**
   SNMCESSKEALAENN
**SEQ ID NO: 606**
   CESSKEALAENNLNL
**SEQ ID NO: 607**
   SKEALAENNLNLPKM
**SEQ ID NO: 608**
   ALAENNLNLPKMAEK
**SEQ ID NO: 609**
   ENNLNLPKMAEKDGC
**SEQ ID NO: 610**
   LNLPKMAEKDGCFQS
**SEQ ID NO: 611**
   PKMAEKDGCFQSGFN
**SEQ ID NO: 612**
   AEKDGCFQSGFNEET
**SEQ ID NO: 613**
   DGCFQSGFNEETCLV
**SEQ ID NO: 614**
   FQSGFNEETCLVKII
**SEQ ID NO: 615**
   GFNEETCLVKIITGL
**SEQ ID NO: 616**
   EETCLVKIITGLLEF
**SEQ ID NO: 617**
   CLVKIITGLLEFEVY
**SEQ ID NO: 618**
   KIITGLLEFEVYLEY
**SEQ ID NO: 619**
   TGLLEFEVYLEYLQN
**SEQ ID NO: 620**
   LEFEVYLEYLQNRFE
**SEQ ID NO: 621**
   EVYLEYLQNRFESSE
**SEQ ID NO: 622**
   LEYLQNRFES SEEQA
**SEQ ID NO: 623**
   LQNRFESSEEQARAV
**SEQ ID NO: 624**
   RFESSEEQARAVQMS
**SEQ ID NO: 625**
   SSEEQARAVQMSTKV
**SEQ ID NO: 626**
   EQARAVQMSTKVLIQ
**SEQ ID NO: 627**
   RAVQMSTKVLIQFLQ
**SEQ ID NO: 628**
   QMSTKVLIQFLQKKA
**SEQ ID NO: 629**
   TKVLIQFLQKKAKNL
**SEQ ID NO: 630**
   LIQFLQKKAKNLDAI
**SEQ ID NO: 631**
   FLQKKAKNLDAITTP
**SEQ ID NO: 632**
   KKAKNLDAITTPDPT
**SEQ ID NO: 633**
   KNLDAITTPDPTTNA
**SEQ ID NO: 634**
   DAITTPDPTTNASLL
**SEQ ID NO: 635**
   TTPDPTTNASLLTKL
**SEQ ID NO: 636**
   DPTTNASLLTKLQAQ
**SEQ ID NO: 637**
   TNASLLTKLQAQNQW
**SEQ ID NO: 638**
   SLLTKLQAQNQWLQD
**SEQ ID NO: 639**
   TKLQAQNQWLQDMTT
**SEQ ID NO: 640**
   QAQNQWLQDMTTHLI
**SEQ ID NO: 641**
   NQWLQDMTTHLILRS
**SEQ ID NO: 642**
   LQDMTTHLILRSFKE
**SEQ ID NO: 643**
   MTTHLILRSFKEFLQ
**SEQ ID NO: 644**
   HLILRSFKEFLQSSL
**SEQ ID NO: 645**
   LRSFKEFLQSSLRAL
**SEQ ID NO: 646**
   FKEFLQSSLRALRQM
**SEQ ID NO: 659**
   IIYGSDETAYATSAIG
**SEQ ID NO: 694**
   CAGGCCAGTCAGAGCATTAACAATGAGTTATCC
**SEQ ID NO: 695**
   CAACAGGGTTATAGTCTGAGGAACATTGATAATGCT
**SEQ ID NO: 696**
   ATCATCTATGGTAGTGATGAAACCGCCTACGCTACCTCCGCTATAGGC
**SEQ ID NO: 697**
   GATGATAGTAGTGACTGGGATGCAAAGTTCAACTTG
**SEQ ID NO: 710**
   RASQGIRNDLG
**SEQ ID NO: 711**
   RASQGISNYLA
**SEQ ID NO: 712**
   RASQSISSWLA
**SEQ ID NO: 713**
   AASSLQS
**SEQ ID NO: 714**
   AASTLQS
**SEQ ID NO: 715**
   KASSLES
**SEQ ID NO: 716**
   SNYMS
**SEQ ID NO: 717**
   VIYSGGSTYYADSVKG
**SEQ ID NO: 718**
   VIYSGGSSTYYADSVKG

## Claims

1. A composition comprising an anti-IL-6 antibody that comprises a light chain polypeptide that comprises the light chain variable region of SEQ ID NO:709 and the light chain constant region of SEQ ID NO:586; and a heavy chain polypeptide that comprises the heavy chain variable region of SEQ ID NO:657 and the heavy chain constant region of SEQ ID NO:588, wherein the anti-IL-6 antibody is aglycosylated, for use in a method of preventing or treating a condition in a human subject associated with elevated IL-6 selected from rheumatoid arthritis, cancer, advanced cancer, liver disease, renal disease, inflammatory bowel disease, celiac disease, trauma, burns, other diseases associated with reduced serum albumin, juvenile rheumatoid arthritis, psoriasis, psoriatic arthropathy, ankylosing spondylitis, systemic lupus erythematosus, Crohn's disease, ulcerative colitis, pemphigus, dermatomyositis, polymyositis, polymyalgia rheumatica, giant cell arteritis, vasculitis, polyarteritis nodosa, Wegener's granulomatosis, Kawasaki disease, isolated CNS vasculitis, Churg-Strauss arteritis, microscopic polyarteritis, microscopic polyangiitis, Henoch-Schönlein purpura, essential cryoglobulinemic vasculitis, rheumatoid vasculitis, cryoglobulinemia, relapsing polychondritis, Behçet's disease, Takayasu's arteritis, ischemic heart disease, stroke, multiple sclerosis, sepsis, vasculitis secondary to viral infection (e.g., hepatitis B, hepatitis C, HIV, cytomegalovirus, Epstein-Barr virus, Parvo B 19 virus, etc.), Buerger's Disease, osteoarthritis, systemic sclerosis, CREST syndrome, Reiter's disease, Paget's disease of bone, Sjogren's syndrome, diabetes type 1, diabetes type 2, familial Mediterranean fever, autoimmune thrombocytopenia, autoimmune hemolytic anemia, autoimmune thyroid diseases, pernicious anemia, vitiligo, alopecia areata, primary biliary cirrhosis, autoimmune chronic active hepatitis, alcoholic cirrhosis, viral hepatitis including hepatitis B and C, idiopathic pulmonary fibrosis, chronic obstructive pulmonary disease, allergic asthma, or any combination thereof.

2. The composition of claim 1, wherein the patient has elevated C-reactive protein prior to treatment.

## Patentansprüche

1. Zusammensetzung, umfassend einen Anti-IL-6-Antikörper, der ein Leichte-Kette-Polypeptid, das die Leichte-Kette-variable-Region der SEQ ID NO:709 und die Leichte-Kette-konstante-Region der SEQ ID NO:586 umfasst; und ein Schwere-Kette-Polypeptid, das die Schwere-Kette-variable-Region der SEQ ID NO:657 und die Schwere-Kette-konstante-Region der SEQ ID NO:588 umfasst, umfasst, wobei der Anti-IL-6-Antikörper aglykosyliert ist, zur Verwendung bei einem Verfahren zur Vorbeugung oder Behandlung eines Leidens in Zusammenhang mit erhöhtem IL-6 bei einem menschlichen Individuum, ausgewählt aus rheumatoider Arthritis, Krebs, Krebs im fortgeschrittenen Stadium, Leberkrankheit, Nierenkrankheit, entzündlicher Darmerkrankung, Zöliakie, Trauma, Verbrennungen, anderen Krankheiten in Zusammenhang mit verringertem Serumalbumin, juveniler rheumatoider Arthritis, Psoriasis, Psoriasisarthropathie, ankylosierender Spondylitis, systemischem Lupus erythematodes, Morbus Crohn, Colitis ulcerosa, Pemphigus, Dennatomyositis, Polymyositis, Polymyalgia rheumatica, Riesenzellarteriitis, Vaskulitis, Polyarteritis nodosa, Wegener-Granulomatose, Kawasaki-Syndrom, isolierter ZNS-Vaskulitis, Churg-Strauss-Arteriitis, mikroskopischer Polyarteriitis, mikroskopischer Polyangiitis, Purpura Schönlein-Henoch, essentieller kryoglobulinämischer Vaskulitis, rheumatoider Vaskulitis, Kryoglobulinämie, rezidiver Polychondritis, Morbus Behçet, Takayasu-Arteriitis, ischämischer Herzkrankheit, Schlaganfall, multipler Sklerose, Sepsis, zu Virusinfektion (z. B. Hepatitis B, Hepatitis C, HIV, Cytomegalovirus, Epstein-Barr-Virus, Parvo-B-19-Virus usw.) sekundärer Vaskulitis, Buerger-Syndrom, Osteoarthritis, systemischer Sklerose, CREST-Syndrom, Reiter-Krankheit, Morbus Paget des Knochens, Sjögren-Syndrom, Typ-1-Diabetes, Typ-2-Diabetes, familiärem Mittelmeerfieber, Autoimmunthrombozytopenie, autoimmunhämolytischer Anämie, Autoimmunkrankheiten der Schilddrüse, perniziöser Anämie, Vitiligo, Alopecia areata, primär biliärer Zirrhose, chronisch aktiver Autoimmunhepatitis, alkoholischer Zirrhose, Virushepatitis, einschließlich Hepatitis B und C, idiopathischer Lungenfibrose, chronisch obstruktiver Lungenerkrankung, allergischem Asthma oder einer beliebigen Kombination davon.

2. Zusammensetzung nach Anspruch 1, wobei der Patient vor der Behandlung erhöhtes C-reaktives Protein aufweist.

## Revendications

1. Composition comprenant un anticorps anti-IL-6 qui comprend un polypeptide à chaîne légère qui comprend le domaine variable à chaîne légère de SEQ ID N° : 709 et le domaine constant à chaîne légère de SEQ ID N° : 586 ; et un polypeptide à chaîne lourde qui comprend le domaine variable à chaîne lourde de SEQ ID N° : 657 et le domaines constant à chaîne lourde de SEQ ID N° : 588, l'anticorps anti-IL-6 étant aglycosylé, pour une utilisation dans un procédé de prévention ou de traitement d'une affection chez un sujet humain associée à des taux élevés d'IL-6 choisie parmi l'arthrite rhumatoïde, un cancer, un cancer avancé, une maladie du foie, une maladie rénale, une maladie intestinale inflammatoire, la maladie cœliaque, un traumatisme, des brûlures, d'autres maladies associées à des taux réduits d'albumine sérique, l'arthrite rhumatoïde juvénile, le psoriasis, l'arthropathie psoriasique, la spondylarthrite ankylosante, le lupus érythémateux systémique, la maladie de Crohn, la colite ulcéreuse, le pemphigus, la dermatomyosite, la polymyosite, la pseudopolyarthrite rhizomélique, l'artérite à cellules géantes, la vascularite, la périartérite noueuse, la granulomatose de Wegener, la maladie de Kawasaki, une vascularite du SNC isolée, l'artérite de Churg-Strauss, la polyartérite microscopique, la polyangéite microscopique, le purpura de Henoch-Schönlein, la vascularite cryoglobulinémique essentielle, la vascularite rhumatoïde, la cryoglobulinémique, la polychondrite récurrente, la maladie de Behçet, l'artérite de Takayasu, une maladie cardiaque ischémique, un accident vasculaire cérébral, la sclérose en plaques, une septicémie, une vascularite secondaire à une infection virale (par ex. l'hépatite B, l'hépatite C, le VIH, un cytomégalovirus, le virus d'Epstein-Barr, le virus Parvo B 19, etc.), la maladie de Buerger, l'ostéoarthrite, la sclérose systémique, le syndrome CREST, la maladie de Reiter, la maladie des os de Paget, le syndrome de Sjogren, le diabète de type 1, le diabète de type 2, la fièvre méditerranéenne familiale, la thrombocytopénie auto-immune, l'anémie hémolytique auto-immune, les maladies auto-immunes de la thyroïde, l'anémie pernicieuse, le vitiligo, l'alopécie en aires, la cirrhose biliaire primaire, l'hépatite active chronique auto-immune, la cirrhose alcoolique, l'hépatite virale y compris l'hépatite B et C, la fibrose pulmonaire idiopathique, une maladie pulmonaire chronique obstructive, l'asthme allergique, et une quelconque combinaison correspondante.

2. Composition selon la revendication 1, le patient ayant des taux élevés de protéine C-réactive avant le traitement.
